# EUROPEAN PATENT APPLICATION

(11) **EP 4 324 825 A1**
(43) Date of publication of application: **21.02.2024**
(21) Application number: 22787662.0
(22) Date of filing: 18.04.2022
(51) Int. Cl.: C07D 307/79, C07D 307/85, C07D 333/70, A61K 31/343, A61P 3/00, A61P 35/00

(54) **FXR REGULATOR, PREPARATION METHOD THEREFOR, PHARMACEUTICAL COMPOSITION THEREOF AND USE THEREOF**

(30) Priority: 16.04.2021 CN 202110414367
(71) Applicant: Beijing Best Pathway Management Consulting Center (Limited Partnership), Beijing 100015 (CN)
(72) Inventor: JIAO, Ning, Beijing 100191 (CN); DOU, Xiaodong, Beijing 100191 (CN); HUO, Tongyu, Beijing 100191 (CN); SU, Lingyu, Beijing 100191 (CN); ZHAO, Xinyi, Beijing 100191 (CN); HUANG, Yingjie, Beijing 100191 (CN); LIU, Yameng, Beijing 100191 (CN)
(74) Representative: Kolster Oy Ab
(86) International application number: PCT/CN2022/087481
(87) International publication number: WO 2022/218440

(57) **Abstract**

The present invention relates to a compound capable of being used as an FXR regulator, a pharmaceutically acceptable salt or ester, a stereoisomer, a tautomer, a geometric isomer, a prodrug, a hydrate, a solvate or a crystalline form thereof, a metabolite form of same, or any combination or mixture of same, and also relates to a preparation method therefor, a pharmaceutical composition thereof and the use thereof.

## Description

### TECHNICAL FIELD

This application relates to but is not limited to medical technology, and specifically relates to an FXR modulator, a preparation method therefor, and pharmaceutical composition and use thereof.

### BACKGROUND

The superfamily of nuclear receptors (NRs), consisting of 48 ligand-dependent transcription factors, controls the expression of specific genes, regulates physiological processes such as metabolism, development, and reproduction in mammals, and is related to a variety of human diseases, including inflammation, cancers, reproductive disorders, metabolic syndrome, and cardiovascular diseases. The activity of the NRs superfamily can be modulated by binding to lipophilic small molecule ligands, which enter the lipid bilayer to modulate physiological activities inside and outside cells. Based on ligand selectivity, the NRs superfamily is usually divided into three classes: class I, class II, and class III. Class I refers to classic endocrine nuclear receptors, with steroid hormones synthesized by endocrine glands as their ligands, including estrogen receptor (ER), progesterone receptor (PR), androgen receptor (AR), glucocorticoid receptor (GR), and mineralocorticoid receptor (MR). These NRs bind to their target DNA sequences in the form of homodimers. Class II refers to orphan NRs for which corresponding ligands have not yet been found. They typically bind to DNA in the form of monomers or homodimers, such as retinoid-related orphan receptors (RORs) and nerve growth factors IB (Nurr77) [9]. Class III refers to adopted orphan NRs, which are the type II orphan nuclear receptors for which ligands have recently been found, including farnesoid X receptor (FXR), peroxisome proliferator-activated receptors (PPARs), liver X receptors (LXR), and retinoid X receptor (RXR).

In 1995, Seol et al. used a two-hybrid yeast system to identify a new protein that could interact with RXRα and named it RXR-interacting protein 14 (RIP14). In the same year, Forman et al. cloned the homologous gene of mouse RIP14 from a rat liver cDNA library and found that farnesol, an intermediate in the mevalonic acid pathway, could activate rat RIP14 at supraphysiological concentrations. Therefore, RIP14 was renamed FXR. It is known that FXR has two genes, namely FXRα (NR1H4) and FXRβ (NR1H5). Existing studies have shown that FXRα plays an important role in various metabolic homeostasis and disease regulation. Different from FXRα, FXRβ is a pseudogene in humans and primates and is expressed in rodents, rabbits and dogs, and its function and role are not yet clear. FXRs are more expressed in tissues such as liver, intestine, kidney and adrenal gland, and less expressed in adipose tissue and heart ^{[19]}. Four FXRα isoforms are all expressed in a tissue-specific manner. FXRα1 and FXRα2 are mainly expressed in the human liver, FXRα3 and FXRα4 are mainly expressed in the human kidney and colon, and all the four isoforms are expressed in the human small intestine.

The protein structure of the FXR consists of 486 amino acids. The full-length crystal structure has not yet been analyzed. However, multiple co-crystal structures of FXR-LBD (amino acid sequence 248-472) combined with ligands have been analyzed, providing a basis for the discovery of FXR ligands and rational drug design. Previous studies have shown that FXR affects various processes of human metabolism by modulating bile acids (BAs), lipids, glucose and energy homeostasis. In view of this, the FXR becomes a potential therapeutic target for the treatment of diseases such as cholestasis, metabolic syndrome, inflammatory bowel disease, cardiovascular diseases, and liver regenerative tumors (Sun, L.; Cai, J.; Gonzalez, F. J., The role of farnesoid X receptor in metabolic diseases, and gastrointestinal and liver cancer. Nat Rev Gastroenterol Hepatol 2021; Han, C. Y, Update on FXR Biology: Promising Therapeutic Target? Int J Mol Sci 2018, 19.).

FXR modulators include agonists and inhibitors (or antagonists). The types of agonists include steroids (such as primary BAs (CA and CDCA), and secondary BAs (DCA and LCA), INT-767, MFA-1, NIHS700, and TC-100) and non-steroidal ones (such as 1.5.1.2.1 isoxazole derivatives-GW4064, aza[4,5-b]indole derivatives-WAY-362450, benzene imidazole derivatives, Fexaramine derivatives, and o-aminobenzoic acid derivatives). The types of antagonists include natural product FXR antagonists (such as steroid derivatives, terpenoid derivatives, Tuberatolide derivatives, atractylodes lactone derivatives, andrographolide derivatives, chalcone derivatives, and phenol derivatives) and synthetic FXR antagonists (such as isoxazole derivatives-GW4064, 1,3,4-trisubstituted pyrazolone derivatives, 2,3,4-trisubstituted pyrazole compounds, pyrazole-4-carboxamide derivatives, phenylalkyl ether derivatives, 3-*tert*-butyl benzoate/amide derivatives, benzimidazole derivatives, *tert-*butylbenzene derivatives, and oxadiazole derivatives).

Those skilled in the art are still eager for a novel structure of FXR modulator, which can be used for the prevention or treatment of metabolism-related diseases, cardiovascular diseases, liver diseases, cancers, kidney diseases, enteropathy, inflammation and other diseases.

### SUMMARY OF THE INVENTION

This application provides a compound that can be used as an FXR modulator, which not only can be used as an FXR antagonist, but also has low toxicity, high activity, significant lipid-lowering activity, and improved safety and/or efficacy.

In one aspect, this application provides a benzofuran derivative of formula (I): 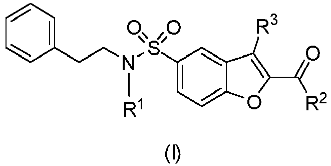
wherein R¹ is -(CH₂)ₙ-R⁴, wherein n is an integer from 0 to 6, R⁴ is an unsubstituted aromatic hydrocarbyl, a substituted aromatic hydrocarbyl, an unsubstituted heteroaryl, or a substituted heteroaryl; the substituted aromatic hydrocarbyl or the substituted heteroaryl means that the aromatic hydrocarbyl or heteroaryl is substituted with one or more groups selected from Group A;
in formula (I), R² is a hydroxyl, an unsubstituted C1-C6 alkoxy, a substituted C1-C6 alkoxy, or -NR⁵R⁶, wherein R⁵ and R⁶ here are each independently selected from a group consisting of hydrogen, unsubstituted C1-C6 alkyl, and substituted C1-C6 alkyl; the substituted C1-C6 alkoxy means that one or more hydrogens on the C1-C6 alkoxy are substituted by groups selected from a group consisting of phenyl, halogen, nitro, hydroxyl and C1-C6 alkyl; the substituted C1-C6 alkyl means that one or more hydrogens on the C1-C6 alkyl are substituted by groups selected from a group consisting of phenyl, halogen, hydroxyl and C1-C6 alkyl;
R³ is an unsubstituted C1-C6 alkyl or a substituted C1-C6 alkyl;
the group in Group A refers to one or more of the following groups: halogen, nitro, cyano, substituted or unsubstituted aromatic hydrocarbyl, substituted or unsubstituted aryloxy, unsubstituted C1-C6 alkyl, unsubstituted C2-C6 alkenyl, carboxyl-substituted C2-C6 alkenyl, unsubstituted C2-C6 alkynyl, carboxyl-substituted C2-C6 alkynyl, carbamoyl, halogenated C1-C6 alkyl, aromatic hydrocarbyl-substituted C1-C6 alkyl, carboxyl-substituted C1-C6 alkyl, C1-C6 alkoxycarbonyl, unsubstituted C1-C6 alkoxy, halogenated C1-C6 alkoxy, unsubstituted C1-C6 alkylthio group, halogenated C1-C6 alkylthio group, C1-C6 alkylamide group, and substituted or unsubstituted heterocycloalkyl, wherein the substituted aromatic hydrocarbyl, substituted aryloxy or substituted heterocycloalkyl means that an aromatic hydrocarbyl, aryloxy or heterocycloalkyl is substituted with one or more groups selected from Group B, or that a C1-C6 alkyl and N in a heterocycloalkyl form a quaternary ammonium;
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C6 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, and C1-C6 alkoxycarbonyl;
it is also required that,
when R¹ is -(CH₂)ₙ-R³ (n is 1), R⁴ is phenyl and R³ is methyl, R² is not hydroxyl;
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

In another aspect, this application provides use of a benzofuran derivative of formula (II) as an FXR modulator: 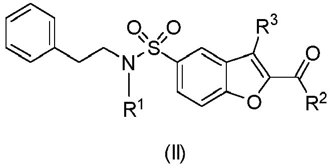
wherein R¹ is -(CH₂)ₙ-R⁴, wherein n is an integer from 0 to 6, R⁴ is an unsubstituted aromatic hydrocarbyl, a substituted aromatic hydrocarbyl, an unsubstituted heteroaryl, or a substituted heteroaryl; the substituted aromatic hydrocarbyl or the substituted heteroaryl means that the aromatic hydrocarbyl or heteroaryl is substituted with one or more groups selected from Group A;
in formula (II), R² is a hydroxyl, an unsubstituted C1-C6 alkoxy, a substituted C1-C6 alkoxy, or -NR⁵R⁶, wherein R⁵ and R⁶ here are each independently selected from a group consisting of hydrogen, unsubstituted C1-C6 alkyl, and substituted C1-C6 alkyl; the substituted C1-C6 alkoxy means that one or more hydrogens on the C1-C6 alkoxy are substituted by groups selected from a group consisting of phenyl, halogen, nitro, hydroxyl and C1-C6 alkyl; the substituted C1-C6 alkyl means that one or more hydrogens on the C1-C6 alkyl are substituted by groups selected from a group consisting of phenyl, halogen, hydroxyl and C1-C6 alkyl;
R³ is an unsubstituted C1-C6 alkyl or a substituted C1-C6 alkyl;
the group in Group A refers to one or more of the following groups: halogen, nitro, cyano, substituted or unsubstituted aromatic hydrocarbyl, substituted or unsubstituted aryloxy, unsubstituted C1-C6 alkyl, unsubstituted C2-C6 alkenyl, carboxyl-substituted C2-C6 alkenyl, unsubstituted C2-C6 alkynyl, carboxyl-substituted C2-C6 alkynyl, carbamoyl, halogenated C1-C6 alkyl, aromatic hydrocarbyl-substituted C1-C6 alkyl, carboxyl-substituted C1-C6 alkyl, C1-C6 alkoxycarbonyl, unsubstituted C1-C6 alkoxy, halogenated C1-C6 alkoxy, unsubstituted C1-C6 alkylthio group, halogenated C1-C6 alkylthio group, C1-C6 alkylamide group, and substituted or unsubstituted heterocycloalkyl, wherein the substituted aromatic hydrocarbyl, substituted aryloxy or substituted heterocycloalkyl means that an aromatic hydrocarbyl, aryloxy or heterocycloalkyl is substituted with one or more groups selected from Group B, or that a C1-C6 alkyl and N in a heterocycloalkyl form a quaternary ammonium;
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C6 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, and C1-C6 alkoxycarbonyl;
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

In a third aspect, this application provides a pharmaceutical composition, comprising a pharmacologically effective dose of the benzofuran derivative of formula (I) or formula (II) or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

In a fourth aspect, this application provides a preparation method of the benzofuran derivative of formula (I) or formula (II) or a pharmaceutically acceptable salt or ester, stereoisomer, tautomer, geometric isomer, prodrug, hydrate, solvate and crystal form of the benzofuran derivative, metabolite forms thereof, or any combination or mixture thereof, comprising the following step:

allowing 2-phenylethylamine to react with a compound of formula (III) to obtain a compound of formula (IV); 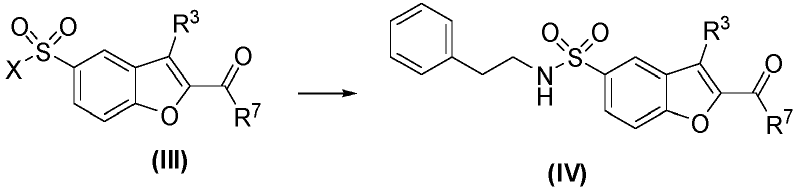
in a case wherein R¹ is not hydrogen, after obtaining the compound of formula (IV), further comprising: allowing the compound of formula (IV) to react with X-R¹ to obtain a compound of formula (V); 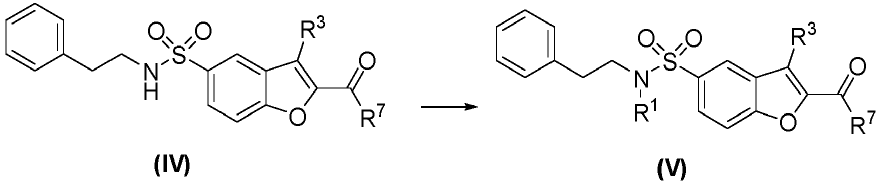
optionally, further comprising: carrying out group transformation on the compound of formula (IV) or the compound of formula (V) to obtain the compound of formula (I) or the compound of formula (II); 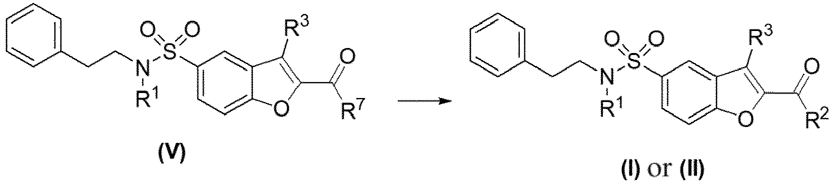
alternatively,
allowing the compound of formula (III) to react with the compound of formula (VI) to obtain the compound of formula (V); 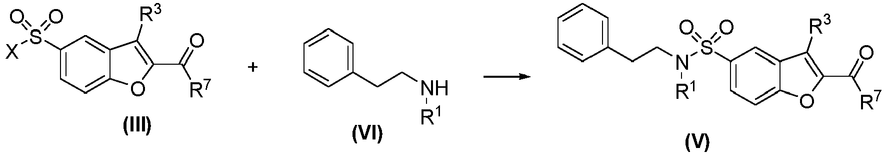
optionally, further comprising: carrying out group transformation on the compound of formula (V) to obtain the compound of formula (I) or the compound of formula (II); 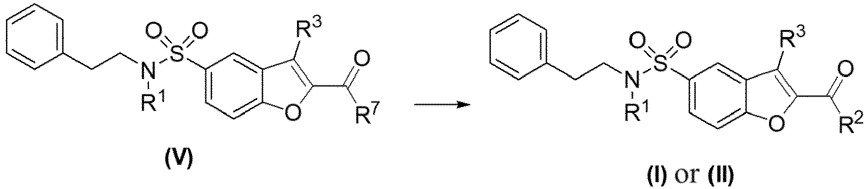 ;
wherein X in formula (III) is chlorine, bromine or iodine; R⁷ in formula (III), formula (IV) and formula (V) is a C1-C6 alkyl; R¹ in formula (V) and formula (VI) and R³ in formula (III), formula (IV) and formula (V) are as defined in the above-described formula (I) or formula (II).

### DETAILED DESCRIPTION

As used herein, "aromatic hydrocarbyl" and "aryl" are used interchangeably. In some embodiments of this application, the aromatic hydrocarbyl in the unsubstituted aromatic hydrocarbyl or the substituted aromatic hydrocarbyl is phenyl, naphthyl, or indene.

As used herein, "heteroaryl" refers to an aromatic cyclic group in which at least one (such as 1, 2 or 3) ring atom is a heteroatom (such as a nitrogen atom, an oxygen atom, or a sulfur atom). Optionally, ring atoms (such as carbon atoms, nitrogen atoms, or sulfur atoms) in the cyclic structure may be oxo-substituted. In a case of multiple heteroatoms, the heteroatoms may be the same or different. The number of ring atoms of the heteroaryl is preferably 5 to 14, more preferably 5 to 6 or 9 to 10. In some embodiments, the heteroaryl is a 5- to 6-membered nitrogen-containing heteroaryl (e.g., pyrrolyl, imidazolyl, oxazolyl, pyridyl, or pyrimidinyl). In some embodiments, the heteroaryl is a 5- to 6-membered oxygen-containing heteroaryl (e.g., furyl, oxazolyl or a group obtained by condensing it with a benzene ring). In some embodiments, the heteroaryl is thienyl.

In some embodiments of this application, the heteroaryl in the unsubstituted heteroaryl or the substituted heteroaryl is furyl, thienyl, pyrrolyl, oxazolyl, thiazolyl, imidazolyl, pyrazolyl, triazolyl, tetrazolyl, isoxazolyl, isothiazolyl, oxadiazolyl, thiadiazolyl, pyridyl, pyrimidinyl, pyrazinyl, pyridazinyl, triazinyl, quinolinyl , isoquinolinyl, cinnolinyl, phthalazinyl, quinazolinyl, quinoxalinyl, naphthyridinyl, pteridinyl, indolizinyl, azaindolizinyl, indolyl, azaindolyl, indazolyl, azaindazolyl, benzimidazolyl, azabenzimidazolyl, benzotriazolyl, azabenzotriazolyl, benzoxazolyl, azabenzoxazolyl, benzisoxazolyl, azabenzisoxazolyl, benzofuranyl, azabenzofuranyl, benzothienyl, azabenzothienyl, purinyl, carbazolyl, or the like.

As used herein, "heterocyclyl" and "heterocycloalkyl" can be used interchangeably. The "heterocyclyl" or "heterocycloalkyl" refers to a non-aromatic cyclic group in which at least one (such as 1, 2 or 3) ring atom is a heteroatom (such as a nitrogen atom, an oxygen atom, or a sulfur atom). Optionally, ring atoms (such as carbon atoms, nitrogen atoms, or sulfur atoms) in the cyclic structure may be oxo-substituted. In a case of multiple heteroatoms, the heteroatoms may be the same or different. The number of ring atoms of the heterocyclyl is preferably 3 to 10, such as 5 to 7, 9 to 10, or 6 to 8.

As used herein, the heterocyclyl may include single heterocyclyl, spiroheterocyclyl, bridged heterocyclyl and fused heterocyclyl.

As used herein, the "spiroheterocyclyl" refers to a ring structure formed by two or more cyclic structures sharing one ring atom, and in which at least one ring atom is a heteroatom (such as a nitrogen atom, an oxygen atom, or a sulfur atom). Optionally, ring atoms (such as carbon atoms, nitrogen atoms, or sulfur atoms) in the cyclic structure may be oxo-substituted. The number of ring atoms of the spiroheterocyclyl is preferably 7 to 14 members, such as 9 to 10 members. Specific examples include but are not limited to 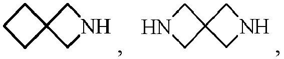 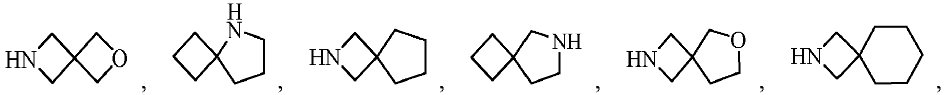 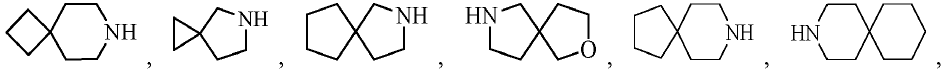 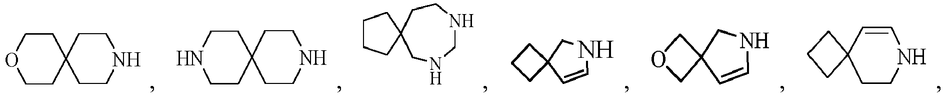 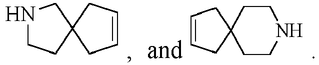 In some embodiments, the spiroheterocyclyl is nitrogen-containing spiroheterocyclyl, such as 9- to 10-membered nitrogen-containing spiroheterocyclyl.

As used herein, the "bridged heterocyclyl" refers to a ring structure formed by two or more cyclic structures sharing two non-adjacent ring atoms, and in which at least one ring atom is a heteroatom (such as a nitrogen atom, an oxygen atom, or an sulfur atom). Optionally, ring atoms (such as carbon atoms, nitrogen atoms, or sulfur atoms) in the cyclic structure may be oxo-substituted. The number of ring atoms of the spiroheterocyclyl is preferably 6 to 14 members, such as 6 to 8 members. Specific examples include but are not limited to 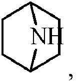 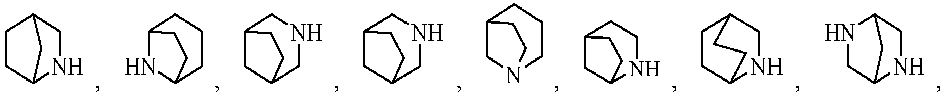 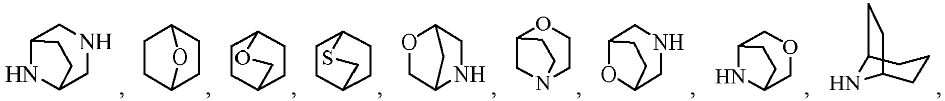 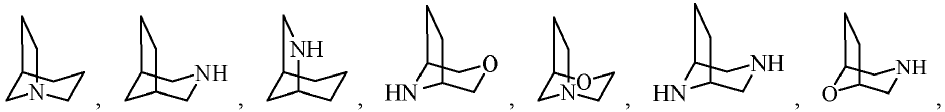 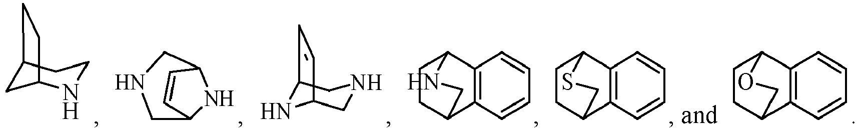 In some embodiments, the bridged heterocyclyl is nitrogen-containing bridged heterocyclyl, such as 6-to 8-membered nitrogen-containing bridged heterocyclyl.

In some embodiments, the heterocyclyl is 5- to -6-membered nitrogen-containing monoheterocyclyl. In some embodiments, the heteroaryl is 5- to -6-membered oxygen-containing monoheterocyclyl.

In some embodiments of this application, the heterocycloalkyl is azeridinyl,azetidinyl, oxetanyl, thietanyl, pyrrolidinyl, tetrahydrofuranyl, tetrahydrothienyl, oxoxazolidinyl, tetrahydropyranyl, piperidyl, morpholinyl, thiomorpholinyl, piperazinyl, homopiperidinyl, oxepanyl, thiazepanyl, oxazazoyl, dioxazoyl, or thiazepanyl.

As used herein, the "3- to 6-membered cycloalkyl" refers to a saturated cyclic alkyl containing 3 to 6 carbon atoms. In some embodiments of this application, the 3- to 6-membered cycloalkyl is selected from a group consisting of cyclopropyl, cyclobutyl, cyclopentyl, and cyclohexyl.

As used herein, "a carbon atom, a nitrogen atom or a sulfur atom is oxo-substituted " means forming a structure of C=O, N=O or S=O.

In some embodiments of this application, the halogen is fluorine, chlorine, bromine, or iodine.

In some embodiments of this application, the unsubstituted C1-C6 alkyl is methyl, ethyl, n-propyl, isopropyl, n-butyl, isobutyl, sec-butyl, *tert*-butyl, n-pentyl, neopentyl, or n-hexyl.

In some embodiments of the application, the unsubstituted C2-C6 alkenyl is vinyl, 1-propen-1-yl, allyl, 1-buten-1-yl, 1-pentene-1 -yl, 1,3-butadien-1-yl, 1,4-pentadien-1-yl, and the like.

In some embodiments of this application, the unsubstituted C2-C6 alkynyl is ethynyl, 1-propyn-1-yl (i.e. propynyl), 1-butyn-1-yl (i.e. butynyl), pentyn-1-yl, 1,3-butadiyn-1-yl, 1,4-pentadiyn-1-yl, and the like.

In some embodiments of this application, the unsubstituted C1-C6 alkoxy is methoxy, ethoxy, n-propoxy, isopropoxy, n-butoxy, isobutoxy, sec-butoxy, *tert*-butoxy, n-pentyloxy, neopentyloxy, or n-hexyloxy.

In some embodiments of this application, the halogenated C1-C6 alkyl refers to unsubstituted C1-C6 alkyl having hydrogens substituted by one or more groups selected from a group consisting of fluorine, chlorine, bromine, and iodine, such as trifluoromethyl.

In some embodiments of this application, the halogenated C1-C6 alkoxy refers to unsubstituted C1-C6 alkoxy having hydrogens substituted by one or more groups selected from a group consisting of fluorine, chlorine, bromine, and iodine, such as trifluoromethoxy.

In some embodiments of this application, the C1-C6 alkanoyl includes but is not limited to formyl, acetyl, n-propionyl, n-butyryl, isobutyryl, n-valeryl, and pivaloyl.

In some embodiments of this application, the pharmaceutically acceptable salt refers to a salt formed by reacting the compound of the present invention with a pharmaceutically acceptable inorganic acid or organic acid or inorganic base or organic base. The inorganic acid includes but is not limited to hydrochloric acid, hydrobromic acid, sulfuric acid, and phosphoric acid. The organic acid includes but is not limited to trifluoroacetic acid, citric acid, maleic acid, fumaric acid, succinic acid, tartaric acid, lactic acid, pyruvic acid, oxalic acid, formic acid, acetic acid, benzoic acid, methanesulfonic acid, benzenesulfonic acid, and p-toluenesulfonic acid. The inorganic base includes but is not limited to sodium hydroxide, potassium hydroxide, calcium hydroxide, and lithium hydroxide. The organic base includes but is not limited to diethylamine, triethylamine, tromethamine, and glucosamine.

In the embodiments of this application, the hydrate includes but is not limited to hemihydrates, monohydrates, dihydrates, and trihydrates. The solvate refers to a substance formed by the compound of the present invention or a pharmaceutically acceptable salt thereof and at least one solvent molecule, including but not limited to methanolates, ethanolates, and acetonates.

In the embodiments of this application, the tautomer refers to structural isomers with different energies that can be interconverted through a low energy barrier. If tautomerism is possible (such as in a solution), then chemical equilibrium of the tautomer is reached. For example, proton tautomers (or proton transfer tautomers) include but are not limited to interconversions through proton migration, such as keto-enol isomerization, imine-enamine isomerization, and amide-iminoalcohol isomerization.

In the embodiments of this application, the geometric isomer refers to a stereoisomer formed by the atoms (or groups) located on both sides of a double bond or ring system due to different positions relative to a reference plane; for example, the geometric isomer is cis or trans form, or E form and Z form.

In embodiments of this application, the prodrug refers to a derivative that can directly or indirectly provide the compound of the present invention after administration to a patient.

In the embodiments of this application, the metabolite refers to a compound that is obtained through biotransformation in vivo of the compound of the present invention.

In a first aspect, this application provides a benzofuran derivative of formula (I): 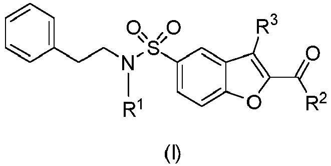
wherein R¹ is -(CH₂)ₙ-R⁴, wherein n is an integer from 0 to 6, R⁴ is an unsubstituted aromatic hydrocarbyl, a substituted aromatic hydrocarbyl, an unsubstituted heteroaryl, or a substituted heteroaryl; the substituted aromatic hydrocarbyl or the substituted heteroaryl means that the aromatic hydrocarbyl or heteroaryl is substituted with one or more groups selected from Group A;
in formula (I), R² is a hydroxyl, an unsubstituted C1-C6 alkoxy, a substituted C1-C6 alkoxy, or -NR⁵R⁶, wherein R⁵ and R⁶ here are each independently selected from a group consisting of hydrogen, unsubstituted C1-C6 alkyl, and substituted C1-C6 alkyl; the substituted C1-C6 alkoxy means that one or more hydrogens on the C1-C6 alkoxy are substituted by groups selected from a group consisting of phenyl, halogen, nitro, hydroxyl and C1-C6 alkyl; the substituted C1-C6 alkyl means that one or more hydrogens on the C1-C6 alkyl are substituted by groups selected from a group consisting of phenyl, halogen, hydroxyl and C1-C6 alkyl;
R³ is an unsubstituted C1-C6 alkyl or a substituted C1-C6 alkyl;
the group in Group A refers to one or more of the following groups: halogen, nitro, cyano, substituted or unsubstituted aromatic hydrocarbyl, substituted or unsubstituted aryloxy, unsubstituted C1-C6 alkyl, unsubstituted C2-C6 alkenyl, carboxyl-substituted C2-C6 alkenyl, unsubstituted C2-C6 alkynyl, carboxyl-substituted C2-C6 alkynyl, carbamoyl, halogenated C1-C6 alkyl, aromatic hydrocarbyl-substituted C1-C6 alkyl, carboxyl-substituted C1-C6 alkyl, C1-C6 alkoxycarbonyl, unsubstituted C1-C6 alkoxy, halogenated C1-C6 alkoxy, unsubstituted C1-C6 alkylthio group, halogenated C1-C6 alkylthio group, C1-C6 alkylamide group, and substituted or unsubstituted heterocycloalkyl, wherein the substituted aromatic hydrocarbyl, substituted aryloxy or substituted heterocycloalkyl means that an aromatic hydrocarbyl, aryloxy or heterocycloalkyl is substituted with one or more groups selected from Group B, or that a C1-C6 alkyl and N in a heterocycloalkyl form a quaternary ammonium;
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C6 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, and C1-C6 alkoxycarbonyl;
it is also required that,
when R¹ is -(CH₂)ₙ-R⁴ (n is 1), R⁴ is phenyl and R³ is methyl, R² is not hydroxyl;
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

In some embodiments of this application, R³ in formula (I) is methyl, ethyl, n-propyl, isopropyl, n-butyl, isobutyl, sec-butyl, *tert*-butyl, n-pentyl, or neopentyl.

In some embodiments of this application, R³ in formula (I) is methyl.

In some embodiments of this application, R² in formula (I) is hydroxyl.

In some embodiments of this application, R² in formula (I) is methoxy, ethoxy, n-propoxy, isopropoxy, n-butoxy, isobutoxy, sec-butoxy, *tert*-butoxy or benzyl; preferably, R² is ethoxy.

In some embodiments of this application, R² in formula (I) is -NR⁵R⁶, wherein R⁵ and R⁶ are each independently selected from a group consisting of hydrogen, methyl, ethyl, n-propyl, isopropyl, n-butyl, isobutyl, sec-butyl, *tert*-butyl, n-pentyl, neopentyl, benzyl, and phenethyl; preferably, R² is phenylethylamino.

In some embodiments of this application, in formula (I), n is 0; R⁴ is phenyl, naphthyl, pyridyl, or quinoline, or, phenyl, naphthyl, pyridyl or quinoline substituted with one or more of the following groups: benzyl, phenoxy, halogen, nitro, cyano, carboxyl, unsubstituted C1-C6 alkyl, halogenated C1-C6 alkyl, halogenated C1-C6 alkoxy, carbamoyl, halogenated C1-C6 alkylthio, C1-C6 alkanoyl, morpholinyl, piperazinyl, piperazinmethyl, and piperidinyl, wherein the piperazinyl, piperazinylmethyl and piperidinyl are optionally substituted with one or more of the following groups: unsubstituted C1-C6 alkyl, phenyl-substituted C1-C6 alkyl, unsubstituted C1-C6 alkoxycarbonyl, unsubstituted C1-C6 alkanoyl, phenyl-substituted C1-C6 alkanoyl, C1-C6 alkane sulfonyl, single- or double- C1-C6 substituted carbamoyl; or C1-C6 alkyl and N in morpholinyl, piperazinyl, piperazinmethyl, and piperidinyl form a quaternary ammonium.

In some embodiments of this application, in formula (I), n is 1 or 2; R⁴ is phenyl, naphthyl, pyridyl, or quinoline, or, phenyl, naphthyl, pyridyl or quinoline substituted with one or more of the following groups: benzyl, phenoxy, halogen, nitro, cyano, carboxyl, unsubstituted C1-C6, halogenated C1-C4 alkyl, halogenated C1-C4 alkoxy, C1-C6 alkanoyl, carboxyl-substituted C1-C6 alkyl, C1-C6 alkoxycarbonyl, carbamoyl, halogenated C1-C4 alkylthio, unsubstituted C2-C6 alkenyl, carboxyl-substituted C2-C6 alkenyl, carbamoyl, morpholinyl, piperazinyl, piperazinylmethyl and piperidinyl, wherein the piperazinyl, piperazinylmethyl and piperidinyl are optionally substituted with one or more of the following groups: C1-C6 alkyl, phenyl-substituted C1-C6 alkyl, C1-C6 alkoxycarbonyl, C1-C6 alkanoyl, phenyl-substituted C1-C6 alkanoyl, C1-C4 alkane sulfonyl, single- or double- C1-C4 substituted carbamoyl.

In some embodiments, n in formula (I) is 0. In some embodiments, n in formula (I) is 1. In some embodiments, n in formula (I) is 2.

In some embodiments, R⁴ in formula (I) is phenyl, naphthyl, pyridyl, or quinolyl.

In some embodiments, R⁴ in formula (I) is phenyl substituted with one or more groups selected from Group A. In some embodiments, R⁴ in formula (I) is naphthyl substituted with one or more groups selected from Group A. In some embodiments, R⁴ in formula (I) is pyridinyl substituted with one or more groups selected from Group A. In some embodiments, R⁴ in formula (I) is quinolinyl substituted with one or more groups selected from Group A.

In some embodiments, the group in Group A is selected from: benzyl, phenoxy, halogen, nitro, cyano, carboxyl, unsubstituted C1-C6 alkyl, halogenated C1-C6 alkyl, halogenated C1-C6 alkoxy, carbamoyl, halogenated C1-C6 alkylthio, C1-C6 alkanoyl, C1-C6 alkoxycarbonyl, C1-C6 alkoxycarbonyl-C1-C6 alkylene, carboxyl-C1-C6 alkylene, - C1-C6 alkoxycarbonyl-C2-C6 alkenylene, carboxyl-C2-C6 alkenylene, morpholinyl, piperazinyl, piperazinemethyl, piperidinyl, phenyl, and phenylcarbonyl;

In some embodiments, the group in Group B is selected from: cyano, unsubstituted C1-C6 alkyl, phenyl-substituted C1-C6 alkyl, unsubstituted C1-C6 alkoxycarbonyl, unsubstituted C1 -C6 alkanoyl, phenyl-substituted C1-C6 alkanoyl, C1-C6 alkane sulfonyl, single- or double- C1-C6 substituted carbamoyl; optionally, the phenyl is substituted with C1-C6 alkyl, cyano, halogen, halogenated C1-C6 alkyl, amino, nitro, acetamido, or C1-C6 alkanoyl. In some examples, the benzofuran derivative of formula (I) is selected from the following compounds:
ethyl 3-methyl-5-(N-benzyl-N-phenethylsulfamoyl)benzofuran-2-carboxylate (F27-S8);
ethyl 3-methyl-5-(N-(2-fluorobenzyl)-N-phenethylsulfamoyl)benzofuran-2-carboxylate (M4-14);
ethyl 3-methyl-5-(N-(2-chlorobenzyl)-N-phenethylsulfamoyl)benzofuran-2-carboxylate (M4-15);
ethyl 3-methyl-5-(N-(2-bromobenzyl)-N-phenethylsulfamoyl)benzofuran-2-carboxylate (M4-16);
ethyl 3-methyl-5-(N-(2-methylbenzyl)-N-phenethylsulfamoyl)benzofuran-2-carboxylate (M4-17);
ethyl 3-methyl-5-(N-(2-trifluoromethylbenzyl)-N-phenethylsulfamoyl)benzofuran-2-carboxylate (M4-18);
ethyl 3-methyl-5-(N-(2-nitrobenzyl)-N-phenethylsulfamoyl)benzofuran-2-carboxylate (M4-19);
ethyl 3-methyl-5-(N-(3-fluorobenzyl)-N-phenethylsulfamoyl)benzofuran-2-carboxylate (M4-20);
ethyl 3-methyl-5-(N-(3-chlorobenzyl)-N-phenethylsulfamoyl)benzofuran-2-carboxylate (M4-21);
ethyl 3-methyl-5-(N-(3-bromobenzyl)-N-phenethylsulfamoyl)benzofuran-2-carboxylate (M4-22);
ethyl 3-methyl-5-(N-(3-methylbenzyl)-N-phenethylsulfamoyl)benzofuran-2-carboxylate (M4-23);
ethyl 3-methyl-5-(N-(3-trifluoromethylbenzyl)-N-phenethylsulfamoyl)benzofuran-2-carboxylate (M4-24);
ethyl 3-methyl-5-(N-(3-nitrobenzyl)-N-phenethylsulfamoyl)benzofuran-2-carboxylate (M4-25);
ethyl 3-methyl-5-(N-([1,1'-biphenyl]-3-ylmethyl)-N-phenethylsulfamoyl)benzofuran-2-carboxylate (M4-26);
ethyl 3-methyl-5-(N-3-phenoxybenzyl)-N-phenethylsulfamoyl)benzofuran-2-carboxylate (M4-27);
ethyl 3-methyl-5-(N-(4-fluorobenzyl)-N-phenethylsulfamoyl)benzofuran-2-carboxylate (M4-28);
ethyl 3-methyl-5-(N-(4-chlorobenzyl)-N-phenethylsulfamoyl)benzofuran-2-carboxylate (M4-29);
ethyl 3-methyl-5-(N-(4-bromobenzyl)-N-phenethylsulfamoyl)benzofuran-2-carboxylate (M4-30);
ethyl 3-methyl-5-(N-(4-methylbenzyl)-N-phenethylsulfamoyl)benzofuran-2-carboxylate (M4-31);
ethyl 3-methyl-5-(N-(4-trifluoromethylbenzyl)-N-phenethylsulfamoyl)benzofuran-2-carboxylate (M4-32);
ethyl 3-methyl-5-(N-(4-cyanobenzyl)-N-phenethylsulfamoyl)benzofuran-2-carboxylate (M4-33);
ethyl 3-methyl-5-(N-(4-(trifluoromethoxy)benzyl)-N-phenethylsulfamoyl)benzofuran-2-carboxylate (M4-34);
ethyl 3-methyl-5-(N-(4-(trifluoromethylthio)benzyl)-N-phenethylsulfamoyl)benzofuran-2-carboxylate (M4-35);
ethyl 3-methyl-5-(N-(4-isopropylbenzyl)-N-phenethylsulfamoyl)benzofuran-2-carboxylate (M4-36);
ethyl 3-methyl-5-(N-(4-(*tert*-butyl)benzyl)-N-phenethylsulfamoyl)benzofuran-2-carboxylate (M4-37);
ethyl 3-methyl-5-(N-(4-acetylbenzyl)-N-phenethylsulfamoyl)benzofuran-2-carboxylate (M4-38);
ethyl 3-methyl-5-(N-(4-(methoxycarbonyl)benzyl)-N-phenethylsulfamoyl)benzofuran-2-carboxylate (M4-39);
ethyl 3-methyl-5-(N-(4-(*tert*-butoxycarbonyl)benzyl)-N-phenethylsulfamoyl)benzofuran-2-carboxylate (M4-40);
ethyl 3-methyl-5-(N-(4-(2-methoxy-2-oxoethyl)benzyl)-N-phenethylsulfamoyl)benzofuran-2-carboxylate (M4-41);
(E)-ethyl 3-methyl-5-(N-(4-(3-methoxy-3-oxoprop-1-en-1-yl)benzyl)-N-phenethylsulfamoyl)benzofuran-2-carboxylate (M4-42);
ethyl 3-methyl-5-(N-(naphthalen-1-ylmethyl)-N-phenethylsulfamoyl)benzofuran-2-carboxylate (M4-43);
ethyl 3-methyl-5-(N-(quinolin-8-ylmethyl)-N-phenethylsulfamoyl)benzofuran-2-carboxylate (M4-44);
ethyl 3-methyl-5-(N-(naphthalen-2-ylmethyl)-N-phenethylsulfamoyl)benzofuran-2-carboxylate (M4-45);
ethyl 3-methyl-5-(N-(2,6-difluorobenzyl)-N-phenethylsulfamoyl)benzofuran-2-carboxylate (M4-46);
ethyl 3-methyl-5-(N-(2-fluoro-6-chlorobenzyl)-N-phenethylsulfamoyl)benzofuran-2-carboxylate (M4-47);
ethyl 3-methyl-5-(N-(2-fluoro-6-(trifluoromethyl)benzyl)-N-phenethylsulfamoyl)benzofuran-2-carboxylate (M4-48);
ethyl 3-methyl-5-(N-(2,6-dichlorobenzyl)-N-phenethylsulfamoyl)benzofuran-2-carboxylate (M4-49);
ethyl 3-methyl-5-(N-(2,6-dimethylbenzyl)-N-phenethylsulfamoyl)benzofuran-2-carboxylate (M4-50);
ethyl 3-methyl-5-(N-(2,4-difluorobenzyl)-N-phenethylsulfamoyl)benzofuran-2-carboxylate (M4-51);
ethyl 3-methyl-5-(N-(2-fluoro-4-chlorobenzyl)-N-phenethylsulfamoyl)benzofuran-2-carboxylate (M4-52);
ethyl 3-methyl-5-(N-(2-fluoro-4-bromobenzyl)-N-phenethylsulfamoyl)benzofuran-2-carboxylate (M4-53);
ethyl 3-methyl-5-(N-(2-fluoro-4-cyanobenzyl)-N-phenethylsulfamoyl)benzofuran-2-carboxylate (M4-54);
ethyl 3-methyl-5-(N-(2-fluoro-4-(trifluoromethyl)benzyl)-N-phenethylsulfamoyl)benzofuran-2-carboxylate (M4-55);
ethyl 3-methyl-5-(N-(2-chloro-4-fluorobenzyl)-N-phenethylsulfamoyl)benzofuran-2-carboxylate (M4-56);
ethyl 3-methyl-5-(N-(2-(trifluoromethyl)-4-fluorobenzyl)-N-phenethylsulfamoyl)benzofuran-2-carboxylate (M4-57);
ethyl 3-methyl-5-(N-(2,4-bis(trifluoromethyl)benzyl)-N-phenethylsulfamoyl)benzofuran-2-carboxylate (M4-58);
ethyl 3-methyl-5-(N-(2-trifluoromethyl-4-bromobenzyl)-N-phenethylsulfamoyl)benzofuran-2-carboxylate (M4-59);
ethyl 3-methyl-5-(N-(2,4-dichlorobenzyl)-N-phenethylsulfamoyl)benzofuran-2-carboxylate (M4-60);
ethyl 3-methyl-5-(N-(2-methyl-5-fluorobenzyl)-N-phenethylsulfamoyl)benzofuran-2-carboxylate (M4-61);
ethyl 3-methyl-5-(N-(2,6-difluoro-4-chlorobenzyl)-N-phenethylsulfamoyl)benzofuran-2-carboxylate (M4-62);
ethyl 3-methyl-5-(N-([1,1'-biphenyl]-4-ylmethyl-N-phenethylsulfamoyl)benzofuran-2-carboxylate (M4-82);
ethyl 3-methyl-5-(N-((2'-cyano-[1,1'-biphenyl]-4-yl)methyl)-N-phenethylsulfamoyl)benzofuran-2-carboxylate (M4-83);
ethyl 3-methyl-5-(N-((2'-(*tert*-butoxycarbonyl)-[1,1'-biphenyl]-4-yl)methyl)-N-phenethylsulfamoyl)benzofuran-2-carboxylate (M4-84);
ethyl 3-methyl-5-(N-(4-benzoylbenzyl)-N-phenethylsulfamoyl)benzofuran-2-carboxylate (M4-85);
3-methyl-5-(N-(2-fluorobenzyl)-N-phenethylsulfamoyl)benzofuran-2-carboxylic acid (F27-S14);
3-methyl-5-(N-(2-chlorobenzyl)-N-phenethylsulfamoyl)benzofuran-2-carboxylic acid (F27-S15);
3-methyl-5-(N-(2-bromobenzyl)-N-phenethylsulfamoyl)benzofuran-2-carboxylic acid (F27-S16);
3-methyl-5-(N-(2-methylbenzyl)-N-phenethylsulfamoyl)benzofuran-2-carboxylic acid (F27-S17);
3-methyl-5-(N-(2-trifluoromethylbenzyl)-N-phenethylsulfamoyl)benzofuran-2-carboxylic acid (F27-S18);
3-methyl-5-(N-(2-nitrobenzyl)-N-phenethylsulfamoyl)benzofuran-2-carboxylic acid (F27-S19);
3-methyl-5-(N-(3-fluorobenzyl)-N-phenethylsulfamoyl)benzofuran-2-carboxylic acid (F27-S20);
3-methyl-5-(N-(3-chlorobenzyl)-N-phenethylsulfamoyl)benzofuran-2-carboxylic acid (F27-S21);
3-methyl-5-(N-(3-bromobenzyl)-N-phenethylsulfamoyl)benzofuran-2-carboxylic acid (F27-S22);
3-methyl-5-(N-(3-methylbenzyl)-N-phenethylsulfamoyl)benzofuran-2-carboxylic acid (F27-S23);
3-methyl-5-(N-(3-trifluoromethylbenzyl)-N-phenethylsulfamoyl)benzofuran-2-carboxylic acid (F27-S24);
3-methyl-5-(N-(3-nitrobenzyl)-N-phenethylsulfamoyl)benzofuran-2-carboxylic acid (F27-S25);
3-methyl-5-(N-([1,1'-biphenyl]-3-ylmethyl)-N-phenethylsulfamoyl)benzofuran-2-carboxylic acid (F27-S26);
3-methyl-5-(N-(3-phenoxybenzyl)-N-phenethylsulfamoyl)benzofuran-2-carboxylic acid (F27-S27);
3-methyl-5-(N-(4-fluorobenzyl)-N-phenethylsulfamoyl)benzofuran-2-carboxylic acid (F27-S28);
3-methyl-5-(N-(4-chlorobenzyl)-N-phenethylsulfamoyl)benzofuran-2-carboxylic acid (F27-S29);
3-methyl-5-(N-(4-bromobenzyl)-N-phenethylsulfamoyl)benzofuran-2-carboxylic acid (F27-S30);
3-methyl-5-(N-(4-methylbenzyl)-N-phenethylsulfamoyl)benzofuran-2-carboxylic acid (F27-S31);
3-methyl-5-(N-(4-(trifluoromethyl)benzyl)-N-phenethylsulfamoyl)benzofuran-2-carboxylic acid (F27-S32);
3-methyl-5-(N-(4-carbamoylbenzyl)-N-phenethylsulfamoyl)benzofuran-2-carboxylic acid (F27-S33);
3-methyl-5-(N-(4-(trifluoromethoxy)benzyl)-N-phenethylsulfamoyl)benzofuran-2-carboxylic acid (F27-S34);
3-methyl-5-(N-(4-(trifluoromethylthio)benzyl)-N-phenethylsulfamoyl)benzofuran-2-carboxylic acid (F27-S35);
3-methyl-5-(N-(4-isopropylbenzyl)-N-phenethylsulfamoyl)benzofuran-2-carboxylic acid (F27-S36);
3-methyl-5-(N-(4-tert-butylbenzyl)-N-phenethylsulfamoyl)benzofuran-2-carboxylic acid (F27-S37);
3-methyl-5-(N-(4-acetylbenzyl)-N-phenethylsulfamoyl)benzofuran-2-carboxylic acid (F27-S38);
3-methyl-5-(N-(4-carboxybenzyl)-N-phenethylsulfamoyl)benzofuran-2-carboxylic acid (F27-S39);
3-methyl-5-(N-(4-(*tert*-butoxycarbonyl)benzyl)-N-phenethylsulfamoyl)benzofuran-2-carboxylic acid (F27-S40);
3-methyl-5-(N-(4-(carboxymethyl)benzyl)-N-phenethylsulfamoyl)benzofuran-2-carboxylic acid (F27-S41);
(E)-3-methyl-5-(N-(4-(2-carboxyvinyl)benzyl)-N-phenethylsulfamoyl)benzofuran-2-carboxylic acid (F27-S42);
3-methyl-5-(N-(naphthalen-2-ylmethyl)-N-phenethylsulfamoyl)benzofuran-2-carboxylic acid (F27-S43);
3-methyl-5-(N-(quinolin-8-ylmethyl)-N-phenethylsulfamoyl)benzofuran-2-carboxylic acid (F27-S44);
3-methyl-5-(N-(naphthalen-2-ylmethyl)-N-phenethylsulfamoyl)benzofuran-2-carboxylic acid (F27-S45);
3-methyl-5-(N-(2,6-difluorobenzyl)-N-phenethylsulfamoyl)benzofuran-2-carboxylic acid (F27-S46);
3-methyl-5-(N-(2-fluoro-6-chlorobenzyl)-N-phenethylsulfamoyl)benzofuran-2-carboxylic acid (F27-S47);
3-methyl-5-(N-(2-fluoro-6-(trifluoromethyl)benzyl)-N-phenethylsulfamoyl)benzofuran-2-carboxylic acid (F27-S48);
3-methyl-5-(N-(2,6-dichlorobenzyl)-N-phenethylsulfamoyl)benzofuran-2-carboxylic acid (F27-S49);
3-methyl-5-(N-(2,6-dimethylbenzyl)-N-phenethylsulfamoyl)benzofuran-2-carboxylic acid (F27-S50);
3-methyl-5-(N-(2,4-difluorobenzyl)-N-phenethylsulfamoyl)benzofuran-2-carboxylic acid (F27-S51);
3-methyl-5-(N-(2-fluoro-4-chlorobenzyl)-N-phenethylsulfamoyl)benzofuran-2-carboxylic acid (F27-S52);
3-methyl-5-(N-(2-fluoro-4-bromobenzyl)-N-phenethylsulfamoyl)benzofuran-2-carboxylic acid (F27-S53);
3-methyl-5-(N-(2-fluoro-4-(trifluoromethyl)benzyl)-N-phenethylsulfamoyl)benzofuran-2-carboxylic acid (F27-S55);
3-methyl-5-(N-(2-chloro-4-fluorobenzyl)-N-phenethylsulfamoyl)benzofuran-2-carboxylic acid (F27-S56);
3-methyl-5-(N-(2-(trifluoromethyl)-4-fluorobenzyl)-N-phenethylsulfamoyl)benzofuran-2-carboxylic acid (F27-S57);
3-methyl-5-(N-(2,4-bis(trifluoromethyl)benzyl)-N-phenethylsulfamoyl)benzofuran-2-carboxylic acid (F27-S58);
3-methyl-5-(N-(2-(trifluoromethyl)-4-bromobenzyl)-N-phenethylsulfamoyl)benzofuran-2-carboxylic acid (F27-S59);
3-methyl-5-(N-(2,4-dichlorobenzyl)-N-phenethylsulfamoyl)benzofuran-2-carboxylic acid (F27-S60);
3-methyl-5-(N-(2-methyl-5-fluorobenzyl)-N-phenethylsulfamoyl)benzofuran-2-carboxylic acid (F27-S61);
3-methyl-5-(N-(2,6-difluoro-4-chlorobenzyl)-N-phenethylsulfamoyl)benzofuran-2-carboxylic acid (F27-S62);
3-methyl-5-(N-([1,1'-biphenyl]-4-ylmethyl)-N-phenethylsulfamoyl)benzofuran-2-carboxylic acid (F27-S82);
3-methyl-5-(N-(2'-cyano-[1,1'-biphenyl]-4-yl)methyl)-N-phenethylsulfamoyl)benzofuran-2-carboxylic acid (F27-S83);
3-methyl-5-(N-((2'-(*tert*-butoxycarbonyl)-[1,1'-biphenyl]-4-yl)methyl)-N-phenethylsulfamoyl)benzofuran-2-carboxylic acid (F27-S84);
3-methyl-5-(N-(4-benzoylbenzyl)-N-phenethylsulfamoyl)benzofuran-2-carboxylic acid (F27-S85);
ethyl 3-methyl-5-(N-phenyl-N-phenethylsulfamoyl)benzofuran-2-carboxylate (M8-10);
ethyl 3-methyl-5-(N-(4-benzylphenyl)-N-phenethylsulfamoyl)benzofuran-2-carboxylate (M8-11);
ethyl 3-methyl-5-(N-(4-phenoxyphenyl)-N-phenethylsulfamoyl)benzofuran-2-carboxylate (M8-12);
ethyl 3-methyl-5-(N,N-diphenylethylsulfamoyl)benzofuran-2-carboxylate (M8-13);
ethyl 3-methyl-5-(N-(4-morpholinophenyl)-N-phenethylsulfamoyl)benzofuran-2-carboxylate (M8-63);
ethyl 3-methyl-5-(N-(4-(4-methylpiperazin-1-yl)phenyl)-N-phenethylsulfamoyl)benzofuran-2-carboxylate (M8-64);
ethyl 3-methyl-5-(N-(4-(4-(*tert*-butoxycarbonyl)piperazin-1-yl)phenyl)-N-phenethylsulfamoyl)benzofuran-2-carboxylate (M8-65);
ethyl 3-methyl-5-(N-(4-(4-(3,3-dimethylbutyryl)piperazin-1-yl)phenyl)-N-phenethylsulfamoyl)benzofuran-2-carboxylate (M8-66);
ethyl 3-methyl-5-(N-(4-(4-(propoxycarbonyl)piperazin-1-yl)phenyl)-N-phenethylsulfamoyl)benzofuran-2-carboxylate (M8-67);
ethyl 3-methyl-5-(N-(4-(4-acetylpiperazin-1-yl)phenyl)-N-phenethylsulfamoyl)benzofuran-2-carboxylate (M8-68);
ethyl 3-methyl-5-(N-(4-(4-benzoylpiperazin-1-yl)phenyl)-N-phenethylsulfamoyl)-3-methylbenzofuran-2-carboxylate (M8-69);
ethyl 3-methyl-5-(N-(4-(4-benzylpiperazin-1-yl)phenyl)-N-phenethylsulfamoyl)benzofuran-2-carboxylate (M8-70);
ethyl 3-methyl-5-(N-(4-(4-(dimethylcarbamoyl)piperazin-1-yl)phenyl)-N-phenethylsulfamoyl)benzofuran-2-carboxylate (M8-71);
ethyl 3-methyl-5-(N-(4-(4-(methylsulfonyl)piperazin-1-yl)phenyl)-N-phenethylsulfamoyl)benzofuran-2-carboxylate (M8-72);
ethyl 3-methyl-5-(N-(4-(1-(*tert*-butoxycarbonyl)piperidin-4-yl)phenyl)-N-phenethylsulfamoyl)benzofuran-2-carboxylate (M8-73);
ethyl 3-methyl-5-(N-(4-(4-(*tert*-butoxycarbonyl)piperazin-1-yl)-3-(trifluoromethyl)phenyl)-N-phenethylsulfamoyl)benzofuran-2-carboxylate (M8-74);
ethyl 3-methyl-5-(N-(6-(4-(*tert*-butoxycarbonyl)piperazin-1-yl) pyridin-3-yl)-N-phenethylsulfamoyl)benzofuran-2-carboxylate (M8-75);
ethyl 3-methyl-5-(N-(4-((4-(*tert*-butoxycarbonyl)piperazin-1-yl)methyl)phenyl)-N-phenethylsulfamoyl) benzofuran-2-carboxylate (M8-76);
ethyl 3-methyl-5-(N-(2-(4-(*tert*-butoxycarbonyl)piperazin-1-yl)phenyl)-N-phenethylsulfamoyl)benzofuran-2-carboxylate (M8-77);
ethyl 3-methyl-5-(N-(3-(4-methylpiperazin-1-yl)phenyl)-N-phenethylsulfamoyl)benzofuran-2-carboxylate (M8-78);
ethyl 3-methyl-5-(N-(4-morpholinobenzyl)-N-phenethylsulfamoyl)benzofuran-2-carboxylate (M8-79);
ethyl 3-methyl-5-(N-(4-(4-methylpiperazin-1-yl)benzyl)-N-phenethylsulfamoyl)benzofuran-2-carboxylate (M8-80);
ethyl 3-methyl-5-(N-(4-(4-(*tert*-butoxycarbonyl)piperazin-1-yl)benzyl)-N-phenethylsulfamoyl)benzofuran-2-carboxylate (M8-81);
ethyl 3-methyl-5-(N-phenethyl-N-(4-phenoxybenzyl)sulfamoyl)benzofuran-2-carboxylate (M8-86);
ethyl 3-methyl-5-(N-(2-(4-(*tert*-butoxycarbonyl)piperazin-1-yl)benzyl)-N-phenethylsulfamoyl)benzofuran-2-carboxylate (M8-87);
ethyl 3-methyl-5-(N-(3-(4-(*tert*-butoxycarbonyl)piperazin-1-yl)benzyl)-N-phenethylsulfamoyl)benzofuran-2-carboxylate (M8-88);
3-methyl-5-(N-phenethyl-N-phenylsulfamoyl)benzofuran-2-carboxylic acid (F27-S10);
3-methyl-5-(N-(4-benzylphenyl)-N-phenethylsulfamoyl)benzofuran-2-carboxylic acid (F27-S11);
3-methyl-5-(N-(4-phenoxyphenyl)-N-phenethylsulfamoyl)benzofuran-2-carboxylic acid (F27-S12);
3-methyl-5-(N,N-diphenylethylsulfamoyl)benzofuran-2-carboxylic acid (F27-S13);
3-methyl-5-(N-(4-morpholinophenyl)-N-phenethylsulfamoyl)benzofuran-2-carboxylic acid (F27-S63);
3-methyl-5-(N-(4-(4-methylpiperazin-1-yl)phenyl)-N-phenethylsulfamoyl)benzofuran-2-carboxylic acid (F27-S64);
3-methyl-5-(N-(4-(4-(*tert*-butoxycarbonyl)piperazin-1-yl)phenyl)-N-phenethylsulfamoyl)benzofuran-2-carboxylic acid (F27-S65);
3-methyl-5-(N-(4-(4-(3,3-dimethylbutyryl)piperazin-1-yl)phenyl)-N-phenethylsulfamoyl)benzofuran-2-carboxylic acid (F27-S66);
3-methyl-5-(N-(4-(4-(propoxycarbonyl)piperazin-1-yl)phenyl)-N-phenethylsulfamoyl)benzofuran-2-carboxylic acid (F27-S67);
3-methyl-5-(N-(4-(4-acetylpiperazin-1-yl)phenyl)-N-phenethylsulfamoyl)benzofuran-2-carboxylic acid (F27-S68);
3-methyl-5-(N-(4-(4-benzoylpiperazin-1-yl)phenyl)-N-phenethylsulfamoyl)benzofuran-2-carboxylic acid (F27-S69);
3-methyl-5-(N-(4-(4-benzylpiperazin-1-yl)phenyl)-N-phenethylsulfamoyl)benzofuran-2-carboxylic acid (F27-S70);
3-methyl-5-(N-(4-(4-(dimethylcarbamoyl)piperazin-1-yl)phenyl)-N-phenethylsulfamoyl)benzofuran-2-carboxylic acid (F27-S71);
3-methyl-5-(N-(4-(4-(methylsulfonyl)piperazin-1-yl)phenyl)-N-phenethylsulfamoyl)benzofuran-2-carboxylic acid (F27-S72);
3-methyl-5-(N-(4-(1-(*tert*-butoxycarbonyl)piperidin-4-yl)phenyl)-N-phenethylsulfamoyl)benzofuran-2-carboxylic acid (F27-S73);
3-methyl-5-(N-(4-(4-(*tert*-butoxycarbonyl)piperazin-1-yl)-3-(trifluoromethyl)phenyl)-N-phenethylsulfamoyl)benzofuran-2-carboxylic acid (F27-S74);
3-methyl-5-(N-(6-(4-(*tert*-butoxycarbonyl)piperazin-1-yl)pyridin-3-yl)-N-phenethylsulfamoyl)benzofuran-2-carboxylic acid (F27-S75);
3-methyl-5-(N-(4-((4-(*tert*-butoxycarbonyl)piperazin-1-yl)methyl)phenyl)-N-phenethylsulfonamide)benzofuran-2-carboxylic acid (F27-S76);
3-methyl-5-(N-(2-(4-(*tert*-butoxycarbonyl)piperazin-1-yl)phenyl)-N-phenethylsulfamoyl)benzofuran-2-carboxylic acid (F27-S77);
3-methyl-5-(N-(3-(4-methylpiperazin-1-yl)phenyl)-N-phenethylsulfamoyl)benzofuran-2-carboxylic acid (F27-S78);
3-methyl-5-(N-(4-(4-morpholinobenzyl)-N-phenethylsulfamoyl)benzofuran-2-carboxylic acid (F27-S79);
3-methyl-5-(N-(4-(4-methylpiperazin-1-yl)benzyl)-N-phenethylsulfamoyl)benzofuran-2-carboxylic acid (F27-S80);
3-methyl-5-(N-(4-(4-(*tert*-butoxycarbonyl)piperazin-1-yl)benzyl)-N-phenethylsulfamoyl)benzofuran-2-carboxylic acid (F27-S81);
3-methyl-5-(N-(4-(4-phenoxybenzyl)-N-phenethylsulfamoyl)benzofuran-2-carboxylic acid (F27-S86);
3-methyl-5-(N-(2-(4-(*tert*-butoxycarbonyl)piperazin-1-yl)benzyl)-N-phenethylsulfamoyl)benzofuran-2-carboxylic acid (F27-S87);
3-methyl-5-(N-(3-(4-(*tert*-butoxycarbonyl)piperazin-1-yl)benzyl)-N-phenethylsulfamoyl)benzofuran-2-carboxylic acid (F27-S88);
ethyl 3-methyl-5-(N-phenethyl-N-(2-(piperazin-1-yl)phenyl)sulfamoyl)benzofuran-2-carboxylate (M9-1);
ethyl 3-methyl-5-(N-phenethyl-N-(2-(piperazin-1-yl)benzyl)sulfamoyl)benzofuran-2-carboxylate (M9-2);
4-(2-((2-(ethoxycarbonyl)-3-methyl-N-phenethylbenzofuran)-5-sulfonylamino)phenyl)-1,1-dimethylpiperazin-1-ium(M10-90);
ethyl 3-methyl-5-(N-(2-(4-acetylpiperazin-1-yl)phenyl)-N-phenethylsulfamoyl)benzofuran-2-carboxylate (M10-91);
ethyl 3-methyl-5-(N-(2-(4-acetylpiperazin-1-yl)phenyl)-N-phenethylsulfamoyl)benzofuran-2-carboxylate (M10-92);
ethyl 3-methyl-5-(N-(2-(4-(methylsulfonyl)piperazin-1-yl)phenyl)-N-phenethylsulfamoyl)benzofuran-2-carboxylate (M10-93);
ethyl 3-methyl-5-(N-(2-(4-(4-(3,3-dimethylbutyryl)piperazin-1-yl)phenyl)-N-phenethylsulfamoyl)benzofuran-2-carboxylate (M10-94);
ethyl 3-methyl-5-(N-(2-(4-(4-(benzylpiperazin-1-yl)phenyl)-N-phenethylsulfamoyl)benzofuran-2-carboxylate (M10-95);
ethyl 3-methyl-5-(N-(2-(4-(benzoylpiperazin-1-yl)phenyl)-N-phenethylsulfamoyl)benzofuran-2-carboxylate (M10-96);
ethyl 3-methyl-5-(N-(2-(4-methylpiperazin-1-yl)benzyl)-N-phenethylsulfamoyl)benzofuran-2-carboxylate (M10-98);
4-(2-((2-(ethoxycarbonyl)-3-methyl-N-benzylethylbenzofuran)-5-sulfonylamino)phenyl)-1,1-dimethylpiperazin-1-ium(M10-99);
ethyl 3-methyl-5-(N-(2-(4-acetylpiperazin-1-yl)benzyl)-N-phenethylsulfamoyl)benzofuran-2-carboxylate (M10-100);
ethyl 3-methyl-5-(N-(2-(4-acetylpiperazin-1-yl)benzyl)-N-phenethylsulfamoyl)benzofuran-2-carboxylate (M10-101);
ethyl 3-methyl-5-(N-(2-(4-methanesulfonylpiperazin-1-yl)benzyl)-N-phenethylsulfamoyl)benzofuran-2-carboxylate (M10-102);
ethyl 3-methyl-5-(N-(2-(4-(3,3-dimethylbutyryl)piperazin-1-yl)benzyl)-N-phenethylsulfamoyl)benzofuran-2-carboxylate (M10-103);
ethyl 3-methyl-5-(N-(2-(4-benzylpiperazin-1-yl)benzyl)-N-phenethylsulfamoyl)benzofuran-2-carboxylate (M10-104);
ethyl 3-methyl-5-(N-(2-(4-benzoylpiperazin-1-yl)benzyl)-N-phenethylsulfamoyl)benzofuran-2-carboxylate (M10-105);
3-methyl-5-(N-phenethyl-N-(2-(piperazin-1-yl)phenyl)sulfamoyl)benzofuran-2-carboxylicacid (F27-S89);
4-(2-((2-carboxy-3-methyl-N-phenethylbenzofuran)-5-sulfonylamino)phenyl)-1,1-dimethylpiperazin-1-ium(F27-S90);
3-methyl-5-(N-(2-(4-acetylpiperazin-1-yl)phenyl)-N-phenethylsulfamoyl)benzofuran-2-carboxylic acid (F27-S91);
3-methyl-5-(N-(2-(4-(dimethylcarbamoyl)piperazin-1-yl)phenyl)-N-phenethylsulfamoyl)benzofuran-2-carboxylic acid (F27-S92);
3-methyl-5-(N-(2-(4-(methylsulfonyl)piperazin-1-yl)phenyl)-N-phenethylsulfamoyl)benzofuran-2-carboxylic acid (F27-S93);
3-methyl-5-(N-(2-(4-(4-(3,3-dimethylbutyryl)piperazin-1-yl)phenyl)-N-phenethylsulfamoyl)benzofuran-2-carboxylic acid (F27-S94);
3-methyl-5-(N-(2-(4-(4-(benzylpiperazin-1-yl)phenyl)-N-phenethylsulfamoyl)benzofuran-2-carboxylic acid (F27-S95);
3-methyl-5-(N-(2-(4-(benzoylpiperazin-1-yl)phenyl)-N-phenethylsulfamoyl)benzofuran-2-carboxylic acid (F27-S96);
3-methyl-5-(N-(2-(piperazin-1-yl)benzyl)-N-phenethylsulfamoyl)benzofuran-2-carboxylic acid (F27-S97);
3-methyl-5-(N-(2-(4-acetylpiperazin-1-yl)benzyl)-N-phenethylsulfamoyl)benzofuran-2-carboxylic acid (F27-S98);
4-(2-((2-carboxy-3-methyl-N-phenethylbenzofuran)-5-sulfonamido)methyl)phenyl)-1,1-dimethylpiperazin-1-ium(F27-S99);
3-methyl-5-(N-(2-(4-acetylpiperazin-1-yl)benzyl)-N-phenethylsulfamoyl)benzofuran-2-carboxylic acid (F27-S100);
3-methyl-5-(N-(2-(4-(dimethylcarbamoyl)piperazin-1-yl)benzyl)-N-phenethylsulfamoyl)benzofuran-2-carboxylic acid (F27-S101);
3-methyl-5-(N-(2-(4-(methylsulfonyl)piperazin-1-yl)benzyl)-N-phenethylsulfamoyl)benzofuran-2-carboxylic acid (F27-S102);
3-methyl-5-(N-(2-(4-(4-(3,3-dimethylbutyryl)piperazin-1-yl)benzyl)-N-phenethylsulfamoyl)benzofuran-2-carboxylic acid (F27-S103);
3-methyl-5-(N-(2-(4-benzylpiperazin-1-yl)benzyl)-N-phenethylsulfamoyl)benzofuran-2-carboxylic acid (F27-S104) and
3-methyl-5-(N-(2-(4-benzoylpiperazin-1-yl)benzyl)-N-phenethylsulfamoyl)benzofuran-2-carboxylic acid (F27-S105); or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

In a second aspect, this application provides use of a benzofuran derivative of formula (II) as an FXR modulator: 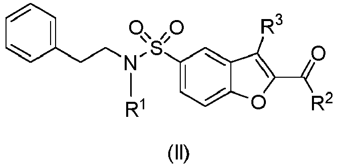
wherein R¹ is -(CH₂)ₙ-R⁴, wherein n is an integer from 0 to 6, R⁴ is an unsubstituted aromatic hydrocarbyl, a substituted aromatic hydrocarbyl, an unsubstituted heteroaryl, or a substituted heteroaryl; the substituted aromatic hydrocarbyl or the substituted heteroaryl means that the aromatic hydrocarbyl or heteroaryl is substituted with one or more groups selected from Group A;
In formula (II), R² is a hydroxyl, an unsubstituted C1-C6 alkoxy, a substituted C1-C6 alkoxy, or -NR⁵R⁶, wherein R⁵ and R⁶ here are each independently selected from a group consisting of hydrogen, unsubstituted C1-C6 alkyl, and substituted C1-C6 alkyl; the substituted C1-C6 alkoxy means that one or more hydrogens on the C1-C6 alkoxy are substituted by groups selected from a group consisting of phenyl, halogen, nitro, hydroxyl and C1-C6 alkyl; the substituted C1-C6 alkyl means that one or more hydrogens on the C1-C6 alkyl are substituted by groups selected from a group consisting of phenyl, halogen, hydroxyl and C1-C6 alkyl;
R³ is an unsubstituted C1-C6 alkyl or a substituted C1-C6 alkyl;
the group in Group A refers to one or more of the following groups: halogen, nitro, cyano, substituted or unsubstituted aromatic hydrocarbyl, substituted or unsubstituted aryloxy, unsubstituted C1-C6 alkyl, unsubstituted C2-C6 alkenyl, carboxyl-substituted C2-C6 alkenyl, unsubstituted C2-C6 alkynyl, carboxyl-substituted C2-C6 alkynyl, carbamoyl, halogenated C1-C6 alkyl, aromatic hydrocarbyl-substituted C1-C6 alkyl, carboxyl-substituted C1-C6 alkyl, C1-C6 alkoxycarbonyl, unsubstituted C1-C6 alkoxy, halogenated C1-C6 alkoxy, unsubstituted C1-C6 alkylthio group, halogenated C1-C6 alkylthio group, C1-C6 alkylamide group, and substituted or unsubstituted heterocycloalkyl, wherein the substituted aromatic hydrocarbyl, substituted aryloxy or substituted heterocycloalkyl means that an aromatic hydrocarbyl, aryloxy or heterocycloalkyl is substituted with one or more groups selected from Group B, or that a C1-C6 alkyl and N in a heterocycloalkyl form a quaternary ammonium;
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C6 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, and C1-C6 alkoxycarbonyl;
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

In the embodiments of this application, the use of the benzofuran derivative as an FXR modulator, preferably as an FXR antagonist or inhibitor, includes but is not limited to lipid-lowering activity, anti-hyperlipidemic activity, anti-dyslipidemic activity, anti-atherosclerotic activity, anti-obesity activity, and anti-diabetic activity.

In some embodiments of this application, R³ in formula (II) is methyl, ethyl, n-propyl, isopropyl, n-butyl, isobutyl, sec-butyl, *tert*-butyl, n-pentyl, or neopentyl.

In some embodiments of this application, R³ in formula (II) is methyl.

In some embodiments of this application, R² in formula (II) is hydroxyl.

In some embodiments of this application, R² in formula (II) is methoxy, ethoxy, n-propoxy, isopropoxy, n-butoxy, isobutoxy, sec-butoxy, *tert*-butoxy or benzyl; preferably, R² is ethoxy.

In some embodiments of this application, R² in formula (II) is -NR⁵R⁶, wherein R⁵ and R⁶ are each independently selected from a group consisting of hydrogen, methyl, ethyl, n-propyl, isopropyl, n-butyl, isobutyl, sec-butyl, *tert*-butyl, n-pentyl, neopentyl, benzyl, and phenethyl; preferably, R² is phenylethylamino.

In some embodiments of this application, in formula (II), n is 0; R⁴ is phenyl, naphthyl, pyridyl, or quinoline, or, phenyl, naphthyl, pyridyl or quinoline substituted with one or more of the following groups: benzyl, phenoxy, halogen, nitro, cyano, carboxyl, unsubstituted C1-C6 alkyl, halogenated C1-C6 alkyl, halogenated C1-C6 alkoxy, carbamoyl, halogenated C1-C6 alkylthio, C1-C6 alkanoyl, morpholinyl, piperazinyl, piperazinmethyl, and piperidinyl, wherein the piperazinyl, piperazinylmethyl and piperidinyl are optionally substituted with one or more of the following groups: unsubstituted C1-C6 alkyl, phenyl-substituted C1-C6 alkyl, unsubstituted C1-C6 alkoxycarbonyl, unsubstituted C1-C6 alkanoyl, phenyl-substituted C1-C6 alkanoyl, C1-C6 alkane sulfonyl, single- or double- C1-C6 substituted carbamoyl; or C1-C6 alkyl and N in morpholinyl, piperazinyl, piperazinmethyl, and piperidinyl form a quaternary ammonium.

In some embodiments of this application, in formula (II), n is 1 or 2; R⁴ is phenyl, naphthyl, pyridyl, or quinoline, or, phenyl, naphthyl, pyridyl, or quinoline substituted with one or more of the following groups: benzyl, phenoxy, halogen, nitro, cyano, carboxyl, unsubstituted C1-C6, halogenated C1-C4 alkyl, halogenated C1-C4 alkoxy, C1-C6 alkanoyl, carboxyl-substituted C1-C6 alkyl, C1-C6 alkoxycarbonyl, carbamoyl, halogenated C1-C4 alkylthio, unsubstituted C2-C6 alkenyl, carboxyl-substituted C2-C6 alkenyl, carbamoyl, morpholinyl, piperazinyl, piperazinylmethyl and piperidinyl, wherein the piperazinyl, piperazinylmethyl and piperidinyl are optionally substituted with one or more of the following groups: C1-C6 alkyl, phenyl-substituted C1-C6 alkyl, C1-C6 alkoxycarbonyl, C1-C6 alkanoyl, phenyl-substituted C1-C6 alkanoyl, C1-C4 alkane sulfonyl, single- or double- C1-C4 substituted carbamoyl.

In some embodiments, n in formula (II) is 0. In some embodiments, n in formula (II) is 1. In some embodiments, n in formula (II) is 2.

In some embodiments, R⁴ in formula (II) is phenyl, naphthyl, pyridyl, or quinolyl.

In some embodiments, R⁴ in formula (II) is phenyl substituted with one or more groups selected from Group A. In some embodiments, R⁴ in formula (II) is naphthyl substituted with one or more groups selected from Group A. In some embodiments, R⁴ in formula (II) is pyridinyl substituted with one or more groups selected from Group A. In some embodiments, R⁴ in formula (II) is quinolinyl substituted with one or more groups selected from Group A.

In some embodiments, the group in Group A is selected from: benzyl, phenoxy, halogen, nitro, cyano, carboxyl, unsubstituted C1-C6 alkyl, halogenated C1-C6 alkyl, halogenated C1-C6 alkoxy, carbamoyl, halogenated C1-C6 alkylthio, C1-C6 alkanoyl, C1-C6 alkoxycarbonyl, C1-C6 alkoxycarbonyl-C1-C6 alkylene, carboxyl-C1-C6 alkylene, - C1-C6 alkoxycarbonyl-C2-C6 alkenylene, carboxyl-C2-C6 alkenylene, morpholinyl, piperazinyl, piperazinemethyl, piperidinyl, phenyl, and phenylcarbonyl;

In some embodiments, the group in Group B is selected from: cyano, unsubstituted C1-C6 alkyl, phenyl-substituted C1-C6 alkyl, unsubstituted C1-C6 alkoxycarbonyl, unsubstituted C1 -C6 alkanoyl, phenyl-substituted C1-C6 alkanoyl, C1-C6 alkane sulfonyl, single- or double- C1-C6 substituted carbamoyl; optionally, the phenyl is substituted with C1-C6 alkyl, cyano, halogen, halogenated C1-C6 alkyl, amino, nitro, acetamido, or C1-C6 alkanoyl. In some examples, the benzofuran derivative of formula (II) is selected from the following compounds:
ethyl 3-methyl-5-(N-benzyl-N-phenethylsulfamoyl)benzofuran-2-carboxylate (F27-S8);
ethyl 3-methyl-5-(N-(2-fluorobenzyl)-N-phenethylsulfamoyl)benzofuran-2-carboxylate (M4-14);
ethyl 3-methyl-5-(N-(2-chlorobenzyl)-N-phenethylsulfamoyl)benzofuran-2-carboxylate (M4-15);
ethyl 3-methyl-5-(N-(2-bromobenzyl)-N-phenethylsulfamoyl)benzofuran-2-carboxylate (M4-16);
ethyl 3-methyl-5-(N-(2-methylbenzyl)-N-phenethylsulfamoyl)benzofuran-2-carboxylate (M4-17);
ethyl 3-methyl-5-(N-(2-trifluoromethylbenzyl)-N-phenethylsulfamoyl)benzofuran-2-carboxylate (M4-18);
ethyl 3-methyl-5-(N-(2-nitrobenzyl)-N-phenethylsulfamoyl)benzofuran-2-carboxylate (M4-19);
ethyl 3-methyl-5-(N-(3-fluorobenzyl)-N-phenethylsulfamoyl)benzofuran-2-carboxylate (M4-20);
ethyl 3-methyl-5-(N-(3-chlorobenzyl)-N-phenethylsulfamoyl)benzofuran-2-carboxylate (M4-21);
ethyl 3-methyl-5-(N-(3-bromobenzyl)-N-phenethylsulfamoyl)benzofuran-2-carboxylate (M4-22);
ethyl 3-methyl-5-(N-(3-methylbenzyl)-N-phenethylsulfamoyl)benzofuran-2-carboxylate (M4-23);
ethyl 3-methyl-5-(N-(3-trifluoromethylbenzyl)-N-phenethylsulfamoyl)benzofuran-2-carboxylate (M4-24);
ethyl 3-methyl-5-(N-(3-nitrobenzyl)-N-phenethylsulfamoyl)benzofuran-2-carboxylate (M4-25);
ethyl 3-methyl-5-(N-([1,1'-biphenyl]-3-ylmethyl)-N-phenethylsulfamoyl)benzofuran-2-carboxylate (M4-26);
ethyl 3-methyl-5-(N-3-phenoxybenzyl)-N-phenethylsulfamoyl)benzofuran-2-carboxylate (M4-27);
ethyl 3-methyl-5-(N-(4-fluorobenzyl)-N-phenethylsulfamoyl)benzofuran-2-carboxylate (M4-28);
ethyl 3-methyl-5-(N-(4-chlorobenzyl)-N-phenethylsulfamoyl)benzofuran-2-carboxylate (M4-29);
ethyl 3-methyl-5-(N-(4-bromobenzyl)-N-phenethylsulfamoyl)benzofuran-2-carboxylate (M4-30);
ethyl 3-methyl-5-(N-(4-methylbenzyl)-N-phenethylsulfamoyl)benzofuran-2-carboxylate (M4-31);
ethyl 3-methyl-5-(N-(4-trifluoromethylbenzyl)-N-phenethylsulfamoyl)benzofuran-2-carboxylate (M4-32);
ethyl 3-methyl-5-(N-(4-cyanobenzyl)-N-phenethylsulfamoyl)benzofuran-2-carboxylate (M4-33);
ethyl 3-methyl-5-(N-(4-(trifluoromethoxy)benzyl)-N-phenethylsulfamoyl)benzofuran-2-carboxylate (M4-34);
ethyl 3-methyl-5-(N-(4-(trifluoromethylthio)benzyl)-N-phenethylsulfamoyl)benzofuran-2-carboxylate (M4-35);
ethyl 3-methyl-5-(N-(4-isopropylbenzyl)-N-phenethylsulfamoyl)benzofuran-2-carboxylate (M4-36);
ethyl 3-methyl-5-(N-(4-(*tert*-butyl)benzyl)-N-phenethylsulfamoyl)benzofuran-2-carboxylate (M4-37);
ethyl 3-methyl-5-(N-(4-acetylbenzyl)-N-phenethylsulfamoyl)benzofuran-2-carboxylate (M4-38);
ethyl 3-methyl-5-(N-(4-(methoxycarbonyl)benzyl)-N-phenethylsulfamoyl)benzofuran-2-carboxylate (M4-39);
ethyl 3-methyl-5-(N-(4-(*tert*-butoxycarbonyl)benzyl)-N-phenethylsulfamoyl)benzofuran-2-carboxylate (M4-40);
ethyl 3-methyl-5-(N-(4-(2-methoxy-2-oxoethyl)benzyl)-N-phenethylsulfamoyl)benzofuran-2-carboxylate (M4-41);
(E)-ethyl 3-methyl-5-(N-(4-(3-methoxy-3-oxoprop-1-en-1-yl)benzyl)-N-phenethylsulfamoyl)benzofuran-2-carboxylate (M4-42);
ethyl 3-methyl-5-(N-(naphthalen-1-ylmethyl)-N-phenethylsulfamoyl)benzofuran-2-carboxylate (M4-43);
ethyl 3-methyl-5-(N-(quinolin-8-ylmethyl)-N-phenethylsulfamoyl)benzofuran-2-carboxylate (M4-44);
ethyl 3-methyl-5-(N-(naphthalen-2-ylmethyl)-N-phenethylsulfamoyl)benzofuran-2-carboxylate (M4-45);
ethyl 3-methyl-5-(N-(2,6-difluorobenzyl)-N-phenethylsulfamoyl)benzofuran-2-carboxylate (M4-46);
ethyl 3-methyl-5-(N-(2-fluoro-6-chlorobenzyl)-N-phenethylsulfamoyl)benzofuran-2-carboxylate (M4-47);
ethyl 3-methyl-5-(N-(2-fluoro-6-(trifluoromethyl)benzyl)-N-phenethylsulfamoyl)benzofuran-2-carboxylate (M4-48);
ethyl 3-methyl-5-(N-(2,6-dichlorobenzyl)-N-phenethylsulfamoyl)benzofuran-2-carboxylate (M4-49);
ethyl 3-methyl-5-(N-(2,6-dimethylbenzyl)-N-phenethylsulfamoyl)benzofuran-2-carboxylate (M4-50);
ethyl 3-methyl-5-(N-(2,4-difluorobenzyl)-N-phenethylsulfamoyl)benzofuran-2-carboxylate (M4-51);
ethyl 3-methyl-5-(N-(2-fluoro-4-chlorobenzyl)-N-phenethylsulfamoyl)benzofuran-2-carboxylate (M4-52);
ethyl 3-methyl-5-(N-(2-fluoro-4-bromobenzyl)-N-phenethylsulfamoyl)benzofuran-2-carboxylate (M4-53);
ethyl 3-methyl-5-(N-(2-fluoro-4-cyanobenzyl)-N-phenethylsulfamoyl)benzofuran-2-carboxylate (M4-54);
ethyl 3-methyl-5-(N-(2-fluoro-4-(trifluoromethyl)benzyl)-N-phenethylsulfamoyl)benzofuran-2-carboxylate (M4-55);
ethyl 3-methyl-5-(N-(2-chloro-4-fluorobenzyl)-N-phenethylsulfamoyl)benzofuran-2-carboxylate (M4-56);
ethyl 3-methyl-5-(N-(2-(trifluoromethyl)-4-fluorobenzyl)-N-phenethylsulfamoyl)benzofuran-2-carboxylate (M4-57);
ethyl 3-methyl-5-(N-(2,4-bis(trifluoromethyl)benzyl)-N-phenethylsulfamoyl)benzofuran-2-carboxylate (M4-58);
ethyl 3-methyl-5-(N-(2-trifluoromethyl-4-bromobenzyl)-N-phenethylsulfamoyl)benzofuran-2-carboxylate (M4-59);
ethyl 3-methyl-5-(N-(2,4-dichlorobenzyl)-N-phenethylsulfamoyl)benzofuran-2-carboxylate (M4-60);
ethyl 3-methyl-5-(N-(2-methyl-5-fluorobenzyl)-N-phenethylsulfamoyl)benzofuran-2-carboxylate (M4-61);
ethyl 3-methyl-5-(N-(2,6-difluoro-4-chlorobenzyl)-N-phenethylsulfamoyl)benzofuran-2-carboxylate (M4-62);
ethyl 3-methyl-5-(N-([1,1'-biphenyl]-4-ylmethyl-N-phenethylsulfamoyl)benzofuran-2-carboxylate (M4-82);
ethyl 3-methyl-5-(N-((2'-cyano-[1,1'-biphenyl]-4-yl)methyl)-N-phenethylsulfamoyl)benzofuran-2-carboxylate (M4-83);
ethyl 3-methyl-5-(N-((2'-(*tert*-butoxycarbonyl)-[1,1'-biphenyl]-4-yl)methyl)-N-phenethylsulfamoyl)benzofuran-2-carboxylate (M4-84);
ethyl 3-methyl-5-(N-(4-benzoylbenzyl)-N-phenethylsulfamoyl)benzofuran-2-carboxylate (M4-85);
3-methyl-5-(*N*-benzyl-*N*-phenethylsulfamoyl)benzofuran-2-carboxylic acid (F27);
3-methyl-5-(N-(2-fluorobenzyl)-N-phenethylsulfamoyl)benzofuran-2-carboxylic acid (F27-S14);
3-methyl-5-(N-(2-chlorobenzyl)-N-phenethylsulfamoyl)benzofuran-2-carboxylic acid (F27-S15);
3-methyl-5-(N-(2-bromobenzyl)-N-phenethylsulfamoyl)benzofuran-2-carboxylic acid (F27-S16);
3-methyl-5-(N-(2-methylbenzyl)-N-phenethylsulfamoyl)benzofuran-2-carboxylic acid (F27-S17);
3-methyl-5-(N-(2-trifluoromethylbenzyl)-N-phenethylsulfamoyl)benzofuran-2-carboxylic acid (F27-S18);
3-methyl-5-(N-(2-nitrobenzyl)-N-phenethylsulfamoyl)benzofuran-2-carboxylic acid (F27-S19);
3-methyl-5-(N-(3-fluorobenzyl)-N-phenethylsulfamoyl)benzofuran-2-carboxylic acid (F27-S20);
3-methyl-5-(N-(3-chlorobenzyl)-N-phenethylsulfamoyl)benzofuran-2-carboxylic acid (F27-S21);
3-methyl-5-(N-(3-bromobenzyl)-N-phenethylsulfamoyl)benzofuran-2-carboxylic acid (F27-S22);
3-methyl-5-(N-(3-methylbenzyl)-N-phenethylsulfamoyl)benzofuran-2-carboxylic acid (F27-S23);
3-methyl-5-(N-(3-trifluoromethylbenzyl)-N-phenethylsulfamoyl)benzofuran-2-carboxylic acid (F27-S24);
3-methyl-5-(N-(3-nitrobenzyl)-N-phenethylsulfamoyl)benzofuran-2-carboxylic acid (F27-S25);
3-methyl-5-(N-([1,1'-biphenyl]-3-ylmethyl)-N-phenethylsulfamoyl)benzofuran-2-carboxylic acid (F27-S26);
3-methyl-5-(N-(3-phenoxybenzyl)-N-phenethylsulfamoyl)benzofuran-2-carboxylic acid (F27-S27);
3-methyl-5-(N-(4-fluorobenzyl)-N-phenethylsulfamoyl)benzofuran-2-carboxylic acid (F27-S28);
3-methyl-5-(N-(4-chlorobenzyl)-N-phenethylsulfamoyl)benzofuran-2-carboxylic acid (F27-S29);
3-methyl-5-(N-(4-bromobenzyl)-N-phenethylsulfamoyl)benzofuran-2-carboxylic acid (F27-S30);
3-methyl-5-(N-(4-methylbenzyl)-N-phenethylsulfamoyl)benzofuran-2-carboxylic acid (F27-S31);
3-methyl-5-(N-(4-(trifluoromethyl)benzyl)-N-phenethylsulfamoyl)benzofuran-2-carboxylic acid (F27-S32);
3-methyl-5-(N-(4-carbamoylbenzyl)-N-phenethylsulfamoyl)benzofuran-2-carboxylic acid (F27-S33);
3-methyl-5-(N-(4-(trifluoromethoxy)benzyl)-N-phenethylsulfamoyl)benzofuran-2-carboxylic acid (F27-S34);
3-methyl-5-(N-(4-(trifluoromethylthio)benzyl)-N-phenethylsulfamoyl)benzofuran-2-carboxylic acid (F27-S35);
3-methyl-5-(N-(4-isopropylbenzyl)-N-phenethylsulfamoyl)benzofuran-2-carboxylic acid (F27-S36);
3-methyl-5-(N-(4-*tert*-butylbenzyl)-N-phenethylsulfamoyl)benzofuran-2-carboxylic acid (F27-S37);
3-methyl-5-(N-(4-acetylbenzyl)-N-phenethylsulfamoyl)benzofuran-2-carboxylic acid (F27-S38);
3-methyl-5-(N-(4-carboxybenzyl)-N-phenethylsulfamoyl)benzofuran-2-carboxylic acid (F27-S39);
3-methyl-5-(N-(4-(*tert*-butoxycarbonyl)benzyl)-N-phenethylsulfamoyl)benzofuran-2-carboxylic acid (F27-S40);
3-methyl-5-(N-(4-(carboxymethyl)benzyl)-N-phenethylsulfamoyl)benzofuran-2-carboxylic acid (F27-S41);
(E)-3-methyl-5-(N-(4-(2-carboxyvinyl)benzyl)-N-phenethylsulfamoyl)benzofuran-2-carboxylic acid (F27-S42);
3-methyl-5-(N-(naphthalen-2-ylmethyl)-N-phenethylsulfamoyl)benzofuran-2-carboxylic acid (F27-S43);
3-methyl-5-(N-(quinolin-8-ylmethyl)-N-phenethylsulfamoyl)benzofuran-2-carboxylic acid (F27-S44);
3-methyl-5-(N-(naphthalen-2-ylmethyl)-N-phenethylsulfamoyl)benzofuran-2-carboxylic acid (F27-S45);
3-methyl-5-(N-(2,6-difluorobenzyl)-N-phenethylsulfamoyl)benzofuran-2-carboxylic acid (F27-S46);
3-methyl-5-(N-(2-fluoro-6-chlorobenzyl)-N-phenethylsulfamoyl)benzofuran-2-carboxylic acid (F27-S47);
3-methyl-5-(N-(2-fluoro-6-(trifluoromethyl)benzyl)-N-phenethylsulfamoyl)benzofuran-2-carboxylic acid (F27-S48);
3-methyl-5-(N-(2,6-dichlorobenzyl)-N-phenethylsulfamoyl)benzofuran-2-carboxylic acid (F27-S49);
3-methyl-5-(N-(2,6-dimethylbenzyl)-N-phenethylsulfamoyl)benzofuran-2-carboxylic acid (F27-S50);
3-methyl-5-(N-(2,4-difluorobenzyl)-N-phenethylsulfamoyl)benzofuran-2-carboxylic acid (F27-S51);
3-methyl-5-(N-(2-fluoro-4-chlorobenzyl)-N-phenethylsulfamoyl)benzofuran-2-carboxylic acid (F27-S52);
3-methyl-5-(N-(2-fluoro-4-bromobenzyl)-N-phenethylsulfamoyl)benzofuran-2-carboxylic acid (F27-S53);
3-methyl-5-(N-(2-fluoro-4-(trifluoromethyl)benzyl)-N-phenethylsulfamoyl)benzofuran-2-carboxylic acid (F27-S55);
3-methyl-5-(N-(2-chloro-4-fluorobenzyl)-N-phenethylsulfamoyl)benzofuran-2-carboxylic acid (F27-S56);
3-methyl-5-(N-(2-(trifluoromethyl)-4-fluorobenzyl)-N-phenethylsulfamoyl)benzofuran-2-carboxylic acid (F27-S57);
3-methyl-5-(N-(2,4-bis(trifluoromethyl)benzyl)-N-phenethylsulfamoyl)benzofuran-2-carboxylic acid (F27-S58);
3-methyl-5-(N-(2-(trifluoromethyl)-4-bromobenzyl)-N-phenethylsulfamoyl)benzofuran-2-carboxylic acid (F27-S59);
3-methyl-5-(N-(2,4-dichlorobenzyl)-N-phenethylsulfamoyl)benzofuran-2-carboxylic acid (F27-S60);
3-methyl-5-(N-(2-methyl-5-fluorobenzyl)-N-phenethylsulfamoyl)benzofuran-2-carboxylic acid (F27-S61);
3-methyl-5-(N-(2,6-difluoro-4-chlorobenzyl)-N-phenethylsulfamoyl)benzofuran-2-carboxylic acid (F27-S62);
3-methyl-5-(N-([1,1'-biphenyl]-4-ylmethyl)-N-phenethylsulfamoyl)benzofuran-2-carboxylic acid (F27-S82);
3-methyl-5-(N-(2'-cyano-[1,1'-biphenyl]-4-yl)methyl)-N-phenethylsulfamoyl)benzofuran-2-carboxylic acid (F27-S83);
3-methyl-5-(N-((2'-(*tert*-butoxycarbonyl)-[1,1'-biphenyl]-4-yl)methyl)-N-phenethylsulfamoyl)benzofuran-2-carboxylic acid (F27-S84);
3-methyl-5-(N-(4-benzoylbenzyl)-N-phenethylsulfamoyl)benzofuran-2-carboxylic acid (F27-S85);
ethyl 3-methyl-5-(N-phenyl-N-phenethylsulfamoyl)benzofuran-2-carboxylate (M8-10);
ethyl 3-methyl-5-(N-(4-benzylphenyl)-N-phenethylsulfamoyl)benzofuran-2-carboxylate (M8-11);
ethyl 3-methyl-5-(N-(4-phenoxyphenyl)-N-phenethylsulfamoyl)benzofuran-2-carboxylate (M8-12);
ethyl 3-methyl-5-(N,N-diphenylethylsulfamoyl)benzofuran-2-carboxylate (M8-13);
ethyl 3-methyl-5-(N-(4-morpholinophenyl)-N-phenethylsulfamoyl)benzofuran-2-carboxylate (M8-63);
ethyl 3-methyl-5-(N-(4-(4-methylpiperazin-1-yl)phenyl)-N-phenethylsulfamoyl)benzofuran-2-carboxylate (M8-64);
ethyl 3-methyl-5-(N-(4-(4-(*tert*-butoxycarbonyl)piperazin-1-yl)phenyl)-N-phenethylsulfamoyl)benzofuran-2-carboxylate (M8-65);
ethyl 3-methyl-5-(N-(4-(4-(3,3-dimethylbutyryl)piperazin-1-yl)phenyl)-N-phenethylsulfamoyl)benzofuran-2-carboxylate (M8-66);
ethyl 3-methyl-5-(N-(4-(4-(propoxycarbonyl)piperazin-1-yl)phenyl)-N-phenethylsulfamoyl)benzofuran-2-carboxylate (M8-67);
ethyl 3-methyl-5-(N-(4-(4-acetylpiperazin-1-yl)phenyl)-N-phenethylsulfamoyl)benzofuran-2-carboxylate (M8-68);
ethyl 3-methyl-5-(N-(4-(4-benzoylpiperazin-1-yl)phenyl)-N-phenethylsulfamoyl)-3-methylbenzofuran-2-carboxylate (M8-69);
ethyl 3-methyl-5-(N-(4-(4-benzylpiperazin-1-yl)phenyl)-N-phenethylsulfamoyl)benzofuran-2-carboxylate (M8-70);
ethyl 3-methyl-5-(N-(4-(4-(dimethylcarbamoyl)piperazin-1-yl)phenyl)-N-phenethylsulfamoyl)benzofuran-2-carboxylate (M8-71);
ethyl 3-methyl-5-(N-(4-(4-(methylsulfonyl)piperazin-1-yl)phenyl)-N-phenethylsulfamoyl)benzofuran-2-carboxylate (M8-72);
ethyl 3-methyl-5-(N-(4-(1-(*tert*-butoxycarbonyl)piperidin-4-yl)phenyl)-N-phenethylsulfamoyl)benzofuran-2-carboxylate (M8-73);
ethyl 3-methyl-5-(N-(4-(4-(*tert*-butoxycarbonyl)piperazin-1-yl)-3-(trifluoromethyl)phenyl)-N-phenethylsulfamoyl)benzofuran-2-carboxylate (M8-74);
ethyl 3-methyl-5-(N-(6-(4-(*tert*-butoxycarbonyl)piperazin-1-yl) pyridin-3-yl)-N-phenethylsulfamoyl)benzofuran-2-carboxylate (M8-75);
ethyl 3-methyl-5-(N-(4-((4-(tert-butoxycarbonyl)piperazin-1-yl)methyl)phenyl)-N-phenethylsulfamoyl) benzofuran-2-carboxylate (M8-76);
ethyl 3-methyl-5-(N-(2-(4-(*tert*-butoxycarbonyl)piperazin-1-yl)phenyl)-N-phenethylsulfamoyl)benzofuran-2-carboxylate (M8-77);
ethyl 3-methyl-5-(N-(3-(4-methylpiperazin-1-yl)phenyl)-N-phenethylsulfamoyl)benzofuran-2-carboxylate (M8-78);
ethyl 3-methyl-5-(N-(4-morpholinobenzyl)-N-phenethylsulfamoyl)benzofuran-2-carboxylate (M8-79);
ethyl 3-methyl-5-(N-(4-(4-methylpiperazin-1-yl)benzyl)-N-phenethylsulfamoyl)benzofuran-2-carboxylate (M8-80);
ethyl 3-methyl-5-(N-(4-(4-(*tert*-butoxycarbonyl)piperazin-1-yl)benzyl)-N-phenethylsulfamoyl)benzofuran-2-carboxylate (M8-81);
ethyl 3-methyl-5-(N-phenethyl-N-(4-phenoxybenzyl)sulfamoyl)benzofuran-2-carboxylate (M8-86);
ethyl 3-methyl-5-(N-(2-(4-(*tert*-butoxycarbonyl)piperazin-1-yl)benzyl)-N-phenethylsulfamoyl)benzofuran-2-carboxylate (M8-87);
ethyl 3-methyl-5-(N-(3-(4-(*tert*-butoxycarbonyl)piperazin-1-yl)benzyl)-N-phenethylsulfamoyl)benzofuran-2-carboxylate (M8-88);
3-methyl-5-(N-phenethyl-N-phenylsulfamoyl)benzofuran-2-carboxylic acid (F27-S10);
3-methyl-5-(N-(4-benzylphenyl)-N-phenethylsulfamoyl)benzofuran-2-carboxylic acid (F27-S11);
3-methyl-5-(N-(4-phenoxyphenyl)-N-phenethylsulfamoyl)benzofuran-2-carboxylic acid (F27-S12);
3-methyl-5-(N,N-diphenylethylsulfamoyl)benzofuran-2-carboxylic acid (F27-S13);
3-methyl-5-(N-(4-morpholinophenyl)-N-phenethylsulfamoyl)benzofuran-2-carboxylic acid (F27-S63);
3-methyl-5-(N-(4-(4-methylpiperazin-1-yl)phenyl)-N-phenethylsulfamoyl)benzofuran-2-carboxylic acid (F27-S64);
3-methyl-5-(N-(4-(4-(*tert*-butoxycarbonyl)piperazin-1-yl)phenyl)-N-phenethylsulfamoyl)benzofuran-2-carboxylic acid (F27-S65);
3-methyl-5-(N-(4-(4-(3,3-dimethylbutyryl)piperazin-1-yl)phenyl)-N-phenethylsulfamoyl)benzofuran-2-carboxylic acid (F27-S66);
3-methyl-5-(N-(4-(4-(propoxycarbonyl)piperazin-1-yl)phenyl)-N-phenethylsulfamoyl)benzofuran-2-carboxylic acid (F27-S67);
3-methyl-5-(N-(4-(4-acetylpiperazin-1-yl)phenyl)-N-phenethylsulfamoyl)benzofuran-2-carboxylic acid (F27-S68);
3-methyl-5-(N-(4-(4-benzoylpiperazin-1-yl)phenyl)-N-phenethylsulfamoyl)benzofuran-2-carboxylic acid (F27-S69);
3-methyl-5-(N-(4-(4-benzylpiperazin-1-yl)phenyl)-N-phenethylsulfamoyl)benzofuran-2-carboxylic acid (F27-S70);
3-methyl-5-(N-(4-(4-(dimethylcarbamoyl)piperazin-1-yl)phenyl)-N-phenethylsulfamoyl)benzofuran-2-carboxylic acid (F27-S71);
3-methyl-5-(N-(4-(4-(methylsulfonyl)piperazin-1-yl)phenyl)-N-phenethylsulfamoyl)benzofuran-2-carboxylic acid (F27-S72);
3-methyl-5-(N-(4-(1-(*tert-*butoxycarbonyl)piperidin-4-yl)phenyl)-N-phenethylsulfamoyl)benzofuran-2-carboxylic acid (F27-S73);
3-methyl-5-(N-(4-(4-(*tert*-butoxycarbonyl)piperazin-1-yl)-3-(trifluoromethyl)phenyl)-N-phenethylsulfamoyl)benzofuran-2-carboxylic acid (F27-S74);
3-methyl-5-(N-(6-(4-(*tert*-butoxycarbonyl)piperazin-1-yl)pyridin-3-yl)-N-phenethylsulfamoyl)benzofuran-2-carboxylic acid (F27-S75);
3-methyl-5-(N-(4-((4-(*tert*-butoxycarbonyl)piperazin-1-yl)methyl)phenyl)-N-phenethylsulfonamide)benzofuran-2-carboxylic acid (F27-S76);
3-methyl-5-(N-(2-(4-(*tert*-butoxycarbonyl)piperazin-1-yl)phenyl)-N-phenethylsulfamoyl)benzofuran-2-carboxylic acid (F27-S77);
3-methyl-5-(N-(3-(4-methylpiperazin-1-yl)phenyl)-N-phenethylsulfamoyl)benzofuran-2-carboxylic acid (F27-S78);
3-methyl-5-(N-(4-(4-morpholinobenzyl)-N-phenethylsulfamoyl)benzofuran-2-carboxylic acid (F27-S79);
3-methyl-5-(N-(4-(4-methylpiperazin-1-yl)benzyl)-N-phenethylsulfamoyl)benzofuran-2-carboxylic acid (F27-S80);
3-methyl-5-(N-(4-(4-(tert-butoxycarbonyl)piperazin-1-yl)benzyl)-N-phenethylsulfamoyl)benzofuran-2-carboxylic acid (F27-S81);
3-methyl-5-(N-(4-(4-phenoxybenzyl)-N-phenethylsulfamoyl)benzofuran-2-carboxylic acid (F27-S86);
3-methyl-5-(N-(2-(4-(tert-butoxycarbonyl)piperazin-1-yl)benzyl)-N-phenethylsulfamoyl)benzofuran-2-carboxylic acid (F27-S87);
3-methyl-5-(N-(3-(4-(tert-butoxycarbonyl)piperazin-1-yl)benzyl)-N-phenethylsulfamoyl)benzofuran-2-carboxylic acid (F27-S88);
ethyl 3-methyl-5-(N-phenethyl-N-(2-(piperazin-1-yl)phenyl)sulfamoyl)benzofuran-2-carboxylate (M9-1);
ethyl 3-methyl-5-(N-phenethyl-N-(2-(piperazin-1-yl)benzyl)sulfamoyl)benzofuran-2-carboxylate (M9-2);
4-(2-((2-(ethoxycarbonyl)-3-methyl-N-phenethylbenzofuran)-5-sulfonylamino)phenyl)-1,1-dimethylpiperazin-1-ium(M10-90);
ethyl 3-methyl-5-(N-(2-(4-acetylpiperazin-1-yl)phenyl)-N-phenethylsulfamoyl)benzofuran-2-carboxylate (M10-91);
ethyl 3-methyl-5-(N-(2-(4-acetylpiperazin-1-yl)phenyl)-N-phenethylsulfamoyl)benzofuran-2-carboxylate (M10-92);
ethyl 3-methyl-5-(N-(2-(4-(methylsulfonyl)piperazin-1-yl)phenyl)-N-phenethylsulfamoyl)benzofuran-2-carboxylate (M10-93);
ethyl 3-methyl-5-(N-(2-(4-(4-(3,3-dimethylbutyryl)piperazin-1-yl)phenyl)-N-phenethylsulfamoyl)benzofuran-2-carboxylate (M10-94);
ethyl 3-methyl-5-(N-(2-(4-(4-(benzylpiperazin-1-yl)phenyl)-N-phenethylsulfamoyl)benzofuran-2-carboxylate (M10-95);
ethyl 3-methyl-5-(N-(2-(4-(benzoylpiperazin-1-yl)phenyl)-N-phenethylsulfamoyl)benzofuran-2-carboxylate (M10-96);
ethyl 3-methyl-5-(N-(2-(4-methylpiperazin-1-yl)benzyl)-N-phenethylsulfamoyl)benzofuran-2-carboxylate (M10-98);
4-(2-((2-(ethoxycarbonyl)-3-methyl-N-benzylethylbenzofuran)-5-sulfonylamino)phenyl)-1,1-dimethylpiperazin-1-ium(M10-99);
ethyl 3-methyl-5-(N-(2-(4-acetylpiperazin-1-yl)benzyl)-N-phenethylsulfamoyl)benzofuran-2-carboxylate (M10-100);
ethyl 3-methyl-5-(N-(2-(4-acetylpiperazin-1-yl)benzyl)-N-phenethylsulfamoyl)benzofuran-2-carboxylate (M10-101);
ethyl 3-methyl-5-(N-(2-(4-methanesulfonylpiperazin-1-yl)benzyl)-N-phenethylsulfamoyl)benzofuran-2-carboxylate (M10-102);
ethyl 3-methyl-5-(N-(2-(4-(3,3-dimethylbutyryl)piperazin-1-yl)benzyl)-N-phenethylsulfamoyl)benzofuran-2-carboxylate (M10-103);
ethyl 3-methyl-5-(N-(2-(4-benzylpiperazin-1-yl)benzyl)-N-phenethylsulfamoyl)benzofuran-2-carboxylate (M10-104);
ethyl 3-methyl-5-(N-(2-(4-benzoylpiperazin-1-yl)benzyl)-N-phenethylsulfamoyl)benzofuran-2-carboxylate (M10-105);
3-methyl-5-(N-phenethyl-N-(2-(piperazin-1-yl)phenyl)sulfamoyl)benzofuran-2-carboxylicacid (F27-S89);
4-(2-((2-carboxy-3-methyl-N-phenethylbenzofuran)-5-sulfonylamino)phenyl)-1,1-dimethylpiperazin-1-ium(F27-S90);
3-methyl-5-(N-(2-(4-acetylpiperazin-1-yl)phenyl)-N-phenethylsulfamoyl)benzofuran-2-carboxylic acid (F27-S91);
3-methyl-5-(N-(2-(4-(dimethylcarbamoyl)piperazin-1-yl)phenyl)-N-phenethylsulfamoyl)benzofuran-2-carboxylic acid (F27-S92);
3-methyl-5-(N-(2-(4-(methylsulfonyl)piperazin-1-yl)phenyl)-N-phenethylsulfamoyl)benzofuran-2-carboxylic acid (F27-S93);
3-methyl-5-(N-(2-(4-(4-(3,3-dimethylbutyryl)piperazin-1-yl)phenyl)-N-phenethylsulfamoyl)benzofuran-2-carboxylic acid (F27-S94);
3-methyl-5-(N-(2-(4-(4-(benzylpiperazin-1-yl)phenyl)-N-phenethylsulfamoyl)benzofuran-2-carboxylic acid (F27-S95);
3-methyl-5-(N-(2-(4-(benzoylpiperazin-1-yl)phenyl)-N-phenethylsulfamoyl)benzofuran-2-carboxylic acid (F27-S96);
3-methyl-5-(N-(2-(piperazin-1-yl)benzyl)-N-phenethylsulfamoyl)benzofuran-2-carboxylic acid (F27-S97);
3-methyl-5-(N-(2-(4-acetylpiperazin-1-yl)benzyl)-N-phenethylsulfamoyl)benzofuran-2-carboxylic acid (F27-S98);
4-(2-((2-carboxy-3-methyl-N-phenethylbenzofuran)-5-sulfonamido)methyl)phenyl)-1,1-dimethylpiperazin-1-ium(F27-S99);
3-methyl-5-(N-(2-(4-acetylpiperazin-1-yl)benzyl)-N-phenethylsulfamoyl)benzofuran-2-carboxylic acid (F27-S100);
3-methyl-5-(N-(2-(4-(dimethylcarbamoyl)piperazin-1-yl)benzyl)-N-phenethylsulfamoyl)benzofuran-2-carboxylic acid (F27-S101);
3-methyl-5-(N-(2-(4-(methylsulfonyl)piperazin-1-yl)benzyl)-N-phenethylsulfamoyl)benzofuran-2-carboxylic acid (F27-S102);
3-methyl-5-(N-(2-(4-(4-(3,3-dimethylbutyryl)piperazin-1-yl)benzyl)-N-phenethylsulfamoyl)benzofuran-2-carboxylic acid (F27-S103);
3-methyl-5-(N-(2-(4-benzylpiperazin-1-yl)benzyl)-N-phenethylsulfamoyl)benzofuran-2-carboxylic acid (F27-S104) and
3-methyl-5-(N-(2-(4-benzoylpiperazin-1-yl)benzyl)-N-phenethylsulfamoyl)benzofuran-2-carboxylic acid (F27-S105);
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

This application further provides a compound of formula (I), or a pharmaceutically acceptable salt or ester, stereoisomer, tautomer, geometric isomer, prodrug, hydrate, solvate and crystal form of the compound, metabolite forms thereof, or any combination or mixture thereof; 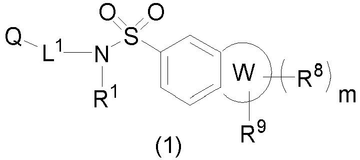
wherein, Q is selected from a group consisting of hydrogen, 6- to 10-membered aryl, 5- to 14-membered heteroaryl, 3- to 6-membered cycloalkyl, and 3- to 6-membered heterocyclyl; optionally, the 6- to 10-membered aryl, the 5- to 14-membered heteroaryl, the 3-to 6-membered cycloalkyl, and the 3- to 6-membered heterocyclyl are substituted with one or more M, M is selected from the following groups: halogen, nitro, hydroxyl, cyano, C₁-C₆ alkyl, C₂-C₆ alkenyl, C₂-C₆ alkynyl, C₁-C₆haloalkyl, C₁-C₆ alkoxy, C₁-C₆ alkanoyl, and C₁-C₆ amido;
L¹ is absent or selected from -(CH₂)ₙ-, n is an integer selected from 1 to 6; optionally, -(CH₂)ₙ- is substituted with one or more groups selected from the following: phenyl, halogen , nitro, cyano, hydroxyl, C₁-C₆ alkyl, deuterium, and tritium;
R¹ is -(CH₂)ₙ-R⁴, wherein n is an integer from 0 to 6, R⁴ and the group in Group A are as defined in any one of claims 1-6 or 9.
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C6 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, C1-C6 alkoxycarbonyl, unsubstituted 3- to 6-membered cycloalkylcarbonyl, substituted 3- to 6-membered cycloalkylcarbonyl, substituted 5- to 14-membered heteroarylcarbonyl, unsubstituted 5- to 14-membered heteroarylcarbonyl, unsubstituted C2-C6 alkenylcarbonyl, unsubstituted 5- to 10-membered heterocyclylcarbonyl, and substituted 5- to 10-membered heterocyclylcarbonyl;
W is 5- to 6-membered heteroaryl;
R⁸ is selected from hydrogen, halogen, unsubstituted C₁-C₆ alkyl or substituted C₁-C₆ alkyl; m is selected from 1, 2, 3, and 4; in a case of multiple R⁸, all the R⁸ can be the same or different;
R⁹ is selected from -CH₂OH or -(C=O)R², and R² is as defined in any one of claims 1-6.

In some embodiments, Q is selected from 6- to 10-membered aryl, 5- to 8-membered monocyclic heteroaryl, 8- to 14-membered fused heteroaryl, 5- to 6-membered cycloalkyl, and 5- to 6-membered heterocyclyl; optionally, the 6- to 10-membered aryl, the 5-to 8-membered monocyclic heteroaryl, the 8- to 14-membered fused heteroaryl, the 5- to 6-membered cycloalkyl, and the 5- to 6-membered heterocyclyl are substituted with one or more M. In some embodiments, Q is selected from 6- to 10-membered aryl, 5- to 8-membered monocyclic heteroaryl, and 5- to 6-membered cycloalkyl. In some embodiments, Q is selected from phenyl, thienyl, and cyclohexyl. In some embodiments, Q is phenyl.

In some embodiments, L¹ is absent or selected from -(CH₂)ₙ-, wherein n is 1, 2 or 3. In some embodiments, L¹ is -(CH₂)₂-.

In some embodiments, R¹ is phenyl substituted with one or more groups selected from Group A.

In some embodiments, A is heterocycloalkyl that is unsubstituted or substituted with one or more groups selected from Group B. In some embodiments, the heterocyclyl is 5-to 7-membered monoheterocyclyl, 9- to 10-membered spiroheterocyclyl, or 6- to 8-membered bridged heterocyclyl. In some embodiments, the heterocyclyl is 5- to 7-membered nitrogen-containing monoheterocyclyl, 9- to 10-membered nitrogen-containing spiroheterocyclyl, or 6-to 8-membered nitrogen-containing bridged heterocyclyl. In some embodiments, the heterocyclyl is piperazinyl.

In some embodiments, B is selected from: -(C=O)-phenyl, -(C=O)-5- to 6-membered heteroaryl, -(C=O)-propenyl, -(C=O )-5- to 6-membered cycloalkyl, -(C=O)-5- to 6-membered heterocyclyl, and -(C=O)-C₁-C₆ alkyl. Optionally, the phenyl, the 5- to 6-membered heteroaryl, the 5- to 6-membered cycloalkyl, 5- to 6-membered heterocyclyl are substituted with 1, 2 or 3 substituents selected from the following: C₁- C₆ alkyl (e.g., methyl), formamido, acetamido, C₁-C₆ haloalkyl (e.g., trifluoromethyl), cyano, halogen (e.g., fluorine, chlorine, bromine), amino, nitro, C₁-C₆ alkanoyl (e.g., acetyl), C₁-C₆ alkoxy (e.g., methoxy), benzyl, and ethinyl phenyl. In some embodiments, the 5- to 6-membered heteroaryl is selected from pyridyl, thienyl, furyl, pyrrolyl, imidazolyl, oxazolyl, thiazolyl, and pyrazolyl; optionally, the 5- to 6-membered heteroaryl is substituted with one or more C₁-C₆ alkyls (e.g., methyl) or halogen (e.g., bromine). In some embodiments, the 5- to 6-membered heterocyclyl is tetrahydrothiophenyl.

In some embodiments, B is selected from -(C=O)-phenyl and -(C=O)-thienyl.

In some embodiments, the compound is as represented by formula (2), (3), (4), or (5), 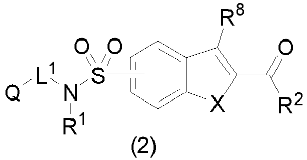 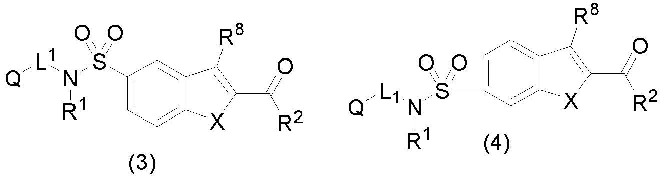 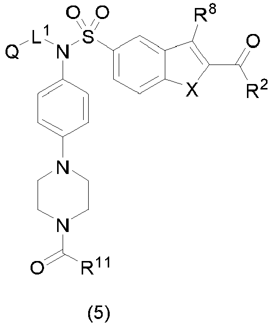 wherein X is O, S, or N-R⁸, and R⁸ is hydrogen or unsubstituted C1-C6 alkyl.

In some embodiments, in formula (2), (3), (4), or (5), R⁸ is methyl.

In some embodiments, in formula (2), (3), (4), or (5), R² is hydroxyl or unsubstituted C1-C6 alkoxy.

In some embodiments, the compound is selected from:

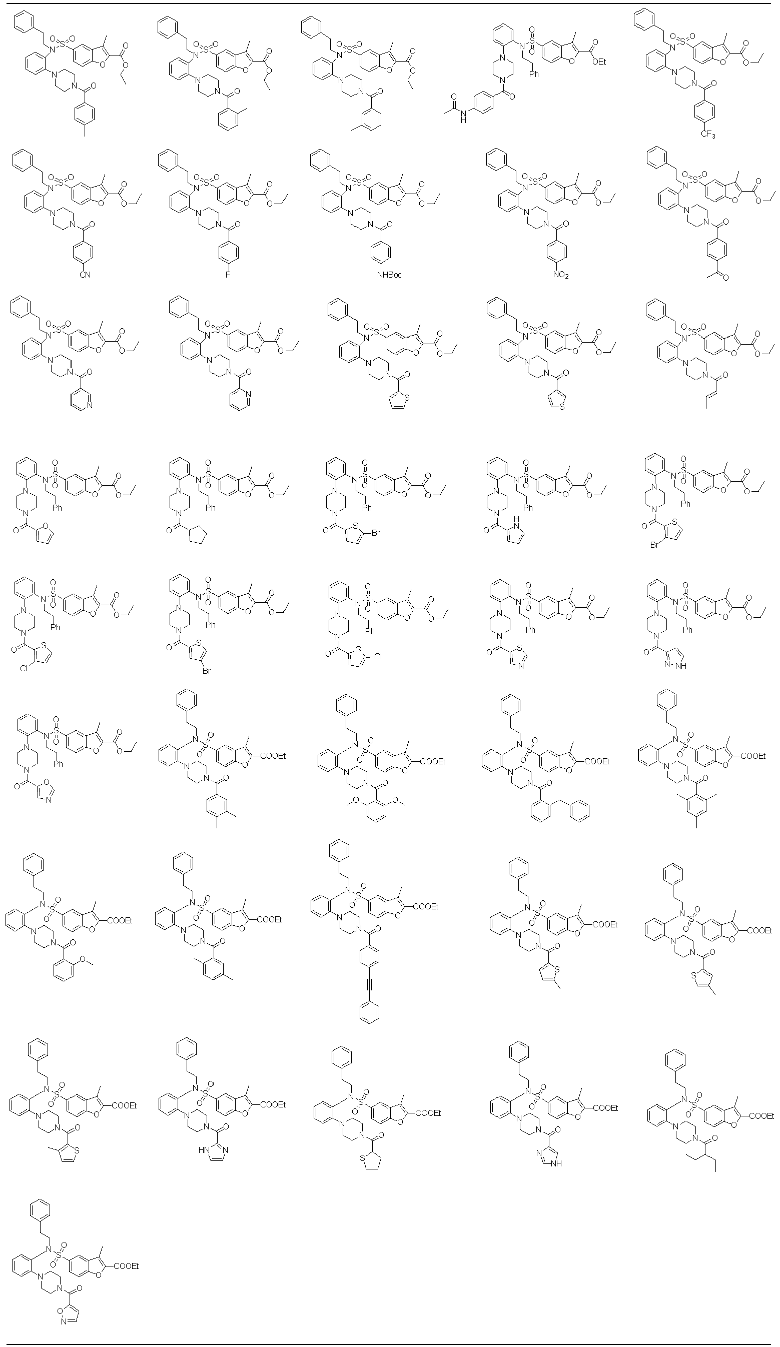

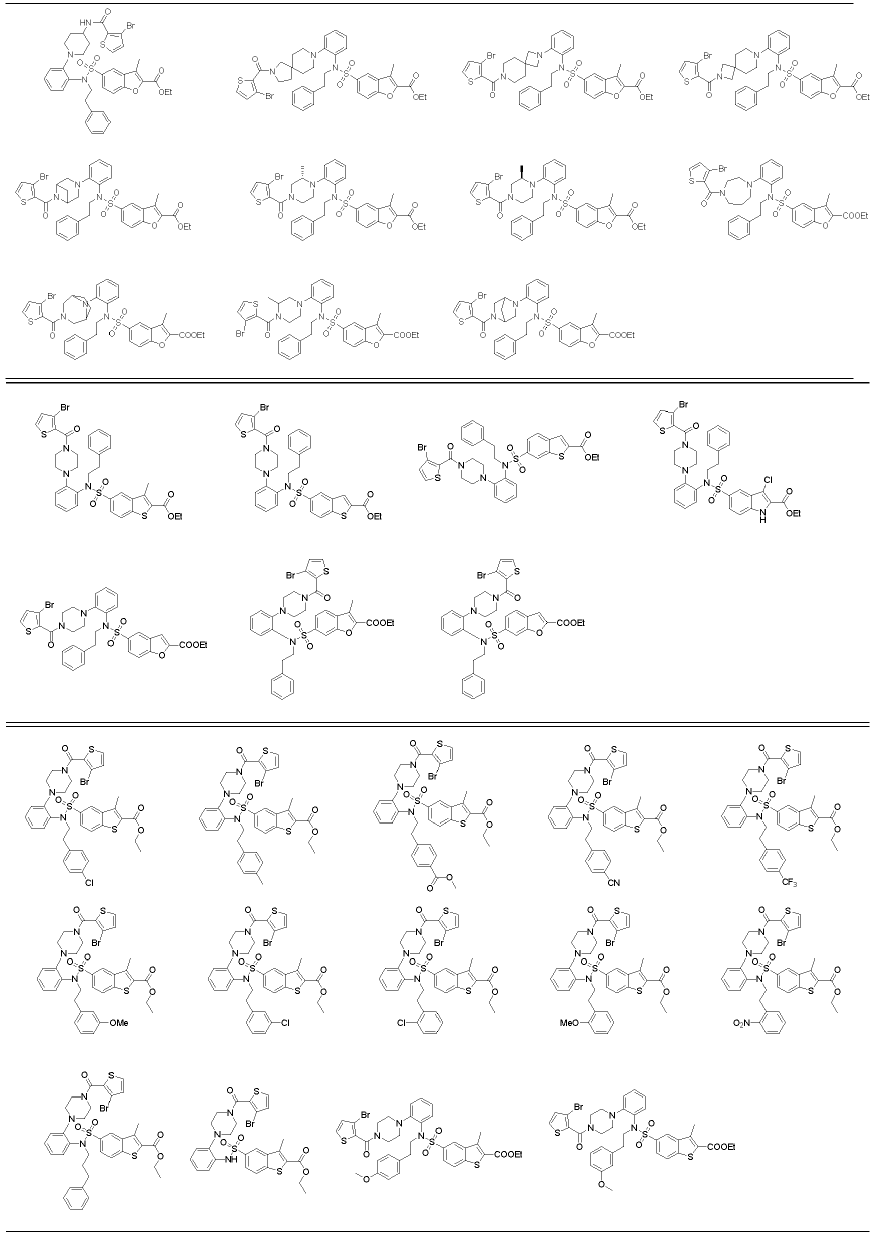

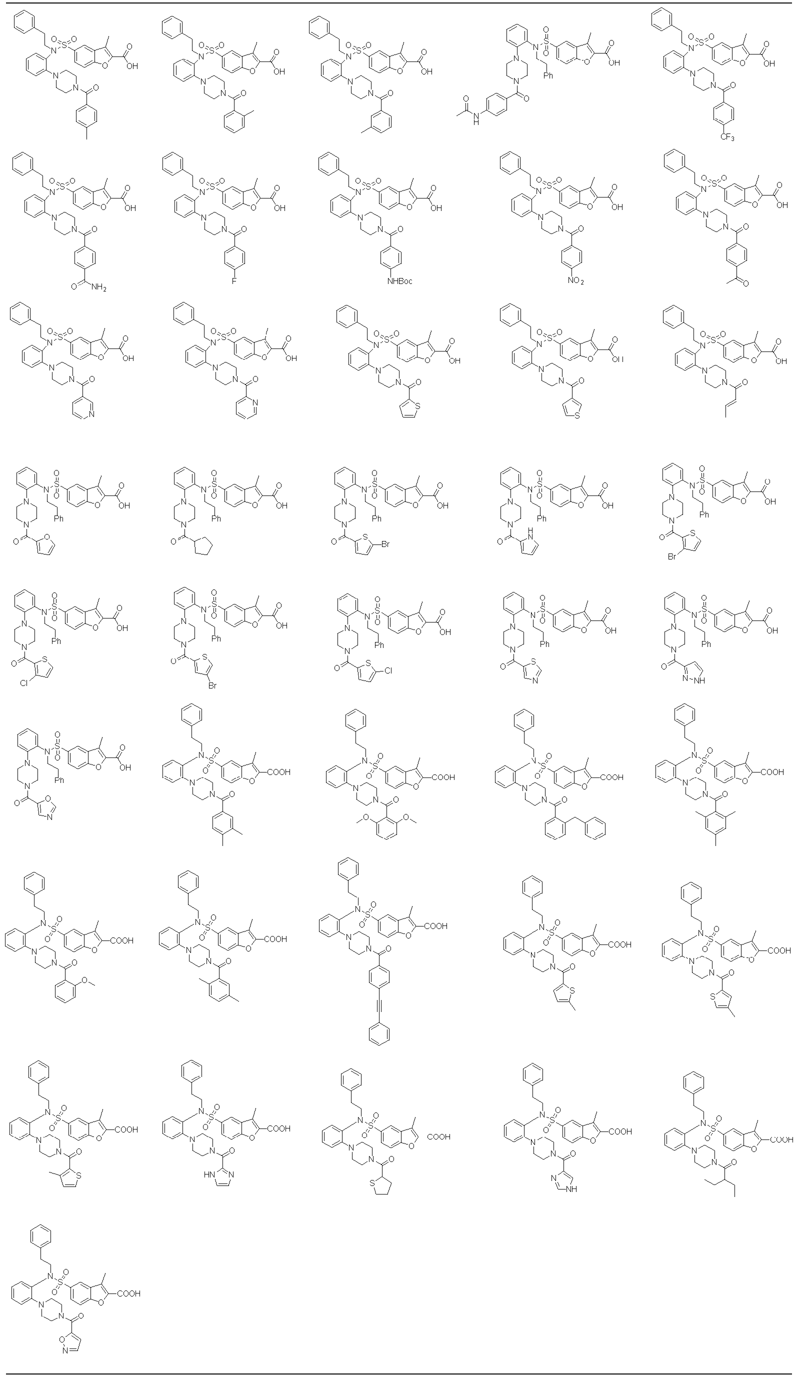

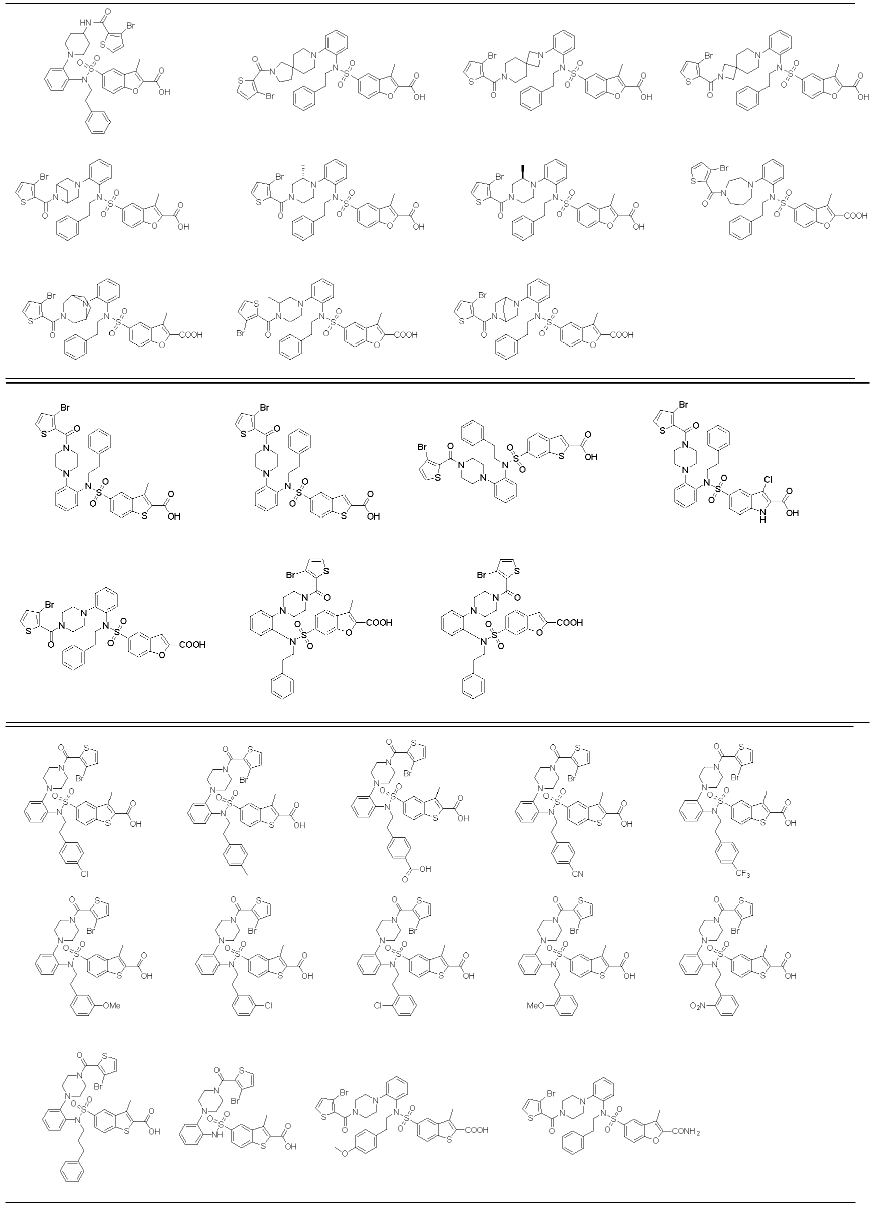

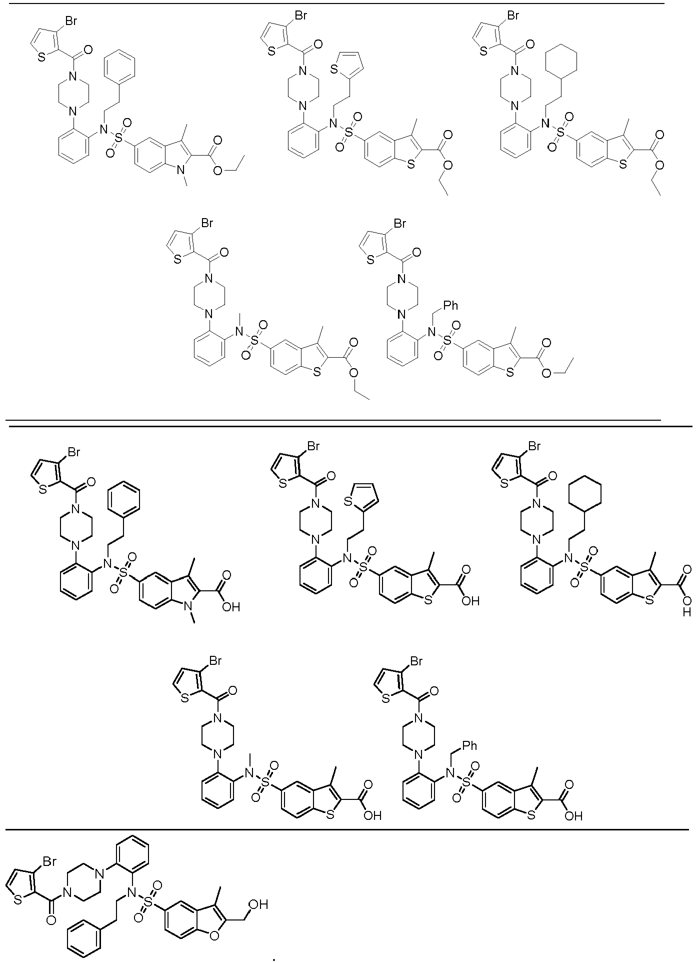

In a third aspect, this application provides a pharmaceutical composition, comprising an effective dose of the compound of the present invention or a pharmaceutically acceptable salt or ester, stereoisomer, tautomer, geometric isomer, prodrug, hydrate, solvate and crystal form of the compound, metabolite forms thereof, or any combination or mixture thereof. Optionally, the pharmaceutical composition may also include one or more pharmaceutically acceptable carriers or excipients. The pharmaceutical composition may be home-made into any pharmaceutically acceptable dosage form. The pharmaceutical composition may also be applied to patients in need of such treatment by way of oral, parenteral, rectal or pulmonary administration. In the case of oral administration, the pharmaceutical composition may be made into conventional solid preparations, such as tablets, capsules, pills, granules, etc.; it may also be made into oral liquid preparations, such as oral solutions and oral suspensions, and syrup. In the case of oral preparations, suitable fillers, binders, disintegrants, lubricants, etc. may be added. In the case of parenteral administration, the pharmaceutical composition may be prepared into injection preparations, including injection solutions, sterile powders for injection, and concentrated solutions for injection. In the case of injection preparations, they may be produced by a conventional method in the current pharmaceutical field. In the case of preparation of injection preparations, it is not required to add additives, or appropriate additives can be added according to the properties of the medicament. In the case of rectal administration, the pharmaceutical composition may be made into suppositories and the like. In the case of pulmonary administration, the pharmaceutical composition may be made into inhalants or sprays. The pharmaceutical composition of the present invention may be in the form of preparation suitable for oral or parenteral administration, and its dosage may be 0.1-1000 mg/time/day.

In a fourth aspect, this application provides a preparation method of the benzofuran derivative of formula (I) or formula (II) or a pharmaceutically acceptable salt or ester, stereoisomer, tautomer, geometric isomer, prodrug, hydrate, solvate and crystal form of the benzofuran derivative, metabolite forms thereof, or any combination or mixture thereof, comprising the following step:
allowing 2-phenylethylamine to react with a compound of formula (III) to obtain a compound of formula (IV); 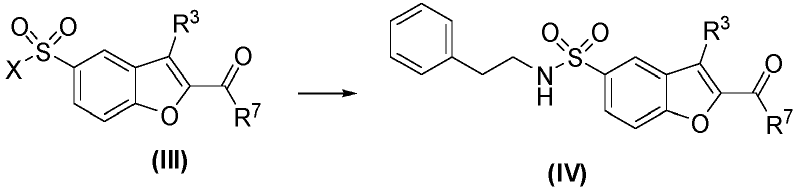
in a case wherein R¹ is not hydrogen, after obtaining the compound of formula (IV), further comprising: allowing the compound of formula (IV) to react with X-R¹ to obtain a compound of formula (V); 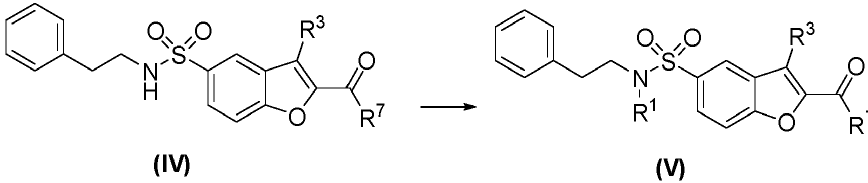
optionally, further comprising: carrying out group transformation on the compound of formula (IV) or the compound of formula (V) to obtain the compound of formula (I) or the compound of formula (II); 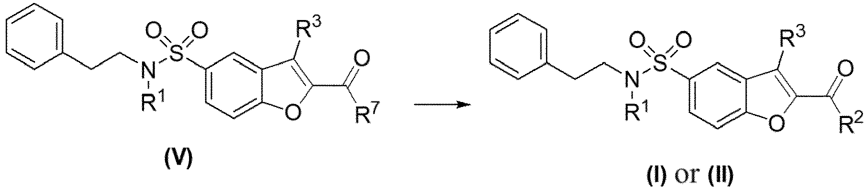
alternatively,
allowing the compound of formula (III) to react with the compound of formula (VI) to obtain the compound of formula (V);

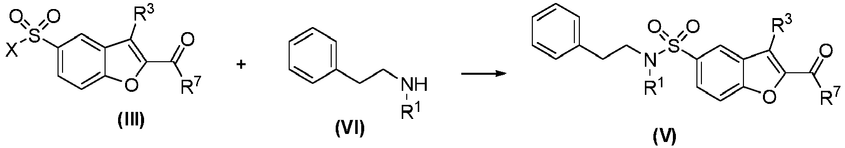

optionally, further comprising: carrying out group transformation on the compound of formula (V) to obtain the compound of formula (I) or the compound of formula (II); 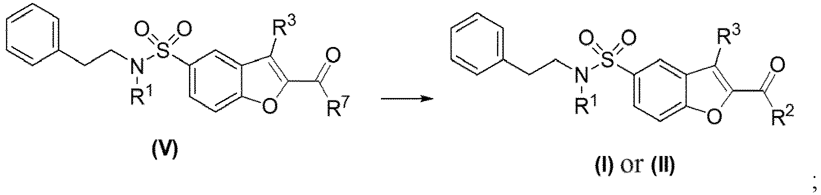
wherein X in formula (III) is chlorine, bromine or iodine; R⁷ in formula (III), formula (IV) and formula (V) is a C1-C6 alkyl; R¹ in formula (V) and formula (VI) and R³ in formula (III), formula (IV) and formula (V) are as defined in the above-described formula (I) or formula (II).

In some embodiments, the benzofuran derivative of formula (I) or formula (II) of the present invention or the pharmaceutically acceptable salt or ester, stereoisomer, tautomer, geometric isomer, prodrug, hydrate, solvate and crystal form of the benzofuran derivative, metabolite forms thereof, or any combination or mixture thereof may be prepared by the following examples:

The synthesis routes of compound F27 and compounds F27-S14 to F27-S62 and F27-S82 to F27-S85 are shown in Solution 1.1. First, o-hydroxyacetophenone and ethyl bromoacetate are refluxed in N,N-dimethylformamide to produce intermediate M1. Intermediate M1 reacts with chlorosulfonic acid in chloroform to obtain intermediate M2, and then the intermediate M2 is condensed with 2-phenylethane-1-amine to produce intermediate M3. The intermediate M3 has nucleophilic substitution with substituted benzyl bromide to produce intermediates M4 and F27-S8. M4 is hydrolyzed under the action of strong base to obtain the final products F27, F27-S14 to F27-S62, and F27-S82 to F27-S85.

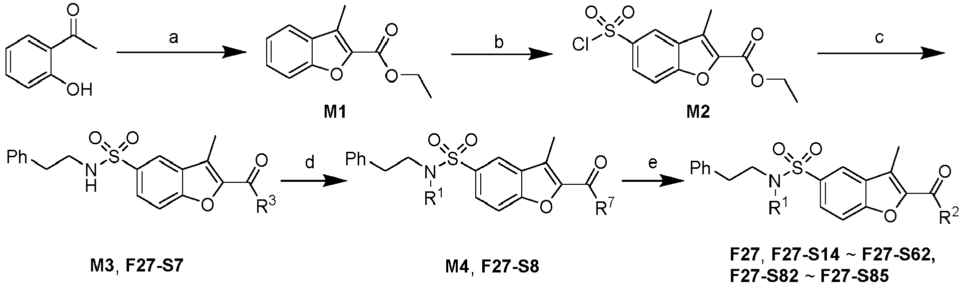

**Solution 1.1** Synthesis routes of compound F27 and compounds F27-S14 to F27-S62 and F27-S82 to F27-S85. Regents and reaction conditions: (a) ethyl bromoacetate, potassium carbonate, and *N*,*N*-dimethylformamide; heating to reflux and react for 3 h, with a yield of 37%; (b) chlorosulfonic acid and chloroform; 0 °C to room temperature; reacting for 4 h, with a yield of 45%; (c) 2-phenylethane-1-amine, potassium carbonate, and anhydrous dichloromethane; reacting for 3 h, with a yield of 87%; (d) substituted benzyl bromide, potassium carbonate, and dichloromethane; heating to 60 °C, reacting for 2-72 h, with a yield of 48%-99%; and (e) sodium hydroxide or lithium hydroxide and ethanol solution (ethanol/water = 4:1); heating to reflux and react for 1 h, with a yield of 34%-99%.

As an embodiment, the designed synthesis routes of compounds F27-S10 to F27-S13, F27-S63 to F27-S81 and F27-S86 to F27-S88 are shown in Solution 1.2. First, 4-nitrophenylpiperazine is condensed with a substituted acid chloride to form intermediate M5. The intermediate M5 is reduced in zinc powder-hydrochloric acid to produce the corresponding amino intermediate M6. Phenyl acetaldehyde or phenethylamine and substituted benzylamine or benzaldehyde have reductive amination reaction to produce intermediate M7. Alternatively, (2-iodoethyl)benzene and substituted aniline or phenethylamine have nucleophilic substitution to produce intermediate M7. The intermediate M7 and the intermediate M2 have amide condensation to produce intermediate M8, and the intermediate M8 is then hydrolyzed under the action of strong base to obtain the final products F27-S10 to F27-S13, F27-S63 to F27-S81, and F27-S86 to F27-S88.

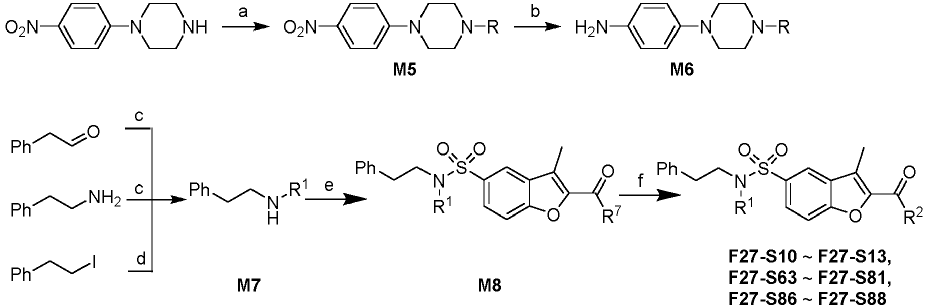

**Solution 1.2** Synthesis routes of compounds F27-S10 to F27-S13, F27-S63 to F27-S81, and F27-S86 to F27-S88. Regents and reaction conditions: (a) substituted acid chloride, triethylamine, and anhydrous tetrahydrofuran; 0 °C to room temperature; reacting for 10 h, with a yield of 87%-93%; (b) zinc powder and concentrated hydrochloric acid; reacting at room temperature for 11 h, with a yield of 60%-99%; (c) substituted benzylamine or benzaldehyde, sodium cyanoborohydride, trimethyl orthoformate, and methanol; 0 °C to room temperature; reacting for 10 h, with a yield of 44%-94%; (d) substituted aniline or phenethylamine, potassium carbonate, and acetonitrile; heating to 60 °C; reacting for 24 h, with a yield of 6%-82%; (e) intermediate M2, potassium carbonate, and dichloromethane; heating to 60 °C; reacting for 2-72 h, with a yield of 25%-97%; and (f) sodium hydroxide and ethanol solution (ethanol/water = 4:1); heating to reflux and react for 1 h, with a yield of 38%-99%.

The designed synthesis routes of compounds F27-S89 to F27-S105 are shown in Solution 1.3. First, Boc protecting groups in intermediates M8-77 and M8-87 are removed in trifluoroacetic acid to produce intermediate M9. The intermediate M9 is condensed with substituted acid anhydride, acid chloride or carboxylic acid to produce intermediate M10; alternatively, the intermediate M9 has nucleophilic substitution with halogenated hydrocarbon to produce intermediate M10. The intermediate M10 is hydrolyzed under the action of strong base to obtain the final compounds F27-S89 to F27-S105.

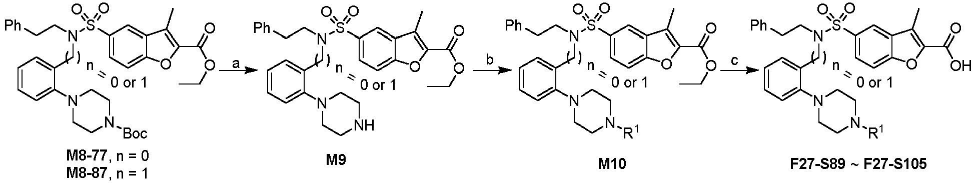

**Solution 1.3** Synthesis routes of compounds F27-S89 to F27-S105. Regents and reaction conditions: (a) trifluoroacetic acid, dichloromethane; reacting at room temperature for 2 h, with a yield of 98%-99%; (b) substituted anhydride, diisopropylethylamine, dimethylaminopyridine, anhydrous dichloromethane, 0 °C to room temperature, reacting for 5 h; alternatively, substituted acid chloride, triethylamine, anhydrous dichloromethane, 0 °C to room temperature, reacting for 5 h; alternatively, substituted carboxylic acid, carbodiimide hydrochloride, hydroxybenzotriazole, diisopropylethylamine, dimethylaminopyridine, and anhydrous dichloromethane, reacting at room temperature for 24 h; alternatively, halogenated hydrocarbon, potassium carbonate, and acetonitrile, heating to 60 °C, reacting for 24 h, with a yield of 52%-99%; and (c) sodium hydroxide and ethanol solution (ethanol/water = 4:1); heating to reflux and react for 1 h, with a yield of 28%-89%.

In another aspect, this application also relates to the use of the compound of the present invention, the pharmaceutically acceptable salt or ester of the compound or stereoisomers thereof in preparation of a medicament, wherein the medicament is used for the prevention and/or treatment of an FXR-related disease in a subject.

This application further provides a method for treating and/or preventing an FXR-related disease in a subject, including: administrating a therapeutically and/or preventively effective dose of the compound of the present invention, the pharmaceutically acceptable salt or ester of the compound, or stereoisomers thereof or the pharmaceutical composition of the present invention to a subject in need.

In this application, the compound, or the pharmaceutically acceptable salt or ester, stereoisomer, tautomer, geometric isomer, prodrug, hydrate, solvate and crystal form of the compound, metabolite forms thereof, or any combination or mixture thereof may be used as an FXR agonist or FXR antagonist.

In this application, the FXR-related disease may be selected from metabolism-related diseases, cardiovascular diseases, liver diseases, cancers, kidney diseases, enteropathies, inflammation or any combination thereof. The metabolism-related diseases include, but are not limited to, glycolipid metabolism diseases, cholesterol metabolism diseases, lipid metabolism diseases, bile acid metabolism diseases or any combination thereof. The glycolipid metabolism diseases include but are not limited to obesity, diabetes (such as type 2 diabetes), dyslipidemia, hyperlipidemia, hypercholesterolemia, cholestasis, non-alcoholic fatty liver disease, non-alcoholic steatohepatitis or any combination thereof. The cardiovascular diseases include, but are not limited to, atherosclerosis, myocardial ischemia-reperfusion injury, or combinations thereof. The liver diseases include, but are not limited to, liver resection, liver injury, or combinations thereof. The cancers include, but are not limited to breast cancer, melanoma, meningioma, soft tissue sarcoma, salivary gland tumor, primary liver cancer, intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia, gastric cancer, nasopharyngeal cancer, laryngeal cancer, oral cancer, esophageal cancer, lung cancer, kidney cancer, cervical cancer, choriocarcinoma, vulvar cancer, skin cancer, endometrial cancer, ovarian cancer, prostate cancer, pancreatic cancer, colon cancer, rectum cancer, colorectal cancer, Kaposi's sarcoma, non-melanoma skin cancer (including squamous cell carcinoma and basal cell carcinoma), hemangioma, glioma, and secondary complications of these diseases or any combination thereof. The kidney diseases include, but are not limited to, nephritis, nephrotic syndrome, atheroembolic renal disease, or any combination thereof.

In this application, the subject or patient may be any animal, preferably a mammal, such as a bovine, an equine, a porcine, a canine, a feline, a rodent, and a primate. Among these, a particularly preferred subject is human. Other features and advantages of this application will be described hereinafter, and partly become obvious from the specification or be understood by implementing this application. Other advantages of this application can be achieved and obtained through the solutions described in the specification and drawings.

### BRIEF DESCRIPTION OF THE DRAWINGS

The accompanying drawings are provided for better understanding of the technical solution of this application, and constitute a part of the specification. Together with the embodiments of this application, they are used to explain the technical solution of this application, and do not constitute a limitation on the technical solution of this application.

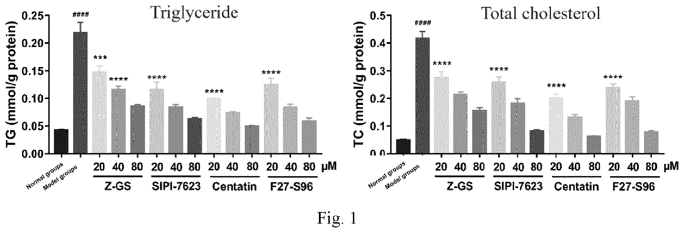

Fig. 1 shows the activity on decreasing TG (triglyceride) (A) and TC (total cholesterol) (B) of the FXR antagonist compound F27-S96 of the present invention. #### p < 0.001 Comparison between model groups and solvent groups; *** p < 0.005, **** p < 0.001, comparison between treated groups and model groups. The data in the figure are the results of three independent repeated experiments.

### DETAILED DESCRIPTION

In order to make the objective, technical solutions and advantages of this application more clear, the embodiments of the invention will be described in detail below. It should be noted that embodiments described herein and the features in the embodiments may be combined in any way with each other without conflict.
MS was measured using Agilent (ESI) mass spectrometer (produced by Agilent, model: Agilent 6120B)
A high-resolution mass spectrum was prepared, and the mass spectrum was recorded using a PE SCLEX QSTAR spectrometer.

The H NMR and C NMR spectra were recorded using a Bruker AVIII-400 spectrometer.

Thin-layer chromatography purification was performed by using GF254 (0.4-0.5nm) silica gel plates.

The reaction was monitored by thin layer chromatography (TLC). The developing solvent system used includes but is not limited to: a dichloromethane and methanol system, an n-hexane and ethyl acetate system, and a petroleum ether and ethyl acetate system. The volume ratio of the solvent was adjusted according to the compound, but a small amount of triethylamine and the like may be added for adjustment.

Unless otherwise specified in the embodiments, reaction temperature is room temperature (20-30 °C).

The reagents used in the examples were purchased from companies such as Acros oRganmes, Aldrich Chemical Company, or J&K Chemical.

Abbreviations as used herein have the following meanings:
EA: ethyl acetate; DCM: dichloromethane; MeOH: methanol; EtOH: ethanol; PE: petroleum ether; THF: tetrahydrofuran; DMF: N,N-dimethylformamide; AcCl: acetyl chloride; Ac₂O: acetic anhydride; DMSO: dimethyl sulfoxide; NaOH: sodium hydroxide; LiOH: lithium hydroxide.

### Example 1

### Ethyl 3-methylbenzofuran-2-carboxylate (M1)

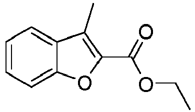

2-hydroxyacetophenone (2.72 g, 20 mmol), ethyl bromoacetate (5.01 g, 30 mmol), and potassium carbonate (5.52 g, 40 mmol) were added to N,N-dimethylformamide (25 mL), and the resulting solution was then heated to 160 °C to react for 3 h. After the reaction was found to be completed from the monitoring of the TLC, the reaction system was cooled to room temperature. The solid was filtered out with suction under reduced pressure, and the filter cake was washed three times with ethyl acetate (25 mL). Filtrates were combined, the solvent was distilled off under reduced pressure, and the obtained product was dissolved in water (100 mL) and then extracted three times with ether (100 mL). The organic phase was washed with saturated aqueous NaCl solution and then dried with anhydrous sodium sulfate. The crude product was separated by column chromatography (PE/EA = 15/1 ~ 10/1) to obtain 1.54 g of white solid, with a yield of 38%.

### Example 2

### ethyl 3-methyl-5-(chlorosulfonyl)benzofuran-2-carboxylate (M2)

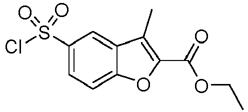

Ethyl 3-methylbenzofuran-2-carboxylate (3.06 g, 15 mmol) was added to chloroform (45 mL) and the resulting solution was stirred at 0 °C for 10 min. The chloroform (45 mL) solution of chlorosulfonic acid (8.70 g, 75 mmol) was then added dropwise, and the mixed solution was stirred at room temperature for 4 h. After the reaction was found to be completed from the monitoring of the TLC, the reaction solution was poured into ice water and then extracted three times with dichloromethane (150 mL). The organic phase was combined and then washed with saturated aqueous NaCl solution and then dried with anhydrous sodium sulfate. The crude product was recrystallized with petroleum ether/ethyl acetate to obtain 2.07 g of white solid, with a yield of 46%.

### Example 3

### ethyl 3-methyl-5-(N-phenethylsulfamoyl)benzofuran-2-carboxylate (M3)

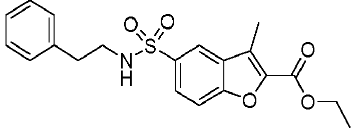

Ethyl 5-(chlorosulfonyl)-3-methylbenzofuran-2-carboxylate (2.07 g, 6.86 mmol) and potassium carbonate (1.89 g, 13.72 mmol) were dissolved in anhydrous dichloromethane (80 mL), anhydrous dichloromethane (20 mL) solution of phenethylamine (0.97 g, 8.23 mmol) was then added dropwise at room temperature, and the mixed solution was stirred for 3 h at room temperature. After the reaction was found to be completed from the monitoring of the TLC, the solvent was distilled off under reduced pressure, and the obtained product was dissolved in water (100 mL) and then extracted three times with ethyl acetate (100 mL). The organic phase was washed with saturated aqueous NaCl solution and then dried with anhydrous sodium sulfate. The crude product was separated by column chromatography (PE/EA = 10/1 ~ 5/1) to obtain 2.33 g of white solid, with a yield of 88%. ¹H NMR (400 MHz, Chloroform-*d*) δ 8.15 (d, *J* = 1.8 Hz, 1H), 7.84 (dd, *J* = 8.8, 1.9 Hz, 1H), 7.59 (d, *J* = 8.8 Hz, 1H), 7.25 - 7.13 (m, 3H), 7.10 - 6.99 (m, 2H), 4.77 (t, *J* = 6.2 Hz, 1H), 4.47 (q, *J* = 7.1 Hz, 2H), 3.24 (q, *J* = 6.9 Hz, 2H), 2.77 (t, *J* = 7.0 Hz, 2H), 2.59 (s, 3H), 1.45 (t, *J =* 7.1 Hz, 3H). ¹³C NMR (101 MHz, Chloroform-*d*) δ 159.9, 155.8, 143.0, 137.6, 135.2, 129.4, 128.7, 126.8, 126.2, 125.6, 121.6, 113.0, 61.6, 44.3, 35.8, 14.4, 9.4. HRMS (ESI) [M + H] ⁺, theoretically calculated for C₂₀H₂₂NO₅S: 388.1219, found 388.1213.

### Example 5

### ethyl 3-methyl-5-(N-benzyl-N-phenethylsulfamoyl)benzofuran-2-carboxylate (F27-S8)

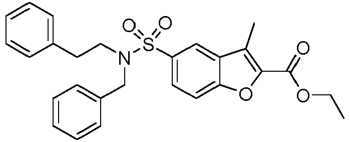

Ethyl 3-methyl-5-(*N*-phenethylsulfamoyl)benzofuran-2-carboxylate (195 mg, 0.5 mmol), benzyl bromide (171 mg, 1.0 mmol) and potassium carbonate (276 mg, 2.0 mmol) were dissolved in anhydrous dichloromethane (10 mL), and the mixed solution was stirred at 60 °C overnight. After the reaction was found to be completed from the monitoring of the TLC, the solvent was distilled off under reduced pressure, and the obtained product was dissolved in water (20 mL) and then extracted three times with dichloromethane (20 mL). The organic phase was washed with saturated aqueous NaCl solution and then dried with anhydrous sodium sulfate. The crude product was separated by column chromatography (PE/EA = 20/1 ~ 2/1) to obtain 229 mg of white solid, with a yield of 96%. ¹H NMR (400 MHz, Chloroform-*d*) δ 8.14 (d, *J =* 1.5 Hz, 1H), 7.87 (dd, *J =* 8.8, 1.7 Hz, 1H), 7.63 (d, *J* = 8.8 Hz, 1H), 7.39 - 7.23 (m, 5H), 7.22 - 7.09 (m, 3H), 6.95 (d, *J* = 6.8 Hz, 2H), 4.48 (q, *J* = 7.1 Hz, 2H), 4.40 (s, 2H), 3.45 - 3.28 (m, 2H), 2.71 - 2.62 (m, 2H), 2.60 (s, 3H), 1.46 (t, *J* = 7.1 Hz, 3H). ¹³C NMR (101 MHz, Chloroform-*d*) δ 159.9, 155.8, 143.0, 138.3, 136.0, 135.6, 129.4, 128.7, 128.7, 128.5, 128.5, 128.0, 126.5, 126.2, 125.6, 121.5, 113.1, 61.6, 52.2, 49.5, 35.3, 14.4, 9.4. HRMS (ESI) [M + H] ⁺, theoretically calculated for C₂₇H₂₈NO₅S: 478.1688, found 478.1680.

### Example 6

### ethyl 3-methyl-5-(N-(2-fluorobenzyl)-N-phenethylsulfamoyl)benzofuran-2-carboxylate (M4-14)

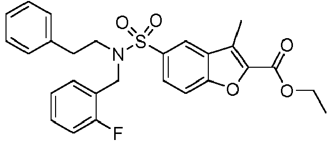

Ethyl 5-(N-benzyl-jV-phenethylsulfamoyl)-3-methylbenzofuran-2-carboxylate (F27-S8), this example was implemented under the same condition except that 2-fluorobenzyl bromide was used instead of benzyl bromide. 219 mg of white solid was obtained, with a yield of 88%. ¹H NMR (400 MHz, Chloroform-*d*) δ 8.10 (s, 1H), 7.84 (d, *J* = 8.8 Hz, 1H), 7.60 (d, *J* = 8.8 Hz, 1H), 7.44 (t, *J* = 7.0 Hz, 1H), 7.19 (ddp, *J* = 28.7, 21.6, 7.4 Hz, 5H), 7.00 (dd, *J* = 12.4, 8.1 Hz, 3H), 4.50 (s, 2H), 4.49 - 4.42 (m, 2H), 3.44 - 3.36 (m, 2H), 2.79 - 2.68 (m, 2H), 2.59 (s, 3H), 1.45 (t, *J* = 7.1 Hz, 3H). ¹³C NMR (101 MHz, Chloroform-*d*) δ 160.9 (d, *J* = 247.5 Hz, 1C), 159.9, 155.8, 142.9, 138.1, 135.4, 131.2 (d, *J* = 3.7 Hz, 1C), 129.8 (d, *J* = 8.2 Hz, 1C), 129.4, 128.7, 128.5, 126.5, 126.2, 125.6, 124.5 (d, *J* = 3.6 Hz, 1C), 123.2 (d, *J* = 14.2 Hz, 1C), 121.5, 115.4 (d, *J* = 21.8 Hz, 1C), 113.0, 61.6, 50.0, 45.1 (d, *J* = 3.8 Hz, 1C), 35.2, 14.4, 9.4. ¹⁹F NMR (376 MHz, Chloroform-*d*) δ -118.62. HRMS (ESI) [M + H] ⁺, theoretically calculated for C₂₇H₂₇NO₅FS: 496.1594, found 496.1595.

### Example 7

### ethyl 3-methyl-5-(N-(2-chlorobenzyl)-N-phenethylsulfamoyl)benzofuran-2-carboxylate (M4-15)

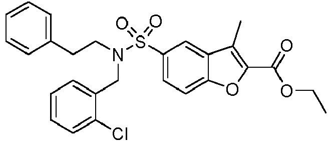

ethyl 5-(N-benzyl-*N-*phenethylsulfamoyl)-3-methylbenzofuran-2-carboxylate (F27-S8), this example was implemented under the same condition except that 2-chlorobenzyl bromide was used instead of benzyl bromide. 204 mg of white solid was obtained, with a yield of 80%. ¹H NMR (400 MHz, Chloroform-*d*) δ 8.14 (s, 1H), 7.88 (d, *J* = 8.8 Hz, 1H), 7.63 (d, *J=* 8.8 Hz, 1H), 7.58 - 7.46 (m, 1H), 7.32 (dd, *J* = 7.3, 1.6 Hz, 1H), 7.28 - 7.09 (m, 5H), 7.01 (d, *J* = 7.0 Hz, 2H), 4.57 (s, 2H), 4.47 (q, *J* = 7.1 Hz, 2H), 3.49 - 3.36 (m, 2H), 2.75 - 2.65 (m, 2H), 2.61 (s, 3H), 1.45 (t, *J* = 7.1 Hz, 3H). ¹³C NMR (101 MHz, Chloroform-*d*) δ 159.9, 155.8, 143.0, 138.1, 135.3, 133.9, 133.3, 130.5, 129.5, 129.4, 129.1, 128.7, 128.5, 127.2, 126.5, 126.2, 125.6, 121.6, 113.1, 61.6, 50.5, 49.3, 35.3, 14.4, 9.4. HRMS (ESI) [M + H] ⁺, theoretically calculated for C₂₇H₂₇NO₅SCl: 512.1298, found 512.1292.

### Example 8

### ethyl 3-methyl-5-(N-(2-bromobenzyl)-N-phenethylsulfamoyl)benzofuran-2-carboxylate (M4-16)

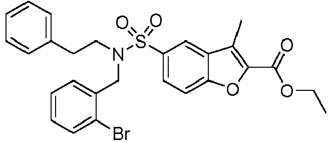

Ethyl 5-(N-benzyl-*N*-phenethylsulfamoyl)-3-methylbenzofuran-2-carboxylate (F27-S8), this example was implemented under the same condition except that 2-bromobenzyl bromide was used instead of benzyl bromide. 267 mg of white solid was obtained, with a yield of 96%. ¹H NMR (400 MHz, Chloroform-*d*) δ 8.15 (s, 1H), 7.88 (d, *J* = 8.8 Hz, 1H), 7.63 (d, *J* = 8.8 Hz, 1H), 7.52 (t, *J* = 7.5 Hz, 2H), 7.29 (t, *J* = 7.6 Hz, 1H), 7.23 - 7.10 (m, 4H), 7.01 (d, *J* = 7.0 Hz, 2H), 4.56 (s, 2H), 4.47 (q, *J* = 7.1 Hz, 2H), 3.52 - 3.36 (m, 2H), 2.76 - 2.65 (m, 2H), 2.61 (s, 3H), 1.46 (t, *J* = 7.1 Hz, 3H). ¹³C NMR (101 MHz, Chloroform-*d*) δ 159.9, 155.8, 143.0, 138.0, 135.5, 135.3, 132.8, 130.5, 129.4, 129.4, 128.7, 128.5, 127.8, 126.5, 126.2, 125.6, 123.3, 121.6, 113.1, 61.6, 51.8, 50.5, 35.2, 14.4, 9.4. HRMS (ESI) [M + H] ⁺, theoretically calculated for C₂₇H₂₇NO₅SBr: 556.0793, found 556.0785.

### Example 9

### ethyl 3-methyl-5-(N-(2-methylbenzyl)-N-phenethylsulfamoyl)benzofuran-2-carboxylate (M4-17)

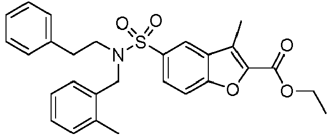

Ethyl 5-(N-benzyl-*N*-phenethylsulfamoyl)-3-methylbenzofuran-2-carboxylate (F27-S8), this example was implemented under the same condition except that 2-methylbenzyl bromide was used instead of benzyl bromide. 208 mg of white solid was obtained, with a yield of 85%. ¹H NMR (400 MHz, Chloroform-*d*) δ 8.17 (s, 1H), 7.92 (d, *J* = 8.8 Hz, 1H), 7.65 (d, *J* = 8.7 Hz, 1H), 7.18 (tt, *J* = 13.3, 7.4 Hz, 7H), 6.88 (d, *J* = 6.8 Hz, 2H), 4.47 (q, *J* = 7.1 Hz, 2H), 4.38 (s, 2H), 3.31 - 3.19 (m, 2H), 2.61 (s, 3H), 2.57 - 2.49 (m, 2H), 2.36 (s, 3H), 1.46 (t, *J* = 7.1 Hz, 3H). ¹³C NMR (101 MHz, Chloroform-*d*) δ 159.9, 155.8, 143.0, 138.3, 137.6, 134.8, 133.3, 130.8, 129.7, 129.5, 128.6, 128.5, 128.3, 126.4, 126.4, 126.0, 125.6, 121.6, 113.1, 61.6, 51.2, 49.8, 35.7, 19.2, 14.4, 9.4. HRMS (ESI) [M + H] ⁺, theoretically calculated for C₂₈H₃₀NO₅S: 492.1845, found 492.1835.

### Example 10

### ethyl 3-methyl-5-(N-(2-trifluoromethylbenzyl)-N-phenethylsulfamoyl)benzofuran-2-carboxylate (M4-18)

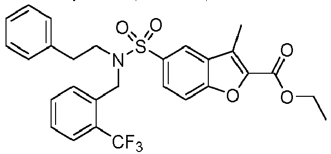

Ethyl 5-(N-benzyl-*N-*phenethylsulfamoyl)-3-methylbenzofuran-2-carboxylate (F27-S8), this example was implemented under the same condition except that 2-trifluoromethyl benzyl bromide was used instead of benzyl bromide. 238 mg of white solid was obtained, with a yield of 87%. ¹H NMR (400 MHz, Chloroform-*d*) δ 8.20 (s, 1H), 7.91 (d, *J* = 8.8 Hz, 1H), 7.81 (d, *J* = 7.9 Hz, 1H), 7.65 (t, *J* = 8.1 Hz, 2H), 7.54 (t, *J* = 7.6 Hz, 1H), 7.38 (t, *J* = 7.6 Hz, 1H), 7.15 (dq, *J* = 14.1, 6.9 Hz, 3H), 6.97 (d, *J* = 7.1 Hz, 2H), 4.65 (s, 2H), 4.48 (q, *J* = 7.1 Hz, 2H), 3.51 - 3.37 (m, 2H), 2.67 - 2.59 (m, 5H), 1.45 (t, *J* = 7.1 Hz, 3H). ¹³C NMR (101 MHz, Chloroform-*d)* δ 159.9, 155.9, 143.1, 137.8, 135.7 (d, *J* = 1.3 Hz, 1C), 135.0, 132.3, 129.7, 129.6, 128.6, 128.5, 127.6 (d, *J* = 30.5 Hz, 1C), 127.6, 126.6, 126.1, 125.8 (t, *J* = 6.0 Hz, 1C), 125.6, 121.6, 113.3, 61.6, 50.7, 48.3 (d, *J* = 2.2 Hz, 1C), 34.9, 14.3, 9.4. ¹⁹F NMR (376 MHz, Chloroform-*d*) δ -59.19. HRMS (ESI) [M + H] ⁺, theoretically calculated for C₂₈H₂₇NO₅F₃S: 546.1562, found 546.1562.

### Example 11

### ethyl 3-methyl-5-(N-(2-nitrobenzyl)-N-phenethylsulfamoyl)benzofuran-2-carboxylate (M4-19)

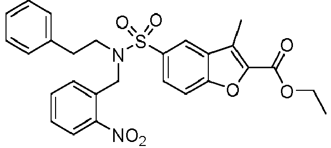

Ethyl 5-(N-benzyl-*N*-phenethylsulfamoyl)-3-methylbenzofuran-2-carboxylate (F27-S8), this example was implemented under the same condition except that 2-nitrobenzyl bromide was used instead of benzyl bromide. 238 mg of white solid was obtained, with a yield of 91%. ¹H NMR (400 MHz, Chloroform-*d*) δ 8.16 (s, 1H), 7.98 (d, *J* = 8.1 Hz, 1H), 7.89 (d, *J* = 8.8 Hz, 1H), 7.84 (d, *J* = 7.9 Hz, 1H), 7.64 (dd, *J* = 19.1, 8.2 Hz, 2H), 7.43 (t, *J* = 7.8 Hz, 1H), 7.18 (q, *J* = 9.4, 8.3 Hz, 3H), 6.99 (d, *J* = 7.3 Hz, 2H), 4.79 (s, 2H), 4.48 (q, *J* = 7.1 Hz, 2H), 3.57 - 3.42 (m, 2H), 2.75 - 2.65 (m, 2H), 2.62 (s, 3H), 1.46 (t, *J* = 7.1 Hz, 3H). ¹³C NMR (101 MHz, Chloroform-*d*) δ 159.8, 155.9, 148.2, 143.1, 137.7, 134.8, 133.6, 132.9, 130.2, 129.6, 128.5, 128.4, 126.6, 126.2, 125.5, 124.8, 121.6, 113.3, 61.6, 51.1, 49.3, 34.9, 14.3, 9.4. HRMS (ESI) [M + H] ⁺, theoretically calculated for C₂₇H₂₇N₂O₇S: 523.1539, found 523.1534.

### Example 12

### ethyl 3-methyl-5-(N-(3-fluorobenzyl)-N-phenethylsulfamoyl)benzofuran-2-carboxylate (M4-20)

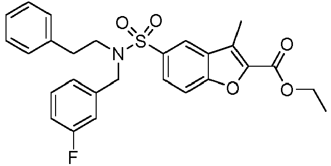

Ethyl 5-(N-benzyl-*N*-phenethylsulfamoyl)-3-methylbenzofuran-2-carboxylate (F27-S8), this example was implemented under the same condition except that 3-fluorobenzyl bromide was used instead of benzyl bromide. 257 mg of white solid was obtained, with a yield of 99%. ¹H NMR (400 MHz, Chloroform-*d*) δ 8.15 (s, 1H), 7.87 (d, *J* = 8.8 Hz, 1H), 7.63 (d, *J* = 8.8 Hz, 1H), 7.27 (q, *J* = 7.7 Hz, 1H), 7.18 (dt, *J* = 11.6, 6.8 Hz, 3H), 7.04 (d, *J* = 7.7 Hz, 1H), 6.97 (t, *J* = 8.1 Hz, 4H), 4.47 (q, *J* = 7.1 Hz, 2H), 4.38 (s, 2H), 3.46 - 3.30 (m, 2H), 2.74 - 2.64 (m, 2H), 2.61 (s, 3H), 1.46 (t, *J* = 7.1 Hz, 3H). ¹³C NMR (101 MHz, Chloroform-*d*) δ 163.0 (d, *J* = 247.9 Hz, 1C), 159.9, 155.8, 143.0, 138.8 (d, *J* = 7.1 Hz, 1C), 138.1, 135.4, 130.2 (d, *J* = 8.3 Hz, 1C), 129.5, 128.6, 128.5, 126.6, 126.2, 125.5, 123.8 (d, *J* = 2.9 Hz, 1C), 121.5, 115.2 (d, *J* = 22.0 Hz, 1C), 114.9 (d, *J* = 21.2 Hz, 1C), 113.1, 61.6, 51.7 (d, *J* = 1.6 Hz, 1C), 49.7, 35.2, 14.4, 9.4. ¹⁹F NMR (376 MHz, Chloroform-*d*) δ -112.36. HRMS (ESI) [M + H] ⁺, theoretically calculated for C₂₇H₂₇NO₅FS: 496.1594, found 496.1593.

### Example 13

### ethyl 3-methyl-5-(N-(3-chlorobenzyl)-N-phenethylsulfamoyl)benzofuran-2-carboxylate (M4-21)

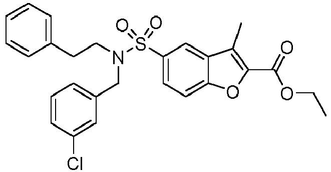

Ethyl 5-(N-benzyl-*N*-phenethylsulfamoyl)-3-methylbenzofuran-2-carboxylate (F27-S8), this example was implemented under the same condition except that 3-chlorobenzyl bromide was used instead of benzyl bromide. 230 mg of white solid was obtained, with a yield of 90%. ¹H NMR (400 MHz, Chloroform-*d*) δ 8.13 (s, 1H), 7.86 (d, *J* = 8.8 Hz, 1H), 7.61 (d, *J* = 8.8 Hz, 1H), 7.25 - 7.08 (m, 7H), 6.96 (d, *J* = 7.1 Hz, 2H), 4.46 (q, *J* = 7.1 Hz, 2H), 4.36 (s, 2H), 3.46 - 3.31 (m, 2H), 2.72 - 2.63 (m, 2H), 2.59 (s, 3H), 1.45 (t, *J* = 7.1 Hz, 3H). ¹³C NMR (101 MHz, Chloroform-*d*) δ 159.8, 155.8, 143.0, 138.4, 138.1, 135.3, 134.5, 130.0, 129.5, 128.6, 128.5, 128.3, 128.1, 126.6, 126.4, 126.2, 125.5, 121.5, 113.1, 61.6, 51.6, 49.7, 35.2, 14.4, 9.4. HRMS (ESI) [M + H] ⁺, theoretically calculated for C₂₇H₂₇NO₅SCl: 512.1298, found 512.1298.

### Example 14

### ethyl 3-methyl-5-(N-(3-bromobenzyl)-N-phenethylsulfamoyl)benzofuran-2-carboxylate (M4-22)

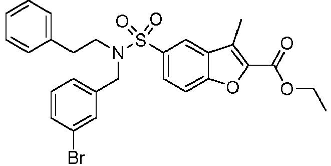

Ethyl 5-(N-benzyl-*N*-phenethylsulfamoyl)-3-methylbenzofuran-2-carboxylate (F27-S8), this example was implemented under the same condition except that 3-bromobenzyl bromide was used instead of benzyl bromide. 265 mg of white solid was obtained, with a yield of 92%. ¹H NMR (400 MHz, Chloroform-*d*) δ 8.12 (s, 1H), 7.85 (d, *J* = 8.7 Hz, 1H), 7.63 (d, *J* = 8.7 Hz, 1H), 7.39 (d, *J* = 7.0 Hz, 1H), 7.31 (s, 1H), 7.18 (dq, *J* = 14.9, 7.1 Hz, 5H), 6.98 (d, *J* = 7.0 Hz, 2H), 4.48 (q, *J* = 7.1 Hz, 2H), 4.35 (s, 2H), 3.48 - 3.26 (m, 2H), 2.74 - 2.64 (m, 2H), 2.60 (s, 3H), 1.46 (t, *J* = 7.1 Hz, 3H). ¹³C NMR (101 MHz, Chloroform-*d*) δ 159.9, 155.8, 143.0, 138.5, 138.1, 135.4, 131.3, 131.1, 130.2, 129.5, 128.7, 128.6, 126.9, 126.6, 126.2, 125.6, 122.7, 121.5, 113.2, 61.6, 51.5, 49.7, 35.3, 14.4, 9.4. HRMS (ESI) [M + H] ⁺, theoretically calculated for C₂₇H₂₇NO₅SBr: 556.0793, found 556.0792.

### Example 15

### ethyl 3-methyl-5-(N-(3-methylbenzyl)-N-phenethylsulfamoyl)benzofuran-2-carboxylate (M4-23)

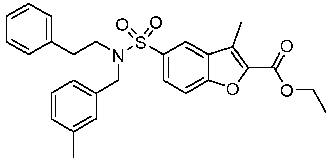

Ethyl 5-(N-benzyl-jV-phenethylsulfamoyl)-3-methylbenzofuran-2-carboxylate (F27-S8), this example was implemented under the same condition except that 3-methylbenzyl bromide was used instead of benzyl bromide. 243 mg of white solid was obtained, with a yield of 99%. ¹H NMR (400 MHz, Chloroform-*d*) δ 8.14 (s, 1H), 7.87 (d, *J* = 8.8 Hz, 1H), 7.63 (d, *J* = 8.8 Hz, 1H), 7.18 (ddd, *J* = 13.0, 8.4, 5.8 Hz, 4H), 7.08 (d, *J* = 7.6 Hz, 1H), 7.03 (s, 2H), 6.96 (d, *J* = 6.9 Hz, 2H), 4.48 (q, *J* = 7.1 Hz, 2H), 4.36 (s, 2H), 3.43 - 3.30 (m, 2H), 2.72 - 2.63 (m, 2H), 2.60 (s, 3H), 2.28 (s, 3H), 1.46 (t*, J* = 7.1 Hz, 3H). ¹³C NMR (101 MHz, Chloroform-*d*) δ 159.9, 155.8, 142.9, 138.4, 135.8, 135.7, 129.4, 129.2, 128.7, 128.7, 128.5, 128.5, 126.5, 126.3, 125.6, 125.5, 121.5, 113.0, 61.6, 52.1, 49.4, 35.2, 21.3, 14.4, 9.4. HRMS (ESI) [M + H] ⁺, theoretically calculated for C₂₈H₃₀NO₅S: 492.1845, found 492.1844.

### Example 16

### ethyl 3-methyl-5-(N-(3-trifluoromethylbenzyl)-N-phenethylsulfamoyl)benzofuran-2-carboxylate (M4-24)

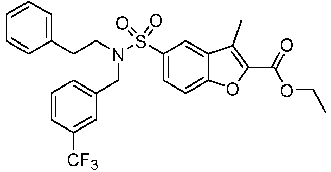

Ethyl 5-(N-benzyl-*N-*phenethylsulfamoyl)-3-methylbenzofuran-2-carboxylate (F27-S8), this example was implemented under the same condition except that 3-trifluoromethyl benzyl bromide was used instead of benzyl bromide. 248 mg of white solid was obtained, with a yield of 90%. ¹H NMR (400 MHz, Chloroform-*d*) δ 8.16 (s, 1H), 7.88 (d, *J* = 8.8 Hz, 1H), 7.63 (d, *J* = 10.2 Hz, 1H), 7.51 (dd, *J* = 13.2, 7.7 Hz, 2H), 7.44 (d, *J* = 7.4 Hz, 2H), 7.17 (q, *J* = 8.5, 7.5 Hz, 3H), 6.96 (d, *J* = 7.4 Hz, 2H), 4.54 - 4.40 (m, 4H), 3.53 - 3.34 (m, 2H), 2.69 (t, *J* = 7.6 Hz, 2H), 2.60 (s, 3H), 1.46 (t, *J=* 6.3 Hz, 3H). ¹³C NMR (101 MHz, Chloroform-*d*) δ 159.8, 155.8, 143.1, 138.0, 137.4, 135.3, 131.7, 130.9 (d, *J* = 32.0 Hz, 1C), 129.5, 129.2, 128.6, 128.5, 126.6, 126.2, 125.5, 124.9 (t, *J* = 3.8 Hz, 1C), 124.7 (d, *J* = 3.7 Hz, 1C), 123.9 (d, *J* = 273.3 Hz, 1C), 121.5, 113.1, 61.6, 51.7, 49.8, 35.2, 14.3, 9.3. ¹⁹F NMR (376 MHz, Chloroform-*d*) δ -62.67. HRMS (ESI) [M + H] ⁺, theoretically calculated for C₂₈H₂₇NO₅F₃S: 546.1562, found 546.1556.

### Example 17

### ethyl 3-methyl-5-(N-(3-nitrobenzyl)-N-phenethylsulfamoyl)benzofuran-2-carboxylate (M4-25)

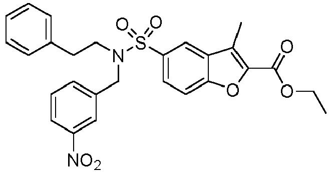

Ethyl 5-(N-benzyl-*N*-phenethylsulfamoyl)-3-methylbenzofuran-2-carboxylate (F27-S8), this example was implemented under the same condition except that 3-nitrobenzyl bromide was used instead of benzyl bromide. 259 mg of white solid was obtained, with a yield of 99%. ¹H NMR (400 MHz, Chloroform-*d*) δ 8.19 (s, 1H), 8.05 (d, *J* = 8.7 Hz, 2H), 7.90 (d, *J* = 8.7 Hz, 1H), 7.64 (dd, *J* = 15.4, 8.2 Hz, 2H), 7.46 (t, *J* = 7.8 Hz, 1H), 7.12 (q, *J* = 10.2, 8.3 Hz, 3H), 6.95 (d, *J* = 6.9 Hz, 2H), 4.46 (dd, *J* = 15.3, 8.2 Hz, 4H), 3.55 - 3.39 (m, 2H), 2.81 - 2.67 (m, 2H), 2.60 (s, 3H), 1.46 (t, *J* = 7.1 Hz, 3H). ¹³C NMR (101 MHz, Chloroform-*d*) δ 159.7, 155.8, 148.2, 143.0, 138.9, 137.9, 134.8, 134.2, 129.6, 129.5, 128.5, 128.5, 126.5, 126.2, 125.4, 122.8, 122.7, 121.6, 113.1, 61.5, 51.5, 50.0, 35.1, 14.3, 9.3. HRMS (ESI) [M + H] ⁺, theoretically calculated for C₂₇H₂₇N₂O₇S: 523.1539, found 523.1531.

### Example 18

### ethyl 3-methyl-5-(N-([1,1'-biphenyl]-3-ylmethyl)-N-phenethylsulfamoyl)benzofuran-2-carboxylate(M4-26)

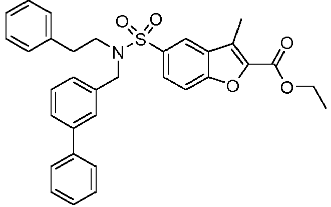

Ethyl 5-(N-benzyl-*N*-phenethylsulfamoyl)-3-methylbenzofuran-2-carboxylate (F27-S8), this example was implemented under the same condition except that 3-phenylbenzyl bromide was used instead of benzyl bromide. 211 mg of white solid was obtained, with a yield of 76%. ¹H NMR (400 MHz, Chloroform-*d*) δ 8.20 (s, 1H), 7.93 (d, *J* = 10.6 Hz, 1H), 7.61 (d, *J* = 8.8 Hz, 1H), 7.52 (d, *J* = 7.8 Hz, 1H), 7.47 (d, *J=* 7.1 Hz, 2H), 7.44 - 7.37 (m, 4H), 7.34 (t, *J* = 7.2 Hz, 1H), 7.28 (d, *J* = 7.6 Hz, 1H), 7.18 (dd, *J=* 13.4, 7.2 Hz, 3H), 7.03 (d, *J* = 6.7 Hz, 2H), 4.53 (s, 2H), 4.49 (t, *J* = 7.1 Hz, 2H), 3.57 - 3.44 (m, 2H), 2.85 - 2.73 (m, 2H), 2.58 (s, 3H), 1.49 (t, *J* = 7.1 Hz, 3H). ¹³C NMR (101 MHz, Chloroform-*d*) δ 159.9, 155.8, 143.0, 141.5, 140.4, 138.4, 136.6, 135.8, 129.5, 129.2, 128.8, 128.7, 128.6, 127.5, 127.4, 127.0, 127.0, 126.7, 126.5, 126.3, 125.6, 121.5, 113.1, 61.6, 52.1, 49.5, 35.4, 14.4, 9.4. HRMS (ESI) [M + H] ⁺, theoretically calculated for C₃₃H₃₂NO₅S: 554.2001, found 554.2007.

### Example 19

### ethyl 3-methyl-5-(N-3-phenoxybenzyl)-N-phenethylsulfamoyl)benzofuran-2-carboxylate (M4-27)

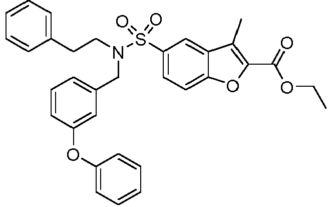

Ethyl 5-(N-benzyl-*N*-phenethylsulfamoyl)-3-methylbenzofuran-2-carboxylate (F27-S8), this example was implemented under the same condition except that 3-phenoxybenzyl bromide was used instead of benzyl bromide. 213 mg of white solid was obtained, with a yield of 75%. ¹H NMR (400 MHz, Chloroform-*d*) δ 8.14 (s, 1H), 7.84 (d, *J* = 8.8 Hz, 1H), 7.57 (d, *J* = 8.8 Hz, 1H), 7.27 (dt, *J* = 10.8, 7.7 Hz, 3H), 7.16 (dq, *J=* 14.3, 7.1 Hz, 3H), 7.10 - 7.00 (m, 2H), 6.97 (d, *J* = 6.7 Hz, 2H), 6.90 (dd, *J* = 18.7, 8.4 Hz, 4H), 4.45 (q, *J* = 7.1 Hz, 2H), 4.36 (s, 2H), 3.46 - 3.31 (m, 2H), 2.76 - 2.64 (m, 2H), 2.58 (s, 3H), 1.44 (t, *J* = 7.1 Hz, 3H). ¹³C NMR (101 MHz, Chloroform-*d*) δ 159.9, 157.6, 156.9, 155.8, 143.0, 138.3, 138.2, 135.5, 130.1, 129.8, 129.4, 128.7, 128.6, 126.6, 126.2, 125.6, 123.5, 123.3, 121.5, 118.8, 118.8, 118.3, 113.1, 61.6, 51.8, 49.5, 35.3, 14.4, 9.4. HRMS (ESI) [M + H] ⁺, theoretically calculated for C₃₃H₃₂NO₆S: 570.1950, found 570.1948.

### Example 20

### ethyl 3-methyl-5-(N-(4-fluorobenzyl)-N-phenethylsulfamoyl)benzofuran-2-carboxylate (M4-28)

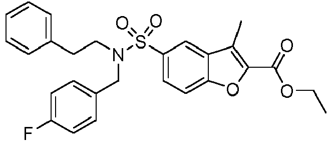

Ethyl 5-(N-benzyl-*N*-phenethylsulfamoyl)-3-methylbenzofuran-2-carboxylate (F27-S8), this example was implemented under the same condition except that 4-fluorobenzylbromide was used instead of benzyl bromide. 220 mg of white solid was obtained, with a yield of 89%. ¹H NMR (400 MHz, Chloroform-*d*) δ 8.14 (s, 1H), 7.86 (d, *J* = 8.8 Hz, 1H), 7.63 (d, *J* = 8.8 Hz, 1H), 7.25 (dd, *J* = 8.4, 5.4 Hz, 2H), 7.21 - 7.11 (m, 3H), 7.04 - 6.90 (m, 4H), 4.47 (q, *J* = 7.1 Hz, 2H), 4.35 (s, 2H), 3.43 - 3.29 (m, 2H), 2.63 (d, *J* = 16.1 Hz, 5H), 1.46 (t, *J* = 7.1 Hz, 3H). ¹³C NMR (101 MHz, Chloroform-*d*) δ 162.5 (d, *J* = 247.6 Hz, 1C), 159.9, 155.8, 143.0, 138.2, 135.4, 131.9 (d, *J* = 3.2 Hz, 1C), 130.1 (d, *J* = 8.2 Hz, 1C), 129.5, 128.6, 128.5, 126.5, 126.2, 125.5, 121.5, 115.6 (d, *J* = 21.6 Hz, 1C), 113.1, 61.6, 51.5, 49.5, 35.2, 14.4, 9.4. ¹⁹F NMR (376 MHz, Chloroform-*d*) δ -114.08. HRMS (ESI) [M + H] ⁺, theoretically calculated for C₂₇H₂₇NO₅FS: 496.1594, found 496.1593.

### Example 21

### ethyl 3-methyl-5-(N-(4-chlorobenzyl)-N-phenethylsulfamoyl)benzofuran-2-carboxylate (M4-29)

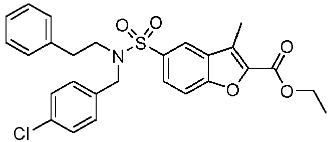

Ethyl 5-(N-benzyl-*N*-phenethylsulfamoyl)-3-methylbenzofuran-2-carboxylate (F27-S8), this example was implemented under the same condition except that 4-chlorobenzyl bromide was used instead of benzyl bromide. 222 mg of white solid was obtained, with a yield of 87%. ¹H NMR (400 MHz, Chloroform-*d*) δ 8.12 (s, 1H), 7.86 (d, *J* = 8.8 Hz, 1H), 7.63 (d, *J* = 8.8 Hz, 1H), 7.30 - 7.23 (m, 2H), 7.22 - 7.11 (m, 5H), 6.95 (d, *J* = 6.5 Hz, 2H), 4.48 (q, *J* = 7.1 Hz, 2H), 4.34 (s, 2H), 3.43 - 3.29 (m, 2H), 2.69 - 2.62 (m, 2H), 2.60 (s, 3H), 1.46 (t, *J* = 7.1 Hz, 3H). ¹³C NMR (101 MHz, Chloroform-*d*) δ 159.9, 155.8, 143.0, 138.1, 135.4, 134.7, 133.8, 129.7, 129.5, 128.8, 128.6, 128.6, 126.6, 126.2, 125.5, 121.5, 113.1, 61.6, 51.6, 49.6, 35.2, 14.4, 9.4. HRMS (ESI) [M + H] ⁺, theoretically calculated for C₂₇H₂₇NO₅SCl: 512.1298, found 512.1299.

### Example 22

### ethyl 3-methyl-5-(N-(4-bromobenzyl)-N-phenethylsulfamoyl)benzofuran-2-carboxylate (M4-30)

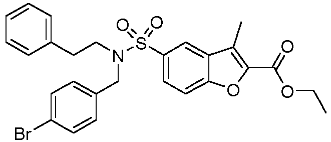

Ethyl 5-(N-benzyl-*N-*phenethylsulfamoyl)-3-methylbenzofuran-2-carboxylate (F27-S8), this example was implemented under the same condition except that 4-bromobenzyl bromide was used instead of benzyl bromide. 193 mg of white solid was obtained, with a yield of 70%. ¹H NMR (400 MHz, Chloroform-*d*) δ 8.14 (d, *J* = 1.8 Hz, 1H), 7.87 (dd, *J* = 8.8, 1.9 Hz, 1H), 7.64 (d, *J* = 8.7 Hz, 1H), 7.42 (d, *J* = 8.3 Hz, 2H), 7.17 (dd, *J* = 17.6, 7.7 Hz, 5H), 6.96 (d, *J* = 6.5 Hz, 2H), 4.49 (q, *J* = 7.1 Hz, 2H), 4.34 (s, 2H), 3.48 - 3.30 (m, 2H), 2.70 - 2.64 (m, 2H), 2.62 (s, 3H), 1.47 (t, *J* = 7.1 Hz, 3H). ¹³C NMR (101 MHz, Chloroform-*d*) δ 159.8, 155.8, 143.0, 138.1, 135.3, 135.2, 131.8, 130.0, 129.4, 128.6, 128.5, 126.6, 126.2, 125.5, 121.9, 121.5, 113.1, 61.6, 51.6, 49.6, 35.2, 14.4, 9.4.

### Example 23

### ethyl 3-methyl-5-(N-(4-methylbenzyl)-N-phenethylsulfamoyl)benzofuran-2-carboxylate (M4-31)

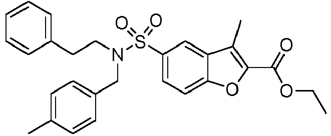

Ethyl 5-(N-benzyl-*N*-phenethylsulfamoyl)-3-methylbenzofuran-2-carboxylate (F27-S8), this example was implemented under the same condition except that 4-methylbenzyl bromide was used instead of benzyl bromide. 101 mg of white solid was obtained, with a yield of 69%. ¹H NMR (400 MHz, Chloroform-*d*) δ 8.14 (s, 1H), 7.88 (dd, *J* = 8.7, 1.5 Hz, 1H), 7.62 (d, *J* = 8.8 Hz, 1H), 7.24 - 7.07 (m, 7H), 6.97 (d, *J* = 7.0 Hz, 2H), 4.48 (q, *J* = 7.1 Hz, 2H), 4.37 (s, 2H), 3.42 - 3.31 (m, 2H), 2.73 - 2.64 (m, 2H), 2.60 (s, 3H), 2.33 (s, 3H), 1.46 (t, *J* = 7.1 Hz, 3H). ¹³C NMR (101 MHz, Chloroform-*d*) δ 159.9, 155.8, 142.9, 138.4, 137.7, 135.7, 132.9, 129.4, 129.4, 128.7, 128.5, 128.5, 126.5, 126.3, 125.6, 121.5, 113.0, 61.6, 51.8, 49.3, 35.2, 21.1, 14.4, 9.4. HRMS (ESI) [M + H] ⁺, theoretically calculated for C₂₈H₃₀NO₅S: 492.1845, found 492.1837.

### Example 24

### ethyl 3-methyl-5-(N-(4-trifluoromethylbenzyl)-N-phenethylsulfamoyl)benzofuran-2-carboxylate (M4-32)

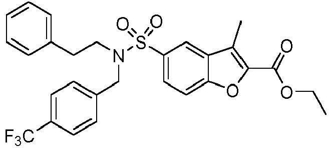

Ethyl 5-(N-benzyl-*N-*phenethylsulfamoyl)-3-methylbenzofuran-2-carboxylate (F27-S8), this example was implemented under the same condition except that 4-trifluoromethyl benzyl bromide was used instead of benzyl bromide. 132 mg of white solid was obtained, with a yield of 80%. ¹H NMR (400 MHz, Chloroform-*d*) δ 8.19 - 8.12 (m, 1H), 7.87 (dd, *J* = 8.8, 1.6 Hz, 1H), 7.64 (d, *J* = 8.8 Hz, 1H), 7.55 (d, *J* = 8.1 Hz, 2H), 7.38 (d, *J* = 8.0 Hz, 2H), 7.16 (t, *J* = 7.7 Hz, 3H), 6.94 (d, *J* = 6.5 Hz, 2H), 4.48 (q, *J* = 7.1 Hz, 2H), 4.43 (s, 2H), 3.48 - 3.32 (m, 2H), 2.74 - 2.64 (m, 2H), 2.60 (s, 3H), 1.46 (t, *J* = 7.1 Hz, 3H). ¹³C NMR (101 MHz, Chloroform-*d*) δ 159.8, 155.8, 143.1, 140.4, 138.0, 135.2, 129.5, 128.6, 128.6, 128.5, 126.6, 126.1, 125.6 (d, *J* = 3.7 Hz, 1C), 125.5, 121.6, 113.2, 61.6, 51.8, 49.9, 35.1, 14.4, 9.4. ¹⁹F NMR (376 MHz, Chloroform-*d*) δ -62.51. HRMS (ESI) [M + H] ⁺, theoretically calculated for C₂₈H₂₇NO₅F₃S: 546.1562, found 546.1567.

### Example 25

### ethyl 3-methyl-5-(N-(4-cyanobenzyl)-N-phenethylsulfamoyl)benzofuran-2-carboxylate (M4-33)

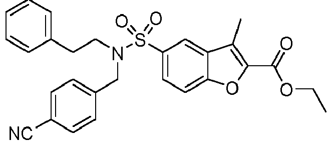

Ethyl 5-(N-benzyl-*N*-phenethylsulfamoyl)-3-methylbenzofuran-2-carboxylate (F27-S8), this example was implemented under the same condition except that 4-cyanobenzyl bromide was used instead of benzyl bromide. 189 mg of white solid was obtained, with a yield of 75%. ¹H NMR (400 MHz, Chloroform-*d*) δ 8.15 (d, *J* = 1.9 Hz, 1H), 7.86 (dd, *J* = 8.8, 1.9 Hz, 1H), 7.63 (dd, *J* = 22.4, 8.5 Hz, 3H), 7.39 (d, *J* = 8.0 Hz, 2H), 7.18 (q, *J* = 5.7 Hz, 3H), 6.98 - 6.89 (m, 2H), 4.48 (q, *J* = 7.1 Hz, 2H), 4.41 (s, 2H), 3.44 - 3.33 (m, 2H), 2.63 (d, *J* = 14.3 Hz, 5H), 1.46 (t, *J* = 7.1 Hz, 3H). ¹³C NMR (101 MHz, Chloroform-*d*) δ 159.8, 155.9, 143.2, 141.9, 137.8, 135.0, 132.5, 129.6, 128.7, 128.6, 128.6, 126.7, 126.1, 125.4, 121.6, 118.5, 113.3, 111.9, 61.7, 52.0, 50.0, 35.1, 14.4, 9.4.

### Example 26

### ethyl 3-methyl-5-(N-(4-(trifluoromethoxy)benzyl)-N-phenethylsulfamoyl)benzofuran-2-carboxylate (M4-34)

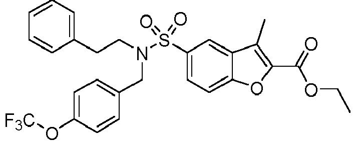

Ethyl 5-(N-benzyl-*N-*phenethylsulfamoyl)-3-methylbenzofuran-2-carboxylate (F27-S8), this example was implemented under the same condition except that 4-(trifluoromethoxy) benzyl bromide was used instead of benzyl bromide. 289 mg of white solid was obtained, with a yield of 99%. ¹H NMR (400 MHz, Chloroform-*d*) δ 8.17 (d, *J* = 1.9 Hz, 1H), 7.87 (dd, *J* = 8.8, 1.9 Hz, 1H), 7.64 (d, *J* = 8.8 Hz, 1H), 7.31 (d, *J* = 8.6 Hz, 2H), 7.18 (q, *J* = 8.9, 7.7 Hz, 5H), 6.95 (d, *J* = 6.6 Hz, 2H), 4.49 (q, *J* = 7.1 Hz, 2H), 4.40 (s, 2H), 3.47 - 3.33 (m, 2H), 2.72 - 2.65 (m, 2H), 2.62 (s, 3H), 1.47 (t, *J* = 7.1 Hz, 3H). ¹³C NMR (101 MHz, Chloroform-*d*) δ 159.8, 155.8, 148.8, 143.0, 138.1, 135.3, 135.0, 129.7, 129.5, 128.6, 128.5, 126.5, 126.1, 125.5, 121.5, 121.1, 113.1, 61.6, 51.5, 49.6, 35.2, 14.3, 9.3.

### Example 27

### ethyl 3-methyl-5-(N-(4-(trifluoromethylthio)benzyl)-N-phenethylsulfamoyl)benzofuran-2-carboxylate (M4-35)

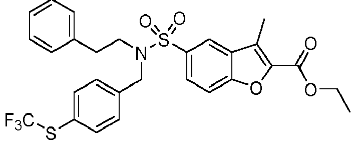

Ethyl 5-(N-benzyl-*N*-phenethylsulfamoyl)-3-methylbenzofuran-2-carboxylate (F27-S8), this example was implemented under the same condition except that 4-(trifluoromethylthio)benzyl bromide was used instead of benzyl bromide. 237 mg of white solid was obtained, with a yield of 82%. ¹H NMR (400 MHz, Chloroform-*d*) δ 8.15 (d, *J* = 1.8 Hz, 1H), 7.85 (dd, *J* = 8.7, 1.9 Hz, 1H), 7.59 (dd, *J* = 17.7, 8.4 Hz, 3H), 7.32 (d, *J* = 8.1 Hz, 2H), 7.22 - 7.08 (m, 3H), 6.92 (dd, *J=* 7.6, 1.5 Hz, 2H), 4.57 - 4.29 (m, 4H), 3.47 - 3.31 (m, 2H), 2.62 (d, *J=* 25.5 Hz, 5H), 1.44 (t, *J* = 7.1 Hz, 3H). ¹³C NMR (101 MHz, Chloroform-*d*) δ 159.8, 155.8, 143.1, 139.7, 138.0, 136.5, 135.2, 129.6 (d, *J* = 309.4 Hz, 1C), 129.5, 129.3, 128.6, 128.5, 126.6, 126.2, 125.5, 123.8 (d, *J* = 2.4 Hz, 1C), 121.6, 113.2, 61.6, 51.8, 49.9, 35.2, 14.3, 9.3. ¹⁹F NMR (376 MHz, Chloroform-*d*) δ -42.65.

### Example 28

### ethyl 3-methyl-5-(N-(4-isopropylbenzyl)-N-phenethylsulfamoyl)benzofuran-2-carboxylate (M4-36)

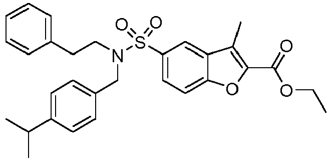

Ethyl 5-(N-benzyl-*N*-phenethylsulfamoyl)-3-methylbenzofuran-2-carboxylate (F27-S8), this example was implemented under the same condition except that 4-isopropyl benzyl bromide was used instead of benzyl bromide. 234 mg of white solid was obtained, with a yield of 90%. ¹H NMR (400 MHz, Chloroform-*d*) δ 8.17 (d, *J* = 1.9 Hz, 1H), 7.88 (dd, *J* = 8.8, 1.9 Hz, 1H), 7.62 (d, *J* = 8.8 Hz, 1H), 7.23 - 7.11 (m, 7H), 6.96 (d, *J* = 6.8 Hz, 2H), 4.48 (q, *J=* 7.1 Hz, 2H), 4.39 (s, 2H), 3.45 - 3.32 (m, 2H), 2.89 (hept, *J* = 6.8 Hz, 1H), 2.74 - 2.64 (m, 2H), 2.61 (s, 3H), 1.46 (t, *J* = 7.1 Hz, 3H), 1.24 (d, *J* = 6.9 Hz, 6H). ¹³C NMR (101 MHz, Chloroform-*d*) δ 159.9, 155.8, 148.7, 142.9, 138.4, 135.7, 133.2, 129.4, 128.7, 128.6, 128.5, 126.7, 126.5, 126.3, 125.6, 121.5, 113.0, 61.6, 51.8, 49.3, 35.3, 33.8, 24.0, 14.4, 9.4.

### Example 29

### ethyl 3-methyl-5-(N-(4-(tert-butyl)benzyl)-N-phenethylsulfamoyl)benzofuran-2-carboxylate (M4-37)

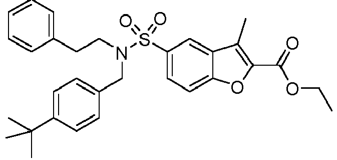

Ethyl 5-(N-benzyl-*N*-phenethylsulfamoyl)-3-methylbenzofuran-2-carboxylate (F27-S8), this example was implemented under the same condition except that 4-(tert-butyl)benzyl bromide was used instead of benzyl bromide. 236 mg of white solid was obtained, with a yield of 89%. ¹H NMR (400 MHz, Chloroform-*d*) δ 8.18 (d, *J* = 1.9 Hz, 1H), 7.88 (dd, *J* = 8.8, 1.9 Hz, 1H), 7.62 (d, *J* = 8.8 Hz, 1H), 7.33 (d, *J* = 8.4 Hz, 2H), 7.23 - 7.11 (m, 5H), 7.00 - 6.92 (m, 2H), 4.48 (q, *J* = 7.1 Hz, 2H), 4.40 (s, 2H), 3.50 - 3.26 (m, 2H), 2.75 - 2.65 (m, 2H), 2.61 (s, 3H), 1.46 (t,*J* = 7.1 Hz, 3H), 1.31 (s, 9H). ¹³C NMR (101 MHz, Chloroform-*d*) δ 159.9, 155.8, 151.0, 142.9, 138.4, 135.7, 132.9, 129.4, 128.7, 128.5, 128.3, 126.5, 126.3, 125.6, 125.6, 121.6, 113.0, 61.6, 51.7, 49.3, 35.3, 34.6, 31.4, 14.4, 9.4.

### Example 30

### ethyl 3-methyl-5-(N-(4-acetylbenzyl)-N-phenethylsulfamoyl)benzofuran-2-carboxylate (M4-38)

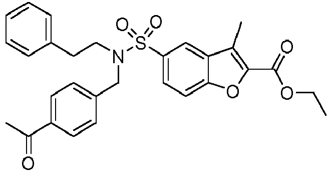

Ethyl 5-(N-benzyl-*N-*phenethylsulfamoyl)-3-methylbenzofuran-2-carboxylate (F27-S8), this example was implemented under the same condition except that 4-(acetyl)benzyl bromide was used instead of benzyl bromide. 256 mg of white solid was obtained, with a yield of 98%. ¹H NMR (400 MHz, Chloroform-*d*) δ 8.11 (d, *J* = 1.9 Hz, 1H), 7.95 - 7.76 (m, 3H), 7.57 (d, *J* = 8.7 Hz, 1H), 7.33 (d, *J* = 8.1 Hz, 2H), 7.20 - 7.02 (m, 3H), 6.97 - 6.84 (m, 2H), 4.50 - 4.33 (m, 4H), 3.42 - 3.28 (m, 2H), 2.67 - 2.49 (m, 8H), 1.40 (t, *J* = 7.1 Hz, 3H). ¹³C NMR (101 MHz, Chloroform-*d*) δ 198.8, 159.9, 155.8, 142.9, 142.2, 138.0, 136.3, 135.1, 129.4, 128.8, 128.6, 128.5, 128.4, 126.5, 126.2, 125.5, 121.5, 113.2, 61.6, 51.9, 49.9, 35.1, 26.5, 14.3, 9.4. HRMS (ESI) [M + H] ⁺, theoretically calculated for C₂₉H₃₀NO₆S: 520.1794, found 520.1786.

### Example 31

### ethyl 3-methyl-5-(N-(4-(methoxycarbonyl)benzyl)-N-phenethylsulfamoyl)benzofuran-2-carboxylate (M4-39)

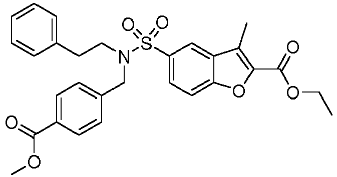

Ethyl 5-(N-benzyl-*N-*phenethylsulfamoyl)-3-methylbenzofuran-2-carboxylate (F27-S8), this example was implemented under the same condition except that 4-(methoxycarbonyl)benzyl bromide was used instead of benzyl bromide. 188 mg of white solid was obtained, with a yield of 70%. ¹H NMR (400 MHz, Chloroform-*d*) δ 8.12 (d, *J* = 1.9 Hz, 1H), 7.94 (d, *J* = 7.9 Hz, 2H), 7.85 (dd, *J* = 8.8, 1.9 Hz, 1H), 7.61 (d, *J* = 8.8 Hz, 1H), 7.32 (d, *J* = 8.0 Hz, 2H), 7.14 (dq, *J* = 14.2, 6.9 Hz, 3H), 6.92 (d, *J* = 6.7 Hz, 2H), 4.57 - 4.34 (m, 4H), 3.88 (s, 3H), 3.48 - 3.29 (m, 2H), 2.61 (d, *J* = 25.1 Hz, 5H), 1.44 (t, *J* = 7.1 Hz, 3H). ¹³C NMR (101 MHz, Chloroform-*d*) δ 166.6, 159.8, 155.8, 143.0, 141.4, 138.0, 135.3, 129.9, 129.8, 129.5, 128.6, 128.5, 128.2, 126.6, 126.2, 125.5, 121.5, 113.1, 61.6, 52.1, 52.0, 49.9, 35.2, 14.4, 9.4.

### Example 32

### ethyl 3-methyl-5-(N-(4-(tert-butoxycarbonyl)benzyl)-N-phenethylsulfamoyl)benzofuran-2-carboxylate (M4-40)

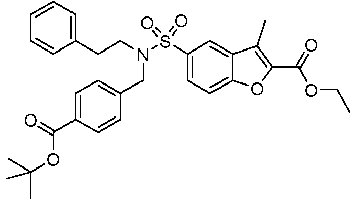

Ethyl 5-(N-benzyl-*N-*phenethylsulfamoyl)-3-methylbenzofuran-2-carboxylate (F27-S8), this example was implemented under the same condition except that 4-(tert-butoxycarbonyl)benzyl bromide was used instead of benzyl bromide. 273 mg of white solid was obtained, with a yield of 94%. ¹H NMR (400 MHz, Chloroform-*d*) δ 8.12 (d, *J* = 1.6 Hz, 1H), 7.92 (d, *J* = 8.2 Hz, 2H), 7.87 (dd, *J* = 8.8, 1.8 Hz, 1H), 7.62 (d, *J* = 8.8 Hz, 1H), 7.31 (d, *J* = 8.2 Hz, 2H), 7.16 (p, *J* = 6.8 Hz, 3H), 6.95 (d, *J* = 6.7 Hz, 2H), 4.43 (s, 4H), 3.43 - 3.31 (m, 2H), 2.70 - 2.61 (m, 2H), 2.59 (s, 3H), 1.59 (s, 9H), 1.45 (t, *J* = 7.1 Hz, 3H). ¹³C NMR (101 MHz, Chloroform-*d*) δ 165.3, 159.8, 155.8, 143.0, 140.8, 138.1, 135.3, 131.7, 129.7, 129.4, 128.6, 128.5, 128.1, 126.5, 126.2, 125.5, 121.5, 113.1, 81.1, 61.5, 51.9, 49.8, 35.2, 28.2, 14.4, 9.4.

### Example 33

### ethyl 3-methyl-5-(N-(4-(2-methoxy-2-oxoethyl)benzyl)-N-phenethylsulfamoyl)benzofuran-2-carboxylate (M4-41)

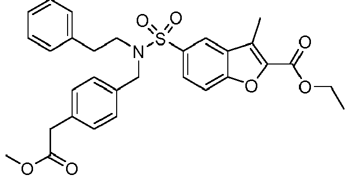

Ethyl 5-(N-benzyl-*N*-phenethylsulfamoyl)-3-methylbenzofuran-2-carboxylate (F27-S8), this example was implemented under the same condition except that methyl 2-(4-(bromomethyl)phenyl) acetate was used instead of benzyl bromide. 150 mg of white solid was obtained, with a yield of 55%. ¹H NMR (400 MHz, Chloroform-*d*) δ 8.15 (d, *J* = 1.9 Hz, 1H), 7.85 (dd, *J* = 8.8, 1.9 Hz, 1H), 7.62 (d, *J* = 8.8 Hz, 1H), 7.25 - 7.10 (m, 7H), 6.94 (d, *J* = 6.8 Hz, 2H), 4.47 (q, *J* = 7.1 Hz, 2H), 4.37 (s, 2H), 3.67 (s, 3H), 3.60 (s, 2H), 3.41 - 3.29 (m, 2H), 2.62 (d, J= 20.1 Hz, 5H), 1.45 (t, *J* = 7.1 Hz, 3H). ¹³C NMR (101 MHz, Chloroform-*d*) δ 171.8, 159.9, 155.8, 143.0, 138.3, 135.5, 134.9, 133.8, 129.6, 129.4, 128.7, 128.5, 126.5, 126.2, 125.6, 121.5, 113.1, 61.6, 52.1, 51.7, 49.4, 40.8, 35.2, 14.4, 9.4.

### Example 34

### (E)ethyl-3-methyl-5-(N-(4-(3-methoxy-3-oxoprop-1-en-1-yl)benzyl)-N-phenethylsulfamoyl)benzofuran-2-carboxylate (M4-42)

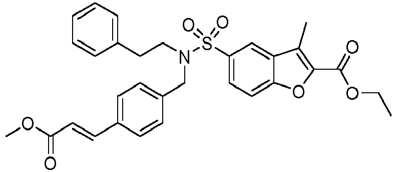

Ethyl 5-(N-benzyl-*N-*phenethylsulfamoyl)-3-methylbenzofuran-2-carboxylate (F27-S8), this example was implemented under the same condition except that methyl (E)-3-(4-(bromomethyl)phenyl) acrylate was used instead of benzyl bromide. 134 mg of white solid was obtained, with a yield of 48%. ¹H NMR (400 MHz, Chloroform-*d*) δ 8.14 (d, *J* = 1.9 Hz, 1H), 7.87 (dd, *J* = 8.8, 1.9 Hz, 1H), 7.69 - 7.59 (m, 2H), 7.44 (d, *J* = 8.1 Hz, 2H), 7.29 (d, *J* = 8.0 Hz, 2H), 7.22 - 7.10 (m, 3H), 6.95 (dd, *J* = 7.9, 1.6 Hz, 2H), 6.42 (d, *J* = 16.0 Hz, 1H), 4.47 (q, *J* = 7.1 Hz, 2H), 4.41 (s, 2H), 3.80 (s, 3H), 3.44 - 3.34 (m, 2H), 2.73 - 2.64 (m, 2H), 2.59 (s, 3H), 1.45 (t, *J* = 7.1 Hz, 3H). ¹³C NMR (101 MHz, Chloroform-*d*) δ 167.2, 159.8, 155.8, 144.0, 143.0, 138.6, 138.1, 135.4, 134.1, 129.4, 128.8, 128.6, 128.5, 128.3, 126.5, 126.2, 125.5, 121.5, 118.1, 113.1, 61.6, 51.9, 51.7, 49.7, 35.2, 14.4, 9.4.

### Example 35

### ethyl 3-methyl-5-(N-(naphthalen-1-ylmethyl)-N-phenethylsulfamoyl)benzofuran-2-carboxylate (M4-43)

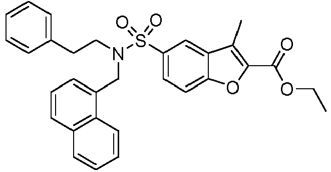

Ethyl 5-(N-benzyl-*N*-phenethylsulfamoyl)-3-methylbenzofuran-2-carboxylate (F27-S8), this example was implemented under the same condition except that 1-(bromomethyl) naphthalene was used instead of benzyl bromide. 220 mg of white solid was obtained, with a yield of 84%. ¹H NMR (400 MHz, Chloroform-*d*) δ 8.28 (d, *J* = 8.1 Hz, 1H), 8.20 (s, 1H), 7.95 (d, *J* = 8.5 Hz, 1H), 7.81 (t, *J* = 6.7 Hz, 2H), 7.62 (d, *J* = 8.7 Hz, 1H), 7.50 (dt, *J* = 15.2, 6.7 Hz, 2H), 7.36 (d, *J* = 8.8 Hz, 2H), 7.08 (d, *J* = 7.3 Hz, 3H), 6.75 (d, *J* = 6.7 Hz, 2H), 4.80 (s, 2H), 4.46 (q, *J* = 6.9 Hz, 2H), 3.40 - 3.10 (m, 2H), 2.58 (s, 3H), 2.46 - 2.29 (m, 2H), 1.44 (t, *J* = 7.0 Hz, 3H). ¹³C NMR (101 MHz, Chloroform-*d*) δ 159.9, 155.9, 143.0, 138.3, 134.5, 133.9, 132.0, 130.9, 129.5, 129.4, 128.7, 128.6, 128.4, 127.9, 126.8, 126.5, 126.4, 126.2, 125.6, 125.0, 124.0, 121.7, 113.1, 61.6, 51.4, 49.9, 35.7, 14.4, 9.4. HRMS (ESI) [M + H] ⁺, theoretically calculated for C₃₁H₃₀NO₅S: 528.1845, found 528.1837.

### Example 36

### ethyl 3-methyl-5-(N-(quinolin-8-ylmethyl)-N-phenethylsulfamoyl)benzofuran-2-carboxylate (M4-44)

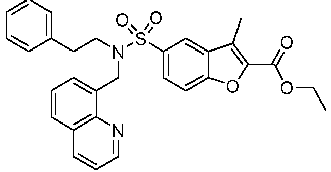

Ethyl 5-(N-benzyl-*N-*phenethylsulfamoyl)-3-methylbenzofuran-2-carboxylate (F27-S8), this example was implemented under the same condition except that 8-(bromomethyl)quinoline was used instead of benzyl bromide. 222 mg of white solid was obtained, with a yield of 84%. ¹H NMR (400 MHz, Chloroform-*d*) δ 8.83 (d, *J* = 2.6 Hz, 1H), 8.19 - 8.07 (m, 2H), 7.95 (d, *J* = 7.1 Hz, 1H), 7.88 (d, *J* = 10.3 Hz, 1H), 7.71 (d, *J* = 8.0 Hz, 1H), 7.61 - 7.47 (m, 2H), 7.36 (dd, *J* = 8.2, 4.2 Hz, 1H), 7.11 (dq, *J* = 14.2, 7.0 Hz, 3H), 6.94 (d, *J* = 7.0 Hz, 2H), 5.22 (s, 2H), 4.46 (q, *J* = 7.1 Hz, 2H), 3.71 - 3.50 (m, 2H), 2.91 - 2.66 (m, 2H), 2.56 (s, 3H), 1.44 (t, *J=* 7.1 Hz, 3H). ¹³C NMR (101 MHz, Chloroform-*d*) δ 159.9, 155.6, 149.5, 146.2, 142.8, 138.4, 136.4, 135.7, 134.8, 129.7, 129.2, 128.7, 128.4, 128.1, 127.7, 126.5, 126.3, 126.3, 125.6, 121.4, 121.2, 112.8, 61.5, 50.8, 47.5, 35.3, 14.4, 9.4. HRMS (ESI) [M + H] ⁺, theoretically calculated for C₃₀H₂₉N₂O₅S: 529.1797, found 529.1790.

### Example 37

### ethyl 3-methyl-5-(N-(naphthalen-2-ylmethyl)-N-phenethylsulfamoyl)benzofuran-2-carboxylate (M4-45)

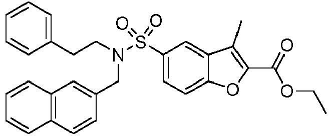

Ethyl 5-(N-benzyl-*N-*phenethylsulfamoyl)-3-methylbenzofuran-2-carboxylate (F27-S8), this example was implemented under the same condition except that 2-(bromomethyl)naphthalene was used instead of benzyl bromide. 214 mg of white solid was obtained, with a yield of 81%. ¹H NMR (400 MHz, Chloroform-*d*) δ 8.15 (s, 1H), 7.92 (d, *J* = 8.8 Hz, 1H), 7.82 - 7.69 (m, 3H), 7.66 - 7.57 (m, 2H), 7.50 - 7.38 (m, 3H), 7.14 (t, *J* = 7.5 Hz, 3H), 6.95 (d, *J* = 6.2 Hz, 2H), 4.58 (s, 2H), 4.48 (q, *J* = 7.1 Hz, 2H), 3.53 - 3.41 (m, 2H), 2.79 - 2.68 (m, 2H), 2.52 (s, 3H), 1.46 (t, *J* = 7.1 Hz, 3H). ¹³C NMR (101 MHz, Chloroform-*d*) δ 159.9, 155.7, 142.9, 138.3, 135.6, 133.5, 133.2, 133.0, 129.4, 128.7, 128.6, 128.5, 127.8, 127.7, 127.4, 126.5, 126.4, 126.3, 126.2, 126.1, 125.6, 121.6, 113.1, 61.6, 52.4, 49.7, 35.3, 14.4, 9.3. HRMS (ESI) [M + H] ⁺, theoretically calculated for C₃₁H₃₀NO₅S: 528.1845, found 528.1853.

### Example 38

### ethyl 3-methyl-5-(N-(2,6-difluorobenzyl)-N-phenethylsulfamoyl)benzofuran-2-carboxylate (M4-46)

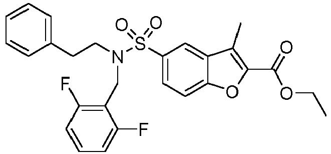

Ethyl 5-(N-benzyl-*N-*phenethylsulfamoyl)-3-methylbenzofuran-2-carboxylate (F27-S8), this example was implemented under the same condition except that 2,6-difluorobenzyl bromide was used instead of benzyl bromide. 203 mg of white solid was obtained, with a yield of 79%. ¹H NMR (400 MHz, Chloroform-*d*) δ 8.15 (d, *J* = 1.6 Hz, 1H), 7.87 (dd, *J* = 8.8, 1.9 Hz, 1H), 7.59 (d, *J* = 8.8 Hz, 1H), 7.31 - 7.21 (m, 3H), 7.20 - 7.13 (m, 1H), 7.12 - 7.05 (m, 2H), 6.85 (t, *J* = 7.9 Hz, 2H), 4.56 (s, 2H), 4.48 (q, *J* = 7.1 Hz, 2H), 3.49 - 3.35 (m, 2H), 2.94 - 2.80 (m, 2H), 2.61 (s, 3H), 1.46 (t, *J=* 7.1 Hz, 3H). ¹³C NMR (101 MHz, Chloroform-*d*) δ 161.8 (dd, *J* = 251.5, 7.8 Hz, 1C), 159.9, 155.8, 142.9, 138.3, 135.2, 130.5 (t, *J* = 10.5 Hz, 1C), 129.2, 128.7 (d, *J* = 20.6 Hz, 1C), 126.4 (d, *J* = 10.4 Hz, 1C), 125.6, 121.6, 112.9, 111.9, 111.6 (dd, *J* = 19.5, 6.1 Hz, 1C), 61.5, 49.9, 39.4 (t, *J* = 2.9 Hz, 1C), 35.5, 14.4, 9.4. ¹⁹F NMR (376 MHz, Chloroform-*d*) δ -112.91. HRMS (ESI) [M + H] ⁺, theoretically calculated for C₂₇H₂₆NO₅F₂S: 514.1500, found 514.1494.

### Example 39

### ethyl 3-methyl-5-(N-(2-fluoro-6-chlorobenzyl)-N-phenethylsulfamoyl)benzofuran-2-carboxylate (M4-47)

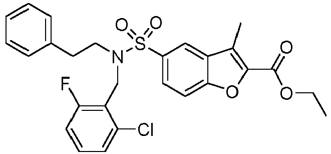

Ethyl 5-(N-benzyl-*N*-phenethylsulfamoyl)-3-methylbenzofuran-2-carboxylate (F27-S8), this example was implemented under the same condition except that 2-fluoro-6-chlorobenzyl bromide was used instead of benzyl bromide. 240 mg of white solid was obtained, with a yield of 91%. ¹H NMR (400 MHz, Chloroform-*d*) δ 8.16 (d, *J* = 1.7 Hz, 1H), 7.91 (dd, *J* = 8.8, 1.9 Hz, 1H), 7.62 (d, *J* = 8.8 Hz, 1H), 7.25 - 7.13 (m, 5H), 7.04 - 6.94 (m, 3H), 4.63 (s, 2H), 4.48 (q, *J* = 7.1 Hz, 2H), 3.40 - 3.28 (m, 2H), 2.78 - 2.70 (m, 2H), 2.62 (s, 3H), 1.47 (t, *J* = 7.1 Hz, 3H). ¹³C NMR (101 MHz, Chloroform-*d*) δ 162.2 (d, *J* = 252.6 Hz, 1C), 159.9, 155.8, 142.9, 138.3, 136.3 (d, *J* = 5.2. Hz, 1C), 134.9, 130.5 (d, *J* = 10.0 Hz, 1C), 129.3, 128.7, 128.5, 126.5, 126.4, 125.8 (d, *J* = 3.4 Hz, 1C), 125.6, 121.7, 121.5 (d, *J* = 17.0 Hz, 1C), 114.3 (d, *J* = 22.8 Hz, 1C), 112.9, 61.6, 50.1, 43.6 (d, *J =* 2.7 Hz, 1C), 35.9, 14.4, 9.4. ¹⁹F NMR (376 MHz, Chloroform-*d*) δ -110.75. HRMS (ESI) [M + H] ⁺, theoretically calculated for C₂₇H₂₆NO₅FSCl: 530.1204, found 530.1207.

### Example 40

### ethyl 3-methyl-5-(N-(2-fluoro-6-(trifluoromethyl)benzyl)-N-phenethylsulfamoyl)benzofuran-2-carboxylate (M4-48)

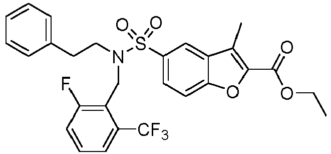

Ethyl 5-(N-benzyl-*N*-phenethylsulfamoyl)-3-methylbenzofuran-2-carboxylate (F27-S8), this example was implemented under the same condition except that 2-fluoro-6-(trifluoromethyl)benzyl bromide was used instead of benzyl bromide. 260 mg of white solid was obtained, with a yield of 92%. ¹H NMR (400 MHz, Chloroform-*d*) δ 8.22 - 8.13 (m, 1H), 7.91 (dd, *J* = 8.8, 1.5 Hz, 1H), 7.61 (d, *J* = 8.8 Hz, 1H), 7.47 (d, *J* = 7.7 Hz, 1H), 7.41 (q, *J* = 7.9 Hz, 1H), 7.14 (tt, *J* = 22.7, 8.2 Hz, 4H), 6.88 (d, *J* = 7.1 Hz, 2H), 4.66 (s, 2H), 4.44 (q, *J* = 7.1 Hz, 2H), 3.35 - 3.18 (m, 2H), 2.58 (d, *J* = 7.2 Hz, 5H), 1.42 (t, *J* = 7.1 Hz, 3H). ¹³C NMR (101 MHz, Chloroform-*d*) δ 162.4 (d, *J =* 252.4 Hz, 1C), 159.8, 155.8, 142.9, 138.2, 134.4, 130.5 (d, *J* = 9.5 Hz, 1C), 129.4, 128.5 (d, *J* = 6.8 Hz, 1C), 126.5 (d, *J* = 13.2 Hz, 1C), 125.5, 122.4, 122.0, 121.8, 119.9 (d, *J* = 22.9 Hz, 1C), 113.0, 61.5, 50.0, 43.3, 35.9, 14.3, 9.3. ¹⁹F NMR (376 MHz, Chloroform-*d*) δ -57.85, -109.39. HRMS (ESI) [M + H] ⁺, theoretically calculated for C₂₈H₂₆NO₅F₄S: 564.1468, found 564.1466.

### Example 41

### ethyl 3-methyl-5-(N-(2,6-dichlorobenzyl)-N-phenethylsulfamoyl)benzofuran-2-carboxylate (M4-49)

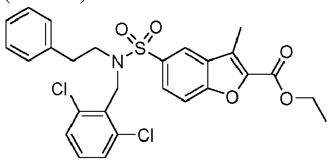

Ethyl 5-(N-benzyl-*N*-phenethylsulfamoyl)-3-methylbenzofuran-2-carboxylate (F27-S8), this example was implemented under the same condition except that 2,6-dichloro benzyl bromide was used instead of benzyl bromide. 254 mg of white solid was obtained, with a yield of 93%. ¹H NMR (400 MHz, Chloroform-*d*) δ 8.17 (d, *J* = 1.5 Hz, 1H), 7.93 (dd, *J* = 8.8, 1.8 Hz, 1H), 7.63 (d, *J* = 8.8 Hz, 1H), 7.27 (d, *J* = 7.7 Hz, 2H), 7.24 - 7.08 (m, 4H), 6.92 (d, *J* = 6.9 Hz, 2H), 4.70 (s, 2H), 4.47 (q, *J* = 7.1 Hz, 2H), 3.41 - 3.21 (m, 2H), 2.62 (d, *J* = 5.8 Hz, 5H), 1.45 (t, *J* = 7.1 Hz, 3H). ¹³C NMR (101 MHz, Chloroform-*d*) δ 159.8, 155.8, 142.9, 138.3, 137.2, 134.3, 130.8, 130.3, 129.3, 128.8, 128.7, 128.5, 126.6, 126.4, 125.6, 121.8, 112.9, 61.6, 50.2, 48.0, 36.6, 14.4, 9.4. HRMS (ESI) [M + H] ⁺, theoretically calculated for C₂₇H₂₆NO₅SCl₂: 546.0909, found 546.0917.

### Example 42

### ethyl 3-methyl-5-(N-(2,6-dimethylbenzyl)-N-phenethylsulfamoyl)benzofuran-2-carboxylate (M4-50)

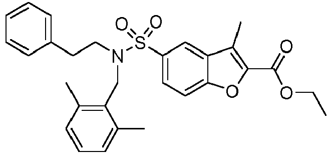

Ethyl 5-(N-benzyl-*N*-phenethylsulfamoyl)-3-methylbenzofuran-2-carboxylate (F27-S8), this example was implemented under the same condition except that 2,6-dimethylbenzyl bromide was used instead of benzyl bromide. 122 mg of white solid was obtained, with a yield of 80%. ¹H NMR (400 MHz, Chloroform-*d*) δ 8.21 - 8.11 (m, 1H), 7.93 (dd, *J* = 8.7, 1.6 Hz, 1H), 7.67 (d, *J* = 8.7 Hz, 1H), 7.23 - 7.09 (m, 4H), 7.04 (d, *J* = 7.5 Hz, 2H), 6.79 (d, *J* = 6.8 Hz, 2H), 4.48 (q, *J* = 7.1 Hz, 2H), 4.42 (s, 2H), 3.15 - 3.04 (m, 2H), 2.63 (s, 3H), 2.55 - 2.45 (m, 2H), 2.34 (s, 6H), 1.46 (t, *J* = 7.1 Hz, 3H). ¹³C NMR (101 MHz, Chloroform-*d*) δ 159.9, 155.8, 143.0, 138.5, 138.5, 133.9, 130.9, 129.5, 128.8, 128.6, 128.5, 126.4, 126.3, 125.6, 121.7, 113.0, 61.6, 49.9, 47.6, 37.0, 20.3, 14.4, 9.4. HRMS (ESI) [M + H] ⁺, theoretically calculated for C₂₉H₃₂NO₅S: 506.2001, found 506.1993.

### Example 43

### ethyl 3-methyl-5-(N-(2,4-difluorobenzyl)-N-phenethylsulfamoyl)benzofuran-2-carboxylate (M4-51)

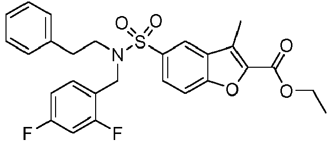

Ethyl 5-(N-benzyl-*N*-phenethylsulfamoyl)-3-methylbenzofuran-2-carboxylate (F27-S8), this example was implemented under the same condition except that 2,4-difluorobenzyl bromide was used instead of benzyl bromide. 252 mg of white solid was obtained, with a yield of 98%. ¹H NMR (400 MHz, Chloroform-*d*) δ 8.10 (s, 1H), 7.83 (d, *J* = 8.7 Hz, 1H), 7.61 (d, *J* = 8.8 Hz, 1H), 7.44 (q, *J* = 8.4 Hz, 1H), 7.17 (dq, *J* = 14.1, 7.0 Hz, 3H), 7.01 (d, *J* = 6.9 Hz, 2H), 6.85 (t, *J* = 8.1 Hz, 1H), 6.80 - 6.70 (m, 1H), 4.47 (dd, *J* = 15.5, 8.3 Hz, 4H), 3.46 - 3.33 (m, 2H), 2.78 - 2.67 (m, 2H), 2.60 (s, 3H), 1.45 (t, *J* = 7.1 Hz, 3H). ¹³C NMR (101 MHz, Chloroform-*d)* δ 163.0 (dd, *J* = 250.8, 12.2 Hz, 1C), 160.8 (dd, *J* = 249.8, 11.9 Hz, 1C), 159.9, 155.8, 143.0, 138.0, 135.3, 132.1 (dd, *J* = 9.7, 5.4 Hz, 1C), 129.4, 128.6 (d, *J* = 11.1 Hz, 1C), 126.6, 126.2, 125.5, 121.5, 119.3 (dd, *J* = 14.3, 3.7 Hz, 1C), 113.1, 111.8 (dd, *J* = 21.3, 3.7 Hz, 1C), 103.7 (t, *J* = 25.6 Hz, 1C), 61.6, 50.0, 44.8 (d, *J* = 3.1 Hz, 1C), 35.1, 14.4, 9.4. ¹⁹F NMR (376 MHz, Chloroform-*d*) δ -109.80, -114.40. HRMS (ESI) [M + H] ⁺, theoretically calculated for C₂₇H₂₆NO₅F₂S: 514.1500, found 514.1497.

### Example 44

### ethyl 3-methyl-5-(N-(2-fluoro-4-chlorobenzyl)-N-phenethylsulfamoyl)benzofuran-2-carboxylate (M4-52)

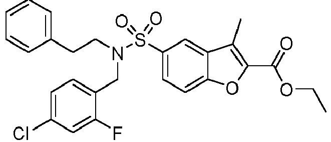

Ethyl 5-(N-benzyl-*N*-phenethylsulfamoyl)-3-methylbenzofuran-2-carboxylate (F27-S8), this example was implemented under the same condition except that 2-fluoro-4-chlorobenzyl bromide was used instead of benzyl bromide. 252 mg of white solid was obtained, with a yield of 95%. ¹H NMR (400 MHz, Chloroform-*d*) δ 8.08 (d, *J* = 1.5 Hz, 1H), 7.83 (dd, *J* = 8.8, 1.8 Hz, 1H), 7.60 (d, *J* = 8.8 Hz, 1H), 7.38 (t, *J* = 8.2 Hz, 1H), 7.17 (dq, *J=* 14.3, 7.1 Hz, 3H), 7.11 - 7.06 (m, 1H), 7.05 - 6.96 (m, 3H), 4.54 - 4.38 (m, 4H), 3.47 - 3.34 (m, 2H), 2.80 - 2.66 (m, 2H), 2.59 (s, 3H), 1.45 (t, *J* = 7.1 Hz, 3H). ¹³C NMR (101 MHz, Chloroform-*d*) δ 160.9 (d, *J* = 250.9 Hz, 1C), 159.8, 155.8, 143.0, 137.9, 135.2, 134.6 (d, *J* = 10.2 Hz, 1C), 131.9 (d, *J* = 4.6 Hz, 1C), 129.4, 128.6 (d, *J* = 10.8 Hz, 1C), 126.6, 126.2, 125.5, 124.9 (d, J= 3.5 Hz, 1C), 122.1 (d, *J* = 14.4 Hz, 1C), 121.5, 116.1 (d, *J* = 25.5 Hz, 1C), 113.1, 61.6, 50.2, 44.9 (d, *J* = 3.3 Hz, 1C), 35.1, 14.4, 9.4. ¹⁹F NMR (376 MHz, Chloroform-*d*) δ -115.97. HRMS (ESI) [M + H] ⁺, theoretically calculated for C₂₇H₂₆NO₅FSCl: 530.1204, found 530.1215.

### Example 45

### ethyl 3-methyl-5-(N-(2-fluoro-4-bromobenzyl)-N-phenethylsulfamoyl)benzofuran-2-carboxylate (M4-53)

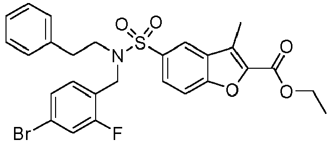

Ethyl 5-(N-benzyl-*N*-phenethylsulfamoyl)-3-methylbenzofuran-2-carboxylate (F27-S8), this example was implemented under the same condition except that 2-fluoro-4-bromobenzyl bromide was used instead of benzyl bromide. 280 mg of white solid was obtained, with a yield of 98%. ¹H NMR (400 MHz, Chloroform-*d*) δ 8.07 (d, *J* = 1.6 Hz, 1H), 7.82 (dd, *J* = 8.8, 1.8 Hz, 1H), 7.60 (d, J= 8.8 Hz, 1H), 7.31 (t, *J* = 8.1 Hz, 1H), 7.25 - 7.11 (m, 5H), 7.05 - 6.97 (m, 2H), 4.52 - 4.38 (m, 4H), 3.45 - 3.36 (m, 2H), 2.78 - 2.68 (m, 2H), 2.59 (s, 3H), 1.44 (t, *J* = 7.1 Hz, 3H). ¹³C NMR (101 MHz, Chloroform-*d*) δ 160.5 (d, *J* = 251.9 Hz, 1C), 159.8, 155.8, 143.0, 137.9, 135.2, 132.2 (d, *J* = 4.4 Hz, 1C), 129.4, 128.6 (d, *J=* 10.6 Hz, 1C), 127.8 (d, *J* = 3.5 Hz, 1C), 126.6, 126.1, 125.5, 122.6 (d, *J* = 14.3 Hz, 1C), 122.1 (d, *J* = 9.5 Hz, 1C), 121.5, 119.0 (d, *J* = 25.1 Hz, 1C), 113.1, 61.6, 50.2, 44.9 (d, *J=* 3.5 Hz, 1C), 35.1, 14.4, 9.4. ¹⁹F NMR (376 MHz, Chloroform-*d*) δ -115.76. HRMS (ESI) [M + H] ⁺, theoretically calculated for C₂₇H₂₆NO₅FSBr: 574.0699, found 574.0704.

### Example 46

### ethyl 3-methyl-5-(N-(2-fluoro-4-cyanobenzyl)-N-phenethylsulfamoyl)benzofuran-2-carboxylate (M4-54)

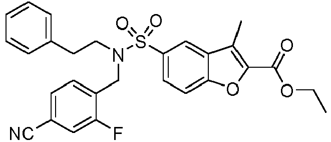

Ethyl 5-(N-benzyl-*N*-phenethylsulfamoyl)-3-methylbenzofuran-2-carboxylate (F27-S8), this example was implemented under the same condition except that 2-fluoro-4-cyanobenzyl bromide was used instead of benzyl bromide. 230 mg of white solid was obtained, with a yield of 92%. ¹H NMR (400 MHz, Chloroform-*d*) δ 8.12 (s, 1H), 7.84 (d, *J* = 8.7 Hz, 1H), 7.62 (dd, *J* = 11.9, 8.0 Hz, 2H), 7.40 (d, *J* = 8.0 Hz, 1H), 7.28 (d, *J* = 9.1 Hz, 1H), 7.16 (t, *J* = 8.0 Hz, 3H), 6.99 (d, *J* = 7.1 Hz, 2H), 4.47 (d, *J=* 9.5 Hz, 4H), 3.43 (t, *J=* 7.7 Hz, 2H), 2.80 - 2.48 (m, 5H), 1.45 (t, *J* = 7.1 Hz, 3H). ¹³C NMR (101 MHz, Chloroform-*d*) δ 159.9 (d, *J* = 251.0 Hz, 1C), 159.8, 155.9, 143.1, 137.6, 134.6, 131.7 (d, *J* = 4.3 Hz, 1C), 129.8 (d, *J* = 14.2 Hz, 1C), 129.5, 128.6, 128.4 (d, *J* = 3.4 Hz, 1C), 126.7, 126.1, 125.4, 121.6, 119.0 (d, *J* = 25.4 Hz, 1C), 117.3 (d, *J* = 2.4 Hz, 1C), 113.3, 113.1 (d, *J* = 9.7 Hz, 1C), 61.6, 50.6, 45.3 (d, *J* = 3.4 Hz, 1C), 35.0, 14.3, 9.1106-4. ¹⁹F NMR (376 MHz, Chloroform-*d*) δ -115.58. HRMS (ESI) [M + H] ⁺, theoretically calculated for C₂₈H₂₆N₂O₅FS: 521.1546, found 521.1552.

### Example 47

### ethyl 3-methyl-5-(N-(2-fluoro-4-(trifluoromethyl)benzyl)-N-phenethylsulfamoyl)benzofuran-2-carboxylate (M4-55)

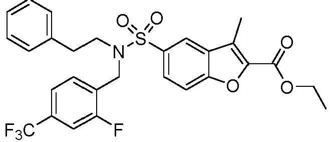

Ethyl 5-(N-benzyl-*N*-phenethylsulfamoyl)-3-methylbenzofuran-2-carboxylate (F27-S8), this example was implemented under the same condition except that 2-fluoro-4- (trifluoromethyl) benzyl bromide was used instead of benzyl bromide. 266 mg of white solid was obtained, with a yield of 95%. ¹H NMR (400 MHz, Chloroform-*d*) δ 8.02 (s, 1H), 7.82 - 7.70 (m, 1H), 7.51 (dd, *J* = 14.2, 8.1 Hz, 2H), 7.27 (d, *J* = 7.9 Hz, 1H), 7.21 - 7.00 (m, 4H), 6.91 (d, *J* = 6.8 Hz, 2H), 4.56 - 4.28 (m, 4H), 3.47 - 3.24 (m, 2H), 2.75 - 2.59 (m, 2H), 2.51 (s, 3H), 1.36 (t, *J* = 7.1 Hz, 3H). ¹³C NMR (101 MHz, Chloroform-*d*) δ 160.2 (d, *J* = 249.7 Hz, 1H), 159.8, 155.9, 143.1, 137.8, 134.9, 131.5 (d, *J* = 3.9 Hz, 1C), 129.5, 128.6, 128.6, 127.8 (d, *J* = 14.2 Hz, 1C), 126.6, 126.1, 125.5, 121.6, 121.4 (t, *J* = 3.4 Hz, 1C), 113.2, 112.9 (d, *J* = 3.9 Hz, 1C), 112.6 (d, *J =* 3.7 Hz, 1C), 61.6, 50.4, 45.1 (d, *J* = 3.7 Hz, 1C), 35.1, 14.3, 9.3. ¹⁹F NMR (376 MHz, Chloroform-*d*) δ -62.76, -116.40. HRMS (ESI) [M + H] ⁺, theoretically calculated for C₂₈H₂₆NO₅F₄S: 564.1468, found 564.1464.

### Example 48

### ethyl 3-methyl-5-(N-(2-chloro-4-fluorobenzyl)-N-phenethylsulfamoyl)benzofuran-2-carboxylate (M4-56)

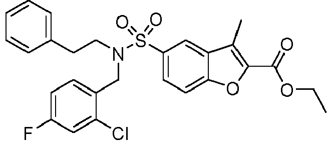

Ethyl 5-(N-benzyl-*N*-phenethylsulfamoyl)-3-methylbenzofuran-2-carboxylate (F27-S8), this example was implemented under the same condition except that 2-chloro-4-fluorobenzylbromide was used instead of benzyl bromide. 268 mg of white solid was obtained, with a yield of 99%. ¹H NMR (400 MHz, Chloroform-*d*) δ 8.23 - 8.07 (m, 1H), 7.87 (d, *J* = 8.7 Hz, 1H), 7.64 (d, *J* = 8.8 Hz, 1H), 7.52 (dd, *J* = 8.5, 6.2 Hz, 1H), 7.17 (dq, *J* = 14.2, 7.0 Hz, 3H), 7.10 - 6.93 (m, 4H), 4.47 (dd, *J* = 14.5, 7.3 Hz, 4H), 3.54 - 3.31 (m, 2H), 2.83 - 2.65 (m, 2H), 2.61 (s, 3H), 1.45 (t, *J* = 7.1 Hz, 3H). ¹³C NMR (101 MHz, Chloroform-*d*) δ 161.9 (d, *J* = 251.4 Hz, 1C), 159.9, 155.8, 143.1, 137.9, 135.1, 133.8 (d, *J* = 10.2, 1C), 131.8 (d, *J* = 8.7, 1C), 130.0 (d, *J* = 3.5, 1C), 129.5, 128.6 (d, *J* = 8.5, 1C), 126.6, 126.2, 125.5, 121.5, 116.7 (d, *J* = 24.8, 1C), 114.6 (d, *J* = 21.1, 1C), 113.2, 61.6, 50.5, 48.8, 35.2, 14.4, 9.4. ¹⁹F NMR (376 MHz, Chloroform-*d*) δ -111.97. HRMS (ESI) [M + H] ⁺, theoretically calculated for C₂₇H₂₆NO₅FSCl: 530.1204, found 530.1206.

### Example 49

### ethyl 3-methyl-5-(N-(2-(trifluoromethyl)-4-fluorobenzyl)-N-phenethylsulfamoyl)benzofuran-2-carboxylate (M4-57)

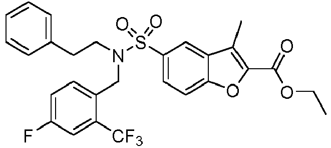

Ethyl 5-(N-benzyl-*N-*phenethylsulfamoyl)-3-methylbenzofuran-2-carboxylate (F27-S8), this example was implemented under the same condition except that 2-(trifluoromethyl)-4-fluorobenzylbromide was used instead of benzyl bromide. 238 mg of white solid was obtained, with a yield of 84%. ¹H NMR (400 MHz, Chloroform-*d*) δ 8.33 - 8.13 (m, 1H), 7.91 (dd, *J* = 8.8, 1.6 Hz, 1H), 7.81 (dt, *J* = 11.6, 5.8 Hz, 1H), 7.65 (d, *J* = 8.8 Hz, 1H), 7.31 (dd, *J* = 8.8, 2.3 Hz, 1H), 7.27 - 7.06 (m, 4H), 6.96 (d, *J* = 6.8 Hz, 2H), 4.59 (s, 2H), 4.46 (q, *J* = 7.1 Hz, 2H), 3.63 - 3.34 (m, 2H), 2.63 (d, *J* = 14.7 Hz, 5H), 1.45 (t, *J* = 7.1 Hz, 3H). ¹³C NMR (101 MHz, Chloroform-*d*) δ 161.3 (d, *J* = 249.9 Hz, 1H), 159.7, 155.9, 143.1, 137.7, 134.8, 132.2 (d, *J* = 7.9 Hz, 1C), 131.6 (d, *J* = 2.8 Hz, 1C), 129.6, 128.5 (d, *J* = 2.2 Hz, 1C), 126.6, 126.1, 125.5, 121.6, 119.2 (d, *J* = 21.2 Hz, 1C), 113.3, 61.5, 50.7, 47.9, 34.8, 14.3, 9.3. ¹⁹F NMR (376 MHz, Chloroform-*d*) δ -59.60, -112.86. HRMS (ESI) [M + H] ⁺, theoretically calculated for C₂₈H₂₆NO₅F₄S: 564.1468, found 564.1465.

### Example 50

### ethyl 3-methyl-5-(N-(2,4-bis(trifluoromethyl)benzyl)-N-phenethylsulfamoyl)benzofuran-2-carboxylate (M4-58)

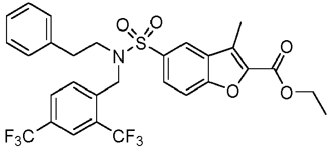

Ethyl 5-(N-benzyl-*N-*phenethylsulfamoyl)-3-methylbenzofuran-2-carboxylate (F27-S8), this example was implemented under the same condition except that 2,4-bis (trifluoromethyl)benzyl bromide was used instead of benzyl bromide. 270 mg of white solid was obtained, with a yield of 88%. ¹H NMR (400 MHz, Chloroform-*d*) δ 8.28 - 8.17 (m, 1H), 8.03 - 7.85 (m, 3H), 7.74 (d, *J* = 8.1 Hz, 1H), 7.65 (d, *J* = 8.8 Hz, 1H), 7.13 (q, *J* = 8.4, 7.3 Hz, 3H), 6.96 (d, *J* = 6.5 Hz, 2H), 4.67 (s, 2H), 4.47 (q, *J* = 7.1 Hz, 2H), 3.67 - 3.37 (m, 2H), 2.64 (d, *J* = 22.4 Hz, 5H), 1.45 (t, *J* = 7.1 Hz, 3H). ¹³C NMR (101 MHz, Chloroform-*d*) δ 159.7, 155.9, 143.1, 140.4, 137.5, 134.4, 130.5, 130.0 (d, *J* = 33.8 Hz, 1C), 129.6, 129.0, 128.5, 128.1 (d, *J* = 31.5 Hz, 1C), 126.6, 126.1, 125.4, 124.8 (d, *J* = 26.8 Hz, 1C), 122.9, 122.1 (d, *J* = 25.1 Hz, 1C), 121.7, 113.3, 61.5, 51.0, 48.4, 34.7, 14.2, 9.2. ¹⁹F NMR (376 MHz, Chloroform-*d*) δ -59.95, -62.92. HRMS (ESI) [M + H] ⁺, theoretically calculated for C₂₉H₂₆NO₅F₆S: 613.1358, found 614.1436.

### Example 51

### ethyl 3-methyl-5-(N-(2-trifluoromethyl-4-bromobenzyl)-N-phenethylsulfamoyl)benzofuran-2-carboxylate (M4-59)

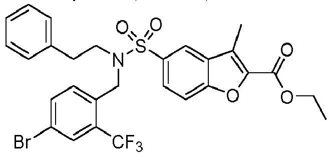

Ethyl 5-(N-benzyl-*N*-phenethylsulfamoyl)-3-methylbenzofuran-2-carboxylate (F27-S8), this example was implemented under the same condition except that 2-trifluoromethyl-4-bromobenzyl bromide was used instead of benzyl bromide. 311 mg of white solid was obtained, with a yield of 99%. ¹H NMR (400 MHz, Chloroform-*d*) δ 8.18 (d, *J* = 1.5 Hz, 1H), 7.89 (dd, *J* = 8.8, 1.8 Hz, 1H), 7.77 - 7.73 (m, 1H), 7.71 - 7.59 (m, 3H), 7.23 - 7.10 (m, 3H), 6.96 (d, *J* = 6.4 Hz, 2H), 4.54 (s, 2H), 4.47 (q, *J* = 7.1 Hz, 2H), 3.50 - 3.35 (m, 2H), 2.71 - 2.56 (m, 5H), 1.45 (t, *J* = 7.1 Hz, 3H). ¹³C NMR (101 MHz, Chloroform-*d*) δ 159.8, 155.9, 143.1, 137.6, 135.4, 135.0, 134.6, 131.6, 129.6, 129.2, 128.8 (t, *J* = 6.3 Hz, 1C), 128.6 (d, *J* = 1.5 Hz, 1C), 126.6, 126.1, 125.5, 121.6, 121.4, 113.4, 61.6, 50.9, 48.1 (d, *J* = 2.4 Hz, 1C), 34.9, 14.4, 9.4. ¹⁹F NMR (376 MHz, Chloroform-*d*) δ -59.55. HRMS (ESI) [M + H] ⁺, theoretically calculated for C₂₈H₂₆NO₅F₃SBr: 624.0667, found 624.0663.

### Example 52

### ethyl 3-methyl-5-(N-(2,4-dichlorobenzyl)-N-phenethylsulfamoyl)benzofuran-2-carboxylate (M4-60)

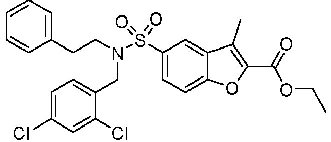

Ethyl 5-(N-benzyl-*N*-phenethylsulfamoyl)-3-methylbenzofuran-2-carboxylate (F27-S8), this example was implemented under the same condition except that 2,4-dichloro-benzyl bromide was used instead of benzyl bromide. 113 mg of white solid was obtained, with a yield of 69%. ¹H NMR (400 MHz, Chloroform-*d*) δ 8.13 (d, *J* = 1.6 Hz, 1H), 7.86 (dd, *J* = 8.8, 1.9 Hz, 1H), 7.64 (d, *J* = 8.8 Hz, 1H), 7.47 (d, *J* = 8.4 Hz, 1H), 7.31 (d, *J* = 2.1 Hz, 1H), 7.24 - 7.13 (m, 4H), 7.05 - 6.98 (m, 2H), 4.60 - 4.37 (m, 4H), 3.54 - 3.34 (m, 2H), 2.76 - 2.66 (m, 2H), 2.61 (s, 3H), 1.46 (t, *J* = 7.1 Hz, 3H). ¹³C NMR (101 MHz, Chloroform-*d*) δ 159.9, 155.9, 143.1, 137.8, 135.0, 134.2, 133.7, 132.7, 131.3, 129.5, 129.2, 128.7, 128.6, 127.5, 126.6, 126.2, 125.5, 121.6, 113.2, 61.6, 50.7, 48.9, 35.2, 14.4, 9.4. HRMS (ESI) [M + H] ⁺, theoretically calculated for C₂₇H₂₆NO₅SCl₂: 546.0909, found 546.0912.

### Example 53

### ethyl 3-methyl-5-(N-(2-methyl-5-fluorobenzyl)-N-phenethylsulfamoyl)benzofuran-2-carboxylate (M4-61)

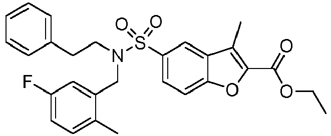

Ethyl 5-(N-benzyl-*N*-phenethylsulfamoyl)-3-methylbenzofuran-2-carboxylate (F27-S8), this example was implemented under the same condition except that 2-methyl-5-fluorobenzyl bromide was used instead of benzyl bromide. 134 mg of white solid was obtained, with a yield of 87%. ¹H NMR (400 MHz, Chloroform-*d*) δ 8.17 (d, *J* = 1.6 Hz, 1H), 7.90 (dd, *J* = 8.8, 1.8 Hz, 1H), 7.65 (d, *J* = 8.8 Hz, 1H), 7.17 (m, 3H), 7.09 (dd, *J* = 8.2, 5.8 Hz, 1H), 6.99 - 6.84 (m, 4H), 4.47 (q, *J* = 7.1 Hz, 2H), 4.34 (s, 2H), 3.40 - 3.25 (m, 2H), 2.61 (d, *J* = 6.1 Hz, 5H), 2.27 (s, 3H), 1.46 (t, *J=* 7.1 Hz, 3H). ¹³C NMR (101 MHz, Chloroform-*d*) δ 162.4, 160.0, 159.9, 155.9, 143.0, 138.1, 135.7 (d, *J* = 6.8 Hz, 1C), 134.7, 132.5 (d, *J* = 3.3 Hz, 1C), 132.0 (d, *J* = 7.7 Hz, 1C), 129.5, 128.6 (d, *J* = 4.8 Hz, 1C), 126.5, 126.3, 125.6, 121.6, 115.9 (d, *J* = 22.2 Hz, 1C), 114.6 (d, *J* = 20.7 Hz, 1C), 113.1, 61.6, 50.6, 50.1, 35.5, 18.5, 14.4, 9.4. ¹⁹F NMR (376 MHz, Chloroform-*d*) δ -116.96. HRMS (ESI) [M + H] ⁺, theoretically calculated for C₂₈H₂₉NO₅FS: 510.1750, found 510.1758.

### Example 54

### ethyl 3-methyl-5-(N-(2,6-difluoro-4-chlorobenzyl)-N-phenethylsulfamoyl)benzofuran-2-carboxylate (M4-62)

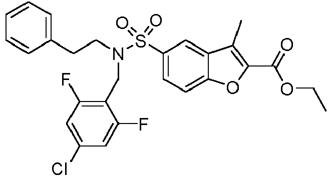

Ethyl 5-(N-benzyl-*N*-phenethylsulfamoyl)-3-methylbenzofuran-2-carboxylate (F27-S8), this example was implemented under the same condition except that 2,6-difluoro-4-chlorobenzyl bromide was used instead of benzyl bromide. 243 mg of white solid was obtained, with a yield of 89%. ¹H NMR (400 MHz, Chloroform-*d*) δ 8.09 (d, *J* = 1.5 Hz, 1H), 7.84 (dd, *J* = 8.8, 1.8 Hz, 1H), 7.59 (d, *J* = 8.8 Hz, 1H), 7.25 - 7.12 (m, 3H), 7.06 (d, *J* = 6.9 Hz, 2H), 6.83 (d, *J=* 7.2 Hz, 2H), 4.46 (d, *J* = 7.2 Hz, 4H), 3.48 - 3.32 (m, 2H), 2.90 - 2.78 (m, 2H), 2.59 (s, 3H), 1.44 (t, *J* = 7.1 Hz, 3H). ¹³C NMR (101 MHz, Chloroform-*d*) δ 161.5 (dd, *J* = 254.1, 9.4 Hz, 1C), 159.8, 155.8, 143.0, 138.1, 135.5 (t, *J* = 14.2 Hz, 1C), 134.9, 129.3, 128.7, 128.6, 126.5, 126.3, 125.5, 121.6, 112.6 (dd, *J* = 29.8, 1.8 Hz, 1C), 110.9 (t, *J* = 18.9 Hz, 1C), 61.6, 50.2, 39.5, 35.4, 14.4, 9.3. ¹⁹F NMR (376 MHz, Chloroform-*d*) δ -110.99. HRMS (ESI) [M + H] ⁺, theoretically calculated for C₂₇H₂₅NO₅F₂SCl: 548.1110, found 548.1105.

### Example 55

### ethyl 3-methyl-5-(N-([1,1'-biphenyl]-4-ylmethyl-N-phenethylsulfamoyl)benzofuran-2-carboxylate (M4-82)

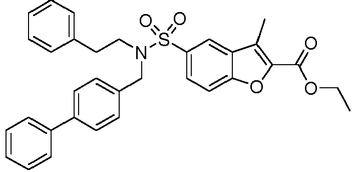

Ethyl 5-(N-benzyl-*N*-phenethylsulfamoyl)-3-methylbenzofuran-2-carboxylate (F27-S8), this example was implemented under the same condition except that 4-phenylbenzyl bromide was used instead of benzyl bromide. 191 mg of white solid was obtained, with a yield of 69%. ¹H NMR (400 MHz, Chloroform-*d*) δ 8.14 (s, 1H), 7.88 (d, *J* = 8.8 Hz, 1H), 7.64 (d, *J* = 8.8 Hz, 1H), 7.54 (dd, *J* = 13.2, 7.7 Hz, 4H), 7.43 (t, *J* = 7.5 Hz, 2H), 7.33 (dd, *J* = 17.2, 7.7 Hz, 3H), 7.18 (dt, *J* = 12.5, 6.8 Hz, 3H), 6.98 (d, *J* = 6.7 Hz, 2H), 4.47 (dd, *J* = 15.6, 8.5 Hz, 4H), 3.49 - 3.34 (m, 2H), 2.80 - 2.67 (m, 2H), 2.58 (s, 3H), 1.45 (t, *J* = 7.1 Hz, 3H). ¹³C NMR (101 MHz, Chloroform-*d*) δ 159.9, 155.8, 143.0, 140.9, 140.5, 138.3, 135.6, 134.9, 129.4, 128.9, 128.8, 128.7, 128.5, 127.5, 127.3, 127.0, 126.5, 126.2, 125.6, 121.6, 113.1, 61.6, 51.9, 49.6, 35.3, 14.4, 9.4. HRMS (ESI) [M + H] ⁺, theoretically calculated for C₃₃H₃₂NO₅S: 554.2001, found 546.1562. HRMS (ESI) [M + H] ⁺, theoretically calculated for C₃₃H₃₂NO₅S: 5554.2001, found 554.2001.

### Example 56

### ethyl 3-methyl-5-(N-((2'-cyano-[1,1'-biphenyl]-4-yl)methyl)-N-phenethylsulfamoyl)benzofuran-2-carboxylate (M4-83)

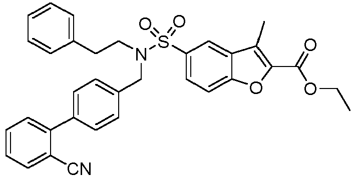

Ethyl 5-(N-benzyl-*N*-phenethylsulfamoyl)-3-methylbenzofuran-2-carboxylate (F27-S8), this example was implemented under the same condition except that 4'-(bromomethyl)-[1,1'-biphenyl]-2-carbonitrile was used instead of benzyl bromide. 270 mg of white solid was obtained, with a yield of 93%. ¹H NMR (400 MHz, Chloroform-*d*) δ 8.19 (d, *J* = 1.5 Hz, 1H), 7.88 (dd, *J* = 8.8, 1.8 Hz, 1H), 7.72 (d, *J* = 7.7 Hz, 1H), 7.62 (dd, *J* = 8.1, 6.0 Hz, 2H), 7.53 - 7.35 (m, 6H), 7.17 (t, *J* = 7.2 Hz, 2H), 7.12 (d, *J* = 7.1 Hz, 1H), 7.02 - 6.95 (m, 2H), 4.45 (q, *J* = 7.2 Hz, 4H), 3.51 - 3.38 (m, 2H), 2.75 - 2.67 (m, 2H), 2.60 (s, 3H), 1.43 (t, *J* = 7.1 Hz, 3H). ¹³ C NMR (101 MHz, Chloroform-*d*) δ 159.9, 155.8, 144.7, 143.0, 138.2, 137.8, 136.8, 135.4, 133.8, 132.9, 130.0, 129.4, 129.1, 128.7, 128.5, 127.8, 126.5, 126.3, 125.6, 121.6, 118.6, 113.1, 111.2, 61.6, 51.8, 49.7, 35.2, 14.4, 9.4. HRMS (ESI) [M + H] ⁺, theoretically calculated for C₃₄H₃₁N₂O₅S: 579.1954, found 579.1950.

### Example 57

### ethyl 3-methyl-5-(N-((2'-(tert-butoxycarbonyl)-[1,1'-biphenyl]-4-yl)methyl)-N-phenethylsulfamoyl)benzofuran-2-carboxylate (M4-84)

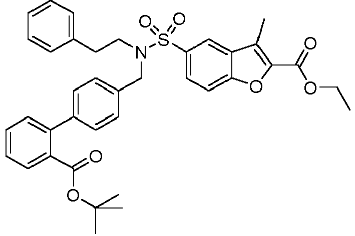

Ethyl 5-(N-benzyl-*N-*phenethylsulfamoyl)-3-methylbenzofuran-2-carboxylate (F27-S8), this example was implemented under the same condition except that tert-butyl 4'-(bromomethyl)-[1,1'-biphenyl]-2-carboxylate was used instead of benzyl bromide. 303 mg of white solid was obtained, with a yield of 93%. ¹H NMR (400 MHz, Chloroform-*d*) δ 8.22 (d, *J* = 1.5 Hz, 1H), 7.92 (dd, *J* = 8.8, 1.7 Hz, 1H), 7.79 (dd, *J* = 7.7, 1.4 Hz, 1H), 7.66 (d, *J* = 8.7 Hz, 1H), 7.52 - 7.44 (m, 1H), 7.43 - 7.37 (m, 1H), 7.36 - 7.26 (m, 5H), 7.18 (dq, *J* = 14.3, 7.1 Hz, 3H), 7.01 (d, *J* = 6.9 Hz, 2H), 4.57 - 4.42 (m, 4H), 3.56 - 3.34 (m, 2H), 2.82 - 2.70 (m, 2H), 2.63 (s, 3H), 1.47 (t,*J* = 7.1 Hz, 3H), 1.27 (s, 9H). ¹³C NMR (101 MHz, Chloroform-*d*) δ 167.9, 159.8, 155.8, 143.0, 141.6, 141.4, 138.3, 135.7, 134.9, 132.9, 130.7, 130.5, 129.6, 129.4, 129.0, 128.7, 128.5, 128.1, 127.3, 126.5, 126.3, 125.6, 121.5, 113.1, 81.2, 61.6, 51.7, 49.3, 35.1, 27.7, 14.4, 9.4. HRMS (ESI) [M + H] ⁺, theoretically calculated for C₃₈H₄₀NO₇S: 653.2447, found 654.2528.

### Example 58

### ethyl 3-methyl-5-(N-(4-benzoylbenzyl)-N-phenethylsulfamoyl)benzofuran-2-carboxylate (M4-85)

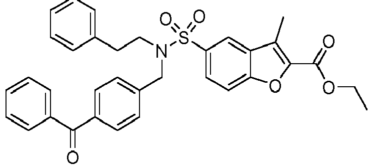

Ethyl 5-(N-benzyl-N phenethylsulfamoyl)-3-methylbenzofuran-2-carboxylate (F27-S8), this example was implemented under the same condition except that (4-(bromomethyl)phenyl)(phenyl)methanone was used instead of benzyl bromide. 142 mg of white solid was obtained, with a yield of 49%. ¹H NMR (400 MHz, Chloroform-*d*) δ 8.16 (d, *J* = 1.9 Hz, 1H), 7.88 (dd, *J* = 8.8, 1.9 Hz, 1H), 7.78 - 7.69 (m, 4H), 7.58 (dd, *J* = 27.2, 8.1 Hz, 2H), 7.50 - 7.35 (m, 4H), 7.14 (dq, *J* = 14.1, 7.0 Hz, 3H), 6.96 (d, *J* = 6.9 Hz, 2H), 4.45 (dd, *J* = 14.9, 7.8 Hz, 4H), 3.52 - 3.34 (m, 2H), 2.74 - 2.64 (m, 2H), 2.58 (s, 3H), 1.43 (t, *J* = 7.1 Hz, 3H). ¹³C NMR (101 MHz, Chloroform-*d*) δ 196.0, 159.8, 155.8, 143.1, 141.0, 138.1, 137.4, 137.1, 135.3, 132.5, 130.4, 130.0, 129.5, 128.6, 128.6, 128.4, 128.1, 126.6, 126.2, 125.5, 121.6, 113.2, 61.6, 51.9, 49.9, 35.2, 14.4, 9.4.

### Example 59

### 3-methyl-5-(N-benzyl-N-phenethylsulfamoyl)benzofuran-2-carboxylic acid (F27)

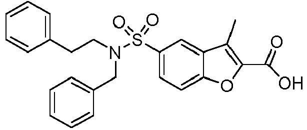

Ethyl 5-(N-benzyl-N-phenethylsulfamoyl)-3-methylbenzofuran-2-carboxylate (95 mg, 0.2 mmol) and sodium hydroxide (40 mg, 1.0 mmol) were dissolved in a mixed solution of ethanol (8 mL) and water (2 mL) and then stirred for 1 h at 100 °C. After the reaction was found to be completed from the monitoring of the TLC, the solvent was distilled off under reduced pressure, and the obtained product was dissolved in water (10 mL) and the pH value was then adjusted to 3.0 with 1M diluted hydrochloric acid. The resulting solution was set still for 5 h, and then filtered with suction under reduced pressure and dried to obtain 75 mg of white solid, with a yield of 84%. M.p. > 380 °C (for decomposition). ¹H NMR (400 MHz, DMSO-*d*₆) δ 8.28 (s, 1H), 7.95 (d, *J* = 8.7 Hz, 1H), 7.84 (d, *J* = 8.8 Hz, 1H), 7.33 (dd, *J* = 16.2, 3.9 Hz, 5H), 7.24 - 7.05 (m, 3H), 6.97 (d, *J* = 7.1 Hz, 2H), 4.43 (s, 2H), 3.41 - 3.20 (m, 2H), 2.58 (s, 3H), 2.56 - 2.51 (m, 2H). ¹³C NMR (101 MHz, DMSO-*d*₆) δ 161.3, 155.5, 144.2, 138.7, 137.3, 135.3, 129.7, 129.0, 128.9, 128.8, 128.8, 128.1, 126.7, 126.5, 124.8, 122.0, 113.5, 51.8, 49.8, 34.8, 9.6. HRMS (ESI) [M + H] ⁺, theoretically calculated for C₂₅H₂₄NO₅S: 450.1375, found 450.1378.

### Example 61

### 3-methyl-5-(N-(2-fluorobenzyl)-N-phenethylsulfamoyl)benzofuran-2-carboxylic acid (F27-S14)

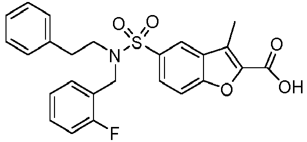

In accordance with the preparation method of 5-(*N*-benzyl-*N*-phenethylsulfamoyl)-3-methylbenzofuran-2-carboxylic acid (F27), this example was implemented under the same condition except that M4-14 was used instead of ethyl 5-(N-benzyl-N-phenethylsulfamoyl)-3-methylbenzofuran-2-carboxylate (F27-S8). 90 mg of white solid was obtained, with a yield of 96%. M.p. > 380 °C (for decomposition). ¹H NMR (400 MHz, DMSO-*d*₆) δ 8.06 (s, 1H), 7.80 (d, *J* = 10.6 Hz, 1H), 7.71 (d, *J* = 8.7 Hz, 1H), 7.43 (t, *J* = 7.7 Hz, 1H), 7.39 - 7.30 (m, 1H), 7.25 - 7.11 (m, 5H), 7.01 (d, *J* = 6.9 Hz, 2H), 4.47 (s, 2H), 3.38 - 3.27 (m, 2H), 2.63 - 2.54 (m, 2H), 2.52 (s, 3H). ¹³C NMR (101 MHz, DMSO-*d*₆) δ 162.8, 160.9 (d, *J* = 246.5 Hz, 1C), 154.8, 138.6, 133.8, 131.4 (d, *J* = 3.9 Hz, 1C), 130.9, 130.3 (d, *J* = 8.2 Hz, 1C), 128.9, 128.8, 126.8, 124.9 (d, *J* = 3.3 Hz, 1C), 124.5, 124.2 (d, *J* = 14.3 Hz, 1C), 120.7, 117.8, 115.9, 115.7, 112.8, 50.2, 45.8 (d, *J* = 3.3 Hz, 1C), 34.9, 9.5. ¹⁹F NMR (376 MHz, DMSO-*d*₆) δ -117.84. HRMS (ESI) [M - H] ⁻, theoretically calculated for C₂₅H₂₁NO₅FS: 466.1124, found 466.1128.

### Example 62

### 3-methyl-5-(N-(2-chlorobenzyl)-N-phenethylsulfamoyl)benzofuran-2-carboxylic acid (F27-S15)

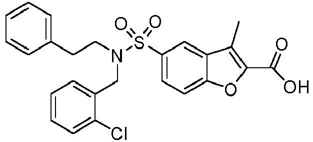

In accordance with the preparation method of 5-(*N*-benzyl-*N*-phenethylsulfamoyl)-3-methylbenzofuran-2-carboxylic acid (F27), this example was implemented under the same condition except that M4-15 was used instead of ethyl 5-(N-benzyl-N-phenethylsulfamoyl)-3-methylbenzofuran-2-carboxylate (F27-S8). 80 mg of white solid was obtained, with a yield of 83%. M.p. 156 - 157 °C. ¹H NMR (400 MHz, DMSO-*d*₆) δ 8.12 (s, 1H), 7.84 (d, *J* = 8.7 Hz, 1H), 7.75 (d, *J* = 8.7 Hz, 1H), 7.50 (s, 1H), 7.46 - 7.40 (m, 1H), 7.37 - 7.29 (m, 2H), 7.20 (t, *J* = 7.2 Hz, 2H), 7.14 (t, *J* = 7.1 Hz, 1H), 7.00 (d, *J* = 7.0 Hz, 2H), 4.50 (s, 2H), 3.41 - 3.27 (m, 2H), 2.56 (d, *J* = 14.9 Hz, 5H). ¹³C NMR (101 MHz, DMSO-*d*₆) δ 154.9, 138.5, 134.8, 133.8, 133.0, 130.9, 130.8, 129.8, 129.8, 128.9, 128.8, 127.7, 126.8, 124.8, 121.0, 112.9, 50.5, 49.7, 35.0, 9.5. HRMS (ESI) [M - H] ⁻, theoretically calculated for C₂₅H₂₁NO₅SCl: 482.0829, found 482.0832.

### Example 63

### 3-methyl-5-(N-(2-bromobenzyl)-N-phenethylsulfamoyl)benzofuran-2-carboxylic acid (F27-S16)

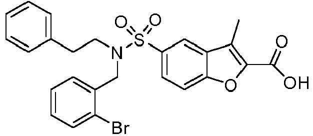

In accordance with the preparation method of 5-(*N*-benzyl-*N*-phenethylsulfamoyl)-3-methylbenzofuran-2-carboxylic acid (F27), this example was implemented under the same condition except that M4-16 was used instead of ethyl 5-(N-benzyl-N-phenethylsulfamoyl)-3-methylbenzofuran-2-carboxylate (F27-S8). 104 mg of white solid was obtained, with a yield of 98%. M.p. 170 - 171 °C. ¹H NMR (400 MHz, DMSO-*d*₆) δ 8.27 (d, *J* = 1.7 Hz, 1H), 7.95 (dd, *J* = 8.8, 1.9 Hz, 1H), 7.85 (d, *J* = 8.8 Hz, 1H), 7.61 (d, *J* = 7.0 Hz, 1H), 7.48 (d, *J* = 6.3 Hz, 1H), 7.37 (t, *J* = 7.0 Hz, 1H), 7.28 - 7.17 (m, 3H), 7.14 (t, *J* = 7.2 Hz, 1H), 7.02 (d, *J* = 8.2 Hz, 2H), 4.50 (s, 2H), 3.42 - 3.34 (m, 2H), 2.58 (s, 5H). ¹³C NMR (101 MHz, DMSO) δ 161.4, 155.5, 138.5, 136.3, 134.6, 133.1, 130.9, 130.1, 129.8, 129.0, 128.3, 126.8, 126.4, 123.2, 122.0, 113.5, 52.2, 50.6, 35.0, 9.6. HRMS (ESI) [M - H] ⁻, theoretically calculated for C₂₅H₂₁NO₅SBr: 526.0324, found 526.0331.

### Example 64

### 3-methyl-5-(N-(2-methylbenzyl)-N-phenethylsulfamoyl)benzofuran-2-carboxylic acid (F27-S17)

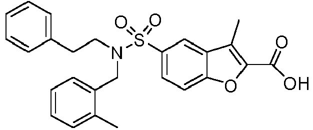

In accordance with the preparation method of 5-(*N*-benzyl-*N*-phenethylsulfamoyl)-3-methylbenzofuran-2-carboxylic acid (F27), this example was implemented under the same condition except that M4-17 was used instead of ethyl 5-(N-benzyl-N-phenethylsulfamoyl)-3-methylbenzofuran-2-carboxylate (F27-S8). 88 mg of white solid was obtained, with a yield of 96%. M.p. 199 - 200 °C. ¹H NMR (400 MHz, DMSO-*d*₆) δ 8.32 (d, *J* = 1.7 Hz, 1H), 8.00 (dd, *J* = 8.8, 1.9 Hz, 1H), 7.88 (d, *J* = 8.8 Hz, 1H), 7.32 (d, *J* = 7.4 Hz, 1H), 7.26 - 7.14 (m, 5H), 7.14 - 7.09 (m, 1H), 6.89 (d, *J* = 6.9 Hz, 2H), 4.39 (s, 2H), 3.28 - 3.18 (m, 2H), 2.59 (s, 3H), 2.45 - 2.36 (m, 2H), 2.32 (s, 3H). ¹³C NMR (101 MHz, DMSO-*d*₆) δ 161.2, 155.6, 143.8, 138.7, 137.4, 134.6, 134.6, 130.8, 129.8, 129.7, 128.9, 128.8, 128.3, 126.8, 126.7, 126.2, 125.2, 122.2, 113.5, 51.0, 49.9, 35.3, 19.2, 9.6. HRMS (ESI) [M - H] ⁻, theoretically calculated for C₂₆H₂₄NO₅S: 462.1375, found 462.1377.

### Example 65

### 3-methyl-5-(N-(2-trifluoromethylbenzyl)-N-phenethylsulfamoyl)benzofuran-2-carboxylic acid (F27-S18)

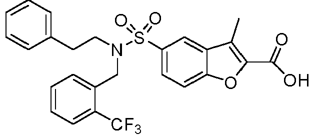

In accordance with the preparation method of 5-(*N*-benzyl-*N*-phenethylsulfamoyl)-3-methylbenzofuran-2-carboxylic acid (F27), this example was implemented under the same condition except that M4-18 was used instead of ethyl 5-(N-benzyl-N-phenethylsulfamoyl)-3-methylbenzofuran-2-carboxylate (F27-S8). 87 mg of white solid was obtained, with a yield of 84%. M.p. 138 - 139 °C. ¹H NMR (400 MHz, DMSO-*d*₆) δ 8.24 (s, 1H), 7.93 (d, *J* = 8.7 Hz, 1H), 7.83 (d, *J* = 8.7 Hz, 1H), 7.72 (d, *J* = 7.8 Hz, 1H), 7.65 (q, *J* = 7.6 Hz, 2H), 7.49 (t, *J* = 7.2 Hz, 1H), 7.16 (dq, *J* = 14.3, 7.1 Hz, 3H), 7.00 (d, *J* = 7.0 Hz, 2H), 4.58 (s, 2H), 3.49 - 3.35 (m, 2H), 2.58 (d, *J* = 12.8 Hz, 5H). ¹³C NMR (101 MHz, DMSO-*d*₆) δ 162.0, 155.4, 146.6, 138.4, 136.4, 134.1, 133.1, 130.2, 130.0, 128.9, 128.8, 128.3, 126.3 (d, *J* = 38.8 Hz, 1C), 126.3 (t*, J* = 5.8 Hz, 1C), 126.1, 125.9, 122.9 (d, *J* = 108.8 Hz, 1C), 121.7, 113.4, 50.9, 48.7, 34.8, 9.5. HRMS (ESI) [M - H] ⁻, theoretically calculated for C₂₆H₂₁NO₅F₃S: 516.1093, found 516.1095.

### Example 66

### 3-methyl-5-(N-(2-nitrobenzyl)-N-phenethylsulfamoyl)benzofuran-2-carboxylic acid (F27-S19)

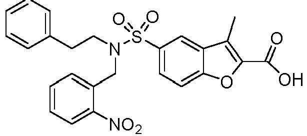

In accordance with the preparation method of 5-(*N*-benzyl-*N*-phenethylsulfamoyl)-3-methylbenzofuran-2-carboxylic acid (F27), this example was implemented under the same condition except that M4-19 was used instead of ethyl 5-(N-benzyl-N-phenethylsulfamoyl)-3-methylbenzofuran-2-carboxylate (F27-S8), and lithium hydroxide was used instead of sodium hydroxide. 31 mg of yellow solid was obtained, with a yield of 63%. M.p. 180 - 181 °C. ¹H NMR (400 MHz, DMSO-*d*₆) δ 8.27 (d, *J* = 1.9 Hz, 1H), 8.02 (d, *J* = 8.1 Hz, 1H), 7.95 (dd, *J* = 8.7, 1.9 Hz, 1H), 7.86 (d, *J* = 8.8 Hz, 1H), 7.71 (q, *J* = 3.6, 3.1 Hz, 2H), 7.55 (td, *J* = 7.0, 5.6, 3.1 Hz, 1H), 7.25 - 7.10 (m, 3H), 7.05 (d, *J* = 7.0 Hz, 2H), 4.77 (s, 2H), 3.54 - 3.34 (m, 2H), 2.61 (d, *J* = 21.8 Hz, 5H). ¹³C NMR (101 MHz, DMSO-*d*₆) δ 161.5, 155.6, 148.5, 138.5, 134.4, 134.0, 133.2, 130.3, 129.9, 129.1, 129.0, 128.8, 126.8, 126.4, 125.1, 122.1, 113.6, 51.2, 49.3, 34.8, 9.5. HRMS (ESI) [M - H] ⁻, theoretically calculated for C₂₅H₂₁N₂O₇S: 493.1069, found 493.1070.

### Example 67

### 3-methyl-5-(N-(3-fluorobenzyl)-N-phenethylsulfamoyl)benzofuran-2-carboxylic acid (F27-S20)

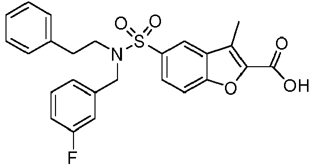

In accordance with the preparation method of 5-(*N*-benzyl-*N*-phenethylsulfamoyl)-3-methylbenzofuran-2-carboxylic acid (F27), this example was implemented under the same condition except that M4-20 was used instead of ethyl 5-(N-benzyl-N-phenethylsulfamoyl)-3-methylbenzofuran-2-carboxylate (F27-S8). 85 mg of white solid was obtained, with a yield of 91%. M.p. > 380 °C (for decomposition). ¹H NMR (400 MHz, DMSO-*d*₆) δ 8.08 (s, 1H), 7.80 (d, *J* = 8.7 Hz, 1H), 7.72 (d, *J* = 8.7 Hz, 1H), 7.44 - 7.34 (m, 1H), 7.19 (d, *J* = 7.4 Hz, 3H), 7.12 (dt, *J=* 9.0, 6.5 Hz, 3H), 7.00 (d, *J=* 6.9 Hz, 2H), 4.42 (s, 2H), 3.37 - 3.28 (m, 2H), 2.61 - 2.54 (m, 2H), 2.52 (s, 3H). ¹³C NMR (101 MHz, DMSO-*d*₆) δ 163.1, 162.6 (d, *J* = 244.6 Hz, 1C), 154.7, 152.0, 140.8 (d, *J* = 7.2 Hz, 1C), 138.7, 133.8, 131.1, 130.8 (d, *J* = 8.4 Hz, 1C), 129.0, 128.8, 126.7, 124.6 (d, *J* = 2.6 Hz, 1C), 124.2, 120.6, 116.9, 115.2 (d, *J* = 21.7 Hz, 1C), 114.7 (d, *J* = 21.0 Hz, 1C), 112.7, 51.3, 50.1, 40.4, 34.7, 9.5. ¹⁹F NMR (376 MHz, DMSO-*d*₆) δ - 113.23. HRMS (ESI) [M - H] ⁻, theoretically calculated for C₂₅H₂₁NO₅FS: 466.1124, found 466.1127.

### Example 68

### 3-methyl-5-(N-(3-chlorobenzyl)-N-phenethylsulfamoyl)benzofuran-2-carboxylic acid (F27-S21)

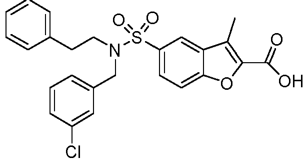

In accordance with the preparation method of 5-(*N*-benzyl-*N*-phenethylsulfamoyl)-3-methylbenzofuran-2-carboxylic acid (F27), this example was implemented under the same condition except that M4-21 was used instead of ethyl 5-(N-benzyl-N-phenethylsulfamoyl)-3-methylbenzofuran-2-carboxylate (F27-S8). 82 mg of white solid was obtained, with a yield of 85%. M.p. > 380 °C (for decomposition). ¹H NMR (400 MHz, DMSO-*d*₆) δ 8.07 (d, *J* = 2.0 Hz, 1H), 7.79 (dd, *J* = 8.6, 2.0 Hz, 1H), 7.71 (d, *J* = 8.7 Hz, 1H), 7.43 - 7.27 (m, 4H), 7.26 - 7.10 (m, 3H), 7.01 (d, *J* = 6.8 Hz, 2H), 4.41 (s, 2H), 3.40 - 3.27 (m, 2H), 2.62 - 2.54 (m, 2H), 2.52 (s, 3H). ¹³C NMR (101 MHz, DMSO-*d*₆) δ 163.2, 154.7, 151.9, 140.4, 138.7, 133.8, 133.5, 131.1, 130.7, 129.0, 128.8, 128.3, 127.9, 127.2, 126.8, 124.2, 120.6, 116.9, 112.8, 51.2, 50.2, 34.8, 9.5. HRMS (ESI) [M - H] ⁻, theoretically calculated for C₂₅H₂₁NO₅SCl: 482.0829, found 482.0829.

### Example 69

### 3-methyl-5-(N-(3-bromobenzyl)-N-phenethylsulfamoyl)benzofuran-2-carboxylic acid (F27-S22)

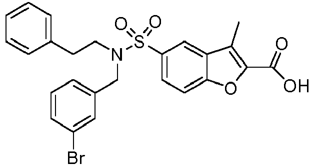

In accordance with the preparation method of 5-(N-benzyl-N-phenethylsulfamoyl)-3-methylbenzofuran-2-carboxylic acid (F27), this example was implemented under the same condition except that M4-22 was used instead of ethyl 5-(N-benzyl-N-phenethylsulfamoyl)-3-methylbenzofuran-2-carboxylate (F27-S8). 93 mg of white solid was obtained, with a yield of 99%. M.p. > 380 °C (for decomposition). ¹H NMR (400 MHz, DMSO-*d*₆) δ 8.09 (s, 1H), 7.81 (d, *J* = 8.7 Hz, 1H), 7.72 (d, *J* = 8.7 Hz, 1H), 7.50 - 7.43 (m, 2H), 7.38 - 7.27 (m, 2H), 7.20 (t, *J* = 7.2 Hz, 2H), 7.14 (dd, *J* = 8.7, 5.8 Hz, 1H), 7.01 (d, *J* = 6.9 Hz, 2H), 4.41 (s, 2H), 3.37 - 3.28 (m, 2H), 2.62 - 2.54 (m, 2H), 2.52 (s, 3H). ¹³C NMR (101 MHz, DMSO-*d*₆) δ 162.5, 154.8, 140.6, 138.7, 133.9, 131.3, 131.0, 131.0, 130.8, 129.0, 128.8, 127.6, 126.8, 124.5, 122.1, 120.7, 112.8, 51.1, 50.2, 34.8, 9.5. HRMS (ESI) [M - H] ⁻, theoretically calculated for C₂₅H₂₁NO₅SBr: 526.0324, found 526.0326.

### Example 70

### 3-methyl-5-(N-(3-methylbenzyl)-N-phenethylsulfamoyl)benzofuran-2-carboxylic acid (F27-S23)

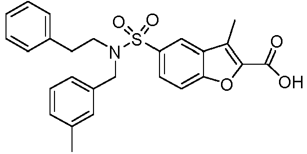

In accordance with the preparation method of 5-(N-benzyl-N-phenethylsulfamoyl)-3-methylbenzofuran-2-carboxylic acid (F27), this example was implemented under the same condition except that M4-23 was used instead of ethyl 5-(N-benzyl-N-phenethylsulfamoyl)-3-methylbenzofuran-2-carboxylate (F27-S8). 93 mg of white solid was obtained, with a yield of 99%. M.p. > 380 °C (for decomposition). ¹H NMR (400 MHz, DMSO-*d*₆) δ 8.08 (d, *J* = 1.7 Hz, 1H), 7.81 (dd, *J* = 8.7, 1.9 Hz, 1H), 7.73 (d, *J* = 8.7 Hz, 1H), 7.21 (q, *J* = 7.3 Hz, 3H), 7.16 - 7.06 (m, 3H), 7.04 (s, 1H), 6.98 (d, *J* = 6.9 Hz, 2H), 4.36 (s, 2H), 3.36 - 3.23 (m, 2H), 2.54 (d, *J* = 9.2 Hz, 5H), 2.23 (s, 3H). ¹³C NMR (101 MHz, DMSO-*d*₆) δ 162.8, 154.8, 138.8, 138.0, 137.2, 134.3, 130.9, 129.3, 129.0, 128.8, 128.8, 128.6, 126.7, 125.9, 124.5, 120.7, 112.8, 51.7, 49.7, 34.7, 21.4, 9.5. HRMS (ESI) [M - H] ⁻, theoretically calculated for C₂₆H₂₄NO₅S: 462.1375, found 462.1376.

### Example 71

### 3-methyl-5-(N-(3-trifluoromethylbenzyl)-N-phenethylsulfamoyl)benzofuran-2-carboxylic acid (F27-S24)

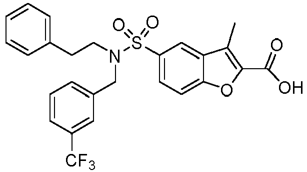

In accordance with the preparation method of 5-(N-benzyl-N-phenethylsulfamoyl)-3-methylbenzofuran-2-carboxylic acid (F27), this example was implemented under the same condition except that M4-24 was used instead of ethyl 5-(N-benzyl-N-phenethylsulfamoyl)-3-methylbenzofuran-2-carboxylate (F27-S8). 89 mg of white solid was obtained, with a yield of 86%. M.p. 188 - 189 °C. ¹H NMR (400 MHz, DMSO-*d*₆) δ 8.14 (s, 1H), 7.85 (d, *J* = 8.7 Hz, 1H), 7.75 (d, *J* = 8.7 Hz, 1H), 7.60 (dq, *J* = 15.2, 7.4 Hz, 4H), 7.16 (dq, *J* = 14.3, 7.1 Hz, 3H), 7.00 (d, *J* = 6.8 Hz, 2H), 4.52 (s, 2H), 3.45 - 3.31 (m, 2H), 2.64 - 2.56 (m, 2H), 2.54 (s, 3H). ¹³C NMR (101 MHz, DMSO-*d*₆) δ 162.6, 155.0, 139.4, 138.6, 134.0, 132.7, 130.7, 129.9, 129.6 (d, *J* = 31.7 Hz, 1C), 129.0, 128.7, 126.7, 125.9, 125.0 (d, *J* = 4.2 Hz, 1C), 124.9, 124.7, 123.2, 121.0, 113.0, 51.3, 50.4, 34.8, 9.5. ¹⁹F NMR (376 MHz, DMSO-*d*₆) δ -61.16. HRMS (ESI) [M - H] ⁻, theoretically calculated for C₂₆H₂₁NO₅F₃S: 516.1093, found 516.1095.

### Example 72

### 3-methyl-5-(N-(3-nitrobenzyl)-N-phenethylsulfamoyl)benzofuran-2-carboxylic acid (F27-S25)

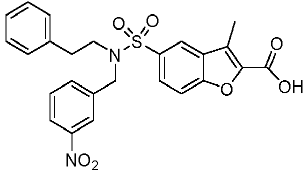

In accordance with the preparation method of 5-(N-benzyl-N-phenethylsulfamoyl)-3-methylbenzofuran-2-carboxylic acid (F27), this example was implemented under the same condition except that M4-25 was used instead of ethyl 5-(N-benzyl-N-phenethylsulfamoyl)-3-methylbenzofuran-2-carboxylate (F27-S8), lithium hydroxide was used instead of sodium hydroxide. 51 mg of white solid was obtained, with a yield of 52%. M.p. > 380 °C (for decomposition). ¹H NMR (400 MHz, DMSO-*d*₆) δ 13.79 (s, 1H), 8.31 (d, *J* = 1.5 Hz, 1H), 8.16 - 8.06 (m, 2H), 7.96 (dd, *J* = 8.8, 1.8 Hz, 1H), 7.85 (d, *J* = 8.8 Hz, 1H), 7.78 (d, *J* = 7.7 Hz, 1H), 7.62 (t, *J* = 7.9 Hz, 1H), 7.23 - 7.06 (m, 3H), 7.01 (d, *J* = 6.9 Hz, 2H), 4.57 (s, 2H), 3.52 - 3.34 (m, 2H), 2.61 (d, *J* = 21.9 Hz, 5H). ¹³C NMR (101 MHz, DMSO-*d*₆) δ 161.2, 155.6, 148.2, 144.0, 140.2, 138.5, 135.0, 134.8, 130.3, 129.7, 129.0, 128.7, 126.7, 125.1, 123.0, 122.8, 122.2, 113.6, 51.2, 50.5, 34.8, 9.5. HRMS (ESI) [M - H] ⁻, theoretically calculated for C₂₅H₂₁N₂O₇S: 493.1069, found 493.1065.

### Example 73

### 3-methyl-5-(N-([1,1'-biphenyl]-3-ylmethyl)-N-phenethylsulfamoyl)benzofuran-2-carboxylic acid (F27-S26)

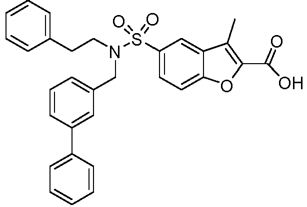

In accordance with the preparation method of 5-(N-benzyl-N-phenethylsulfamoyl)-3-methylbenzofuran-2-carboxylic acid (F27), this example was implemented under the same condition except that M4-26 was used instead of ethyl 5-(N-benzyl-N-phenethylsulfamoyl)-3-methylbenzofuran-2-carboxylate (F27-S8). 106 mg of white solid was obtained, with a yield of 95%. M.p. > 380 °C (for decomposition). ¹H NMR (400 MHz, DMSO-*d*₆) δ 8.10 (s, 1H), 7.81 (d, *J* = 8.7 Hz, 1H), 7.71 (d, *J* = 8.6 Hz, 1H), 7.57 (d, *J* = 7.8 Hz, 1H), 7.53 - 7.47 (m, 2H), 7.47 - 7.39 (m, 4H), 7.34 (dd, *J* = 11.7, 7.4 Hz, 2H), 7.15 (dq, *J* = 14.4, 7.1 Hz, 3H), 7.00 (d, *J* = 6.8 Hz, 2H), 4.48 (s, 2H), 3.39 - 3.28 (m, 2H), 2.66 - 2.56 (m, 2H), 2.52 (s, 3H). ¹³C NMR (101 MHz, DMSO-*d*₆) δ 163.1, 154.7, 140.8, 140.3, 138.8, 138.1, 134.2, 131.2, 129.5, 129.4, 129.0, 128.8, 128.0, 127.9, 127.0, 127.0, 126.7, 126.4, 124.2, 120.5, 116.7, 112.7, 51.6, 49.7, 34.8, 9.5. HRMS (ESI) [M - H] ⁻, theoretically calculated for C₃₁H₂₆NO₅S: 524.1532, found 524.1522.

### Example 74

### 3-methyl-5-(N-(3-phenoxybenzyl)-N-phenethylsulfamoyl)benzofuran-2-carboxylic acid (F27-S27)

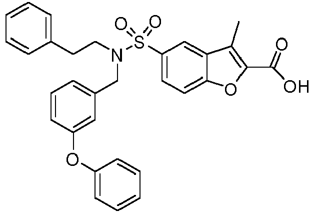

In accordance with the preparation method of 5-(N-benzyl-N-phenethylsulfamoyl)-3-methylbenzofuran-2-carboxylic acid (F27), this example was implemented under the same condition except that M4-27 was used instead of ethyl 5-(N-benzyl-N-phenethylsulfamoyl)-3-methylbenzofuran-2-carboxylate (F27-S8). 99 mg of white solid was obtained, with a yield of 91%. M.p. > 183 -184 °C. ¹H NMR (400 MHz, DMSO-*d*₆) δ 8.18 (s, 1H), 7.86 (d, *J* = 8.7 Hz, 1H), 7.76 (d, *J* = 8.7 Hz, 1H), 7.36 (td, *J* = 8.0, 3.0 Hz, 3H), 7.16 (dq, *J* = 19.7, 7.0 Hz, 5H), 6.96 (dt, *J* = 21.2, 6.5 Hz, 6H), 4.41 (s, 2H), 3.48 - 3.22 (m, 2H), 2.56 (d, *J* = 9.0 Hz, 5H). ¹³C NMR (101 MHz, DMSO-*d*₆) δ 162.1, 157.1, 157.0, 155.2, 139.9, 138.7, 134.7, 130.6, 130.5, 130.3, 129.0, 128.8, 126.7, 125.5, 123.9, 123.8, 121.4, 118.9, 118.9, 118.3, 113.1, 51.5, 50.0, 34.9, 9.5. HRMS (ESI) [M - H] ⁻, theoretically calculated for C₃₁H₂₆NO₆S: 540.1481, found 540.1477.

### Example 75

### 3-methyl-5-(N-(4-fluorobenzyl)-N-phenethylsulfamoyl)benzofuran-2-carboxylic acid (F27-S28)

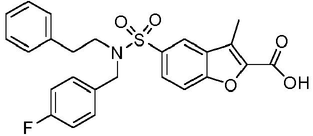

In accordance with the preparation method of 5-(N-benzyl-N-phenethylsulfamoyl)-3-methylbenzofuran-2-carboxylic acid (F27), this example was implemented under the same condition except that M4-28 was used instead of ethyl 5-(N-benzyl-N-phenethylsulfamoyl)-3-methylbenzofuran-2-carboxylate (F27-S8). 81 mg of white solid was obtained, with a yield of 87%. M.p. 188 - 189 °C. ¹H NMR (400 MHz, DMSO-*d*₆) δ 8.17 (s, 1H), 7.87 (d, *J* = 10.5 Hz, 1H), 7.78 (d, *J* = 8.7 Hz, 1H), 7.38 (dd, *J* = 8.4, 5.7 Hz, 2H), 7.16 (dq, *J* = 13.9, 7.2 Hz, 5H), 6.99 (d, *J* = 7.0 Hz, 2H), 4.40 (s, 2H), 3.38 - 3.24 (m, 2H), 2.55 (d, *J* = 11.9 Hz, 5H). ¹³C NMR (101 MHz, DMSO-*d*₆) δ 162.1 (d, *J* = 244.2 Hz, 1C), 162.1, 155.1, 138.7, 134.6, 133.7 (d, *J* = 2.9 Hz, 1C), 130.8 (d, *J* = 8.3 Hz, 1C), 130.7, 130.4, 129.0, 128.8, 126.7, 125.4, 121.3, 115.8, 115.6, 113.1, 51.1, 49.9, 34.8, 9.5. ¹⁹F NMR (376 MHz, DMSO-*d*₆) δ -115.01. HRMS (ESI) [M - H] ⁻, theoretically calculated for C₂₅H₂₁NO₅FS: 466.1124, found 466.1123.

### Example 76

### 3-methyl-5-(N-(4-chlorobenzyl)-N-phenethylsulfamoyl)benzofuran-2-carboxylic acid (F27-S29)

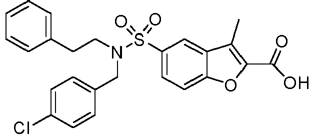

In accordance with the preparation method of 5-(N-benzyl-N-phenethylsulfamoyl)-3-methylbenzofuran-2-carboxylic acid (F27), this example was implemented under the same condition except that M4-29 was used instead of ethyl 5-(N-benzyl-N-phenethylsulfamoyl)-3-methylbenzofuran-2-carboxylate (F27-S8). 89 mg of white solid was obtained, with a yield of 92%. M.p. 172 - 173 °C. ¹H NMR (400 MHz, DMSO-*d*₆) δ 8.25 (s, 1H), 7.93 (d, *J* = 8.8 Hz, 1H), 7.83 (d, *J* = 8.8 Hz, 1H), 7.37 (q, *J* = 8.5 Hz, 4H), 7.19 (t, *J* = 7.3 Hz, 2H), 7.13 (t, *J* = 7.2 Hz, 1H), 7.01 (d, *J* = 7.1 Hz, 2H), 4.42 (s, 2H), 3.41 - 3.26 (m, 2H), 2.58 (d, *J* = 4.6 Hz, 5H). ¹³C NMR (101 MHz, DMSO-*d*₆) δ 161.3, 155.5, 144.4, 138.6, 136.6, 135.1, 132.6, 130.5, 129.7, 129.0, 128.9, 128.8, 126.7, 126.5, 124.6, 122.0, 113.5, 51.1, 50.1, 34.8, 9.5. HRMS (ESI) [M - H] ⁻, theoretically calculated for C₂₅H₂₁NO₅SCl: 482.0829, found 482.0829.

### Example 77

### 3-methyl-5-(N-(4-bromobenzyl)-N-phenethylsulfamoyl)benzofuran-2-carboxylic acid (F27-S30)

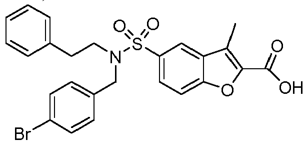

In accordance with the preparation method of 5-(N-benzyl-N-phenethylsulfamoyl)-3-methylbenzofuran-2-carboxylic acid (F27), this example was implemented under the same condition except that M4-30 was used instead of ethyl 5-(N-benzyl-N-phenethylsulfamoyl)-3-methylbenzofuran-2-carboxylate (F27-S8). 227 mg of white solid was obtained, with a yield of 85%. M.p. > 380 °C (for decomposition). ¹H NMR (400 MHz, DMSO-*d*₆) δ 8.15 (s, 1H), 7.93 - 7.69 (m, 2H), 7.42 (dd, *J* = 89.8, 8.2 Hz, 4H), 7.23 - 7.10 (m, 3H), 7.00 (d, *J* = 7.2 Hz, 2H), 4.39 (s, 2H), 3.39 - 3.27 (m, 2H), 2.56 (s, 5H). ¹³C NMR (101 MHz, DMSO-*d*₆) δ 162.0, 154.6, 148.2, 138.2, 136.7, 133.8, 131.3, 130.4, 130.0, 128.5, 128.3, 126.3, 124.7, 120.7, 120.7, 119.7, 112.6, 50.8, 49.6, 34.3, 9.1. HRMS (ESI) [M - H] ⁻, theoretically calculated for C₂₅H₂₁BrNO₅S: 526.0329, found 506.0323.

### Example 78

### 3-methyl-5-(N-(4-methylbenzyl)-N-phenethylsulfamoyl)benzofuran-2-carboxylic acid (F27-S31)

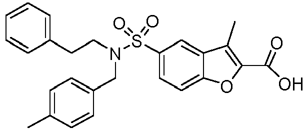

In accordance with the preparation method of 5-(N-benzyl-N-phenethylsulfamoyl)-3-methylbenzofuran-2-carboxylic acid (F27), this example was implemented under the same condition except that M4-31 was used instead of ethyl 5-(N-benzyl-N-phenethylsulfamoyl)-3-methylbenzofuran-2-carboxylate (F27-S8). 63 mg of white solid was obtained, with a yield of 68%. M.p. > 380 °C (for decomposition). ¹H NMR (400 MHz, DMSO-*d*₆) δ 8.15 (d, *J* = 1.9 Hz, 1H), 7.86 (dd, *J* = 8.6, 1.9 Hz, 1H), 7.77 (d, *J* = 8.7 Hz, 1H), 7.26 - 7.07 (m, 7H), 6.98 (d, *J* = 7.2 Hz, 2H), 4.36 (s, 2H), 3.28 (t, *J* = 8.0 Hz, 2H), 2.61 - 2.51 (m, 5H), 2.27 (s, 3H). ¹³C NMR (101 MHz, DMSO-*d*₆) δ 162.4, 155.0, 148.3, 138.8, 137.2, 134.8, 134.2, 130.4, 129.5, 128.9, 128.8, 126.7, 125.3, 121.1, 120.5, 113.1, 51.5, 49.6, 34.8, 21.2, 9.5. HRMS (ESI) [M - H] ⁻, theoretically calculated for C₂₆H₂₄NO₅S: 462.1375, found 462.1378.

### Example 79

### 3-methyl-5-(N-(4-(trifluoromethyl)benzyl)-N-phenethylsulfamoyl)benzofuran-2-carboxylic acid (F27-S32)

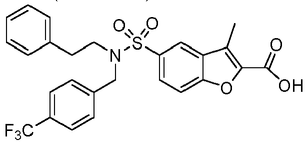

In accordance with the preparation method of 5-(N-benzyl-N-phenethylsulfamoyl)-3-methylbenzofuran-2-carboxylic acid (F27), this example was implemented under the same condition except that M4-32 was used instead of ethyl 5-(N-benzyl-N-phenethylsulfamoyl)-3-methylbenzofuran-2-carboxylate (F27-S8). 72 mg of white solid was obtained, with a yield of 70%. M.p. > 192 -193 °C. ¹H NMR (400 MHz, DMSO-*d*₆) δ 8.33 - 8.19 (m, 1H), 7.94 (dd, *J=* 8.7, 1.9 Hz, 1H), 7.83 (d, *J* = 8.8 Hz, 1H), 7.68 (d, *J* = 7.9 Hz, 2H), 7.55 (d, *J* = 7.9 Hz, 2H), 7.14 (dq, *J* = 14.1, 7.3 Hz, 3H), 7.00 (d, *J* = 7.3 Hz, 2H), 4.53 (s, 2H), 3.37 (t, *J* = 7.8 Hz, 2H), 2.59 (d, J= 10.2 Hz, 5H). ¹³C NMR (101 MHz, DMSO-*d*₆) δ 161.6, 155.4, 145.2, 142.7, 138.6, 134.7, 129.9, 129.2, 129.0, 128.7, 126.7, 126.2, 126.1, 125.7 (d, *J* = 3.7 Hz, 1C), 123.5 (d, *J* = 34.9 Hz, 1C), 121.9, 113.4, 51.5, 50.5, 34.8, 9.5. ¹⁹F NMR (376 MHz, DMSO-*d*₆) δ -60.94. HRMS (ESI) [M - H] ⁻, theoretically calculated for C₂₆H₂₁NO₅SF₃: 516.1093, found 516.1096.

### Example 80

### 3-methyl-5-(N-(4-carbamoylbenzyl)-N-phenethylsulfamoyl)benzofuran-2-carboxylic acid (F27-S33)

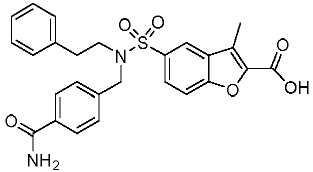

In accordance with the preparation method of 5-(N-benzyl-N-phenethylsulfamoyl)-3-methylbenzofuran-2-carboxylic acid (F27), this example was implemented under the same condition except that M4-33 was used instead of ethyl 5-(N-benzyl-N-phenethylsulfamoyl)-3-methylbenzofuran-2-carboxylate (F27-S8). 103 mg of white solid was obtained, with a yield of 43%. M.p. > 380 °C (for decomposition). ¹H NMR (400 MHz, DMSO-*d*₆) δ 13.56 (s, 1H), 8.29 (s, 1H), 8.03 - 7.89 (m, 2H), 7.86 (d, *J* = 8.7 Hz, 2H), 7.50 - 7.41 (m, 1H), 7.38 (d, *J* = 11.8 Hz, 1H), 7.16 (dq, *J* = 14.1, 7.1, 6.6 Hz, 3H), 7.01 (d, *J* = 7.8 Hz, 2H), 4.50 (d, *J* = 9.7 Hz, 2H), 3.35 (q, *J* = 7.5 Hz, 2H), 2.58 (s, 5H). ¹³C NMR (101 MHz, DMSO-*d*₆) δ 168.0, 163.7, 154.9, 149.8, 140.9, 138.7, 134.2, 133.9, 130.7, 128.9, 128.8, 128.4, 128.1, 126.7, 124.9, 121.0, 119.2, 113.0, 51.5, 50.1, 34.8, 9.5. HRMS (ESI) [M - H]⁻, theoretically calculated for C₂₆H₂₃N₂O₆S: 491.1282, found 491.1278.

### Example 81

### 3-methyl-5-(N-(4-(trifluoromethoxy)benzyl)-N-phenethylsulfamoyl)benzofuran-2-carboxylic acid (F27-S34)

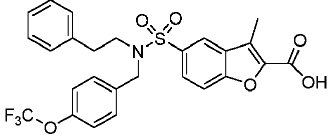

In accordance with the preparation method of 5-(N-benzyl-N-phenethylsulfamoyl)-3-methylbenzofuran-2-carboxylic acid (F27), this example was implemented under the same condition except that M4-34 was used instead of ethyl 5-(N-benzyl-N-phenethylsulfamoyl)-3-methylbenzofuran-2-carboxylate (F27-S8). 227 mg of white solid was obtained, with a yield of 85%. M.p. 216 -217 °C. ¹H NMR (400 MHz, DMSO-*d*₆) δ 8.20 (s, 1H), 7.92 - 7.74 (m, 2H), 7.40 (dd, *J* = 60.2, 8.2 Hz, 4H), 7.21 - 7.08 (m, 3H), 6.98 (d, *J* = 7.1 Hz, 2H), 4.45 (s, 2H), 3.34 (t, *J* = 7.8 Hz, 2H), 2.65 - 2.53 (m, 5H). ¹³C NMR (101 MHz, DMSO-*d*₆) δ 162.4, 155.1, 148.1, 138.6, 137.1, 134.4, 130.5, 130.4, 128.9, 128.7, 126.7, 125.4, 121.8, 121.4, 121.3, 120.8, 113.1, 51.2, 50.2, 34.8, 9.5. ¹⁹F NMR (376 MHz, DMSO) δ -56.83. HRMS (ESI) [M - H] ⁻, theoretically calculated for C₂₆H₂₁F₃NO₆S: 532.1047, found 532.1038.

### Example 82

### 3-methyl-5-(N-(4-(trifluoromethylthio)benzyl)-N-phenethylsulfamoyl)benzofuran-2-carboxylic acid (F27-S35)

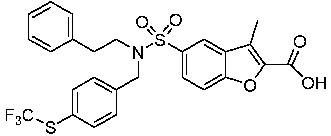

In accordance with the preparation method of 5-(N-benzyl-N-phenethylsulfamoyl)-3-methylbenzofuran-2-carboxylic acid (F27), this example was implemented under the same condition except that M4-35 was used instead of ethyl 5-(N-benzyl-N-phenethylsulfamoyl)-3-methylbenzofuran-2-carboxylate (F27-S8). 192 mg of white solid was obtained, with a yield of 70%. M.p. 165 -166 °C. ¹H NMR (400 MHz, DMSO-*d*₆) δ 8.26 (s, 1H), 7.92 (dd, *J* = 8.7, 1.8 Hz, 1H), 7.82 (d, *J* = 8.8 Hz, 1H), 7.70 (d, *J* = 7.9 Hz, 2H), 7.51 (d, *J* = 7.9 Hz, 2H), 7.17 (dt, *J* = 13.7, 6.9 Hz, 3H), 7.00 (d, *J* = 7.2 Hz, 2H), 4.50 (s, 2H), 3.36 (t, *J* = 7.9 Hz, 2H), 2.65 - 2.54 (m, 5H). ¹³C NMR (101 MHz, DMSO-*d*₆) δ 161.7, 155.4, 141.8, 138.6, 136.7, 134.5, 131.6, 130.0, 130.0, 129.0, 128.7, 128.5, 126.7, 126.0, 122.3, 121.8, 113.3, 51.4, 50.5, 34.9, 9.5. ¹⁹F NMR (376 MHz, DMSO-*d*₆) δ -42.23. HRMS (ESI) [M - H] ⁻, theoretically calculated for C₂₆H₂₁F₃NO₅S₂: 548.0819, found 548.0805.

### Example 83

### 3-methyl-5-(N-(4-isopropylbenzyl)-N-phenethylsulfamoyl)benzofuran-2-carboxylic acid (F27-S36)

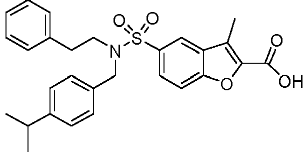

In accordance with the preparation method of 5-(N-benzyl-N-phenethylsulfamoyl)-3-methylbenzofuran-2-carboxylic acid (F27), this example was implemented under the same condition except that M4-36 was used instead of ethyl 5-(N-benzyl-N-phenethylsulfamoyl)-3-methylbenzofuran-2-carboxylate (F27-S8). 220 mg of white solid was obtained, with a yield of 88%. M.p. 206 - 207 °C. ¹H NMR (400 MHz, DMSO-*d*₆) δ 13.74 (s, 1H), 8.26 (d, *J* = 1.9 Hz, 1H), 7.93 (dd, *J* = 8.8, 1.9 Hz, 1H), 7.84 (d, *J* = 8.8 Hz, 1H), 7.19 (dq, *J* = 16.2, 9.2, 8.7 Hz, 7H), 7.02 - 6.94 (m, 2H), 4.38 (s, 2H), 3.34 - 3.27 (m, 2H), 2.85 (h, *J* = 6.8 Hz, 1H), 2.58 (s, 5H), 1.17 (d, *J* = 6.9 Hz, 6H). ¹³C NMR (101 MHz, DMSO-*d*₆) δ 161.1, 155.5, 148.3, 143.8, 138.8, 135.5, 134.5, 129.6, 129.0, 128.8, 128.8, 126.8, 126.7, 126.6, 125.2, 122.0, 113.5, 51.4, 49.6, 34.8, 33.6, 24.3, 9.6. HRMS (ESI) [M - H] ⁻, theoretically calculated for C₂₈H₂₈NO₅S: 490.1694, found 490.1684.

### Example 84

### 3-methyl-5-(N-(4-tert-butylbenzyl)-N-phenethylsulfamoyl)benzofuran-2-carboxylic acid (F27-S37)

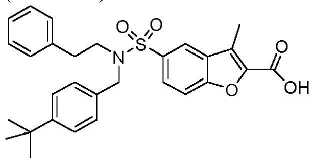

In accordance with the preparation method of 5-(N-benzyl-N-phenethylsulfamoyl)-3-methylbenzofuran-2-carboxylic acid (F27), this example was implemented under the same condition except that M4-37 was used instead of ethyl 5-(N-benzyl-N-phenethylsulfamoyl)-3-methylbenzofuran-2-carboxylate (F27-S8). 212 mg of white solid was obtained, with a yield of 84%. M.p. 206 - 207 °C. ¹H NMR (400 MHz, DMSO-*d*₆) δ 8.25 (s, 1H), 7.97 - 7.77 (m, 2H), 7.34 (d, *J* = 8.0 Hz, 2H), 7.18 (ddd, *J* = 22.9, 15.8, 7.5 Hz, 5H), 6.97 (d, *J* = 6.6 Hz, 2H), 4.39 (s, 2H), 3.36 - 3.27 (m, 2H), 2.58 (s, 5H), 1.25 (s, 9H). ¹³C NMR (101 MHz, DMSO) δ 161.4, 155.4, 150.5, 138.8, 135.4, 134.1, 129.7, 129.0, 128.7, 128.5, 126.7, 126.4, 125.6, 124.5, 121.9, 113.4, 107.1, 51.3, 49.6, 34.8, 34.7, 31.6, 9.6. HRMS (ESI) [M - H] ⁻, theoretically calculated for C₂₉H₃₀NO₅S: 504.1850, found 504.1838.

### Example 85

### 3-methyl-5-(N-(4-acetylbenzyl)-N-phenethylsulfamoyl)benzofuran-2-carboxylic acid (F27-S38)

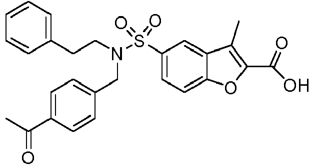

In accordance with the preparation method of 5-(N-benzyl-N-phenethylsulfamoyl)-3-methylbenzofuran-2-carboxylic acid (F27), this example was implemented under the same condition except that M4-38 was used instead of ethyl 5-(N-benzyl-N-phenethylsulfamoyl)-3-methylbenzofuran-2-carboxylate (F27-S8). 48 mg of white solid was obtained, with a yield of 49%. M.p. > 380 °C (for decomposition). ¹H NMR (400 MHz, DMSO-*d*₆) δ 13.75 (s, 1H), 8.28 (d, *J* = 1.9 Hz, 1H), 8.00 - 7.80 (m, 4H), 7.47 (d, *J* = 8.0 Hz, 2H), 7.21 - 7.09 (m, 3H), 7.05 - 6.98 (m, 2H), 4.52 (s, 2H), 3.40 - 3.31 (m, 2H), 2.57 (t, *J* = 5.6 Hz, 8H). ¹³C NMR (101 MHz, DMSO-*d*₆) δ 197.9, 161.1, 155.6, 143.7, 143.1, 138.6, 136.5, 135.1, 129.6, 129.0, 128.8, 128.8, 128.7, 126.7, 126.7, 125.3, 122.1, 113.6, 51.6, 50.3, 34.8, 27.2, 9.5. HRMS (ESI) [M - H] ⁻, theoretically calculated for C₂₇H₂₄NO₆S: 490.1324, found 490.1322.

### Example 86

### 3-methyl-5-(N-(4-carboxybenzyl)-N-phenethylsulfamoyl)benzofuran-2-carboxylic acid (F27-S39)

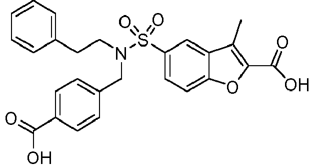

In accordance with the preparation method of 5-(N-benzyl-N-phenethylsulfamoyl)-3-methylbenzofuran-2-carboxylic acid (F27), this example was implemented under the same condition except that M4-39 was used instead of ethyl 5-(N-benzyl-N-phenethylsulfamoyl)-3-methylbenzofuran-2-carboxylate (F27-S8). 105 mg of white solid was obtained, with a yield of 43%. M.p. 254 - 255 °C. ¹H NMR (400 MHz, DMSO-*d*₆) δ 13.36 (s, 2H), 8.27 (d, *J* = 1.9 Hz, 1H), 8.01 - 7.81 (m, 4H), 7.46 (d, *J* = 8.0 Hz, 2H), 7.22 - 7.09 (m, 3H), 7.00 (d, *J* = 7.2 Hz, 2H), 4.51 (s, 2H), 3.42 - 3.28 (m, 2H), 2.57 (s, 5H). ¹³C NMR (101 MHz, DMSO-*d*₆) δ 167.5, 161.2, 155.5, 144.0, 142.7, 138.6, 135.1, 130.4, 129.9, 129.7, 129.0, 128.8, 128.6, 126.7, 126.6, 125.0, 122.1, 113.5, 51.6, 50.4, 34.8, 9.5. HRMS (ESI) [M - H] ⁻, theoretically calculated for C₂₆H₂₂NO₇S: 492.1122, found 491.1115.

### Example 87

### 3-methyl-5-(N-(4-(tert-butoxycarbonyl)benzyl)-N-phenethylsulfamoyl)benzofuran-2-carboxylic acid (F27-S40)

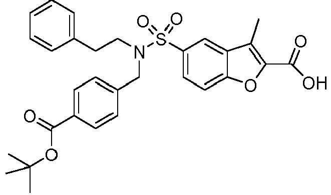

In accordance with the preparation method of 5-(N-benzyl-N-phenethylsulfamoyl)-3-methylbenzofuran-2-carboxylic acid (F27), this example was implemented under the same condition except that M4-40 was used instead of ethyl 5-(N-benzyl-N-phenethylsulfamoyl)-3-methylbenzofuran-2-carboxylate (F27-S8). 200 mg of white solid was obtained, with a yield of 77%. M.p. 188 - 189 °C. ¹H NMR (400 MHz, DMSO-*d*₆) δ 13.61 (s, 1H), 8.21 (s, 1H), 7.88 (dd, *J* = 47.6, 7.6 Hz, 4H), 7.42 (d, *J* = 8.1 Hz, 2H), 7.15 (dt, *J* = 16.9, 7.1 Hz, 3H), 7.01 (d, *J* = 6.2 Hz, 2H), 4.50 (s, 2H), 3.37 (s, 2H), 2.58 (d, *J* = 19.8 Hz, 5H), 1.52 (s, 9H). ¹³C NMR (101 MHz, DMSO) δ 165.1, 161.1, 155.5, 143.8, 142.5, 138.6, 135.2, 131.0, 129.9, 129.5, 129.0, 128.7, 128.6, 126.7, 126.6, 125.1, 122.0, 113.5, 81.1, 51.6, 50.3, 34.9, 28.2, 9.5. HRMS (ESI) [M - H] ⁻, theoretically calculated for C₃₀H₃₀NO₇S: 548.1748, found 548.1738.

### Example 88

### 3-methyl-5-(N-(4-(carboxymethyl)benzyl)-N-phenethylsulfamoyl)benzofuran-2-carboxylic acid (F27-S41)

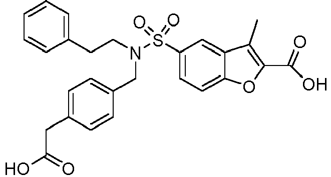

In accordance with the preparation method of 5-(N-benzyl-N-phenethylsulfamoyl)-3-methylbenzofuran-2-carboxylic acid (F27), this example was implemented under the same condition except that M4-41 was used instead of ethyl 5-(N-benzyl-N-phenethylsulfamoyl)-3-methylbenzofuran-2-carboxylate (F27-S8). 95 mg of white solid was obtained, with a yield of 34%. M.p. 182 - 183 °C. ¹H NMR (400 MHz, DMSO-*d*₆) δ 12.92 (s, 2H), 8.29 (d, *J* = 1.9 Hz, 1H), 7.94 (dd, *J* = 8.8, 1.9 Hz, 1H), 7.85 (d, *J* = 8.8 Hz, 1H), 7.20 (ddd, *J* = 42.8, 17.3, 7.5 Hz, 7H), 7.03 - 6.94 (m, 2H), 4.40 (s, 2H), 3.56 (s, 2H), 3.30 (t, *J* = 8.0 Hz, 2H), 2.57 (d, *J* = 14.7 Hz, 5H). ¹³C NMR (101 MHz, DMSO) δ 173.1, 161.1, 155.5, 143.7, 138.7, 135.6, 135.4, 134.9, 130.0, 129.6, 129.0, 128.8, 128.7, 126.7, 125.4, 122.0, 113.5, 51.5, 49.7, 34.8, 9.6. HRMS (ESI) [M - H] ⁻, theoretically calculated for C₂₇H₂₄NO₇S: 507.1352, found 506.1267.

### Example 89

### (E)-3-methyl-5-(N-(4-(2-carboxyvinyl)benzyl)-N-phenethylsulfamoyl)benzofuran-2-carboxylic acid (F27-S42)

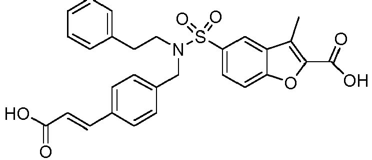

In accordance with the preparation method of 5-(N-benzyl-N-phenethylsulfamoyl)-3-methylbenzofuran-2-carboxylic acid (F27), this example was implemented under the same condition except that M4-42 was used instead of ethyl 5-(N-benzyl-N-phenethylsulfamoyl)-3-methylbenzofuran-2-carboxylate (F27-S8). 87 mg of white solid was obtained, with a yield of 83%. M.p. 270 -271 °C. ¹H NMR (400 MHz, DMSO-*d*₆) δ 13.03 (s, 2H), 8.28 (d, *J* = 1.9 Hz, 1H), 7.96 (dd, *J* = 8.8, 1.9 Hz, 1H), 7.85 (d, *J* = 8.7 Hz, 1H), 7.66 (d, *J* = 7.8 Hz, 2H), 7.57 (d, *J* = 16.0 Hz, 1H), 7.37 (d, *J* = 7.8 Hz, 2H), 7.26 - 7.10 (m, 3H), 7.06 - 6.94 (m, 2H), 6.52 (d, *J* = 16.0 Hz, 1H), 4.46 (s, 2H), 3.48 - 3.23 (m, 3H), 2.58 (s, 5H). ¹³C NMR (101 MHz, DMSO-*d*₆) δ 168.0, 161.1, 155.6, 143.9, 143.7, 139.7, 138.7, 135.3, 134.0, 129.6, 129.1, 129.0, 128.8, 126.7, 126.7, 125.3, 122.1, 119.7, 113.6, 51.5, 50.1, 34.8, 9.5. HRMS (ESI) [M - H] ⁻, theoretically calculated for C₂₈H₂₄NO₇S: 518.1273, found 518.1268.

### Example 90

### 3-methyl-5-(N-(naphthalen-2-ylmethyl)-N-phenethylsulfamoyl)benzofuran-2-carboxylic acid (F27-S43)

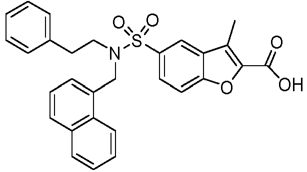

In accordance with the preparation method of 5-(N-benzyl-N-phenethylsulfamoyl)-3-methylbenzofuran-2-carboxylic acid (F27), this example was implemented under the same condition except that M4-43 was used instead of ethyl 5-(N-benzyl-N-phenethylsulfamoyl)-3-methylbenzofuran-2-carboxylate (F27-S8). 94 mg of white solid was obtained, with a yield of 89%. M.p. > 380 °C (for decomposition). ¹H NMR (400 MHz, DMSO-*d*₆) δ 8.48 - 8.30 (m, 2H), 8.06 (dd, *J* = 8.7, 1.9 Hz, 1H), 8.01 - 7.86 (m, 3H), 7.67 - 7.53 (m, 3H), 7.49 (t, *J* = 7.6 Hz, 1H), 7.09 (dq, *J* = 14.0, 7.1 Hz, 3H), 6.86 - 6.70 (m, 2H), 4.86 (s, 2H), 3.24 (t, *J* = 8.2 Hz, 2H), 2.60 (s, 3H), 2.22 (t, *J* = 8.2 Hz, 2H). ¹³C NMR (101 MHz, DMSO-*d*₆) δ 161.4, 155.6, 144.4, 138.5, 134.3, 133.9, 132.0, 131.9, 129.8, 129.3, 129.0, 128.8, 128.7, 128.4, 126.9, 126.8, 126.7, 126.5, 125.7, 124.3, 122.2, 113.5, 51.1, 49.9, 35.3, 9.6. HRMS (ESI) [M - H] ⁻, theoretically calculated for C₂₉H₂₄NO₅S: 498.1375, found 498.1376.

### Example 91

### 3-methyl-5-(N-(quinolin-8-ylmethyl)-N-phenethylsulfamoyl)benzofuran-2-carboxylic acid (F27-S44)

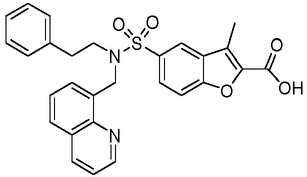

In accordance with the preparation method of 5-(N-benzyl-N-phenethylsulfamoyl)-3-methylbenzofuran-2-carboxylic acid (F27), this example was implemented under the same condition except that M4-44 was used instead of ethyl 5-(N-benzyl-N-phenethylsulfamoyl)-3-methylbenzofuran-2-carboxylate (F27-S8). 103 mg of white solid was obtained, with a yield of 99%. M.p. 190 - 191 °C. ¹H NMR (400 MHz, DMSO-*d*₆) δ 8.88 (s, 1H), 8.36 (d, *J* = 7.9 Hz, 1H), 8.20 (s, 1H), 7.91 (t, *J* = 9.1 Hz, 2H), 7.80 (d, *J* = 5.6 Hz, 2H), 7.68 - 7.48 (m, 2H), 7.12 (dd, *J* = 15.3, 6.9 Hz, 3H), 6.95 (d, *J* = 7.0 Hz, 2H), 5.10 (s, 2H), 3.60 - 3.38 (m, 2H), 2.73 - 2.60 (m, 2H), 2.54 (s, 3H). ¹³C NMR (101 MHz, DMSO-*d*₆) δ 161.1, 155.4, 150.2, 145.8, 143.6, 138.7, 137.2, 135.4, 135.0, 129.8, 129.4, 129.0, 128.7, 128.2, 126.8, 126.7, 125.2, 122.0, 121.9, 113.4, 50.8, 47.7, 35.1, 9.5. HRMS (ESI) [M - H] ⁻, theoretically calculated for C₂₈H₂₃N₂O₅S: 499.1328, found 499.1321.

### Example 92

### 3-methyl-5-(N-(naphthalen-2-ylmethyl)-N-phenethylsulfamoyl)benzofuran-2-carboxylic acid (F27-S45)

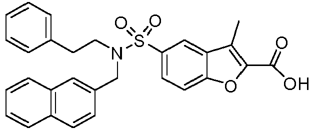

In accordance with the preparation method of 5-(N-benzyl-N-phenethylsulfamoyl)-3-methylbenzofuran-2-carboxylic acid (F27), this example was implemented under the same condition except that M4-45 was used instead of ethyl 5-(N-benzyl-N-phenethylsulfamoyl)-3-methylbenzofuran-2-carboxylate (F27-S8). 101 mg of white solid was obtained, with a yield of 99%. M.p. > 380 °C (for decomposition). ¹H NMR (400 MHz, DMSO-*d*₆) δ 8.09 (s, 1H), 7.89 (d, *J* = 8.1 Hz, 2H), 7.84 (d, *J* = 8.8 Hz, 3H), 7.73 (d, *J* = 8.6 Hz, 1H), 7.58 - 7.43 (m, 3H), 7.13 (dq, *J* = 14.2, 6.9 Hz, 3H), 6.95 (d, *J* = 6.7 Hz, 2H), 4.57 (s, 2H), 3.46 (s, 3H), 3.38 - 3.29 (m, 2H), 2.62 - 2.54 (m, 2H). ¹³C NMR (101 MHz, DMSO-*d*₆) δ 163.2, 154.7, 152.3, 138.8, 135.0, 134.0, 133.2, 132.9, 131.2, 128.9, 128.8, 128.6, 128.1, 128.1, 127.4, 126.8, 126.7, 126.7, 126.5, 124.2, 120.5, 116.6, 112.7, 52.0, 49.9, 34.8, 9.5. HRMS (ESI) [M - H] ⁻, theoretically calculated for C₂₉H₂₄NO₅S: 498.1375, found 498.1379.

### Example 93

### 3-methyl-5-(N-(2,6-difluorobenzyl)-N-phenethylsulfamoyl)benzofuran-2-carboxylic acid (F27-S46)

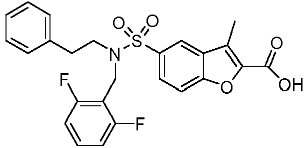

In accordance with the preparation method of 5-(N-benzyl-N-phenethylsulfamoyl)-3-methylbenzofuran-2-carboxylic acid (F27), this example was implemented under the same condition except that M4-46 was used instead of ethyl 5-(N-benzyl-N-phenethylsulfamoyl)-3-methylbenzofuran-2-carboxylate (F27-S8). 89 mg of white solid was obtained, with a yield of 92%. M.p. 180- 181. ¹H NMR (400 MHz, DMSO-*d*₆) δ 8.09 (d, *J* = 1.9 Hz, 1H), 7.85 (dd, *J=* 8.8, 1.9 Hz, 1H), 7.78 (d, *J* = 8.8 Hz, 1H), 7.40 (ddd, *J* = 15.0, 8.5, 6.6 Hz, 1H), 7.24 (t, *J=* 7.3 Hz, 2H), 7.16 (ddd, *J* = 7.3, 5.1, 1.3 Hz, 1H), 7.12 -7.00 (m, 4H), 4.49 (s, 2H), 3.37 - 3.26 (m, 2H), 2.72 - 2.63 (m, 2H), 2.55 (s, 3H). ¹³C NMR (101 MHz, DMSO-*d*₆) δ 162.0, 161.6 (dd, *J* = 249.6, 8.0 Hz, 1C), 155.3, 146.0, 134.4, 131.5 (t, *J=* 10.6 Hz, 1C), 129.9, 128.9, 128.9, 126.8, 125.9, 122.8, 121.5, 113.2, 112.5, 112.2 (dd, *J* = 18.9, 120 Hz, 1C), 112.1 (d, *J* = 25.1 Hz, 1C), 50.4, 35.2, 9.5. ¹⁹F NMR (376 MHz, DMSO-*d*₆) δ -113.36. HRMS (ESI) [M - H] ⁻, theoretically calculated for C₂₅H₂₀NO₅F₂S: 484.1030, found 484.1036.

### Example 94

### 3-methyl-5-(N-(2-fluoro-6-chlorobenzyl)-N-phenethylsulfamoyl)benzofuran-2-carboxylic acid (F27-S47)

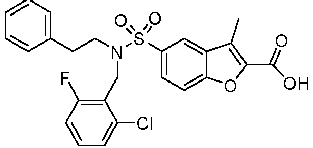

In accordance with the preparation method of 5-(N-benzyl-N-phenethylsulfamoyl)-3-methylbenzofuran-2-carboxylic acid (F27), this example was implemented under the same condition except that M4-47 was used instead of ethyl 5-(N-benzyl-N-phenethylsulfamoyl)-3-methylbenzofuran-2-carboxylate (F27-S8). 93 mg of white solid was obtained, with a yield of 94%. M.p. 195 - 196. ¹H NMR (400 MHz, DMSO-*d*₆) δ 8.18 (d, *J* = 1.9 Hz, 1H), 7.92 (dd, *J* = 8.8, 2.0 Hz, 1H), 7.83 (d, *J* = 8.8 Hz, 1H), 7.39 (td, *J* = 8.2, 6.1 Hz, 1H), 7.30 (d, *J* = 8.0 Hz, 1H), 7.26 - 7.12 (m, 4H), 6.98 (d, *J* = 7.0 Hz, 2H), 4.55 (s, 2H), 3.35 - 3.23 (m, 2H), 2.56 (s, 5H). ¹³C NMR (101 MHz, DMSO-*d*₆) δ 162.1 (d, *J* = 250.9 Hz, 1C), 161.2, 155.5, 143.9, 138.5, 135.6 (d, *J* = 5.3 Hz, 1C), 134.5, 131.6 (d, *J* = 10.0 Hz, 1C), 129.5, 128.9 (d, *J* = 2.2 Hz, 1C), 126.8, 126.7, 126.3 (d, *J* = 3.1 Hz, 1C), 125.0, 122.0, 121.8 (d, *J* = 16.9 Hz, 1C), 115.2 (d, *J* = 22.6 Hz, 1C), 113.5, 50.5, 44.3 (d, *J* = 1.9 Hz, 1C), 35.7, 9.5. ¹⁹F NMR (376 MHz, DMSO-*d*₆) δ -111.00. HRMS (ESI) [M - H] ⁻, theoretically calculated for C₂₅H₂₀NO₅FSCl: 500.0735, found 500.0743.

### Example 95

### 3-methyl-5-(N-(2-fluoro-6-(trifluoromethyl)benzyl)-N-phenethylsulfamoyl)benzofuran-2-carboxylic acid (F27-S48)

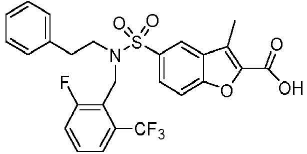

In accordance with the preparation method of 5-(N-benzyl-N-phenethylsulfamoyl)-3-methylbenzofuran-2-carboxylic acid (F27), this example was implemented under the same condition except that M4-48 was used instead of ethyl 5-(N-benzyl-N-phenethylsulfamoyl)-3-methylbenzofuran-2-carboxylate (F27-S8). 98 mg of white solid was obtained, with a yield of 91%. M.p. 99 - 100. ¹H NMR (400 MHz, DMSO-*d*₆) δ 8.17 (s, 1H), 7.99 - 7.76 (m, 2H), 7.57 (dd, *J* = 40.4, 6.1 Hz, 3H), 7.17 (dt, *J* = 17.0, 7.2 Hz, 3H), 6.90 (d, *J* = 7.5 Hz, 2H), 4.60 (s, 2H), 3.20 (t, *J* = 8.1 Hz, 2H), 2.56 (s, 3H), 2.50 - 2.36 (m, 2H). ¹³C NMR (101 MHz, DMSO-*d*₆) δ 162.3 (d, J= 250.0 Hz, 1C), 161.8, 155.4, 146.1, 138.4, 133.8, 131.6 (d, *J* = 9.7 Hz, 1C), 130.2 (d, *J* = 33.4 Hz, 1C), 130.0, 128.9, 128.7, 126.8, 126.1, 125.2, 122.9 (d, *J* = 41.2 Hz, 1C), 122.5, 122.1 (d, *J=* 14.6 Hz, 1C), 121.8, 120.8 (d, *J* = 23.3 Hz, 1C), 113.3, 50.3, 43.7, 35.7, 9.4. ¹⁹F NMR (376 MHz, DMSO-*d*₆) δ -56.89, -110.29. HRMS (ESI) [M - H] ⁻, theoretically calculated for C₂₆H₂₀NO₅F₄S: 534.0998, found 534.0999.

### Example 96

### 3-methyl-5-(N-(2,6-dichlorobenzyl)-N-phenethylsulfamoyl)benzofuran-2-carboxylic acid (F27-S49)

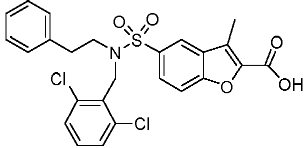

In accordance with the preparation method of 5-(N-benzyl-N-phenethylsulfamoyl)-3-methylbenzofuran-2-carboxylic acid (F27), this example was implemented under the same condition except that M4-49 was used instead of ethyl 5-(N-benzyl-N-phenethylsulfamoyl)-3-methylbenzofuran-2-carboxylate (F27-S8). 84 mg of white solid was obtained, with a yield of 81%. M.p. 176 - 177 °C. ¹H NMR (400 MHz, DMSO-*d*₆) δ 8.21 (s, 1H), 7.94 (d, *J* = 8.6 Hz, 1H), 7.84 (d, *J* = 8.7 Hz, 1H), 7.46 (d, *J* = 7.9 Hz, 2H), 7.42 - 7.32 (m, 1H), 7.16 (dt, *J* = 23.5, 6.4 Hz, 3H), 6.89 (d, *J=* 7.0 Hz, 2H), 4.62 (s, 2H), 3.33 - 3.13 (m, 2H), 2.57 (s, 3H), 2.46 (d, *J* = 7.9 Hz, 2H). ¹³C NMR (101 MHz, DMSO-*d*₆) δ 161.5, 155.5, 145.0, 138.4, 136.7, 133.8, 131.5, 131.0, 129.7, 129.5, 128.9, 128.8, 126.8, 126.5, 124.0, 122.0, 113.4, 50.3, 48.5, 36.3, 9.5. HRMS (ESI) [M - H] ⁻, theoretically calculated for C₂₅H₂₀NO₅SCl₂: 516.0439, found 516.0431.

### Example 97

### 3-methyl-5-(N-(2,6-dimethylbenzyl)-N-phenethylsulfamoyl)benzofuran-2-carboxylic acid (F27-S50)

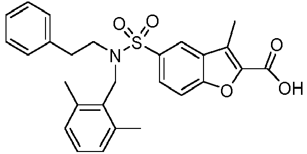

In accordance with the preparation method of 5-(N-benzyl-N-phenethylsulfamoyl)-3-methylbenzofuran-2-carboxylic acid (F27), this example was implemented under the same condition except that M4-50 was used instead of ethyl 5-(N-benzyl-N-phenethylsulfamoyl)-3-methylbenzofuran-2-carboxylate (F27-S8). 62 mg of white solid was obtained, with a yield of 65%. M.p. 211 - 212 °C. ¹H NMR (400 MHz, DMSO-*d*₆) δ 13.76 (s, 1H), 8.22 (s, 1H), 7.96 (d, *J* = 8.7 Hz, 1H), 7.86 (d, *J* = 8.7 Hz, 1H), 7.15 (dt, *J* = 14.1, 6.9 Hz, 4H), 7.02 (d, *J* = 7.4 Hz, 2H), 6.77 (d, *J* = 7.0 Hz, 2H), 4.37 (s, 2H), 3.15 - 2.98 (m, 2H), 2.58 (s, 3H), 2.41 - 2.31 (m, 2H), 2.26 (s, 6H). ¹³C NMR (101 MHz, DMSO-*d*₆) δ 161.2, 155.6, 143.7, 138.6, 133.8, 131.6, 129.6, 128.9, 128.8, 128.8, 128.6, 126.8, 126.7, 125.2, 122.2, 113.5, 49.9, 47.9, 40.4, 36.6, 20.2, 9.6. HRMS (ESI) [M - H] ⁻, theoretically calculated for C₂₇H₂₆NO₅S: 476.1532, found 476.1533.

### Example 98

### 3-methyl-5-(N-(2,4-difluorobenzyl)-N-phenethylsulfamoyl)benzofuran-2-carboxylic acid (F27-S51)

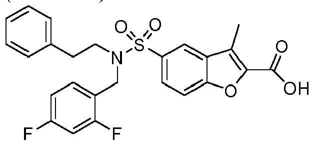

In accordance with the preparation method of 5-(N-benzyl-N-phenethylsulfamoyl)-3-methylbenzofuran-2-carboxylic acid (F27), this example was implemented under the same condition except that M4-51 was used instead of ethyl 5-(N-benzyl-N-phenethylsulfamoyl)-3-methylbenzofuran-2-carboxylate (F27-S8). 92 mg of white solid was obtained, with a yield of 95%. M.p. 155 - 156 °C. ¹H NMR (400 MHz, DMSO-*d*₆) δ 8.15 (d, *J* = 1.9 Hz, 1H), 7.87 (dd, *J* = 8.7, 2.0 Hz, 1H), 7.79 (d, *J* = 8.8 Hz, 1H), 7.47 (q, *J* = 8.5 Hz, 1H), 7.28 - 7.11 (m, 4H), 7.11 - 6.99 (m, 3H), 4.44 (s, 2H), 3.41 - 3.28 (m, 2H), 2.58 (d, *J* = 20.0 Hz, 5H). ¹³C NMR (101 MHz, DMSO-*d*₆) δ 162.1, 155.2, 138.6, 134.4, 132.7 (dd, *J=* 9.7, 5.1 Hz, 1C), 130.2, 129.0, 128.8, 126.8, 125.7, 121.7 (d, *J* = 2.8 Hz, 1C), 121.4, 120.6 (dd, *J* = 14.8, 3.7 Hz, 1C), 113.2, 112.0 (dd, *J* = 21.1, 3.2 Hz, 1C), 104.3 (t, *J* = 26.2 Hz, 1C), 50.3, 45.5 (d, *J* = 1.4 Hz, 1C), 35.0, 9.5. ¹⁹F NMR (376 MHz, DMSO-*d*₆) δ -110.70, -113.08. HRMS (ESI) [M - H] ⁻, theoretically calculated for C₂₅H₂₀NO₅SF₂: 484.1030, found 484.1037.

### Example 99

### 3-methyl-5-(N-(2-fluoro-4-chlorobenzyl)-N-phenethylsulfamoyl)benzofuran-2-carboxylic acid (F27-S52)

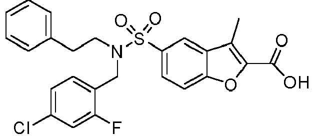

In accordance with the preparation method of 5-(N-benzyl-N-phenethylsulfamoyl)-3-methylbenzofuran-2-carboxylic acid (F27), this example was implemented under the same condition except that M4-52 was used instead of ethyl 5-(N-benzyl-N-phenethylsulfamoyl)-3-methylbenzofuran-2-carboxylate (F27-S8). 90 mg of white solid was obtained, with a yield of 90%. M.p. > 380 °C (for decomposition). ¹H NMR (400 MHz, DMSO-*d*₆) δ 8.03 (d, *J* = 1.9 Hz, 1H), 7.79 (dd, *J* = 8.6, 2.0 Hz, 1H), 7.71 (d, *J* = 8.7 Hz, 1H), 7.44 (t, *J* = 8.3 Hz, 1H), 7.37 (dd, *J* = 10.0, 2.1 Hz, 1H), 7.26 (dd, *J* = 8.3, 2.1 Hz, 1H), 7.24 - 7.11 (m, 3H), 7.03 (d, *J=* 6.9 Hz, 2H), 4.43 (s, 2H), 3.39 - 3.28 (m, 2H), 2.67 - 2.58 (m, 2H), 2.52 (s, 3H). ¹³C NMR (101 MHz, DMSO-*d*₆) δ 163.1, 160.6 (d, *J* = 250.7 Hz, 1C), 154.7, 151.5, 138.6, 133.7, 133.6, 132.6 (d, *J* = 4.8 Hz, 1C), 131.0, 129.0, 128.8, 126.8, 125.1 (d, *J* = 3.5 Hz, 1C), 124.4, 123.7 (d, *J* = 14.4 Hz, 1C), 120.6, 117.3, 116.4 (d, *J* = 25.5 Hz, 1C), 112.8, 50.4, 45.5 (d, *J* = 2.2 Hz, 1C), 34.9, 9.5. ¹⁹F NMR (376 MHz, DMSO-*d*₆) δ -114.61. HRMS (ESI) [M - H] ⁻, theoretically calculated for C₂₅H₂₀NO₅FSCl: 500.0735, found 500.0736.

### Example 100

### 3-methyl-5-(N-(2-fluoro-4-bromobenzyl)-N-phenethylsulfamoyl)benzofuran-2-carboxylic acid (F27-S53)

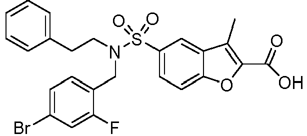

In accordance with the preparation method of 5-(N-benzyl-N-phenethylsulfamoyl)-3-methylbenzofuran-2-carboxylic acid (F27), this example was implemented under the same condition except that M4-53 was used instead of ethyl 5-(N-benzyl-N-phenethylsulfamoyl)-3-methylbenzofuran-2-carboxylate (F27-S8). 98 mg of white solid was obtained, with a yield of 90%. M.p. > 380 °C (for decomposition). ¹H NMR (400 MHz, DMSO-*d*₆) δ 8.03 (d, *J* = 1.9 Hz, 1H), 7.79 (dd, *J* = 8.7, 2.0 Hz, 1H), 7.71 (d, *J* = 8.7 Hz, 1H), 7.53 - 7.46 (m, 1H), 7.38 (d, *J* = 5.0 Hz, 2H), 7.26 - 7.11 (m, 3H), 7.03 (d, *J* = 6.7 Hz, 2H), 4.42 (s, 2H), 3.38 - 3.28 (m, 2H), 2.67 - 2.58 (m, 2H), 2.52 (s, 3H). ¹³C NMR (101 MHz, DMSO-*d*₆) δ 163.1, 160.6 (d, *J=* 251.6 Hz, 1C), 154.7, 151.8, 138.6, 133.5, 132.9 (d, *J=* 4.7 Hz, 1C), 131.0, 129.0, 128.8, 128.1 (d, *J* = 3.4 Hz, 1C), 126.8, 124.3, 124.2 (d, *J* = 14.4 Hz, 1C), 121.6 (d, *J* = 9.7 Hz, 1C), 120.6, 119.2 (d, *J* = 25.0 Hz, 1C), 117.1, 112.8, 50.4, 45.6 (d, *J* = 1.7 Hz, 1C), 34.9, 9.5. ¹⁹F NMR (376 MHz, DMSO-*d*₆) δ -114.52. HRMS (ESI) [M - H] ⁻, theoretically calculated for C₂₅H₂₀NO₅FSBr: 544.0230, found 544.0230.

### Example 102

### 3-methyl-5-(N-(2-fluoro-4-(trifluoromethyl)benzyl)-N-phenethylsulfamoyl)benzofuran-2-carboxylic acid (F27-S55)

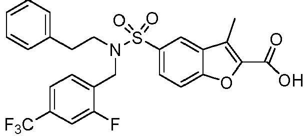

In accordance with the preparation method of 5-(N-benzyl-N-phenethylsulfamoyl)-3-methylbenzofuran-2-carboxylic acid (F27), this example was implemented under the same condition except that M4-55was used instead of ethyl 5-(N-benzyl-N-phenethylsulfamoyl)-3-methylbenzofuran-2-carboxylate (F27-S8). 82 mg of white solid was obtained, with a yield of 77%. M.p. 174 - 175 °C. ¹H NMR (400 MHz, DMSO-*d*₆) δ 8.23 (d, *J* = 1.9 Hz, 1H), 7.93 (dd, *J* = 8.7, 1.9 Hz, 1H), 7.83 (d, *J* = 8.8 Hz, 1H), 7.62 (q, *J* = 10.7, 9.2 Hz, 2H), 7.54 (d, *J* = 8.0 Hz, 1H), 7.24 - 7.11 (m, 3H), 7.05 (d, *J* = 7.0 Hz, 2H), 4.55 (s, 2H), 3.49 - 3.36 (m, 2H), 2.72 - 2.63 (m, 2H), 2.56 (s, 3H). ¹³C NMR (101 MHz, DMSO-*d*₆) δ 161.3, 160.3 (d, *J* = 248.9 Hz, 1C), 155.5, 144.8, 138.5, 134.6, 132.2 (d, *J* = 4.1 Hz, 1C), 129.8, 129.6 (d, *J* = 14.3 Hz, 1C), 129.0, 128.8, 126.7, 126.4, 124.6 (d, *J* = 99.9 Hz, 1C), 121.9, 121.7 (t, *J* = 3.5 Hz, 1C), 113.4, 113.2 (d, *J* = 25.6 Hz, 1C), 50.8, 45.8 (d, *J* = 3.3 Hz, 1C), 35.0, 9.5. ¹⁹F NMR (376 MHz, DMSO-*d*₆) δ -61.16, -114.91. HRMS (ESI) [M - H] ⁻, theoretically calculated for C₂₆H₂₀NO₅SF₄: 534.0998, found 534.0992.

### Example 103

### 3-methyl-5-(N-(2-chloro-4-fluorobenzyl)-N-phenethylsulfamoyl)benzofuran-2-carboxylic acid (F27-S56)

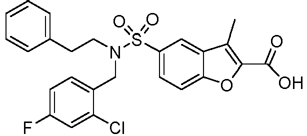

In accordance with the preparation method of 5-(N-benzyl-N-phenethylsulfamoyl)-3-methylbenzofuran-2-carboxylic acid (F27), this example was implemented under the same condition except that M4-56 was used instead of ethyl 5-(N-benzyl-N-phenethylsulfamoyl)-3-methylbenzofuran-2-carboxylate (F27-S8). 65 mg of white solid was obtained, with a yield of 65%. M.p. 145 - 146 °C. ¹H NMR (400 MHz, DMSO-*d*₆) δ 13.82 (s, 1H), 8.27 (s, 1H), 8.10 - 7.72 (m, 2H), 7.53 (t, *J* = 7.5 Hz, 1H), 7.40 (d, *J* = 8.9 Hz, 1H), 7.30 - 7.09 (m, 4H), 7.02 (d, *J* = 7.3 Hz, 2H), 4.48 (s, 2H), 3.37 (t, *J=* 8.0 Hz, 2H), 2.58 (s, 5H). ¹³C NMR (101 MHz, DMSO-*d*₆) δ 161.8 (d, *J* = 248.6 Hz, 1C), 161.2, 155.6, 144.1, 138.5, 134.7, 133.8 (d, *J=* 10.7 Hz, 1C), 132.4 (d, *J* = 8.9 Hz, 1C), 131.2 (d, *J* = 3.4 Hz, 1C), 129.7, 129.0, 128.8, 126.8, 126.7, 124.9, 122.1, 117.1 (d, *J* = 25.1 Hz, 1C), 114.9 (d, *J* = 21.3 Hz, 1C), 113.6, 50.6, 49.3, 35.1, 9.5. ¹⁹F NMR (376 MHz, DMSO-*d*₆) δ -112.54. HRMS (ESI) [M - H] ⁻, theoretically calculated for C₂₅H₂₀NO₅FSCl: 500.0735, found 500.0732.

### Example 104

### 3-methyl-5-(N-(2-(trifluoromethyl)-4-fluorobenzyl)-N-phenethylsulfamoyl)benzofuran-2-carboxylic acid (F27-S57)

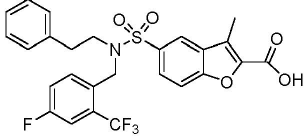

In accordance with the preparation method of 5-(N-benzyl-N-phenethylsulfamoyl)-3-methylbenzofuran-2-carboxylic acid (F27), this example was implemented under the same condition except that M4-57 was used instead of ethyl 5-(N-benzyl-N-phenethylsulfamoyl)-3-methylbenzofuran-2-carboxylate (F27-S8). 109 mg of white solid was obtained, with a yield of 99%. M.p. 156- 157 °C. ¹H NMR (400 MHz, DMSO-*d*₆) δ 8.29 (s, 1H), 7.91 (dd, *J* = 36.9, 8.4 Hz, 2H), 7.70 (s, 1H), 7.60 (d, *J* = 8.1 Hz, 1H), 7.55 - 7.44 (m, 1H), 7.16, 7.00 (d, *J* = 6.0 Hz, 2H), 4.54 (s, 2H), 3.42 (s, 2H), 2.58 (s, 5H). ¹³C NMR (101 MHz, DMSO-*d*₆) δ 161.3, 161.2 (d, *J* = 247.6, 1C), 160.0, 155.6, 144.4, 138.3, 134.3, 132.8, 132.7, 129.8, 128.8 (d, *J=* 13.3, 1C), 126.8, 126.5, 124.6, 122.2, 120.0 (d, *J* = 20.9, 1C), 114.0 (d, *J* = 6.2, 1C), 113.6, 50.9, 48.3, 34.8, 9.5. ¹⁹F NMR (376 MHz, DMSO) δ -58.58, -113.37. HRMS (ESI) [M - H] ⁻, theoretically calculated for C₂₆H₂₀NO₅F₄S: 534.0998, found 534.0988.

### Example 105

### 3-methyl-5-(N-(2,4-bis(trifluoromethyl)benzyl)-N-phenethylsulfamoyl)benzofuran-2-carboxylic acid (F27-S58)

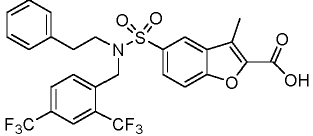

In accordance with the preparation method of 5-(N-benzyl-N-phenethylsulfamoyl)-3-methylbenzofuran-2-carboxylic acid (F27), this example was implemented under the same condition except that M4-58 was used instead of ethyl 5-(N-benzyl-N-phenethylsulfamoyl)-3-methylbenzofuran-2-carboxylate (F27-S8). 62 mg of white solid was obtained, with a yield of 53%. M.p. 155 - 156 °C. ¹H NMR (400 MHz, DMSO-*d*₆) δ 8.26 (s, 1H), 8.05 - 7.92 (m, 3H), 7.90 - 7.83 (m, 2H), 7.15 (dq, *J=* 14.1, 7.0 Hz, 3H), 7.04 (d, *J* = 6.9 Hz, 2H), 4.64 (s, 2H), 3.58 - 3.41 (m, 2H), 2.74 - 2.61 (m, 2H). 2.57 (s, 3H). ¹³C NMR (101 MHz, DMSO-*d*₆) δ 161.6, 155.6, 141.9, 138.3, 133.9, 131.1, 129.9, 129.0, 128.8, 127.4 (d, *J* = 31.4 Hz, 1C), 126.8, 126.4, 125.2 (d, *J* = 5.5 Hz, 1C), 123.8 (d, *J* = 3.6 Hz, 1C), 123.1, 122.5 (d, *J* = 3.1 Hz, 1C), 122.1, 113.6, 51.3, 48.7, 34.8, 9.5. ¹⁹F NMR (376 MHz, DMSO-*d*₆) δ -58.84, -61.35. HRMS (ESI) [M - H] ⁻, theoretically calculated for C₂₇H₂₀NO₅F₆S: 584.0966, found 584.0955.

### Example 106

### 3-methyl-5-(N-(2-(trifluoromethyl)-4-bromobenzyl)-N-phenethylsulfamoyl)benzofuran-2-carboxylic acid (F27-S59)

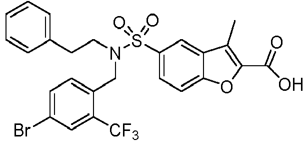

In accordance with the preparation method of 5-(N-benzyl-N-phenethylsulfamoyl)-3-methylbenzofuran-2-carboxylic acid (F27), this example was implemented under the same condition except that M4-59 was used instead of ethyl 5-(N-benzyl-N-phenethylsulfamoyl)-3-methylbenzofuran-2-carboxylate (F27-S8). 75 mg of white solid was obtained, with a yield of 63%. M.p. 150 - 151 °C. ¹H NMR (400 MHz, DMSO-*d*₆) δ 13.82 (s, 1H), 8.28 (s, 1H), 8.10 - 7.72 (m, 4H), 7.57 (d, *J* = 8.5 Hz, 1H), 7.38 - 6.87 (m, 5H), 4.53 (s, 2H), 3.44 (t, *J* = 7.8 Hz, 2H), 2.59 (d, *J* = 15.7 Hz, 5H). ¹³C NMR (101 MHz, DMSO-*d*₆) δ 161.2, 155.7, 144.0, 138.3, 136.1 (d, *J* = 14.9 Hz, 1C), 134.3, 132.2, 129.7, 129.0, 128.9, 128.8, 128.4 (d, *J=* 31.4 Hz, 1C), 126.7 (d, *J* = 10.7 Hz, 1C), 125.0 (d, *J=* 4.3 Hz, 1C), 122.3, 121.0, 113.7, 51.1, 48.4, 34.8, 9.5. ¹⁹F NMR (376 MHz, DMSO-*d*₆) δ -58.57. HRMS (ESI) [M - H] ⁻, theoretically calculated for C₂₆H₂₀NO₅F₃SBr: 594.0198, found 594.0205.

### Example 107

### 3-methyl-5-(N-(2,4-dichlorobenzyl)-N-phenethylsulfamoyl)benzofuran-2-carboxylic acid (F27-S60)

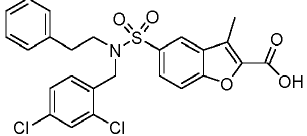

In accordance with the preparation method of 5-(N-benzyl-N-phenethylsulfamoyl)-3-methylbenzofuran-2-carboxylic acid (F27), this example was implemented under the same condition except that M4-60 was used instead of ethyl 5-(N-benzyl-N-phenethylsulfamoyl)-3-methylbenzofuran-2-carboxylate (F27-S8). 48 mg of white solid was obtained, with a yield of 46%. M.p. 177 - 178 °C. ¹H NMR (400 MHz, DMSO-*d*₆) δ 13.77 (s, 1H), 8.27 (s, 1H), 7.96 (d, *J* = 8.5 Hz, 1H), 7.85 (d, *J* = 8.6 Hz, 1H), 7.55 (s, 1H), 7.48 (d, *J* = 8.2 Hz, 1H), 7.39 (d, *J* = 7.9 Hz, 1H), 7.17 (dt, *J* = 16.7, 6.5 Hz, 3H), 7.03 (d, *J* = 6.8 Hz, 2H), 4.49 (s, 2H), 3.50 - 3.32 (m, 2H), 2.59 (d, *J* = 12.3 Hz, 5H). ¹³C NMR (101 MHz, DMSO-*d*₆) δ 161.1, 155.6, 143.8, 138.5, 134.7, 134.1, 133.8, 133.4, 132.1, 129.6, 129.2, 129.0, 128.8, 127.9, 126.8, 126.7, 125.2, 122.2, 113.6, 50.8, 49.4, 35.1, 9.5. HRMS (ESI) [M - H] ⁻, theoretically calculated for C₂₅H₂₀NO₅SCl₂: 516.0439, found 516.0430.

### Example 108

### 3-methyl-5-(N-(2-methyl-5-fluorobenzyl)-N-phenethylsulfamoyl)benzofuran-2-carboxylic acid (F27-S61)

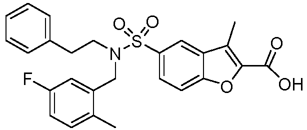

In accordance with the preparation method of 5-(N-benzyl-N-phenethylsulfamoyl)-3-methylbenzofuran-2-carboxylic acid (F27), this example was implemented under the same condition except that M4-61 was used instead of ethyl 5-(N-benzyl-N-phenethylsulfamoyl)-3-methylbenzofuran-2-carboxylate (F27-S8). 72 mg of white solid was obtained, with a yield of 75%. M.p. > 380 °C (for decomposition). ¹H NMR (400 MHz, DMSO-*d*₆) δ 8.19 (d, *J* = 2.0 Hz, 1H), 7.89 (dd, *J* = 8.7, 2.0 Hz, 1H), 7.79 (d, *J* = 8.7 Hz, 1H), 7.28 - 7.09 (m, 5H), 7.02 (td, *J* = 8.5, 2.9 Hz, 1H), 6.95 (d, *J* = 6.8 Hz, 2H), 4.37 (s, 2H), 3.37 - 3.22 (m, 2H), 2.56 (s, 3H), 2.46 (d, *J* = 7.7 Hz, 2H), 2.26 (s, 3H). ¹³C NMR (101 MHz, DMSO-*d*₆) δ 162.5, 161.0 (d, *J* = 242.1 Hz, 1C), 155.0, 138.6, 137.6 (d, *J* = 6.9 Hz, 1C), 133.6, 133.0 (d, *J* = 2.8 Hz, 1C), 132.2 (d, *J* = 7.9 Hz, 1C), 130.7, 128.8 (d, *J* = 9.0 Hz, 1C), 126.7, 125.1, 121.2, 115.9 (d, *J* = 22.3 Hz, 1C), 114.4 (d, *J* = 20.8 Hz, 1C), 113.0, 50.5, 50.2, 35.1, 18.4, 9.5. ¹⁹F NMR (376 MHz, DMSO-*d*₆) δ -117.53. HRMS (ESI) [M - H] ⁻, theoretically calculated for C₂₆H₂₃NO₅SF: 480.1281, found 480.1284.

### Example 109

### 3-methyl-5-(N-(2,6-difluoro-4-chlorobenzyl)-N-phenethylsulfamoyl)benzofuran-2-carboxylic acid(F27-S62)

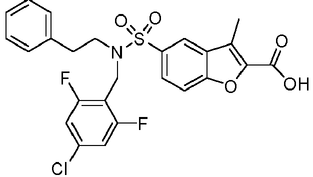

In accordance with the preparation method of 5-(N-benzyl-N-phenethylsulfamoyl)-3-methylbenzofuran-2-carboxylic acid (F27), this example was implemented under the same condition except that M4-62 was used instead of ethyl 5-(N-benzyl-N-phenethylsulfamoyl)-3-methylbenzofuran-2-carboxylate (F27-S8). 102 mg of white solid was obtained, with a yield of 98%. M.p. > 380 °C (for decomposition). ¹H NMR (400 MHz, DMSO-*d*₆) δ 8.08 (d, *J* = 1.9 Hz, 1H), 7.88 (dd, *J* = 8.8, 1.9 Hz, 1H), 7.81 (d, *J=* 8.8 Hz, 1H), 7.24 (t, *J=* 7.7 Hz, 4H), 7.17 (t, *J* = 7.3 Hz, 1H), 7.09 (d, *J* = 7.0 Hz, 2H), 4.43 (s, 2H), 3.42 - 3.32 (m, 2H), 2.77 - 2.69 (m, 2H), 2.55 (s, 3H). ¹³C NMR (101 MHz, DMSO-*d*₆) δ 161.4 (dd, *J* = 252.4, 9.9 Hz, 1C), 161.2, 155.5, 144.1, 138.6, 134.7 (t, *J* = 14.2 Hz, 1C), 134.7, 129.5, 129.0, 128.9, 126.8, 126.5, 124.7, 121.7, 113.4, 113.2 (d, *J* = 29.6 Hz, 1C), 111.8 (t, *J* = 19.3 Hz, 1C), 50.8, 35.3, 9.5. ¹⁹F NMR (376 MHz, DMSO-*d*₆) δ -111.06. HRMS (ESI) [M - H] ⁻, theoretically calculated for C₂₅H₁₉NO₅F₂SCl: 518.0641, found 518.0642.

### Example 110

### 3-methyl-5-(N-([1,1'-biphenyl]-4-ylmethyl)-N-phenethylsulfamoyl)benzofuran-2-carboxylic acid (F27-S82)

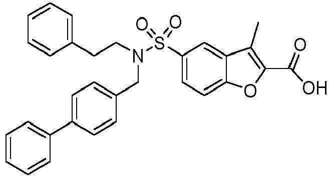

In accordance with the preparation method of 5-(N-benzyl-N-phenethylsulfamoyl)-3-methylbenzofuran-2-carboxylic acid (F27), this example was implemented under the same condition except that M4-82 was used instead of ethyl 5-(N-benzyl-N-phenethylsulfamoyl)-3-methylbenzofuran-2-carboxylate (F27-S8). 93 mg of white solid was obtained, with a yield of 84%. M.p. > 380 °C (for decomposition). ¹H NMR (400 MHz, DMSO-*d*₆) δ 8.26 (s, 1H), 7.96 (d, *J* = 8.8 Hz, 1H), 7.84 (d, *J* = 8.8 Hz, 1H), 7.63 (dd, *J* = 7.6, 5.2 Hz, 4H), 7.50 - 7.39 (m, 4H), 7.35 (t, *J* = 7.3 Hz, 1H), 7.18 (t, *J* = 7.2 Hz, 2H), 7.12 (t, *J* = 7.2 Hz, 1H), 7.02 (d, *J* = 6.9 Hz, 2H), 4.48 (s, 2H), 3.41 - 3.34 (m, 2H), 2.67 - 2.58 (m, 2H), 2.56 (s, 3H). ¹³C NMR (101 MHz, DMSO-*d*₆) δ 161.3, 155.5, 144.1, 140.1, 139.9, 138.7, 136.5, 135.4, 129.7, 129.4, 129.3, 129.0, 128.8, 127.9, 127.1, 127.1, 126.7, 126.6, 124.9, 122.0, 113.5, 51.4, 49.9, 34.8, 9.5. HRMS (ESI) [M - H] ⁻, theoretically calculated for C₃₁H₂₆NO₅S: 524.1532, found 524.1528.

### Example 111

### 3-methyl-5-(N-(2'-cyano-[1,1'-biphenyl]-4-yl)methyl)-N-phenethylsulfamoyl)benzofuran-2-carboxylic acid (F27-S83)

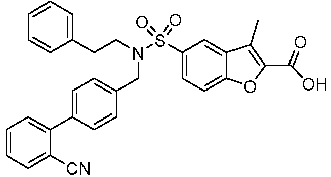

In accordance with the preparation method of 5-(N-benzyl-N-phenethylsulfamoyl)-3-methylbenzofuran-2-carboxylic acid (F27), this example was implemented under the same condition except that M4-83 was used instead of ethyl 5-(N-benzyl-N-phenethylsulfamoyl)-3-methylbenzofuran-2-carboxylate (F27-S8). 96 mg of white solid was obtained, with a yield of 86%. M.p. 197 - 198 °C. ¹H NMR (400 MHz, DMSO-*d*₆) δ 8.25 (d, *J* = 1.9 Hz, 1H), 8.00 - 7.89 (m, 2H), 7.79 (dd, *J* = 15.5, 8.1 Hz, 2H), 7.58 (dd, *J* = 14.5, 7.9 Hz, 4H), 7.50 (d, *J* = 8.2 Hz, 2H), 7.24 - 7.08 (m, 3H), 7.02 (d, *J* = 7.0 Hz, 2H), 4.51 (s, 2H), 3.45 - 3.32 (m, 2H), 2.60 (d, *J* = 14.5 Hz, 5H). ¹³C NMR (101 MHz, DMSO-*d*₆) δ 155.2, 144.6, 138.7, 138.3, 137.5, 134.7, 134.3, 134.0, 130.5, 130.2, 129.3, 129.0, 129.0, 128.8, 128.7, 126.7, 125.8, 121.6, 119.0, 113.3, 110.7, 51.6, 50.2, 34.9, 9.6. HRMS (ESI) [M - H] ⁻, theoretically calculated for C₃₂H₂₅N₂O₅S: 549.1484, found 549.1487.

### Example 112

### 3-methyl-5-(N-((2'-(tert-butoxycarbonyl)-[1,1'-biphenyl]-4-yl)methyl)-N-phenethylsulfamoyl)benzofuran-2-carboxylic acid (F27-S84)

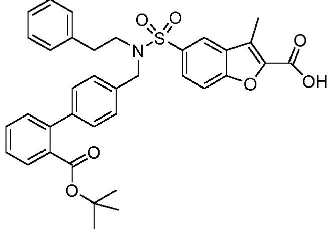

In accordance with the preparation method of 5-(N-benzyl-N-phenethylsulfamoyl)-3-methylbenzofuran-2-carboxylic acid (F27), this example was implemented under the same condition except that M4-84 was used instead of ethyl 5-(N-benzyl-N-phenethylsulfamoyl)-3-methylbenzofuran-2-carboxylate (F27-S8). 52 mg of white solid was obtained, with a yield of 42%. M.p. 197 - 198 °C. ¹H NMR (400 MHz, DMSO-*d*₆) δ 8.35 - 8.23 (m, 1H), 7.95 (dd, *J* = 8.8, 1.7 Hz, 1H), 7.83 (d, *J* = 8.8 Hz, 1H), 7.66 (d, *J* = 7.6 Hz, 1H), 7.56 (t, *J* = 7.4 Hz, 1H), 7.43 (dd, *J=* 22.4, 7.8 Hz, 3H), 7.34 (d, *J* = 7.5 Hz, 1H), 7.27 (d, *J* = 8.0 Hz, 2H), 7.20 (t, *J* = 7.2 Hz, 2H), 7.13 (t, *J* = 7.2 Hz, 1H), 7.03 (d, *J* = 7.1 Hz, 2H), 4.51 (s, 2H), 3.43 - 3.28 (m, 2H), 2.60 (d, *J* = 12.8 Hz, 5H), 1.17 (s, 9H). ¹³C NMR (101 MHz, DMSO-*d*₆) δ 167.9, 161.8, 155.4, 141.0, 140.7, 138.7, 136.4, 135.2, 133.2, 131.3, 130.8, 130.0, 129.5, 129.0, 128.9, 128.8, 128.4, 127.8, 126.7, 126.2, 121.7, 113.4, 81.2, 51.2, 49.7, 34.7, 27.6, 9.6. HRMS (ESI) [M - H] ⁻, theoretically calculated for C₃₆H₃₄NO₇S: 624.2056, found 624.2044.

### Example 113

### 3-methyl-5-(N-(4-benzoylbenzyl)-N-phenethylsulfamoyl)benzofuran-2-carboxylic acid (F27-S85)

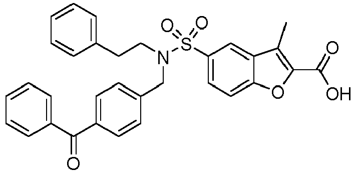

In accordance with the preparation method of 5-(N-benzyl-N-phenethylsulfamoyl)-3-methylbenzofuran-2-carboxylic acid (F27), this example was implemented under the same condition except that M4-85 was used instead of ethyl 5-(N-benzyl-N-phenethylsulfamoyl)-3-methylbenzofuran-2-carboxylate (F27-S8). 70 mg of white solid was obtained, with a yield of 63%. M.p. 192 - 193 °C. ¹H NMR (400 MHz, DMSO-*d*₆) δ 13.70 (s, 1H), 8.30 (d, *J* = 1.9 Hz, 1H), 7.97 (dd, *J* = 8.8, 2.0 Hz, 1H), 7.86 (d, *J* = 8.7 Hz, 1H), 7.69 (d, *J* = 8.0 Hz, 5H), 7.60 - 7.48 (m, 4H), 7.19 (t, *J* = 7.3 Hz, 2H), 7.13 (t, *J* = 7.2 Hz, 1H), 7.08 - 6.99 (m, 2H), 4.55 (s, 2H), 3.41 (t, *J* = 7.9 Hz, 2H), 2.64 (t, *J* = 7.9 Hz, 2H), 2.58 (s, 3H). ¹³C NMR (101 MHz, DMSO-*d*₆) δ 195.8, 161.1, 155.6, 143.8, 142.6, 138.7, 137.5, 136.6, 135.1, 133.1, 130.3, 130.0, 129.6, 129.0, 129.0, 128.8, 128.6, 126.7, 126.7, 125.3, 122.2, 113.6, 51.6, 50.4, 34.8, 9.6. HRMS (ESI) [M - H] ⁻, theoretically calculated for C₃₂H₂₆NO₆S: 552.1481, found 552.1484.

### Example 114

### 3,3-dimethyl-1-(4-(4-nitrophenyl)piperazin-1-yl)butan-1-one (M5-1)

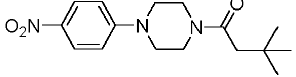

4-nitrophenylpiperazine (2.07 g, 10 mmol) and triethylamine (1.22 g, 12 mmol) were added to anhydrous tetrahydrofuran (30 mL), and the solution was stirred at 0 °C for 10 min. Anhydrous tetrahydrofuran (10 mL) solution of 3, 3-dimethylbutyrylchloride (1.62 g, 12 mmol) was slowly added dropwise and the resulting solution was then heated to room temperature to react for 10 h. After the reaction was found to be completed from the monitoring of the TLC, the solvent was distilled off under reduced pressure, and the obtained product was dissolved in water (100 mL) and then extracted three times with ethyl acetate (100 mL). The organic phase was washed with saturated aqueous NaCl solution and then dried with anhydrous sodium sulfate. The crude product was separated by column chromatography (DCM/MeOH = 150/1) to obtain 2.82 g of orange red solid, with a yield of 92%.

### Example 115

### propyl-4-(4-nitrophenyl)piperazin-1-carboxylate (M5-2)

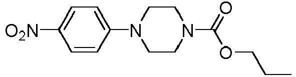

In accordance with the preparation method of 3,3-dimethyl-1-(4-(4-nitrophenyl)piperazin-1-yl)butan-1-one (M5-1), this example was implemented under the same condition except that propyl chlorocarbonate was used instead of 3,3-dimethylbutyrylchloride. 1.35 of yellow solid was obtained, with a yield of 92%. ¹H NMR (400 MHz, Chloroform-*d*) δ 8.22 - 7.95 (m, 2H), 6.87 - 6.71 (m, 2H), 4.06 (t, *J* = 6.7 Hz, 2H), 3.71 - 3.58 (m, 4H), 3.49 - 3.35 (m, 4H), 1.66 (h, *J* = 7.2 Hz, 2H), 0.94 (t, *J* = 7.4 Hz, 3H). ¹³C NMR (101 MHz, Chloroform-*d*) δ 155.4, 154.6, 138.9, 125.9, 112.9, 67.3, 46.9, 43.0, 22.3, 10.4.

### Example 116

### 1-(4-(4-nitrophenyl)piperazin-1-yl)ethan-1-one (M5-3)

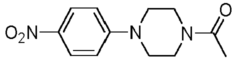

In accordance with the preparation method of 3,3-dimethyl-1-(4-(4-nitrophenyl)piperazin-1-yl)butan-1-one (M5-1), this example was implemented under the same condition except that acetyl chloride was used instead of 3,3-dimethylbutyrylchloride. 1.08 of yellow solid was obtained, with a yield of 87%.

### Example 117

### (4-(4-nitrophenyl)piperazin-1-yl)(phenyl)methanone (M5-4)

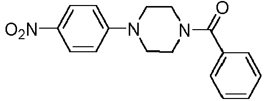

In accordance with the preparation method of 3,3-dimethyl-1-(4-(4-nitrophenyl)piperazin-1-yl)butan-1-one (M5-1), this example was implemented under the same condition except that benzoyl chloride was used instead of 3,3-dimethylbutyrylchloride. 1.45 of yellow solid was obtained, with a yield of 93%.

### Example 118

### N,N-dimethyl-4-(4-nitrophenyl)piperazine-1-carboxamide (M5-5)

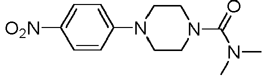

In accordance with the preparation method of 3,3-dimethyl-1-(4-(4-nitrophenyl)piperazin-1-yl)butan-1-one (M5-1), this example was implemented under the same condition except that dimethylcarbamoyl chloride was used instead of 3,3-dimethylbutyrylchloride. 2.47 of yellow solid was obtained, with a yield of 88%.

### Example 119

### 3,3-dimethyl-1-(4-(4-aminophenyl)piperazin-1-yl)butan-1-one (M6-1)

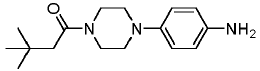

3,3-dimethyl-1-(4-(4-nitrophenyl)piperazin-1-yl)butan-1-one (94 mg, 0.3 mmol) and 38% concentrated hydrochloric acid (0.5 mL, 6 mmol) were mixed and stirred, zinc powder (0.14 g, 2.1 mmol) was then added step by step, the reaction was performed at room temperature for 11 h. After the reaction was found to be completed from the monitoring of the TLC, ammonia water was then slowly added dropwise until PH = 5 ~ 6. The reaction product was dissolved in water (5 mL) and then extracted three times with dichloromethane (15 mL). The organic phase was washed with saturated aqueous NaCl solution and then dried with anhydrous sodium sulfate. The solvent was distilled off under reduced pressure to obtain 90 mg of white solid, with a yield of 99%.

### Example 120

### Propyl-4-(4-aminophenyl)piperazine-1-carboxylate (M6-2)

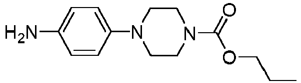

In accordance with the preparation method of 3,3-dimethyl-1-(4-(4-aminophenyl)piperazin-1-yl)butan-1-one (M6-1), this example was implemented under the same condition except that propyl-4-(4-nitrophenyl)piperazin-1-carboxylate was used instead of 3,3-dimethyl-1-(4-(4-nitrophenyl)piperazin-1-yl)butan-1-one. 0.50 g of white solid was obtained, with a yield of 64%. ¹H NMR (400 MHz, Chloroform-*d*) δ 6.80 (d, *J* = 8.8 Hz, 2H), 6.69 - 6.59 (m, 2H), 4.06 (t, *J* = 6.7 Hz, 2H), 3.69 - 3.57 (m, 4H), 3.51 (s, 2H), 2.96 (t, *J* = 5.1 Hz, 4H), 1.66 (h, *J=* 7.1 Hz, 2H), 0.95 (t, *J* = 7.4 Hz, 3H). ¹³C NMR (101 MHz, Chloroform-*d*) δ 155.6, 144.2, 140.8, 119.3, 116.1, 67.1, 51.2, 43.9, 22.4, 10.4.

### Example 121

### 1-(4-(4-aminophenyl)piperazin-1-yl)ethan-1-one (M6-3)

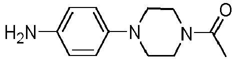

In accordance with the preparation method of 3,3-dimethyl-1-(4-(4-aminophenyl)piperazin-1-yl)butan-1-one (M6-1), this example was implemented under the same condition except that1-(4-(4-nitrophenyl)piperazin-1-yl)ethan-1-one was used instead of 3,3-dimethyl-1-(4-(4-nitrophenyl)piperazin-1-yl)butan-1-one. 0.39 of yellow solid was obtained, with a yield of 60%.

### Example 122

### (4-(4-aminophenyl)piperazin-1-yl) (phenyl)methanone (M6-4)

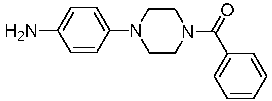

In accordance with the preparation method of 3,3-dimethyl-1-(4-(4-aminophenyl)piperazin-1-yl)butan-1-one (M6-4), this example was implemented under the same condition except that (4-(4-nitrophenyl)piperazin-1-yl)phenylmethyl ketone was used instead of 3,3-dimethyl-1-(4-(4-nitrophenyl)piperazin-1-yl)butan-1-one. 0.62 of yellow solid was obtained, with a yield of 74%.

### Example 123

### N,N-dimethyl-4-(4-aminophenyl)piperazine-1-carboxamide (M6-5)

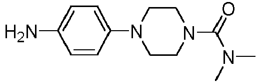

In accordance with the preparation method of 3,3-dimethyl-1-(4-(4-aminophenyl)piperazin-1-yl)butan-1-one (M6-1), this example was implemented under the same condition except that *N,N-*dimethyl-4-(4-nitrophenyl)piperazine-1-amide was used instead of 3,3-dimethyl-1-(4-(4-nitrophenyl)piperazin-1-yl)butan-1-one. 0.55 g of white solid was obtained, with a yield of 74%.

### Example 124

### General synthesis method of intermediate M7

Method A: Substituted aniline or phenethylamine (1.8 mmol), 2-iodoethylbenzene (1.2 mmol), and potassium carbonate (0.25 g, 2.7 mmol) were dissolved in acetonitrile (8 mL), and the resulting solution was then heated to 60 °C to react for 24 h. After the reaction was found to be completed from the monitoring of the TLC, the reaction system was cooled to room temperature. The solvent was distilled off under reduced pressure, and the obtained product was dissolved in water (10 mL) and then extracted three times with ethyl acetate (10 mL). The organic phase was washed with saturated aqueous NaCl solution and then dried with anhydrous sodium sulfate. The crude product was separated by column chromatography to obtain the intermediate M7.

Method B: Substituted benzaldehyde or phenylacetaldehyde (0.5 mmol), substituted aniline or phenethylamine (0.5 mmol), and trimethyl orthoformate (85 mg, 0.8 mmol) were dissolved in methanol (10 mL), and the resulting solution was stirred at 0 °C. Sodium cyanoborohydride (38 mg, 0.6 mmol) was added step by step and the reaction was performed at room temperature for 10 h. After the reaction was found to be completed from the monitoring of the TLC, the solvent was distilled off under reduced pressure, and the obtained product was dissolved in water (10 mL) and then extracted three times with ethyl acetate (10 mL). The organic phase was washed with saturated aqueous NaCl solution and then dried with anhydrous sodium sulfate. The crude product was separated by column chromatography to obtain the intermediate M7.

### Example 125

### N-phenethylaniline (M7-10)

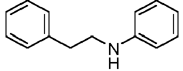

Method A was performed to obtain 32 mg of yellow oil product, with a yield of 32%.

### Example 126

### 4-benzyl-N-phenethylaniline (M7-11)

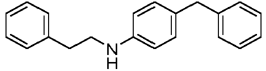

Method B was performed to obtain 190 mg of yellow oil product, with a yield of 79%. ¹H NMR (400 MHz, Chloroform-*d*) δ 7.79 - 7.51 (m, 10H), 7.43 (d, *J* = 8.4 Hz, 2H), 6.91 (d, *J* = 8.5 Hz, 2H), 4.30 (s, 2H), 3.88 (s, 1H), 3.70 (t, *J* = 7.1 Hz, 2H), 3.21 (t, *J* = 7.1 Hz, 2H). ¹³C NMR (101 MHz, Chloroform-*d*) δ 146.2, 142.0, 139.3, 129.8, 129.7, 128.7, 128.7, 128.5, 128.3, 126.3, 125.7, 113.0, 45.1, 41.0, 35.4.

### Example 127

### N-phenethyl-4-phenoxyaniline (M7-12)

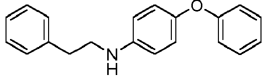

Method B was performed to obtain 140 mg of yellow solid product, with a yield of 65%.

### Example 128

### Diphenylethylamine (M7-13)

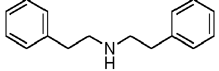

Method A was performed to obtain 89 mg of yellow oil product, with a yield of 80%.

### Example 129

### 4-morpholine-N-phenethylaniline (M7-63)

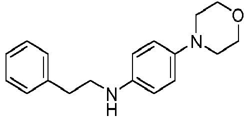

Method A was performed to obtain 120 mg of yellow solid product, with a yield of 43%.

### Example 130

### 4-(4-methylpiperazin-1-yl)-N-phenethylaniline (M7-64)

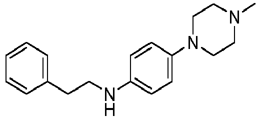

Method A was performed to obtain 39 mg of yellow oil product, with a yield of 13%.

### Example 131

### Tert-butyl 4-(4-(phenylethylamino)phenyl)piperazine-1-carboxylate (M7-65)

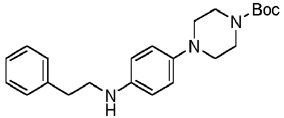

Method A was performed to obtain 340 mg of yellow solid product, with a yield of 45%. ¹H NMR (400 MHz, Chloroform-*d*) δ 7.36 (t, *J* = 7.3 Hz, 2H), 7.31 - 7.22 (m, 3H), 6.89 (d, *J* = 8.0 Hz, 2H), 6.63 (s, 2H), 3.68 - 3.58 (m, 4H), 3.39 (s, 2H), 3.14 - 2.83 (m, 6H), 1.56 (s, 9H). ¹³C NMR (101 MHz, Chloroform-*d*) δ 154.8, 143.6, 142.9, 139.5, 128.8, 128.6, 126.4, 119.4, 114.0, 79.7, 60.4, 51.4, 45.8, 35.7, 28.5.

### Example 132

### 3,3-dimethyl-1-(4-(phenethylamino)phenyl)piperazine-1-yl)butanone (M7-66)

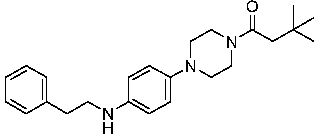

Method A was performed to obtain 245 mg of yellow solid product, with a yield of 53%. ¹H NMR (400 MHz, Chloroform-*d*) δ 7.30 (t, *J* = 7.3 Hz, 2H), 7.21 (t, *J* = 8.4 Hz, 3H), 6.83 (d, *J* = 8.7 Hz, 2H), 6.58 (d, *J* = 8.6 Hz, 2H), 3.77 (t, *J* = 5.1 Hz, 2H), 3.69 - 3.60 (m, 2H), 3.50 (s, 1H), 3.34 (t, *J* = 7.0 Hz, 2H), 2.98 (q, *J* = 5.8 Hz, 4H), 2.88 (t, *J* = 7.0 Hz, 2H), 2.29 (s, 2H), 1.07 (s, 9H). ¹³C NMR (101 MHz, Chloroform-*d*) δ 170.4, 143.2, 143.0, 139.4, 128.8, 128.6, 126.4, 119.4, 114.1, 51.7, 51.4, 46.9, 45.7, 44.7, 41.6, 35.6, 31.5, 30.2.

### Example 133

### Propyl 4-(4-(phenethylamino)phenyl)piperazine-1-carboxylate (M7-67)

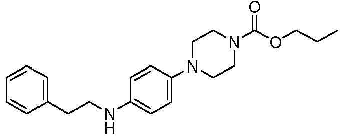

Method A was performed to obtain 328 mg of yellow solid product, with a yield of 56%. ¹H NMR (400 MHz, Chloroform-*d*) δ 7.32 - 7.10 (m, 5H), 6.79 (d, *J* = 8.3 Hz, 2H), 6.53 (d, *J* = 8.3 Hz, 2H), 4.05 (t, *J* = 6.6 Hz, 2H), 3.57 (q, *J* = 11.4, 8.2 Hz, 5H), 3.29 (t, *J* = 7.1 Hz, 2H), 2.87 (dt, *J* = 36.4, 6.0 Hz, 6H), 1.64 (h, *J* = 7.1 Hz, 2H), 0.93 (t, *J* = 7.4 Hz, 3H). ¹³C NMR (101 MHz, Chloroform-*d*) δ 155.6, 143.5, 143.1, 139.5, 128.9, 128.6, 126.4, 119.5, 114.0, 67.1, 51.3, 45.8, 44.0, 35.7, 22.5, 10.6.

### Example 134

### 1-(4-(4-(phenethylamino)phenyl)piperazin-1-yl)ethan-1-one (M7-68)

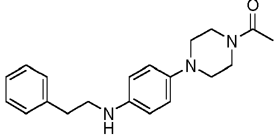

Method A was performed to obtain 23 mg of yellow solid product, with a yield of 6%. ¹H NMR (400 MHz, Chloroform-*d*) δ 7.34 (t, *J=* 7.4 Hz, 2H), 7.25 (t, *J=* 8.1 Hz, 3H), 6.87 (s, 2H), 6.62 (s, 2H), 3.78 (t, *J* = 5.1 Hz, 2H), 3.62 (t, *J* = 5.1 Hz, 2H), 3.39 (s, 2H), 3.15 - 2.84 (m, 6H), 2.15 (s, 3H).

### Example 135

### (4-(4-(phenethylamino)phenyl)piperazin-1-yl)(phenyl)methanone (M7-69)

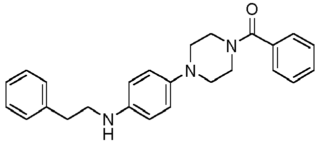

Method A was performed to obtain 257 mg of yellow solid product, with a yield of 33%. ¹H NMR (400 MHz, Chloroform-*d*) δ 7.54 - 7.40 (m, 5H), 7.35 (t, *J* = 7.3 Hz, 2H), 7.30 - 7.18 (m, 3H), 6.88 (d, *J* = 8.6 Hz, 2H), 6.61 (d, *J* = 8.5 Hz, 2H), 3.96 (s, 2H), 3.62 (d, *J* = 41.1 Hz, 3H), 3.36 (t, *J* = 7.1 Hz, 2H), 3.19 - 2.82 (m, 6H). ¹³C NMR (101 MHz, Chloroform-*d*) δ 170.3, 143.2, 143.1, 139.6, 136.0, 129.8, 128.9, 128.7, 128.6, 127.3, 126.4, 119.5, 114.0, 51.7, 48.0, 45.8, 42.5, 35.7.

### Example 136

### 4-(4-benzylpiperazin-1-yl)-N-phenethylaniline (M7-70)

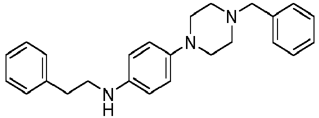

Method A was performed to obtain 138 mg of yellow solid product, with a yield of 37%. ¹H NMR (400 MHz, Chloroform-*d*) δ 7.53 - 7.28 (m, 10H), 6.95 (s, 2H), 6.68 (s, 2H), 3.69 (s, 2H), 3.44 (s, 2H), 3.18 (s, 4H), 2.97 (t, *J* = 6.9 Hz, 2H), 2.73 (t, *J* = 5.0 Hz, 4H). ¹³C NMR (101 MHz, Chloroform-*d*) δ 143.9, 142.4, 139.6, 138.0, 129.4, 129.0, 128.7, 128.5, 127.3, 126.5, 118.9, 114.2, 63.2, 53.4, 51.0, 46.0, 35.7.

### Example 137

### N,N-dimethyl-4-(4-(phenylethylamino)phenyl)piperazine-1-carboxamide (M7-71)

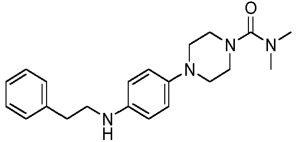

Method A was performed to obtain 120 mg of yellow solid product, with a yield of 34%. ¹H NMR (400 MHz, Chloroform-*d*) δ 7.34 (t, *J* = 7.3 Hz, 2H), 7.29 - 7.21 (m, 3H), 6.89 (d, *J* = 7.4 Hz, 2H), 6.62 (s, 2H), 3.42 (t, *J* = 5.0 Hz, 6H), 3.05 (s, 4H), 2.93 (t, *J* = 7.0 Hz, 2H), 2.88 (s, 6H). ¹³C NMR (101 MHz, Chloroform-*d*) δ 164.9, 139.5, 128.9, 128.7, 126.5, 119.3, 114.2, 51.3, 47.0, 38.7.

### Example 138

### 4-(4-methylsulfonyl)piperazin-1-yl)-N-phenethylaniline (M7-72)

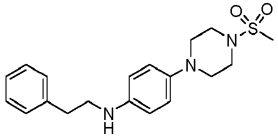

Method A was performed to obtain 258 mg of yellow solid product, with a yield of 36%. ¹H NMR (400 MHz, Chloroform-*d*) δ 7.26 (dt, *J* = 35.0, 7.5 Hz, 5H), 6.83 (d, *J* = 7.5 Hz, 2H), 6.57 (d, *J* = 8.3 Hz, 2H), 3.54 - 3.23 (m, 7H), 3.09 (s, 4H), 2.88 (t, *J* = 6.9 Hz, 2H), 2.79 (s, 3H). ¹³C NMR (101 MHz, Chloroform-*d*) δ 143.4, 142.8, 139.4, 128.8, 128.6, 126.5, 119.7, 114.0, 51.1, 46.1, 45.7, 35.6, 34.2.

### Example 139

### Tert-butyl 4-(4-(phenethylamino)phenyl)piperidine-1-carboxylate (M7-73)

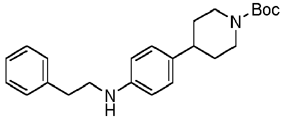

Method A was performed to obtain 212 mg of yellow solid product, with a yield of 20%. ¹H NMR (400 MHz, Chloroform-*d*) δ 7.37 (t, *J* = 7.4 Hz, 2H), 7.29 (t, *J* = 7.1 Hz, 3H), 7.08 (d, *J* = 8.4 Hz, 2H), 6.64 (d, *J* = 8.4 Hz, 2H), 4.29 (s, 2H), 3.70 (s, 1H), 3.43 (t, *J* = 7.0 Hz, 2H), 2.96 (t, *J* = 7.0 Hz, 2H), 2.84 (t, *J* = 11.0 Hz, 2H), 2.59 (t, *J* = 12.1 Hz, 1H), 1.84 (d, *J* = 12.6 Hz, 2H), 1.63 (dt, *J* = 12.6, 6.2 Hz, 2H), 1.55 (s, 9H). ¹³C NMR (101 MHz, Chloroform-*d*) δ 155.0, 146.5, 139.4, 135.0, 128.8, 128.6, 127.6, 126.5, 113.2, 79.4, 45.4, 41.8, 35.6, 33.5, 28.6.

### Example 140

### Tert-butyl 4-(4-(phenylethylamino)-2-(trifluoromethyl)phenyl)piperazine-1-carboxylate (M7-74)

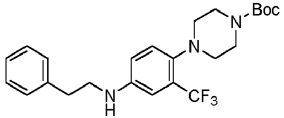

Method A was performed to obtain 288 mg of yellow oil product, with a yield of 64%. ¹H NMR (400 MHz, Chloroform-*d*) δ 7.36 (t, *J* = 7.3 Hz, 2H), 7.28 (t, *J* = 8.6 Hz, 3H), 7.20 (d, *J* = 8.6 Hz, 1H), 6.88 (d, *J* = 2.3 Hz, 1H), 6.79 - 6.70 (m, 1H), 4.06 (s, 1H), 3.59 (s, 4H), 3.41 (t, *J* = 7.0 Hz, 2H), 2.94 (t, *J* = 6.9 Hz, 2H), 2.82 (t, *J* = 5.0 Hz, 4H), 1.57 (s, 9H). ¹³C NMR (101 MHz, Chloroform-*d*) δ 155.0, 145.7, 141.6, 139.1, 128.8, 128.7, 127.0 (q, *J* = 295.3 Hz, 1C), 126.6, 125.4, 116.0,110.6 (q, *J* = 4.9 Hz, 1C), 79.6, 53.6, 45.1, 35.4, 28.5. ¹⁹F NMR (376 MHz, Chloroform-*d*) δ -60.46.

### Example 141

### Tert-butyl 4-(5-(phenylethylamino)pyridin-2-yl)piperazine-1-carboxylate (M7-75)

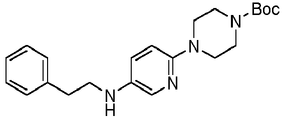

Method A was performed to obtain 340 mg of brown oil product, with a yield of 45%. ¹H NMR (400 MHz, Chloroform-*d*) δ 7.71 (d, *J* = 2.7 Hz, 1H), 7.29 (t, *J* = 7.4 Hz, 2H), 7.24 - 7.15 (m, 3H), 6.90 (dd, *J* = 8.9, 2.8 Hz, 1H), 6.59 (d, *J* = 8.9 Hz, 1H), 3.61 - 3.47 (m, 5H), 3.32 (q, *J* = 5.6, 4.7 Hz, 6H), 2.86 (t, *J* = 6.9 Hz, 2H), 1.48 (s, 9H). ¹³C NMR (101 MHz, Chloroform-*d*) δ 154.8, 153.4, 139.2, 137.5, 133.3, 128.8, 128.6, 126.5, 124.1, 109.1, 79.7, 46.9, 45.9, 35.5, 28.5.

### Example 142

### Tert-butyl 4-(4-(phenylethylamino)benzyl)piperazine-l-carboxylate M7-76

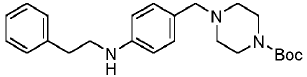

Method A was performed to obtain 230 mg of yellow oil product, with a yield of 29%. ¹H NMR (400 MHz, Chloroform-*d*) δ 7.30 (dd, *J* = 34.6, 6.5 Hz, 5H), 7.16 (d, *J* = 7.3 Hz, 2H), 6.61 (d, *J=* 7.3 Hz, 2H), 3.89 (s, 1H), 3.65 - 3.32 (m, 8H), 2.93 (s, 2H), 2.41 (s, 4H), 1.53 (s, 9H). ¹³C NMR (101 MHz, Chloroform-*d*) δ 154.8, 147.4, 139.4, 130.5, 128.9, 128.7, 126.5, 126.2, 112.7, 79.4, 62.8, 52.8, 45.2, 35.6, 28.6.

### Example 143

### Tert-butyl 4-(2-(phenylethylamino)phenyl)piperazine-1-carboxylate (M7-77)

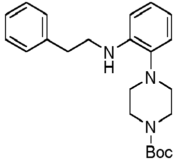

Method A was performed to obtain 314 mg of colourless oil product, with a yield of 82%. ¹H NMR (400 MHz, Chloroform-*d*) δ 7.29 (d, *J* = 14.8 Hz, 2H), 7.25 - 7.18 (m, 3H), 7.03 (td, *J=* 7.8, 1.4 Hz, 1H), 6.92 (dd, *J* = 8.0, 1.4 Hz, 1H), 6.65 (ddt, *J* = 7.8, 4.0, 1.5 Hz, 2H), 4.67 (s, 1H), 3.71 - 2.44 (m, 12H), 1.48 (s, 9H). ¹³C NMR (101 MHz, Chloroform-*d*) δ 154.8, 143.0, 139.5, 138.6, 128.9, 128.6, 126.5, 125.5, 119.8, 116.7, 110.3, 79.8, 51.2, 44.5, 35.4, 28.5.

### Example 144

### 3-(4-methylpiperazin-1-yl)-N-phenethylaniline (M7-78)

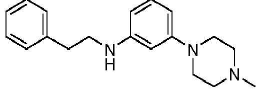

Method A was performed to obtain 33 mg of yellow oil product, with a yield of 11%. ¹H NMR (400 MHz, Chloroform-*d*) δ 7.36 (dd, *J=* 10.8, 4.4 Hz, 2H), 7.27 (t, *J=* 6.7 Hz, 3H), 7.11 (td, *J* = 8.4, 1.6 Hz, 1H), 6.37 (d, *J* = 8.4 Hz, 1H), 6.21 (d, *J* = 5.8 Hz, 2H), 3.77 (s, 1H), 3.43 (t, *J* = 7.0 Hz, 2H), 3.31 - 3.15 (m, 4H), 2.95 (t, *J=* 7.0 Hz, 2H), 2.68 - 2.54 (m, 4H), 2.39 (s, 3H). ¹³C NMR (101 MHz, Chloroform-*d*) δ 152.5, 149.0, 139.4, 129.9, 128.8, 128.6, 126.4, 106.0, 105.1, 101.0, 55.2, 49.2, 46.1, 45.2, 35.7.

### Example 145

### N-(4-morpholinbenzyl)-2-phenylethane-1-amine (M7-79)

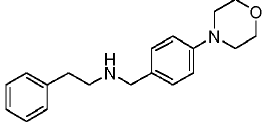

Method B was performed to obtain 132 mg of white solid product, with a yield of 44%.

### Example 146

### N-(4-(4-methylpiperazin-1-yl)benzyl)-2-phenylethane-1-amine (M7-80)

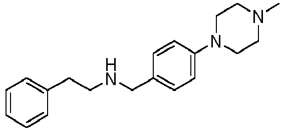

Method B was performed to obtain 270 mg of white solid product, with a yield of 87%.

### Example 147

### Tert-butyl 4-(4-((phenethylamino)methyl)phenyl)piperazine-1-carboxylate (M7-81)

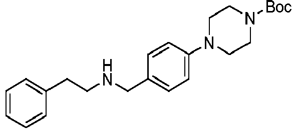

Method B was performed to obtain 185 mg of white solid product, with a yield of 94%. ¹H NMR (400 MHz, Chloroform-*d*) δ 7.23 - 7.15 (m, 2H), 7.10 (dd, *J=* 7.1, 5.0 Hz, 5H), 6.78 (d, *J=* 8.6 Hz, 2H), 3.63 (s, 2H), 3.55 - 3.42 (m, 4H), 3.12 - 2.93 (m, 4H), 2.80 (t, *J=* 6.8 Hz, 2H), 2.72 (t, *J* = 6.5 Hz, 2H), 1.52 (s, 1H), 1.40 (s, 9H). ¹³C NMR (101 MHz, Chloroform-*d*) δ 154.7, 150.3, 140.1, 132.1, 129.1, 128.7, 128.5, 126.1, 116.6, 79.9, 53.3, 50.5, 49.6, 36.4, 28.5.

### Example 148

### N-(4-phenoxybenzyl)-2-phenylethane-1-amine (M7-86)

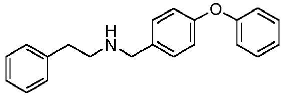

Method B was performed to obtain 497 mg of light yellow liquid product, with a yield of 82%.

### Example 149

### Tert-butyl 4-(2-((phenethylamino)methyl)phenyl)piperazine-1-carboxylate (M7-87)

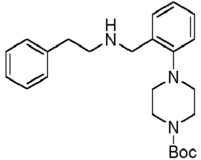

Method B was performed to obtain 326 mg of white solid product, with a yield of 83%. ¹H NMR (400 MHz, Chloroform-*d*) δ 7.37 - 7.31 (m, 2H), 7.29 (d, *J=* 6.8 Hz, 1H), 7.25 - 7.14 (m, 5H), 7.14 - 7.08 (m, 1H), 5.28 (s, 1H), 4.18 (s, 2H), 3.28 (s, 4H), 3.16 (t, *J=* 6.9 Hz, 2H), 3.00 (t, *J* = 6.8 Hz, 2H), 2.66 (s, 4H), 1.47 (s, 9H). ¹³C NMR (101 MHz, Chloroform-*d*) δ 154.6, 151.1, 136.7, 131.5, 130.5, 129.1, 128.8, 127.6, 127.3, 125.8, 121.3, 80.0, 53.5, 52.7, 49.5, 48.9, 33.1, 28.4.

### Example 150

### Tert-butyl 4-(3-((phenethylamino)methyl)phenyl)piperazine-1-carboxylate (M7-88)

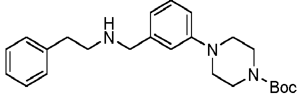

Method B was performed to obtain 200 mg of white solid product, with a yield of 50%. ¹H NMR (400 MHz, Chloroform-*d*) δ 7.63 (s, 2H), 7.30 - 7.13 (m, 5H), 6.84 (dd, *J* = 18.3, 6.7 Hz, 2H), 3.99 (s, 2H), 3.61 - 3.34 (m, 4H), 3.20 - 2.72 (m, 8H), 1.95 (s, 1H), 1.47 (s, 9H). ¹³C NMR (101 MHz, Chloroform-*d*) δ 154.8, 151.6, 136.5, 132.0, 129.9, 128.8, 128.8, 127.1, 120.9, 117.3, 116.7, 80.1, 51.8, 48.6, 48.3, 32.5, 28.4.

### Example 151

### ethyl 3-methyl-5-(N-phenyl-N-phenethylsulfamoyl)benzofuran-2-carboxylate (M8-10)

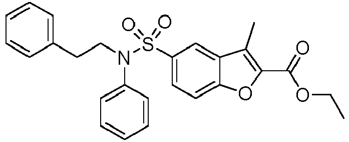

Ethyl 3-methyl-5-(chlorosulfonyl)benzofuran-2-carboxylate (91 mg, 0.3 mmol), N-phenethylaniline (59 mg, 0.3 mmol) and potassium carbonate (46 mg, 0.33 mmol) were dissolved in anhydrous dichloromethane (10 mL), and the resulting solution was stirred at 60 °C overnight. After the reaction was found to be completed from the monitoring of the TLC, the solvent was distilled off under reduced pressure, and the obtained product was dissolved in water (10 mL) and then extracted three times with dichloromethane (20 mL). The organic phase was washed with saturated aqueous NaCl solution and then dried with anhydrous sodium sulfate. The crude product was separated by column chromatography (PE/EA = 10/1 ~ 5/1) to obtain 90 mg of yellow solid, with a yield of 65%. ¹H NMR (400 MHz, Chloroform-*d*) δ 7.88 (s, 1H), 7.63 (d, *J* = 8.8 Hz, 1H), 7.56 (d, *J* = 8.7 Hz, 1H), 7.39 - 7.31 (m, 3H), 7.30 - 7.24 (m, 2H), 7.23 - 7.18 (m, 1H), 7.17 - 7.10 (m, 2H), 7.09 - 7.00 (m, 2H), 4.48 (q, *J* = 7.1 Hz, 2H), 3.90 - 3.77 (m, 2H), 2.89 - 2.76 (m, 2H), 2.54 (s, 3H), 1.46 (t, *J* = 7.1 Hz, 3H). ¹³C NMR (101 MHz, Chloroform-*d*) δ 159.9, 155.9, 142.9, 139.0, 138.0, 133.6, 129.2, 128.9, 128.8, 128.5, 128.2, 126.8, 126.6, 125.6, 122.0, 112.6, 61.5, 52.2, 35.2, 14.4, 9.3. HRMS (ESI) [M + H] ⁺, theoretically calculated for C₂₆H₂₆NO₅S: 464.1532, found 464.1525.

### Example 152

### ethyl 3-methyl-5-(N-(4-benzylphenyl)-N-phenethylsulfamoyl)benzofuran-2-carboxylate (M8-11)

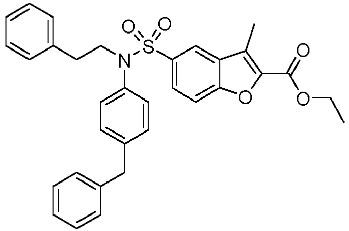

In accordance with the preparation method of ethyl 5-(*N*-phenyl-*N*-phenethylsulfamoyl)-3-methylbenzofuran-2-carboxylate (M8-10), this example was implemented under the same condition except that M7-11 was used instead of N-phenethylaniline. 193 mg of white solid was obtained, with a yield of 70%. ¹H NMR (400 MHz, Chloroform-*d*) δ 7.85 (d, *J* = 1.8 Hz, 1H), 7.64 (dd, *J* = 8.8, 1.9 Hz, 1H), 7.53 (d, *J* = 8.8 Hz, 1H), 7.31 (t, *J* = 7.3 Hz, 2H), 7.26 - 7.08 (m, 10H), 7.03 - 6.93 (m, 2H), 4.46 (q, *J* = 7.1 Hz, 2H), 3.99 (s, 2H), 3.89 - 3.72 (m, 2H), 2.88 - 2.73 (m, 2H), 2.49 (s, 3H), 1.45 (t, *J* = 7.1 Hz, 3H). ¹³C NMR (101 MHz, Chloroform-*d*) δ 159.9, 155.8, 142.9, 141.4, 140.5, 138.1, 137.0, 133.6, 129.6, 129.1, 129.0, 128.9, 128.9, 128.8, 128.6, 128.5, 126.8, 126.6, 126.4, 125.6, 122.1, 112.6, 61.5, 52.2, 41.5, 35.2, 14.4, 9.3.

### Example 153

### ethyl 3-methyl-5-(N-(4-phenoxyphenyl)-N-phenethylsulfamoyl)benzofuran-2-carboxylate (M8-12)

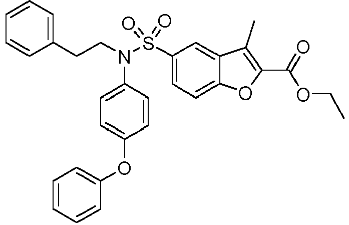

In accordance with the preparation method of ethyl 5-(*N*-phenyl-*N*-phenethylsulfamoyl)-3-methylbenzofuran-2-carboxylate (M8-10), this example was implemented under the same condition except that M7-12 was used instead of N-phenethylaniline. 123 mg of white solid was obtained, with a yield of 44%. ¹H NMR (400 MHz, Chloroform-*d*) δ 7.92 (d, *J* = 1.9 Hz, 1H), 7.67 (dd, *J* = 8.8, 1.9 Hz, 1H), 7.58 (d, *J=* 8.8 Hz, 1H), 7.39 (t, *J* = 7.9 Hz, 2H), 7.28 (t, *J* = 7.2 Hz, 2H), 7.18 (dt, *J* = 22.1, 7.0 Hz, 4H), 7.07 (t, *J* = 7.8 Hz, 2H), 7.02 - 6.97 (m, 2H), 6.93 (d, *J* = 9.0 Hz, 2H), 4.49 (q, *J* = 7.1 Hz, 2H), 3.93 - 3.65 (m, 2H), 2.94 - 2.79 (m, 2H), 2.57 (s, 3H), 1.47 (t, *J* = 7.1 Hz, 3H). ¹³C NMR (101 MHz, Chloroform-*d*) δ 159.9, 157.3, 156.3, 155.9, 142.9, 138.0, 133.6, 133.6, 130.4, 129.9, 129.2, 128.8, 128.5, 126.8, 126.6, 125.6, 124.1, 122.0, 119.5, 118.6, 112.7, 61.6, 52.3, 35.2, 14.4, 9.3.

### Example 154

### ethyl 3-methyl-5-(N,N-diphenylethylsulfamoyl)benzofuran-2-carboxylate (M8-13)

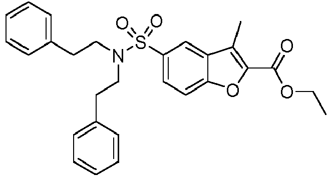

In accordance with the preparation method of ethyl 5-(*N*-phenyl-*N*-phenethylsulfamoyl)-3-methylbenzofuran-2-carboxylate (M8-10), this example was implemented under the same condition except that M7-13 was used instead of N-phenethylaniline. 112 mg of white oil product was obtained, with a yield of 76%. ¹H NMR (400 MHz, Chloroform-*d*) δ 8.16 (s, 1H), 7.84 (d, *J* = 8.7 Hz, 1H), 7.60 (d, *J* = 8.7 Hz, 1H), 7.29 (t, *J* = 7.2 Hz, 4H), 7.22 (t, *J* = 7.3 Hz, 2H), 7.16 (d, *J* = 7.0 Hz, 4H), 4.49 (q, *J* = 7.1 Hz, 2H), 3.56 - 3.40 (m, 4H), 2.96 - 2.82 (m, 4H), 2.63 (s, 3H), 1.48 (t, *J* = 7.1 Hz, 3H). ¹³C NMR (101 MHz, Chloroform-*d*) δ 159.9, 155.7, 142.9, 138.4, 135.4, 129.4, 128.8, 128.6, 126.6, 126.3, 125.6, 121.5, 113.0, 61.6, 50.0, 35.6, 14.4, 9.4. HRMS (ESI) [M + H] ⁺, theoretically calculated for C₂₈H₃₀NO₅S: 492.1845, found 492.1836.

### Example 155

### ethyl 3-methyl-5-(N-(4-morpholinophenyl)-N-phenethylsulfamoyl)benzofuran-2-carboxylate (M8-63)

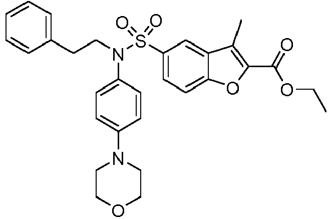

In accordance with the preparation method of ethyl 5-(*N*-phenyl-*N*-phenethylsulfamoyl)-3-methylbenzofuran-2-carboxylate (M8-10), this example was implemented under the same condition except that M7-63 was used instead of N-phenethylaniline. 158 mg of white solid was obtained, with a yield of 96%. ¹H NMR (400 MHz, Chloroform-*d*) δ 7.90 (s, 1H), 7.65 - 7.57 (m, 1H), 7.54 (d, *J* = 8.7 Hz, 1H), 7.21 (dd, *J* = 18.1, 7.2 Hz, 3H), 7.11 (d, *J* = 7.1 Hz, 2H), 6.90 (d, *J* = 8.8 Hz, 2H), 6.81 (d, *J* = 8.8 Hz, 2H), 4.46 (q, *J* = 7.1 Hz, 2H), 3.92 - 3.81 (m, 4H), 3.79 - 3.69 (m, 2H), 3.22 - 3.12 (m, 4H), 2.83 - 2.72 (m, 2H), 2.53 (s, 3H), 1.44 (t, *J* = 7.1 Hz, 3H). ¹³C NMR (101 MHz, Chloroform-*d*) δ 159.9, 155.8, 150.8, 142.8, 138.2, 133.9, 130.2, 129.8, 129.1, 128.8, 128.5, 126.9, 126.5, 125.6, 122.0, 115.4, 112.6, 66.8, 61.5, 52.3, 48.8, 35.1, 14.4, 9.4. HRMS (ESI) [M + H] ⁺, theoretically calculated for C₃₀H₃₃N₂O₆S: 549.2059, found 549.2054.

### Example 156

### ethyl 3-methyl-5-(N-(4-(4-methylpiperazin-1-yl)phenyl)-N-phenethylsulfamoyl)benzofuran-2-carboxylate (M8-64)

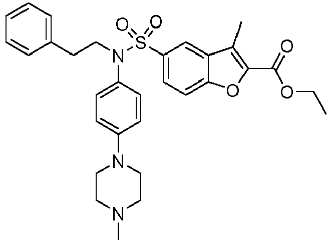

In accordance with the preparation method of ethyl 5-(*N*-phenyl-*N*-phenethylsulfamoyl)-3-methylbenzofuran-2-carboxylate (M8-10), this example was implemented under the same condition except that M7-64 was used instead of N-phenethylaniline. 56 mg of white oil product was obtained, with a yield of 84%. ¹H NMR (400 MHz, Chloroform-*d*) δ 7.90 (s, 1H), 7.63 (d, *J* = 8.8 Hz, 1H), 7.55 (d, *J* = 8.7 Hz, 1H), 7.31 - 7.22 (m, 2H), 7.22 - 7.16 (m, 1H), 7.15 - 7.07 (m, 2H), 6.89 (d, *J* = 9.0 Hz, 2H), 6.82 (d, *J* = 9.1 Hz, 2H), 4.47 (q, *J* = 7.1 Hz, 2H), 3.86 - 3.69 (m, 2H), 3.33 - 3.18 (m, 4H), 2.84 - 2.74 (m, 2H), 2.63 - 2.55 (m, 4H), 2.54 (s, 3H), 2.36 (s, 3H), 1.45 (t, *J* = 7.1 Hz, 3H). ¹³C NMR (101 MHz, Chloroform-*d*) δ 159.9, 155.8, 150.7, 142.8, 138.2, 133.9, 129.8, 129.7, 129.7, 129.1, 128.8, 128.5, 126.9, 126.5, 125.6, 122.0, 115.7, 112.5, 61.5, 55.0, 52.3, 48.5, 46.1, 35.1, 14.4, 9.4. HRMS (ESI) [M + H] ⁺, theoretically calculated for C₃₁H₃₆N₃O₅S: 562.2376, found 562.2380.

### Example 157

### ethyl 3-methyl-5-(N-(4-(4-(tert-butoxycarbonyl)piperazin-1-yl)phenyl)-N-phenethylsulfamoyl)benzofuran-2-carboxylate (M8-65)

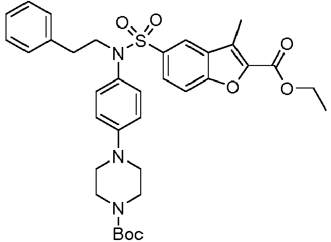

In accordance with the preparation method of ethyl 5-(*N*-phenyl-*N*-phenethylsulfamoyl)-3-methylbenzofuran-2-carboxylate (M8-10), this example was implemented under the same condition except that M7-65 was used instead of N-phenethylaniline. 288 mg of white oil product was obtained, with a yield of 74%. ¹H NMR (400 MHz, Chloroform-*d*) δ 7.88 (s, 1H), 7.60 (d, *J* = 8.8 Hz, 1H), 7.51 (d, *J* = 8.8 Hz, 1H), 7.25 - 7.11 (m, 3H), 7.08 (d, *J* = 6.9 Hz, 2H), 6.89 (d, *J* = 8.9 Hz, 2H), 6.80 (d, *J=* 9.0 Hz, 2H), 4.43 (q, *J* = 7.1 Hz, 2H), 3.84 - 3.68 (m, 2H), 3.61 - 3.50 (m, 4H), 3.22 - 3.02 (m, 4H), 2.80 - 2.70 (m, 2H), 2.51 (s, 3H), 1.48 (s, 9H), 1.41 (t, *J* = 7.1 Hz, 3H). ¹³C NMR (101 MHz, Chloroform-*d*) δ 159.8, 155.8, 154.6, 150.7, 142.8, 138.2, 133.9, 130.3, 129.8, 129.1, 128.8, 128.4, 126.9, 126.5, 125.6, 122.0, 116.2, 112.5, 79.9, 79.6, 61.5, 52.3, 48.7, 35.1, 28.4, 14.4, 9.3. HRMS (ESI) [M + H] ⁺, theoretically calculated for C₃₅H₄₂N₃O₇S: 648.2743, found 648.2745.

### Example 158

### ethyl 3-methyl-5-(N-(4-(4-(3,3-dimethylbutyryl)piperazin-1-yl)phenyl)-N-phenethylsulfamoyl)benzofuran-2-carboxylate (M8-66)

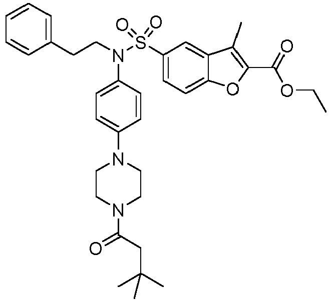

In accordance with the preparation method of ethyl 5-(*N*-phenyl-*N*-phenethylsulfamoyl)-3-methylbenzofuran-2-carboxylate (M8-10), this example was implemented under the same condition except that M7-66was used instead of N-phenethylaniline. 308 mg of white solid was obtained, with a yield of 95%. ¹H NMR (400 MHz, Chloroform-*d*) δ 7.85 (d, *J* = 1.8 Hz, 1H), 7.56 (dd, *J* = 8.7, 1.9 Hz, 1H), 7.48 (d, *J* = 8.8 Hz, 1H), 7.22 - 7.01 (m, 5H), 6.95 - 6.71 (m, 4H), 4.40 (q, *J* = 7.1 Hz, 2H), 3.82 - 3.58 (m, 6H), 3.23 - 3.07 (m, 4H), 2.83 - 2.64 (m, 2H), 2.48 (s, 3H), 2.27 (s, 2H), 1.38 (t, *J* = 7.1 Hz, 3H), 1.03 (s, 9H). ¹³C NMR (101 MHz, Chloroform-*d*) δ 170.4, 159.8, 155.8, 150.4, 142.8, 138.1, 133.8, 130.4, 129.8, 129.1, 128.8, 128.4, 126.9, 126.5, 125.5, 121.9, 116.1, 112.5, 61.5, 52.2, 49.0, 48.8, 46.3, 44.6, 41.1, 35.1, 31.4, 30.1, 14.4, 9.4.

### Example 159

### ethyl 3-methyl-5-(N-(4-(4-(propoxycarbonyl)piperazin-1-yl)phenyl)-N-phenethylsulfamoyl)benzofuran-2-carboxylate (M8-67)

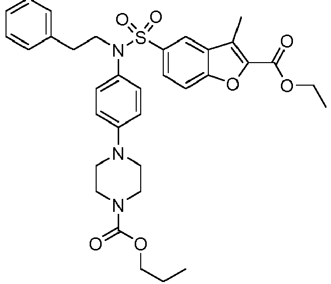

In accordance with the preparation method of ethyl 5-(*N*-phenyl-*N*-phenethylsulfamoyl)-3-methylbenzofuran-2-carboxylate (M8-10), this example was implemented under the same condition except that M7-67 was used instead of N-phenethylaniline. 308 mg of white solid was obtained, with a yield of 97%. ¹H NMR (400 MHz, Chloroform-*d*) δ 7.88 (d, *J* = 1.8 Hz, 1H), 7.60 (dd, *J* = 8.8, 1.8 Hz, 1H), 7.52 (d, *J* = 8.7 Hz, 1H), 7.23 (dd, *J* = 8.0, 6.4 Hz, 2H), 7.19 - 7.14 (m, 1H), 7.12 - 7.05 (m, 2H), 6.93 - 6.86 (m, 2H), 6.84 - 6.77 (m, 2H), 4.44 (q, *J* = 7.1 Hz, 2H), 4.07 (t, *J* = 6.7 Hz, 2H), 3.81 - 3.70 (m, 2H), 3.66 - 3.58 (m, 4H), 3.22 - 3.09 (m, 4H), 2.82 - 2.68 (m, 2H), 2.52 (s, 3H), 1.67 (h, *J=* 7.2 Hz, 2H), 1.43 (t, *J=* 7.1 Hz, 3H), 0.95 (t, *J* = 7.4 Hz, 3H). ¹³C NMR (101 MHz, Chloroform-*d*) δ 159.9, 155.8, 155.5, 150.7, 142.8, 138.2, 133.9, 130.5, 129.8, 129.1, 128.8, 128.5, 126.9, 126.5, 125.6, 122.0, 116.3, 112.6, 67.2, 61.5, 52.3, 48.8, 43.5, 35.1, 22.4, 14.4, 10.4, 9.4.

### Example 160

### ethyl 3-methyl-5-(N-(4-(4-acetylpiperazin-1-yl)phenyl)-N-phenethylsulfamoyl)benzofuran-2-carboxylate (M8-68)

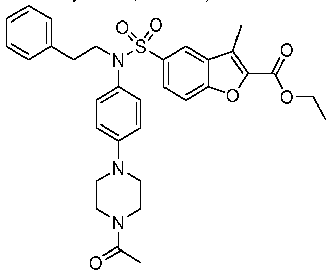

In accordance with the preparation method of ethyl 5-(*N*-phenyl-*N*-phenethylsulfamoyl)-3-methylbenzofuran-2-carboxylate (M8-10), this example was implemented under the same condition except that M7-68 was used instead of N-phenethylaniline. 254 mg of white solid was obtained, with a yield of 86%. ¹H NMR (400 MHz, Chloroform-*d*) δ 7.82 (d, *J* = 1.8 Hz, 1H), 7.58 - 7.42 (m, 2H), 7.21 - 7.08 (m, 3H), 7.07 - 6.99 (m, 2H), 6.89 - 6.70 (m, 4H), 4.38 (q, *J* = 7.1 Hz, 2H), 3.77 - 3.62 (m, 4H), 3.56 (t, *J* = 5.1 Hz, 2H), 3.12 (dt, *J* = 16.2, 5.3 Hz, 4H), 2.76 - 2.63 (m, 2H), 2.46 (s, 3H), 2.07 (s, 3H), 1.37 (t, *J=* 7.1 Hz, 3H). ¹³C NMR (101 MHz, Chloroform-*d*) δ 169.1, 159.9, 155.8, 150.4, 142.9, 138.1, 133.9, 130.7, 129.9, 129.2, 128.9, 128.5, 126.9, 126.6, 125.6, 122.0, 116.3, 112.6, 61.6, 52.3, 49.1, 48.8, 46.1, 41.2, 35.1, 21.4, 14.4, 9.4.

### Example 161

### ethyl 3-methyl-5-(N-(4-(4-benzoylpiperazin-1-yl)phenyl)-N-phenethylsulfamoyl)-3-methylbenzofuran-2-carboxylate (M8-69)

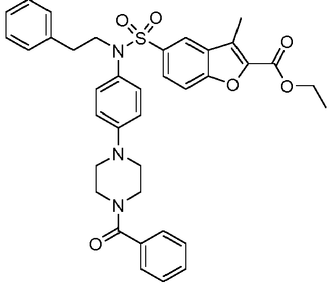

In accordance with the preparation method of ethyl 5-(*N*-phenyl-*N*-phenethylsulfamoyl)-3-methylbenzofuran-2-carboxylate (M8-10), this example was implemented under the same condition except that M7-69 was used instead of N-phenethylaniline. 282 mg of white solid was obtained, with a yield of 86%. ¹H NMR (400 MHz, Chloroform-*d*) δ 7.91 (d, *J* = 1.7 Hz, 1H), 7.61 (dd, *J* = 8.8, 1.9 Hz, 1H), 7.54 (d, *J* = 8.7 Hz, 1H), 7.44 (s, 5H), 7.28 - 7.15 (m, 3H), 7.15 - 7.07 (m, 2H), 6.97 - 6.89 (m, 2H), 6.87 - 6.79 (m, 2H), 4.46 (q, *J* = 7.1 Hz, 2H), 3.92 (s, 2H), 3.79 - 3.73 (m, 2H), 3.61 (s, 2H), 3.22 (d, *J* = 22.6 Hz, 4H), 2.84 - 2.70 (m, 2H), 2.54 (s, 3H), 1.44 (t, *J* = 7.1 Hz, 3H). ¹³C NMR (101 MHz, Chloroform-*d*) δ 170.4, 159.9, 155.8, 150.4, 142.8, 138.1, 135.5, 133.9, 130.7, 130.0, 129.9, 129.1, 128.8, 128.6, 128.5, 127.1, 126.9, 126.5, 125.6, 122.0, 116.4, 112.6, 61.6, 52.3, 49.1, 35.1, 14.4, 9.4.

### Example 162

### ethyl 3-methyl-5-(N-(4-(4-benzylpiperazin-1-yl)phenyl)-N-phenethylsulfamoyl)benzofuran-2-carboxylate (M8-70)

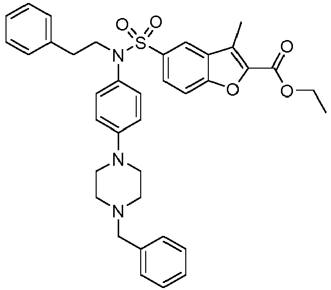

In accordance with the preparation method of ethyl 5-(*N*-phenyl-*N*-phenethylsulfamoyl)-3-methylbenzofuran-2-carboxylate (M8-10), this example was implemented under the same condition except that M7-70 was used instead of N-phenethylaniline. 251 mg of white solid was obtained, with a yield of 79%. ¹H NMR (400 MHz, Chloroform-*d*) δ 7.93 (d, *J* = 1.3 Hz, 1H), 7.65 (dd, *J* = 8.8, 1.6 Hz, 1H), 7.57 (d, *J* = 8.7 Hz, 1H), 7.37 (q, *J* = 7.4 Hz, 4H), 7.29 (dd, *J* = 14.7, 7.4 Hz, 3H), 7.21 (t, *J* = 7.2 Hz, 1H), 7.15 (d, *J* = 7.0 Hz, 2H), 6.91 (d, *J* = 8.9 Hz, 2H), 6.84 (d, *J* = 9.0 Hz, 2H), 4.49 (q, *J* = 7.1 Hz, 2H), 3.84 - 3.75 (m, 2H), 3.61 (s, 2H), 3.31 - 3.21 (m, 4H), 2.85 - 2.78 (m, 2H), 2.68 - 2.61 (m, 4H), 2.56 (s, 3H), 1.47 (t, *J* = 7.1 Hz, 3H). ¹³C NMR (101 MHz, Chloroform-*d*) δ 159.9, 155.8, 150.9, 142.8, 138.3, 137.8, 133.9, 129.7, 129.7, 129.2, 129.1, 128.9, 128.5, 128.3, 127.2, 127.0, 126.5, 125.7, 122.1, 115.7, 112.5, 63.0, 61.5, 53.0, 52.4, 48.5, 35.2, 14.4, 9.4. HRMS (ESI) [M + H] ⁺, theoretically calculated for C₃₇H₄₀N₃O₅S: 638.2689, found 638.2697.

### Example 163

### ethyl 3-methyl-5-(N-(4-(4-(dimethylcarbamoyl)piperazin-1-yl)phenyl)-N-phenethylsulfamoyl)benzofuran-2-carboxylate (M8-71)

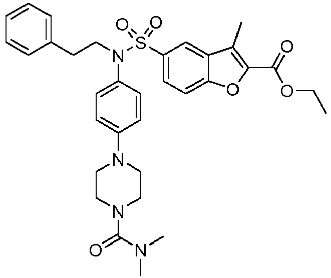

In accordance with the preparation method of ethyl 5-(*N*-phenyl-*N*-phenethylsulfamoyl)-3-methylbenzofuran-2-carboxylate (M8-10), this example was implemented under the same condition except that M7-71 was used instead of N-phenethylaniline. 143 mg of white solid was obtained, with a yield of 92%. ¹H NMR (400 MHz, Chloroform-*d*) δ 7.87 (d, *J* = 1.8 Hz, 1H), 7.58 (dd, *J* = 8.7, 1.9 Hz, 1H), 7.51 (d, *J* = 8.7 Hz, 1H), 7.21 (t, *J* = 7.2 Hz, 2H), 7.14 (dd, *J* = 8.6, 5.9 Hz, 1H), 7.11 - 7.03 (m, 2H), 6.87 (d, *J* = 9.0 Hz, 2H), 6.79 (d, *J* = 9.0 Hz, 2H), 4.43 (q, *J* = 7.1 Hz, 2H), 3.82 - 3.67 (m, 2H), 3.46 - 3.29 (m, 4H), 3.25 - 3.14 (m, 4H), 2.85 (s, 6H), 2.80 - 2.70 (m, 2H), 2.51 (s, 3H), 1.41 (t, *J* = 7.1 Hz, 3H). ¹³C NMR (101 MHz, Chloroform-*d*) δ 164.6, 159.9, 155.8, 150.7, 142.8, 138.2, 133.9, 130.2, 129.8, 129.1, 128.8, 128.4, 126.9, 126.5, 125.6, 122.0, 116.0, 112.5, 61.5, 52.3, 48.5, 46.6, 38.4, 35.1, 14.4, 9.4.

### Example 164

### ethyl 3-methyl-5-(N-(4-(4-(methylsulfonyl)piperazin-1-yl)phenyl)-N-phenethylsulfamoyl)benzofuran-2-carboxylate (M8-72)

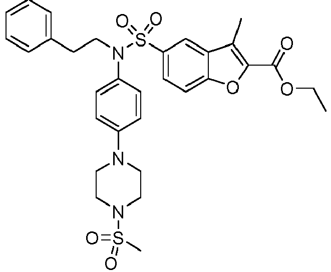

In accordance with the preparation method of ethyl 5-(*N*-phenyl-*N*-phenethylsulfamoyl)-3-methylbenzofuran-2-carboxylate (M8-10), this example was implemented under the same condition except that M7-72 was used instead of N-phenethylaniline. 275 mg of yellow oil product with a yield of 87%. ¹H NMR (400 MHz, Chloroform-*d*) δ 7.87 (d, *J* = 1.8 Hz, 1H), 7.58 (dd, *J* = 8.8, 1.9 Hz, 1H), 7.50 (d, *J* = 8.8 Hz, 1H), 7.20 (t, *J* = 7.2 Hz, 2H), 7.13 (dd, *J* = 8.6, 5.9 Hz, 1H), 7.07 (d, *J* = 6.9 Hz, 2H), 6.89 (d, *J* = 9.0 Hz, 2H), 6.81 (d, *J* = 9.0 Hz, 2H), 4.41 (q, *J* = 7.1 Hz, 2H), 3.79 - 3.69 (m, 2H), 3.39 - 3.31 (m, 4H), 3.30 - 3.22 (m, 4H), 2.80 (s, 3H), 2.77 - 2.68 (m, 2H), 2.50 (s, 3H), 1.40 (t, *J* = 7.1 Hz, 3H). ¹³C NMR (101 MHz, Chloroform-*d*) δ 159.8, 155.8, 150.2, 142.8, 138.1, 133.8, 130.9, 129.9, 129.1, 128.8, 128.5, 126.9, 126.5, 125.5, 122.0, 116.6, 112.6, 61.5, 52.2, 48.7, 45.7, 35.0, 34.5, 14.4, 9.4.

### Example 165

### ethyl 3-methyl-5-(N-(4-(1-(tert-butoxycarbonyl)piperidin-4-yl)phenyl)-N-phenethylsulfamoyl)benzofuran-2-carboxylate (M8-73)

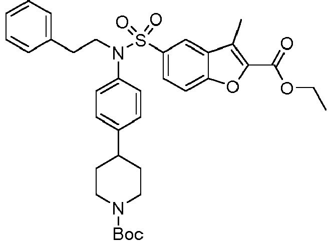

In accordance with the preparation method of ethyl 5-(*N*-phenyl-*N*-phenethylsulfamoyl)-3-methylbenzofuran-2-carboxylate (M8-10), this example was implemented under the same condition except that M7-73 was used instead of N-phenethylaniline. 309 mg of white solid was obtained, with a yield of 95%. ¹H NMR (400 MHz, Chloroform-*d*) δ 7.84 (d, *J* = 1.5 Hz, 1H), 7.61 (dd, *J* = 8.8, 1.8 Hz, 1H), 7.54 (d, *J* = 8.7 Hz, 1H), 7.16 (ddd, *J* = 35.7, 17.1, 7.2 Hz, 7H), 6.96 (d, *J* = 8.4 Hz, 2H), 4.46 (q, *J* = 7.1 Hz, 2H), 4.25 (s, 2H), 3.81 - 3.72 (m, 2H), 2.79 (q, *J* = 10.4, 7.5 Hz, 4H), 2.65 (ddd, *J* = 15.1, 7.6, 3.6 Hz, 1H), 2.51 (s, 3H), 1.82 (d, *J* = 12.4 Hz, 2H), 1.65 - 1.55 (m, 2H), 1.46 (d, *J* = 16.9 Hz, 12H). ¹³C NMR (101 MHz, Chloroform-*d*) δ 159.9, 155.8, 154.8, 145.9, 142.9, 138.1, 137.1, 133.7, 129.1, 128.9, 128.8, 128.5, 127.5, 126.8, 126.5, 125.5, 122.0, 112.6, 79.5, 61.6, 52.2, 42.3, 35.2, 33.1, 28.5, 14.4, 9.3.

### Example 166

### ethyl 3-methyl-5-(N-(4-(4-(tert-butoxycarbonyl)piperazin-1-yl)-3-(trifluoromethyl)phenyl)-N-phenethylsulfamoyl)benzofuran-2-carboxylate (M8-74)

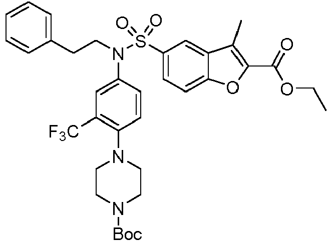

In accordance with the preparation method of ethyl 5-(*N*-phenyl-*N*-phenethylsulfamoyl)-3-methylbenzofuran-2-carboxylate (M8-10), this example was implemented under the same condition except that M7-74 was used instead of N-phenethylaniline. 175 mg of white solid was obtained, with a yield of 48%. ¹H NMR (400 MHz, Chloroform-*d*) δ 7.82 (d, *J* = 1.8 Hz, 1H), 7.65 - 7.54 (m, 2H), 7.25 - 7.09 (m, 6H), 7.08 - 7.00 (m, 2H), 4.44 (q, *J* = 7.1 Hz, 2H), 3.83 - 3.71 (m, 2H), 3.55 (t, *J* = 4.8 Hz, 4H), 2.86 (t, *J* = 4.8 Hz, 4H), 2.81 - 2.73 (m, 2H), 2.50 (s, 3H), 1.45 (d, *J* = 17.8 Hz, 12H). ¹³C NMR (101 MHz, Chloroform-*d*) δ 159.8, 155.9, 154.8, 151.8, 143.1, 137.6, 135.8, 133.9, 133.2, 129.3, 128.8, 128.5, 127.5 (t, *J* = 29.6 Hz, 1C), 127.5 (d, *J* = 5.3 Hz, 1C), 126.6, 126.6, 125.4, 124.5, 123.2 (d, *J* = 274.9 Hz, 1C), 122.0, 112.8, 79.8, 61.6, 53.3, 52.1, 35.2, 28.4, 14.3, 9.2. ¹⁹F NMR (376 MHz, Chloroform-*d*) δ -60.46.

### Example 167

### ethyl 3-methyl-5-(N-(6-(4-(tert-butoxycarbonyl)piperazin-1-yl) pyridin-3-yl)-N-phenethylsulfamoyl)benzofuran-2-carboxylate (M8-75)

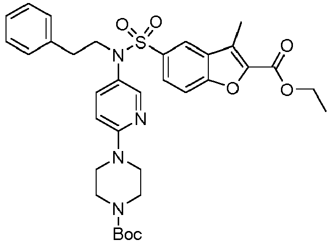

In accordance with the preparation method of ethyl 5-(*N*-phenyl-*N*-phenethylsulfamoyl)-3-methylbenzofuran-2-carboxylate (M8-10), this example was implemented under the same condition except that M7-75 was used instead of N-phenethylaniline. 294 mg of white solid was obtained, with a yield of 75%. ¹H NMR (400 MHz, Chloroform-*d*) δ 7.90 (d, *J* = 1.4 Hz, 1H), 7.72 (d, *J* = 2.5 Hz, 1H), 7.61 (dd, *J* = 8.8, 1.7 Hz, 1H), 7.53 (d, *J* = 8.7 Hz, 1H), 7.21 (t, *J* = 7.2 Hz, 2H), 7.18 - 7.11 (m, 2H), 7.08 (d, *J* = 6.9 Hz, 2H), 6.54 (d, *J* = 9.0 Hz, 1H), 4.43 (q, *J* = 7.1 Hz, 2H), 3.81 - 3.71 (m, 2H), 3.52 (s, 8H), 2.81 - 2.73 (m, 2H), 2.51 (s, 3H), 1.46 (s, 9H), 1.41 (t, *J* = 7.1 Hz, 3H). ¹³C NMR (101 MHz, Chloroform-*d*) δ 159.8, 158.1, 155.8, 154.7, 148.2, 142.9, 138.4, 137.8, 133.8, 129.2, 128.8, 128.5, 126.7, 126.6, 125.5, 125.5, 121.9, 112.8, 106.7, 80.0, 61.5, 52.4, 44.8, 35.1, 28.4, 14.4, 9.4. HRMS (ESI) [M + H] ⁺, theoretically calculated for C₃₄H₄₁N₄O₇S: 649.2696, found 649.2701.

### Example 168

### ethyl 3-methyl-5-(N-(4-((4-(tert-butoxycarbonyl)piperazin-1-yl)methyl)phenyl)-N-phenethylsulfamoyl) benzofuran-2-carboxylate (M8-76)

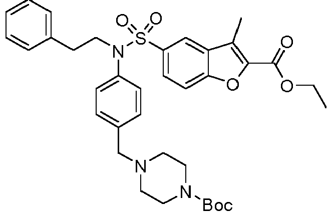

In accordance with the preparation method of ethyl 5-(*N*-phenyl-*N*-phenethylsulfamoyl)-3-methylbenzofuran-2-carboxylate (M8-10), this example was implemented under the same condition except that M7-76 was used instead of N-phenethylaniline. 247 mg of white solid was obtained, with a yield of 74%. ¹H NMR (400 MHz, Chloroform-*d*) δ 7.83 (d, *J* = 1.4 Hz, 1H), 7.58 (dd, *J* = 8.8, 1.7 Hz, 1H), 7.51 (d, *J* = 8.8 Hz, 1H), 7.26 - 7.11 (m, 5H), 7.07 (d, *J* = 7.0 Hz, 2H), 6.96 (d, *J* = 8.3 Hz, 2H), 4.43 (q, *J* = 7.1 Hz, 2H), 3.84 - 3.71 (m, 2H), 3.48 (s, 2H), 3.42 (t, *J* = 5.0 Hz, 4H), 2.83 - 2.72 (m, 2H), 2.49 (s, 3H), 2.36 (t, *J* = 5.0 Hz, 4H), 1.43 (d, *J* = 9.5 Hz, 12H). ¹³C NMR (101 MHz, Chloroform-*d*) δ 159.8, 155.8, 154.8, 142.9, 138.1, 138.0, 137.9, 133.6, 129.6, 129.1, 128.8, 128.7, 128.5, 126.8, 126.5, 125.5, 122.0, 112.6, 79.6, 62.4, 61.5, 52.9, 52.2, 35.2, 28.4, 14.4, 9.3. HRMS (ESI) [M + H] ⁺, theoretically calculated for C₃₆H₄₄N₃O₇S: 662.2900, found 662.2896.

### Example 169

### ethyl 3-methyl-5-(N-(2-(4-(tert-butoxycarbonyl)piperazin-1-yl)phenyl)-N-phenethylsulfamoyl)benzofuran-2-carboxylate (M8-77)

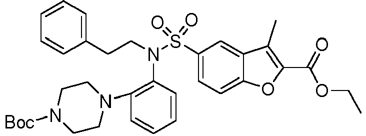

In accordance with the preparation method of ethyl 5-(*N*-phenyl-*N*-phenethylsulfamoyl)-3-methylbenzofuran-2-carboxylate (M8-10), this example was implemented under the same condition except that M7-77 was used instead of N-phenethylaniline. 102 mg of white solid was obtained, with a yield of 31%. ¹H NMR (400 MHz, Chloroform-*d*) δ 8.14 (d, *J* = 1.6 Hz, 1H), 7.89 (dd, *J* = 8.8, 1.8 Hz, 1H), 7.61 (d, *J=* 8.8 Hz, 1H), 7.36 - 7.26 (m, 1H), 7.20 - 6.98 (m, 6H), 6.93 (d, *J* = 6.6 Hz, 2H), 4.46 (q, *J* = 7.1 Hz, 2H), 4.23 - 3.76 (m, 2H), 3.48 (s, 4H), 3.16 (s, 2H), 2.79 (dd, *J=* 40.6, 17.5 Hz, 2H), 2.68 - 2.45 (m, 5H), 1.46 (d, *J=* 12.3 Hz, 12H). ¹³C NMR (101 MHz, Chloroform-*d*) δ 159.8, 155.8, 154.8, 151.2, 143.0, 138.2, 136.1, 133.8, 130.5, 129.4, 129.3, 128.5, 128.4, 126.8, 126.4, 125.5, 124.3, 122.6, 122.0, 112.9, 79.7, 61.5, 51.2, 34.7, 28.4, 14.4, 9.4. HRMS (ESI) [M + H] ⁺, theoretically calculated for C₃₅H₄₂N₃O₇S: 648.2743, found 648.2736.

### Example 170

### ethyl 3-methyl-5-(N-(3-(4-methylpiperazin-1-yl)phenyl)-N-phenethylsulfamoyl)benzofuran-2-carboxylate (M8-78)

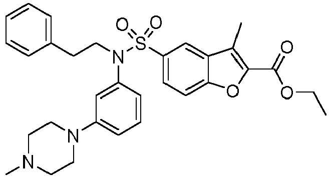

In accordance with the preparation method of ethyl 5-(*N*-phenyl-*N*-phenethylsulfamoyl)-3-methylbenzofuran-2-carboxylate (M8-10), this example was implemented under the same condition except that M7-78 was used instead of N-phenethylaniline. 42 mg of white solid was obtained, with a yield of 76%. ¹H NMR (400 MHz, Chloroform-*d*) δ 7.92 (d, *J* = 1.8 Hz, 1H), 7.65 (dd, *J* = 8.7, 1.9 Hz, 1H), 7.56 (d, *J* = 8.7 Hz, 1H), 7.31 - 7.09 (m, 6H), 6.87 (dd, *J* = 8.3, 2.5 Hz, 1H), 6.65 (t, *J* = 2.2 Hz, 1H), 6.39 (dd, *J* = 7.7, 1.9 Hz, 1H), 4.48 (q, *J* = 7.1 Hz, 2H), 3.88 - 3.71 (m, 2H), 3.26 - 3.09 (m, 4H), 2.89 - 2.77 (m, 2H), 2.62 (t, *J=* 4.8 Hz, 4H), 2.55 (s, 3H), 2.41 (s, 3H), 1.46 (t, *J* = 7.1 Hz, 3H). ¹³C NMR (101 MHz, Chloroform-*d*) δ 159.9, 155.8, 151.7, 142.9, 139.9, 138.1, 133.8, 129.4, 129.1, 128.9, 128.5, 126.9, 126.5, 125.6, 122.1, 118.9, 117.4, 115.4, 112.5, 61.5, 54.8, 52.2, 48.3, 45.8, 35.2, 14.3, 9.3. HRMS (ESI) [M + H] ⁺, theoretically calculated for C₃₁H₃₆N₃O₅S: 562.2376, found 562.2380.

### Example 171

### ethyl 3-methyl-5-(N-(4-morpholinobenzyl)-N-phenethylsulfamoyl)benzofuran-2-carboxylate (M8-79)

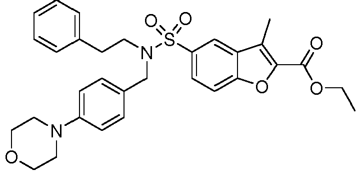

In accordance with the preparation method of ethyl 5-(*N*-phenyl-*N*-phenethylsulfamoyl)-3-methylbenzofuran-2-carboxylate (M8-10), this example was implemented under the same condition except that M7-79 was used instead of N-phenethylaniline. 142 mg of white solid was obtained, with a yield of 63%. ¹H NMR (400 MHz, Chloroform-*d*) δ 8.23 - 8.04 (m, 1H), 7.93 - 7.78 (m, 1H), 7.60 (d, *J* = 8.8 Hz, 1H), 7.15 (dq, *J* = 16.9, 8.2, 7.5 Hz, 5H), 6.96 (d, *J* = 7.1 Hz, 2H), 6.81 (d, *J* = 8.0 Hz, 2H), 4.46 (q, *J* = 7.0 Hz, 2H), 4.32 (s, 2H), 3.83 (s, 4H), 3.43 - 3.28 (m, 2H), 3.11 (s, 4H), 2.74 - 2.53 (m, 5H), 1.44 (t, *J* = 7.1 Hz, 3H). ¹³C NMR (101 MHz, Chloroform-*d*) δ 159.9, 155.7, 150.9, 142.9, 138.5, 135.8, 129.6, 129.3, 128.7, 128.5, 126.9, 126.4, 126.3, 125.6, 121.5, 115.5, 113.0, 66.8, 61.5, 51.5, 49.2, 49.1, 35.3, 14.4, 9.4. HRMS (ESI) [M + H] ⁺, theoretically calculated for C₃₁H₃₅N₂O₆S: 563.2216, found 563.2211.

### Example 172

### ethyl 3-methyl-5-(N-(4-(4-methylpiperazin-1-yl)benzyl)-N-phenethylsulfamoyl)benzofuran-2-carboxylate (M8-80)

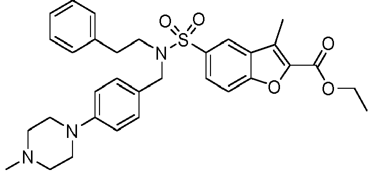

In accordance with the preparation method of ethyl 5-(*N*-phenyl-*N*-phenethylsulfamoyl)-3-methylbenzofuran-2-carboxylate (M8-10), this example was implemented under the same condition except that M7-80 was used instead of N-phenethylaniline. 129 mg of white solid was obtained, with a yield of 75%. ¹H NMR (400 MHz, Chloroform-*d*) δ 8.10 (s, 1H), 7.84 (d, *J* = 8.8 Hz, 1H), 7.59 (d, *J* = 8.8 Hz, 1H), 7.13 (dt, *J* = 22.9, 7.7 Hz, 5H), 6.95 (d, *J* = 6.9 Hz, 2H), 6.81 (d, *J* = 8.6 Hz, 2H), 4.44 (q, *J* = 7.1 Hz, 2H), 4.30 (s, 2H), 3.42 - 3.27 (m, 2H), 3.23 - 3.08 (m, 4H), 2.71 - 2.61 (m, 2H), 2.57 (s, 3H), 2.56 - 2.49 (m, 4H), 2.32 (s, 3H), 1.43 (t, *J* = 7.1 Hz, 3H). ¹³C NMR (101 MHz, Chloroform-*d*) δ 159.9, 155.7, 151.0, 142.9, 138.5, 135.8, 129.6, 129.3, 128.7, 128.5, 126.4, 126.3, 126.3, 125.6, 121.5, 115.8, 113.0, 61.5, 55.0, 51.5, 49.1, 48.8, 46.1, 35.3, 14.4, 9.4. HRMS (ESI) [M + H] ⁺, theoretically calculated for C₃₂H₃₈N₃O₅S: 576.2532, found 576.2535.

### Example 173

### ethyl 3-methyl-5-(N-(4-(4-(tert-butoxycarbonyl)piperazin-1-yl)benzyl)-N-phenethylsulfamoyl)benzofuran-2-carboxylate (M8-81)

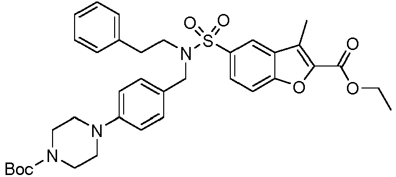

In accordance with the preparation method of ethyl 5-(*N*-phenyl-*N*-phenethylsulfamoyl)-3-methylbenzofuran-2-carboxylate (M8-10), this example was implemented under the same condition except that M7-81 was used instead of N-phenethylaniline. 223 mg of white solid was obtained, with a yield of 85%. ¹H NMR (400 MHz, Chloroform-*d*) δ 8.11 (s, 1H), 7.84 (d, *J* = 8.8 Hz, 1H), 7.60 (d, *J* = 8.8 Hz, 1H), 7.15 (dt, *J* = 15.3, 7.7 Hz, 5H), 6.95 (d, *J* = 6.8 Hz, 2H), 6.82 (d, *J* = 8.6 Hz, 2H), 4.45 (q, *J* = 7.1 Hz, 2H), 4.31 (s, 2H), 3.69 - 3.46 (m, 4H), 3.42 - 3.28 (m, 2H), 3.21 - 3.04 (m, 4H), 2.72 - 2.61 (m, 2H), 2.58 (s, 3H), 1.47 (s, 9H), 1.44 (t, *J* = 7.1 Hz, 3H). ¹³C NMR (101 MHz, Chloroform-*d*) δ 159.9, 155.7, 154.7, 151.0, 142.9, 138.4, 135.8, 129.6, 129.3, 128.7, 128.5, 127.0, 126.4, 126.2, 125.6, 121.5, 116.4, 113.0, 79.9, 61.5, 51.5, 49.1, 35.3, 28.4, 14.4, 9.4. HRMS (ESI) [M + H] ⁺, theoretically calculated for C₃₆H₄₄N₃O₇S: 662.2900, found 662.2891.

### Example 174

### ethyl 3-methyl-5-(N-phenethyl-N-(4-phenoxybenzyl)sulfamoyl)benzofuran-2-carboxylate (M8-86)

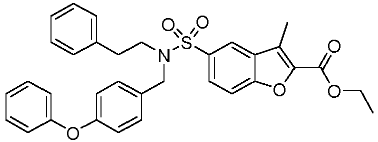

In accordance with the preparation method of ethyl 5-(*N*-phenyl-*N*-phenethylsulfamoyl)-3-methylbenzofuran-2-carboxylate (M8-10), this example was implemented under the same condition except that M7-86 was used instead of N-phenethylaniline. 70 mg of white solid was obtained, with a yield of 25%. ¹H NMR (400 MHz, Chloroform-*d*) δ 8.16 (d, *J* = 1.9 Hz, 1H), 7.88 (dd, *J* = 8.8, 1.9 Hz, 1H), 7.64 (d, *J* = 8.7 Hz, 1H), 7.34 (dd, *J* = 8.6, 7.3 Hz, 2H), 7.26 - 7.08 (m, 6H), 7.03 - 6.91 (m, 6H), 4.48 (q, *J* = 7.1 Hz, 2H), 4.38 (s, 2H), 3.47 - 3.34 (m, 2H), 2.75 - 2.66 (m, 2H), 2.62 (s, 3H), 1.46 (t, *J* = 7.1 Hz, 3H). ¹³C NMR (101 MHz, Chloroform-*d*) δ 159.9, 157.2, 156.9, 155.8, 143.0, 138.3, 135.6, 130.6, 130.0, 129.8, 129.4, 128.7, 128.5, 126.5, 126.2, 125.6, 123.6, 121.5, 119.0, 118.8, 113.1, 61.6, 51.6, 49.4, 35.3, 14.4, 9.4.

### Example 175

### ethyl 3-methyl-5-(N-(2-(4-(tert-butoxycarbonyl)piperazin-1-yl)benzyl)-N-phenethylsulfamoyl)benzofuran-2-carboxylate (M8-87)

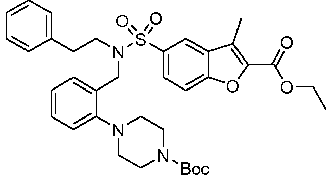

In accordance with the preparation method of ethyl 5-(*N*-phenyl-*N*-phenethylsulfamoyl)-3-methylbenzofuran-2-carboxylate (M8-10), this example was implemented under the same condition except that M7-87 was used instead of N-phenethylaniline. 250 mg of white solid was obtained, with a yield of 75%. ¹H NMR (400 MHz, Chloroform-*d*) δ 8.07 (d, *J* = 1.5 Hz, 1H), 7.80 (dd, *J* = 8.8, 1.8 Hz, 1H), 7.55 (d, *J* = 8.8 Hz, 1H), 7.33 (d, *J* = 7.7 Hz, 1H), 7.23 - 7.16 (m, 1H), 7.12 - 6.98 (m, 5H), 6.88 - 6.78 (m, 2H), 4.49 (s, 2H), 4.39 (q, *J* = 7.1 Hz, 2H), 3.45 (s, 4H), 3.29 - 3.20 (m, 2H), 2.78 - 2.67 (m, 4H), 2.53 (d, *J* = 4.4 Hz, 5H), 1.39 (d, *J* = 9.6 Hz, 12H). ¹³C NMR (101 MHz, Chloroform-*d*) δ 159.9, 155.8, 154.8, 151.4, 143.0, 138.4, 135.5, 130.8, 130.0, 129.4, 128.7, 128.5, 128.5, 126.4, 126.2, 125.5, 124.7, 121.5, 120.2, 113.0, 79.9, 61.6, 52.9, 49.5, 46.4, 34.8, 28.4, 14.4, 9.4. HRMS (ESI) [M + H] ⁺, theoretically calculated for C₃₆H₄₄N₃OS: 662.2900, found 662.2896.

### Example 176

### ethyl 3-methyl-5-(N-(3-(4-(tert-butoxycarbonyl)piperazin-1-yl)benzyl)-N-phenethylsulfamoyl)benzofuran-2-carboxylate (M8-88)

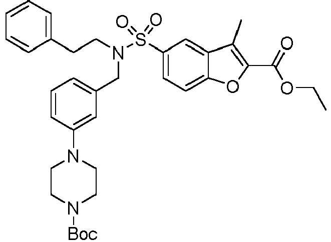

In accordance with the preparation method of ethyl 5-(*N*-phenyl-*N*-phenethylsulfamoyl)-3-methylbenzofuran-2-carboxylate (M8-10), this example was implemented under the same condition except that M7-88 was used instead of N-phenethylaniline. 129 mg of white solid was obtained, with a yield of 49%. ¹H NMR (400 MHz, Chloroform-*d*) δ 8.14 (d, *J* = 1.9 Hz, 1H), 7.86 (dd, *J* = 8.7, 1.9 Hz, 1H), 7.62 (d, *J* = 8.7 Hz, 1H), 7.17 (dddt, *J* = 14.2, 9.9, 7.3, 3.3 Hz, 4H), 6.96 (d, *J* = 6.7 Hz, 2H), 6.84 (d, *J* = 6.1 Hz, 2H), 6.74 (d, *J* = 7.2 Hz, 1H), 4.47 (q, *J* = 7.1 Hz, 2H), 4.35 (s, 2H), 3.62 - 3.50 (m, 4H), 3.42 - 3.32 (m, 2H), 3.07 (t, *J* = 5.2 Hz, 4H), 2.63 (d, *J* = 25.7 Hz, 5H), 1.47 (d, *J* = 11.5 Hz, 12H). ¹³C NMR (101 MHz, Chloroform-*d*) δ 159.9, 155.8, 154.7, 143.0, 138.4, 137.0, 135.6, 129.4, 128.7, 128.5, 126.5, 126.2, 125.6, 121.5, 120.1, 116.3, 116.0, 113.0, 80.0, 61.6, 52.3, 49.4, 49.2, 35.2, 28.4, 14.4, 9.4. HRMS (ESI) [M + H] ⁺, theoretically calculated for C₃₆H₄₄N₃O₇S: 662.2900, found 662.2904.

### Example 177

### 3-methyl-5-(N-phenethyl-N-phenylsulfamoyl)benzofuran-2-carboxylic acid (F27-S10)

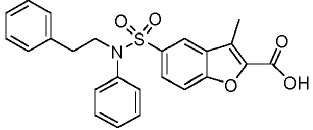

In accordance with the preparation method of 5-(N-benzyl-N-phenethylsulfamoyl)-3-methylbenzofuran-2-carboxylic acid (F27), this example was implemented under the same condition except that M8-10 was used instead of ethyl 5-(N-benzyl-N-phenethylsulfamoyl)-3-methylbenzofuran-2-carboxylate (F27-S8). 50 mg of white solid was obtained, with a yield of 76%. M.p. > 380 °C (for decomposition). ¹H NMR (400 MHz, DMSO-*d*₆) δ 7.73 (s, 1H), 7.67 (d, *J* = 8.7 Hz, 1H), 7.44 (d, *J* = 8.6 Hz, 1H), 7.35 (d, *J* = 6.8 Hz, 3H), 7.21 (dt, *J* = 27.5, 7.2 Hz, 3H), 7.12 (d, *J* = 7.1 Hz, 2H), 7.04 (d, *J* = 7.8 Hz, 2H), 3.82 (t, *J* = 7.2 Hz, 2H), 2.64 (t, *J* = 7.2 Hz, 2H), 2.45 (s, 3H). ¹³C NMR (101 MHz, DMSO-*d*₆) δ 154.9, 139.2, 138.7, 132.2, 130.6, 129.4, 129.2, 129.0, 128.7, 128.3, 126.8, 124.9, 121.0, 112.5, 51.5, 34.6, 9.4. HRMS (ESI) [M - H] ⁻, theoretically calculated for C₂₄H₂₀NO₅S: 434.1062, found 434.1054.

### Example 178

### 3-methyl-5-(N-(4-benzylphenyl)-N-phenethylsulfamoyl)benzofuran-2-carboxylic acid (F27-S11)

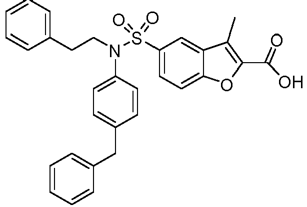

In accordance with the preparation method of 5-(N-benzyl-N-phenethylsulfamoyl)-3-methylbenzofuran-2-carboxylic acid (F27), this example was implemented under the same condition except that M8-11 was used instead of ethyl 5-(N-benzyl-N-phenethylsulfamoyl)-3-methylbenzofuran-2-carboxylate (F27-S8). 92 mg of white solid was obtained, with a yield of 88%. M.p. 301 - 302 °C. ¹H NMR (400 MHz, DMSO-*d*₆) δ 7.90 - 7.66 (m, 2H), 7.56 (dd, *J* = 8.6, 1.9 Hz, 1H), 7.34 (tq, *J* = 15.6, 6.5, 5.5 Hz, 10H), 7.21 (d, *J* = 7.4 Hz, 2H), 7.05 (d, *J* = 8.0 Hz, 2H), 4.04 (s, 2H), 3.87 (t, *J* = 7.3 Hz, 2H), 2.72 (t, *J* = 7.3 Hz, 2H), 2.60 (s, 3H). ¹³C NMR (101 MHz, DMSO-*d*₆) δ 162.6, 154.9, 141.4, 141.4, 138.7, 137.1, 132.2, 130.5, 129.7, 129.2, 129.2, 129.2, 129.0, 128.9, 128.7, 126.7, 126.5, 124.9, 121.1, 112.5, 51.5, 41.0, 34.6, 9.3. HRMS (ESI) [M - H] ⁻, theoretically calculated for C₃₁H₂₆NO₅S: 524.1532, found 524.1533.

### Example 179

### 3-methyl-5-(N-(4-phenoxyphenyl)-N-phenethylsulfamoyl)benzofuran-2-carboxylic acid (F27-S12)

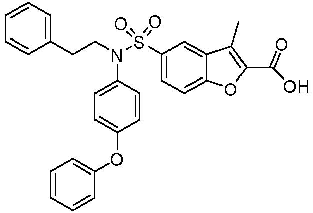

In accordance with the preparation method of 5-(N-benzyl-N-phenethylsulfamoyl)-3-methylbenzofuran-2-carboxylic acid (F27), this example was implemented under the same condition except that M8-12 was used instead of ethyl 5-(N-benzyl-N-phenethylsulfamoyl)-3-methylbenzofuran-2-carboxylate (F27-S8). 85 mg of white solid was obtained, with a yield of 81%. M.p. 285 - 286 °C. ¹H NMR (400 MHz, DMSO-*d*₆) δ 7.85 (d, *J* = 1.9 Hz, 1H), 7.75 (d, *J* = 8.8 Hz, 1H), 7.54 (dd, *J* = 8.7, 1.9 Hz, 1H), 7.46 - 7.38 (m, 2H), 7.25 (t, *J* = 7.1 Hz, 2H), 7.22 - 7.11 (m, 4H), 7.07 - 6.99 (m, 4H), 6.97 - 6.91 (m, 2H), 3.81 (t, *J* = 7.2 Hz, 2H), 2.67 (t, *J* = 7.2 Hz, 2H), 2.51 (s, 3H). ¹³C NMR (101 MHz, DMSO-*d*₆) δ 162.0, 156.7, 156.6, 155.3, 138.7, 134.1, 132.6, 130.8, 130.6, 130.0, 129.2, 128.7, 126.7, 126.0, 124.3, 121.7, 119.4, 119.1, 112.9, 51.6, 34.6, 9.4. HRMS (ESI) [M - H] ⁻, theoretically calculated for C₃₀H₂₄NO₆S: 526.1324, found 526.1316.

### Example 180

### 3-methyl-5-(N,N-diphenylethylsulfamoyl)benzofuran-2-carboxylic acid (F27-S13)

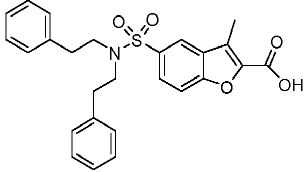

In accordance with the preparation method of 5-(N-benzyl-N-phenethylsulfamoyl)-3-methylbenzofuran-2-carboxylic acid (F27), this example was implemented under the same condition except that M8-13 was used instead of ethyl 5-(N-benzyl-N-phenethylsulfamoyl)-3-methylbenzofuran-2-carboxylate (F27-S8). 54 mg of white solid was obtained, with a yield of 78%. M.p. 212 - 213 °C. ¹H NMR (400 MHz, DMSO-*d*₆) δ 13.75 (s, 1H), 8.25 (s, 1H), 7.96 - 7.86 (m, 1H), 7.82 (d, *J* = 8.8 Hz, 1H), 7.32 - 7.22 (m, 4H), 7.18 (d, *J* = 7.4 Hz, 6H), 3.49 - 3.29 (m, 4H), 2.86 - 2.66 (m, 4H), 2.58 (s, 3H). ¹³CNMR (101 MHz, DMSO-*d*₆) δ 161.2, 155.5, 143.8, 139.0, 135.3, 129.6, 129.2, 128.8, 126.8, 126.6, 125.2, 121.8, 113.5, 49.8, 35.2, 9.6. HRMS (ESI) [M - H] ⁻, theoretically calculated for C₂₆H₂₄NO₅S: 462.1375, found 462.1377.

### Example 181

### 3-methyl-5-(N-(4-morpholinophenyl)-N-phenethylsulfamoyl)benzofuran-2-carboxyli acid (F27-S63)

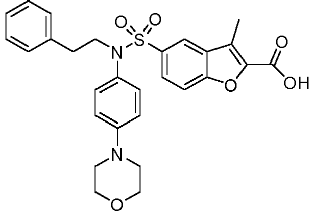

In accordance with the preparation method of 5-(N-benzyl-N-phenethylsulfamoyl)-3-methylbenzofuran-2-carboxylic acid (F27), this example was implemented under the same condition except that M8-63 was used instead of ethyl 5-(N-benzyl-N-phenethylsulfamoyl)-3-methylbenzofuran-2-carboxylate (F27-S8). 81 mg of white solid was obtained, with a yield of 78%. M.p. > 380 °C (for decomposition). ¹H NMR (400 MHz, DMSO-*d*₆) δ 7.74 - 7.58 (m, 2H), 7.43 (d, *J* = 8.4 Hz, 1H), 7.26 (t, *J* = 7.1 Hz, 2H), 7.16 (dd, *J* = 24.8, 7.0 Hz, 3H), 6.85 (q, *J* = 8.8 Hz, 4H), 3.73 (d, *J* = 9.7 Hz, 6H), 3.11 (s, 4H), 2.63 (t, *J* = 6.9 Hz, 2H), 2.44 (s, 3H). ¹³C NMR (101 MHz, DMSO-*d*₆) δ 163.1, 154.7, 150.7, 138.8, 132.3, 130.8, 129.9, 129.7, 129.2, 128.7, 126.7, 124.4, 120.7, 116.3, 115.1, 112.3, 66.5, 51.7, 48.4, 34.6, 9.4. HRMS (ESI) [M - H] ⁻, theoretically calculated for C₂₈H₂₇N₂O₆S: 519.1590, found 519.1584.

### Example 182

### 3-methyl-5-(N-(4-(4-methylpiperazin-1-yl)phenyl)-N-phenethylsulfamoyl)benzofuran-2-carboxylic acid (F27-S64)

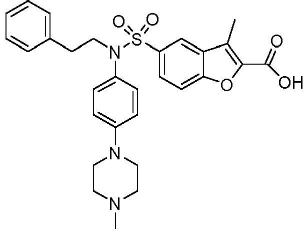

In accordance with the preparation method of 5-(N-benzyl-N-phenethylsulfamoyl)-3-methylbenzofuran-2-carboxylic acid (F27), this example was implemented under the same condition except that M8-64 was used instead of ethyl 5-(N-benzyl-N-phenethylsulfamoyl)-3-methylbenzofuran-2-carboxylate (F27-S8). 40 mg of white solid was obtained, with a yield of 94%. M.p. 152 - 153 °C. ¹H NMR (400 MHz, DMSO-*d*₆) δ 11.66 (s, 1H), 7.96 (d, *J* = 1.7 Hz, 1H), 7.79 (d, *J* = 8.8 Hz, 1H), 7.52 (dd, *J* = 8.8, 1.9 Hz, 1H), 7.24 (t, *J* = 7.2 Hz, 2H), 7.17 (td, *J* = 7.3, 6.2, 3.0 Hz, 1H), 7.14 - 7.08 (m, 2H), 6.94 (d, *J* = 9.1 Hz, 2H), 6.87 (d, *J* = 9.0 Hz, 2H), 3.90 - 3.71 (m, 4H), 3.45 (d, *J* = 10.8 Hz, 2H), 3.15 (dq, *J* = 21.9, 11.6, 10.7 Hz, 4H), 2.78 (d, *J* = 4.0 Hz, 3H), 2.62 (t, *J* = 7.2 Hz, 2H), 2.51 (s, 3H). ¹³C NMR (101 MHz, DMSO-*d*₆) δ 161.0, 155.6, 149.4, 143.7, 138.7, 133.6, 130.3, 129.9, 129.4, 129.2, 128.7, 127.1, 126.7, 125.2, 122.2, 116.1, 113.2, 52.4, 51.7, 45.3, 42.2, 34.5, 9.5. HRMS (ESI) [M - H] ⁻, theoretically calculated for C₂₉H₃₀N₃O₅S: 532.1906, found 532.1904.

### Example 183

### 3-methyl-5-(N-(4-(4-(tert-butoxycarbonyl)piperazin-1-yl)phenyl)-N-phenethylsulfamoyl)benzofuran-2-carboxylic acid (F27-S65)

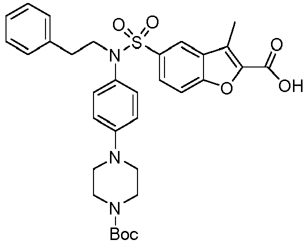

In accordance with the preparation method of 5-(N-benzyl-N-phenethylsulfamoyl)-3-methylbenzofuran-2-carboxylic acid (F27), this example was implemented under the same condition except that M8-65was used instead of ethyl 5-(N-benzyl-*N*-phenethylsulfamoyl)-3-methylbenzofuran-2-carboxylate (F27-S8). 205 mg of white solid was obtained, with a yield of 83%. M.p. > 380 °C (for decomposition). ¹H NMR (400 MHz, DMSO-*d*₆) δ 7.68 (s, 1H), 7.63 (d, *J* = 8.6 Hz, 1H), 7.42 (d, *J* = 8.6 Hz, 1H), 7.26 (t, *J* = 7.2 Hz, 2H), 7.19 (t, *J* = 7.2 Hz, 1H), 7.13 (d, *J* = 7.0 Hz, 2H), 6.94 - 6.79 (m, 4H), 3.74 (t, *J* = 7.2 Hz, 2H), 3.44 (s, 4H), 3.22 - 3.03 (m, 4H), 2.62 (t, *J* = 7.2 Hz, 2H), 2.43 (s, 3H), 1.42 (s, 9H). ¹³C NMR (101 MHz, DMSO-*d*₆) δ 163.0, 154.6, 154.4, 152.3, 150.5, 138.8, 132.2, 130.8, 129.9, 129.8, 129.2, 128.7, 126.7, 124.5, 120.7, 116.3, 115.9, 112.3, 79.5, 51.7, 48.1, 34.6, 28.5, 9.4. HRMS (ESI) [M - H] ⁻, theoretically calculated for C₃₃H₃₆N₃O₇S: 618.2274, found 618.2267.

### Example 184

### 3-methyl-5-(N-(4-(4-(3,3-dimethylbutyryl)piperazin-1-yl)phenyl)-N-phenethylsulfamoyl)benzofuran-2-carboxylic acid (F27-S66)

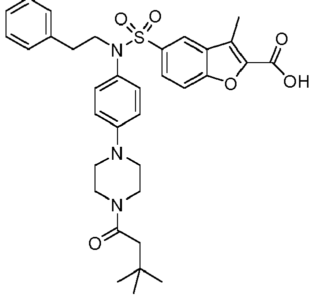

In accordance with the preparation method of 5-(N-benzyl-N-phenethylsulfamoyl)-3-methylbenzofuran-2-carboxylic acid (F27), this example was implemented under the same condition except that M8-66was used instead of ethyl 5-(N-benzyl-N-phenethylsulfamoyl)-3-methylbenzofuran-2-carboxylate (F27-S8). 108 mg of white solid was obtained, with a yield of 87%. M.p. > 380 °C (for decomposition). ¹H NMR (400 MHz, DMSO-*d*₆) δ 7.82 - 7.56 (m, 2H), 7.44 (d, *J=* 8.6 Hz, 1H), 7.31 -7.10 (m, 5H), 6.87 (q, *J=* 8.6 Hz, 4H), 3.75 (t, *J=* 7.3 Hz, 2H), 3.63 (p, *J* = 4.8 Hz, 4H), 3.14 (dt, *J* = 10.7, 4.9 Hz, 4H), 2.63 (t, *J* = 7.3 Hz, 2H), 2.45 (s, 3H), 2.28 (s, 2H), 1.00 (s, 9H). ¹³C NMR (101 MHz, DMSO) δ 169.9, 162.8, 154.7, 150.4, 138.8, 132.3, 130.7, 129.8, 129.8, 129.2, 128.7, 126.7, 124.7, 120.9, 115.7, 112.4, 51.7, 48.6, 48.3, 46.0, 44.1, 41.1, 34.6, 31.5, 30.2, 9.4. HRMS (ESI) [M - H] ⁻, theoretically calculated for C₃₄H₃₈N₃O₆S: 616.2481, found 616.2490.

### Example 185

### 3-methyl-5-(N-(4-(4-(propoxycarbonyl)piperazin-1-yl)phenyl)-N-phenethylsulfamoyl)benzofuran-2-carboxylic acid (F27-S67)

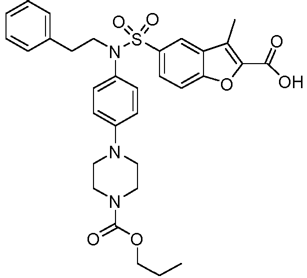

In accordance with the preparation method of 5-(N-benzyl-N-phenethylsulfamoyl)-3-methylbenzofuran-2-carboxylic acid (F27), this example was implemented under the same condition except that M8-67 was used instead of ethyl 5-(N-benzyl-N-phenethylsulfamoyl)-3-methylbenzofuran-2-carboxylate (F27-S8). 130 mg of light yellow solid was obtained, with a yield of 99%. M.p. > 380 °C (for decomposition). ¹H NMR (400 MHz, DMSO-*d*₆) δ 7.70 (d, *J* = 1.9 Hz, 1H), 7.65 (d, *J* = 8.7 Hz, 1H), 7.43 (dd, *J* = 8.6, 1.9 Hz, 1H), 7.26 (dd, *J* = 7.9, 6.5 Hz, 2H), 7.22 - 7.11 (m, 3H), 6.91 - 6.80 (m, 4H), 3.98 (t, *J* = 6.6 Hz, 2H), 3.74 (t, *J* = 7.2 Hz, 2H), 3.49 (t, *J* = 5.2 Hz, 4H), 3.15 (t, *J* = 5.2 Hz, 4H), 2.62 (t, *J* = 7.2 Hz, 2H), 2.44 (s, 3H), 1.59 (h, *J* = 7.1 Hz, 2H), 0.90 (t, *J* = 7.4 Hz, 3H). ¹³C NMR (101 MHz, DMSO-*d*₆) δ 162.9, 155.1, 154.7, 151.6, 150.4, 138.8, 132.3, 130.7, 129.9, 129.8, 129.2, 128.7, 126.7, 124.7, 120.8, 117.1, 115.9, 112.4, 66.8, 51.7, 48.1, 43.6, 34.6, 22.4, 10.8, 9.4. HRMS (ESI) [M - H] ⁻, theoretically calculated for C₃₂H₃₄N₃O₇S: 604.2117, found 604.2109.

### Example 186

### 3-methyl-5-(N-(4-(4-acetylpiperazin-1-yl)phenyl)-N-phenethylsulfamoyl)benzofuran-2-carboxylic acid (F27-S68)

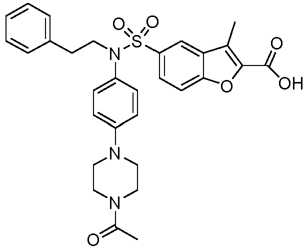

In accordance with the preparation method of 5-(N-benzyl-N-phenethylsulfamoyl)-3-methylbenzofuran-2-carboxylic acid (F27), this example was implemented under the same condition except that M8-68 was used instead of ethyl 5-(N-benzyl-*N*-phenethylsulfamoyl)-3-methylbenzofuran-2-carboxylate (F27-S8). 72 mg of white solid was obtained, with a yield of 64%. M.p. 183 - 184 °C. ¹H NMR (400 MHz, DMSO-*d*₆) δ 7.83 (d, *J* = 8.2 Hz, 1H), 7.70 (d, *J* = 8.6 Hz, 1H), 7.54 - 7.39 (m, 1H), 7.26 - 7.04 (m, 5H), 6.94 - 6.76 (m, 4H), 3.72 (t, *J* = 7.1 Hz, 2H), 3.59 - 3.30 (m, 4H), 3.14 (tt, *J* = 25.5, 5.2 Hz, 4H), 2.59 (t, *J* = 7.3 Hz, 2H), 2.46 (s, 3H), 1.99 (s, 3H). ¹³C NMR (101 MHz, DMSO-*d*₆) δ 168.8, 161.8, 155.4, 150.4, 149.8, 138.7, 133.4, 130.4, 129.8, 129.6, 129.2, 128.7, 126.7, 121.9, 116.1, 115.8, 112.9, 51.7, 48.4, 48.0, 45.8, 41.0, 34.6, 21.6, 9.5. HRMS (ESI) [M - H] ⁻, theoretically calculated for C₃₀H₃₀N₃O₆S: 560.1855, found 560.1852.

### Example 187

### 3-methyl-5-(N-(4-(4-benzoylpiperazin-1-yl)phenyl)-N-phenethylsulfamoyl)benzofuran-2-carboxylic acid (F27-S69)

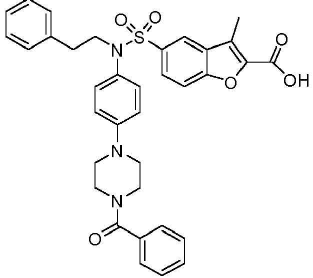

In accordance with the preparation method of 5-(N-benzyl-N-phenethylsulfamoyl)-3-methylbenzofuran-2-carboxylic acid (F27), this example was implemented under the same condition except that M8-69 was used instead of ethyl 5-(N-benzyl-*N*-phenethylsulfamoyl)-3-methylbenzofuran-2-carboxylate (F27-S8). 90 mg of white solid was obtained, with a yield of 72%. M.p. > 380 °C (for decomposition). ¹H NMR (400 MHz, DMSO-*d*₆) δ 7.78 - 7.61 (m, 2H), 7.45 (q, *J* = 9.0, 6.4 Hz, 6H), 7.31 -7.10 (m, 5H), 6.95 - 6.79 (m, 4H), 3.75 (t, *J* = 7.4 Hz, 4H), 3.47 (s, 2H), 3.19 (s, 4H), 2.63 (t, *J* = 7.3 Hz, 2H), 2.45 (s, 3H). ¹³C NMR (101 MHz, DMSO-*d*₆) δ 169.5, 163.0, 154.7, 151.8, 150.3, 138.8, 136.3, 132.3, 130.7, 130.1, 129.9, 129.8, 129.2, 128.9, 128.7, 127.4, 126.7, 124.6, 120.8, 116.9, 115.9, 112.3, 51.7, 48.3, 34.6, 9.5. HRMS (ESI) [M - H] ⁻, theoretically calculated for C₃₅H₃₂N₃O₆S: 622.2012, found 622.2010.

### Example 188

### 3-methyl-5-(N-(4-(4-benzylpiperazin-1-yl)phenyl)-N-phenethylsulfamoyl)benzofuran-2-carboxylic acid (F27-S70)

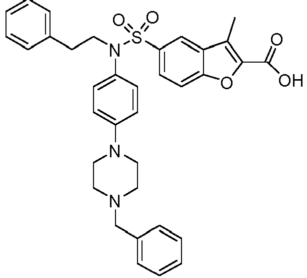

In accordance with the preparation method of 5-(N-benzyl-N-phenethylsulfamoyl)-3-methylbenzofuran-2-carboxylic acid (F27), this example was implemented under the same condition except that M8-70 was used instead of ethyl 5-(N-benzyl-*N*-phenethylsulfamoyl)-3-methylbenzofuran-2-carboxylate (F27-S8). 62 mg of white solid was obtained, with a yield of 51%. M.p. > 380 °C (for decomposition). ¹H NMR (400 MHz, DMSO-*d*₆) δ 7.77 (s, 1H), 7.69 (d, *J* = 8.6 Hz, 1H), 7.48 (d, *J* = 8.2 Hz, 1H), 7.33 (d, *J* = 4.1 Hz, 4H), 7.25 (t, *J* = 6.6 Hz, 3H), 7.16 (dd, *J* = 24.4, 7.1 Hz, 3H), 6.83 (q, *J* = 9.0 Hz, 4H), 3.74 (t, *J* = 7.4 Hz, 2H), 3.53 (s, 2H), 3.15 (s, 4H), 2.62 (t, *J* = 7.4 Hz, 2H), 2.50 (s, 4H), 2.47 (s, 3H). ¹³C NMR (101 MHz, DMSO-*d*₆) δ 162.5, 155.0, 150.6, 149.2, 138.8, 138.3, 132.9, 130.3, 129.7, 129.4, 129.2, 128.7, 128.7, 127.5, 126.7, 125.4, 121.3, 119.5, 115.4, 112.6, 62.4, 52.9, 51.8, 48.1, 34.6, 9.4. HRMS (ESI) [M - H] ⁻, theoretically calculated for C₃₅H₃₄N₃O₅S: 608.2219, found 608.2211.

### Example 189

### 3-methyl-5-(N-(4-(4-(dimethylcarbamoyl)piperazin-1-yl)phenyl)-N-phenethylsulfamoyl)benzofuran-2-carboxylic acid (F27-S71)

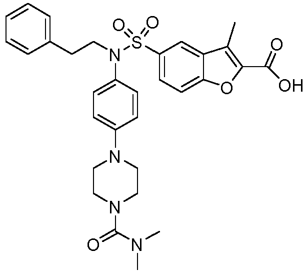

In accordance with the preparation method of 5-(N-benzyl-N-phenethylsulfamoyl)-3-methylbenzofuran-2-carboxylic acid (F27), this example was implemented under the same condition except that M8-71 was used instead of ethyl 5-(*N*-benzyl-*N*-phenethylsulfamoyl)-3-methylbenzofuran-2-carboxylate (F27-S8). 69 mg of white solid was obtained, with a yield of 58%. M.p. 192 - 193 °C. ¹H NMR (400 MHz, DMSO-*d*₆) δ 13.75 (s, 1H), 7.96 (s, 1H), 7.78 (d, *J* = 8.8 Hz, 1H), 7.55 (d, *J* = 8.7 Hz, 1H), 7.32 - 7.06 (m, 5H), 6.87 (q, *J* = 9.0 Hz, 4H), 3.76 (t, *J* = 7.3 Hz, 2H), 3.36 - 3.00 (m, 8H), 2.76 (s, 6H), 2.62 (t, *J* = 7.3 Hz, 2H), 2.51 (s, 3H). ¹³C NMR (101 MHz, DMSO-*d*₆) δ 164.1, 161.1, 155.6, 150.6, 143.6, 138.7, 133.7, 129.8, 129.5, 129.3, 129.2, 128.7, 127.1, 126.7, 125.2, 122.2, 115.7, 113.2, 51.8, 48.0, 46.7, 38.5, 34.6, 9.5. HRMS (ESI) [M - H] ⁻, theoretically calculated for C₃₁H₃₃N₄O₆S: 589.2121, found 589.2115.

### Example 190

### 3-methyl-5-(N-(4-(4-(methylsulfonyl)piperazin-1-yl)phenyl)-N-phenethylsulfamoyl)benzofuran-2-carboxylic acid (F27-S72)

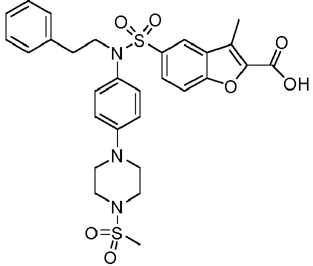

In accordance with the preparation method of 5-(N-benzyl-N-phenethylsulfamoyl)-3-methylbenzofuran-2-carboxylic acid (F27), this example was implemented under the same condition except that M8-72 was used instead of ethyl 5-(N-benzyl-*N*-phenethylsulfamoyl)-3-methylbenzofuran-2-carboxylate (F27-S8). 126 mg of white solid was obtained, with a yield of 99%. M.p. > 380 °C (for decomposition). ¹H NMR (400 MHz, DMSO-*d*₆) δ 7.75 (d, *J =* 1.9 Hz, 1H), 7.67 (d, *J* = 8.7 Hz, 1H), 7.46 (dd, *J* = 8.7, 1.9 Hz, 1H), 7.26 (dd, *J* = 8.0, 6.5 Hz, 2H), 7.22 - 7.10 (m, 3H), 6.96 - 6.82 (m, 4H), 3.75 (t, *J* = 7.3 Hz, 2H), 3.25 (q, *J* = 5.7 Hz, 8H), 2.93 (s, 3H), 2.63 (t, *J=* 7.3 Hz, 2H), 2.46 (s, 3H). ¹³C NMR (101 MHz, DMSO-*d*₆) δ 162.7, 154.9, 150.1, 138.8, 132.5, 130.5, 130.2, 129.8, 129.2, 128.7, 126.7, 125.0, 121.1, 118.3, 116.1, 112.5, 51.7, 48.0, 45.7, 34.6, 34.4, 9.5. HRMS (ESI) [M - H] ⁻, theoretically calculated for C₂₉H₃₀N₃O₇S₂: 596.1525, found 596.1525.

### Example 191

### 3-methyl-5-(N-(4-(1-(tert-butoxycarbonyl)piperidin-4-yl)phenyl)-N-phenethylsulfamoyl)benzofuran-2-carboxylic acid (F27-S73)

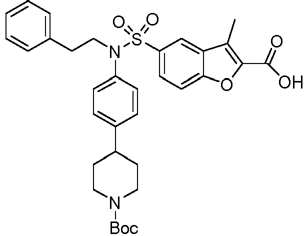

In accordance with the preparation method of 5-(N-benzyl-N-phenethylsulfamoyl)-3-methylbenzofuran-2-carboxylic acid (F27), this example was implemented under the same condition except that M8-73 was used instead of ethyl 5-(N-benzyl-*N*-phenethylsulfamoyl)-3-methylbenzofuran-2-carboxylate (F27-S8). 96 mg of white solid was obtained, with a yield of 38%. M.p. > 380 °C (for decomposition). ¹H NMR (400 MHz, DMSO-*d*₆) δ 7.71 - 7.55 (m, 2H), 7.44 (d, *J* = 8.5 Hz, 1H), 7.21 (tt, *J* = 14.6, 7.2 Hz, 5H), 7.12 (d, *J* = 7.3 Hz, 2H), 6.95 (d, *J* = 8.2 Hz, 2H), 4.06 (d, *J* = 12.4 Hz, 2H), 3.77 (t, *J* = 7.3 Hz, 2H), 3.02 - 2.66 (m, 3H), 2.63 (t, *J* = 7.4 Hz, 2H), 2.41 (s, 3H), 1.75 (d, *J* = 12.2 Hz, 2H), 1.54 - 1.44 (m, 2H), 1.41 (s, 9H). ¹³C NMR (101 MHz, DMSO-*d*₆) δ 163.1, 154.7, 154.4, 152.2, 145.9, 138.7, 137.2, 132.0, 130.8, 129.2, 129.0, 128.7, 127.7, 126.7, 124.4, 120.8, 116.5, 112.4, 79.0, 51.5, 41.6, 34.7, 33.1, 28.6, 9.4. HRMS (ESI) [M - H] ⁻, theoretically calculated for C₃₄H₃₇N₂O₇S: 617.2321, found 617.2318.

### Example 192

### 3-methyl-5-(N-(4-(4-(tert-butoxycarbonyl)piperazin-1-yl)-3-(trifluoromethyl)phenyl)-N-phenethylsulfamoyl)benzofuran-2-carboxylic acid (F27-S74)

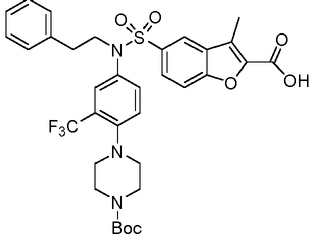

In accordance with the preparation method of 5-(N-benzyl-N-phenethylsulfamoyl)-3-methylbenzofuran-2-carboxylic acid (F27), this example was implemented under the same condition except that M8-74 was used instead of ethyl 5-(N-benzyl-N-phenethylsulfamoyl)-3-methylbenzofuran-2-carboxylate (F27-S8). 114 mg of light yellow solid was obtained, with a yield of 83%. M.p. 215 - 216 °C. ¹H NMR (400 MHz, DMSO-*d*₆) δ 7.86 - 7.70 (m, 2H), 7.60 - 7.43 (m, 2H), 7.31 - 7.05 (m, 7H), 3.85 (t, *J* = 7.1 Hz, 2H), 3.43 (t, *J* = 4.8 Hz, 4H), 2.81 (t, *J* = 4.8 Hz, 4H), 2.68 (t, *J* = 7.1 Hz, 2H), 2.48 (s, 3H), 1.42 (s, 9H). ¹³C NMR (101 MHz, DMSO-*d*₆) δ 162.2, 155.4, 154.3, 151.6, 138.5, 136.0, 133.9, 132.3, 130.1, 129.2, 128.6, 126.7, 126.2, 125.8, 125.7, 125.2, 122.5, 121.7, 113.0, 79.5, 53.2, 51.4, 34.8, 28.5, 9.3. ¹⁹F NMR (376 MHz, DMSO-*d*₆) δ -59.16. HRMS (ESI) [M - H] ⁻, theoretically calculated for C₃₄H₃₅N₃O₇SF₃: 686.2148, found 686.2136.

### Example 193

### 3-methyl-5-(N-(6-(4-(tert-butoxycarbonyl)piperazin-1-yl)pyridin-3-yl)-N-phenethylsulfamoyl)benzofuran-2-carboxylic acid (F27-S75)

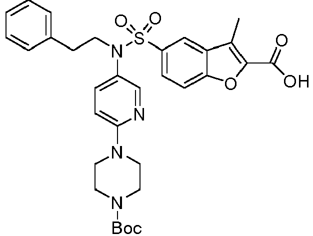

In accordance with the preparation method of 5-(N-benzyl-N-phenethylsulfamoyl)-3-methylbenzofuran-2-carboxylic acid (F27), this example was implemented under the same condition except that M8-75 was used instead of ethyl 5-(N-benzyl-*N*-phenethylsulfamoyl)-3-methylbenzofuran-2-carboxylate (F27-S8). 207 mg of white solid was obtained, with a yield of 83%. M.p. > 380 °C (for decomposition). ¹H NMR (400 MHz, DMSO-*d*₆) δ 7.71 (d, *J* = 31.0 Hz, 3H), 7.44 (d, *J* = 8.7 Hz, 1H), 7.35 - 6.96 (m, 6H), 6.86 - 6.63 (m, 1H), 3.76 (s, 2H), 3.49 (s, 4H), 3.41 (s, 4H), 2.65 (s, 2H), 2.45 (s, 3H), 1.42 (s, 9H). ¹³C NMR (101 MHz, DMSO-*d*₆) δ 162.9, 158.0, 154.8, 154.4, 152.3, 148.7, 138.6, 137.8, 132.2, 131.0, 129.2, 128.7, 126.7, 125.5, 124.4, 120.7, 116.4, 112.5, 107.1, 79.5, 51.6, 44.7, 34.7, 28.5, 9.4. HRMS (ESI) [M - H] ⁻, theoretically calculated for C₃₂H₃₅N₄O₇S: 619.2226, found 619.2230.

### Example 194

### 3-methyl-5-(N-(4-((4-(tert-butoxycarbonyl)piperazin-1-yl)methyl)phenyl)-N-phenethylsulfonamide)benzofuran-2-carboxylic acid (F27-S76)

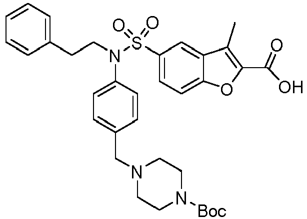

In accordance with the preparation method of 5-(N-benzyl-N-phenethylsulfamoyl)-3-methylbenzofuran-2-carboxylic acid (F27), this example was implemented under the same condition except that M8-76 was used instead of ethyl 5-(N-benzyl-*N*-phenethylsulfamoyl)-3-methylbenzofuran-2-carboxylate (F27-S8). 185 mg of white solid was obtained, with a yield of 97%. M.p. 234 - 235 °C. ¹H NMR (400 MHz, DMSO-*d*₆) δ 7.77 (d, *J* = 1.9 Hz, 1H), 7.73 (d, *J* = 8.7 Hz, 1H), 7.51 (dd, *J* = 8.7, 1.9 Hz, 1H), 7.24 (dd, *J* = 13.8, 7.7 Hz, 4H), 7.18 (d, *J* = 7.1 Hz, 1H), 7.11 (d, *J* = 7.1 Hz, 2H), 6.99 (d, *J* = 8.2 Hz, 2H), 3.81 (t, *J* = 7.3 Hz, 2H), 3.49 (s, 2H), 3.32 (t, *J* = 5.1 Hz, 4H), 2.64 (t, *J* = 7.3 Hz, 2H), 2.46 (s, 3H), 2.30 (t, *J* = 4.9 Hz, 4H), 1.38 (s, 9H). ¹³C NMR (101 MHz, DMSO-*d*₆) δ 162.0, 155.3, 154.3, 147.5, 138.7, 138.0, 137.9, 132.7, 130.0, 129.8, 129.2, 128.8, 128.7, 126.7, 125.8, 121.6, 121.1, 112.8, 79.2, 61.7, 52.7, 51.5, 34.7, 28.5, 9.3. HRMS (ESI) [M - H] ⁻, theoretically calculated for C₃₄H₃₈N₃O₇S: 632.2430, found 632.2432.

### Example 195

### 3-methyl-5-(N-(2-(4-(tert-butoxycarbonyl)piperazin-1-yl)phenyl)-N-phenethylsulfamoyl)benzofuran-2-carboxylic acid (F27-S77)

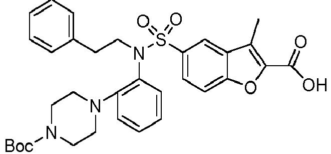

In accordance with the preparation method of 5-(N-benzyl-N-phenethylsulfamoyl)-3-methylbenzofuran-2-carboxylic acid (F27), this example was implemented under the same condition except that M8-77 was used instead of ethyl 5-(N-benzyl-*N*-phenethylsulfamoyl)-3-methylbenzofuran-2-carboxylate (F27-S8). 63 mg of white solid was obtained, with a yield of 67%. M.p. 168 - 169 °C. ¹H NMR (400 MHz, DMSO-*d*₆) δ 8.25 (d, *J* = 1.8 Hz, 1H), 7.97 - 7.77 (m, 2H), 7.38 - 7.29 (m, 1H), 7.22 (d, *J* = 8.0 Hz, 1H), 7.18 - 7.03 (m, 5H), 6.95 (d, *J* = 7.0 Hz, 2H), 3.96 (d, *J* = 74.5 Hz, 2H), 3.28 (d, *J* = 24.7 Hz, 4H), 2.94 (s, 2H), 2.58 (s, 7H), 1.39 (s, 9H). ¹³C NMR (101 MHz, DMSO-*d*₆) δ 161.3, 155.6, 154.4, 151.0, 144.3, 138.6, 135.9, 133.6, 131.3, 129.7, 129.6, 128.9, 128.7, 127.0, 126.6, 124.7, 123.1, 122.2, 113.5, 79.4, 52.1, 50.6, 34.4, 28.5, 9.5. HRMS (ESI) [M - H] ⁻, theoretically calculated for C₃₃H₃₆N₃O₇S: 618.2274, found 618.2280.

### Example 196

### 3-methyl-5-(N-(3-(4-methylpiperazin-1-yl)phenyl)-N-phenethylsulfamoyl)benzofuran-2-carboxylic acid (F27-S78)

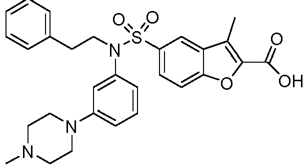

In accordance with the preparation method of 5-(N-benzyl-N-phenethylsulfamoyl)-3-methylbenzofuran-2-carboxylic acid (F27), this example was implemented under the same condition except that M8-78 was used instead of ethyl 5-(N-benzyl-*N*-phenethylsulfamoyl)-3-methylbenzofuran-2-carboxylate (F27-S8). 112 mg of white solid was obtained, with a yield of 99%. M.p. 232 - 233 °C. ¹H NMR (400 MHz, DMSO-*d*₆) δ 7.90 (s, 1H), 7.71 (d, *J* = 8.7 Hz, 1H), 7.50 (d, *J* = 8.7 Hz, 1H), 7.25 - 7.03 (m, 6H), 6.95 - 6.86 (m, 1H), 6.57 (s, 1H), 6.44 (d, *J* = 7.9 Hz, 1H), 3.76 (t, *J* = 7.2 Hz, 2H), 3.27 (d, *J* = 6.3 Hz, 4H), 3.08 (t, *J* = 5.0 Hz, 4H), 2.63 (d, *J* = 21.3 Hz, 5H), 2.47 (s, 3H). ¹³C NMR (101 MHz, DMSO-*d*₆) δ 162.1, 155.4, 150.8, 145.9, 140.1, 138.7, 133.2, 129.9, 129.8, 129.2, 128.7, 126.7, 126.5, 122.9, 122.0, 120.0, 116.7, 115.6, 112.9, 52.7, 51.6, 46.1, 43.0, 34.7, 9.5. HRMS (ESI) [M - H] ⁻, theoretically calculated for C₂₉H₃₀N₃O₅S: 532.1906, found 532.1906.

### Example 197

### 3-methyl-5-(N-(4-(4-morpholinobenzyl)-N-phenethylsulfamoyl)benzofuran-2-carboxylic acid (F27-S79)

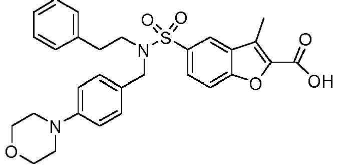

In accordance with the preparation method of 5-(N-benzyl-N-phenethylsulfamoyl)-3-methylbenzofuran-2-carboxylic acid (F27), this example was implemented under the same condition except that M8-79 was used instead of ethyl 5-(N-benzyl-*N*-phenethylsulfamoyl)-3-methylbenzofuran-2-carboxylate (F27-S8). 73 mg of white solid was obtained, with a yield of 68%. M.p. > 380 °C (for decomposition). ¹H NMR (400 MHz, DMSO-*d*₆) δ 8.27 - 8.13 (m, 1H), 7.91 (dd, *J* = 8.8, 1.6 Hz, 1H), 7.82 (d, *J* = 8.8 Hz, 1H), 7.16 (dq, *J* = 15.5, 7.1 Hz, 5H), 7.00 (d, *J* = 7.0 Hz, 2H), 6.87 (d, *J* = 8.5 Hz, 2H), 4.32 (s, 2H), 3.76 - 3.67 (m, 4H), 3.34 - 3.24 (m, 2H), 3.12 - 3.00 (m, 4H), 2.55 (d, *J* = 10.5 Hz, 5H). ¹³C NMR (101 MHz, DMSO) δ 161.4, 155.4, 151.0, 138.8, 135.6, 129.8, 129.6, 129.0, 128.8, 127.1, 126.7, 126.4, 121.8, 115.3, 113.4, 66.5, 51.2, 49.4, 48.7, 34.9, 9.6. HRMS (ESI) [M - H] ⁻, theoretically calculated for C₂₉H₂₉N₂O₆S: 533.1746, found 533.1737.

### Example 198

### 3-methyl-5-(N-(4-(4-methylpiperazin-1-yl)benzyl)-N-phenethylsulfamoyl)benzofuran-2-carboxylic acid (F27-S80)

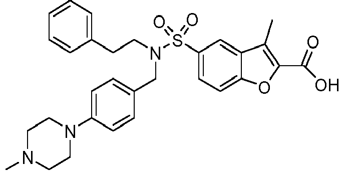

In accordance with the preparation method of 5-(N-benzyl-N-phenethylsulfamoyl)-3-methylbenzofuran-2-carboxylic acid (F27), this example was implemented under the same condition except that M8-80 was used instead of ethyl 5-(N-benzyl-*N*-phenethylsulfamoyl)-3-methylbenzofuran-2-carboxylate (F27-S8). 77 mg of white solid was obtained, with a yield of 94%. M.p. > 380 °C (for decomposition). ¹H NMR (400 MHz, DMSO-*d*₆) δ 8.22 (s, 1H), 7.90 (d, *J* = 8.8 Hz, 1H), 7.82 (d, *J* = 8.8 Hz, 1H), 7.23 - 7.14 (m, 4H), 7.11 (t, *J* = 7.2 Hz, 1H), 7.05 - 6.88 (m, 4H), 4.32 (s, 2H), 3.45 (s, 4H), 3.25 (d, *J* = 7.8 Hz, 6H), 2.75 (s, 3H), 2.61 - 2.51 (m, 5H). ¹³C NMR (101 MHz, DMSO-*d*₆) δ 155.4, 149.6, 138.8, 135.4, 129.9, 129.7, 129.0, 128.8, 128.0, 126.7, 126.4, 121.7, 116.2, 113.4, 52.4, 51.1, 49.3, 45.8, 42.4, 34.8, 9.6. HRMS (ESI) [M - H] ⁻, theoretically calculated for C₃₀H₃₂N₃O₅S: 546.2063, found 546.2056.

### Example 198

### 3-methyl-5-(N-(4-(4-(tert-butoxycarbonyl)piperazin-1-yl)benzyl)-N-phenethylsulfamoyl)benzofuran-2-carboxylic acid (F27-S81)

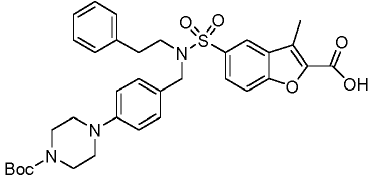

In accordance with the preparation method of 5-(N-benzyl-N-phenethylsulfamoyl)-3-methylbenzofuran-2-carboxylic acid (F27), this example was implemented under the same condition except that M8-81 was used instead of ethyl 5-(N-benzyl-*N*-phenethylsulfamoyl)-3-methylbenzofuran-2-carboxylate (F27-S8). 160 mg of white solid was obtained, with a yield of 84%. M.p. 166 - 167 °C. ¹H NMR (400 MHz, DMSO-*d*₆) δ 8.21 (s, 1H), 7.91 (d, *J* = 8.6 Hz, 1H), 7.82 (d, *J* = 8.7 Hz, 1H), 7.17 (p, *J* = 9.8, 8.4 Hz, 5H), 6.99 (d, *J* = 7.0 Hz, 2H), 6.89 (d, *J* = 8.3 Hz, 2H), 4.31 (s, 2H), 3.43 (s, 4H), 3.34 - 3.22 (m, 2H), 3.07 (s, 4H), 2.55 (d, *J* = 12.9 Hz, 5H), 1.41 (s, 9H). ¹³C NMR (101 MHz, DMSO-*d*₆) δ 161.4, 155.4, 154.3, 150.8, 138.8, 135.6, 129.8, 129.7, 129.0, 128.8, 127.3, 126.7, 126.4, 121.8, 116.1, 113.4, 79.4, 51.2, 49.3, 48.6, 34.8, 28.5, 9.5. HRMS (ESI) [M - H] ⁻, theoretically calculated for C₃₄H₃₈N₃O₇S: 632.2430, found 632.2438.

### Example 199

### 3-methyl-5-(N-(4-(4-phenoxybenzyl)-N-phenethylsulfamoyl)benzofuran-2-carboxylic acid (F27-S86)

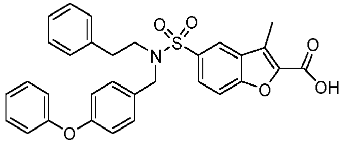

In accordance with the preparation method of 5-(N-benzyl-N-phenethylsulfamoyl)-3-methylbenzofuran-2-carboxylic acid (F27), this example was implemented under the same condition except that M8-86 was used instead of ethyl 5-(N-benzyl-*N*-phenethylsulfamoyl)-3-methylbenzofuran-2-carboxylate (F27-S8). 30 mg of white solid was obtained, with a yield of 55%. M.p. 302 - 303 °C. ¹H NMR (400 MHz, DMSO-*d*₆) δ 8.06 (d, *J* = 1.9 Hz, 1H), 7.89 - 7.59 (m, 2H), 7.45 - 7.30 (m, 4H), 7.26 - 7.09 (m, 4H), 7.06 - 6.91 (m, 6H), 4.38 (s, 2H), 3.35 - 3.26 (m, 2H), 2.54 (d, *J* = 19.3 Hz, 5H). ¹³C NMR (101 MHz, DMSO-*d*₆) δ 162.9, 157.1, 156.5, 154.6, 152.1, 138.8, 134.0, 132.4, 131.1, 130.6, 130.5, 129.0, 128.8, 126.7, 124.1, 123.9, 120.5, 119.1, 119.0, 116.8, 112.7, 51.2, 49.8, 34.9, 9.5. HRMS (ESI) [M - H] ⁻, theoretically calculated for C₃₁H₂₆NO₆S: 540.1481, found 540.1477.

### Example 200

### 3-methyl-5-(N-(2-(4-(tert-butoxycarbonyl)piperazin-1-yl)benzyl)-N-phenethylsulfamoyl)benzofuran-2-carboxylic acid (F27-S87)

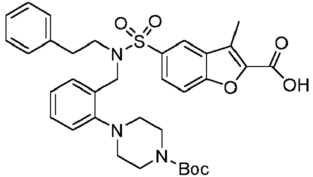

In accordance with the preparation method of 5-(N-benzyl-N-phenethylsulfamoyl)-3-methylbenzofuran-2-carboxylic acid (F27), this example was implemented under the same condition except that M8-87 was used instead of ethyl 5-(N-benzyl-*N*-phenethylsulfamoyl)-3-methylbenzofuran-2-carboxylate (F27-S8). 144 mg of white solid was obtained, with a yield of 75%. M.p. 158 - 159 °C. ¹H NMR (400 MHz, DMSO-*d*₆) δ 8.29 (d, *J* = 1.9 Hz, 1H), 7.97 (dd, *J* = 8.8, 1.9 Hz, 1H), 7.87 (d, *J* = 8.8 Hz, 1H), 7.42 (d, *J* = 7.0 Hz, 1H), 7.30 (t, *J* = 7.6 Hz, 1H), 7.23 - 7.09 (m, 5H), 6.95 (d, *J* = 7.0 Hz, 2H), 4.53 (s, 2H), 3.41 (s, 4H), 3.33 - 3.23 (m, 2H), 2.78 (t, *J* = 4.8 Hz, 4H), 2.61 (s, 3H), 2.56 - 2.52 (m, 2H), 1.43 (s, 9H). ¹³C NMR (101 MHz, DMSO-*d*₆) δ 161.6, 155.4, 154.4, 151.7, 138.8, 135.0, 131.6, 129.9, 128.8, 128.8, 126.7, 126.3, 124.4, 121.7, 120.6, 113.3, 79.4, 52.8, 49.9, 46.8, 34.5, 28.5, 9.5. HRMS (ESI) [M - H] ⁻, theoretically calculated for C₃₄H₃₈N₃O₇S: 632.2430, found 632.2423.

### Example 201

### 3-methyl-5-(N-(3-(4-(tert-butoxycarbonyl)piperazin-1-yl)benzyl)-N-phenethylsulfamoyl)benzofuran-2-carboxylic acid (F27-S88)

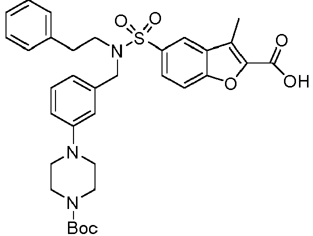

In accordance with the preparation method of 5-(N-benzyl-N-phenethylsulfamoyl)-3-methylbenzofuran-2-carboxylic acid (F27), this example was implemented under the same condition except that M8-88 was used instead of ethyl 5-(N-benzyl-*N*-phenethylsulfamoyl)-3-methylbenzofuran-2-carboxylate (F27-S8). 53 mg of white solid was obtained, with a yield of 42%. M.p. 104 - 105 °C. ¹H NMR (400 MHz, DMSO-*d*₆) δ 8.26 (d, *J* = 1.6 Hz, 1H), 7.94 (dd, *J* = 8.8, 1.8 Hz, 1H), 7.83 (d, *J* = 8.8 Hz, 1H), 7.25 - 7.09 (m, 4H), 7.01 (d, *J* = 7.0 Hz, 2H), 6.89 - 6.81 (m, 1H), 6.77 (d, *J* = 7.5 Hz, 1H), 6.72 (s, 1H), 4.36 (s, 2H), 3.47 - 3.36 (m, 4H), 3.35 - 3.28 (m, 2H), 2.94 (s, 4H), 2.59 (d, *J* = 10.7 Hz, 5H), 1.41 (s, 9H). ¹³C NMR (101 MHz, DMSO-*d*₆) δ 161.2, 155.5, 154.3, 151.4, 144.0, 138.9, 137.8, 135.6, 129.6, 129.6, 129.0, 128.8, 126.7, 126.6, 125.0, 121.9, 119.9, 116.1, 115.6, 113.5, 79.4, 51.9, 49.5, 48.6, 34.8, 28.5, 9.5. HRMS (ESI) [M - H] ⁻, theoretically calculated for C₃₄H₃₈N₃O₇S: 632.2430, found 632.2421.

### Example 202

### ethyl 3-methyl-5-(N-phenethyl-N-(2-(piperazin-1-yl)phenyl)sulfamoyl)benzofuran-2-carboxylate (M9-1)

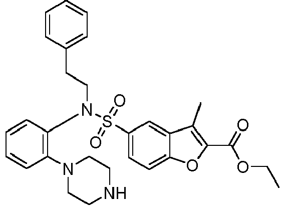

Tert-butyl 4-(2-(((2-(ethoxycarbonyl)-3-methyl-*N*-phenethylbenzofuran)-5-sulfonylamino)phenyl)piperazine-1-carboxylate (96 mg, 0.15 mmol) and trifluoroacetic acid (85.5 mg, 0.75mmol) were added to dichloromethane (5 mL), and the resulting solution was then stirred at room temperature for 2 h. After the reaction was found to be completed from the monitoring of the TLC, the solvent was distilled off under reduced pressure, and the obtained product was dissolved in water (20 mL). A proper amount of sodium bicarbonate was added to adjust the pH value to 7-8, and extraction with dichloromethane (20 mL) was carried out three times. The organic phase was washed with saturated aqueous NaCl solution and then dried with anhydrous sodium sulfate. The crude product was separated by column chromatography (DCM/CH₃OH = 15/1 ~ 10/1)) to obtain 85 mg of light yellow solid, with a yield of 99%. ¹H NMR (400 MHz, Chloroform-*d*) δ 8.17 (d, J = 1.5 Hz, 1H), 7.91 (dd, J = 8.8, 1.8 Hz, 1H), 7.63 (d, J = 8.8 Hz, 1H), 7.34 (dt, J = 8.5, 4.5 Hz, 1H), 7.23 - 7.13 (m, 4H), 7.04 (d, J = 4.1 Hz, 2H), 6.97 (d, J = 6.6 Hz, 2H), 4.49 (q, J = 7.1 Hz, 2H), 4.17 (s, 1H), 3.88 (s, 1H), 3.26 - 2.75 (m, 8H), 2.61 (s, 5H), 1.47 (t, J = 7.1 Hz, 3H), 1.27 (s, 1H). ¹³C NMR (101 MHz, Chloroform-*d*) δ 159.9, 155.8, 151.5, 143.0, 138.4, 136.3, 133.6, 130.7, 129.3, 129.3, 128.6, 128.4, 126.8, 126.4, 125.6, 124.0, 122.6, 122.0, 112.9, 61.6, 51.1, 34.7, 29.7, 14.4, 9.4. HRMS (ESI) [M + H] ⁺, theoretically calculated for C₃₀H₃₄N₃O₅S: 548.2219, found 548.2230.

### Example 203

### ethyl 3-methyl-5-(N-phenethyl-N-(2-(piperazin-1-yl)benzyl)sulfamoyl)benzofuran-2-carboxylate (M9-2)

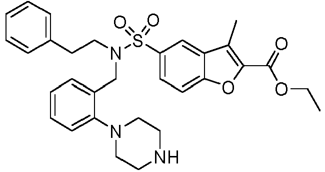

Ethyl 5-(N-(2-(4-(tert-butoxycarbonyl)piperazine)benzyl)-N-phenethylsulfamoyl)-3-methylbenzofuran-2-carboxylate (1.981 g, 3.0 mmol) was dissolved in dichloromethane (20 mL), trifluoroacetic acid (1.337 mL, 18.0 mmol) was then added, and the resulting solution was stirred for 1 h. After the reaction was found to be completed from the monitoring of the TLC, the solvent was distilled off under reduced pressure and the product was dried with a vacuum pump to obtain 1.65 g of white solid, with a yield of 98%. ¹H NMR (400 MHz, Chloroform-*d*) δ 8.17 (d, *J* = 2.0 Hz, 1H), 7.90 (dd, *J* = 8.7, 2.0 Hz, 1H), 7.67 (d, *J* = 8.7 Hz, 1H), 7.47 - 7.08 (m, 7H), 6.98 - 6.86 (m, 2H), 4.61 - 4.42 (m, 4H), 3.83 (d, *J* = 41.7 Hz, 2H), 3.31 (t, *J* = 8.1 Hz, 2H), 2.61 (d, *J* = 23.8 Hz, 5H), 1.48 (t, *J* = 7.1 Hz, 3H). ¹³C NMR (101 MHz, Chloroform-*d*) δ 159.9, 155.8, 143.0, 138.3, 135.3, 131.0, 130.2, 129.5, 129.0, 128.5, 128.5, 126.5, 126.2, 125.5, 121.5, 120.8, 113.1, 61.6, 49.4, 47.1, 47.1, 45.2, 35.0, 14.4, 9.4.

### Example 204

### 4-(2-((2-(ethoxycarbonyl)-3-methyl-N-phenethylbenzofuran)-5-sulfonylamino)phenyl)-1,1-dimethylpiperazin-1-ium (M10-90)

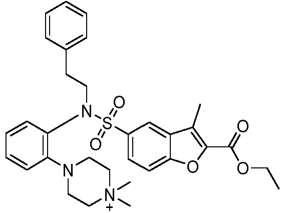

Ethyl 3-methyl-5-N-phenethyl-N-(2-(piperazin-1-yl)phenyl)sulfamoyl)benzofuran-2-carboxylate (200 mg, 0.36 mmol), methyl iodide (102 mg, 0.72 mmol) and potassium carbonate (100 mg, 0.72 mmol) were added to acetonitrile (2 mL), and the reaction was performed overnight at room temperature. After the reaction was found to be completed from the monitoring of the TLC, the solvent was distilled off under reduced pressure, and the obtained product was dissolved in water (10 mL) and then extracted three times with dichloromethane (10 mL). The organic phase was washed with saturated aqueous NaCl solution and then dried with anhydrous sodium sulfate. The crude product was separated by column chromatography (DCM/CH₃OH = 20/1 ~ 10/1) to obtain 163 mg of white solid, with a yield of 79%. ¹H NMR (400 MHz, DMSO-*d*₆) δ 8.25 (s, 1H), 7.91 (d, *J* = 8.8 Hz, 2H), 7.53 (d, *J* = 7.2 Hz, 1H), 7.42 (t, *J* = 8.2 Hz, 1H), 7.14 (dt, *J* = 13.9, 7.5 Hz, 4H), 6.98 (t, *J* = 6.3 Hz, 3H), 4.39 (q, *J* = 7.1 Hz, 2H), 4.00 (s, 1H), 3.85 (s, 1H), 3.48 (s, 4H), 3.36 (s, 4H), 3.24 (s, 6H), 3.15 (s, 2H), 2.60 (s, 3H), 1.36 (t, *J* = 7.1 Hz, 3H). ¹³C NMR (101 MHz, DMSO-*d*₆) δ 159.6, 155.7, 149.9, 142.8, 138.6, 135.7, 134.1, 131.0, 129.8, 129.4, 129.0, 128.7, 127.5, 126.7, 126.1, 125.8, 124.3, 122.6, 113.8, 61.7, 51.4, 45.9, 34.3, 14.6, 9.6. HRMS (ESI) [M] ⁺, theoretically calculated for C₃₂H₃₈N₃O₅S⁺: 576.2532, found 576.2535.

### Example 205

### ethyl 3-methyl-5-(N-(2-(4-acetylpiperazin-1-yl)phenyl)-N-phenethylsulfamoyl)benzofuran-2-carboxylate (M10-91)

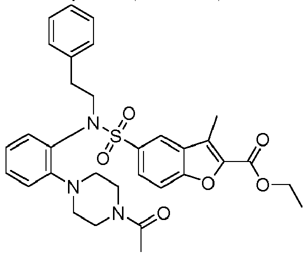

Ethyl 3-methyl-5-N-phenethyl-N-(2-(piperazin-1-yl)phenyl)sulfamoyl)benzofuran-2-carboxylate (150 mg, 0.27 mmol), *N, N*-diisopropylethylamine (70 mg, 0.54 mmol) and 4-dimethylaminopyridine (4 mg, 0.03 mmol) were added to dichloromethane (1 mL), and the solution was stirred at 0 °C for 30 min. Dichloromethane (1 mL) solution of acetic anhydride (37 mg, 0.32 mmol) was then added dropwise and the resulting solution was then stirred at room temperature for 5 h. After the reaction was found to be completed from the monitoring of the TLC, the solvent was distilled off under reduced pressure, and the obtained product was dissolved in water (10 mL) and then extracted three times with dichloromethane (10 mL). The organic phase was washed with saturated aqueous NaCl solution and then dried with anhydrous sodium sulfate. The crude product was separated by column chromatography (PE/EA = 1/2) to obtain 129 mg of white solid, with a yield of 81%. ¹H NMR (400 MHz, Chloroform-*d*) δ 8.14 (d, *J* = 1.8 Hz, 1H), 7.88 (dd, *J* = 8.8, 1.9 Hz, 1H), 7.63 (d, *J* = 8.7 Hz, 1H), 7.35 (td, *J* = 7.7, 1.6 Hz, 1H), 7.21 - 7.12 (m, 4H), 7.07 (td, *J* = 7.6, 1.4 Hz, 1H), 6.94 (ddd, *J* = 7.6, 3.6, 1.5 Hz, 3H), 4.48 (q, *J* = 7.1 Hz, 2H), 4.17 - 3.93 (m, 2H), 3.81 (s, 1H), 3.59 (s, 2H), 3.44 (d, *J* = 18.9 Hz, 2H), 3.10 (s, 1H), 2.84 (s, 2H), 2.68 (s, 1H), 2.60 (s, 4H), 2.12 (s, 3H), 1.46 (t, *J* = 7.1 Hz, 3H). ¹³C NMR (101 MHz, Chloroform-*d*) δ 169.2, 159.8, 155.8, 151.1, 143.1, 138.1, 136.0, 134.1, 130.3, 129.5, 129.4, 128.5, 126.8, 126.5, 125.5, 124.6, 122.9, 122.1, 112.9, 61.6, 53.0, 51.8, 51.5, 46.9, 42.0, 34.6, 21.4, 14.4, 9.4. HRMS (ESI) [M + H] ⁺, theoretically calculated for C₃₂H₃₆N₃O₆S: 590.2325, found 590.2331.

### Example 206

### ethyl 3-methyl-5-(N-(2-(4-acetylpiperazin-1-yl)phenyl)-N-phenethylsulfamoyl)benzofuran-2-carboxylate (M10-92)

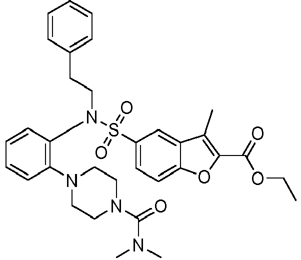

Ethyl 3-methyl-5-N-phenethyl-N-(2-(piperazin-1-yl)phenyl)sulfamoyl)benzofuran-2-carboxylate (168 mg, 0.3 mmol) and triethylamine (50 µL, 0.36 mmol) were added to dichloromethane (1 mL), and the resulting solution was then stirred at 0 °C for 30 min. Dichloromethane (1 mL) solution of N,N-dimethylcarbamoyl chloride (53 µL, 0.36 mmol) was then added dropwise and the resulting solution was stirred at room temperature for 5 h. After the reaction was found to be completed from the monitoring of the TLC, the solvent was distilled off under reduced pressure, and the obtained product was dissolved in water (10 mL) and then extracted three times with dichloromethane (10 mL). The organic phase was washed with saturated aqueous NaCl solution and then dried with anhydrous sodium sulfate. The crude product was separated by column chromatography (DCM/MeOH = 50/1) to obtain 141 mg of white solid, with a yield of 76%. ¹H NMR (400 MHz, Chloroform-*d*) δ 8.14 (d, *J* = 1.6 Hz, 1H), 7.89 (dd, *J* = 8.8, 1.8 Hz, 1H), 7.62 (d, *J* = 8.8 Hz, 1H), 7.32 (t, *J* = 7.0 Hz, 1H), 7.22 - 7.09 (m, 4H), 7.06 - 6.96 (m, 2H), 6.93 (d, *J* = 6.6 Hz, 2H), 4.47 (q, *J* = 7.1 Hz, 2H), 3.97 (d, *J* = 113.4 Hz, 2H), 3.34 (d, *J* = 46.4 Hz, 6H), δ 2.90 - 2.78 (m, 8H), 2.70 - 2.50 (m, 5H), 1.45 (t, *J* = 7.1 Hz, 3H). ¹³C NMR (101 MHz, Chloroform-*d*) δ 164.7, 159.8, 155.8, 151.0, 143.0, 138.2, 136.2, 133.8, 130.5, 129.3, 129.3, 128.5, 128.4, 126.8, 126.4, 125.5, 124.2, 122.6, 122.0, 112.9, 61.5, 52.2, 51.2, 47.2, 38.5, 34.7, 14.3, 9.3.

### Example 207

### ethyl 3-methyl-5-(N-(2-(4-(methylsulfonyl)piperazin-1-yl)phenyl)-N-phenethylsulfamoyl)benzofuran-2-carboxylate (M10-93)

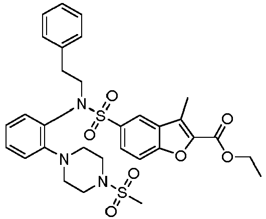

In accordance with the preparation method of ethyl 3-methyl-5-(N-(2-(4-acetylpiperazin-1-yl)phenyl)-N-phenethylsulfamoyl)benzofuran-2-carboxylate (M10-92), this example was implemented under the same condition except that methanesulfonyl chloride was used instead of *N, N*-dimethylcarbamoyl chloride. 158 mg of light yellow liquid product was obtained, with a yield of 94%. ¹H NMR (400 MHz, Chloroform-*d*) δ 8.12 (d, *J* = 1.9 Hz, 1H), 7.86 (dd, *J* = 8.8, 1.9 Hz, 1H), 7.64 (d, *J* = 8.8 Hz, 1H), 7.37 (td, *J* = 7.7, 1.6 Hz, 1H), 7.29 - 7.23 (m, 1H), 7.22 - 7.05 (m, 4H), 7.00 - 6.93 (m, 2H), 6.90 (dd, *J* = 7.9, 1.4 Hz, 1H), 4.48 (q, *J* = 7.1 Hz, 2H), 4.01 (s, 1H), 3.78 (s, 1H), 3.36 (d, *J* = 12.9 Hz, 6H), 2.97 (s, 2H), 2.83 (s, 3H), 2.69 (s, 1H), 2.60 (s, 3H), 2.54 (s, 1H), 1.46 (t, *J* = 7.1 Hz, 3H). ¹³C NMR (101 MHz, Chloroform-*d*) δ 159.8, 155.9, 151.3, 143.1, 138.0, 135.7, 134.5, 130.0, 129.6, 129.4, 128.5, 126.8, 126.6, 125.5, 125.1, 123.4, 122.1, 113.0, 61.6, 52.0, 51.7, 46.4, 34.6, 34.5, 31.6, 14.4, 9.4. HRMS (ESI) [M + H] ⁺, theoretically calculated for C₃₁H₃₆N₃O₇S₂: 626.1995, found 626.1990.

### Example 208

### ethyl 3-methyl-5-(N-(2-(4-(4-(3,3-dimethylbutyryl)piperazin-1-yl)phenyl)-N-phenethylsulfamoyl)benzofuran-2-carboxylate (M10-94)

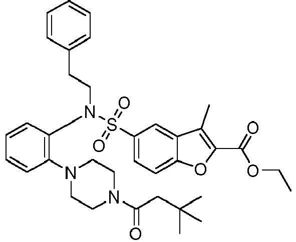

Ethyl 3-methyl-5-N-phenethyl-N-(2-(piperazin-1-yl)phenyl)sulfamoyl)benzofuran-2-carboxylate (200 mg, 0.36 mmol), 3,3-dimethyl-1-butanoic acid (92 mg, 0.79 mmol), 1-(3-dimethylaminopropyl)-3-ethylcarbodiimide hydrochloride (152 mg, 0.79 mmol), 1-hydroxybenzotriazole (107 mg, 0.79 mmol), N, N-diisopropylethylamine (150 mg, 1.08 mmol), and 4-dimethylaminopyridine (5 mg, 0.04 mmol) were added to dichloromethane (2 mL), and the resulting solution was then stirred at room temperature overnight. After the reaction was found to be completed from the monitoring of the TLC, the solvent was distilled off under reduced pressure, and the obtained product was dissolved in water (10 mL) and then extracted three times with dichloromethane (10 mL). The organic phase was washed with saturated aqueous NaCl solution and then dried with anhydrous sodium sulfate. The crude product was separated by column chromatography (PE/EA = 8/1 ~ 6/1) to obtain 120 mg of white solid, with a yield of 52%. ¹H NMR (400 MHz, Chloroform-*d*) δ 8.15 (d, *J* = 1.8 Hz, 1H), 7.89 (dd, *J* = 8.8, 1.9 Hz, 1H), 7.64 (d, *J* = 8.8 Hz, 1H), 7.38 - 7.31 (m, 1H), 7.23 - 7.12 (m, 4H), 7.10-7.04 (m, 1H), 6.99 - 6.92 (m, 3H), 4.49 (q, *J* = 7.1 Hz, 2H), 4.25 - 3.94 (m, 2H), 3.88 - 3.76 (m, 1H), 3.75 - 3.30 (m, 4H), 3.10 (s, 1H), 2.84 (s, 2H), 2.74 - 2.65 (m, 1H), 2.61 (s, 4H), 2.30 (s, 2H), 1.47 (t, *J* = 7.1 Hz, 3H), 1.08 (s, 9H). ¹³C NMR (101 MHz, Chloroform-*d*) δ 170.6, 159.8, 155.8, 150.9, 143.1, 138.1, 136.0, 134.0, 130.4, 129.5, 129.4, 128.5, 128.5, 126.8, 126.5, 125.5, 124.6, 122.8, 122.1, 112.9, 61.6, 51.4, 44.7, 34.6, 31.5, 30.1, 29.6, 14.4, 9.4. HRMS (ESI) [M + H] ⁺, theoretically calculated for C₃₆H₄₄N₃O₆S: 646.2951, found 646.2958.

### Example 209

### ethyl 3-methyl-5-(N-(2-(4-(4-(benzylpiperazin-1-yl)phenyl)-N-phenethylsulfamoyl)benzofuran-2-carboxylate (M10-95)

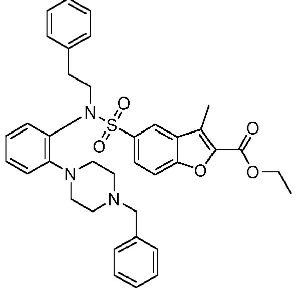

Ethyl 3-methyl-5-N-phenethyl-N-(2-(piperazin-1-yl)phenyl)sulfamoyl)benzofuran-2-carboxylate (164 mg, 0.3 mmol) and potassium carbonate (83 mg, 0.6 mmol) were dissolved in acetonitrile (2 mL), benzyl bromide (54 µL, 0.45 mmol) was then added, and the reaction was performed at 60 °C overnight. After the reaction was found to be completed from the monitoring of the TLC, the solvent was distilled off under reduced pressure, and the obtained product was dissolved in water (20 mL) and then extracted three times with ethyl acetate (20 mL). The organic phase was washed with saturated aqueous NaCl solution and then dried with anhydrous sodium sulfate. The crude product was separated by column chromatography (DCM/MeOH = 50/1) to obtain 129 mg of white solid, with a yield of 67%. ¹H NMR (400 MHz, Chloroform-*d*) δ 8.19 (d, *J* = 1.6 Hz, 1H), 7.93 (dd, *J* = 8.8, 1.9 Hz, 1H), 7.63 (d, *J* = 8.8 Hz, 1H), 7.42 - 7.30 (m, 6H), 7.24 - 7.14 (m, 4H), 7.06 (qd, *J* = 7.9, 1.7 Hz, 2H), 7.02 - 6.96 (m, 2H), 4.51 (q, *J* = 7.1 Hz, 2H), 4.29 - 4.09 (m, 1H), 3.92 (s, 1H), 3.55 (s, 2H), 3.23 (s, 2H), 3.00 - 2.79 (m, 2H), 2.74 - 2.63 (m, 2H), 2.56 (dd, *J* = 16.2, 5.9 Hz, 4H), 1.49 (t, *J* = 7.1 Hz, 3H). ¹³C NMR (101 MHz, Chloroform-*d*) δ 159.9, 155.8, 151.2, 143.0, 138.4, 137.9, 136.4, 133.5, 130.8, 129.3, 129.2, 128.6, 128.5, 128.4, 128.3, 127.5, 127.2, 126.9, 126.9, 126.4, 125.6, 123.8, 122.4, 122.0, 112.8, 63.2, 61.6, 53.6, 52.2, 51.1, 34.8, 14.4, 9.4.

### Example 210

### ethyl 3-methyl-5-(N-(2-(4-(benzoylpiperazin-1-yl)phenyl)-N-phenethylsulfamoyl)benzofuran-2-carboxylate (M10-96)

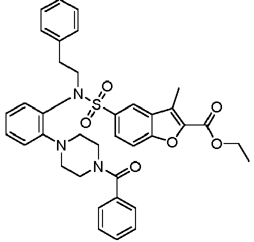

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that benzoic acid was used instead of 3,3-dimethyl-1-butanoic acid. 148 mg of white solid was obtained, with a yield of 63%. ¹H NMR (400 MHz, Chloroform-*d*) δ 8.14 (d, *J* = 1.9 Hz, 1H), 7.88 (dd, *J* = 8.8, 1.9 Hz, 1H), 7.62 (d, *J* = 8.8 Hz, 1H), 7.43 (dt, *J* = 4.9, 3.4 Hz, 5H), 7.35 (td, *J* = 7.7, 1.6 Hz, 1H), 7.25 - 7.05 (m, 5H), 6.96 (ddd, *J* = 9.5, 7.8, 1.5 Hz, 3H), 4.48 (q, *J* = 7.1 Hz, 2H), 4.29 - 3.94 (m, 2H), 3.90 - 3.45 (m, 4H), 3.27 (s, 2H), 3.04 - 2.63 (m, 3H), 2.59 (s, 4H), 1.46 (t, *J* = 7.1 Hz, 3H). ¹³C NMR (101 MHz, Chloroform-*d*) δ 170.5, 159.8, 155.8, 151.0, 143.1, 138.1, 135.9, 135.8, 134.1, 130.3, 129.7, 129.5, 129.4, 128.5, 127.1, 126.8, 126.5, 125.5, 124.7, 122.9, 122.1, 112.9, 61.6, 51.5, 34.7, 14.4, 9.4. HRMS (ESI) [M + H] ⁺, theoretically calculated for C₃₇H₃₈N₃O₆S: 652.2481, found 652.2487.

### Example 211

### ethyl 3-methyl-5-(N-(2-(4-methylpiperazin-1-yl)benzyl)-N-phenethylsulfamoyl)benzofuran-2-carboxylate (M10-98)

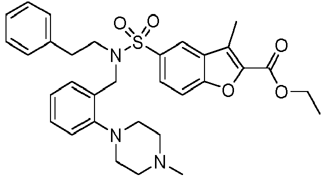

In accordance with the preparation method of ethyl 3-methyl-5-(N-(2-(4-methylpiperazin-1-yl)phenyl)-N-phenethylsulfamoyl)benzofuran-2-carboxylate (M10-91), this example was implemented under the same condition except that ethyl 3-methyl-5-(N-phenethyl-N-(2-(piperazin-1-yl)benzyl)sulfamoyl)benzofuran-2-carboxylate (M9-2), and methyl iodide (102 mg, 0.36 mmol) were used instead of ethyl 3-methyl-5-(N-phenethyl-N-(2-(piperazin-1-yl)phenyl)sulfamoyl)benzofuran-2-carboxylate (M9-1). 113 mg of white solid was obtained, with a yield of 66%. ¹H NMR (400 MHz, Chloroform-*d*) δ 8.15 (s, 1H), 7.89 (d, *J* = 8.6 Hz, 1H), 7.65 (d, *J* = 8.7 Hz, 1H), 7.38 - 7.25 (m, 3H), 7.24 - 7.04 (m, 5H), 6.87 (d, *J* = 6.8 Hz, 2H), 4.61 - 4.36 (m, 4H), 3.26 (t, *J* = 8.1 Hz, 2H), 3.00 (d, *J* = 69.2 Hz, 8H), 2.61 (s, 3H), 2.53 (s, 5H), 1.45 (t, *J* = 7.1 Hz, 3H). ¹³C NMR (101 MHz, Chloroform-*d*) δ 159.8, 155.8, 151.3, 143.0, 138.2, 135.1, 130.9, 130.4, 129.5, 129.2, 128.5, 128.4, 126.4, 126.2, 125.5, 124.9, 121.4, 121.2, 113.1, 61.6, 54.9, 51.6, 49.3, 47.5, 45.1, 34.9, 14.4, 9.4.

### Example 212

### 4-(2-((2-(ethoxycarbonyl)-3-methyl-N-benzylethylbenzofuran)-5-sulfonylamino)phenyl)-1,1-dimethylpiperazin-1-ium (M10-99)

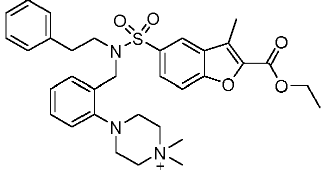

In accordance with the preparation method of ethyl 3-methyl-5-(N-(2-(4-methylpiperazin-1-yl)phenyl)-N-phenethylsulfamoyl)benzofuran-2-carboxylate (M10-90), this example was implemented under the same condition except that ethyl 3-methyl-5-(N-phenethyl-N-(2-(piperazin-1-yl)benzyl)sulfamoyl)benzofuran-2-carboxylate (M9-2) was used instead of ethyl 3-methyl-5-(N-phenethyl-N-(2-(piperazin-1-yl)phenyl)sulfamoyl)benzofuran-2-carboxylate (M9-1). 81 mg of yellow solid was obtained, with a yield of 76%. ¹H NMR (400 MHz, Chloroform-*d*) δ 8.16 (s, 1H), 7.93 (d, *J* = 8.8 Hz, 1H), 7.68 (d, *J* = 8.8 Hz, 1H), 7.49 (d, *J* = 7.8 Hz, 1H), 7.37 (t, *J* = 7.2 Hz, 1H), 7.26 - 7.04 (m, 5H), 6.80 (d, *J* = 6.7 Hz, 2H), 4.44 (d, *J* = 7.6 Hz, 4H), 3.94 (s, 4H), 3.63 (s, 6H), 3.30 (s, 4H), 3.22 - 3.11 (m, 2H), 2.61 (s, 3H), 2.44 - 2.27 (m, 2H), 1.42 (t, *J* = 7.0 Hz, 3H). ¹³C NMR (101 MHz, Chloroform-*d*) δ 159.8, 155.8, 150.1, 143.0, 138.0, 134.2, 131.7, 131.2, 130.0, 129.6, 128.5, 128.5, 126.5, 126.4, 126.1, 125.6, 123.3, 121.6, 113.4, 62.6, 61.6, 52.7, 49.9, 49.5, 46.8, 35.5, 14.3, 9.6.

### Example 213

### ethyl 3-methyl-5-(N-(2-(4-acetylpiperazin-1-yl)benzyl)-N-phenethylsulfamoyl)benzofuran-2-carboxylate (M10-100)

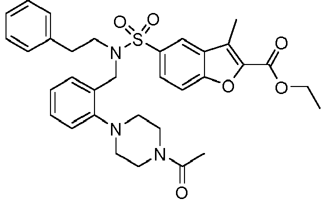

In accordance with the preparation method of ethyl 3-methyl-5-(N-(2-(4-acetylpiperazin-1-yl)phenyl)-N-phenethylsulfamoyl)benzofuran-2-carboxylate (M10-91), this example was implemented under the same condition except that ethyl 3-methyl-5-(N-phenethyl-N-(2-(piperazin-1-yl)benzyl)sulfamoyl)benzofuran-2-carboxylate (M9-2) was used instead of ethyl 3-methyl-5-(N-phenethyl-N-(2-(piperazin-1-yl)phenyl)sulfamoyl)benzofuran-2-carboxylate (M9-1). 154 mg of white solid was obtained, with a yield of 86%. ¹H NMR (400 MHz, Chloroform-*d*) δ 8.16 (s, 1H), 7.88 (d, *J* = 10.4 Hz, 1H), 7.63 (d, *J* = 8.7 Hz, 1H), 7.38 (d, *J* = 7.0 Hz, 1H), 7.29 (t, *J* = 7.6 Hz, 1H), 7.18 - 7.06 (m, 5H), 6.89 (s, 2H), 4.58 (s, 2H), 4.47 (q, *J* = 7.1 Hz, 2H), 3.65 (d, *J* = 56.1 Hz, 4H), 3.37 - 3.28 (m, 2H), 2.84 (d, *J* = 20.5 Hz, 4H), 2.65 - 2.53 (m, 5H), 2.12 (s, 3H), 1.45 (t, *J* = 7.1 Hz, 3H). ¹³C NMR (101 MHz, Chloroform-*d*) δ 169.1, 159.8, 155.7, 151.0, 143.0, 138.3, 135.3, 130.9, 130.1, 129.4, 128.9, 128.5, 126.5, 126.2, 125.5, 124.9, 121.5, 120.4, 113.0, 61.6, 53.2, 52.8, 49.4, 46.8, 46.6, 41.8, 35.0, 21.4, 14.4, 9.4.

### Example 214

### ethyl 3-methyl-5-(N-(2-(4-acetylpiperazin-1-yl)benzyl)-N-phenethylsulfamoyl)benzofuran-2-carboxylate (M10-101)

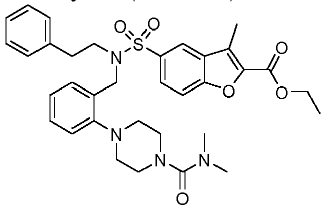

In accordance with the preparation method of ethyl 3-methyl-5-(N-(2-(4-acetylpiperazin-1-yl)phenyl)-N-phenethylsulfamoyl)benzofuran-2-carboxylate (M10-92), this example was implemented under the same condition except that ethyl 3-methyl-5-(N-phenethyl-N-(2-(piperazin-1-yl)benzyl)sulfamoyl)benzofuran-2-carboxylate (M9-2) was used instead of ethyl 3-methyl-5-(N-phenethyl-N-(2-(piperazin-1-yl)phenyl)sulfamoyl)benzofuran-2-carboxylate (M9-1). 162 mg of white solid was obtained, with a yield of 85%. ¹H NMR (400 MHz, Chloroform-*d*) δ 8.16 (d, *J* = 1.8 Hz, 1H), 7.88 (dd, *J* = 8.8, 1.9 Hz, 1H), 7.64 (d, *J* = 8.7 Hz, 1H), 7.43 (d, *J* = 7.7 Hz, 1H), 7.28 (t, *J* = 8.3 Hz, 1H), 7.20 - 7.05 (m, 5H), 6.90 (d, *J* = 6.6 Hz, 2H), 4.59 (s, 2H), 4.48 (q, *J* = 7.1 Hz, 2H), 3.35 (d, *J* = 3.9 Hz, 6H), 2.91 - 2.79 (m, 10H), 2.65 - 2.56 (m, 5H), 1.46 (t, *J* = 7.1 Hz, 3H). ¹³C NMR (101 MHz, Chloroform-*d*) δ 164.7, 159.9, 155.7, 151.2, 142.9, 138.3, 135.4, 130.9, 130.0, 129.4, 128.7, 128.5, 128.5, 126.4, 126.2, 125.5, 124.7, 121.5, 120.2, 113.0, 61.6, 52.9, 49.4, 47.1, 46.4, 38.5, 34.8, 14.4, 9.4.

### Example 215

### ethyl 3-methyl-5-(N-(2-(4-methanesulfonylpiperazin-1-yl)benzyl)-N-phenethylsulfamoyl)benzofuran-2-carboxylate (M10-102)

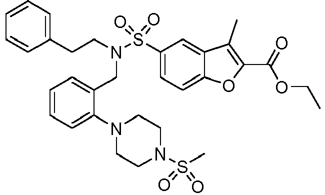

In accordance with the preparation method of ethyl 3-methyl-5-(N-(2-(4-methanesulfonylpiperazin-1-yl)phenyl)-N-phenethylsulfamoyl)benzofuran-2-carboxylate (M10-93), this example was implemented under the same condition except that ethyl 3-methyl-5-(N-phenethyl-N-(2-(piperazin-1-yl)benzyl)sulfamoyl)benzofuran-2-carboxylate (M9-2) was used instead of ethyl 3-methyl-5-(N-phenethyl-N-(2-(piperazin-1-yl)phenyl)sulfamoyl)benzofuran-2-carboxylate (M9-1). 86 mg of white solid was obtained, with a yield of 83%. ¹H NMR (400 MHz, Chloroform-*d*) δ 8.17 (s, 1H), 7.91 (d, *J* = 8.8 Hz, 1H), 7.67 (d, *J* = 8.7 Hz, 1H), 7.38 - 7.27 (m, 2H), 7.16 (dd, *J* = 18.9, 7.8 Hz, 5H), 6.91 *(d, J=* 7.2 Hz, 2H), 4.56 (s, 2H), 4.49 (q, *J* = 7.1 Hz, 2H), 3.40 (s, 4H), 3.30 (t, *J* = 8.1 Hz, 2H), 2.99 (s, 4H), 2.84 (s, 3H), 2.67 - 2.52 (m, 5H), 1.47 (t, *J* = 7.1 Hz, 3H). ¹³C NMR (101 MHz, Chloroform-*d*) δ 159.8, 155.8, 151.2, 143.0, 138.3, 135.2, 131.0, 130.4, 129.4, 129.2, 128.5, 126.5, 126.2, 125.5, 125.1, 121.5, 121.1, 113.1, 61.6, 52.5, 49.4, 47.5, 46.2, 35.2, 34.5, 14.4, 9.4.

### Example 216

### ethyl 3-methyl-5-(N-(2-(4-(3,3-dimethylbutyryl)piperazin-1-yl)benzyl)-N-phenethylsulfamoyl)benzofuran-2-carboxylate (M10-103)

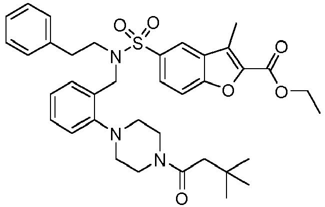

In accordance with the preparation method of ethyl 3-methyl-5-(N-(2-(4-methanesulfonylpiperazin-1-yl)phenyl)-N-phenethylsulfamoyl)benzofuran-2-carboxylate (M10-93), this example was implemented under the same condition except that ethyl 3-methyl-5-(N-phenethyl-N-(2-(piperazin-1-yl)benzyl)sulfamoyl)benzofuran-2-carboxylate (M9-2) was used instead of ethyl 3-methyl-5-(N-phenethyl-N-(2-(piperazin-1-yl)phenyl)sulfamoyl)benzofuran-2-carboxylate (M9-1). 197 mg of white solid was obtained, with a yield of 99%. ¹H NMR (400 MHz, Chloroform-*d*) δ 8.16 (d, *J* = 1.8 Hz, 1H), 7.88 (dd, J= 8.8, 1.8 Hz, 1H), 7.63 (d, J= 8.8 Hz, 1H), 7.38 (d, *J* = 7.7 Hz, 1H), 7.28 (t, *J* = 7.6 Hz, 1H), 7.19 - 7.05 (m, 5H), 6.89 (d, *J* = 6.6 Hz, 2H), 4.57 (s, 2H), 4.47 (q, *J* = 7.1 Hz, 2H), 3.70 (d, *J* = 49.1 Hz, 4H), 3.36 - 3.28 (m, 2H), 2.84 (dt, *J* = 16.4, 4.4 Hz, 4H), 2.61 (d, *J* = 3.8 Hz, 5H), 2.32 (s, 2H), 1.45 (t, *J* = 7.1 Hz, 3H), 1.07 (s, 9H). ¹³C NMR (101 MHz, Chloroform-*d*) δ 170.8, 159.8, 155.7, 151.0, 143.0, 138.3, 135.3, 130.9, 130.1, 129.4, 128.8, 128.5, 128.5, 126.4, 126.2, 125.5, 124.8, 121.5, 120.4, 113.0, 61.6, 53.3, 52.9, 49.4, 47.8, 46.8, 44.6, 41.9, 35.0, 31.6, 30.1, 14.4, 9.4.

### Example 217

### ethyl 3-methyl-5-(N-(2-(4-benzylpiperazin-1-yl)benzyl)-N-phenethylsulfamoyl)benzofuran-2-carboxylate (M10-104)

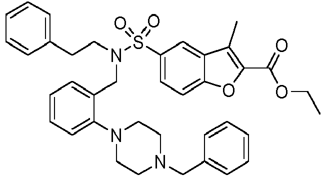

In accordance with the preparation method of ethyl 3-methyl-5-(N-(2-(4-benzylpiperazin-1-yl)phenyl)-N-phenethylsulfamoyl)benzofuran-2-carboxylate (M10-95), this example was implemented under the same condition except that ethyl 3-methyl-5-(N-phenethyl-N-(2-(piperazin-1-yl)benzyl)sulfamoyl)benzofuran-2-carboxylate (M9-2) was used instead of ethyl 3-methyl-5-(N-phenethyl-N-(2-(piperazin-1-yl)phenyl)sulfamoyl)benzofuran-2-carboxylate (M9-1). 138 mg of white solid was obtained, with a yield of 71%. ¹H NMR (400 MHz, Chloroform-*d*) δ 8.18 (d, *J* = 1.5 Hz, 1H), 7.91 (dd, *J* = 8.8, 1.9 Hz, 1H), 7.66 (d, *J* = 8.8 Hz, 1H), 7.44 (dd, *J* = 7.7, 1.6 Hz, 1H), 7.40 - 7.34 (m, 4H), 7.33 - 7.25 (m, 2H), 7.22 - 7.05 (m, 5H), 6.95 (d, *J* = 6.6 Hz, 2H), 4.61 (s, 2H), 4.52 (q, *J* = 7.1 Hz, 2H), 3.58 (s, 2H), 3.42 - 3.26 (m, 2H), 2.92 (s, 4H), 2.65 (s, 9H), 1.50 (t, *J* = 7.1 Hz, 3H). ¹³C NMR (101 MHz, Chloroform-*d*) 6 159.9, 155.8, 151.7, 142.9, 138.5, 137.8, 135.7, 130.7, 129.9, 129.4, 129.2, 128.6, 128.4, 128.3, 127.2, 127.0, 126.4, 126.3, 125.6, 124.3, 121.5, 120.1, 113.0, 63.1, 61.6, 53.5, 53.0, 49.3, 46.0, 34.7, 14.4, 9.5.

### Example 218

### ethyl 3-methyl-5-(N-(2-(4-benzoylpiperazin-1-yl)benzyl)-N-phenethylsulfamoyl)benzofuran-2-carboxylate (M10-105)

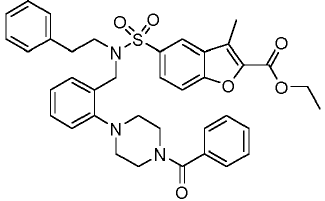

In accordance with the preparation method of ethyl 3-methyl-5-(N-(2-(4-(benzoylpiperazin-1-yl)phenyl)-N-phenethylsulfamoyl)benzofuran-2-carboxylate (M10-96), this example was implemented under the same condition except that ethyl 3-methyl-5-(N-phenethyl-N-(2-(piperazin-1-yl)benzyl)sulfamoyl)benzofuran-2-carboxylate (M9-2) was used instead of ethyl 3-methyl-5-(N-phenethyl-N-(2-(piperazin-1-yl)phenyl)sulfamoyl)benzofuran-2-carboxylate (M9-1). 181 mg of white solid was obtained, with a yield of 95%. ¹H NMR (400 MHz, Chloroform-*d*) δ 8.18 (d, *J* = 1.8 Hz, 1H), 8.12 (dd, *J* = 8.3, 1.2 Hz, 1H), 7.94 - 7.86 (m, 1H), 7.66 (d, *J* = 8.7 Hz, 1H), 7.45 (s, 6H), 7.22 - 7.10 (m, 5H), 6.92 (dd, *J* = 7.7, 1.4 Hz, 2H), 4.60 (s, 2H), 4.50 (q, *J* = 7.1 Hz, 2H), δ 3.59 (s, 4H), 3.38 - 3.29 (m, 2H), 2.91 (s, 4H) 2.63 (s, 5H), 1.48 (t, *J* = 7.1 Hz, 3H). ¹³C NMR (101 MHz, Chloroform-*d*) δ 170.6, 159.9, 155.8, 150.9, 143.0, 138.3, 135.6, 135.3, 133.5, 130.9, 130.2, 130.1, 129.8, 129.4, 129.0, 128.6, 128.5, 128.4, 127.1, 126.5, 126.2, 125.5, 125.0, 121.5, 120.5, 113.1, 61.6, 49.5, 46.9, 35.0, 14.4, 9.4.

### Example 219

### 3-methyl-5-(N-phenethyl-N-(2-(piperazin-1-yl)phenyl)sulfamoyl)benzofuran-2-carboxylic acid (F27-S89)

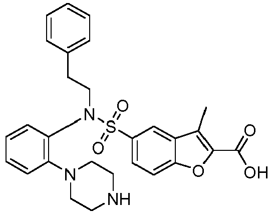

In accordance with the preparation method of 5-(N-benzyl-N-phenethylsulfamoyl)-3-methylbenzofuran-2-carboxylic acid (F27), this example was implemented under the same condition except that M10-89 was used instead of ethyl 5-(N-benzyl-*N*-phenethylsulfamoyl)-3-methylbenzofuran-2-carboxylate (F27-S8). 49 mg of white solid was obtained, with a yield of 55%. Mp 172 - 173 °C. ¹H NMR (400 MHz, DMSO-*d*₆) δ 8.24 (s, 1H), 7.92 - 7.81 (m, 2H), 7.39 (t, *J* = 7.3 Hz, 1H), 7.28 (d, *J* = 7.7 Hz, 1H), 7.17 - 7.04 (m, 4H), 7.01 (d, *J* = 7.7 Hz, 1H), 6.95 (d, *J* = 7.1 Hz, 2H), 3.96 (s, 2H), 3.79 (s, 2H), 3.28 (s, 2H), 3.11 (s, 4H), 3.03 (s, 2H), 2.58 (s, 3H), 2.43 (s, 1H). ¹³C NMR (101 MHz, DMSO-*d*₆) δ 161.1, 155.7, 150.6, 143.8, 138.5, 135.5, 134.1, 131.0, 129.9, 129.6, 128.9, 128.7, 127.1, 126.6, 125.4, 125.3, 123.3, 122.5, 113.6, 51.0, 49.2, 43.7, 34.2, 9.5. HRMS (ESI) [M - H] ⁻, theoretically calculated for C₂₈H₂₈N₃O₅S: 518.1750, found 518.1755.

### Example 220

### 4-(2-((2-carboxy-3-methyl-N-phenethylbenzofuran)-5-sulfonylamino)phenyl)-1,1-dimethylpiperazin-1-ium (F27-S90)

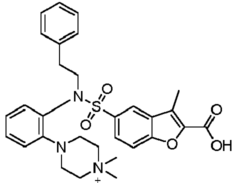

In accordance with the preparation method of 5-(N-benzyl-N-phenethylsulfamoyl)-3-methylbenzofuran-2-carboxylic acid (F27), this example was implemented under the same condition except that M10-90 was used instead of ethyl 5-(N-benzyl-N-phenethylsulfamoyl)-3-methylbenzofuran-2-carboxylate (F27-S8). 97 mg of white solid was obtained, with a yield of 74%. Mp 246 - 247 °C. ¹H NMR (400 MHz, DMSO-*d*₆) δ 8.19 (s, 1H), 7.85 (s, 2H), 7.53 (d, *J* = 8.0 Hz, 1H), 7.40 (t, *J* = 7.6 Hz, 1H), 7.12 (dt, *J* = 13.9, 7.4 Hz, 4H), 6.98 (d, *J*= 7.5 Hz, 3H), 4.09 - 3.92 (m, 1H), 3.89 - 3.71 (m, 1H), 3.49 (t, *J* = 5.3 Hz, 4H), 3.38 (d, *J* = 13.5 Hz, 2H), 3.26 (s, 6H), 3.12 (d, *J* = 13.9 Hz, 2H), 2.57 (s, 4H), 2.44 (s, 1H). ¹³C NMR (101 MHz, DMSO-*d*₆) δ 161.3, 155.6, 149.9, 145.0, 138.6, 135.4, 134.2, 131.0, 129.8, 129.0, 128.7, 126.8, 126.6, 125.7, 124.2, 124.0, 122.2, 113.5, 61.6, 51.3, 45.9, 34.3, 9.5. HRMS (ESI) [M] ⁺, theoretically calculated for C₃₀H₃₄N₃O₅S⁺: 548.2219, found 548.2222.

### Example 221

### 3-methyl-5-(N-(2-(4-acetylpiperazin-1-yl)phenyl)-N-phenethylsulfamoyl)benzofuran-2-carboxylic acid (F27-S91)

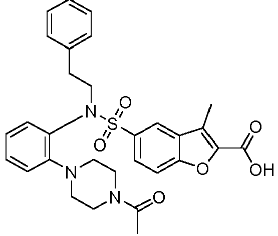

In accordance with the preparation method of 5-(N-benzyl-N-phenethylsulfamoyl)-3-methylbenzofuran-2-carboxylic acid (F27), this example was implemented under the same condition except that M10-91 was used instead of ethyl 5-(N-benzyl-*N*-phenethylsulfamoyl)-3-methylbenzofuran-2-carboxylate (F27-S8). 59 mg of white solid was obtained, with a yield of 75%. Mp 196 - 197 °C. ¹H NMR (400 MHz, DMSO-*d*₆) δ 8.24 (s, 1H), 7.89 (q, *J* = 8.8 Hz, 2H), 7.36 (t, *J* = 7.7 Hz, 1H), 7.24 (d, *J* = 8.0 Hz, 1H), 7.12 (dt, *J* = 12.6, 7.1 Hz, 4H), 7.02 (d, *J* = 7.8 Hz, 1H), 6.95 (d, *J* = 7.2 Hz, 2H), 4.06 (s, 1H), 3.84 (s, 1H), 3.43 (d, *J* = 37.5 Hz, 6H), 3.13 (s, 1H), 2.95 (s, 1H), 2.73 (s, 2H), 2.59 (s, 3H), 2.02 (s, 3H). ¹³C NMR (101 MHz, DMSO-*d*₆) δ 168.8, 161.3, 155.6, 151.1, 138.6, 135.9, 133.8, 131.0, 129.7, 129.7, 128.9, 128.7, 126.9, 126.6, 124.7, 123.1, 122.3, 113.5, 52.6, 52.0, 50.8, 46.6, 41.7, 34.3, 21.7, 9.5. HRMS (ESI) [M - H] ⁻, theoretically calculated for C₃₀H₃₀N₃O₆S: 560.1855, found 560.1865.

### Example 222

### 3-methyl-5-(N-(2-(4-(dimethylcarbamoyl)piperazin-1-yl)phenyl)-N-phenethylsulfamoyl)benzofuran-2-carboxylic acid (F27-S92)

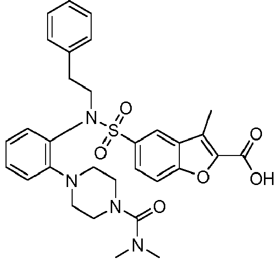

In accordance with the preparation method of 5-(N-benzyl-N-phenethylsulfamoyl)-3-methylbenzofuran-2-carboxylic acid (F27), this example was implemented under the same condition except that M10-92 was used instead of ethyl 5-(N-benzyl-*N*-phenethylsulfamoyl)-3-methylbenzofuran-2-carboxylate (F27-S8). 97 mg of white solid was obtained, with a yield of 83%. Mp > 380 °C (for decomposition).¹H NMR (400 MHz, DMSO-*d*₆) δ 8.19 (s, 1H), 7.93 - 7.77 (m, 2H), 7.34 (t, *J* = 7.4 Hz, 1H), 7.22 (d, *J* = 7.9 Hz, 1H), 7.18 - 7.02 (m, 5H), 6.95 (d, *J* = 7.1 Hz, 2H), 3.86 (s, 2H), 3.17 (s, 2H), 3.13 - 2.91 (m, 4H), 2.74 (s, 8H), 2.58 (s, 3H), 2.51 (s, 2H). ¹³C NMR (101 MHz, DMSO-*d*₆) δ 164.1, 155.3, 151.0, 138.6, 135.5, 133.6, 131.1, 130.3, 129.5, 128.8, 128.7, 126.6, 126.0, 124.4, 122.8, 121.7, 113.2, 52.0, 50.6, 47.3, 38.5, 34.4, 9.5. HRMS (ESI) [M - H] ⁻, theoretically calculated for C₃₁H₃₃N₄O₆S: 589.2126, found 589.2122.

### Example 223

### 3-methyl-5-(N-(2-(4-(methylsulfonyl)piperazin-1-yl)phenyl)-N-phenethylsulfamoyl)benzofuran-2-carboxylic acid (F27-S93)

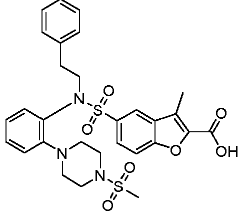

In accordance with the preparation method of 5-(N-benzyl-N-phenethylsulfamoyl)-3-methylbenzofuran-2-carboxylic acid (F27), this example was implemented under the same condition except that M10-93 was used instead of ethyl 5-(N-benzyl-*N*-phenethylsulfamoyl)-3-methylbenzofuran-2-carboxylate (F27-S8). 60 mg of white solid was obtained, with a yield of 72%. Mp 144 - 145 °C. ¹H NMR (400 MHz, DMSO-*d*₆) δ 8.22 (s, 1H), 7.94 - 7.81 (m, 2H), 7.41 - 7.27 (m, 2H), 7.11 (dd, *J* = 13.5, 6.9 Hz, 4H), 6.99 (dd, *J* = 19.9, 7.3 Hz, 3H), 3.93 (d, *J* = 62.2 Hz, 4H), 3.13 (s, 6H), 2.87 (d, *J* = 23.0 Hz, 5H), 2.58 (s, 3H). ¹³C NMR (101 MHz, DMSO-*d*₆) δ 161.3, 155.6, 151.0, 144.4, 138.6, 135.8, 134.0, 131.1, 129.7, 129.7, 128.9, 128.7, 127.0, 126.6, 125.1, 124.7, 123.6, 122.3, 113.5, 51.9, 51.0, 46.3, 34.6, 34.3, 9.5. HRMS (ESI) [M - H] ⁻, theoretically calculated for C₂₉H₃₀N₃O₇S₂: 596.1525, found 596.1534.

### Example 224

### 3-methyl-5-(N-(2-(4-(4-(3,3-dimethylbutyryl)piperazin-1-yl)phenyl)-N-phenethylsulfamoyl)benzofuran-2-carboxylic acid (F27-S94)

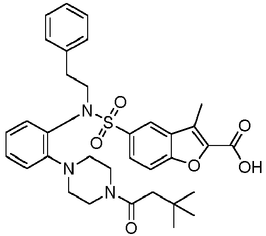

In accordance with the preparation method of 5-(N-benzyl-N-phenethylsulfamoyl)-3-methylbenzofuran-2-carboxylic acid (F27), this example was implemented under the same condition except that M10-94 was used instead of ethyl 5-(N-benzyl-*N*-phenethylsulfamoyl)-3-methylbenzofuran-2-carboxylate (F27-S8). 66 mg of white solid was obtained, with a yield of 89%. Mp 191 - 192 °C. ¹H NMR (400 MHz, DMSO-*d*₆) δ 8.25 (s, 1H), 7.88 (q, *J* = 8.1 Hz, 2H), 7.35 (t, *J* = 6.5 Hz, 1H), 7.23 (d, *J* = 7.6 Hz, 1H), 7.19 - 7.02 (m, 5H), 6.96 (d, *J* = 6.4 Hz, 2H), 4.06 (s, 1H), 3.87 (s, 1H), 3.71 - 3.27 (m, 6H), 2.98 (d, *J* = 37.3 Hz, 2H), 2.70 (s, 2H), 2.59 (s, 3H), 2.22 (s, 2H), 0.98 (s, 9H). ¹³C NMR (101 MHz, DMSO-*d*₆) δ 170.0, 161.3, 155.6, 151.0, 138.6, 135.9, 133.7, 131.2, 129.7, 129.6, 128.9, 128.7, 126.9, 126.6, 124.7, 123.1, 122.3, 113.5, 52.9, 52.0, 50.8, 46.9, 44.1, 41.7, 34.4, 31.5, 30.2, 9.5. HRMS (ESI) [M - H] ⁻, theoretically calculated for C₃₄H₃₈N₃O₆S: 616.2481, found 616.2470.

### Example 225

### 3-methyl-5-(N-(2-(4-(4-(benzylpiperazin-1-yl)phenyl)-N-phenethylsulfamoyl)benzofuran-2-carboxylic acid (F27-S95)

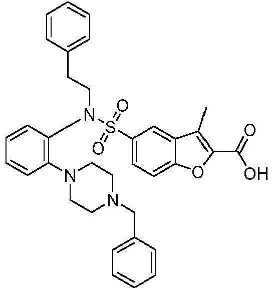

In accordance with the preparation method of 5-(N-benzyl-N-phenethylsulfamoyl)-3-methylbenzofuran-2-carboxylic acid (F27), this example was implemented under the same condition except that M10-95 was used instead of ethyl 5-(N-benzyl-*N*-phenethylsulfamoyl)-3-methylbenzofuran-2-carboxylate (F27-S8). 90 mg of white solid was obtained, with a yield of 74%. Mp 161 - 162 °C. ¹H NMR (400 MHz, DMSO-*d*₆) δ 8.24 (s, 1H), 7.95 - 7.79 (m, 2H), 7.66 - 7.52 (m, 2H), 7.48 - 7.33 (m, 4H), 7.26 (d, *J* = 7.9 Hz, 1H), 7.15 (dt, *J* = 13.8, 6.2 Hz, 4H), 7.05 - 6.92 (m, 3H), 4.16 (d, *J* = 8.5 Hz, 2H), 4.00 (s, 1H), 3.88 - 3.72 (m, 1H), 3.27 (s, 2H), 3.00 (d, *J* = 37.0 Hz, 6H), 2.59 (s, 3H), 2.51 (s, 2H). ¹³C NMR (101 MHz, DMSO-*d*₆) δ 161.2, 155.6, 150.3, 144.0, 138.6, 135.5, 133.8, 131.3, 131.1, 129.8, 129.6, 129.3, 129.0, 128.9, 128.7, 127.1, 126.7, 125.1, 125.0, 123.0, 122.4, 113.5, 59.8, 51.9, 51.0, 49.6, 34.3, 9.6. HRMS (ESI) [M - H] ⁻, theoretically calculated for C₃₅H₃₄N₃O₅S: 608.2225, found 608.2216.

### Example 226

### 3-methyl-5-(N-(2-(4-(benzoylpiperazin-1-yl)phenyl)-N-phenethylsulfamoyl)benzofuran-2-carboxylic acid (F27-S96)

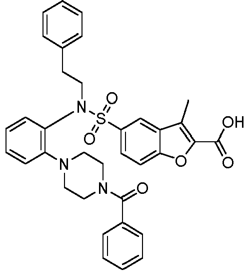

In accordance with the preparation method of 5-(N-benzyl-N-phenethylsulfamoyl)-3-methylbenzofuran-2-carboxylic acid (F27), this example was implemented under the same condition except that M10-96 was used instead of ethyl 5-(N-benzyl*-N*-phenethylsulfamoyl)-3-methylbenzofuran-2-carboxylate (F27-S8). 68 mg of white solid was obtained, with a yield of 73%. Mp 128 - 129 °C. ¹H NMR (400 MHz, DMSO-*d*₆) δ 8.23 (s, 1H), 7.87 (q, *J* = 9.0 Hz, 2H), 7.52 - 7.21 (m, 7H), 7.09 (dt, *J* = 22.3, 7.0 Hz, 5H), 6.97 (d, *J* = 6.3 Hz, 2H), 4.06 (s, 1H), 3.86 (s, 1H), 3.44 (d, *J* = 120.0 Hz, 6H), 2.99 (d, *J* = 26.6 Hz, 2H), 2.76 (d, *J* = 26.4 Hz, 2H), 2.56 (s, 3H). ¹³C NMR (101 MHz, DMSO-*d*₆) δ 169.5, 161.3, 155.6, 150.9, 138.6, 136.3, 135.8, 133.7, 131.3, 130.0, 129.7, 129.6, 128.9, 128.8, 128.7, 127.3, 127.0, 126.6, 124.8, 124.6, 123.2, 122.2, 113.5, 50.8, 34.4, 9.5. HRMS (ESI) [M - H] ⁻, theoretically calculated for C₃₅H₃₂N₃O₆S: 622.2012, found 622.2005.

### Example 227

### 3-methyl-5-(N-(2-(piperazin-1-yl)benzyl)-N-phenethylsulfamoyl)benzofuran-2-carboxylic acid (F27-S97)

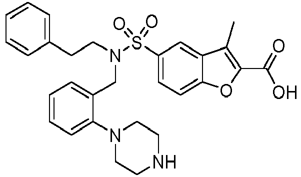

In accordance with the preparation method of 5-(N-benzyl-N-phenethylsulfamoyl)-3-methylbenzofuran-2-carboxylic acid (F27), this example was implemented under the same condition except that M10-97 was used instead of ethyl 5-(N-benzyl-*N*-phenethylsulfamoyl)-3-methylbenzofuran-2-carboxylate (F27-S8). 80 mg of white solid was obtained, with a yield of 75%. Mp 223 - 224 °C. ¹H NMR (400 MHz, DMSO-*d*₆) δ 9.83 (s, 1H), 8.25 (s, 1H), 7.95 (d, *J* = 8.8 Hz, 1H), 7.85 (d, *J* = 8.8 Hz, 1H), 7.41 (d, *J* = 7.6 Hz, 1H), 7.30 (d, *J* = 7.8 Hz, 1H), 7.14 (dt, *J* = 15.6, 7.0 Hz, 5H), 6.91 (d, *J* = 7.2 Hz, 2H), 4.51 (s, 2H), 3.23 (d, *J* = 21.6 Hz, 6H), 3.07 (s, 4H), 2.58 (s, 3H), 2.46 (s, 2H). ¹³C NMR (101 MHz, DMSO-*d*₆) δ 161.2, 155.5, 151.0, 138.7, 136.3, 135.0, 131.9, 130.2, 129.7, 129.1, 128.9, 128.8, 126.7, 125.0, 122.0, 120.7, 113.5, 110.0, 49.9, 49.7, 47.4, 43.6, 34.7, 9.6. HRMS (ESI) [M - H] ⁻, theoretically calculated for C₂₉H₃₀N₃O₅S: 532.1912, found 532.1896.

### Example 228

### 3-methyl-5-(N-(2-(4-acetylpiperazin-1-yl)benzyl)-N-phenethylsulfamoyl)benzofuran-2-carboxylic acid (F27-S98)

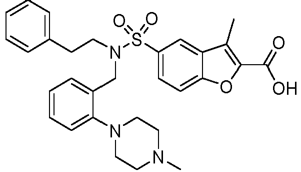

In accordance with the preparation method of 5-(N-benzyl-N-phenethylsulfamoyl)-3-methylbenzofuran-2-carboxylic acid (F27), this example was implemented under the same condition except that M10-98 was used instead of ethyl 5-(N-benzyl-*N*-phenethylsulfamoyl)-3-methylbenzofuran-2-carboxylate (F27-S8). 77 mg of white solid was obtained, with a yield of 71%. Mp 190 - 191 °C. ¹H NMR (400 MHz, DMSO-*d*₆) δ 8.34 (s, 1H), 8.01 (d, *J* = 8.7 Hz, 1H), 7.89 (d, *J* = 8.7 Hz, 1H), 7.41 (d, *J* = 7.4 Hz, 1H), 7.32 (t, *J* = 7.6 Hz, 1H), 7.16 (dt, *J* = 15.7, 6.6 Hz, 5H), 6.93 (d, *J* = 7.0 Hz, 2H), 4.53 (s, 2H), 3.42 (s, 4H), 3.13 (s, 6H), 2.78 (s, 3H), 2.60 (s, 3H), 2.49 - 2.40 (m, 2H). ¹³C NMR (101 MHz, DMSO) δ 161.1, 155.6, 150.4, 138.7, 135.1, 131.9, 130.0, 129.7, 129.0, 128.9, 128.8, 126.8, 126.7, 125.3, 125.0, 122.1, 120.6, 113.6, 110.0, 53.3, 50.1, 49.7, 47.1, 42.5, 34.7, 9.6. HRMS (ESI) [M - H] ⁻, theoretically calculated for C₃₀H₃₂N₃O₅S: 546.2068, found 546.2057.

### Example 229

### 4-(2-(((2-carboxy-3-methyl-N-phenethylbenzofuran)-5-sulfonamido)methyl)phenyl)-1,1-dimethylpiperazin-1-ium (F27-S99)

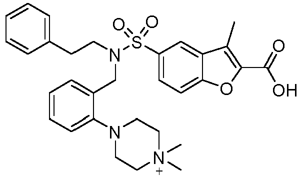

In accordance with the preparation method of 5-(N-benzyl-N-phenethylsulfamoyl)-3-methylbenzofuran-2-carboxylic acid (F27), this example was implemented under the same condition except that M10-99 was used instead of ethyl 5-(N-benzyl-*N*-phenethylsulfamoyl)-3-methylbenzofuran-2-carboxylate (F27-S8). 81 mg of white solid was obtained, with a yield of 76%. Mp 143 - 144 °C. ¹H NMR (400 MHz, DMSO-*d*₆) δ 8.36 (s, 1H), 8.03 (d, *J* = 8.7 Hz, 1H), 7.90 (d, *J* = 8.7 Hz, 1H), 7.46 - 7.31 (m, 3H), 7.16 (dt, *J* = 15.7, 7.1 Hz, 4H), 6.93 (d, *J* = 7.1 Hz, 2H), 4.56 (s, 2H), 3.61 - 3.53 (m, 4H), 3.25 (d, *J* = 7.5 Hz, 8H), 3.21 (d, *J* = 5.5 Hz, 4H), 2.60 (s, 3H), 2.45 (t, *J* = 8.1 Hz, 2H). ¹³C NMR (101 MHz, DMSO-*d*₆) δ 161.1, 155.6, 150.1, 143.7, 138.7, 135.1, 132.3, 129.9, 129.7, 128.9, 128.9, 128.8, 126.9, 126.7, 125.3, 125.3, 122.1, 121.6, 113.6, 61.6, 56.5, 50.3, 47.4, 46.4, 34.8, 9.6. HRMS (ESI) [M] ⁺, theoretically calculated for C₃₁H₃₆N₃O₅S: 562.2370, found 562.2383.

### Example 230

### 3-methyl-5-(N-(2-(4-acetylpiperazin-1-yl)benzyl)-N-phenethylsulfamoyl)benzofuran-2-carboxylic acid (F27-S100)

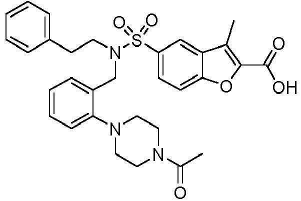

In accordance with the preparation method of 5-(N-benzyl-N-phenethylsulfamoyl)-3-methylbenzofuran-2-carboxylic acid (F27), this example was implemented under the same condition except that M10-100 was used instead of ethyl 5-(N-benzyl-*N*-phenethylsulfamoyl)-3-methylbenzofuran-2-carboxylate (F27-S8). 92 mg of white solid was obtained, with a yield of 61%. Mp 236 - 237 °C. ¹H NMR (400 MHz, DMSO-*d*₆) δ 13.74 (s, 1H), δ 8.30 (s, 1H), 8.03 - 7.82 (m, 2H), 7.39 (d, *J* = 7.3 Hz, 1H), 7.29 (t, *J* = 7.6 Hz, 1H), 7.14 (dq, *J* = 15.0, 8.0, 7.3 Hz, 5H), 6.94 (d, *J* = 7.2 Hz, 2H), 4.54 (s, 3H), 3.51 (s, 4H), 3.37 - 3.23 (m, 4H), 2.78 (dt, *J* = 28.9, 4.9 Hz, 4H), 2.59 (s, 3H), 2.54 - 2.49 (m, 2H), 2.02 (s, 3H). ¹³C NMR (101 MHz, DMSO-*d*₆) δ 168.8, 161.1, 155.5, 151.6, 143.7, 138.7, 135.2, 131.7, 129.9, 129.6, 128.9, 128.8, 128.8, 126.8, 126.7, 125.3, 124.5, 122.0, 120.7, 113.5, 56.5, 53.1, 52.9, 49.8, 46.7, 46.5, 41.7, 34.5, 21.7, 19.0, 9.6. HRMS (ESI) [M - H] ⁻, theoretically calculated for C₃₁H₃₂N₃O₆S: 574.2017, found 574.2017.

### Example 231

### 3-methyl-5-(N-(2-(4-(dimethylcarbamoyl)piperazin-1-yl)benzyl)-N-phenethylsulfamoyl)benzofuran-2-carboxylic acid (F27-S101)

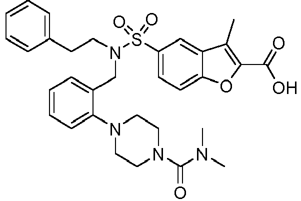

In accordance with the preparation method of 5-(N-benzyl-N-phenethylsulfamoyl)-3-methylbenzofuran-2-carboxylic acid (F27), this example was implemented under the same condition except that M10-101 was used instead of ethyl 5-(N-benzyl-*N*-phenethylsulfamoyl)-3-methylbenzofuran-2-carboxylate (F27-S8). 45 mg of white solid was obtained, with a yield of 28%. Mp 118 - 119 °C. ¹H NMR (400 MHz, DMSO-*d*₆) δ 8.29 (s, 1H), 7.90 (dd, *J* = 53.2, 8.7 Hz, 2H), 7.41 (d, *J* = 7.5 Hz, 1H), 7.24 (dd, *J* = 26.0, 7.5 Hz, 3H), 7.10 (dd, *J* = 17.5, 7.1 Hz, 4H), 6.92 (d, *J* = 7.3 Hz, 2H), 4.60 (s, 2H), 3.33 - 3.15 (m, 6H), 2.87 (s, 4H), 2.73 (s, 6H), 2.49 (s, 2H). ¹³C NMR (101 MHz, DMSO-*d*₆) δ 164.0, 161.0, 155.5, 143.7, 138.7, 135.2, 131.5, 130.0, 129.6, 129.0, 128.8, 128.7, 126.7, 126.6, 125.2, 125.0, 122.0, 120.7, 113.5, 53.0, 49.9, 46.9, 46.8, 38.5, 34.5, 9.5. HRMS (ESI) [M - H] ⁻, theoretically calculated for C₃₂H₃₅N₄O₆S: 603.2283, found 603.2270.

### Example 232

### 3-methyl-5-(N-(2-(4-(methylsulfonyl)piperazin-1-yl)benzyl)-N-phenethylsulfamoyl)benzofuran-2-carboxylic acid (F27-S102)

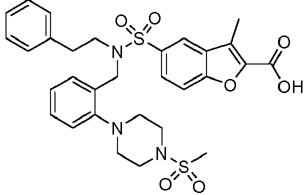

In accordance with the preparation method of 5-(N-benzyl-N-phenethylsulfamoyl)-3-methylbenzofuran-2-carboxylic acid (F27), this example was implemented under the same condition except that M10-102 was used instead of ethyl 5-(N-benzyl-*N*-phenethylsulfamoyl)-3-methylbenzofuran-2-carboxylate (F27-S8). 52 mg of white solid was obtained, with a yield of 57%. Mp 150 - 151 °C. ¹H NMR (400 MHz, DMSO-*d*₆) δ 8.29 (s, 1H), 7.92 (dd, *J* = 41.7, 9.3 Hz, 2H), 7.38 (d, *J* = 7.4 Hz, 1H), 7.29 (t, *J* = 7.4 Hz, 1H), 7.15 (ddd, *J* = 29.1, 12.9, 7.2 Hz, 5H), 6.94 (d, *J* = 7.1 Hz, 2H), 4.54 (s, 2H), 3.29 (t, *J* = 7.9 Hz, 2H), 3.21 (s, 4H), 2.97 - 2.84 (m, 7H), 2.59 (s, 3H), 2.54 (t, 2H). ¹³C NMR (101 MHz, DMSO-*d*₆) δ 161.3, 155.5, 151.3, 144.1, 138.7, 135.2, 131.7, 129.8, 129.7, 128.9, 128.8, 126.7, 126.6, 124.8, 124.7, 122.0, 120.8, 113.5, 52.4, 49.9, 46.8, 46.3, 34.6, 34.5, 9.6. HRMS (ESI) [M - H] ⁻, theoretically calculated for C₃₀H₃₂N₃O₇S₂: 610.1687, found 610.1687.

### Example 233

### 3-methyl-5-(N-(2-(4-(4-(3,3-dimethylbutyryl)piperazin-1-yl)benzyl)-N-phenethylsulfamoyl)benzofuran-2-carboxylic acid (F27-S103)

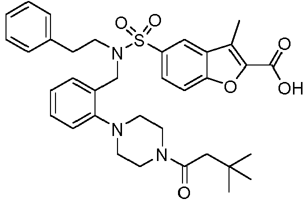

In accordance with the preparation method of 5-(N-benzyl-N-phenethylsulfamoyl)-3-methylbenzofuran-2-carboxylic acid (F27), this example was implemented under the same condition except that M10-94 was used instead of ethyl 5-(N-benzyl-*N*-phenethylsulfamoyl)-3-methylbenzofuran-2-carboxylate (F27-S8). 75 mg of white solid was obtained, with a yield of 39%. Mp 190 - 191 °C. ¹H NMR (400 MHz, DMSO-*d*₆) δ 8.29 (s, 1H), 7.98 (d, *J* = 10.5 Hz, 1H), 7.87 (d, *J* = 8.8 Hz, 1H), 7.40 (d, *J* = 7.3 Hz, 1H), 7.28 (t, *J* = 7.5 Hz, 1H), 7.14 (dq, *J* = 15.0, 7.4 Hz, 5H), 6.94 (d, *J* = 7.0 Hz, 2H), 4.54 (s, 2H), 3.56 (s, 4H), 3.32 - 3.24 (m, 2H), 2.76 (dt, *J* = 17.5, 4.9 Hz, 4H), 2.59 (s, 3H), 2.52 (d, *J* = 10.3 Hz, 2H), 2.25 (s, 2H), 0.99 (s, 9H). ¹³C NMR (101 MHz, DMSO-*d*₆) δ 170.0, 161.1, 155.5, 151.5, 143.7, 138.7, 135.2, 131.6, 129.9, 129.6, 128.9, 128.8, 128.8, 126.8, 126.7, 125.3, 124.5, 122.0, 120.6, 113.5, 53.3, 53.0, 49.8, 46.8, 44.1, 41.6, 34.5, 31.5, 30.2, 9.6. HRMS (ESI) [M - H] ⁻, theoretically calculated for C₃₅H₄₂N₃O₆S: 632.2789, found 632.2802.

### Example 234

### 3-methyl-5-(N-(2-(4-benzylpiperazin-1-yl)benzyl)-N-phenethylsulfamoyl)benzofuran-2-carboxylic acid (F27-S104)

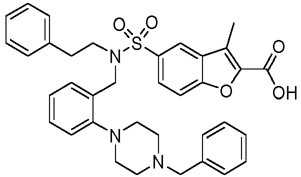

In accordance with the preparation method of 5-(N-benzyl-N-phenethylsulfamoyl)-3-methylbenzofuran-2-carboxylic acid (F27), this example was implemented under the same condition except that M10-104 was used instead of ethyl 5-(N-benzyl-*N*-phenethylsulfamoyl)-3-methylbenzofuran-2-carboxylate (F27-S8). 107 mg of white solid was obtained, with a yield of 82%. Mp 154 - 155 °C. ¹H NMR (400 MHz, DMSO-*d*₆) δ 8.30 (s, 1H), 7.98 (d, *J* = 10.4 Hz, 1H), 7.87 (d, *J* = 8.8 Hz, 1H), 7.59 (d, *J* = 5.7 Hz, 2H), 7.46 - 7.25 (m, 5H), 7.20 - 7.05 (m, 5H), 6.91 (d, *J* = 7.1 Hz, 2H), 4.50 (s, 2H), 4.14 (s, 2H), 3.25 (s, 2H), 3.07 (d, *J* = 23.3 Hz, 8H), 2.59 (s, 3H), 2.51 - 2.43 (m, 2H). ¹³C NMR (101 MHz, DMSO-*d*₆) δ 161.2, 155.5, 150.7, 144.2, 138.7, 135.1, 132.0, 131.6, 131.4, 129.8, 129.7, 129.3, 129.0, 128.9, 128.8, 128.8, 126.7, 126.6, 124.8, 124.8, 121.9, 120.5, 113.5, 59.8, 51.8, 50.2, 50.0, 46.9, 34.6, 9.6. HRMS (ESI) [M - H] ⁻, theoretically calculated for C₃₆H₃₆N₃O₅S: 622.2381, found 622.2374.

### Example 235

### 3-methyl-5-(N-(2-(4-benzoylpiperazin-1-yl)benzyl)-N-phenethylsulfamoyl)benzofuran-2-carboxylic acid (F27-S105)

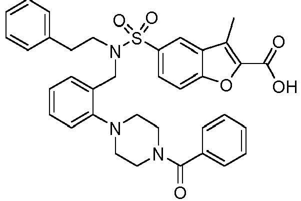

In accordance with the preparation method of 5-(N-benzyl-N-phenethylsulfamoyl)-3-methylbenzofuran-2-carboxylic acid (F27), this example was implemented under the same condition except that M10-105 was used instead of ethyl 5-(N-benzyl-*N*-phenethylsulfamoyl)-3-methylbenzofuran-2-carboxylate (F27-S8). 133 mg of white solid was obtained, with a yield of 78%. Mp 163 - 164 °C. ¹H NMR (400 MHz, DMSO-*d*₆) δ 8.18 (d, *J* = 1.8 Hz, 1H), 7.93 - 7.76 (m, 2H), 7.51 - 7.36 (m, 6H), 7.28 (t, *J* = 8.1 Hz, 1H), 7.14 (dt, *J* = 25.5, 7.5 Hz, 5H), 6.92 (d, *J* = 7.0 Hz, 2H), 4.53 (s, 2H), 3.56 (d, *J* = 124.8 Hz, 4H), 3.30 - 3.20 (m, 2H), 2.82 (s, 4H), 2.59 - 2.47 (m, 5H). ¹³C NMR (101 MHz, DMSO-*d*₆) δ 169.5, 162.8, 155.0, 151.5, 149.0, 138.8, 136.3, 134.3, 131.7, 130.5, 130.0, 129.9, 129.7, 128.9, 128.8, 128.8, 127.4, 126.7, 125.2, 124.5, 121.1, 120.7, 119.9, 113.0, 52.8, 49.9, 46.9, 34.5, 9.5. HRMS (ESI) [M - H] ⁻, theoretically calculated for C₃₆H₃₄N₃O₆S: 636.2174, found 636.2177.

### Example 236

### ethyl 3-methyl-5-(N-(2-(4-(4-methylbenzoyl)piperazin-1-yl)phenyl)-N-phenethylsulfamoyl)benzofuran-2-carboxylate (M10-106)

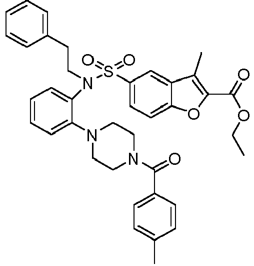

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 4-methylbenzoic acid was used instead of 3,3-dimethyl-1-butanoic acid. 170 mg of white solid was obtained, with a yield of 100%. ¹H NMR (400 MHz, Chloroform-*d*) δ 8.15 (d, *J* = 1.5 Hz, 1H), 7.89 (dd, *J* = 8.8, 1.9 Hz, 1H), 7.64 (d, *J* = 8.8 Hz, 1H), 7.36 (d, *J* = 8.0 Hz, 3H), 7.29 - 7.13 (m, 6H), 7.11 - 7.04 (m, 1H), 7.00 - 6.93 (m, 3H), 4.50 (q, *J* = 7.1 Hz, 2H), 4.24 - 2.51 (m, 15H), 2.40 (s, 3H), 1.48 (t, *J* = 7.1 Hz, 3H). ¹³C NMR (101 MHz, Chloroform-*d*) δ 170.8, 159.9, 155.8, 151.1, 143.0, 139.9, 138.1, 135.9, 134.1, 132.7, 130.3, 130.1, 129.5, 129.4, 129.1, 128.5, 127.3, 126.8, 126.5, 125.5, 124.6, 122.9, 122.1, 112.9, 61.6, 51.5, 34.7, 21.4, 14.4, 9.4.

### Example 237

### Ethyl 3-methyl-5-(N-(2-(4-(2-methylbenzoyl)piperazin-1-yl)phenyl)-N-phenethylsulfamoyl)benzofuran-2-carboxylate (M10-107)

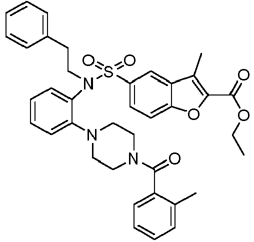

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that O-methylbenzoic acid was used instead of 3,3-dimethyl-1-butanoic acid. 122.2 mg of white solid was obtained, with a yield of 92%. 1H NMR (400 MHz, Chloroform-*d*) δ 8.14 (d, *J* = 1.5 Hz, 1H), 7.88 (dd, *J* = 8.8, 1.8 Hz, 1H), 7.64 (d, *J* = 8.8 Hz, 1H), 7.37 (t, *J* = 7.0 Hz, 1H), 7.32 - 7.13 (m, 8H), 7.08 (t, *J* = 7.6 Hz, 1H), 6.95 (d, *J* = 7.9 Hz, 3H), 4.50 (q, *J* = 7.1 Hz, 2H), 4.31 - 4.02 (m, 2H), 3.88 - 3.61 (m, 2H), 3.45 - 3.16 (m, 4H), 3.00 (s, 1H), 2.81 - 2.48 (m, 6H), 2.36 (s, 3H), 1.48 (t, *J* = 7.1 Hz, 3H). 13C NMR (101 MHz, Chloroform-*d*) δ 170.2, 159.8, 155.8, 151.0, 143.1, 138.1, 136.0, 135.8, 134.1, 132.4, 131.3, 130.4, 130.3, 129.5, 129.4, 128.9, 128.5, 126.8, 126.5, 126.0, 125.9, 125.5, 124.7, 122.8, 122.1, 113.0, 61.6, 53.4, 51.7 (d, *J* = 38.5 Hz), 42.0, 34.6, 19.1, 14.4, 9.4.

### Example 238

### ethyl 3-methyl-5-(N-(2-(4-(3-methylbenzoyl)piperazin-1-yl)phenyl)-N-phenethylsulfamoyl)benzofuran-2-carboxylate (M10-108)

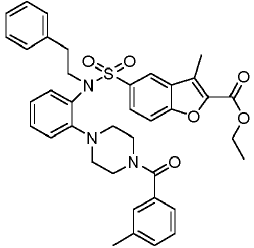

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that M-methyl benzoic acid was used instead of 3,3-dimethyl-1-butanoic acid. 119.3 mg of white solid was obtained, with a yield of 90%. ¹H NMR (400 MHz, Chloroform-*d*) δ 8.15 (d, *J* = 1.6 Hz, 1H), 7.95 - 7.86 (m, 1H), 7.64 (d, *J* = 8.8 Hz, 1H), 7.41 - 7.11 (m, 9H), 7.08 (t, *J* = 8.3 Hz, 1H), 6.96 (d, *J* = 9.5 Hz, 3H), 4.50 (q, *J* = 7.1 Hz, 2H), 4.26 - 4.04 (m, 2H), 3.92 - 3.52 (m, 4H), 3.38 - 3.19 (m, 2H), 3.07 - 2.55 (m, 7H), 2.40 (s, 3H), 1.48 (t, *J* = 7.1 Hz, 3H). ¹³C NMR (101 MHz, Chloroform-*d*) δ 170.8, 159.9, 155.8, 151.1, 143.1, 138.4, 138.1, 135.9, 135.7, 134.1, 130.5, 130.3, 129.5, 129.4, 128.5, 128.3, 127.7, 127.2, 126.8, 126.5, 125.5, 124.6, 124.0, 122.8, 122.1, 113.0, 61.6, 51.5, 34.7, 21.4, 14.4, 9.4.

### Example 239

### Ethyl 5-(N-(2-(4-(4-Acetylaminobenzoyl)piperazin-1-yl)phenyl)-N-phenethylsulfamoyl)-3-methylbenzofuran-2-carboxylate (M10-109)

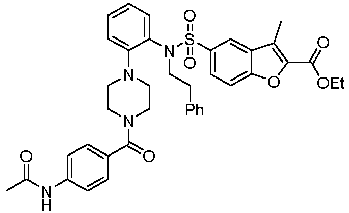

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that paracetamol benzoic acid was used instead of 3, 3-dimethyl-1-butanoic acid. 72.8 mg of white solid was obtained, with a yield of 51%. ¹H NMR (400 MHz, Chloroform-*d*) δ 8.46 (s, 1H), 8.14 (s, 1H), 7.88 (d, *J* = 8.8 Hz, 1H), 7.64 (d, *J* = 8.8 Hz, 1H), 7.52 (d, *J* = 8.3 Hz, 2H), 7.41 - 7.30 (m, 3H), 7.25 - 7.11 (m, 4H), 7.08 (t, *J* = 7.5 Hz, 1H), 6.99 - 6.90 (m, 3H), 4.50 (q, *J* = 7.1 Hz, 2H), 4.16 - 3.96 (m, 2H), 3.88 - 3.52 (m, 4H), 3.35 - 3.19 (m, 2H), 3.07 - 2.66 (m, 3H), 2.64 - 2.50 (m, 1H), 2.15 (s, 3H), 1.47 (t, *J* = 7.1 Hz, 3H). ¹³C NMR (101 MHz, Chloroform-*d*) δ 170.4, 169.0, 159.9, 155.8, 151.1, 143.1, 139.9, 138.0, 135.8, 134.1, 130.6, 130.2, 129.6, 129.4, 128.5, 128.1, 126.8, 126.5, 125.5, 124.7, 122.9, 122.1, 119.6, 113.0, 61.6, 51.5, 34.6, 29.7, 24.4, 14.4, 9.4.

### Example 240

### Ethyl 5-(N-(2-(4-(4-Acetylaminobenzoyl)piperazin-1-yl)phenyl)-N-phenethylsulfamoyl)-3-methylbenzofuran-2-carboxylate (M10-110)

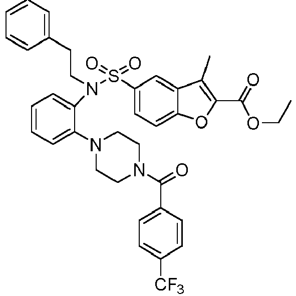

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that paratrifluoromethyl benzoic acid was used instead of 3, 3-dimethyl-1-butanoic acid. 113.1 mg of white solid was obtained, with a yield of 79%. ¹H NMR (400 MHz, Chloroform-*d*) δ 8.14 (d, *J* = 1.4 Hz, 1H), 7.93 - 7.84 (m, 1H), 7.72 (d, *J* = 8.1 Hz, 2H), 7.65 (d, *J* = 8.8 Hz, 1H), 7.57 (d, *J* = 8.1 Hz, 2H), 7.38 (t, *J* = 7.7 Hz, 1H), 7.25 - 7.06 (m, 5H), 6.94 (dd, *J* = 12.0, 7.3 Hz, 3H), 4.50 (q, *J* = 7.1 Hz, 2H), 4.24 - 4.03 (m, 2H), 3.88 - 3.65 (m, 2H), 3.62 - 3.49 (m, 2H), 3.40 - 3.21 (m, 2H), 3.08 - 2.68 (m, 3H), 2.64 - 2.48 (m, 4H), 1.48 (t, *J* = 7.1 Hz, 3H). HRMS (ESI) [M + H] ⁺, theoretically calculated for C₃₈H₃₇F₃N₃O₆S: 720.2350, found 720.2343.

### Example 241

### Ethyl 5-(N-(2-(4-(4-cyanophenyl)piperazin-1-yl)phenyl)-N-phenethylsulfamoyl)-3-methylbenzofuran-2-carboxylate (M10-111)

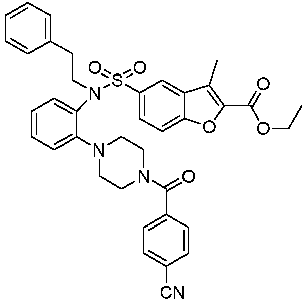

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that para cyanobenzoic acid was used instead of 3,3-dimethyl-1-butanoic acid. 101.0 mg of white solid was obtained, with a yield of 75%. ¹H NMR (400 MHz, Chloroform-*d*) δ 8.13 (d, *J* = 1.6 Hz, 1H), 7.87 (dd, *J* = 8.8, 1.8 Hz, 1H), 7.75 (d, *J* = 8.3 Hz, 2H), 7.65 (d, *J* = 8.8 Hz, 1H), 7.56 (d, *J* = 8.3 Hz, 2H), 7.37 (d, *J* = 8.0 Hz, 1H), 7.25 - 7.13 (m, 4H), 7.09 (t, *J* = 7.6 Hz, 1H), 6.99 - 6.87 (m, 3H), 4.50 (q, *J* = 7.1 Hz, 2H), 4.25 - 4.00 (m, 2H), 3.83 - 3.65 (m, 2H), 3.60 - 3.44 (m, 2H), 3.39 - 3.24 (m, 2H), 3.10 - 2.94 (m, 1H), 2.87 - 2.64 (m, 2H), 2.64 - 2.50 (m, 4H), 1.48 (t, *J* = 7.1 Hz, 3H). ¹³C NMR (101 MHz, Chloroform-*d*) δ 168.4, 159.8, 155.9, 151.0, 143.1, 140.1, 138.0, 135.7, 134.3, 132.5, 130.1, 129.6, 129.4, 128.5, 128.5, 127.8, 126.8, 126.6, 125.5, 124.9, 123.0, 122.1, 118.1, 113.6, 113.0, 61.6, 51.7, 34.6, 14.4, 9.4. HRMS (ESI) [M + H] ⁺, theoretically calculated for C₃₈H₃₇N₄O₆S: 677.2428, found 677.2439.

### Example 242

### Ethyl 5-(N-(2-(4-(4-fluorobenzoyl)piperazin-1-yl)phenyl)-N-phenethylsulfamoyl)-3-methylbenzofuran-2-carboxylate (M10-112)

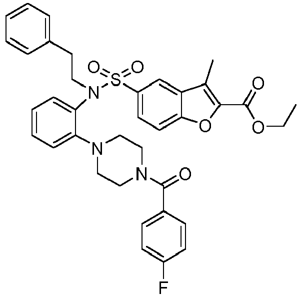

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that P-fluorobenzoic acid was used instead of 3,3-dimethyl-1-butanoic acid. 107.5 mg of white solid was obtained, with a yield of 80%. ¹H NMR (400 MHz, Chloroform-*d*) δ 8.14 (d, *J* = 1.6 Hz, 1H), 7.89 (dd, *J* = 8.8, 1.9 Hz, 1H), 7.65 (d, *J* = 8.8 Hz, 1H), 7.46 (dd, *J* = 8.7, 5.3 Hz, 2H), 7.41 - 7.33 (m, 1H), 7.24 - 7.04 (m, 7H), 6.99 - 6.91 (m, 3H), 4.51 (q, *J* = 7.1 Hz, 2H), 4.19 - 2.48 (m, 15H), 1.48 (t, *J* = 7.1 Hz, 3H). ¹³C NMR (101 MHz, Chloroform-*d*) δ 169.6, 162.2, 159.9, 155.8, 151.0, 143.1, 138.0, 135.8, 134.2, 130.2, 129.5, 129.5, 129.4, 129.4, 128.5 (d, *J* = 1.7 Hz), 126.8, 126.6, 125.5, 124.7, 122.9, 122.1, 115.7, 115.5, 113.0, 61.6, 51.6, 34.7, 14.4, 9.4. HRMS (ESI) [M + H] ⁺, theoretically calculated for C₃₇H₃₇FN₃O₆S: 670.2382, found 670.2396.

### Example 243

### Ethyl -(N-(2-(4-(4-((tert-butoxycarbonyl)amino)benzoyl)piperazin-1-yl)phenyl)-N-phenethylsulfamoyl)-3-methylbenzofuran-2-carboxylate (M10-113)

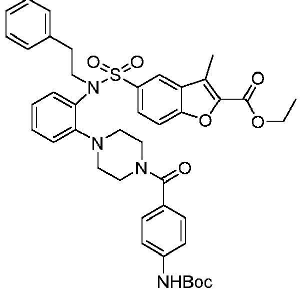

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 4-(N-tert-butoxycarbonyl) aminobenzoic acid was used instead of 3,3-dimethyl-1-butanoic acid. 121.6 mg of white solid was obtained, with a yield of 79%. ¹H NMR (400 MHz, Chloroform-*d*) δ 8.13 (d, *J* = 1.6 Hz, 1H), 7.88 (dd, *J* = 8.8, 1.9 Hz, 1H), 7.62 (d, *J* = 8.8 Hz, 1H), 7.49 - 7.32 (m, 5H), 7.23 - 7.00 (m, 6H), 6.98 - 6.92 (m, 3H), 4.48 (q, *J* = 7.1 Hz, 2H), 4.16 - 2.44 (m, 15H), 1.51 (s, 9H), 1.46 (t, *J* = 7.1 Hz, 3H). ¹³C NMR (101 MHz, Chloroform-*d*) δ 170.3, 159.9, 155.8, 152.6, 151.1, 143.0, 140.2, 138.1, 135.9, 134.1, 130.3, 129.7, 129.5, 129.3, 128.5, 128.4, 126.8, 126.5, 125.5, 124.6, 122.9, 122.1, 118.0, 112.9, 80.8, 61.6, 60.4, 51.5, 34.7, 28.3, 14.4, 14.2, 9.4.

### Example 244

### Ethyl 3-methyl-5-(N-(2-(4-(4-nitrobenzoyl)piperazin-1-yl)phenyl)-N-phenethylsulfamoyl)benzofuran-2-carboxylate (M10-114)

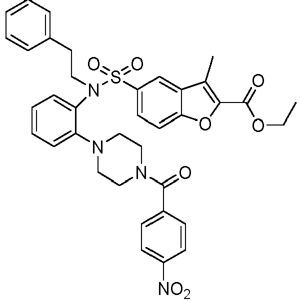

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 4-(N-tert-butoxycarbonyl) aminobenzoic acid was used instead of 3,3-dimethyl-1-butanoic acid. 56.6 mg of white solid was obtained, with a yield of 41%. ¹H NMR (400 MHz, Chloroform-*d*) δ 8.30 (d, *J* = 8.8 Hz, 2H), 8.13 (d, *J* = 1.6 Hz, 1H), 7.87 (dd, *J* = 8.8, 1.9 Hz, 1H), 7.63 (t, *J* = 8.8 Hz, 3H), 7.38 (t, *J* = 8.4 Hz, 1H), 7.27 - 7.04 (m, 5H), 6.99 - 6.84 (m, 3H), 4.49 (q, *J* = 7.1 Hz, 2H), 4.27 - 3.97 (m, 2H), 3.86 - 3.66 (m, 2H), 3.60 - 3.47 (m, 2H), 3.41 - 3.25 (m, 2H), 3.08 - 2.66 (m, 3H), 2.63 - 2.47 (m, 4H), 1.47 (t, *J* = 7.1 Hz, 3H). ¹³C NMR (101 MHz, Chloroform-*d*) δ 168.1, 159.8, 155.8, 151.0, 148.4, 143.1, 142.0, 138.0, 135.7, 134.3, 130.1, 129.6, 129.4, 128.5, 128.5, 128.2, 126.8, 126.6, 125.5, 124.9, 123.9, 123.0, 122.1, 113.0, 61.6, 51.7, 34.6, 14.4, 9.4.

### Example 245

### Ethyl 5-(N-(2-(4-(4-acetylbenzoyl)piperazin-1-yl)phenyl)-N-phenethylsulfamoyl)-3-methylbenzofuran-2-carboxylate (M10-115)

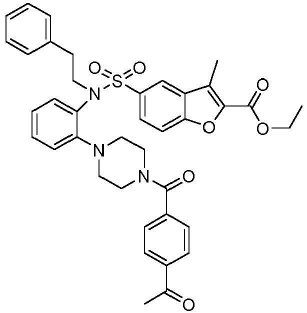

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that Para-acetyl benzoic acid was used instead of 3,3-dimethyl-1-butanoic acid. 84.2 mg of white solid was obtained, with a yield of 61%. ¹H NMR (400 MHz, Chloroform-*d*) δ 8.12 (d, *J* = 1.5 Hz, 1H), 8.01 (d, *J* = 8.3 Hz, 2H), 7.86 (dd, *J* = 8.8, 1.8 Hz, 1H), 7.62 (d, *J* = 8.8 Hz, 1H), 7.52 (d, *J* = 8.3 Hz, 2H), 7.36 (t, *J* = 8.2 Hz, 1H), 7.24 - 7.03 (m, 5H), 6.97 - 6.90 (m, 3H), 4.47 (q, *J* = 7.1 Hz, 2H), 4.24 - 3.97 (m, 2H), 3.90 - 3.63 (m, 2H), 3.59 - 3.45 (m, 2H), 3.41 - 3.18 (m, 2H), 3.05 - 2.64 (m, 3H), 2.63 (s, 3H), 2.61 - 2.50 (m, 4H), 1.45 (t, *J* = 7.1 Hz, 3H). ¹³C NMR (101 MHz, Chloroform-*d*) δ 197.3, 169.3, 159.8, 155.8, 151.0, 143.0, 140.2, 138.0, 137.8, 135.8, 134.2, 130.2, 129.6, 129.3, 1286, 128.5, 128.5, 127.3, 126.8, 126.5, 125.5, 124.8, 122.9, 122.1, 113.0, 61.6, 60.4, 51.6, 34.6, 26.7, 14.4, 14.2, 9.4.

### Example 246

### Ethyl 3-methyl-5-(N-(2-(4-nicotinoylpiperazin-1-yl)phenyl)-N-phenethylsulfamoyl)benzofuran-2-carboxylate (M10-116)

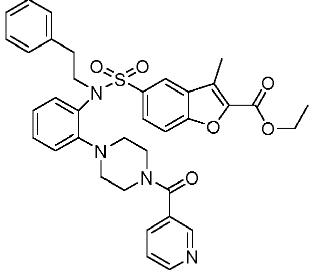

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that nicotinic acid was used instead of 3,3-dimethyl-1-butanoic acid. 71.1 mg of white solid was obtained, with a yield of 55%. ¹H NMR (400 MHz, Chloroform-*d*) δ 8.76 - 8.62 (m, 2H), 8.12 (s, 1H), 7.82 (dd, *J* = 36.0, 8.3 Hz, 2H), 7.63 (d, *J* = 8.8 Hz, 1H), 7.43 - 7.32 (m, 2H), 7.14 (ddd, *J* = 36.9, 18.4, 7.5 Hz, 5H), 6.98 - 6.88 (m, 3H), 4.48 (q, *J* = 7.1 Hz, 2H), 4.23 - 3.97 (m, 2H), 3.86 - 3.49 (m, 4H), 3.40 - 3.20 (m, 2H), 3.07 - 2.65 (m, 3H), 2.64 - 2.50 (m, 4H), 1.46 (t, *J* = 7.1 Hz, 3H). ¹³C NMR (101 MHz, Chloroform-*d*) δ 167.9, 159.8, 155.8, 151.0, 150.8, 148.1, 143.1, 138.0, 135.7, 135.0, 134.2, 131.6, 130.1, 129.6, 129.4, 128.5, 128.5, 126.8, 126.5, 125.5, 124.8, 123.4, 123.0, 122.1, 113.0, 61.6, 51.6, 34.6, 14.4, 9.4.

### Example 247

### ethyl 3-methyl-5-(N-phenethyl-N-(2-(4-pyridineformylpiperazin-1-yl)phenyl)sulfamoyl)benzofuran-2-carboxylate (M10-117)

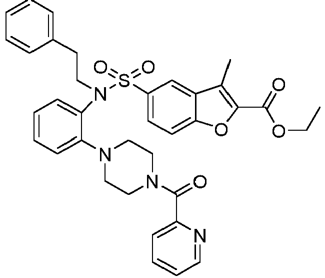

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2-pyridinecarboxylic acid was used instead of 3,3-dimethyl-1-butanoic acid. 69.5 mg of white solid was obtained, with a yield of 53%. ¹H NMR (400 MHz, Chloroform-*d*) δ 8.61 (d, *J* = 4.6 Hz, 1H), 8.15 (s, 1H), 7.89 (d, *J* = 10.5 Hz, 1H), 7.82 (t, *J* = 8.5 Hz, 1H), 7.70 - 7.59 (m, 2H), 7.36 (t, *J* = 6.8 Hz, 2H), 7.27 - 7.11 (m, 4H), 7.07 (t, *J* = 7.1 Hz, 1H), 6.97 (t, *J* = 8.7 Hz, 3H), 4.49 (q, *J* = 7.1 Hz, 2H), 4.22 - 4.04 (m, 2H), 3.92 - 3.61 (m, 4H), 3.40 - 3.21 (m, 2H), 3.07 - 2.66 (m, 3H), 2.64 - 2.50 (m, 4H), 1.47 (t, *J* = 7.1 Hz, 3H). ¹³C NMR (101 MHz, Chloroform-*d*) δ 167.7, 159.9, 155.8, 154.1, 151.0, 148.4, 143.0, 138.1, 137.1, 136.0, 134.0, 130.4, 129.5, 129.3, 128.5, 126.8, 126.5, 125.6, 124.6, 124.5, 123.8, 122.8, 122.1, 112.9, 61.6, 53.1, 51.5, 47.8, 34.7, 14.4, 9.4.

### Example 248

### Ethyl 3-methyl-5-(N-phenethyl-N-(2-(4-(thiophene-2-carbonyl)piperazin-1-yl)phenyl)sulfamoyl)benzofuran-2-carboxylate(M10-118)

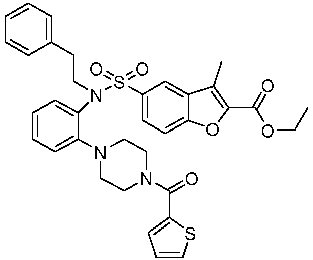

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2-thiophenecarboxylic acid was used instead of 3,3-dimethyl-1-butanoic acid. 28.4 mg of white solid was obtained, with a yield of 21%. ¹H NMR (400 MHz, Chloroform-*d*) δ 8.16 (s, 1H), 7.90 (dd, *J* = 8.8, 1.8 Hz, 1H), 7.65 (d, *J* = 8.8 Hz, 1H), 7.47 (dd, *J=* 5.0, 1.2 Hz, 1H), 7.37 (t, *J* = 8.3 Hz, 1H), 7.32 (d, *J* = 3.5 Hz, 1H), 7.25 - 7.13 (m, 4H), 7.13 - 7.04 (m, 2H), 6.97 (d, *J* = 5.9 Hz, 3H), 4.50 (q, *J* = 7.1 Hz, 2H), 4.21 - 3.68 (m, 6H), 3.41 - 3.18 (m, 2H), 3.01 - 2.67 (m, 3H), 2.67 - 2.49 (m, 4H), 1.48 (t, *J=* 7.1 Hz, 3H). ¹³C NMR (101 MHz, Chloroform-*d*) δ 163.7, 159.9, 155.8, 151.0, 138.1, 137.0, 135.9, 134.1, 130.3, 129.5, 129.4, 128.9, 128.6, 128.5, 126.8, 126.7, 126.5, 125.5, 124.7, 122.9, 122.1, 113.0, 61.6, 51.5, 34.7, 14.4, 9.4.

### Example 249

### Ethyl 3-methyl-5-(N-phenethyl-N-(2-(4-(thiophene-3-carbonyl)piperazin-1-yl)phenyl)sulfamoyl)benzofuran-2-carboxylate (M10-119)

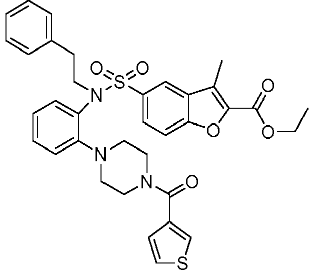

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 3-thiophenecarboxylic acid was used instead of 3,3-dimethyl-1-butanoic acid. 69.7 mg of white solid was obtained, with a yield of 53%. ¹³C NMR (101 MHz, Chloroform-*d*) δ 165.8, 159.8, 155.8, 151.0, 143.1, 138.1, 136.3, 135.9, 134.1, 130.3, 129.5, 129.4, 128.5, 127.1, 126.8, 126.5, 126.0, 125.5, 124.6, 122.9, 122.1, 113.0, 61.6, 51.5, 34.7, 14.4, 9.4.

### Example 250

### (E)ethyl-5-(N-(2-(4-(but-2-enoyl)piperazin-1-yl)phenyl)-N-phenethylsulfamoyl)-3-methylbenzofuran-2-carboxylate (M10-120)

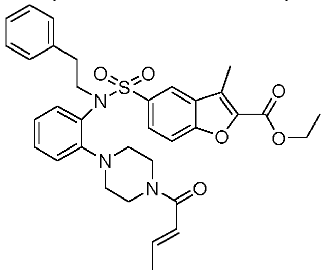

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that Trans-2-butenoic acid was used instead of 3,3-dimethyl-1-butanoic acid. 67.3 mg of white solid was obtained, with a yield of 55%. ¹H NMR (400 MHz, Chloroform-*d*) δ 8.16 (s, 1H), 7.90 (dd, *J* = 8.8, 1.7 Hz, 1H), 7.65 (d, *J* = 8.8 Hz, 1H), 7.36 (t, *J* = 8.3 Hz, 1H), 7.22 - 7.12 (m, 4H), 7.08 (t, *J* = 7.6 Hz, 1H), 7.00 - 6.93 (m, 3H), 6.93 - 6.86 (m, 1H), 6.33 - 6.25 (m, 1H), 4.50 (q, *J* = 7.1 Hz, 2H), 4.22 - 4.00 (m, 2H), 3.89 - 3.29 (m, 5H), 3.20 - 3.08 (m, 1H), 2.96 - 2.66 (m, 3H), 2.66 - 2.49 (m, 4H), 1.91 (d, *J* = 8.0 Hz, 3H), 1.48 (t, *J* = 7.1 Hz, 3H). ¹³C NMR (101 MHz, Chloroform-*d*) δ 165.8, 159.9, 155.8, 151.1, 143.1, 141.7, 138.1, 136.0, 134.1, 130.3, 129.5, 129.4, 128.5, 126.8, 126.5, 125.6, 124.6, 122.8, 122.1, 121.5, 113.0, 61.6, 51.5, 34.7, 18.3, 14.4, 9.4. HRMS (ESI) [M + H] ⁺, theoretically calculated for C₃₄H₃₈N₃O₆S: 616.2476, found 616.2479.

### Example 251

### Ethyl 5-(N-(2-(4-(furan-2-carbonyl)piperazin-1-yl)phenyl)-N-phenethylsulfamoyl)-3-methylbenzofuran-2-carboxylate (M10-121)

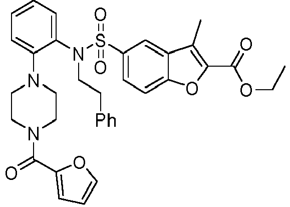

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2-furancarboxylic acid was used instead of 3,3-dimethyl-1-butanoic acid. 106.2 mg of white solid was obtained, with a yield of 83%. ¹H NMR (400 MHz, Chloroform-*d*) δ 8.16 (s, 1H), 7.90 (d, *J* = 8.0 Hz, 1H), 7.65 (d, *J* = 8.2 Hz, 1H), 7.51 (s, 1H), 7.43 - 7.31 (m, 1H), 7.24 - 6.88 (m, 9H), 6.50 (s, 1H), 4.50 (q, *J* = 7.3 Hz, 2H), 4.22 - 3.67 (m, 6H), 3.41 - 3.22 (m, 2H), 3.08 - 2.84 (m, 2H), 2.78 - 2.42 (m, 5H), 1.47 (t, *J* = 7.2 Hz, 3H). ¹³C NMR (101 MHz, Chloroform-*d*) δ 159.8, 159.3, 155.8, 151.0, 147.9, 143.7, 143.1, 138.1, 136.0, 134.0, 130.4, 129.5, 129.4, 128.5, 126.8, 126.5, 125.5, 124.6, 122.8, 122.1, 116.4, 113.0, 111.3, 61.6, 51.5, 34.7, 14.4, 9.4.

### Example 252

### Ethyl 5-(N-(2-(4-(cyclopentanecarbonyl)piperazin-1-yl)phenyl)-N-phenethylsulfamoyl)-3-methylbenzofuran-2-carboxylate (M10-122)

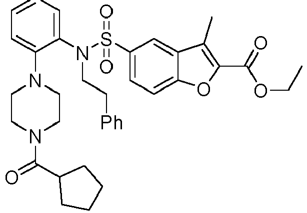

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that cyclopentylcarboxylic acid was used instead of 3,3-dimethyl-1-butanoic acid. 104.5 mg of yellow liquid product was obtained, with a yield of 81%. ¹H NMR (400 MHz, Chloroform-*d*) δ 8.14 (d, *J* = 1.6 Hz, 1H), 7.88 (dd, *J* = 8.8, 1.9 Hz, 1H), 7.62 (d, *J* = 8.8 Hz, 1H), 7.33 (td, *J* = 7.7, 1.6 Hz, 1H), 7.22 -7.09 (m, 4H), 7.06 (td, *J* = 7.6, 1.5 Hz, 1H), 6.94 (ddd, *J* = 8.8, 5.5, 1.5 Hz, 2H), 4.47 (q, *J* = 7.1 Hz, 2H), 4.16 - 3.95 (m, 2H), 3.62 - 3.52 (m, 2H), 3.46 - 3.33 (m, 2H), 3.15 - 3.02 (m, 1H), 2.95 - 2.64 (m, 4H), 2.62 - 2.48 (m, 4H), 1.95 - 1.65 (m, 8H), 1.45 (t, *J* = 7.1 Hz, 3H). ¹³C NMR (101 MHz, Chloroform-*d*) δ 174.8, 159.9, 155.8, 151.0, 143.0, 138.1, 136.0, 134.0, 130.3, 129.4, 129.3, 128.4, 128.4, 126.8, 126.4, 125.5, 124.5, 122.7, 122.1, 112.9, 61.6, 58.2, 51.4, 41.1, 34.6, 30.0, 26.0, 18.3, 14.4, 9.4.

### Example 253

### Ethyl 5-(N-(2-(4-(5-bromothiophene-2-carbonyl)piperazin-1-yl)phenyl)-N-phenethylsulfamoyl)-3-methylbenzofuran-2-carboxylate (M10-123)

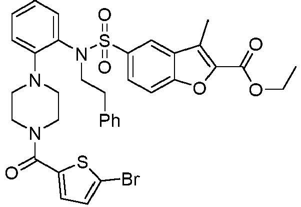

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 5-bromo-2-thiophenecarboxylic acid was used instead of 3,3-dimethyl-1-butanoic acid. 104.7 mg of white solid was obtained, with a yield of 71%. ¹H NMR (400 MHz, Chloroform-*d*) δ 8.16 (d, *J* = 1.5 Hz, 1H), 7.90 (d, *J* = 10.5 Hz, 1H), 7.66 (d, *J* = 8.8 Hz, 1H), 7.38 (t, *J* = 7.7 Hz, 1H), 7.20 (dd, *J* = 10.4, 7.8 Hz, 4H), 7.09 (d, *J* = 4.0 Hz, 2H), 7.04 (d, *J* = 3.9 Hz, 1H), 6.96 (t, *J* = 8.8 Hz, 3H), 4.51 (q, *J* = 7.1 Hz, 2H), 4.20 - 3.69 (m, 6H), 3.39 - 3.23 (m, 2H), 3.03 - 2.83 (m, 2H), 2.83 - 2.46 (m, 5H), 1.49 (t, *J* = 7.1 Hz, 3H). ¹³C NMR (101 MHz, Chloroform-*d*) δ 162.4, 159.9, 155.9, 151.0, 143.1, 138.8, 138.0, 135.8, 134.2, 130.2, 129.7, 129.6, 129.4, 128.5, 128.5, 126.8, 126.6, 125.5, 124.8, 122.9, 122.1, 116.7, 113.0, 61.6, 51.6, 34.7, 14.4, 9.4.

### Example 254

### Ethyl 5-(N-(2-(4-(1H-pyrrole-2-carbonyl)piperazin-1-yl)phenyl)-N-phenethylsulfamoyl)-3-methylbenzofuran-2-carboxylate (M10-124)

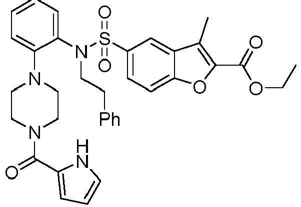

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2-pyrrolecarboxylic acid was used instead of 3,3-dimethyl-1-butanoic acid. 125.2 mg of white solid was obtained, with a yield of 98%. ¹H NMR (400 MHz, Chloroform-*d*) δ 9.62 (s, 1H), 8.18 (s, 1H), 7.92 (d, *J* = 10.3 Hz, 1H), 7.66 (d, *J* = 8.8 Hz, 1H), 7.37 (t, *J* = 8.0 Hz, 2H), 7.25 - 7.14 (m, 4H), 7.09 (t, *J* = 7.3 Hz, 1H), 7.02 - 6.93 (m, 4H), 6.54 (s, 1H), 6.32 - 6.23 (m, 1H), 4.51 (q, *J* = 7.1 Hz, 2H), 4.25 - 3.76 (m, 6H), 3.38 - 3.20 (m, 2H), 2.99 - 2.87 (m, 2H), 2.80 - 2.52 (m, 5H), 1.49 (t, *J* = 7.1 Hz, 3H). ¹³C NMR (101 MHz, Chloroform-*d*) δ 161.8, 159.9, 155.8, 151.1, 143.1, 138.14, 136.0, 134.1, 130.4, 129.5, 129.4, 128.5, 128.5, 126.8, 126.5, 125.6, 124.6, 122.8, 122.1, 121.0, 113.0, 112.2, 110.0, 109.6, 61.6, 52.5, 51.5, 34.7, 14.4, 9.4.

### Example 255

### ethyl 5-(N-(2-(4-(3-bromothiophene-2-carbonyl)piperazin-1-yl)phenyl)-N-phenethylsulfamoyl)-3-methylbenzofuran-2-carboxylate (M10-125)

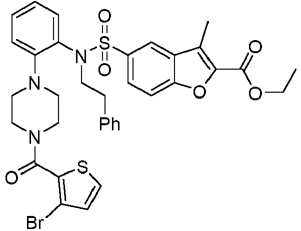

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 3-bromo-2-thiophenecarboxylic acid was used instead of 3,3-dimethyl-1-butanoic acid. 97.5 mg of white solid was obtained, with a yield of 66%. ¹H NMR (400 MHz, Chloroform-*d*) δ 8.15 (d, *J* = 1.5 Hz, 1H), 7.89 (dd, *J* = 8.8, 1.8 Hz, 1H), 7.65 (d, *J* = 8.8 Hz, 1H), 7.38 (q, *J* = 5.4, 4.4 Hz, 2H), 7.25 - 7.14 (m, 4H), 7.10 (t, *J* = 7.6 Hz, 1H), 7.02 - 6.92 (m, 4H), 4.51 (q, *J* = 7.1 Hz, 2H), 4.21 - 3.99 (m, 2H), 3.89 - 3.22 (m, 6H), 3.01 - 2.85 (m, 2H), 2.81 - 2.51 (m, 5H), 1.49 (t, *J* = 7.1 Hz, 3H). ¹³C NMR (101 MHz, Chloroform-*d*) δ 162.2, 159.9, 155.8, 151.0, 143.1, 138.1, 135.9, 134.1, 132.0, 130.3, 130.2, 129.5, 129.4, 128.5, 127.3, 126.8, 126.6, 125.5, 124.7, 122.9, 122.1, 113.0, 109.7, 61.6, 51.5, 34.7, 14.4, 9.4.

### Example 256

### Ethyl 5-(N-(2-(4-(3-chlorothiophene-2-carbonyl)piperazin-1-yl)phenyl)-N-phenethylsulfamoyl)-3-methylbenzofuran-2-carboxylate (M10-126)

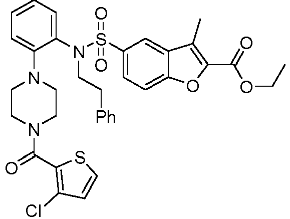

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 3-chloro-2-thiophenecarboxylic acid was used instead of 3,3-dimethyl-1-butanoic acid. 97.5 mg of white solid was obtained, with a yield of 71%. ¹H NMR (400 MHz, Chloroform-*d*) δ 8.16 (d, *J* = 1.6 Hz, 1H), 7.89 (dd, *J* = 8.8, 1.9 Hz, 1H), 7.65 (d, *J* = 8.8 Hz, 1H), 7.43 - 7.33 (m, 2H), 7.26 - 7.13 (m, 4H), 7.09 (d, *J* = 7.7 Hz, 1H), 7.01 - 6.91 (m, 4H), 4.51 (q, *J* = 7.1 Hz, 2H), 4.21 - 4.03 (m, 2H), 3.91 - 3.51 (m, 4H), 3.41 - 3.23 (m, 2H), 2.99 - 2.83 (m, 2H), 2.78 - 2.52 (m, 5H), 1.49 (t, *J* = 7.1 Hz, 3H). ¹³C NMR (101 MHz, Chloroform-*d*) δ 161.7, 159.9, 155.8, 151.0, 143.1, 138.1, 135.9, 134.1, 130.3, 130.1, 129.5, 129.4, 128.5, 127.7, 126.8, 126.6, 125.5, 124.7, 124.0, 122.9, 122.1, 113.0, 61.6, 51.5, 34.7, 14.4, 9.4.

### Example 257

### Ethyl 5-(N-(2-(4-(4-bromothiophene-2-carbonyl)piperazin-1-yl)phenyl)-N-phenethylsulfamoyl)-3-methylbenzofuran-2-carboxylate (M10-127)

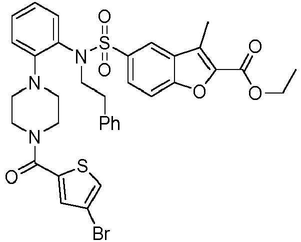

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 4-bromo-2-thiophenecarboxylic acid was used instead of 3,3-dimethyl-1-butanoic acid. 96.6 mg of white solid was obtained, with a yield of 66%. ¹H NMR (400 MHz, Chloroform-*d*) δ 8.16 (d, *J* = 1.5 Hz, 1H), 7.90 (dd, *J* = 8.8, 1.8 Hz, 1H), 7.66 (d, *J* = 8.8 Hz, 1H), 7.42 - 7.34 (m, 1H), 7.26 - 7.14 (m, 5H), 7.12 (s, 1H), 6.96 (d, *J* = 23.0 Hz, 3H), 4.51 (q, *J* = 7.1 Hz, 2H), 4.18 - 3.68 (m, 6H), 3.40 - 3.26 (m, 2H), 3.00 - 2.70 (m, 3H), 2.65 - 2.53 (m, 4H), 1.49 (t, *J* = 7.1 Hz, 3H). ¹³C NMR (101 MHz, Chloroform-*d*) δ 162.2, 159.9, 155.9, 151.1, 143.1, 138.4, 138.0, 135.8, 134.2, 131.0, 130.1, 129.6, 129.4, 128.5, 128.5, 126.8, 126.6, 126.1, 125.5, 124.7, 122.9, 122.2, 113.0, 109.4, 61.6, 51.6, 34.6, 14.4, 9.4.

### Example 258

### Ethyl 5-(N-(2-(4-(5-chlorothiophene-2-carbonyl)piperazin-1-yl)phenyl)-N-phenethylsulfamoyl)-3-methylbenzofuran-2-carboxylate (M10-128)

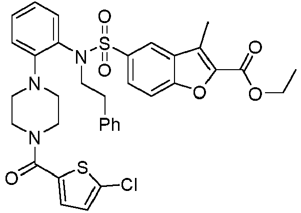

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 5-chloro-2-thiophenecarboxylic acid was used instead of 3,3-dimethyl-1-butanoic acid. 101.5 mg of white solid was obtained, with a yield of 74%. ¹H NMR (400 MHz, Chloroform-*d*) δ 8.16 (d, *J* = 1.6 Hz, 1H), 7.90 (dd, *J* = 8.8, 1.8 Hz, 1H), 7.66 (d, *J* = 8.8 Hz, 1H), 7.38 (t, *J* = 8.3 Hz, 1H), 7.26 - 7.14 (m, 2H), 7.15 - 7.05 (m, 4H), 6.96 (t, *J* = 8.7 Hz, 3H), 6.90 (d, *J* = 3.9 Hz, 1H), 4.51 (q, *J* = 7.1 Hz, 2H), 4.20 - 3.68 (m, 6H), 3.41 - 3.23 (m, 2H), 3.00 - 2.85 (m, 2H), 2.77 - 2.54 (m, 5H), 1.49 (t, *J* = 7.1 Hz, 3H). ¹³C NMR (101 MHz, Chloroform-*d*) δ 162.4, 159.9, 155.9, 151.0, 143.1, 138.0, 135.9, 135.8, 134.2, 134.1, 130.2, 129.6, 129.4, 128.6, 128.5, 128.5, 126.8, 126.6, 126.0, 125.5, 124.8, 122.9, 122.1, 113.0, 61.6, 51.6, 34.7, 14.4, 9.4.

### Example 259

### Ethyl 3-methyl-5-(N-phenethyl-N-(2-(4-(thiazole-5-carbonyl)piperazin-1-yl)phenyl)sulfamoyl)benzofuran-2-carboxylate (M10-129)

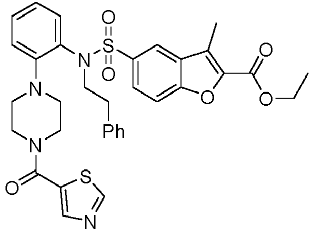

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that thiazole-5-carboxylic acid was used instead of 3,3-dimethyl-1-butanoic acid. 115.4 mg of white solid was obtained, with a yield of 84%. ¹H NMR (400 MHz, Chloroform-*d*) δ 8.91 (s, 1H), 8.15 (d, *J* = 1.6 Hz, 1H), 8.10 (s, 1H), 7.89 (dd, *J* = 8.8, 1.9 Hz, 1H), 7.66 (d, *J* = 8.8 Hz, 1H), 7.38 (t, *J* = 8.4 Hz, 1H), 7.27 - 7.14 (m, 4H), 7.10 (t, *J* = 7.6 Hz, 1H), 7.02 - 6.89 (m, 3H), 4.51 (q, *J* = 7.1 Hz, 2H), 4.16 - 3.69 (m, 6H), 3.42 - 3.27 (m, 2H), 3.05 - 2.68 (m, 3H), 2.66 - 2.52 (m, 4H), 1.48 (t, *J* = 7.1 Hz, 3H). ¹³C NMR (101 MHz, Chloroform-*d*) δ 161.4, 159.9, 155.9, 155.1, 151.0, 143.8, 143.1, 138.0, 135.7, 134.3, 130.1, 129.6, 129.4, 128.5, 128.5, 126.8, 126.6, 125.5, 124.9, 123.0, 122.1, 113.0, 61.6, 51.7, 34.6, 14.4, 9.4.

### Example 260

### Ethyl 5-(N-(2-(4-(1H-pyrazole-3-carbonyl)piperazin-1-yl)phenyl)-N-phenethylsulfamoyl)-3-methylbenzofuran-2-carboxylate (M10-130)

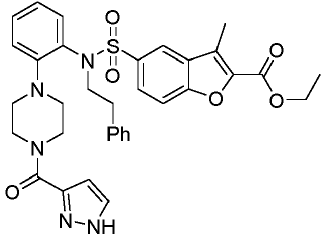

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that pyrazole-3-carboxylic acid was used instead of 3,3-dimethyl-1-butanoic acid. 84.3 mg of white solid was obtained, with a yield of 66%. ¹H NMR (400 MHz, Chloroform-*d*) δ 8.16 (s, 1H), 7.90 (d, *J* = 8.7 Hz, 1H), 7.67 - 7.57 (m, 2H), 7.35 (t, *J* = 7.4 Hz, 1H), 7.24 - 6.92 (m, 8H), 6.69 (s, 1H), 4.50 (q, *J* = 7.1 Hz, 2H), 4.24 - 3.55 (m, 6H), 3.39 - 3.15 (m, 2H), 3.04 - 2.52 (m, 7H), 1.47 (t, *J* = 7.1 Hz, 3H). ¹³C NMR (101 MHz, Chloroform-*d*) δ 162.7, 160.0, 155.8, 150.9, 143.0, 138.1, 136.0, 133.9, 130.5, 129.5, 129.4, 128.5, 128.5, 126.8, 126.5, 125.6, 124.5, 122.7, 122.1, 113.0, 107.5, 61.7, 51.4, 34.7, 14.4, 9.4.

### Example 261

### Ethyl 3-methyl-5-(N-(2-(4-(oxazole-5-carbonyl)piperazin-1-yl)phenyl)-N-phenethylsulfamoyl)benzofuran-2-carboxylate (M10-131)

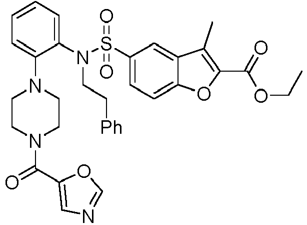

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that oxazole-5-carboxylic acid was used instead of 3,3-dimethyl-1-butanoic acid. 68.6 mg of white solid was obtained, with a yield of 54%. ¹H NMR (400 MHz, Chloroform-*d*) δ 8.15 (s, 1H), 7.97 (s, 1H), 7.89 (dd, *J* = 8.8, 1.6 Hz, 1H), 7.68 - 7.57 (m, 2H), 7.36 (t, *J* = 7.6 Hz, 1H), 7.25 - 7.03 (m, 5H), 6.95 (t, *J* = 7.4 Hz, 3H), 4.49 (q, *J* = 7.1 Hz, 3H), 4.16 - 3.67 (m, 6H), 3.52 - 3.16 (m, 2H), 3.01 - 2.84 (m, 2H), 2.78 - 2.52 (m, 5H), 1.47 (t, *J* = 7.1 Hz, 3H). ¹³C NMR (101 MHz, Chloroform-*d*) δ 159.8, 157.4, 155.8, 151.5, 151.0, 145.2, 143.1, 138.02, 135.8, 134.2, 131.6, 130.2, 129.6, 129.4, 128.5, 1285, 126.8, 126.6, 125.5, 124.8, 122.9, 122.1, 113.0, 61.6, 51.6, 34.6, 14.4, 9.4.

### Example 262

### Ethyl 5-(N-(2-(4-(3,4-Dimethylbenzoyl)piperazin-1-yl)phenyl)-N-phenethylsulfamoyl) 3-methylbenzofuran-2-carboxylate (M10-132)

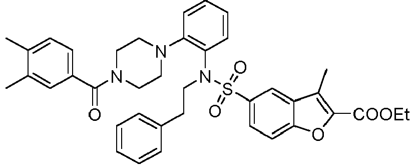

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 3,5-dimethylbenzoic acid was used instead of 3,3-dimethyl-1-butanoic acid. 82.4 mg of white solid was obtained, with a yield of 30%. ¹H NMR (400 MHz, Chloroform-*d*) δ 8.13 (d, *J* = 1.9 Hz, 1H), 7.87 (dd, *J* = 8.8, 1.9 Hz, 1H), 7.62 (d, *J* = 8.8 Hz, 1H), 7.34 (td, *J* = 7.6, 1.6 Hz, 1H), 7.23 - 7.02 (m, 8H), 6.98 - 6.85 (m, 3H), 4.47 (q, *J* = 7.1 Hz, 2H), 4.23 - 3.11 (m, 8H), 3.07 - 2.59 (m, 1H), 2.58 (s, 3H), 2.28 (d, *J* = 1.9 Hz, 6H), 1.45 (t, *J* = 7.1 Hz, 3H). ¹³C NMR (101 MHz, CDCl₃) δ 170.8, 159.8, 155.8, 151.1, 143.1, 138.5, 138.1, 136.9, 136.0, 134.1, 133.2, 130.3, 129.5, 129.5, 129.4, 128.5, 128.4, 126.8, 126.5, 125.5, 124.6, 122.8, 122.1, 113.0, 61.6, 51.5, 34.7, 19.8, 19.7, 14.4, 9.4. HRMS (ESI) [M + H] ⁺, theoretically calculated for C₃₉H₄₂N₃O₆S: 680.2794, found 680.2798.

### Example 263

### Ethyl 5-(N-(2-(4-(2,6-Dimethoxybenzoyl)piperazin-1-yl)phenyl)-N-phenethylsulfamoyl)3-methylbenzofuran-2-carboxylate (M10-133)

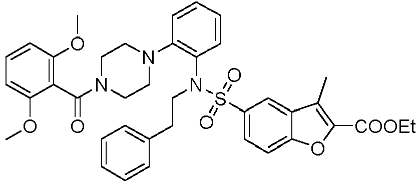

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2,6-dimethoxybenzoic acid was used instead of 3,3-dimethyl-1-butanoic acid. 58.7 mg of white solid was obtained, with a yield of 22%. ¹H NMR (400 MHz, Chloroform-*d*) δ 8.13 (d, *J* = 1.9 Hz, 1H), 7.88 (dd, *J* = 8.8, 1.9 Hz, 1H), 7.62 (d, *J* = 8.8 Hz, 1H), 7.43 - 6.86 (m, 10H), 6.56 (d, *J* = 8.4 Hz, 2H), 4.48 (q, *J* = 7.1 Hz, 2H), 4.34 - 4.01 (m, 2H), 3.82 (s, 8H), 3.44 - 3.11 (m, 4H), 2.58 (s, 7H), 1.46 (t, *J* = 7.1 Hz, 3H). ¹³C NMR (101 MHz, CDCl₃) δ 165.5, 159.9, 156.7, 155.9, 151.1, 143.1, 138.2, 136.1, 133.8, 130.5, 130.4, 129.5, 129.4, 128.6, 128.5, 126.9, 126.6, 125.6, 124.4, 122.6, 122.1, 114.3, 113.0, 104.0, 61.6, 55.9, 53.4, 51.6, 51.3, 47.2, 41.9, 34.7, 14.4, 9.4. HRMS (ESI) [M + H] ⁺, theoretically calculated for C₃₉H₄₂N₃O₈S: 712.2693, found 712.2689.

### Example 264

### Ethyl 5-(N-(2-(4-(2-benzylbenzoyl)piperazin-1-yl)phenyl)-N-phenethylsulfamoyl)3-methylbenzofuran-2-carboxylate (M10-134)

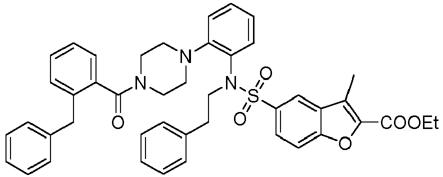

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2-benzylbenzoic acid was used instead of 3,3-dimethyl-1-butanoic acid. 57.5 mg of white solid was obtained, with a yield of 19%. ¹H NMR (400 MHz, Chloroform-*d*) δ 8.12 (s, 1H), 7.86 (dd, *J* = 8.8, 1.9 Hz, 1H), 7.62 (d, *J* = 8.8 Hz, 1H), 7.43 - 6.82 (m, 18H), 4.48 (q, *J* = 7.1 Hz, 2H), 4.28 - 3.89 (m, 4H), 3.84 - 3.41 (m, 2H), 3.26 - 2.31 (m, 10H), 1.46 (t, *J* = 7.1 Hz, 3H). ¹³C NMR (101 MHz, Chloroform-*d*) δ 169.9, 159.9, 155.9, 143.1, 140.4, 138.4, 138.2, 136.1, 136.1, 131.0, 130.4, 129.4, 129.2, 128.6, 128.5, 126.8, 126.6, 126.5, 126.4, 126.3, 125.6, 122.1, 113.0, 61.7, 52.4, 51.5, 47.3, 41.8, 39.2, 34.8, 14.5, 9.5. HRMS (ESI) [M + H] ⁺, theoretically calculated for C₄₄H₄₄N₃O₆S: 742.2951, found 742.2938.

### Example 265

### Ethyl 5-(N-(2-(4-(2,4,6-trimethylbenzoyl)piperazin-1-yl)phenyl)-N-phenethylsulfamoyl)3-methylbenzofuran-2-carboxylate (M10-135)

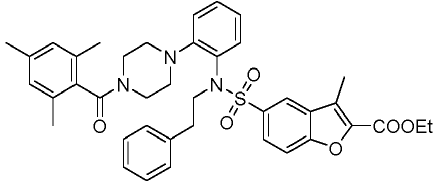

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2,4,6-trimethylbenzoic acid was used instead of 3,3-dimethyl-1-butanoic acid. 21.2 mg of white solid was obtained, with a yield of 25%. ¹H NMR (400 MHz, Chloroform-*d*) δ 8.12 (d, *J* = 1.9 Hz, 1H), 7.86 (dd, *J* = 8.8, 1.9 Hz, 1H), 7.62 (d, *J* = 8.8 Hz, 1H), 7.35 (td, *J* = 7.7, 1.6 Hz, 1H), 7.23 - 7.02 (m, 5H), 6.96 - 6.90 (m, 3H), 6.85 (s, 2H), 4.48 (q, *J* = 7.1 Hz, 2H), 4.34 - 3.53 (m, 4H), 3.39 - 3.09 (m, 4H), 2.58 (s, 7H), 2.26 (d, *J* = 9.8 Hz, 9H), 1.46 (t, *J* = 7.1 Hz, 3H). ¹³C NMR (101 MHz, CDCl₃) δ 170.0, 159.9, 155.9, 151.2, 143.1, 138.2, 138.1, 135.9, 134.1, 133.6, 133.2, 130.3, 129.6, 129.4, 128.6, 128.4, 126.9, 126.6, 125.6, 124.7, 122.8, 122.2, 113.0, 61.7, 53.6, 52.0, 51.6, 46.7, 41.7, 34.7, 21.1, 19.2, 14.4, 9.4. HRMS (ESI) [M + H] ⁺, theoretically calculated for C₄₀H₄₄N₃O₆S: 694.2951, found 694.2955.

### Example 266

### Ethyl 5-(N-(2-(4-(2-methoxybenzoyl)piperazin-1-yl)phenyl)-N-phenethylsulfamoyl)3-methylbenzofuran-2-carboxylate (M10-136)

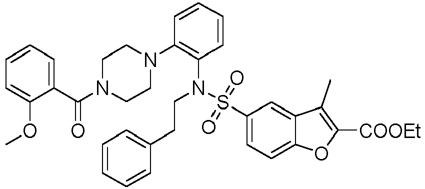

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2-methoxybenzoic acid was used instead of 3,3-dimethyl-1-butanoic acid. 76.5 mg of white solid was obtained, with a yield of 56%. ¹H NMR (400 MHz, Chloroform-*d*) δ 8.13 (d, *J* = 1.9 Hz, 1H), 7.88 (dd, *J* = 8.8, 1.9 Hz, 1H), 7.62 (d, *J* = 8.8 Hz, 1H), 7.35 (td, *J* = 8.0, 1.7 Hz, 2H), 7.29 - 7.22 (m, 1H), 7.22 - 7.10 (m, 4H), 7.06 (td, *J* = 7.6, 1.4 Hz, 1H), 7.01 - 6.88 (m, 5H), 4.48 (q, *J* = 7.1 Hz, 2H), 4.25 - 4.00 (m, 2H), 3.94 - 3.51 (m, 5H), 3.44 - 3.10 (m, 4H), 3.07 - 2.46 (m, 7H), 1.46 (t, *J* = 7.1 Hz, 3H). ¹³C NMR (101 MHz, CDCl₃) δ 167.9, 159.8, 155.8, 155.3, 151.1, 143.1, 138.1, 136.0, 133.9, 130.5, 130.4, 129.5, 129.4, 128.5, 128.5, 128.0, 126.8, 126.5, 125.7, 125.5, 124.5, 122.7, 122.1, 120.9, 112.9, 111.0, 61.6, 55.6, 53.2, 51.7, 51.4, 47.5, 42.1, 34.7, 14.4, 9.4. HRMS (ESI) [M + H] ⁺, theoretically calculated for C₃₈H₄₀N₃O₇S: 682.2587, found 682.2577.

### Example 267

### Ethyl 5-(N-(2-(4-(2,5-Dimethylbenzoyl)piperazin-1-yl)phenyl)-N-phenethylsulfamoyl)3-methylbenzofuran-2-carboxylate (M10-137)

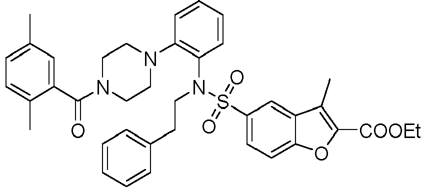

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2,5-dimethylbenzoic acid was used instead of 3,3-dimethyl-1-butanoic acid. 28.2 mg of white solid was obtained, with a yield of 21%. ¹H NMR (400 MHz, Chloroform-*d*) δ 8.12 (d, *J* = 1.9 Hz, 1H), 7.86 (dd, *J* = 8.8, 1.9 Hz, 1H), 7.62 (d, *J* = 8.8 Hz, 1H), 7.35 (td, *J* = 7.7, 1.6 Hz, 1H), 7.23 - 7.02 (m, 7H), 6.99 (s, 1H), 6.93 (dd, *J* = 7.8*,* 1.7 Hz, 3H), 4.48 (q, *J* = 7.1 Hz, 2H), 4.34 - 3.51 (m, 4H), 3.48 - 3.07 (m, 4H), 3.05 - 2.41 (m, 7H), 2.30 (d, *J* = 14.5 Hz, 6H), 1.46 (t, *J* = 7.1 Hz, 3H). ¹³C NMR (101 MHz, CDCl₃) δ 170.5, 160.0, 156.0, 151.2, 143.2, 138.2, 136.1, 136.0, 135.7, 134.2, 130.9, 130.4, 130.4, 129.8, 129.6, 129.5, 128.6, 126.9, 126.7, 126.5, 125.6, 124.7, 122.9, 122.2, 113.1, 61.7, 53.5, 52.0, 51.6, 42.1, 34.7, 21.0, 18.7, 14.5, 9.5. HRMS (ESI) [M + H] ⁺, theoretically calculated for C₃₉H₄₂N₃O₆S: 680.2794, found 680.2795.

### Example 268

### Ethyl 5-(N-(2-(4-(4-(phenylethynyl)benzoyl)piperazin-1-yl)phenyl)-N-phenethylsulfamoyl)3-methylbenzofuran-2-carboxylate (M10-138)

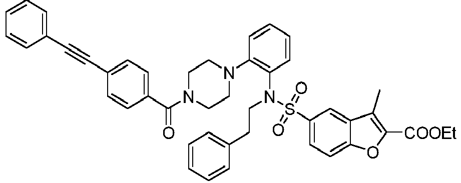

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 4-(phenylethynyl) benzoic acid was used instead of 3,3-dimethyl-1-butanoic acid. 128.3 mg of yellow solid was obtained, with a yield of 85%. ¹H NMR (400 MHz, Chloroform-*d*) δ 8.13 (d, *J* = 1.9 Hz, 1H), 7.87 (dd, *J* = 8.8, 1.9 Hz, 1H), 7.64 - 7.51 (m, 5H), 7.43 (d, *J* = 8.1 Hz, 2H), 7.38 - 7.28 (m, 4H), 7.23 - 7.03 (m, 5H), 7.00 - 6.85 (m, 3H), 4.47 (q, *J* = 7.1 Hz, 2H), 4.26 - 3.94 (m, 2H), 3.87 - 3.46 (m, 4H), 3.27 (s, 2H), 2.59 (s, 7H), 1.45 (t, *J* = 7.1 Hz, 3H). ¹³C NMR (101 MHz, CDCl₃) δ 169.8, 159.8, 155.8, 151.1, 143.1, 138.1, 135.8, 135.3, 134.2, 131.7, 131.7, 130.2, 129.6, 129.4, 128.6, 128.5, 128.4, 127.3, 126.8, 126.6, 125.5, 124.9, 124.8, 123.0, 122.9, 122.1, 113.0, 90.9, 88.7, 61.6, 51.6, 34.7, 14.4, 9.4. HRMS (ESI) [M + H] ⁺, theoretically calculated for C₄₅H₄₂N₃O₆S: 752.2794, found 752.2789.

### Example 269

### Ethyl 5-(N-(2-(4-(5-methylthiophene-2-formyl)piperazin-1-yl)phenyl)-N-phenethylsulfamoyl)3-methylbenzofuran-2-carboxylate (M10-139)

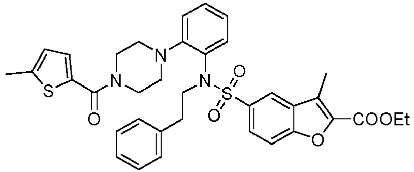

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 5-methyl-2-thiophenecarboxylic acid was used instead of 3,3-dimethyl-1-butanoic acid. 93.9 mg of yellow solid was obtained, with a yield of 23%. ¹H NMR (400 MHz, Chloroform-d) δ 8.13 (d, *J* = 1.9 Hz, 1H), 7.87 (dd, *J* = 8.8, 1.9 Hz, 1H), 7.62 (d, *J* = 8.8 Hz, 1H), 7.34 (td, *J* = 7.6, 1.6 Hz, 1H), 7.22 - 7.02 (m, 6H), 6.95 (ddd, *J* = 7.6, 5.7, 1.6 Hz, 3H), 6.69 (dd, *J* = 3.7, 1.3 Hz, 1H), 4.47 (q, *J* = 7.1 Hz, 2H), 4.20 - 3.60 (m, 6H), 3.21 (d, *J* = 45.4 Hz, 2H), 2.99 - 2.51 (m, 7H), 2.49 (s, 3H), 1.45 (t, *J* = 7.1 Hz, 3H). ¹³C NMR (101 MHz, CDCl₃) δ 163.7, 159.8, 155.8, 151.0, 143.9, 143.1, 138.1, 136.0, 134.6, 134.1, 130.3, 129.5, 129.5, 129.4, 128.5, 126.8, 126.5, 125.5, 125.2, 125.1, 124.6, 122.9, 122.1, 113.0, 61.6, 52.5, 51.5, 34.7, 15.3, 14.4, 9.4. HRMS (ESI) [M + H] ⁺, theoretically calculated for C₃₆H₃₈N₃O₆S₂: 672.2202, found 672.2194.

### Example 270

### Ethyl 5-(N-(2-(4-(4-methylthiophene-2-formyl)piperazin-1-yl)phenyl)-N-phenethylsulfamoyl)3-methylbenzofuran-2-carboxylate (M10-140)

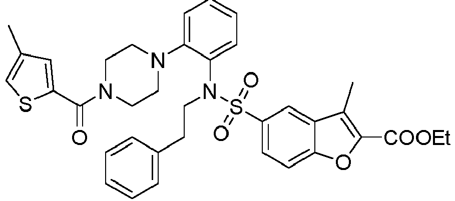

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 4-methyl-2-thiophenecarboxylic acid was used instead of 3,3-dimethyl-1-butanoic acid. 80.4 mg of yellow solid was obtained, with a yield of 20%. ¹H NMR (400 MHz, Chloroform-*d*) δ 8.14 (d, *J* = 1.9 Hz, 1H), 7.88 (dd, *J* = 8.7, 1.9 Hz, 1H), 7.63 (d, *J* = 8.8 Hz, 1H), 7.35 (td, *J* = 7.7, 1.6 Hz, 1H), 7.22 - 7.00 (m, 7H), 6.94 (dd, *J* = 7.7, 1.6 Hz, 3H), 4.47 (q, *J* = 7.1 Hz, 2H), 4.27 - 3.57 (m, 6H), 3.29 (s, 2H), 3.03 - 2.44 (m, 7H), 2.27 (s, 3H), 1.45 (t, *J* = 7.1 Hz, 3H). ¹³C NMR (101 MHz, CDCl₃) δ 163.9, 159.9, 155.9, 151.1, 143.1, 138.1, 137.4, 136.7, 136.0, 134.1, 131.2, 130.3, 129.6, 129.4, 128.6, 126.9, 126.6, 125.6, 124.7, 124.2, 122.9, 122.2, 113.0, 61.6, 52.5, 51.6, 34.7, 15.6, 14.4, 9.4. HRMS (ESI) [M + H] ⁺, theoretically calculated for C₃₆H₃₈N₃O₆S₂: 672.2202, found 672.2191.

### Example 271

### Ethyl 5-(N-(2-(4-(3-methylthiophene-2-formyl)piperazin-1-yl)phenyl)-N-phenethylsulfamoyl)3-methylbenzofuran-2-carboxylate (M10-141)

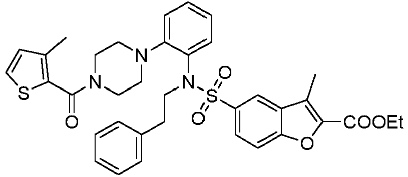

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 3-methyl-2-thiophenecarboxylic acid was used instead of 3,3-dimethyl-1-butanoic acid. 119.5 mg of yellow solid was obtained, with a yield of 30%. ¹H NMR (400 MHz, Chloroform-*d*) δ 8.13 (d, *J* = 1.8 Hz, 1H), 7.87 (dd, *J* = 8.7, 1.9 Hz, 1H), 7.62 (d, *J* = 8.8 Hz, 1H), 7.35 (td, *J* = 7.7, 1.6 Hz, 1H), 7.27 (d, *J* = 4.9 Hz, 1H), 7.22 - 7.10 (m, 4H), 7.07 (td, *J* = 7.6, 1.5 Hz, 1H), 6.95 (ddd, *J* = 7.8, 4.6, 1.6 Hz, 3H), 6.84 (d, *J* = 5.0 Hz, 1H), 4.48 (q, *J* = 7.1 Hz, 2H), 4.21 - 3.51 (m, 6H), 3.26 (s, 2H), 2.59 (s, 7H), 2.28 (s, 3H), 1.46 (t, *J* = 7.1 Hz, 3H).¹³C NMR (101 MHz, CDCl₃) δ 164.8, 159.9, 155.9, 151.0, 143.1, 138.1, 137.3, 135.9, 134.1, 130.3, 130.3, 129.8, 129.5, 129.4, 128.5, 126.8, 126.6, 126.0, 125.5, 124.7, 122.9, 122.1, 113.0, 61.6, 52.6, 51.5, 34.7, 14.7, 14.4, 9.4. HRMS (ESI) [M + H] ⁺, theoretically calculated for C₃₆H₃₈N₃O₆S₂: 672.2202, found 672.2195.

### Example 272

### Ethyl 5-(N-(2-(4-(1H-imidazole-2-formyl)piperazin-1-yl)phenyl)-N-phenethylsulfamoyl)3-methylbenzofuran-2-carboxylate (M10-142)

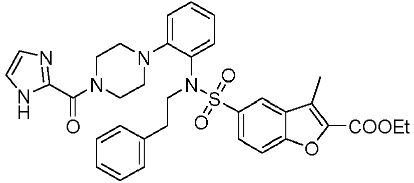

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 1H-imidazole-2-carboxylic acid was used instead of 3,3-dimethyl-1-butanoic acid. 50.5 mg of white solid was obtained, with a yield of 13%. ¹H NMR (400 MHz, Chloroform-*d*) δ 11.8 (s, 1H), 8.2 (d, *J* = 1.9 Hz, 1H), 7.9 (dd, *J* = 8.8, 1.9 Hz, 1H), 7.6 (d, *J* = 8.8 Hz, 1H), 7.3 (td, *J* = 7.6, 1.7 Hz, 1H), 7.2 -7.1 (m, 6H), 7.1 (td, *J* = 7.6, 1.7 Hz, 1H), 7.0 - 6.9 (m, 3H), 4.8 (d, *J* = 141.8 Hz, 2H), 4.5 (q, *J* = 7.1 Hz, 2H), 4.2 - 3.7 (m, 4H), 3.5 - 2.6 (m, 9H), 1.5 (t, *J* = 7.1 Hz, 3H). ¹³C NMR (101 MHz, CDCl₃) δ 160.0, 158.1, 155.9, 151.1, 143.1, 141.4, 138.3, 136.1, 134.2, 130.5, 129.8, 129.6, 129.5, 128.6, 128.6, 127.0, 126.6, 125.7, 124.6, 122.9, 122.2, 118.5, 113.0, 61.7, 52.9, 52.5, 51.6, 47.2, 43.5, 34.9, 14.5, 9.5. HRMS (ESI) [M + H] ⁺, theoretically calculated for C₃₄H₃₆N₅O₆S: 642.2386, found 642.2388.

### Example 273

### Ethyl 5-(N-(2-(4-(tetrahydrothiophene-2-formyl)piperazin-1-yl)phenyl)-N-phenethylsulfamoyl)3-methylbenzofuran-2-carboxylate (M10-143)

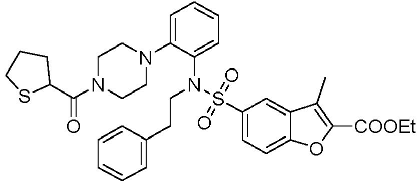

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that tetrahydrothiophene-2-carboxylic acid was used instead of 3,3-dimethyl-1-butanoic acid. 113.5 mg of white solid was obtained, with a yield of 28%. ¹H NMR (400 MHz, Chloroform-*d*) δ 8.15 (d, *J* = 1.9 Hz, 1H), 7.88 (dd, *J* = 8.8, 1.9 Hz, 1H), 7.63 (d, *J* = 8.8 Hz, 1H), 7.34 (td, *J* = 7.6, 1.7 Hz, 1H), 7.23 - 7.03 (m, 5H), 6.97 (dd, *J* = 17.6, 7.4 Hz, 3H), 4.48 (q, *J* = 7.1 Hz, 2H), 4.16 - 3.73 (m, 4H), 3.71 - 3.24 (m, 4H), 3.12 (s, 1H), 3.05 - 2.75 (m, 4H), 2.74 - 2.42 (m, 6H), 2.30 - 2.21 (m, 1H), 2.10 - 1.87 (m, 2H), 1.46 (t, *J* = 7.1 Hz, 3H). ¹³C NMR (101 MHz, CDCl₃) δ 170.8, 159.8, 155.7, 150.9, 142.9, 138.0, 135.8, 133.9, 130.3, 129.4, 129.3, 128.4, 128.4, 126.7, 126.4, 125.5, 124.6, 122.8, 122.0, 112.9, 61.5, 52.7, 51.6, 51.3, 46.3, 45.1, 42.5, 34.6, 33.4, 33.0, 31.3, 14.3, 9.4. HRMS (ESI) [M + H] ⁺, theoretically calculated for C₃₅H₄₀N₃O₆S₂: 662.2359, found 662.2357.

### Example 274

### Ethyl 5-(N-(2-(4-(1H-imidazole-4-carbonyl)piperazin-1-yl)phenyl)-N-phenethylsulfamoyl)3-methylbenzofuran-2-carboxylate (M10-144)

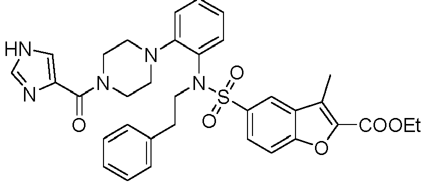

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 1H-imidazole-4-carboxylic acid was used instead of 3,3-dimethyl-1-butanoic acid. 118.5 mg of white solid was obtained, with a yield of 31%. ¹H NMR (400 MHz, Chloroform-*d*) δ 11.55 (s, 1H), 8.13 (d, *J* = 1.9 Hz, 1H), 7.87 (dd, *J* = 8.8, 1.9 Hz, 1H), 7.65 - 7.57 (m, 2H), 7.46 (s, 1H), 7.32 (t, *J* = 7.7 Hz, 1H), 7.21 - 6.82 (m, 8H), 4.45 (q, *J* = 7.1 Hz, 2H), 4.35 - 3.59 (m, 6H), 3.30 (s, 2H), 2.91 (d, *J* = 10.6 Hz, 2H), 2.76 - 2.42 (m, 5H), 1.43 (t, *J* = 7.1 Hz, 3H). ¹³C NMR (101 MHz, CDCl₃) δ 163.4, 159.9, 155.8, 151.0, 143.0, 138.1, 135.9, 135.5, 133.9, 130.3, 129.5, 129.3, 128.5, 128.5, 126.8, 126.5, 125.6, 124.5, 122.8, 122.1, 112.9, 61.6, 51.4, 34.6, 29.6, 14.3, 9.4. HRMS (ESI) [M + H] ⁺, theoretically calculated for C₃₄H₃₆N₅O₆S: 642.2386, found 642.2394.

### Example 275

### Ethyl 5-(N-(2-(4-(2-ethylbutyryl)piperazin-1-yl)phenyl)-N-phenethylsulfamoyl)3-methylbenzofuran-2-carboxylate (M10-145)

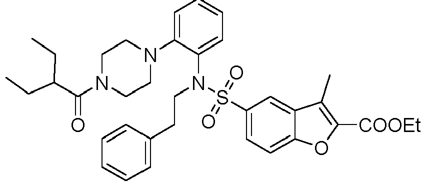

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that 2-ethylbutyric acid was used instead of 3,3-dimethyl-1-butanoic acid. 138.2 mg of white solid was obtained, with a yield of 31%. ¹H NMR (400 MHz, Chloroform-*d*) δ 8.16 (s, 1H), 7.89 (d, *J* = 8.8 Hz, 1H), 7.64 (d, *J* = 8.8 Hz, 1H), 7.35 (t, *J* = 7.7 Hz, 1H), 7.23 - 7.03 (m, 5H), 6.95 (t, *J* = 8.2 Hz, 3H), 4.48 (q, *J* = 7.1 Hz, 2H), 4.11 (dd, *J* = 13.4, 7.0 Hz, 2H), 3.92 - 3.33 (m, 5H), 3.22 - 2.48 (m, 9H), 1.86 - 1.39 (m, 7H), 0.89 (t, *J* = 7.4 Hz, 6H). ¹³C NMR (101 MHz, CDCl₃) δ 174.5, 159.8, 155.8, 151.0, 143.1, 138.1, 136.0, 134.0, 130.4, 129.5, 129.4, 128.5, 128.5, 126.8, 126.5, 125.5, 124.6, 122.7, 122.1, 113.0, 61.6, 53.5, 52.1, 51.4, 46.3, 44.0, 42.2, 34.6, 25.6, 14.4, 12.1, 9.4. HRMS (ESI) [M + H] ⁺, theoretically calculated for C₃₆H₄₄N₃O₆S: 646.2951, found 646.2940.

### Example 276

### Ethyl 5-(N-(2-(4-(isoxazole-5-carbonyl)piperazin-1-yl)phenyl)-N-phenethylsulfamoyl)3-methylbenzofuran-2-carboxylate (M10-146)

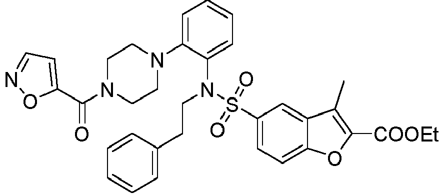

In accordance with the preparation method of ethyl 3-methyl-5-(N-(2-(4-(4-(3,3-dimethylbutyryl)piperazin-1-yl)phenyl)-N-phenethylsulfamoyl)benzofuran-2-carboxylate (M10-94), this example was implemented under the same condition except that isoxazole-5-carboxylic acid was used instead of 3,3-dimethyl-1-butanoic acid. 42.1 mg of white solid was obtained, with a yield of 21%.

### Example 277

### ethyl 5-(N-(2-(4-(3-bromothiophene-2-carboxamido)piperidin-1-yl)phenyl)-N-phenethylsulfamoyl)-3-methylbenzofuran-2-carboxylate (M10-147)

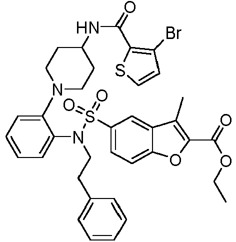

P-3-bromothiophene-2-carboxylic acid (223 mg, 1.078 mmol), EDCI (207 mg, 1.078 mmol), HOBt (146 mg, 1.078 mmol), and DIPEA (1.47 mmol, 242 µL) were dissolved in 4 mL of DCM and stirred at room temperature. When it was found from the monitoring of the TLC that there was no raw material carboxylic acid left, ethyl 5-(N-(2-(4-aminopiperidin-1-yl)phenyl)-N-phenethylsulfamoyl)-3-methylbenzofuran-2-carboxylate (275 mg, 0.49 mmol) was added to the system. The reaction was performed overnight at room temperature. After the reaction was found to be completed from the monitoring of the TLC, the product was dissolved in 20 mL of water and then extracted three times with 20 mL of ethyl acetate. The organic phases were combined and then concentrated under reduced pressure and then separated by column chromatography (PE : EtOAc = 3 : 1) to obtain 366.5 mg of white solid, with a yield of 99%. ¹H NMR (400 MHz, Chloroform-*d*) δ 8.16 (s, 1H), 7.90 (dd, *J* = 8.8, 1.9 Hz, 1H), 7.62 (d, *J* = 8.8 Hz, 1H), 7.45 (d, *J* = 5.3 Hz, 2H), 7.34 (ddd, *J* = 8.8, 7.0, 1.9 Hz, 1H), 7.28 - 7.10 (m, 5H), 7.08 - 6.92 (m, 6H), 4.48 (q, *J* = 7.1 Hz, 2H), 4.19 - 2.95 (m, 6H), 2.80 - 2.48 (m, 6H), 2.28 - 1.57 (m, 4H), 1.46 (t, *J* = 7.1 Hz, 3H). ¹³C NMR (101 MHz, Chloroform-*d*) δ 159.9, 159.7, 155.8, 151.7, 143.0, 138.3, 136.2, 135.1, 133.8, 132.0, 130.6, 130.1, 129.3, 128.6, 128.5, 126.8, 126.5, 125.6, 124.2, 122.9, 122.1, 112.9, 110.0, 108.4, 61.6, 51.3, 47.0, 34.8, 14.4, 9.4.

### Example 278

### Ethyl 5-(N-(2-(3-bromothiophene-2-carbonyl)-2,8-diazospiro[4. 5]decan-8-yl)phenyl)-N-phenethylsulfamoyl)-3-methylbenzofuran-2-carboxylate (M10-148)

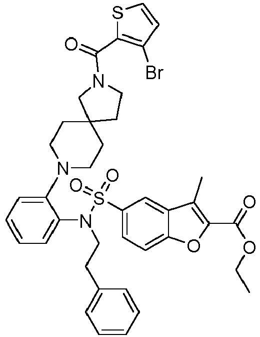

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 5-(N-(2,8-diazospiro[4.5]decan-8-yl)phenyl)-N-phenethylsulfamoyl)-3-methylbenzofuran-2-carboxylatewas used instead of ethyl 5-(N-(2-(4-aminopiperidin-1-yl)phenyl)-N-phenethylsulfamoyl)-3-methylbenzofuran-2-carboxylate. 98.0 mg of white solid was obtained, with a yield of 57%. ¹H NMR (400 MHz, Chloroform-*d*) δ 8.17 (s, 1H), 7.91 (t, *J* = 8.1 Hz, 1H), 7.63 (d, *J* = 8.6 Hz, 1H), 7.35 (s, 2H), 7.25 - 7.10 (m, 4H), 7.08 - 6.86 (m, 4H), 4.49 (q, *J* = 6.9 Hz, 2H), 3.87 (s, 1H), 3.74 (s, 1H), 3.64 - 3.45 (m, 2H), 3.32 (s, 1H), 3.28 - 3.00 (m, 2H), 2.96 - 2.48 (m, 8H), 1.97 - 1.80 (m, 2H), 1.80 - 1.57 (m, 4H), 1.47 (t, *J* = 7.0 Hz, 3H). ¹³C NMR (101 MHz, Chloroform-*d*) δ 162.0, 159.9, 155. 8, 151.8, 143.0, 138.3, 136.2, 133.7, 133.3, 130.5, 130.3, 129.3, 128.5, 128.4, 126.9, 126.8, 126.4, 125.6, 124.0, 122.6, 122.5, 122.1, 112.9, 109.4, 61.6, 58.2, 55.3, 51.1, 50.0, 46.5, 44.5, 39.4, 34.9, 34.7, 21.1, 14.4, 9.4.

### Example 279

### Ethyl 5-(N-(2-(7-(3-bromothiophene-2-carbonyl)-2,7-diazaspiro[3.5]nonan-2-yl)phenyl)-N-phenethylsulfamoyl)-3-methylbenzofuran-2-carboxylate(M10-149)

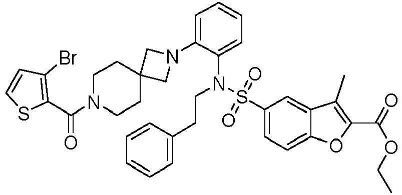

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 5-(N-(2,7-diazaspiro[3.5]nonan-2-yl)phenyl)-N-phenethylsulfamoyl)-3-methylbenzofuran-2-carboxylate was used instead of ethyl 5-(N-(2-(4-aminopiperidin-1-yl)phenyl)-N-phenethylsulfamoyl)-3-methylbenzofuran-2-carboxylate. 220.2 mg of white solid was obtained, with a yield of 59%. ¹H NMR (400 MHz, Chloroform-*d*) δ 8.04 (s, 1H), 7.83 (d, *J* = 8.6 Hz, 1H), 7.64 (d, *J* = 8.6 Hz, 1H), 7.44 - 7.04 (m, 7H), 6.99 (s, 1H), 6.51 (s, 2H), 6.34 (d, *J* = 7.6 Hz, 1H), 4.49 (q, *J* = 6.7 Hz, 2H), 4.09 - 3.26 (m, 10H), 2.93 - 2.76 (m, 1H), 2.76 - 2.62 (m, 1H), 2.00 - 1.81 (m, 4H), 1.47 (t, *J* = 6.9 Hz, 3H). ¹³C NMR (101 MHz, Chloroform-*d*) δ 162.1, 159.9, 156.0, 148.7, 143.0, 138.0, 134.0, 132.3, 130.2, 129.3, 129.3, 128.7, 128.6, 128.5, 127.4, 127.0, 126.7, 125.6, 123.8, 122.7, 116.9, 113.9, 112.7, 109.6, 62.4, 61.6, 52.9, 34.7, 34.2, 14.4, 9.4.

### Example 280

### Ethyl 5-(N-(2-(3-bromothiophene-2-carbonyl)-2,7-diazaspiro[3.5]nonan-7-yl)phenyl)-N-phenethylsulfamoyl)-3-methylbenzofuran-2-carboxylate (M10-150)

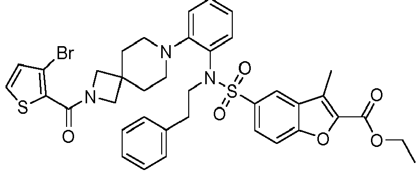

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 5-(N-(2,7-diazospiro[3.5]nonan-7-yl)phenyl)-N-phenethylsulfamoyl)-3-methylbenzofuran-2-carboxylate was used instead of ethyl 5-(N-(2-(4-aminopiperidin-1-yl)phenyl)-N-phenethylsulfamoyl)-3-methylbenzofuran-2-carboxylate. 539.7 mg of white solid was obtained, with a yield of 68%. ¹H NMR (400 MHz, Chloroform-*d*) δ 8.15 (d, *J* = 1.9 Hz, 1H), 7.89 (dd, *J* = 8.7, 1.9 Hz, 1H), 7.62 (d, *J* = 8.8 Hz, 1H), 7.38 (d, *J* = 5.2 Hz, 1H), 7.36 - 7.28 (m, 1H), 7.21 - 7.09 (m, 4H), 7.09 - 6.96 (m, 3H), 6.97 - 6.90 (m, 2H), 4.49 (q, *J* = 7.1 Hz, 2H), 4.15 - 3.74 (m, 7H), 3.12 (s, 2H), 2.84 - 2.47 (m, 6H), 1.89 (d, *J* = 21.8 Hz, 4H), 1.47 (t, *J* = 7.1 Hz, 3H). ¹³C NMR (101 MHz, Chloroform-*d*) δ 163.2, 159.9, 155.8, 151.6, 143.0, 138.3, 136.2, 133.8, 131.4, 130.5, 130.1, 129.3, 129.3, 128.5, 128.5, 128.1, 126.8, 126.5, 125.6, 124.2, 122.7, 122.1, 112.9, 111.8, 61.6, 51.2, 49.8, 36.0, 34.7, 33.9, 14.4, 14.2, 9.4.

### Example 281

### Ethyl 5-(N-(2-(6-(3-bromothiophene-2-carbonyl)-3,6-diazabicyclo[3.1.1]heptan-3-yl)phenyl)-N-phenethylsulfamoyl)-3-methylbenzofuran-2-carboxylate (M10-151)

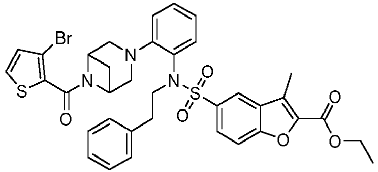

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 5-(N-(2-(3,6-diazabicyclo[3.1.1]heptan-3-yl)phenyl)-N-phenethylsulfamoyl)-3-methylbenzofuran-2-carboxylate was used instead of ethyl 5-(N-(2-(4-aminopiperidin-1-yl)phenyl)-N-phenethylsulfamoyl)-3-methylbenzofuran-2-carboxylate. 291 mg of white solid was obtained, with a yield of 30%. ¹H NMR (400 MHz, Chloroform-*d*) δ 7.94 (s, 1H), 7.73 (d, *J* = 8.8 Hz, 1H), 7.59 (d, *J* = 8.8 Hz, 1H), 7.42 - 7.10 (m, 6H), 7.02 (d, *J* = 19.6 Hz, 3H), 6.78 (s, 1H), 6.55 (s, 1H), 4.65 (s, 2H), 4.47 (q, *J* = 7.1 Hz, 2H), 4.12 - 3.90 (m, 2H), 3.74 - 3.30 (m, 3H), 2.91 - 2.48 (m, 6H), 1.53 - 1.34 (m, 5H). ¹³C NMR (101 MHz, Chloroform-*d*) δ 162.8, 159.8, 155.9, 149.2, 143.0, 137.9, 131.5, 131.1, 129.5, 129.2, 129.0, 128.6, 128.6, 128.1, 127.3, 126.6, 125.6, 122.6, 121.7, 120.9, 112.6, 111.5, 61.6, 52.4, 49.9, 34.3, 26.9, 14.4, 9.3.

### Example 282

### (S)-ethyl-5-(N-(2-(4-(3-bromothiophene-2-carbonyl)-2-methylpiperazin-1-yl)phenyl)-N-phenethylsulfamoyl)-3-methylbenzofuran-2-carboxylate (M10-152)

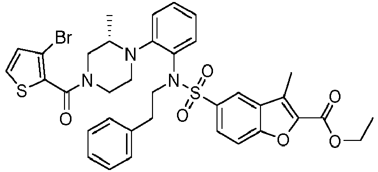

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that (S)ethyl-3-methyl-5-(N-(2-(2-methylpiperazin-1-yl)phenyl)-N-phenethylsulfamoyl)benzofuran-2-carboxylate was used instead of ethyl 5-(N-(2-(4-aminopiperidin-1-yl)phenyl)-N-phenethylsulfamoyl)-3-methylbenzofuran-2-carboxylate. 398 mg of white solid was obtained, with a yield of 73%.

### Example 283

### (R)-ethyl-5-(N-(2-(4-(3-bromothiophene-2-carbonyl)-2-methylpiperazin-1-yl) phenyl)-N-phenethylsulfamoyl)-3-methylbenzofuran-2-carboxylate (M10-153)

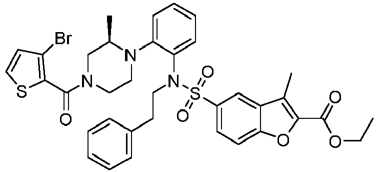

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that (R)ethyl-3-methyl-5-(N-(2-(2-methylpiperazin-1-yl)phenyl)-N-phenethylsulfamoyl)benzofuran-2-carboxylate was used instead of ethyl 5-(N-(2-(4-aminopiperidin-1-yl)phenyl)-N-phenethylsulfamoyl)-3-methylbenzofuran-2-carboxylate. 354 mg of white solid was obtained, with a yield of 73%.

### Example 284

### Ethyl 5-(N-(2-(4-(3-bromothiophene-2-formyl)-1,4-diazepin-1-yl)phenyl)-N-phenethylsulfamoyl)-3-methylbenzofuran-2-carboxylate (M10-154)

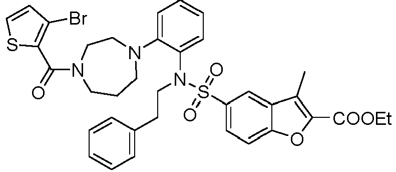

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 5-(N-(2-(1,4-diazepan-1-yl)phenyl)-N-phenethylsulfamoyl)-3-methylbenzofuran-2-carboxylatewas used instead of ethyl 5-(N-(2-(4-aminopiperidin-1-yl)phenyl)-N-phenethylsulfamoyl)-3-methylbenzofuran-2-carboxylate. 727.1 mg of white solid was obtained, with a yield of 53%. ¹H NMR (400 MHz, DMSO-*d*₆) δ 8.23 (d, *J* = 26.6 Hz, 1H), 7.88 (d, *J* = 22.2 Hz, 2H), 7.72 (d, *J* = 16.7 Hz, 1H), 7.41 - 7.05 (m, 6H), 7.03 - 6.81 (m, 4H), 4.39 (q, *J* = 7.1 Hz, 2H), 4.17 - 2.93 (m, 12H), 2.58 (s, 3H), 2.09 - 1.63 (m, 2H), 1.36 (t, *J* = 7.1 Hz, 3H).¹³C NMR (101 MHz, DMSO) δ 162.3, 159.1, 155.2, 151.4, 142.3, 138.0, 135.3, 132.5, 132.0, 130.0, 129.7, 129.1, 128.9, 128.5, 128.3, 127.0, 126.2, 125.7, 122.8, 122.1, 113.2, 108.4, 61.2, 54.8, 50.5, 49.9, 46.7, 44.3, 33.7, 27.3, 14.1, 9.1. HRMS (ESI) [M + H] ⁺, theoretically calculated for C₃₆H₃₇N₃O₆S₂Br: 750.1307, found 750.1295.

### Example 285

### Ethyl 5-(N-(2-(3-(3-bromothiophene-2-formyl)-3,8-diazepine[3.2.1]cyclooctan-8-yl)phenyl)-N-phenethylsulfamoyl)3-methylbenzofuran-2-carboxylate (M10-155)

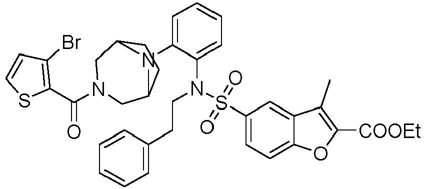

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 5-(N-(2-(3,8-diazabicyclo[3.2.1]oct-8-yl)phenyl)-N-phenethylsulfamoyl)-3-methylbenzofuran-2-carboxylatewas used instead of ethyl 5-(N-(2-(4-aminopiperidin-1-yl)phenyl)-N-phenethylsulfamoyl)-3-methylbenzofuran-2-carboxylate. 149.0 mg of white solid was obtained, with a yield of 43.4%. ¹H NMR (400 MHz, Chloroform-*d*) δ 8.16 (s, 1H), 7.90 (t, *J* = 8.9 Hz, 1H), 7.65 (d, *J* = 8.8 Hz, 1H), 7.35 (d, *J* = 5.2 Hz, 1H), 7.21 (ddd, *J* = 21.5, 14.9, 6.7 Hz, 4H), 7.06 - 6.66 (m, 6H), 4.73 - 3.98 (m, 6H), 3.89 - 3.58 (m, 2H), 3.55 - 3.08 (m, 2H), 2.86 - 2.46 (m, 5H), 2.14 (s, 1H), 2.02 - 1.74 (m, 3H), 1.46 (t, *J* = 7.1 Hz, 3H). ¹³C NMR (101 MHz, CDCl₃) δ 163.9, 159.9, 156.0, 143.2, 138.1, 132.1, 130.4, 129.5, 129.0, 128.6, 127.2, 127.0, 126.7, 125.6, 125.3, 122.4, 120.4, 119.2, 113.1, 109.7, 109.4, 61.7, 34.4, 26.8, 14.4, 9.4, 1.1. HRMS (ESI) [M + H] ⁺, theoretically calculated for C₃₇H₃₇N₃O₆S₂Br: 762.1307, found 762.1312.

### Example 286

### Ethyl 5-(N-(2-(4-(3-bromothiophene-2-formyl)-3-methylpiperazin-1-yl)phenyl)-N-phenethylsulfamoyl)3-methylbenzofuran-2-carboxylate (M10-156)

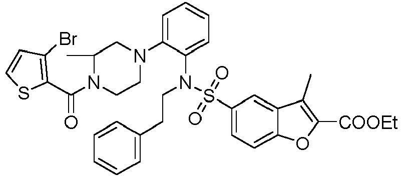

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 3-methyl-5-(N-(2-(3-methylpiperazin-1-yl)phenyl)-N-phenethylsulfamoyl)benzofuran-2-carboxylate was used instead of ethyl 5-(N-(2-(4-aminopiperidin-1-yl)phenyl)-N-phenethylsulfamoyl)-3-methylbenzofuran-2-carboxylate. 1.751 mg of white solid was obtained, with a yield of 54.5%. ¹H NMR (400 MHz, Chloroform-*d*) δ 8.13 (d, *J* = 1.8 Hz, 1H), 7.87 (dd, *J* = 8.8, 1.9 Hz, 1H), 7.64 (t, *J* = 8.4 Hz, 1H), 7.42 - 7.29 (m, 2H), 7.28 - 7.12 (m, 4H), 7.10 - 6.69 (m, 5H), 4.49 (qd, *J* = 7.1, 1.9 Hz, 2H), 4.07 (dtd, *J* = 13.3, 10.6, 5.8 Hz, 1H), 3.83 (tdd, *J* = 13.3, 10.6, 5.8 Hz, 1H), 3.71 - 3.31 (m, 2H), 3.29 - 2.40 (m, 9H), 1.61 (d, *J* = 7.2 Hz, 2H), 1.46 (td, *J* = 7.1, 2.9 Hz, 5H). ¹³C NMR (101 MHz, CDCl₃) δ 162.2, 160.0, 156.0, 151.9, 151.4, 143.2, 134.5, 134.2, 130.3, 129.7, 128.7, 128.7, 128.6, 128.6, 127.1, 126.9, 126.8, 126.7, 124.8, 124.5, 122.4, 122.2, 113.1, 109.6, 61.7, 52.6, 51.5, 34.7, 34.4, 14.5, 9.5. HRMS (ESI) [M + H] ⁺, theoretically calculated for C₃₆H₃₇N₃O₆S₂Br: 750.1307, found 750.1309.

### Example 287

### Ethyl 5-(N-(2-(5-(3-bromothiophene-2-formyl)-2,5-diazabicyclo[2.2.1]hept-2-yl)phenyl)-N-phenethylsulfamoyl)3-methylbenzofuran-2-carboxylate (M10-157)

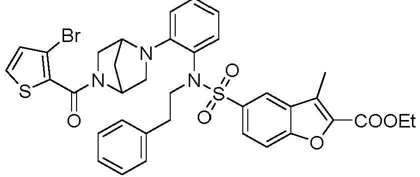

In accordance with the preparation method of ethyl 5-(N-(2-(4-(3-bromothiophene-2-carboxamido)piperidin-1-yl)phenyl)-N-phenethylsulfamoyl)-3-methylbenzofuran-2-carboxylate (M10-132), this example was implemented under the same condition except that ethyl 5-(N-(2,5-diazabicyclo[2.2.1]heptan-2-yl)phenyl)-N-phenethylsulfamoyl)-3-methylbenzofuran-2-carboxylate was used instead of ethyl 5-(N-(2-(4-aminopiperidin-1-yl)phenyl)-N-phenethylsulfamoyl)-3-methylbenzofuran-2-carboxylate. 782 mg of white solid was obtained, with a yield of 40%. ¹H NMR (400 MHz, Chloroform-*d*) δ 8.19 - 7.52 (m, 3H), 7.41 - 7.15 (m, 5H), 7.10 - 6.69 (m, 4H), 6.65 - 6.27 (m, 2H), 5.37 - 4.34 (m, 4H), 4.27 - 3.31 (m, 6H), 3.06 - 2.40 (m, 5H), 2.21 - 1.89 (m, 2H), 1.45 (td, *J* = 8.4, 7.2, 2.5 Hz, 3H).¹³C NMR (101 MHz, CDCl₃) δ 159.9, 156.1, 146.1, 143.0, 138.0, 130.7, 130.5, 129.9, 129.5, 128.8, 128.7, 128.7, 127.5, 127.2, 126.8, 126.7, 126.1, 125.7, 123.0, 122.8, 116.6, 113.0, 112.7, 110.0, 61.7, 58.6, 53.4, 33.9, 14.5, 9.5, 9.4. HRMS (ESI) [M + H] ⁺, theoretically calculated for C₃₆H₃₅N₃O₆S₂Br: 748.1151, found 748.1147.

### Example 288

### Ethyl 5-(N-(2-(4-(3-bromothiophene-2-carbonyl)piperazin-1-yl)phenyl)-N-phenethylsulfamoyl)-3-toluothiophene-2-carboxylate (M10-158)

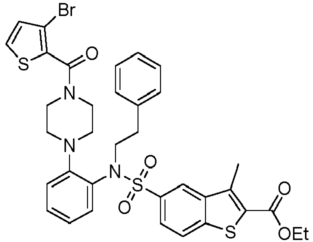

P-3-bromothiophene-2-carboxylic acid (167 mg, 0.814 mmol), EDCI (156 mg, 0.814 mmol), HOBt (110 mg, 0.814 mmol) and DIPEA (1.14 mmol, 183 µL)were dissolved in 4 mL of DCM and stirred at room temperature. When it was found from the monitoring of the TLC that there was no raw material carboxylic acid left, ethyl 3-methyl-5-(N-phenethyl-N-(2-(piperazin-1-yl)phenyl)sulfamoyl)benzo[b]thiophene-2-carboxylate (206.7 mg, 0.37 mmol) was added to the system. The reaction was performed overnight at room temperature. After the reaction was found to be completed from the monitoring of the TLC, the product was dissolved in 20 mL of water and then extracted three times with 20 mL of ethyl acetate. The organic phases were combined and then concentrated under reduced pressure and then separated by column chromatography (PE : EtOAc = 3 : 1) to obtain 203.3 mg of white solid, with a yield of 73%. ¹H NMR (400 MHz, Chloroform-*d*) δ 8.28 (s, 1H), 7.99 - 7.79 (m, 2H), 7.44 - 7.33 (m, 2H), 7.25 - 7.05 (m, 5H), 7.03 - 6.89 (m, 4H), 4.44 (q, *J* = 7.0 Hz, 2H), 4.16 - 2.83 (m, 10H), 2.77 (s, 3H), 2.73 - 2.52 (m, 2H), 1.45 (t, *J* = 7.1 Hz, 3H). ¹³C NMR (101 MHz, Chloroform-*d*) δ 162.7, 162.2, 150.9, 144.1, 140.8, 139.9, 138.1, 137.0, 134.1, 132.0, 130.4, 130.2, 129.8, 129.5, 128.5, 127.3, 126.5, 124.9, 124.7, 123.7, 123.5, 122.8, 109.7, 61.6, 51.5, 34.7, 14.4, 13.2. HRMS (ESI) [M + H] ⁺, theoretically calculated for C₃₅H₃₅BrN₃O₅S₃: 752.0917, found 752.0921.

### Example 289

### Ethyl 5-(N-(2-(4-(3-bromothiophene-2-carbonyl)piperazin-1-yl)phenyl)-N-phenethylsulfamoyl)benzo[b]thiophene-2-carboxylate (M10-159)

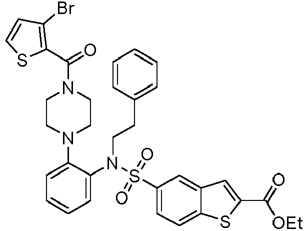

In accordance with the preparation method of ethyl 5-(N-(2-(4-(3-bromothiophene-2-carbonyl)piperazin-1-yl)phenyl)-N-phenethylsulfamoyl)-3-toluothiophene-2-carboxylate (M10-158), this example was implemented under the same condition except that ethyl 5-(N-phenethyl-N-(2-(piperazin-1-yl)phenyl)sulfamoyl)benzo[b]thiophene-2-carboxylate was used instead of ethyl 3-methyl-5-(N-phenethyl-N-(2-(piperazin-1-yl)phenyl)sulfamoyl)benzo[b]thiophene-2-carboxylate. 79.4 mg of white solid was obtained, with a yield of 57%. ¹H NMR (400 MHz, Chloroform-*d*) δ 8.34 (s, 1H), 8.12 (s, 1H), 7.97 (d, *J* = 8.5 Hz, 1H), 7.84 (d, *J* = 8.5 Hz, 1H), 7.49 - 6.89 (m, 11H), 4.47 (q, *J* = 7.0 Hz, 2H), 4.16 - 2.48 (m, 12H), 1.46 (t, *J* = 7.0 Hz, 3H). ¹³C NMR (101 MHz, Chloroform-*d*) δ 162.2, 162.0, 150.9, 145.6, 138.3, 138.0, 137.7, 136.8, 134.1, 132.0, 130.4, 130.2, 129.5, 129.2, 128.6, 128.5, 127.3, 126.6, 125.4, 124.8, 124.8, 123.6, 122.9, 109.7, 62.1, 51.6, 34.7, 14.3. HRMS (ESI) [M + H] ⁺, theoretically calculated for C₃₄H₃₃BrN₃O₅S₃: 738.0760, found 738.0760.

### Example 290

### Ethyl 6-(N-(2-(4-(3-bromothiophene-2-carbonyl)piperazin-1-yl)phenyl)-N-phenethylsulfamoyl)benzo[b]thiophene-2-carboxylate (M10-160)

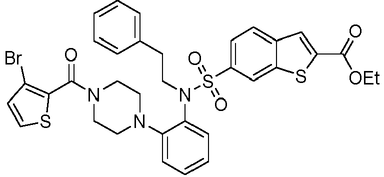

In accordance with the preparation method of ethyl 5-(N-(2-(4-(3-bromothiophene-2-carbonyl)piperazin-1-yl)phenyl)-N-phenethylsulfamoyl)-3-toluothiophene-2-carboxylate (M10-158), this example was implemented under the same condition except that ethyl 6-(N-phenethyl-N-(2-(piperazin-1-yl)phenyl)sulfamoyl)benzo[b]thiophene-2-carboxylate was used instead of ethyl 3-methyl-5-(N-phenethyl-N-(2-(piperazin-1-yl)phenyl)sulfamoyl)benzo[b]thiophene-2-carboxylate. 45.7 mg of white solid was obtained, with a yield of 62%. ¹H NMR (400 MHz, Chloroform-*d*) δ 8.34 (s, 1H), 8.12 (s, 1H), 7.98 (d, *J* = 8.5 Hz, 1H), 7.81 (d, *J* = 8.5 Hz, 1H), 7.37 (d, *J* = 5.3 Hz, 2H), 7.19 (d, *J* = 7.8 Hz, 4H), 7.10 (t, *J* = 7.7 Hz, 1H), 6.99 (d, *J* = 5.1 Hz, 4H), 4.47 (q, *J* = 7.2 Hz, 2H), 4.11 (dd, *J* = 21.1, 11.4 Hz, 2H), 3.88 (s, 1H), 3.56 (s, 3H), 3.26 (s, 2H), 3.02 - 2.49 (m, 4H), 1.46 (t, *J* = 7.1 Hz, 3H). ¹³C NMR (101 MHz, Chloroform-*d*) δ 162.2, 162.0, 150.9, 150.7, 141.7, 141.3, 138.8, 138.4, 138.0, 134.0, 132.0, 130.6, 130.2, 129.6, 129.4, 128.5, 127.2, 126.6, 126.0, 124.9, 123.5, 123.0, 122.9, 109.7, 62.1, 60.4, 51.6, 34.8, 14.3, 14.2. HRMS (ESI) [M + H] ⁺, theoretically calculated for C₃₄H₃₃BrN₃O₅S₃: 738.0760, found 738.0770.

### Example 291

### Ethyl 5-(N-(2-(4-(3-bromothiophene-2-carbonyl)piperazin-1-yl)phenyl)-N-phenethylsulfamoyl)-3-chloro-1H-indole-2-carboxylate (M10-161)

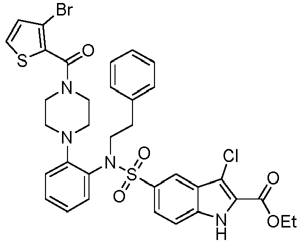

In accordance with the preparation method of ethyl 5-(N-(2-(4-(3-bromothiophene-2-carbonyl)piperazin-1-yl)phenyl)-N-phenethylsulfamoyl)-3-toluothiophene-2-carboxylate (M10-158), this example was implemented under the same condition except that ethyl 3-chloro-5-(N-phenethyl-N-(2-(piperazin-1-yl)phenyl)sulfamoyl)-1H-indole-2-carboxylate was used instead of ethyl 3-methyl-5-(N-phenethyl-N-(2-(piperazin-1-yl)phenyl)sulfamoyl)benzo[b]thiophene-2-carboxylate. 179.5 mg of white solid was obtained, with a yield of 70%. ¹H NMR (400 MHz, Chloroform-*d*) δ 10.15 (s, 1H), 8.50 - 8.09 (m, 1H), 7.87 - 7.65 (m, 1H), 7.56 - 6.75 (m, 11H), 4.50 (q, *J* = 8.2, 7.7 Hz, 2H), 4.09 - 2.46 (m, 12H), 1.48 (t, *J* = 8.1 Hz, 3H). ¹³C NMR (101 MHz, Chloroform-*d*) δ 162.4, 160.5, 150.7, 138.1, 136.3, 134.2, 133.4, 131.9, 130.5, 130.2, 129.4, 128.5, 127.4, 126.5, 125.6, 124.9, 124.8, 124.7, 122.5, 121.6, 113.3, 113.2, 109.7, 61.9, 51.5, 34.6, 14.4. HRMS (ESI) [M + H] ⁺, theoretically calculated for C₃₄H₃₃BrClN₄O₅S₂: 755.0759, found 755.0770.

### Example 292

### Ethyl 5-(N-(2-(4-(3-bromothiophene-2-formyl)piperazin-1-yl)phenyl)-N-phenethylsulfamoyl)benzofuran-2-carboxylate (M10-162)

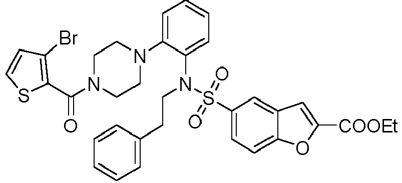

In accordance with the preparation method of ethyl 5-(N-(2-(4-(3-bromothiophene-2-carbonyl)piperazin-1-yl)phenyl)-N-phenethylsulfamoyl)-3-toluothiophene-2-carboxylate (M10-158), this example was implemented under the same condition except that ethyl 5-(N-phenethyl-N-(2-(piperazin-1-yl)phenyl)sulfamoyl)benzofuran-2-carboxylate was used instead of ethyl 3-methyl-5-(N-phenethyl-N-(2-(piperazin-1-yl)phenyl)sulfamoyl)benzo[b]thiophene-2-carboxylate. 183.0 mg of white solid was obtained, with a yield of 47%. ¹H NMR (400 MHz, Chloroform-*d*) δ 8.17 (d, *J* = 1.9 Hz, 1H), 7.85 (dd, *J* = 8.8, 1.9 Hz, 1H), 7.64 (d, *J* = 8.8 Hz, 1H), 7.56 (s, 1H), 7.32 (t, *J* = 6.7 Hz, 2H), 7.20 - 7.01 (m, 5H), 6.93 (dd, *J* = 6.5, 4.2 Hz, 4H), 4.44 (q, *J* = 7.1 Hz, 2H), 4.28 - 3.43 (m, 6H), 3.25 (d, *J* = 11.6 Hz, 2H), 2.97 - 2.44 (m, 4H), 1.42 (t, *J* = 7.1 Hz, 3H). ¹³C NMR (101 MHz, CDCl₃) δ 162.1, 158.7, 156.9, 150.8, 147.8, 138.0, 136.5, 134.0, 131.9, 130.3, 130.1, 129.4, 128.4, 128.4, 127.3, 127.1, 126.6, 126.5, 124.7, 123.5, 122.8, 113.6, 113.0, 109.5, 61.9, 51.4, 34.5, 14.2. HRMS (ESI) [M + H] ⁺, theoretically calculated for C₃₄H₃₃N₃O₆S₂Br: 722.0994, found 722.0997.

### Example 293

### Ethyl 6-(N-(2-(4-(3-bromothiophene-2-formyl)piperazin-1-yl)phenyl)-N-phenethylsulfamoyl)-3-methylbenzofuran-2-carboxylate (M10-163)

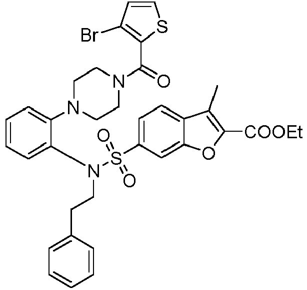

In accordance with the preparation method of Ethyl 5-(N-(2-(4-(3-bromothiophene-2-carbonyl)piperazin-1-yl)phenyl)-N-phenethylsulfamoyl)-3-toluothiophene-2-carboxylate (M10-158), this example was implemented under the same condition except that ethyl 3-methyl-6-(N-phenethyl-N-(2-(piperazin-1-yl)phenyl)sulfamoyl)benzofuran-2-carboxylate was used instead of ethyl 3-methyl-5-(N-phenethyl-N-(2-(piperazin-1-yl)phenyl)sulfamoyl)benzo[b]thiophene-2-carboxylate. 92.2 mg of white solid was obtained, with a yield of 22%. ¹H NMR (400 MHz, Chloroform-*d*) δ 8.07 - 8.00 (m, 1H), 7.76 (dq, *J* = 12.8, 8.1, 6.2 Hz, 2H), 7.35 (h, *J* = 4.9 Hz, 2H), 7.24 - 7.04 (m, 5H), 7.02 - 6.80 (m, 4H), 4.51 - 4.43 (m, 2H), 4.34 - 3.11 (m, 9H), 2.89 (s, 2H), 2.71 - 2.40 (m, 5H), 1.50 - 1.42 (m, 3H). ¹³C NMR (101 MHz, CDCl₃) δ 162.2, 159.9, 153.2, 150.9, 144.0, 139.9, 138.1, 134.2, 132.7, 132.1, 130.3, 130.2, 129.6, 128.6, 128.6, 127.3, 126.6, 124.9, 122.9, 122.3, 121.9, 112.4, 109.7, 61.7, 51.7, 34.7, 14.4, 9.4. HRMS (ESI) [M + H] ⁺, theoretically calculated for C₃₅H₃₅N₃O₆S₂Br: 736.1151, found 736.1158.

### Example 294

### Ethyl 6-(N-(2-(4-(3-bromothiophene-2-formyl)piperazin-1-yl)phenyl)-N-phenethylsulfamoyl)benzofuran-2-carboxylate (M10-164)

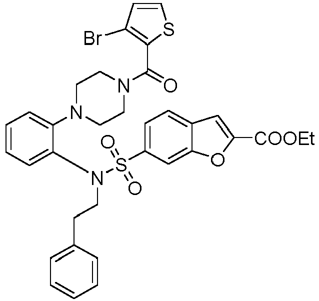

In accordance with the preparation method of ethyl 5-(N-(2-(4-(3-bromothiophene-2-carbonyl)piperazin-1-yl)phenyl)-N-phenethylsulfamoyl)-3-toluothiophene-2-carboxylate (M10-158), this example was implemented under the same condition except that ethyl 6-(N-phenethyl-N-(2-(piperazin-1-yl) phenyl)sulfamoyl)benzofuran-2-carboxylate was used instead of ethyl 3-methyl-5-(N-phenethyl-N-(2-(piperazin-1-yl)phenyl)sulfamoyl)benzo[b]thiophene-2-carboxylate. 386.9 mg of white solid was obtained, with a yield of 30%. ¹H NMR (400 MHz, Chloroform-*d*) δ 8.06 (d, *J* = 1.4 Hz, 1H), 7.83 - 7.72 (m, 2H), 7.57 (d, *J* = 1.4 Hz, 1H), 7.39 - 7.30 (m, 2H), 7.23 - 7.03 (m, 5H), 7.00 - 6.91 (m, 4H), 4.47 (q, *J* = 7.1 Hz, 2H), 4.21 - 3.10 (m, 8H), 3.04 - 2.45 (m, 4H), 1.44 (t, *J* = 7.1 Hz, 3H). ¹³C NMR (101 MHz, CDCl₃) δ 162.2, 158.9, 154.4, 150.9, 148.9, 139.7, 138.0, 134.1, 132.0, 130.7, 130.3, 130.2, 129.6, 128.6, 128.5, 127.3, 126.5, 125.0, 123.5, 122.9, 122.9, 113.1, 112.6, 109.7, 62.1, 51.7, 34.7, 14.3. HRMS (ESI) [M + H] ⁺, theoretically calculated for C₃₄H₃₃N₃O₆S₂Br: 722.0994, found 722.0992.

### Example 295

### ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165)

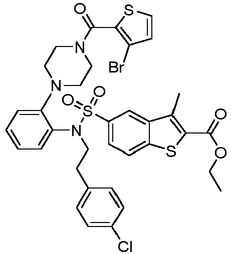

P-(3-bromothien-2-yl)(4-(2-((4-chlorophenylethyl)amino)phenyl)piperazin-1-yl)methanone (119.4 mg, 0.24 mmol), ethyl 5-(chlorosulfonyl)-3-methylbenzo[b]thiophene-2-carboxylate (90 mg, 0.29 mmol) and DIPEA (0.6 mmol, 99 µL) were dissolved in 4 mL of DCM. The resulting solution was stirred at 40 °C to react until there was no raw material left under the monitoring of the TLC. After the reaction was found to be completed from the monitoring of the TLC, the product was dissolved in 20 mL of water and then extracted three times with 20 mL of ethyl acetate. The organic phases were combined and then concentrated under reduced pressure and then separated by column chromatography (PE : EtOAc = 5 : 1) to obtain 33.0 mg of white solid, with a yield of 18%. ¹H NMR (400 MHz, Chloroform-*d*) δ 8.24 (s, 1H), 7.96 - 7.76 (m, 2H), 7.36 (d, *J* = 5.1 Hz, 2H), 7.21 (d, *J* = 7.3 Hz, 1H), 7.08 (d, *J* = 8.3 Hz, 3H), 7.02 - 6.93 (m, 2H), 6.84 (d, *J* = 8.3 Hz, 2H), 4.44 (q, *J* = 7.1 Hz, 2H), 4.14 - 2.80 (m, 10H), 2.76 (s, 3H), 2.71 - 2.46 (m, 2H), 1.44 (t, *J* = 7.1 Hz, 3H). ¹³C NMR (101 MHz, Chloroform-*d*) δ 162.9, 162.4, 151.0, 144.3, 140.9, 140.0, 137.0, 136.6, 134.1, 132.5, 132.10, 130.7, 130.3, 130.1, 129.9, 129.7, 128.7, 127.4, 124.9, 123.8, 123.6, 123.0, 109.8, 61.8, 51.3, 34.1, 14.5, 13.3.

### Example 296

### Ethyl 5-(N-(2-(4-(3-bromothiophene-2-carbonyl)piperazin-1-yl)phenyl)-N-(4-methylphenylethyl)sulfamoyl)-3-methylbenzothiophene-2-carboxylate (M10-166)

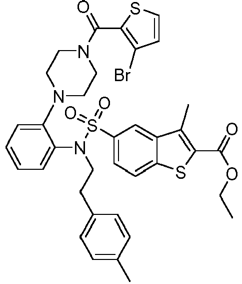

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((4-methylphenylethyl)amino)phenyl)piperazin-1-yl)methanone was used instead of (3-bromothien-2-yl)(4-(2-((4-chlorophenylethyl)amino)phenyl)piperazin-1-yl)methanone. 76.6 mg of white solid was obtained, with a yield of 29%. ¹H NMR (400 MHz, Chloroform-*d*) δ 8.26 (s, 1H), 7.95 - 7.76 (m, 2H), 7.35 (d, *J* = 5.0 Hz, 2H), 7.20 (d, *J* = 7.9 Hz, 1H), 7.08 (t, *J* = 7.4 Hz, 1H), 7.03 - 6.91 (m, 4H), 6.82 (d, *J* = 7.7 Hz, 2H), 4.43 (q, *J* = 7.1 Hz, 2H), 4.13 - 2.85 (m, 10H), 2.76 (s, 3H), 2.69 - 2.45 (m, 2H), 2.24 (s, 3H), 1.44 (t, *J* = 7.1 Hz, 3H). ¹³C NMR (101 MHz, Chloroform-*d*) δ 162.8, 162.2, 150.9, 144.1, 140.9, 139.9, 137.0, 136.1, 134.9, 134.1, 132.0, 130.4, 130.2, 129.8, 129.5, 129.2, 128.4, 127.3, 125.0, 124.7, 123.7, 123.4, 122.8, 109.7, 61.6, 51.6, 34.2, 21.0, 14.4, 13.2.

### Example 297

### ethyl 5-(N-(2-(4-(3-bromothiophene-2-carbonyl)piperazin-1-yl)phenyl)-N-(4-(methoxycarbonyl)phenethyl)sulfamoyl)-3-methylbenzothiophene-2-carboxylate (M10-167)

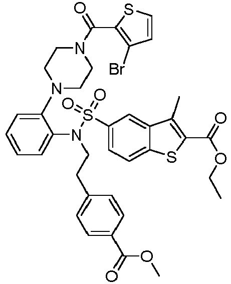

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that methyl 4-(2-(4-(3-bromothiophene-2-carbonyl) piperazin-1-yl) phenyl) amino) ethyl) benzoate was used instead of (3-bromothien-2-yl)(4-(2-((4-chlorophenylethyl)amino)phenyl)piperazin-1-yl)methanone. 79.8 mg of white solid was obtained, with a yield of 25%. ¹H NMR (400 MHz, Chloroform-*d*) δ 8.23 (d, *J* = 1.7 Hz, 1H), 7.92 (d, *J* = 8.5 Hz, 1H), 7.84 (dd, *J* = 8.6, 1.8 Hz, 1H), 7.80 - 7.75 (m, 2H), 7.40 - 7.34 (m, 2H), 7.23 (dd, *J* = 8.1, 1.5 Hz, 1H), 7.12 (d, *J* = 1.5 Hz, 1H), 7.06 - 6.95 (m, 4H), 4.45 (q, *J* = 7.1 Hz, 2H), 4.22 - 4.08 (m, 2H), 3.96 - 3.83 (m, 4H), 3.69 - 3.07 (m, 5H), 2.97 - 2.84 (m, 2H), 2.78 - 2.73 (m, 4H), 2.63 (q, *J* = 7.8 Hz, 1H). ¹³C NMR (101 MHz, Chloroform-*d*) δ 166.6, 162.7, 162.2, 150.9, 144.1, 143.5, 140.7, 139.9, 136.9, 133.9, 132.0, 130.7, 130.2, 130.0, 129.7, 128.5, 128.4, 127.3, 124.9, 124.8, 123.6, 123.4, 122.9, 109.7, 61.6, 52.0, 50.9, 34.6, 14.3, 13.2.

### Example 298

### Ethyl 5-(N-(2-(4-(3-bromothiophene-2-carbonyl)piperazin-1-yl)phenyl)-N-(4-cyanophenyl)sulfamoyl)-3-methylbenzothiophene-2-carboxylate (M10-168)

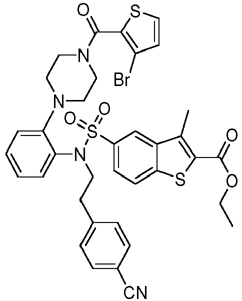

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that 4-(2-((2-(4-(3-bromothiophene-2-carbonyl)piperazin-1-yl)phenyl)amino)ethyl)benzonitrile was used instead of (3-bromothien-2-yl)(4-(2-((4-chlorophenylethyl)amino)phenyl)piperazin-1-yl)methanone. 43.0 mg of white solid was obtained, with a yield of 14%. ¹H NMR (400 MHz, Chloroform-*d*) δ 8.24 (d, *J* = 1.6 Hz, 1H), 7.94 (d, *J* = 8.5 Hz, 1H), 7.83 (dd, *J* = 8.6, 1.7 Hz, 1H), 7.47 - 7.34 (m, 4H), 7.22 (d, *J* = 7.6 Hz, 1H), 7.14 - 6.96 (m, 5H), 4.45 (q, *J* = 7.2 Hz, 2H), 4.12 - 3.00 (m, 8H), 2.91 - 2.57 (m, 7H), 1.46 (t, *J* = 7.1 Hz, 3H). ¹³C NMR (101 MHz, Chloroform-*d*) δ 162.7, 162.2, 150.8, 144.2, 143.7, 140.6, 139.9, 136.7, 133.8, 132.2, 131.9, 130.6, 130.2, 129.8, 129.3, 127.3, 124.9, 124.7, 123.7, 123.5, 123.0, 118.5, 110.5, 109.7, 61.7, 50.69, 34.9, 14.3, 13.2.

### Example 299

### Ethyl 5-(N-(2-(4-(3-bromothiophene-2-carbonyl)piperazin-1-yl)phenyl)-N-(4-(trifluoromethyl)phenethyl)sulfamoyl)-3-methylbenzothiophene-2-carboxylate (M10-169)

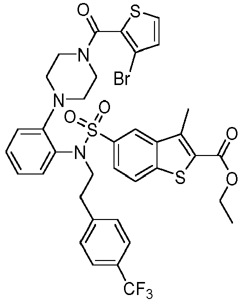

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((4-(trifluoromethyl)phenethyl)amino)phenyl)piperazin-1-yl)methanone was used instead of (3-bromothien-2-yl)(4-(2-((4-chlorophenylethyl)amino)phenyl)piperazin-1-yl)methanone. 51 mg of white solid was obtained, with a yield of 18%. ¹H NMR (400 MHz, Chloroform-*d*) δ 8.29 (d, *J* = 1.7 Hz, 1H), 7.98 - 7.76 (m, 2H), 7.40 (dd, *J* = 16.5, 6.8 Hz, 4H), 7.23 (dd, *J* = 8.1, 1.5 Hz, 1H), 7.13 - 6.93 (m, 5H), 4.46 (q, *J* = 7.1 Hz, 2H), 4.17 - 3.11 (m, 7H), 2.98 - 2.54 (m, 8H), 1.46 (t, *J* = 7.1 Hz, 3H). ¹³C NMR (101 MHz, Chloroform-*d*) δ 162.7, 162.2, 150.8, 144.2, 142.2, 140.7, 139.9, 136.8, 133.9, 132.0, 130.5, 130.2, 130.1, 129.7, 128.8, 127.3, 125.4, 125.4, 124.9, 124.7, 123.7, 123.5, 122.9, 109.7, 61.7, 51.0, 34.6, 14.3, 13.2.

### Example 300

### Ethyl 5-(N-(2-(4-(3-bromothiophene-2-carbonyl)piperazin-1-yl)phenyl)-N-(3-methoxyphenethyl)sulfamoyl)-3-methylbenzothiophene-2-carboxylate (M10-170)

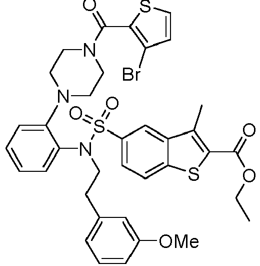

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((3-methoxyphenethyl)amino)phenyl)piperazin-1-yl)methanone was used instead of (3-bromothien-2-yl)(4-(2-((4-chlorophenylethyl)amino)phenyl)piperazin-1-yl)methanone. 81 mg of white solid was obtained, with a yield of 36%.

### Example 301

### Ethyl 5-(N-(2-(4-(3-bromothiophene-2-carbonyl)piperazin-1-yl)phenyl)-N-(3-chlorophenylethyl)sulfamoyl)-3-methylbenzothiophene-2-carboxylate (M10-171)

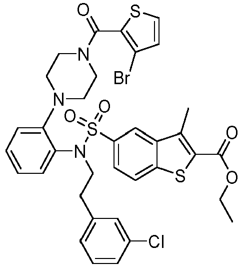

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((3-chlorophenylethyl)amino)phenyl)piperazin-1-yl)methanone was used instead of (3-bromothien-2-yl)(4-(2-((4-chlorophenylethyl)amino)phenyl)piperazin-1-yl)methanone. 38.8 mg of white solid was obtained, with a yield of 24%. ¹H NMR (400 MHz, Chloroform-*d*) δ 8.38 - 8.19 (m, 1H), 8.02 - 7.78 (m, 2H), 7.38 (d, *J* = 4.7 Hz, 2H), 7.22 (d, *J* = 7.9 Hz, 1H), 7.10 (q, *J* = 7.1, 6.5 Hz, 3H), 7.01 (t, *J* = 6.7 Hz, 2H), 6.91 (s, 1H), 6.84 (d, *J* = 6.9 Hz, 1H), 4.46 (q, *J* = 7.1 Hz, 2H), 4.18 - 3.14 (m, 8H), 2.98 - 2.47 (m, 7H), 1.47 (t, *J* = 7.0 Hz, 3H). ¹³C NMR (101 MHz, Chloroform-*d*) δ 162.8, 162.2, 150.8, 144.2, 140.8, 140.1, 139.9, 136.9, 134.2, 134.0, 132.0, 130.5, 130.2, 129.7, 129.6, 128.7, 127.3, 126.6, 124.8, 123.7, 123.5, 122.8, 109.7, 61.7, 51.1, 34.3, 29.7, 14.3, 13.2.

### Example 302

### Ethyl 5-(N-(2-(4-(3-bromothiophene-2-carbonyl)piperazin-1-yl)phenyl)-N-(2-chlorophenylethyl) sulfamoyl)-3-methylbenzothiophene-2-carboxylate (M10-172)

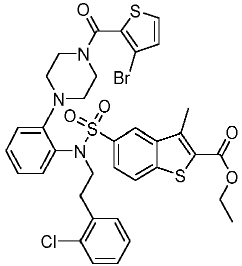

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((2-chlorophenylethyl)amino)phenyl)piperazin-1-yl)methanone was used instead of (3-bromothien-2-yl)(4-(2-((4-chlorophenylethyl)amino)phenyl)piperazin-1-yl)methanone. 15 mg of white solid was obtained, with a yield of 13%. ¹H NMR (400 MHz, Chloroform-*d*) δ 8.36 (d, *J* = 1.6 Hz, 1H), 8.03 - 7.86 (m, 2H), 7.37 (d, *J* = 5.2 Hz, 2H), 7.25 - 7.15 (m, 2H), 7.14 - 7.06 (m, 4H), 6.99 (d, *J* = 5.0 Hz, 2H), 4.46 (q, *J* = 7.1 Hz, 2H), 4.17 - 3.18 (m, 8H), 2.96 - 2.60 (m, 7H), 1.47 (t, *J* = 7.2 Hz, 3H). ¹³C NMR (101 MHz, Chloroform-*d*) δ 162.8, 162.2, 150.7, 144.1, 140.9, 139.9, 137.2, 135.7, 134.0, 133.9, 132.0, 130.8, 130.6, 130.2, 129.5, 128.1, 127.2, 126.9, 124.8, 124.7, 123.8, 123.5, 122.7, 109.7, 61.6, 49.2, 32.9, 14.3, 13.2.

### Example 303

### Ethyl 5-(N-(2-(4-(3-bromothiophene-2-carbonyl)piperazin-1-yl)phenyl)-N-(2-methoxyphenethyl)sulfamoyl)-3-methylbenzothiophene-2-carboxylate (M10-173)

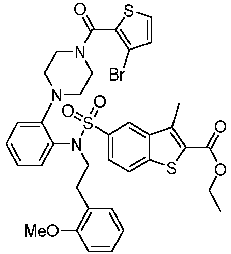

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((2-methoxyphenethyl)amino)phenyl)piperazin-1-yl)methanone was used instead of (3-bromothien-2-yl)(4-(2-((4-chlorophenylethyl)amino)phenyl)piperazin-1-yl)methanone. 70.7 mg of white solid was obtained, with a yield of 28%. ¹H NMR (400 MHz, Chloroform-*d*) δ 8.32 (s, 1H), 7.98 - 7.83 (m, 2H), 7.37 (d, *J* = 4.9 Hz, 2H), 7.22 (d, *J* = 7.9 Hz, 1H), 7.16 - 7.05 (m, 2H), 7.05 - 6.96 (m, 2H), 6.87 (dd, *J* = 7.4, 1.7 Hz, 1H), 6.78 (t, *J* = 7.3 Hz, 1H), 6.69 (d, *J* = 8.2 Hz, 1H), 4.45 (q, *J* = 7.1 Hz, 2H), 4.16 - 3.77 (m, 2H), 3.72 - 3.43 (m, 6H), 3.37 - 2.75 (m, 8H), 2.69 - 2.53 (m, 2H). ¹³C NMR (101 MHz, Chloroform-*d*) δ 162.8, 162.2, 157.4, 150.9, 144.0, 141.0, 139.8, 137.4, 134.2, 132.1, 130.6, 130.2, 130.2, 129.7, 129.3, 127.9, 127.2, 126.3, 125.0, 124.6, 123.8, 123.3, 122.7, 120.4, 110.2, 109.7, 61.6, 55.0, 49.5, 29.9, 14.3, 13.2.

### Example 304

### Ethyl 5-(N-(2-(4-(3-bromothiophene-2-carbonyl)piperazin-1-yl)phenyl)-N-(2-nitrophenylethyl)sulfamoyl)-3-toluothiophene-2-carboxylate (M10-174)

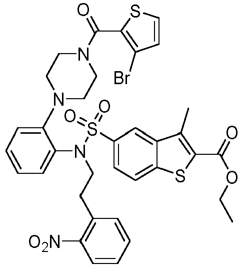

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((2-nitrophenylethyl) amino)phenyl)piperazin-1-yl)methanone was used instead of (3-bromothien-2-yl)(4-(2-((4-chlorophenylethyl)amino)phenyl)piperazin-1-yl)methanone. 27.6 mg of white solid was obtained, with a yield of 15%. ¹H NMR (400 MHz, Chloroform-*d*) δ 8.37 (d, *J* = 1.6 Hz, 1H), 8.04 - 7.90 (m, 2H), 7.83 (d, *J* = 8.2 Hz, 1H), 7.53 - 7.43 (m, 1H), 7.39 - 7.30 (m, 3H), 7.23 - 7.15 (m, 2H), 7.10 (d, *J* = 4.4 Hz, 2H), 6.98 (d, *J* = 5.2 Hz, 1H), 4.46 (q, *J* = 7.1 Hz, 2H), 4.30 - 3.11 (m, 7H), 3.05 - 2.68 (m, 8H), 1.46 (t, *J* = 7.1 Hz, 3H). ¹³C NMR (101 MHz, Chloroform-*d*) δ 162.7, 162.2, 150.4, 144.2, 140.9, 140.0, 137.2, 133.7, 133.3, 132.5, 132.0, 130.6, 130.2, 130.0, 129.6, 127.9, 127.2, 124.9, 124.8, 124.7, 123.7, 123.6, 122.7, 109.7, 61.7, 50.0, 32.9, 14.3, 13.2.

### Example 305

### ethyl 5-(N-(2-(4-(3-bromothiophene-2-carbonyl)piperazin-1-yl)phenyl)-N-(3-phenylpropyl) sulfamoyl)-3-methylbenzothiophene-2-carboxylate (M10-175)

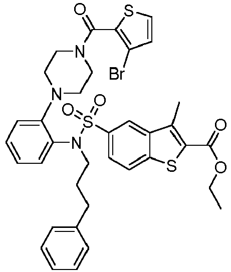

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that ((3-bromothien-2-yl)(4-(2-((3-phenylpropyl)amino)phenyl)piperazin-1-yl)methanone was used instead of (3-bromothien-2-yl)(4-(2-((4-chlorophenylethyl)amino)phenyl)piperazin-1-yl)methanone. 62.5 mg of white solid was obtained, with a yield of 30%. ¹H NMR (400 MHz, Chloroform-*d*) δ 8.28 (s, 1H), 8.01 - 7.80 (m, 2H), 7.42 - 7.31 (m, 2H), 7.23 - 6.90 (m, 9H), 4.45 (q, *J* = 7.1 Hz, 2H), 3.90 - 3.02 (m, 8H), 2.89 - 2.61 (m, 5H), 2.62 - 2.38 (m, 2H), 1.46 (t, *J* = 7.1 Hz, 3H). ¹³C NMR (101 MHz, Chloroform-*d*) δ 162.8, 162.1, 150.8, 144.0, 140.7, 139.9, 137.3, 134.2, 132.0, 130.4, 130.2, 129.9, 129.4, 128.4, 128.1, 127.2, 126.1, 124.9, 124.8, 123.7, 123.5, 122.8, 109.6, 61.6, 49.4, 32.8, 29.4, 14.4, 13.2.

### Example 306

### Ethyl 5-(N-(2-(4-(3-bromothiophene-2-carbonyl)piperazin-1-yl)phenyl)sulfamoyl)-3-toluothiophene-2-carboxylate (M10-176)

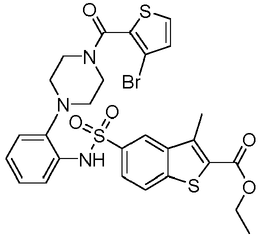

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (4-(2-aminophenyl)piperazin-1-yl)(3-bromothien-2-yl)methanone was used instead of (3-bromothien-2-yl)(4-(2-((4-chlorophenylethyl)amino)phenyl)piperazin-1-yl)methanone. 180.0 mg of white solid was obtained, with a yield of 93%. ¹H NMR (400 MHz, Chloroform-*d*) δ 8.31 (d, *J* = 1.4 Hz, 1H), 8.03 (s, 1H), 7.91 - 7.81 (m, 2H), 7.66 (d, *J* = 8.1 Hz, 1H), 7.38 (d, *J* = 5.2 Hz, 1H), 7.21 - 6.92 (m, 4H), 4.42 (p, *J* = 7.1 Hz, 2H), 3.92 - 3.24 (m, 4H), 2.80 - 2.68 (m, 3H), 2.53 (t, *J* = 5.1 Hz, 4H), 1.42 (t, *J* = 7.1 Hz, 3H). ¹³C NMR (101 MHz, Chloroform-*d*) δ 162.6, 162.2, 144.4, 141.4, 140.6, 139.8, 136.0, 132.9, 131.7, 130.3, 130.1, 127.6, 126.7, 125.0, 123.9, 123.5, 123.0, 122.1, 119.4, 109., 61.7, 52.6, 14.3, 13.1.

### Example 307

### Ethyl 5-(N-(2-(4-(3-bromothiophene-2-formyl)piperazin-1-yl)phenyl)-N-(4-methoxyphenethyl) sulfamoyl)3-methylbenzothiophene-2-carboxylate (M10-177)

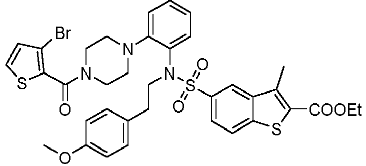

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((4-methoxyphenethyl)amino)phenyl)piperazin-1-yl)methanone was used instead of (3-bromothien-2-yl)(4-(2-((4-chlorophenylethyl)amino)phenyl)piperazin-1-yl)methanone. 112.3 mg of white solid was obtained, with a yield of 31%. ¹H NMR (400 MHz, Chloroform-*d*) δ 8.25 (d, *J* = 1.7 Hz, 1H), 7.90 (d, *J* = 8.6 Hz, 1H), 7.83 (dd, *J* = 8.5, 1.7 Hz, 1H), 7.39 - 7.30 (m, 2H), 7.20 (dd, *J* = 8.2, 1.5 Hz, 1H), 7.08 (td, *J* = 7.6, 1.5 Hz, 1H), 7.03 - 6.93 (m, 2H), 6.87 - 6.81 (m, 2H), 6.70 - 6.64 (m, 2H), 4.42 (q, *J* = 7.1 Hz, 2H), 4.20 - 3.75 (m, 3H), 3.71 (s, 3H), 3.42 (d, *J* = 122.1 Hz, 5H), 2.90 (s, 2H), 2.75 (s, 3H), 2.69 - 2.40 (m, 2H), 1.43 (t, *J* = 7.1 Hz, 3H). ¹³C NMR (101 MHz, CDCl₃) δ 162.8, 162.2, 158.2, 150.9, 144.0, 140.8, 139.9, 137.1, 134.1, 132.1, 130.5, 130.2, 130.0, 129.9, 129.5, 129.4, 127.3, 125.0, 124.7, 123.7, 123.4, 122.8, 113.9, 109.7, 61.6, 55.2, 51.7, 33.7, 29.7, 14.4, 13.2, 1.1. HRMS (ESI) [M + H] ⁺, theoretically calculated for C₃₆H₃₇N₃O₆S₃ Br: 782.1028, found 782.1033.

### Example 308

### Ethyl 5-(N-(2-(4-(3-bromothiophene-2-formyl)piperazin-1-yl)phenyl)-N-(3-methoxyphenethyl)sulfamoyl)3-methylbenzothiophene-2-carboxylate (M10-178)

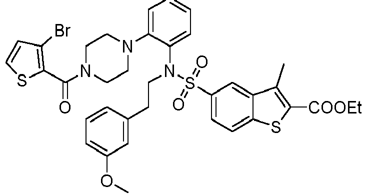

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((3-methoxyphenethyl)amino)phenyl)piperazin-1-yl)methanone was used instead of (3-bromothien-2-yl)(4-(2-((4-chlorophenylethyl)amino)phenyl)piperazin-1-yl)methanone. 81.0 mg of white solid was obtained, with a yield of 36%. ¹H NMR (400 MHz, Chloroform-*d*) δ 8.26 (d, *J* = 1.8 Hz, 1H), 7.91 (d, *J* = 8.5 Hz, 1H), 7.83 (dd, *J* = 8.5, 1.8 Hz, 1H), 7.40 - 7.31 (m, 2H), 7.20 (dd, *J* = 8.1, 1.4 Hz, 1H), 7.12 - 6.91 (m, 4H), 6.64 (dd, *J* = 8.5, 2.9 Hz, 1H), 6.58 - 6.46 (m, 2H), 4.43 (q, *J* = 7.1 Hz, 2H), 4.18 - 3.74 (m, 3H), 3.70 (s, 3H), 3.66 - 2.81 (m, 7H), 2.76 (s, 3H), 2.60 (dt, *J* = 35.8, 8.0 Hz, 2H), 1.43 (t, *J* = 7.1 Hz, 3H). ¹³C NMR (101 MHz, CDCl₃) δ 162.8, 162.3, 159.7, 151.0, 144.2, 141.0, 139.9, 139.7, 137.1, 134.2, 132.1, 130.5, 130.3, 129.9, 129.6, 129.6, 127.3, 125.0, 124.8, 123.8, 123.5, 122.9, 120.8, 114.6, 111.5, 109.7, 61.7, 55.2, 51.5, 34.7, 14.4, 13.3. HRMS (ESI) [M + H] ⁺, theoretically calculated for C₃₆H₃₇N₃O₆S₃ Br: 782.1028, found 782.1046.

### Example 309

### 3-methyl-5-(N-(2-(4-(4-methylbenzoyl)piperazin-1-yl)phenyl)-N-phenethylsulfamoyl)benzofuran-2-carboxylic acid (F27-S106)

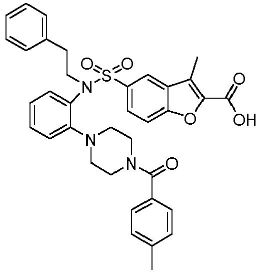

Ethyl 3-methyl-5-(N-(2-(4-(4-methylbenzoyl)piperazin-1-yl)phenyl)-N-phenethylsulfamoyl)benzofuran-2-carboxylate (170 mg, 0.26 mmol) was dissolved in a mixed solvent of ethanol (3 mL) and water (1 mL), and NaOH(31 mg, 0.78 mmol) was then added. The resulting solution was refluxed and stirred until it was found from the monitoring of the TLC that there was no raw material left. The reaction system was concentrated under reduced pressure to remove the ethanol out of the system. 5 mL of water was added to the system, and 1M diluted hydrochloric acid was then added dropwise until the pH showed acidity. The system was then stirred rapidly for 10 min, during which solid precipitated. The system was filtered by using a suction funnel and washed with water to obtain 168.6 mg of white solid, with a yield of 100%. ¹H NMR (400 MHz, DMSO-*d*₆) δ 8.26 (s, 1H), 7.96 - 7.79 (m, 2H), 7.43 - 7.20 (m, 6H), 7.18 - 7.05 (m, 5H), 6.98 (d, *J* = 7.1 Hz, 2H), 4.17 - 2.54 (m, 15H), 2.35 (s, 3H). ¹³C NMR (101 MHz, DMSO-*d*₆) δ 169.1, 160.7, 155.1, 150.4, 143.6, 139.2, 138.2, 135.4, 133.2, 132.8, 130.8, 129.3, 129.2, 129.1, 128.8, 128.5, 128.2, 127.0, 126.6, 126.2, 124.3, 122.8, 121.8, 113.0, 50.3, 34.0, 20.9, 9.0. HRMS (ESI) [M + H] ⁺, theoretically calculated for C₃₆H₃₄N₃O₆S: 636.2174, found 636.2170.

### Example 310

### 3-methyl-5-(N-(2-(4-(2-methylbenzoyl)piperazin-1-yl)phenyl)-N-phenethylsulfamoyl)benzofuran-2-carboxylic acid (F27-S107)

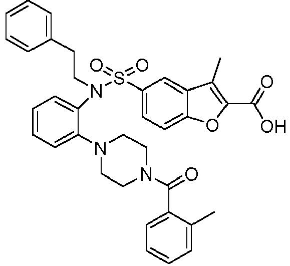

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-(2-(4-(2-methylbenzoyl)piperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 99.5 mg of white solid was obtained, with a yield of 87%. ¹H NMR (400 MHz, DMSO-*d*₆) δ 8.23 (s, 1H), 7.96 - 7.74 (m, 2H), 7.43 - 7.21 (m, 5H), 7.17 - 7.04 (m, 6H), 6.97 (d, *J* = 7.1 Hz, 2H), 4.14 - 2.53 (m, 15H), 2.21 (s, 3H). ¹³C NMR (101 MHz, DMSO-*d*₆) δ 169.0, 161.3, 155.5, 150.8, 138.6, 136.7, 135.7, 134.0, 133.7, 131.3, 130.6, 129.7, 129.6, 129.1, 128.9, 128.7, 126.9, 126.6, 126.2, 126.1, 124.8, 124.4, 123.2, 122.2, 113.4, 52.7, 51.4 (d, *J* = 122.5 Hz), 41.7, 34.4, 19.0, 9.5. HRMS (ESI) [M + H] ⁺, theoretically calculated for C₃₆H₃₄N₃O₆S: 636.2174, found 636.2155.

### Example 311

### 3-methyl-5-(N-(2-(4-(3-methylbenzoyl)piperazin-1-yl)phenyl)-N-phenethylsulfamoyl)benzofuran-2-carboxylic acid (F27-S108)

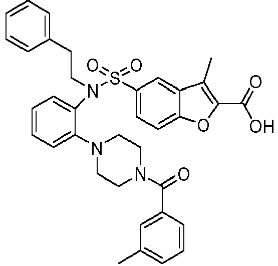

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-(2-(4-(3-methylbenzoyl)piperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 50.3 mg of white solid was obtained, with a yield of 44%. ¹H NMR (400 MHz, DMSO-*d*₆) δ 13.73 (s, 1H), 8.25 (s, 1H), 7.94 - 7.81 (m, 2H), 7.40 - 7.23 (m, 4H), 7.21 - 7.04 (m, 7H), 6.97 (s, 2H), 4.14 - 2.53 (m, 15H), 2.34 (s, 3H). ¹³C NMR (101 MHz, DMSO-*d*₆) δ 169.6, 161.1, 155.6, 150.9, 143.8, 138.6, 138.3, 136.3, 135.9, 133.7, 131.2, 130.5, 129.6, 128.9, 128.7, 127.8, 127.1, 126.6, 125.2, 124.8, 124.3, 123.2, 122.3, 113.5, 50.8, 34.4, 21.4, 9.5. HRMS (ESI) [M + H] ⁺, theoretically calculated for C₃₆H₃₄N₃O₆S: 636.2174, found 636.2163.

### Example 312

### 5-(N-(2-(4-(4-acetamidobenzoyl)piperazin-1-yl)phenyl)-N-phenethylsulfamoyl)-3-methylbenzofuran-2-carboxylic acid (F27-S109)

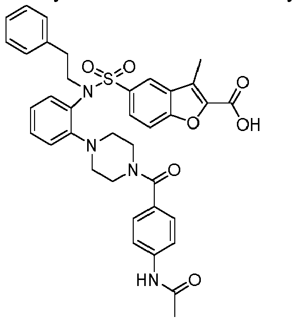

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(4-Acetylaminobenzoyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 127.6 mg of white solid was obtained, with a yield of 100%. ¹H NMR (400 MHz, DMSO-*d*₆) δ 8.23 (s, 1H), 7.88 (q, *J* = 8.7 Hz, 2H), 7.65 (d, *J* = 8.0 Hz, 2H), 7.44 - 7.25 (m, 4H), 7.19 - 6.90 (m, 7H), 4.16 - 2.51 (m, 15H), 2.07 (s, 3H). ¹³C NMR (101 MHz, DMSO-*d*₆) δ 169.4, 169.1, 161.2, 155.6, 151.0, 141.0, 138.6, 135.7, 133.7, 131.0, 130.4, 129.7, 128.9, 128.7, 128.5, 127.0, 126.6, 124.8, 123.3, 122.3, 118.9, 113.5, 110.0, 52.3, 50.8, 34.3, 24.5, 9.5.

### Example 313

### 3-methyl-5-(N-phenethyl-N-(2-(4-(4-(trifluoromethyl)benzoyl)piperazin-1-yl)phenyl)sulfamoyl)benzofuran-2-carboxylic acid (F27-S110)

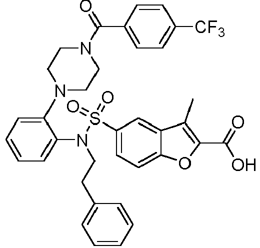

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-phenethyl-N-(2-(4-(4-(trifluoromethyl)benzoyl)piperazin-1-yl)phenyl)sulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 127.6 mg of white solid was obtained, with a yield of 100%. ¹H NMR (400 MHz, DMSO-*d*₆) δ 8.26 (s, 1H), 7.87 (dd, *J* = 24.6, 9.0 Hz, 4H), 7.66 (d, *J* = 7.9 Hz, 2H), 7.38 (t, *J* = 7.4 Hz, 1H), 7.29 (d, *J* = 7.7 Hz, 1H), 7.21 - 7.02 (m, 5H), 6.98 (d, *J* = 7.1 Hz, 2H), 4.21 - 2.69 (m, 10H), 2.59 (s, 4H). ¹³C NMR (101 MHz, DMSO-*d*₆) δ 168.1, 161.3, 155.6, 150.9, 144.37, 140.43, 138.59, 135.70, 133.83, 131.02, 129.68, 129.65, 128.89, 128.66, 128.22, 127.0, 126.6, 125.9, 125.9, 124.8, 124.7, 123.2, 122.3, 113.5, 50.9, 34.3, 9.5. HRMS (ESI) [M + H] ⁺, theoretically calculated for C₃₆H₃₁F₃N₃O₆S: 690.1891, found 690.1889.

### Example 314

### 5-(N-(2-(4-(4-cyanophenyl)piperazin-1-yl)phenyl)-N-phenethylsulfamoyl)-3-methylbenzofuran-2-carboxylic acid (F27-S111)

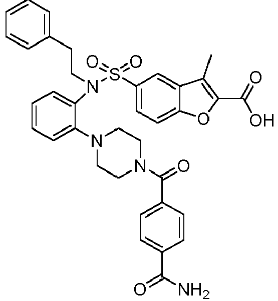

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(4-cyanophenyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 54.4 mg of white solid was obtained, with a yield of 56%. ¹H NMR (400 MHz, DMSO-*d*₆) δ 8.19 (s, 1H), 7.95 (d, *J* = 8.2 Hz, 2H), 7.84 (d, *J* = 5.3 Hz, 2H), 7.48 (d, *J* = 8.0 Hz, 2H), 7.31 (dd, *J* = 30.1, 7.1 Hz, 2H), 7.18 - 7.00 (m, 5H), 6.97 (d, *J* = 7.1 Hz, 2H), 4.14 - 2.66 (m, 13H), 2.58 (s, 4H). ¹³C NMR (101 MHz, DMSO-*d*₆) δ 168.8, 167.8, 162.1, 155.3, 150.9, 138.9, 138.6, 137.9, 135.6, 135.3, 133.8, 133.0, 131.1, 130.1, 129.6, 128.9, 128.7, 128.1, 127.3, 126.6, 126.3, 124.8, 123.3, 121.9, 113.3, 50.8, 34.4, 9.5. HRMS (ESI) [M + H] ⁺, theoretically calculated for C₃₆H₃₃N₄O₇S: 665.2075, found 665.2065.

### Example 315

### 5-(N-(2-(4-(4-fluorobenzoyl)piperazin-1-yl)phenyl)-N-phenethylsulfamoyl)-3-methylbenzofuran-2-carboxylic acid (F27-S112)

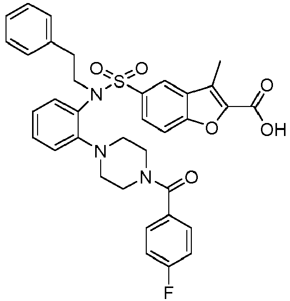

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(4-fluorobenzoyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 67.1 mg of white solid was obtained, with a yield of 65%. ¹H NMR (400 MHz, DMSO-*d*₆) δ 8.25 (s, 1H), 7.88 (q, *J* = 8.6 Hz, 2H), 7.57 - 7.43 (m, 2H), 7.42 - 7.21 (m, 4H), 7.20 - 7.03 (m, 5H), 6.98 (d, *J* = 6.9 Hz, 2H), 4.13 - 2.69 (m, 11H), 2.58 (s, 4H). ¹³C NMR (101 MHz, DMSO-*d*₆) δ 168.6, 163.0 (d, *J* = 246.6 Hz), 161.4, 155.5, 150.9, 144.7, 138.6, 135.7, 133.8, 132.7, 131.2, 130.0 (d, *J* = 8.4 Hz), 129.7 (d, *J* = 12.0 Hz), 128.9, 128.7, 126.9, 126.6, 124.8, 124.3, 123.2, 122.2, 115.9, 115.7, 113.5, 50.8, 34.4, 9.5. HRMS (ESI) [M + H] ⁺, theoretically calculated for C₃₅H₃₁FN₃O₆S: 640.1923, found 640.1909.

### Example 316

### Ethyl-(N-(2-(4-(4-((tert-butoxycarbonyl)amino)benzoyl)piperazin-1-yl)phenyl)-N-phenethylsulfamoyl)-3-methylbenzofuran-2-carboxylate (F27-S113)

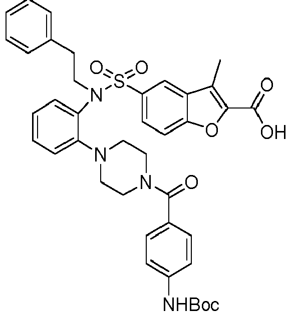

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl -(N-(2-(4-(4-((tert-butoxycarbonyl)amino)benzoyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 83.7 mg of white solid was obtained, with a yield of 72%. ¹H NMR (400 MHz, DMSO-*d*₆) δ 9.59 (s, 1H), 8.23 (d, *J* = 1.8 Hz, 1H), 8.00 - 7.77 (m, 2H), 7.55 (d, *J* = 8.2 Hz, 2H), 7.40 - 7.31 (m, 3H), 7.28 (d, *J* = 8.0 Hz, 1H), 7.12 (ddd, *J* = 12.7, 8.2, 4.3 Hz, 4H), 7.03 (dd, *J* = 7.9, 1.5 Hz, 1H), 6.99 - 6.94 (m, 2H), 4.15 - 2.70 (m, 12H), 2.59 (s, 3H), 1.49 (s, 9H). ¹³C NMR (101 MHz, DMSO-*d*₆) δ 169.4, 161.5, 155.5, 153.1, 151.0, 141.3, 138.6, 135.6, 133.8, 130.9, 129.8, 129.6, 129.4, 128.9, 128.7, 128.6, 126.7, 126.6, 124.7, 123.2, 122.2, 117.9, 113.4, 110.0, 79.8, 52.3, 50.8, 34.3, 28.5, 9.5. HRMS (ESI) [M + H] ⁺, theoretically calculated for C₄₀H₄₁N₄O₈S: 737.2651, found 737.2644.

### Example 317

### 3-methyl-5-(N-(2-(4-(4-nitrobenzoyl)piperazin-1-yl)phenyl)-N-phenethylsulfamoyl)benzofuran-2-carboxylic acid (F27-S114)

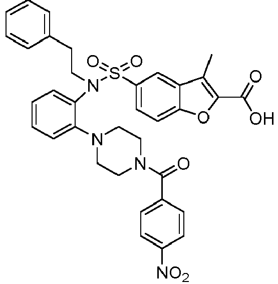

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-(2-(4-(4-nitrobenzoyl)piperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 29.3 mg of white solid was obtained, with a yield of 53%. ¹H NMR (400 MHz, DMSO-*d*₆) δ 9.38 (s, 1H), 8.31 (t, *J* = 9.0 Hz, 1H), 8.23 (s, 1H), 8.21 - 8.14 (m, 1H), 7.92 - 7.84 (m, 2H), 7.71 (d, *J* = 8.6 Hz, 1H), 7.45 - 7.32 (m, 1H), 7.33 - 7.23 (m, 1H), 7.19 - 6.92 (m, 7H), 4.20 - 2.70 (m, 11H), 2.63 - 2.54 (m, 4H). ¹³C NMR (101 MHz, DMSO-*d*₆) δ 167.6, 161.4, 155.6, 150.9, 150.6, 148.3, 142.7, 138.5, 135.3, 134.1, 133.8, 131.1, 129.9, 129.8, 128.9, 128.7, 126.9, 126.6, 125.3, 124.2, 124.1, 123.2, 122.4, 113.5, 51.1, 49.2, 43.8, 34.2, 9.5. HRMS (ESI) [M + H] ⁺, theoretically calculated for C₃₅H₃₁N₄O₈S: 667.1868, found 667.1864.

### Example 318

### 5-(N-(2-(4-(4-acetylbenzoyl)piperazin-1-yl)phenyl)-N-phenethylsulfamoyl)-3-methylbenzofuran-2-carboxylic acid (F27-S115)

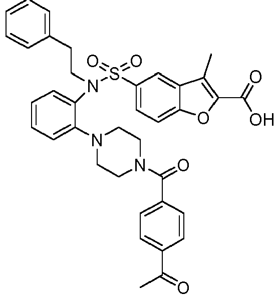

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(4-acetylbenzoyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 66.4 mg of white solid was obtained, with a yield of 82%. ¹H NMR (400 MHz, DMSO-*d*₆) δ 9.60 (s, 1H), 8.23 (s, 1H), 8.11 - 7.98 (m, 2H), 7.86 (t, *J* = 7.7 Hz, 2H), 7.56 (d, *J* = 8.2 Hz, 1H), 7.44 - 7.34 (m, 1H), 7.29 (t, *J* = 6.6 Hz, 1H), 7.19 - 6.92 (m, 7H), 4.17 - 2.69 (m, 11H), 2.66 - 2.58 (m, 4H). ¹³C NMR (101 MHz, DMSO-*d*₆) δ 197.9, 168.6, 161.8, 155.4, 150.9, 140.6, 138.6, 138.5, 137.7, 135.5, 135.4, 134.1, 133.8, 131.1, 129.9, 129.6, 128.9, 128.8, 128.7, 127.6, 126.6, 124.8, 123.2, 122.1, 113.4, 50.8, 49.3, 43.7, 34.4, 27.3, 9.5. HRMS (ESI) [M + H] ⁺, theoretically calculated for C₃₇H₃₄N₃O₇S: 664.2123, found 664.2110.

### Example 319

### 3-methyl-5-(N-(2-(4-nicotinoylpiperazin-1-yl)phenyl)-N-phenethylsulfamoyl)benzofuran-2-carboxylic acid (F27-S116)

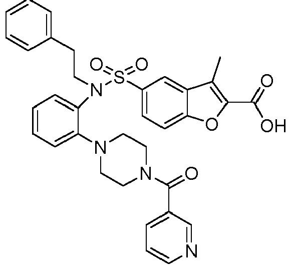

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-(2-(4-nicotinoylpiperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 37.2 mg of white solid was obtained, with a yield of 54%. ¹H NMR (400 MHz, DMSO-*d*₆) δ 8.74 - 8.60 (m, 1H), 8.20 (s, 1H), 7.94 - 7.78 (m, 3H), 7.54 - 7.45 (m, 1H), 7.42 - 7.25 (m, 2H), 7.21 - 6.80 (m, 8H), 4.19 - 2.69 (m, 11H), 2.65 - 2.51 (m, 4H). ¹³C NMR (101 MHz, DMSO-*d*₆) δ 167.3, 161., 155.4, 150.9, 148.1, 145.9, 138.6, 135.5, 135.2, 133.8, 132.1, 131.1, 131.0, 129.9, 129.6, 128.9, 128.7, 126.6, 126.5, 124.8, 123.9, 123.2, 122.0, 113.3, 52.2, 50.8, 49.4, 34.4, 9.5. HRMS (ESI) [M + H] ⁺, theoretically calculated for C₃₄H₃₁N₄O₆S: 623.1970, found 623.1971.

### Example 320

### 3-methyl-5-(N-phenethyl-N-(2-(4-pyridinecarbonylpiperazin-1-yl)phenyl)sulfamoyl)benzofuran-2-carboxylic acid (F27-S117)

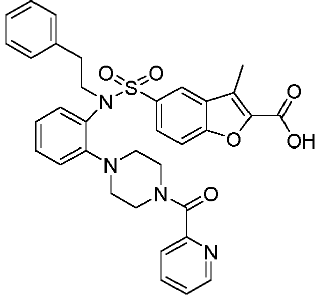

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-phenethyl-N-(2-(4-pyridineformylpiperazin-1-yl)phenyl)sulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 47.1 mg of white solid was obtained, with a yield of 71%. ¹H NMR (400 MHz, DMSO-*d*₆) δ 8.60 (d, *J* = 4.6 Hz, 1H), 8.26 (s, 1H), 8.04 - 7.80 (m, 3H), 7.59 (d, *J* = 7.8 Hz, 1H), 7.49 (dd, *J* = 7.5, 4.9 Hz, 1H), 7.36 (t, *J* = 6.8 Hz, 1H), 7.27 (d, *J* = 7.2 Hz, 1H), 7.21 - 7.03 (m, 5H), 6.98 (d, *J* = 6.9 Hz, 2H), 4.21 - 2.65 (m, 11H), 2.64 - 2.55 (m, 4H). ¹³C NMR (101 MHz, DMSO-*d*₆) δ 167.2, 161.2, 155.6, 154.3, 150.8, 148.8, 144.2, 139.1, 138.6, 137.8, 135.8, 133.7, 131.3, 129.6 (d, *J* = 3.8 Hz), 128.9, 128.7, 127.0, 126.6, 125.3, 125.0, 124.8, 123.6, 123.2, 122.3, 113.5, 52.3 (d, *J* = 81.1 Hz), 50.7, 44.9 (d, *J* = 511.4 Hz), 34.4, 9.5. HRMS (ESI) [M + H] ⁺, theoretically calculated for C₃₄H₃₁N₄O₆S: 623.1970, found 623.1961.

### Example 321

### 3-methyl-5-(N-phenethyl-N-(2-(4-(thiophene-2-carbonyl)piperazin-1-yl)phenyl) sulfamoyl)benzofuran-2-carboxylic acid(F27-S118)

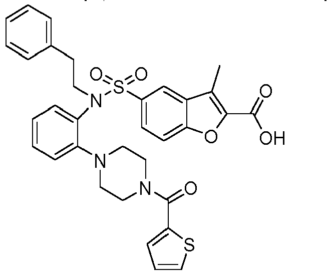

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-(2-(4-(4-(3,3-dimethylbutyryl)piperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 10.6 mg of white solid was obtained, with a yield of 40%. ¹H NMR (400 MHz, DMSO-*d*₆) δ 8.20 (d, *J* = 14.9 Hz, 1H), 7.86 (td, *J* = 8.8, 5.7 Hz, 2H), 7.76 (d, *J* = 5.0 Hz, 1H), 7.37 (dd, *J* = 14.1, 5.8 Hz, 2H), 7.27 (d, *J* = 7.8 Hz, 1H), 7.21 - 6.94 (m, 8H), 4.22 - 3.50 (m, 7H), 3.09 - 2.69 (m, 4H), 2.57 (s, 4H). ¹³C NMR (101 MHz, DMSO-*d*₆) δ 168.1, 162.8, 155.5, 150.8, 145.5, 138.6, 137.5, 135.6, 133.8, 132.0, 131.2, 129.9, 129.6, 129.6, 128.9, 128.7, 127.5, 126.6, 124.8, 123.2, 122.1, 113.4, 110.0, 52.3, 50.8, 34.4, 9.5. HRMS (ESI) [M + H] ⁺, theoretically calculated for C₃₃H₃₀N₃O₆S₂: 628.1582, found 628.1580.

### Example 322

### 3-methyl-5-(N-phenethyl-N-(2-(4-(thiophene-3-carbonyl)piperazin-1-yl)phenyl) sulfamoyl)benzofuran-2-carboxylic acid (F27-S119)

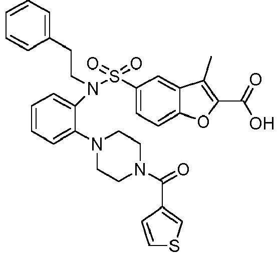

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-phenethyl-N-(2-(4-(thiophene-3-carbonyl)piperazin-1-yl)phenyl)sulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 28.0 mg of white solid was obtained, with a yield of 42%. ¹H NMR (400 MHz, DMSO-*d*₆) δ 8.22 (d, *J* = 1.8 Hz, 1H), 7.94 - 7.82 (m, 2H), 7.79 (d, *J* = 2.8 Hz, 1H), 7.62 (dd, *J* = 5.0, 2.9 Hz, 1H), 7.42 - 7.32 (m, 1H), 7.27 (d, *J* = 8.0 Hz, 1H), 7.21 (d, *J* = 5.0 Hz, 1H), 7.19 - 7.02 (m, 5H), 7.02 - 6.94 (m, 2H), 4.15 - 2.70 (m, 11H), 2.58 (d, *J* = 3.5 Hz, 4H). ¹³C NMR (101 MHz, DMSO-*d*₆) δ 165.0, 161.6, 155.5, 150.9, 138.6, 136.7, 135.6, 133.8, 131.1, 129.9, 128.9, 128.7, 127.8, 127.3, 127.0, 126.6, 124.8, 123.6, 123.2, 122.1, 113.4, 103.5, 52.5, 50.8, 34.4, 9.5. HRMS (ESI) [M + H] ⁺, theoretically calculated for C₃₃H₃₀N₃O₆S₂: 628.1582, found 628.1579.

### Example 323

### (E)-5-(N-(2-(4-(but-2-enoyl)piperazin-1-yl)phenyl)-N-phenethylsulfamoyl)-3-methylbenzofuran-2-carboxylic acid (F27-S120)

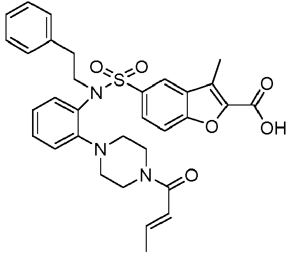

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that (E)ethyl-5-(N-(2-(4-(but-2-enoyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 41.5mg of white solid was obtained, with a yield of 46%. ¹H NMR (400 MHz, DMSO-*d*₆) δ 8.25 (s, 1H), 7.89 (q, *J* = 8.8 Hz, 2H), 7.36 (t, *J* = 7.7 Hz, 1H), 7.25 (d, *J* = 8.0 Hz, 1H), 7.12 (dd, *J* = 13.0, 7.0 Hz, 4H), 7.03 (d, *J* = 7.9 Hz, 1H), 6.97 (d, *J* = 7.3 Hz, 2H), 6.70 (dd, *J* = 14.6, 7.0 Hz, 1H), 6.52 (d, *J* = 15.0 Hz, 1H), 4.16 - 3.54 (m, 6H), 3.20 - 2.67 (m, 5H), 2.59 (s, 4H), 1.85 (d, *J* = 6.5 Hz, 3H). ¹³C NMR (101 MHz, DMSO-*d*₆) δ 164.8, 161.4, 155.5, 151.0, 141.2, 138.6, 135.8, 133.8, 131.1, 129.7, 129.6, 128.9, 128.7, 126.8, 126.6, 124.8, 124.7, 124.3, 123.2, 122.4, 122.3, 113.5, 52.9, 50.8, 34.3, 18.2, 9.5. HRMS (ESI) [M + H] ⁺, theoretically calculated for C₃₂H₃₂N₃O₆S: 586.2017, found 586.2007.

### Example 324

### 5-(N-(2-(4-(furan-2-carbonyl)piperazin-1-yl)phenyl)-N-phenethylsulfamoyl)-3-methylbenzofuran-2-carboxylic acid (F27-S121)

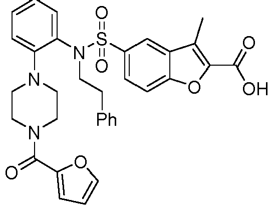

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(furan-2-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 87.2 mg of white solid was obtained, with a yield of 85%. ¹H NMR (400 MHz, DMSO-*d*₆) δ 8.20 (d, *J* = 12.6 Hz, 1H), 7.89 - 7.80 (m, 2H), 7.44 - 7.32 (m, 1H), 7.30 - 7.24 (m, 1H), 7.19 - 6.95 (m, 8H), 6.63 (dd, *J* = 3.4, 1.7 Hz, 1H), 4.15 - 3.52 (m, 6H), 3.19 - 2.72 (m, 5H), 2.64 - 2.54 (m, 4H). ¹³C NMR (101 MHz, DMSO-*d*₆) δ 167.3, 158.8, 155.4, 150.8, 147.4, 145.2, 138.6, 138.6, 134.1, 133.7, 131.2, 130.0, 129.6, 128.9, 128.7, 126.7, 126.6, 124.8, 123.2, 122.0, 122.0, 116.1, 113.3, 111.7, 50.8, 49.3, 43.8, 34.4, 9.5. HRMS (ESI) [M + H] ⁺, theoretically calculated for C₃₃H₃₀N₃O₇S: 612.1810, found 612.1808.

### Example 325

### 5-(N-(2-(4-(cyclopentanecarbonyl)piperazin-1-yl)phenyl)-N-phenethylsulfamoyl)-3-methylbenzofuran-2-carboxylic acid (F27-S122)

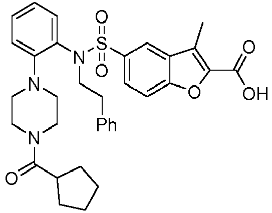

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(cyclopentanecarbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 80.7 mg of white solid was obtained, with a yield of 82%. ¹H NMR (400 MHz, DMSO-*d*₆) δ 13.75 (s, 1H), δ 8.28 (s, 1H), 7.99 - 7.83 (m, 2H), 7.36 (t, *J* = 7.6 Hz, 1H), 7.25 (d, *J* = 9.0 Hz, 1H), 7.19 - 7.02 (m, 5H), 6.97 (d, *J* = 6.7 Hz, 2H), 4.20 - 3.24 (m, 6H), 3.10 - 2.65 (m, 6H), 2.60 (s, 4H), 1.79 - 1.43 (m, 8H). ¹³C NMR (101 MHz, DMSO-*d*₆) δ 173.9, 161.1, 155.6, 151.0, 143.9, 138.6, 136.0, 133.7, 131.2, 129.6, 128.9, 128.7, 127.1, 126.6, 125.2, 124.7, 123.1, 122.4, 113.5, 52.5 (d, *J* = 87.1 Hz), 50.7, 45.8, 42.1, 34.4, 30.0, 26.1, 9.5. HRMS (ESI) [M + H] ⁺, theoretically calculated for C₃₄H₃₆N₃O₆S: 614.2330, found 614.2326.

### Example 326

### 5-(N-(2-(4-(5-bromothiophene-2-carbonyl)piperazin-1-yl)phenyl)-N-phenethylsulfamoyl)-3-methylbenzofuran-2-carboxylic acid (F27-S123)

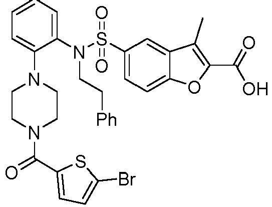

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(5-bromothiophene-2-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 83.3 mg of white solid was obtained, with a yield of 74%. ¹H NMR (400 MHz, DMSO-*d*₆) δ 8.23 (s, 1H), 7.88 (q, *J* = 9.3, 8.7 Hz, 2H), 7.36 (t, *J* = 7.4 Hz, 1H), 7.30 - 7.23 (m, 3H), 7.19 - 6.93 (m, 9H), 4.15 - 3.52 (m, 7H), 3.16 - 2.73 (m, 4H), 2.67 - 2.54 (m, 4H). ¹³C NMR (101 MHz, DMSO-*d*₆) δ 161.5, 161.4, 155.5, 150.9, 139.7, 138.6, 135.7, 133.8, 131.1, 131.0, 130.6, 129.8, 129.7, 128.9, 128.7, 126.8, 126.6, 124.8, 124.1, 123.3, 122.2, 116.1, 113.5, 52.2, 50.9, 34.4, 9.5. HRMS (ESI) [M + H] ⁺, theoretically calculated for C₃₃H₂₉BrN₃O₆S₂: 706.0687, found 706.0677.

### Example 327

### 5-(N-(2-(4-(1H-pyrrole-2-carbonyl)piperazin-1-yl)phenyl)-N-phenethylsulfamoyl)-3-methylbenzofuran-2-carboxylic acid (F27-S124)

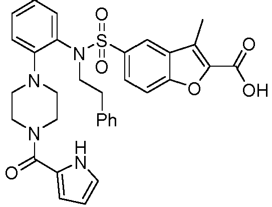

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(1H-pyrrole-2-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 58.6 mg of white solid was obtained, with a yield of 48%. ¹H NMR (400 MHz, DMSO-*d*₆) δ 11.47 (s, 1H), 8.19 (s, 1H), 7.85 (q, *J* = 8.8 Hz, 2H), 7.35 (t, *J* = 6.9 Hz, 1H), 7.25 (d, *J* = 7.9 Hz, 1H), 7.20 - 6.93 (m, 7H), 6.89 (s, 1H), 6.42 (s, 1H), 6.12 (s, 1H), 4.17 - 3.50 (m, 7H), 3.10 - 2.69 (m, 4H), 2.64 - 2.52 (m, 4H). ¹³C NMR (101 MHz, DMSO-*d*₆) δ 162.1, 161.9, 155.3, 150.9, 138.7, 135.4, 133.7, 131.2, 130.2, 129.6, 128.9, 128.7, 126.6, 126.1, 124.6, 124.6, 123.1, 121.8, 121.6, 113.2, 112.3, 108.8, 52.4, 50.7, 45.2, 34.4, 9.5. HRMS (ESI) [M + H] ⁺, theoretically calculated for C₃₃H₃₁N₄O₆S: 611.1970, found 611.1971.

### Example 328

### 5-(N-(2-(4-(3-bromothiophene-2-carbonyl)piperazin-1-yl)phenyl)-N-phenethylsulfamoyl)-3-methylbenzofuran-2-carboxylic acid (F27-S125)

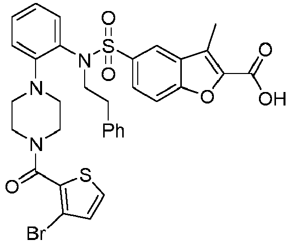

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 74.1 mg of white solid was obtained, with a yield of 80%. ¹H NMR (400 MHz, DMSO-*d*₆) δ 8.24 (s, 1H), 7.86 (q, *J* = 9.5, 8.8 Hz, 2H), 7.79 (d, *J* = 5.2 Hz, 1H), 7.36 (t, *J* = 7.6 Hz, 1H), 7.26 (d, *J* = 7.7 Hz, 1H), 7.11 (ddt, *J* = 18.4, 12.7, 6.4 Hz, 6H), 6.98 (d, *J* = 7.0 Hz, 2H), 4.10 - 3.77 (m, 4H), 3.62 - 2.68 (m, 9H), 2.62 - 2.52 (m, 4H). ¹³C NMR (101 MHz, DMSO-*d*₆) δ 161.6, 161.4, 155.5, 150.7, 138.6, 135.7, 133.7, 132.4, 131.3, 130.3, 129.7, 129.7, 129.3, 128.9, 128.7, 126.9, 126.6, 124.8, 123.2, 122.2, 113.4, 110.0, 109.2, 50.8, 34.4, 9.5. HRMS (ESI) [M + H] ⁺, theoretically calculated for C₃₃H₂₉BrN₃O₆S₂: 706.0687, found 706.0686.

### Example 329

### 5-(N-(2-(4-(3-chlorothiophene-2-carbonyl)piperazin-1-yl)phenyl)-N-phenethylsulfamoyl)-3-methylbenzofuran-2-carboxylic acid (F27-S126)

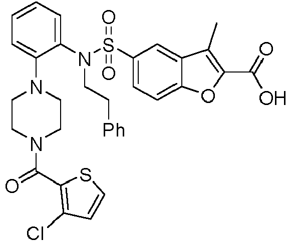

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-chlorothiophene-2-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 69.6 mg of white solid was obtained, with a yield of 75%. ¹H NMR (400 MHz, DMSO-*d*₆) δ 8.19 (d, *J* = 5.2 Hz, 1H), 7.89 - 7.77 (m, 3H), 7.44 - 7.32 (m, 1H), 7.27 (t, *J* = 8.1 Hz, 1H), 7.18 - 6.91 (m, 8H), 4.13 - 2.71 (m, 11H), 2.66 - 2.53 (m, 4H). ¹³C NMR (101 MHz, DMSO-*d*₆) δ 161.9, 161.0, 155.4, 150.7, 150.6, 138.6, 138.6, 135.4, 135.0, 134.1, 133.8, 131.2, 130.4, 130.1, 130.0, 129.8, 129.6, 128.9, 128.7, 127.9, 126.6, 126.4, 125.3, 124.8, 123.2, 121.9, 50.9 (d, *J* = 26.3 Hz), 49.4, 43.7, 34.3 (d, *J* = 11.4 Hz), 9.5. HRMS (ESI) [M + H] ⁺, theoretically calculated for C₃₃H₂₉ClN₃O₆S₂: 662.1192, found 662.1185.

### Example 330

### 5-(N-(2-(4-(4-bromothiophene-2-carbonyl)piperazin-1-yl)phenyl)-N-phenethylsulfamoyl)-3-methylbenzofuran-2-carboxylic acid (F27-S127)

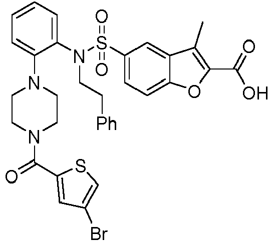

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(4-bromothiophene-2-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 65.3 mg of white solid was obtained, with a yield of 71%. ¹H NMR (400 MHz, DMSO-*d*₆) δ 8.20 (d, *J* = 7.7 Hz, 1H), 7.87 (dd, *J* = 17.3, 6.4 Hz, 3H), 7.49 (s, 1H), 7.42 - 7.32 (m, 1H), 7.28 (d, *J* = 7.7 Hz, 1H), 7.12 (dt, *J* = 12.6, 7.1 Hz, 4H), 7.04 (d, *J* = 7.1 Hz, 1H), 6.98 (d, *J* = 6.9 Hz, 2H), 4.22 - 2.75 (m, 11H), 2.66 - 2.53 (m, 4H). ¹³C NMR (101 MHz, DMSO-*d*₆) δ 161.8, 161.4, 155.4, 151.0, 150.6, 139.1, 138.6, 138.6, 135.4, 135.1, 134.1, 133.9, 131.3, 130.9, 129.9, 129.6, 128.9, 128.7, 128.7, 127.7, 126.6, 126.6, 126.4, 124.8, 123.2, 122.1, 113.4, 108.8, 52.2, 50.9, 49.4, 43.8, 34.3, 34.24, 9.5. HRMS (ESI) [M + H] ⁺, theoretically calculated for C₃₃H₂₉BrN₃O₆S₂: 706.0687, found 706.0675.

### Example 331

### 5-(N-(2-(4-(5-chlorothiophene-2-carbonyl)piperazin-1-yl)phenyl)-N-phenethylsulfamoyl)-3-methylbenzofuran-2-carboxylic acid (F27-S128)

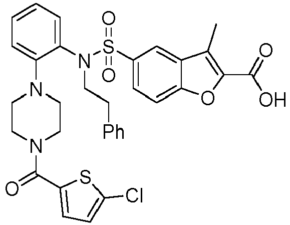

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(5-chlorothiophene-2-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 65.2 mg of white solid was obtained, with a yield of 67%. ¹H NMR (400 MHz, DMSO-*d*₆) δ 8.18 (d, *J* = 7.1 Hz, 1H), 7.84 (d, *J* = 9.1 Hz, 2H), 7.50 - 7.22 (m, 3H), 7.13 (dq, *J* = 12.2, 7.1, 5.1 Hz, 5H), 7.08 - 7.00 (m, 1H), 6.98 (t, *J* = 5.7 Hz, 2H), 4.16 - 3.49 (m, 4H), 3.33 - 2.95 (m, 6H), 2.87 - 2.73 (m, 1H), 2.67 - 2.53 (m, 4H). ¹³C NMR (101 MHz, DMSO-*d*₆) δ 162.2, 161.3, 155.3, 150.7 (d, *J* = 23.0 Hz), 138.6 (d, *J* = 6.8 Hz), 137.0, 135.3, 134.9, 134.1, 133.8, 132.4, 131.0, 130.2, 129.8, 129.6, 128.9, 128.7, 127.6, 126.6, 126.2, 125.3, 123.1, 121.9, 113.2, 51.0(d, *J* = 18.5 Hz), 49.4, 43.7, 34.3 (d, *J* = 15.0 Hz), 9.5. HRMS (ESI) [M + H] ⁺, theoretically calculated for C₃₃H₂₉ClN₃O₆S₂: 662.1192, found 662.1184.

### Example 332

### 3-methyl-5-(N-phenethyl-N-(2-(4-(thiazole-5-carbonyl)piperazin-1-yl)phenyl)sulfamoyl)benzofuran-2-carboxylic acid (F27-S129)

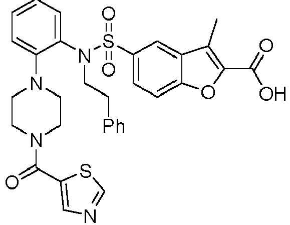

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-phenethyl-N-(2-(4-(thiazole-5-carbonyl)piperazin-1-yl)phenyl)sulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 63.8 mg of white solid was obtained, with a yield of 58%. ¹H NMR (400 MHz, DMSO-*d*₆) δ 9.25 (s, 1H), 8.21 (d, *J* = 4.0 Hz, 1H), 7.92 - 7.81 (m, 2H), 7.43 - 7.33 (m, 1H), 7.28 (d, *J* = 8.0 Hz, 1H), 7.18 - 7.07 (m, 4H), 7.03 (t, *J* = 7.3 Hz, 1H), 6.97 (d, *J* = 6.6 Hz, 2H), 4.13 - 3.58 (m, 4H), 3.35 - 2.97 (m, 6H), 2.84 (s, 1H), 2.65 - 2.54 (m, 4H). ¹³C NMR (101 MHz, DMSO-*d*₆) δ 161.7 (d, *J* = 6.3 Hz), 161.0, 157.1, 155.5, 150.6, 144.2, 138.6, 135.5, 135.2, 134.1, 133.8, 132.8, 130.9, 130.0, 129.9, 129.7, 128.9, 128.7, 128.7, 126.6, 125.3, 123.2, 122.2, 113.4, 51.0, 49.3, 43.7, 34.2, 9.5. HRMS (ESI) [M + H] ⁺, theoretically calculated for C₃₂H₂₉N₄O₆S₂: 629.1534, found 629.1536.

### Example 333

### 5-(N-(2-(4-(1H-pyrazole-3-carbonyl)piperazin-1-yl)phenyl)-N-phenethylsulfamoyl)-3-methylbenzofuran-2-carboxylic acid (F27-S130)

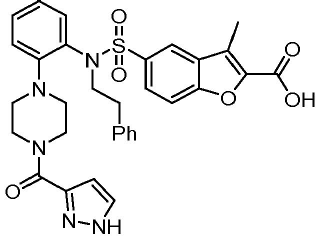

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(1H-pyrazole-3-carbonyl)piperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 49.8 mg of white solid was obtained, with a yield of 61%. ¹H NMR (400 MHz, DMSO-*d*₆) δ 8.28 - 8.22 (m, 1H), 7.96 - 7.82 (m, 2H), 7.78 (d, *J* = 1.9 Hz, 1H), 7.35 (t, *J* = 7.6 Hz, 1H), 7.27 (d, *J* = 7.9 Hz, 1H), 7.18 - 7.01 (m, 5H), 6.97 (d, *J* = 6.8 Hz, 2H), 6.58 (d, *J* = 2.3 Hz, 1H), 4.17 - 3.49 (m, 7H), 3.18 - 3.00 (m, 2H), 2.88 - 2.72 (m, 2H), 2.64 - 2.53 (m, 4H). ¹³C NMR (101 MHz, DMSO-*d*₆) δ 162.5, 161.3, 155.6, 151.0, 144.4, 138.6, 135.8, 133.7, 131.1, 129.7, 129.6, 128.9, 128.7, 126.9, 126.6, 124.7, 124.6, 123.2, 122.3, 113.5, 107.5, 50.7, 34.3, 9.5. HRMS (ESI) [M + H] ⁺, theoretically calculated for C₃₂H₃₀N₅O₆S: 612.1922, found 612.1919.

### Example 334

### 3-methyl-5-(N-(2-(4-(oxazole-5-carbonyl)piperazin-1-yl)phenyl)-N-phenethylsulfamoyl)benzofuran-2-carboxylic acid (F27-S131)

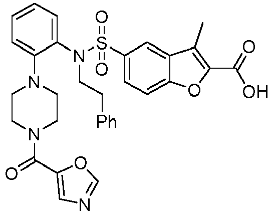

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 3-methyl-5-(N-(2-(4-(oxazole-5-carbonyl)piperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 60.0 mg of white solid was obtained, with a yield of 91%. ¹H NMR (400 MHz, DMSO-*d*₆) δ 8.57 (s, 1H), 8.20 (d, *J* = 7.3 Hz, 1H), 7.86 (q, *J* = 9.7, 9.1 Hz, 2H), 7.73 (s, 1H), 7.45 - 7.32 (m, 1H), 7.30 - 7.23 (m, 1H), 7.17 - 6.88 (m, 7H), 4.16 - 2.92 (m, 10H), 2.87 - 2.69 (m, 1H), 2.65 - 2.53 (m, 4H). ¹³C NMR (101 MHz, DMSO-*d*₆) δ 161.8, 160.3, 157.3, 155.4, 153.6, 150.9, 150.6, 138.6, 138.6, 135.4, 135.1, 134.1, 133.9, 133.9, 130.9, 128.9, 128.7, 126.6, 126.4, 122.0, 113.3, 49.4, 43.8, 34.3, 9.5. HRMS (ESI) [M + H] ⁺, theoretically calculated for C₃₂H₂₉N₄O₇S: 613.1762, found 613.1752.

### Example 335

### 5-(N-(2-(4-(3,4-dimethylbenzoyl)piperazin-1-yl)phenyl)-N-phenethylsulfamoyl)3-methylbenzofuran-2-carboxylic acid (F27-S132)

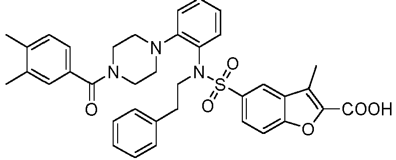

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3,4-Dimethylbenzoyl)piperazin-1-yl)phenyl)-N-phenethylsulfamoyl) 3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 58.1 mg of white solid was obtained, with a yield of 59%. ¹H NMR (400 MHz, DMSO-*d*₆) δ 8.22 (d, *J* = 1.8 Hz, 1H), 7.92 - 7.81 (m, 2H), 7.41 - 7.30 (m, 1H), 7.26 (dd, *J* = 8.1, 1.5 Hz, 1H), 7.22 - 7.02 (m, 8H), 7.00 - 6.94 (m, 2H), 4.26 - 3.40 (m, 6H), 3.26 - 2.57 (m, 6H), 2.56 (s, 3H), 2.25 (s, 6H). ¹³C NMR (101 MHz, DMSO-*d*₆) δ 169.2, 161.0, 155.1, 150.4, 138.2, 137.9, 136.4, 135.2, 133.2, 130.8, 129.3, 129.2, 129.1, 128.5, 128.2, 128.1, 126.4, 126.2, 124.4, 124.3, 122.8, 121.7, 113.0, 50.3, 33.9, 19.3, 9.0. HRMS (ESI) [M + H] ⁺, theoretically calculated for C₃₇H₃₆N₃O₆S: 650.2325, found 650.2327.

### Example 336

### 5-(N-(2-(4-(2,6-dimethoxybenzoyl)piperazin-1-yl)phenyl)-N-phenethylsulfamoyl)3-methylbenzofuran-2-carboxylic acid (F27-S133)

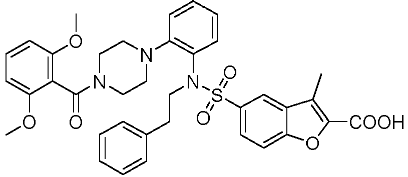

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(2,6-Dimethoxybenzoyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 36.4 mg of white solid was obtained, with a yield of 69%. ¹H NMR (400 MHz, DMSO-*d*₆) δ 8.24 (d, *J* = 1.8 Hz, 1H), 7.92 - 7.80 (m, 2H), 7.41 - 7.26 (m, 2H), 7.22 (d, *J* = 8.0 Hz, 1H), 7.17 - 6.99 (m, 5H), 6.96 (d, *J* = 7.2 Hz, 2H), 6.67 (d, *J* = 8.4 Hz, 2H), 4.23 - 3.60 (m, 10H), 3.17 - 2.67 (m, 6H), 2.64 - 2.51 (m, 5H). ¹³C NMR (101 MHz, DMSO) δ 163.9, 160.7, 156.0, 155.1, 150.3, 138.2, 135.3, 133.0, 130.9, 130.3, 129.1, 128.4, 128.2, 126.6, 126.1, 124.6, 124.1, 122.5, 121.8, 113.9, 113.0, 104.1, 55.7, 52.4, 51.4, 50.1, 46.2, 41.1, 33.9, 9.0. HRMS (ESI) [M + H] ⁺, theoretically calculated for C₃₇H₃₆N₃O₈S: 682.2223, found 682.2219.

### Example 337

### 5-(N-(2-(4-(2-benzylbenzoyl)piperazin-1-yl)phenyl)-N-phenethylsulfamoyl)3-methylbenzofuran-2-carboxylic acid (F27-S134)

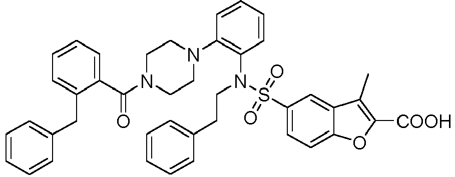

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(2-benzylbenzoyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 38.3 mg of white solid was obtained, with a yield of 80%. ¹H NMR (400 MHz, DMSO-*d*₆) δ 8.05 (d, *J* = 22.4 Hz, 1H), 7.85 - 7.67 (m, 2H), 7.42 - 7.24 (m, 4H), 7.20 - 7.03 (m, 11H), 6.93 (d, *J* = 29.9 Hz, 3H), 4.07 - 3.25 (m, 11H), 3.15 - 2.50 (m, 6H). ¹³C NMR (101 MHz, DMSO) δ 168.5, 154.4, 140.2, 138.2, 138.0, 135.9, 130.6, 130.5, 129.0, 128.9, 128.4, 128.3, 128.2, 126.2, 126.0, 124.5, 120.5, 112.3, 46.5, 41.0, 34.0, 9.0. HRMS (ESI) [M + H] ⁺, theoretically calculated for C₄₂H₃₈N₃O₆S: 712.2481, found 712.2490.

### Example 338

### 5-(N-(2-(4-(2,4,6-trimethylbenzoyl)piperazin-1-yl)phenyl)-N-phenethylsulfamoyl)3-methylbenzofuran-2-carboxylic acid (F27-S135)

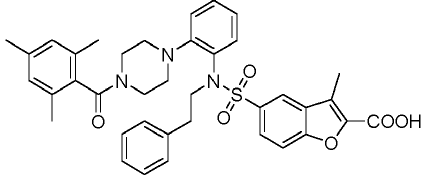

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(2-benzylbenzoyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 39.1 mg of white solid was obtained, with a yield of 74%. ¹H NMR (400 MHz, DMSO-*d*₆) δ 8.20 (d, *J* = 1.8 Hz, 1H), 7.88 - 7.77 (m, 2H), 7.34 (td, *J* = 7.6, 7.1, 1.7 Hz, 1H), 7.25 (dd, *J* = 8.1, 1.6 Hz, 1H), 7.18 - 7.02 (m, 5H), 6.99 - 6.93 (m, 2H), 6.87 (s, 2H), 4.10 - 3.49 (m, 5H), 3.18 - 2.51 (m, 10H), 2.24 (s, 3H), 2.10 (s, 6H). ¹³C NMR (101 MHz, DMSO) δ 168.3, 154.9, 150.4, 138.2, 137.3, 135.1, 133.3, 133.3, 132.9, 130.9, 129.5, 129.2, 128.4, 128.2, 127.9, 126.1, 124.4, 122.7, 121.5, 112.8, 52.5, 51.6, 50.4, 45.7, 40.8, 33.9, 20.6, 18.6, 9.0. HRMS (ESI) [M + H] ⁺, theoretically calculated for C₃₈H₃₈N₃O₆S: 664.2481, found 664.2471.

### Example 339

### 5-(N-(2-(4-(2-methoxybenzoyl)piperazin-1-yl)phenyl)-N-phenethylsulfamoyl)3-methylbenzofuran-2-carboxylic acid (F27-S136)

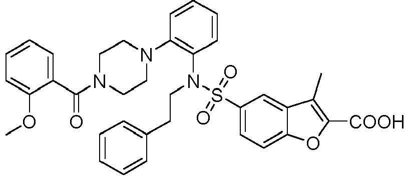

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(2-methoxybenzoyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 46.2 mg of white solid was obtained, with a yield of 73%. ¹H NMR (400 MHz, DMSO-*d*₆) δ 8.23 (d, *J* = 1.9 Hz, 1H), 7.92 - 7.80 (m, 2H), 7.43 - 7.30 (m, 2H), 7.24 (d, *J* = 8.0 Hz, 1H), 7.20 - 7.03 (m, 7H), 6.98 (q, *J* = 7.2 Hz, 3H), 4.13 - 3.83 (m, 2H), 3.81 - 3.33 (m, 7H), 2.93 - 2.51 (m, 8H). ¹³C NMR (101 MHz, DMSO) δ 166.5, 160.8, 155.1, 154.8, 150.4, 138.2, 135.3, 133.1, 130.9, 130.4, 129.2, 128.4, 128.2, 127.6, 126.5, 126.1, 125.5, 124.2, 121.8, 120.6, 113.0, 111.3, 55.4, 51.4, 50.2, 41.4, 33.9, 9.0. HRMS (ESI) [M + H] ⁺, theoretically calculated for C₃₆H₃₄N₃O₇S: 652.2117, found 652.2118.

### Example 340

### 5-(N-(2-(4-(2,5-Dimethylbenzoyl)piperazin-1-yl)phenyl)-N-phenethylsulfamoyl)3-methylbenzofuran-2-carboxylic acid(F27-S137)

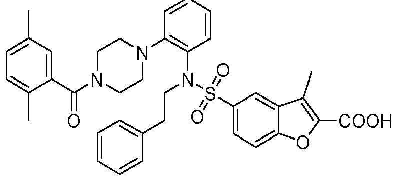

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(2,5-Dimethylbenzoyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 18.3 mg of white solid was obtained, with a yield of 79%. ¹H NMR (400 MHz, DMSO-*d*₆) δ 8.03 (d, *J* = 1.9 Hz, 1H), 7.81 - 7.69 (m, 2H), 7.38 - 7.30 (m, 1H), 7.28 - 7.21 (m, 1H), 7.12 (dq, *J* = 13.8, 7.6, 7.0 Hz, 6H), 7.05 - 6.88 (m, 4H), 4.24 - 3.49 (m, 4H), 3.36 - 2.53 (m, 11H), 2.28 (s, 3H), 2.16 (s, 3H). ¹³C NMR (101 MHz, DMSO) δ 168.7, 154.3, 150.4, 138.2, 136.2, 134.9, 134.0, 130.4, 130.0, 129.2, 129.0, 128.4, 128.2, 126.2, 126.1, 124.1, 120.5, 112.2, 52.0, 51.6, 50.0, 46.6, 41.2, 33.8, 20.4, 18.1, 9.0. HRMS (ESI) [M + H] ⁺, theoretically calculated for C₃₇H₃₆N₃O₆S: 650.2325, found 650.2325.

### Example 341

### 5-(N-(2-(4-(4-(phenylethynyl)benzoyl)piperazin-1-yl)phenyl)-N-phenethylsulfamoyl)3-methylbenzofuran-2-carboxylic acid (F27-S138)

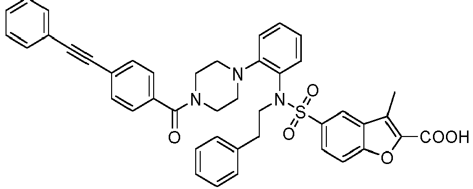

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(4-(phenylethynyl)benzoyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 82.3 mg of white solid was obtained, with a yield of 70%. ¹H NMR (400 MHz, DMSO-*d*₆) δ 8.20 (d, *J* = 1.9 Hz, 1H), 7.91 - 7.79 (m, 2H), 7.69 - 7.54 (m, 4H), 7.50 - 7.40 (m, 5H), 7.35 (td, *J* = 7.7, 1.6 Hz, 1H), 7.27 (dd, *J* = 8.2, 1.5 Hz, 1H), 7.17 - 6.93 (m, 7H), 4.29 - 3.72 (m, 3H), 3.69 - 3.27 (m, 4H), 3.25 - 2.53 (m, 8H). ¹³C NMR (101 MHz, DMSO) δ 168.3, 154.9, 150.5, 138.1, 135.8, 135.0, 133.4, 131.5, 131.4, 130.5, 129.6, 129.2, 129.0, 128.8, 128.4, 128.2, 127.5, 126.2, 125.9, 124.3, 123.4, 122.8, 122.0, 121.5, 112.8, 90.5, 88.7, 50.4, 33.9, 9.0. HRMS (ESI) [M + H] ⁺, theoretically calculated for C₄₃H₃₆N₃O₆S: 722.2325, found 722.2310.

### Example 342

### 5-(N-(2-(4-(5-methylthiophene-2-formyl)piperazin-1-yl)phenyl)-N-phenethylsulfamoyl)3-methylbenzofuran-2-carboxylic acid (F27-S139)

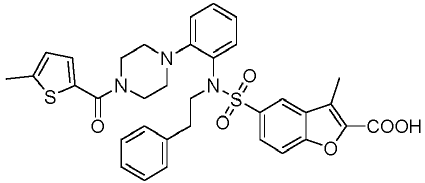

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(5-methylthiophene-2-formyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 76.0 mg of white solid was obtained, with a yield of 83%. ¹H NMR (400 MHz, DMSO-*d*₆) δ 8.24 (d, *J* = 1.9 Hz, 1H), 7.93 - 7.82 (m, 2H), 7.51 - 6.89 (m, 10H), 6.81 (d, *J* = 3.6 Hz, 1H), 3.97 (d, *J* = 74.9 Hz, 2H), 3.62 (d, *J* = 30.4 Hz, 4H), 3.37 - 2.52 (m, 9H), 2.46 (s, 3H). ¹³C NMR (101 MHz, DMSO) δ 162.2, 160.9, 155.1, 150.4, 143.4, 138.2, 135.3, 134.6, 133.3, 130.8, 129.5, 129.3, 129.1, 128.4, 128.2, 126.4, 126.2, 125.5, 124.3, 122.8, 121.7, 113.0, 51.9, 50.3, 34.0, 14.9, 9.0. HRMS (ESI) [M + H] ⁺, theoretically calculated for C₃₄H₃₂N₃O₆S₂: 642.1733, found 642.1740.

### Example 343

### 5-(N-(2-(4-(4-methylthiophene-2-formyl)piperazin-1-yl)phenyl)-N-phenethylsulfamoyl)3-methylbenzofuran-2-carboxylic acid (F27-S140)

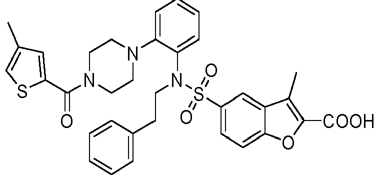

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(4-methylthiophene-2-formyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 57.3 mg of white solid was obtained, with a yield of 77%. ¹H NMR (400 MHz, DMSO-*d*₆) δ 8.21 (d, *J* = 1.9 Hz, 1H), 7.92 - 7.81 (m, 2H), 7.50 - 7.01 (m, 10H), 6.98 (d, *J* = 7.3 Hz, 2H), 4.15 - 3.49 (m, 7H), 3.06 (s, 2H), 2.79 (s, 2H), 2.57 (s, 4H), 2.24 (s, 3H). ¹³C NMR (101 MHz, DMSO) δ 162.3, 155.0, 150.4, 138.2, 137.1, 136.7, 135.1, 133.3, 131.1, 130.6, 129.4, 129.2, 128.4, 128.2, 126.2, 124.6, 124.3, 122.8, 121.6, 112.9, 51.9, 50.4, 33.9, 15.3, 9.0. HRMS (ESI) [M + H] ⁺, theoretically calculated for C₃₄H₃₂N₃O₆S₂: 642.1733, found 642.1736.

### Example 344

### 5-(N-(2-(4-(3-methylthiophene-2-formyl)piperazin-1-yl)phenyl)-N-phenethylsulfamoyl)3-methylbenzofuran-2-carboxylic acid (F27-S141)

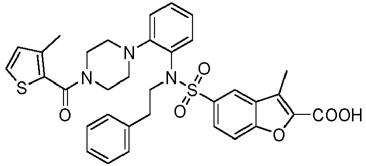

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-methylthiophene-2-formyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 78.4 mg of white solid was obtained, with a yield of 68%. ¹H NMR (400 MHz, DMSO-*d*₆) δ 8.23 (d, *J* = 1.8 Hz, 1H), 7.91 - 7.80 (m, 2H), 7.56 (d, *J* = 5.0 Hz, 1H), 7.39 - 7.31 (m, 1H), 7.26 (dd, *J* = 8.1, 1.5 Hz, 1H), 7.19 - 7.02 (m, 5H), 7.01 - 6.95 (m, 2H), 6.92 (d, *J* = 5.0 Hz, 1H), 4.27 - 3.20 (m, 7H), 3.06 - 2.54 (s, 8H), 2.17 (s, 3H). ¹³C NMR (101 MHz, DMSO) δ 163.4, 160.9, 155.1, 150.3, 138.2, 136.5, 135.2, 133.2, 130.8, 130.2, 129.7, 129.3, 129.1, 128.4, 128.2, 126.5, 126.4, 126.1, 124.3, 122.8, 121.7, 112.9, 51.9, 50.3, 33.9, 14.3, 9.0. HRMS (ESI) [M + H] ⁺, theoretically calculated for C₃₄H₃₂N₃O₆S₂: 642.1733, found 642.1741.

### Example 345

### 5-(N-(2-(4-(1H-imidazole-2-formyl)piperazin-1-yl)phenyl)-N-phenethylsulfamoyl)3-methylbenzofuran-2-carboxylic acid (F27-S142)

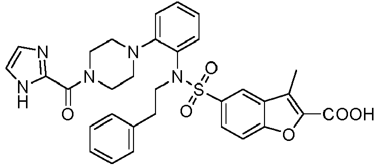

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(1H-imidazole-2-formyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 30.3 mg of white solid was obtained, with a yield of 63%. ¹H NMR (400 MHz, DMSO-*d*₆) δ 12.90 (s, 1H), 8.22 (d, *J* = 16.8 Hz, 1H), 7.96 - 7.79 (m, 2H), 7.74 - 7.45 (m, 1H), 7.42 - 6.89 (m, 10H), 4.48 (s, 1H), 4.17 - 3.58 (m, 3H), 3.54 - 2.57 (m, 11H). ¹³C NMR (101 MHz, DMSO) δ 161.0, 157.4, 155.1, 150.4, 150.1, 140.8, 138.2, 138.1, 133.6, 133.2, 129.4, 129.4, 129.1, 128.4, 128.2, 126.3, 126.2, 126.2, 124.9, 124.2, 121.8, 121.7, 113.0, 50.2, 48.8, 43.3, 33.9, 33.8, 9.1. HRMS (ESI) [M + H] ⁺, theoretically calculated for C₃₂H₃₀N₅O₆S: 612.1917, found 612.1924.

### Example 346

### 5-(N-(2-(4-(tetrahydrothiophene-2-formyl)piperazin-1-yl) phenyl)-N-phenethylsulfamoyl)3-methylbenzofuran-2-carboxylic acid (F27-S143)

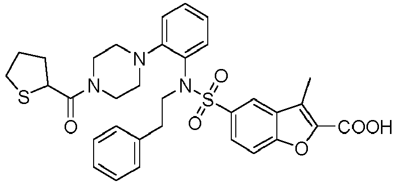

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(tetrahydrothiophene-2-formyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 81.7 mg of white solid was obtained, with a yield of 75%. ¹H NMR (400 MHz, DMSO-*d*₆) δ 13.71 (s, 1H), 8.26 (s, 1H), 7.94 - 7.82 (m, 2H), 7.39 - 7.31 (m, 1H), 7.26 - 7.19 (m, 1H), 7.17 - 7.01 (m, 5H), 7.01 - 6.93 (m, 2H), 4.19 (t, *J* = 6.1 Hz, 1H), 3.96 (d, *J* = 69.3 Hz, 2H), 3.41 (d, *J* = 61.7 Hz, 5H), 3.13 - 2.65 (m, 6H), 2.59 (s, 4H), 2.32 - 1.74 (m, 4H). ¹³C NMR (101 MHz, DMSO) δ 170.1, 160.7, 155.2, 150.4, 143.4, 138.2, 135.5, 133.3, 129.2, 129.2, 128.4, 128.2, 126.6, 126.1, 124.7, 124.3, 122.7, 121.9, 113.1, 51.4, 50.3, 45.7, 44.4, 41.9, 33.9, 32.6, 30.8, 9.1. HRMS (ESI) [M + H] ⁺, theoretically calculated for C₃₃H₃₄N₃O₆S₂: 632.1889, found 632.1891.

### Example 347

### 5-(N-(2-(4-(1H-imidazole-4-formyl)piperazin-1-yl)phenyl)-N-phenethylsulfamoyl)3-methylbenzofuran-2-carboxylic acid (F27-S144)

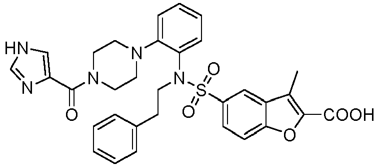

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(1H-imidazole-4-formyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 73.5 mg of white solid was obtained, with a yield of 64%. ¹H NMR (400 MHz, DMSO-*d*₆) δ 13.09 (s, 1H), 8.27 (d, *J* = 1.9 Hz, 1H), 7.96 - 7.75 (m, 3H), 7.64 (s, 1H), 7.38 - 7.30 (m, 1H), 7.25 (d, *J* = 8.0 Hz, 1H), 7.17 - 7.02 (m, 5H), 7.00 - 6.90 (m, 2H), 4.67 - 3.31 (m, 7H), 3.06 (s, 2H), 2.76 (d, *J* = 12.8 Hz, 2H), 2.58 (s, 4H). ¹³C NMR (101 MHz, DMSO) δ 161.7, 160.7, 155.2, 150.5, 143.3, 138.2, 138.1, 135.5, 133.2, 130.7, 129.1, 128.4, 128.2, 126.6, 126.1, 124.8, 124.2, 122.6, 121.9, 113.1, 52.2, 50.2, 33.9, 9.1. HRMS (ESI) [M + H] ⁺, theoretically calculated for C₃₂H₃₀N₅O₆S: 612.1917, found 612.1909.

### Example 348

### 5-(N-(2-(4-(2-ethylbutyryl)piperazin-1-yl)phenyl)-N-phenethylsulfamoyl)3-methylbenzofuran-2-carboxylic acid (F27-S145)

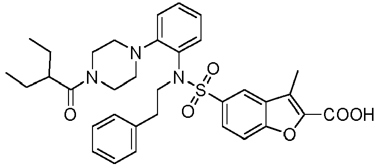

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(2-ethylbutyryl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 88.6 mg of white solid was obtained, with a yield of 81%. ¹H NMR (400 MHz, DMSO-*d*₆) δ 8.25 (d, *J* = 1.9 Hz, 1H), 7.93 - 7.81 (m, 2H), 7.38 - 7.30 (m, 1H), 7.23 (d, *J* = 8.0 Hz, 1H), 7.17 - 7.02 (m, 5H), 7.00 - 6.93 (m, 2H), 4.25 - 3.28 (m, 7H), 2.98 (d, *J* = 42.7 Hz, 2H), 2.70 (s, 2H), 2.63 - 2.55 (m, 5H), 1.57 - 1.42 (m, 2H), 1.41 - 1.28 (m, 2H), 0.78 (t, *J* = 7.4 Hz, 6H). ¹³C NMR (101 MHz, DMSO) δ 173.2, 160.9, 155.1, 150.5, 138.2, 135.4, 133.3, 130.7, 129.3, 129.2, 128.4, 128.2, 126.4, 126.1, 124.3, 122.6, 121.8, 113.0, 52.7, 51.8, 50.3, 45.5, 42.5, 41.5, 33.9, 25.1, 11.7, 9.1. HRMS (ESI) [M + H] ⁺, theoretically calculated for C₃₄H₃₈N₃O₆S: 616.2481, found 616.2484.

### Example 349

### 5-(N-(2-(4-(isoxazole-5-carbonyl)piperazin-1-yl)phenyl)-N-phenethylsulfamoyl)3-methylbenzofuran-2-carboxylic acid (F27-S146)

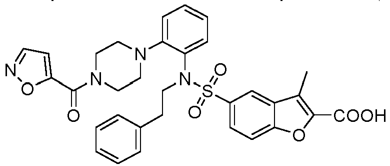

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(isoxazole-5-carbonyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 26.0 mg of white solid was obtained, with a yield of 16%. ¹H NMR (400 MHz, DMSO-*d*₆) δ 13.72 (s, 1H), 8.66 (s, 1H), 8.25 (t, *J* = 5.7 Hz, 1H), 7.86 (td, *J* = 14.4, 12.4, 8.0 Hz, 2H), 7.49 - 6.78 (m, 10H), 4.18 - 3.64 (m, 3H), 3.61 - 3.36 (m, 3H), 3.04 (d, *J* = 32.6 Hz, 2H), 2.76 (d, *J* = 44.1 Hz, 2H), 2.57 (d, *J* = 6.5 Hz, 4H), 2.48 - 2.30 (m, 1H). ¹³C NMR (101 MHz, DMSO) δ 167.2, 160.6, 155.2, 155.2, 155.2, 150.0, 143.3, 138.1, 138.1, 135.4, 135.0, 133.6, 133.4, 128.4, 128.4, 128.2, 128.2, 128.1, 126.1, 124.8, 113.1, 50.6, 50.3, 48.8, 43.5, 33.8, 9.0. HRMS (ESI) [M + H] ⁺, theoretically calculated for C₃₂H₂₉N₄O₇S: 613.1757, found 613.1747.

### Example 350

### 5-(N-(2-(4-(3-bromothiophene-2-carboxamido)piperidin-1-yl)phenyl)-N-phenethylsulfamoyl)-3-methylbenzofuran-2-carboxylic acid (F27-S147)

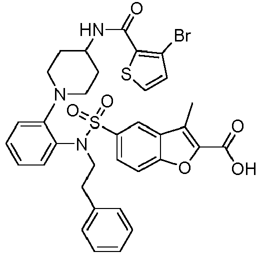

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carboxamido)piperidin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 102.6 mg of white solid was obtained, with a yield of 60%. ¹H NMR (400 MHz, DMSO-*d*₆) δ 8.24 - 8.09 (m, 2H), 7.87 (d, *J* = 8.7 Hz, 1H), 7.82 - 7.76 (m, 2H), 7.39 - 7.30 (m, 1H), 7.26 (d, *J* = 7.9 Hz, 1H), 7.21 - 7.01 (m, 5H), 6.95 (d, *J* = 7.0 Hz, 2H), 4.21 - 2.86 (m, 8H), 2.67 - 2.52 (m, 4H), 2.02 - 1.51 (m, 4H). ¹³C NMR (101 MHz, DMSO-*d*₆) δ 162.7, 160.2, 155.0, 151.5, 138.7, 135.2, 134.1, 133.6, 131.7, 130.9, 130.7, 129.8, 129.4, 128.8, 128.8, 126.7, 125.3, 124.1, 122.8, 121.2, 119.1, 113.0, 110.2, 50.5, 47.2, 34.5, 32.3, 9.5. HRMS (ESI) [M + H] ⁺, theoretically calculated for C₃₄H₃₁BrN₃O₆S₂: 720.0843, found 720.0836.

### Example 351

### 5-(N-(2-(3-bromothiophene-2-carbonyl)-2,8-diazospiro[4.5]decan-8-yl)phenyl)-N-phenethylsulfamoyl)-3-methylbenzofuran-2-carboxylic acid (F27-S148)

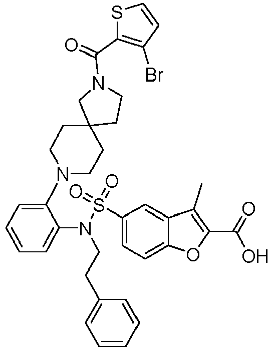

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(3-bromothiophene-2-carbonyl)-2,8-diazospiro[4.5]decan-8-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 51.1 mg of white solid was obtained, with a yield of 54%. ¹H NMR (400 MHz, DMSO-*d*₆) δ 8.26 (s, 1H), 7.97 - 7.81 (m, 2H), 7.77 (d, *J* = 5.0 Hz, 1H), 7.38 - 7.25 (m, 2H), 7.18 - 7.00 (m, 6H), 6.94 (s, 2H), 3.99 (d, *J* = 68.4 Hz, 2H), 3.53 (s, 1H), 3.38 (s, 3H), 3.26 - 2.64 (m, 5H), 2.58 (s, 4H), 1.80 (s, 2H), 1.52 (s, 4H). ¹³C NMR (101 MHz, DMSO-*d*₆) δ 161.3, 155.5, 151.8, 151.6, 144.3, 138.7, 136.2, 133.8, 133.5, 131.3, 130.4, 129.7, 129.5, 128.8, 128.7, 126.9, 126.6, 124.7, 124.3, 123.1, 122.2, 113.4, 108.7, 50.5, 49.9, 46.4, 44.5, 35.2, 34.6, 34.4, 9.5. HRMS (ESI) [M + H] ⁺, theoretically calculated for C₃₇H₃₅BrN₃O₆S₂: 760.1156, found 760.1144.

### Example 352

### 5-(N-(2-(7-(3-bromothiophene-2-carbonyl)-2,7-diazaspiro[3.5]nonan-2-yl)phenyl)-N-phenethylsulfamoyl)-3-methylbenzofuran-2-carboxylic acid (F27-S149)

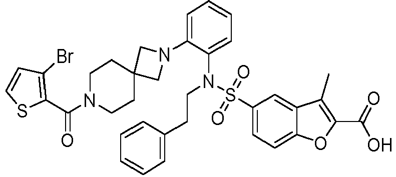

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(7-(3-bromothiophene-2-carbonyl)-2,7-diazaspiro[3.5]nonan-2-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 88.7 mg of white solid was obtained, with a yield of 42%. ¹H NMR (400 MHz, DMSO-*d*₆) δ 8.09 (d, *J* = 8.6 Hz, 1H), 7.85 (d, *J* = 8.8 Hz, 1H), 7.80 - 7.69 (m, 2H), 7.30 - 7.01 (m, 7H), 6.57 - 6.41 (m, 3H), 3.91 - 3.76 (m, 3H), 3.73 - 3.63 (m, 2H), 3.60 - 3.48 (m, 1H), 2.80 - 2.68 (m, 1H), 2.66 - 2.58 (m, 1H), 2.54 (s, 3H). ¹³C NMR (101 MHz, DMSO-*d*₆) δ 161.4, 161.3, 155.6, 148.9, 148.8, 144.5, 138.6, 134.1, 133.9, 133.3, 132.8, 130.3, 129.6, 129.4, 129.1, 128.9, 127.3, 126.8, 123.9, 122.7, 117.1, 114.1, 113.2, 109.0, 62.3, 52.4, 34.5, 34.0, 33.1, 31.8, 9.5. HRMS (ESI) [M + H] ⁺, theoretically calculated for C₃₆H₃₃BrN₃O₆S₂: 746.1000, found 746.0996.

### Example 353

### 5-(N-(2-(3-bromothiophene-2-carbonyl)-2,7-diazaspiro[3.5]nonan-7-yl)phenyl)-N-phenethylsulfamoyl)-3-methylbenzofuran-2-carboxylic acid (F27-S150)

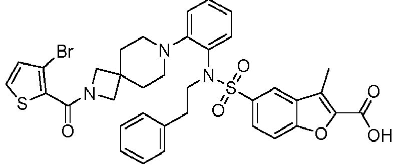

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(3-bromothiophene-2-carbonyl)-2,7-diazaspiro[3.5]nonan-7-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 87.5 mg of white solid was obtained, with a yield of 29%. ¹H NMR (400 MHz, DMSO-*d*₆) δ 8.26 (s, 1H), 7.99 - 7.77 (m, 3H), 7.36 - 7.28 (m, 1H), 7.26 - 7.00 (m, 7H), 6.92 (d, *J* = 6.3 Hz, 2H), 4.21 - 3.52 (m, 9H), 3.15 - 2.83 (m, 2H), 2.71 - 2.53 (m, 4H), 1.88 - 1.63 (m, 4H). ¹³C NMR (101 MHz, DMSO-*d*₆) δ 162.5, 161.1, 155.6, 151.7, 143.8, 138.6, 136.2, 133.4, 131.5, 131.1, 130.5, 130.2, 129.6, 129.5, 128.8, 128.7, 127.0, 126.6, 125.2, 124.4, 123.2, 122.3, 113.5, 111.1, 50.6, 49.7, 35.8, 34.4, 33.8, 9.5. HRMS (ESI) [M + H] ⁺, theoretically calculated for C₃₆H₃₃BrN₃O₆S₂: 746.1000, found 746.0992.

### Example 354

### 5-(N-(2-(6-(3-bromothiophene-2-carbonyl)-3,6-diazabicyclo[3.1.1]heptan-3-yl)phenyl)-N-phenethylsulfamoyl)-3-methylbenzofuran-2-carboxylic acid (F27-S151)

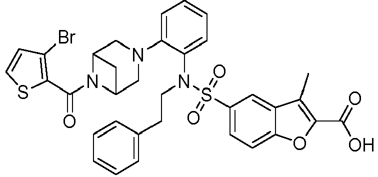

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(6-(3-bromothiophene-2-carbonyl)-3,6-diazabicyclo[3.1.1]heptan-3-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 186.2 mg of white solid was obtained, with a yield of 64%. ¹H NMR (400 MHz, DMSO-*d*₆) δ 8.05 (s, 1H), 7.90 - 7.66 (m, 3H), 7.37 - 6.96 (m, 8H), 6.88 - 6.65 (m, 2H), 4.61 - 4.40 (m, 2H), 4.03 - 3.71 (m, 3H), 3.68 - 3.47 (m, 2H), 2.82 - 2.44 (m, 6H), 1.90 (d, *J* = 8.4 Hz, 1H), 1.25 (d, *J* = 11.6 Hz, 1H). ¹³C NMR (101 MHz, DMSO-*d*₆) δ 162.9, 161.4, 155.6, 149.0, 144.7, 138.5, 133.9, 131.8, 131.2, 130.4, 129.8, 129.6, 129.0, 128.8, 127.2, 126.8, 124.3, 122.6, 121.1, 113.2, 111.3, 110.0, 63.6, 59.9, 51.7, 33.9, 28.5, 9.5. HRMS (ESI) [M + H] ⁺, theoretically calculated for C₃₄H₂₉BrN₃O₆S₂: 718.0687, found 718.0674.

### Example 355

### (S)-5-(N-(2-(4-(3-bromothiophene-2-carbonyl)-2-methylpiperazin-1-yl)phenyl)-N-phenethylsulfamoyl)-3-methylbenzofuran-2-carboxylic acid (F27-S152)

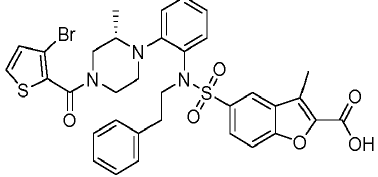

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that (S) ethyl-5-(N-(2-(4-(3-bromothiophene-2-carbonyl)-2-methylpiperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 250.7 mg of white solid was obtained, with a yield of 63%. ¹H NMR (400 MHz, DMSO-*d*₆) δ 8.23 (s, 1H), 8.00 - 7.71 (m, 3H), 7.52 - 6.73 (m, 10H), 4.24 - 2.74 (m, 10H), 2.65 - 2.53 (m, 4H), 0.99 - 0.62 (m, 3H). ¹³C NMR (101 MHz, DMSO-*d*₆) δ 162.3, 161.8, 155.4, 148.7, 145.6, 138.5, 138.4, 135.9, 133.1, 132.6, 132.3, 130.3, 129.9, 129.7, 129.3, 128.8 (d, *J* = 6.3 Hz), 128.6 (d, *J* = 6.6 Hz), 126.6, 125.2, 123.4, 122.1, 115.1, 113.4, 109.3, 53.0, 49.0, 43.6, 34.3, 34.1, 16.2, 9.6. HRMS (ESI) [M + H] ⁺, theoretically calculated for C₃₄H₃₁BrN₃O₆S₂: 720.0843, found 720.0840.

### Example 356

### (R)-5-(N-(2-(4-(3-bromothiophene-2-carbonyl)-2-methylpiperazin-1-yl)phenyl)-N-phenethylsulfamoyl)-3-methylbenzofuran-2-carboxylic acidF27-S153)

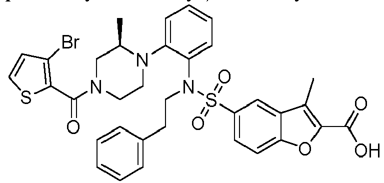

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that (R) ethyl-5-(N-(2-(4-(3-bromothiophene-2-carbonyl)-2-methylpiperazin-1-yl)phenyl)-N-phenethylsulfamoyl)-3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 237.2 mg of white solid was obtained, with a yield of 67%. ¹H NMR (400 MHz, DMSO-*d*₆) δ 8.21 (s, 1H), 7.95 - 7.69 (m, 3H), 7.48 - 6.76 (m, 10H), 4.29 - 2.70 (m, 10H), 2.66 - 2.52 (m, 4H), 0.96 - 0.67 (m, 3H). ¹³C NMR (101 MHz, DMSO-*d*₆) δ 162.0, 161.6, 155.3, 148.7, 146.3, 138.4, 138.3, 134.5, 133.0, 132.1, 130.2, 130.0, 129.3, 128.7, 128.6, 126.5, 126.2, 125.1, 122.6, 121.8 (d, *J* = 18.6 Hz), 113.3, 109.2, 53.0, 50.2, 43.6, 34.3, 34.1, 16.1, 14.9, 9.5. HRMS (ESI) [M + H] ⁺, theoretically calculated for C₃₄H₃₁BrN₃O₆S₂: 720.0843, found 720.0829.

### Example 357

### 5-(N-(2-(4-(3-bromothiophene-2-formyl)-1,4-diazepin-1-yl)phenyl)-N-phenethylsulfamoyl)3-methylbenzofuran-2-carboxylic acid (F27-S154)

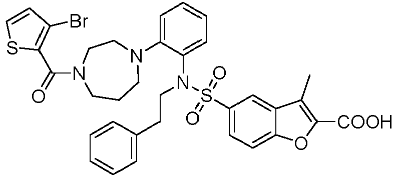

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-formyl)-1,4-diazepin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 577.6 mg of white solid was obtained, with a yield of 85%. ¹H NMR (400 MHz, DMSO-*d*₆) δ 8.19 (d, *J* = 25.8 Hz, 1H), 7.97 - 7.63 (m, 3H), 7.37 - 6.80 (m, 10H), 4.23 - 2.94 (m, 11H), 2.57 (s, 4H), 2.08 - 1.70 (m, 2H). ¹³C NMR (101 MHz, DMSO) δ 162.3, 160.9, 155.1, 138.1, 132.5, 129.7, 129.2, 129.1, 128.5, 128.3, 126.5, 126.2, 123.9, 122.8, 121.9, 113.0, 107.8, 54.6, 50.5, 33.7, 9.1. HRMS (ESI) [M + H] ⁺, theoretically calculated for C₃₄H₃₁N₃O₆S₂Br: 720.0838, found 720.0832.

### Example 358

### 5-(N-(2-(3-(3-bromothiophene-2-formyl)-3,8-diazepine[3.2.1]cyclooctan-8-yl)phenyl)-N-phenethylsulfamoyl)3-methylbenzofuran-2-carboxylic acid (F27-S155)

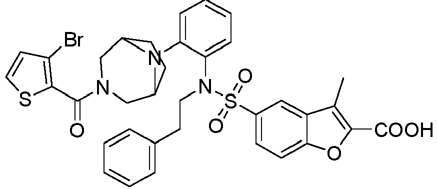

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(3-(3-bromothiophene-2-formyl)-3,8-diazepine[3.2.1]cyclooctan-8-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 99.6 mg of white solid was obtained, with a yield of 69%. ¹H NMR (400 MHz, DMSO-*d*₆) δ 8.24 (s, 1H), 7.93 - 7.81 (m, 2H), 7.78 (d, *J* = 5.2 Hz, 1H), 7.30 - 7.20 (m, 1H), 7.20 - 7.06 (m, 4H), 7.06 - 6.97 (m, 3H), 6.91 - 6.86 (m, 2H), 4.55 - 3.52 (m, 6H), 3.06 (t, *J* = 59.2 Hz, 3H), 2.55 (s, 4H), 2.03 (s, 1H), 1.85 - 1.51 (m, 3H). ¹³C NMR (101 MHz, DMSO) δ 162.8, 161.0, 155.1, 138.1, 134.3, 131.6, 129.9, 129.4, 129.1, 128.7, 128.5, 128.3, 126.4, 126.2, 123.5, 122.2, 119.2, 113.0, 108.6, 56.7, 33.5, 26.3, 9.0. HRMS (ESI) [M + H] ⁺, theoretically calculated for C₃₅H₃₁N₃O₆S₂Br: 732.0838, found 732.0830.

### Example 359

### 5-(N-(2-(4-(3-bromothiophene-2-formyl)-3-methylpiperazin-1-yl)phenyl)-N-phenethylsulfamoyl)3-methylbenzofuran-2-carboxylic acid (F27-S156)

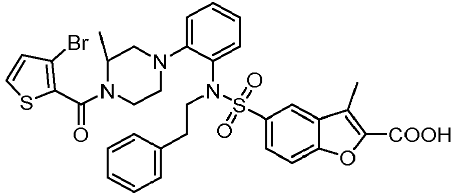

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-formyl)-3-methylpiperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 240.8 mg of white solid was obtained, with a yield of 62%. ¹H NMR (400 MHz, DMSO-*d*₆) δ 8.25 - 8.19 (m, 1H), 7.89 - 7.74 (m, 3H), 7.50 - 7.24 (m, 2H), 7.22 - 6.76 (m, 8H), 4.93 - 3.57 (m, 4H), 3.55 - 3.32 (m, 1H), 3.22 - 2.71 (m, 4H), 2.56 (d, *J* = 8.8 Hz, 4H), 2.50 - 2.36 (m, 1H), 1.36 (dd, *J* = 62.4, 6.8 Hz, 3H). ¹³C NMR (101 MHz, DMSO) δ 161.1, 155.1, 151.1, 150.5, 138.1, 138.0, 135.0, 133.3, 131.9, 129.9, 129.3, 129.2, 128.5, 128.4, 128.3, 128.2, 126.3, 126.2, 124.4, 121.7, 112.9, 108.6, 51.3, 50.3, 33.9, 33.4, 9.0. HRMS (ESI) [M + H] ⁺, theoretically calculated for C₃₄H₃₁ N₃O₆S₂Br: 720.0838, found 720.0833.

### Example 360

### 5-(N-(2-(5-(3-bromothiophene-2-formyl)-2,5-diazabicyclo[2.2.1]hept-2-yl)phenyl)-N-phenethylsulfamoyl)3-methylbenzofuran-2-carboxylic acid (F27-S157)

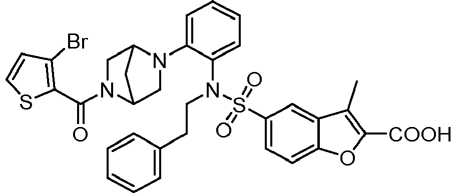

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(5-(3-bromothiophene-2-formyl)-2,5-diazabicyclo[2.2.1]hept-2-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 467.0 mg of white solid was obtained, with a yield of 68%. ¹H NMR (400 MHz, DMSO-*d*₆) δ 13.62 (s, 1H), 8.18 (s, 1H), 7.98 - 7.57 (m, 3H), 7.31 - 6.73 (m, 8H), 6.51 (dd, *J* = 34.7, 19.5 Hz, 2H), 4.96 - 4.10 (m, 2H), 4.04 - 3.06 (m, 7H), 2.76 (s, 1H), 2.45 (s, 3H), 1.81 (d, *J* = 56.1 Hz, 2H). ¹³C NMR (101 MHz, DMSO) δ 160.7, 155.3, 145.7, 143.8, 137.9, 130.1, 129.9, 129.2, 129.0, 128.7, 128.6, 128.4, 128.3, 127.0, 126.4, 126.3, 125.6, 122.5, 122.3, 117.8, 116.6, 116.2, 113.0, 112.7, 61.4, 58.6, 57.0, 52.8, 37.1, 34.1, 32.8, 9.0, 9.0. HRMS (ESI) [M + H] ⁺, theoretically calculated for C₃₄H₂₉N₃O₆S₂Br: 718.0681, found 718.0675.

### Example 361

### 5-(N-(2-(4-(3-bromothiophene-2-carbonyl)piperazin-1-yl)phenyl)-N-phenethylsulfamoyl)-3-toluothiophene-2-carboxylic acid (F27-S158)

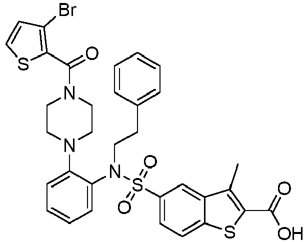

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-phenethylsulfamoyl)-3-toluothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 127.4 mg of white solid was obtained, with a yield of 65%. ¹H NMR (400 MHz, DMSO-*d*₆) δ 8.31 - 8.13 (m, 2H), 7.86 (dd, *J* = 8.5, 1.7 Hz, 1H), 7.80 (d, *J* = 5.2 Hz, 1H), 7.44 - 7.31 (m, 1H), 7.26 (d, *J* = 8.1 Hz, 1H), 7.18 - 7.03 (m, 6H), 7.01 - 6.93 (m, 2H), 4.14 - 2.91 (m, 9H), 2.85 - 2.69 (m, 5H), 2.65 - 2.56 (m, 1H). ¹³C NMR (101 MHz, DMSO-*d*₆) δ 164.6, 161.6, 150.8, 143.7, 140.3, 138.6, 136.9, 133.7, 132.4, 131.2, 130.3, 129.7, 129.3, 128.9, 128.7, 126.6, 124.9, 124.9, 124.6, 123.4, 123.2, 110.0, 109.2, 52.2, 50.8, 34.4, 13.1. HRMS (ESI) [M + H] ⁺, theoretically calculated for C₃₃H₂₉BrN₃O₅S₃: 722.0458, found 722.0453.

### Example 362

### 5-(N-(2-(4-(3-bromothiophene-2-carbonyl)piperazin-1-yl)phenyl)-N-phenethylsulfamoyl)benzo[b]thiophene-2-carboxylic acid (F27-S159)

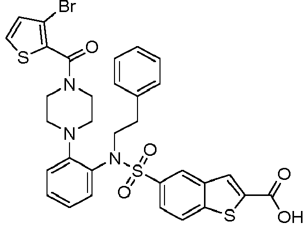

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-phenethylsulfamoyl)benzo[b]thiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 24.9 mg of white solid was obtained, with a yield of 32%. ¹H NMR (400 MHz, DMSO-*d*₆) δ 8.54 (s, 1H), 8.25 (d, *J* = 16.5 Hz, 2H), 7.90 - 7.70 (m, 2H), 7.46 - 6.87 (m, 10H), 4.11 - 2.71 (m, 11H), 2.67 - 2.40 (m, 4H). ¹³C NMR (101 MHz, DMSO-*d*₆) δ 163.8, 161.6, 150.8, 145.4, 139.3, 138.6, 137.2, 133.8, 132.4, 131.3, 130.3, 130.0, 129.7, 129.3, 128.9, 128.7, 126.7, 125.6, 125.0, 124.7, 124.5, 123.3, 109.3, 52.5, 51.0, 34.4. HRMS (ESI) [M + H] ⁺, theoretically calculated for C₃₂H₂₇BrN₃O₅S₃: 708.0302, found 708.0295.

### Example 363

### 6-(N-(2-(4-(3-bromothiophene-2-carbonyl)piperazin-1-yl)phenyl)-N-phenethylsulfamoyl)benzo[b]thiophene-2-carboxylic acid (F27-S160)

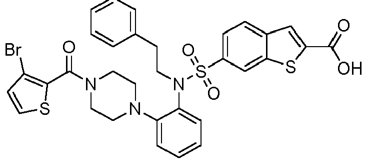

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 6-(N-(2-(4-(3-bromothiophene-2-carbonyl)piperazin-1-yl)phenyl)-N-phenethylsulfamoyl)benzo[b]thiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 23.2 mg of white solid was obtained, with a yield of 51%. ¹H NMR (400 MHz, DMSO-*d*₆) δ 8.64 (s, 1H), 8.21 - 8.06 (m, 2H), 7.86 - 7.69 (m, 2H), 7.42 - 6.84 (m, 10H), 4.18 - 2.68 (m, 10H), 2.64 - 2.43 (m, 5H). ¹³C NMR (101 MHz, DMSO-*d*₆) δ 163.8, 161.6, 154.4, 150.7, 142.4, 141.6, 138.6, 133.7, 132.3, 131.5, 130.3, 129.7, 129.3, 129.0, 128.7, 128.6, 128.6, 126.7, 123.6, 123.5, 123.3, 116.4, 109.2, 53.2, 50.9, 34.5. HRMS (ESI) [M + H] ⁺, theoretically calculated for C₃₂H₂₇BrN₃O₅S₃: 708.0302, found 708.0299.

### Example 364

### 5-(N-(2-(4-(3-bromothiophene-2-carbonyl)piperazin-1-yl)phenyl)-N-phenethylsulfamoyl)-3-chloro-1H-indole-2-carboxylic acid (F27-S161)

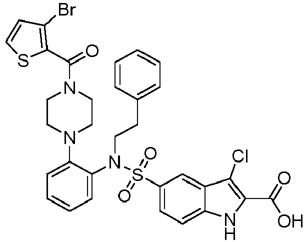

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-phenethylsulfamoyl)-3-chloro-1H-indole-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 98.4 mg of white solid was obtained, with a yield of 57%. ¹H NMR (400 MHz, DMSO-*d*₆) δ 12.48 (s, 1H), 8.01 (s, 1H), 7.86 - 6.57 (m, 13H), 4.18 - 2.57 (m, 12H). ¹³C NMR (101 MHz, DMSO-*d*₆) δ 162.1, 161.6, 150.7, 138.6, 136.5, 134.0, 132.4, 131.0, 130.3, 129.5, 129.3, 128.9, 128.7, 126.7, 125.1, 124.7, 123.8, 123.0, 120.0, 114.5, 113.7, 109.2, 52.2, 51.0, 34.4. HRMS (ESI) [M + H] ⁺, theoretically calculated for C₃₂H₂₇BrClN₄O₅S₂ :725.0300, found 725.0303.

### Example 365

### 5-(N-(2-(4-(3-bromothiophene-2-formyl)piperazin-1-yl)phenyl)-N-phenethylsulfamoyl)benzofuran-2-carboxylic acid (F27-S162)

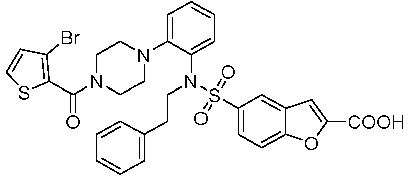

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-formyl)piperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 336.2 mg of white solid was obtained, with a yield of 24%. ¹H NMR (400 MHz, DMSO-*d*₆) δ 8.31 (d, *J* = 1.9 Hz, 1H), 7.93 - 7.83 (m, 2H), 7.79 (d, *J* = 5.2 Hz, 1H), 7.72 (s, 1H), 7.39 - 7.31 (m, 1H), 7.26 (d, *J* = 8.0 Hz, 1H), 7.20 - 7.06 (m, 5H), 7.05 - 6.96 (m, 3H), 4.22 - 3.35 (m, 6H), 3.18 - 2.53 (m, 6H). ¹³C NMR (101 MHz, DMSO) δ 161.2, 159.9, 156.4, 150.3, 138.1, 135.6, 133.3, 131.9, 130.7, 129.8, 129.2, 128.9, 128.5, 128.3, 127.6, 126.2, 126.0, 124.5, 123.5, 122.8, 113.1, 108.8, 50.5, 33.9. HRMS (ESI) [M + H] ⁺, theoretically calculated for C₃₂H₂₇N₃O₆S₂Br: 692.0525, found 692.0532.

### Example 366

### 6-(N-(2-(4-(3-bromothiophene-2-formyl)piperazin-1-yl)phenyl)-N-phenethylsulfamoyl)3-methylbenzofuran-2-carboxylic acid (F27-S163)

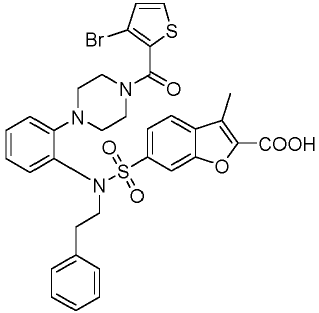

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 6-(N-(2-(4-(3-bromothiophene-2-formyl)piperazin-1-yl)phenyl)-N-phenethylsulfamoyl)3-methylbenzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 71.0 mg of white solid was obtained, with a yield of 80%. ¹H NMR (400 MHz, DMSO-*d*₆) δ 13.77 (s, 1H), 8.12 (d, *J* = 1.5 Hz, 1H), 7.99 (d, *J* = 8.3 Hz, 1H), 7.82 - 7.67 (m, 2H), 7.39 - 7.32 (m, 1H), 7.29 - 7.21 (m, 1H), 7.13 (h, *J* = 6.9 Hz, 5H), 7.05 - 6.94 (m, 3H), 3.96 (d, *J* = 64.6 Hz, 2H), 3.34 (s, 3H), 2.84 (dd, *J* = 80.9, 39.5 Hz, 5H), 2.56 (s, 4H). ¹³C NMR (101 MHz, DMSO) δ 161.1, 160.6, 152.5, 150.3, 144.4, 138.9, 138.1, 133.3, 132.5, 131.9, 130.7, 129.8, 129.2, 128.9, 128.5, 128.2, 126.1, 124.5, 123.9, 122.8, 122.6, 122.0, 111.6, 108.8, 50.4, 33.9, 9.1. HRMS (ESI) [M + H] ⁺, theoretically calculated for C₃₃H₂₉N₃O₆S₂Br: 706.0681, found 706.0682.

### Example 367

### 6-(N-(2-(4-(3-bromothiophene-2-formyl)piperazin-1-yl)phenyl)-N-phenethylsulfamoyl)benzofuran-2-carboxylic acid (F27-S164)

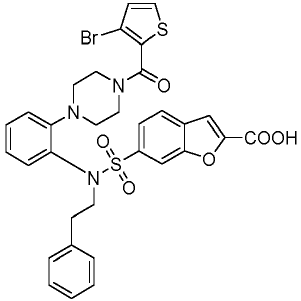

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 6-(N-(2-(4-(3-bromothiophene-2-formyl)piperazin-1-yl)phenyl)-N-phenethylsulfamoyl)benzofuran-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 69.3 mg of white solid was obtained, with a yield of 60%. ¹H NMR (400 MHz, DMSO-*d*₆) δ 8.17 (s, 1H), 7.98 (d, *J* = 8.3 Hz, 1H), 7.79 (d, *J* = 5.1 Hz, 1H), 7.74 (dd, *J* = 8.4, 1.4 Hz, 2H), 7.35 (td, *J* = 7.6, 1.6 Hz, 1H), 7.26 (dd, *J* = 8.4, 1.6 Hz, 1H), 7.18 - 7.06 (m, 5H), 7.05 - 6.95 (m, 3H), 3.96 (d, *J* = 64.4 Hz, 2H), 3.42 (s, 4H), 3.10 - 2.56 (m, 6H). ¹³C NMR (101 MHz, DMSO) δ 161.1, 159.8, 153.8, 150.3, 138.4, 138.1, 133.3, 131.9, 131.0, 130.7, 129.8, 129.2, 128.8, 128.5, 128.2, 126.1, 124.5, 124.0, 122.8, 122.4, 112.4, 111.8, 108.8, 50.4, 33.9. HRMS (ESI) [M + H] ⁺, theoretically calculated for C₃₂H₂₇N₃O₆S₂Br: 692.0525, found 692.0527.

### Example 368

### 5-(N-(2-(4-(3-bromothiophene-2-formyl)piperazin-1-yl)phenyl)-N-(4-methoxyphenethyl)sulfamoyl)3-methylbenzothiophene-2-carboxylic acid (F27-S165)

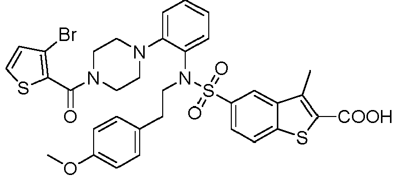

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-formyl)piperazin-1-yl)phenyl)-N-(4-methoxyphenethyl) sulfamoyl)3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 81.9 mg of white solid was obtained, with a yield of 73%. ¹H NMR (400 MHz, DMSO-*d*₆) δ 8.25 - 8.18 (m, 2H), 7.85 (dd, *J* = 8.5, 1.8 Hz, 1H), 7.79 (d, *J* = 5.1 Hz, 1H), 7.40 - 7.32 (m, 1H), 7.26 (dd, *J* = 8.5, 1.8 Hz, 1H), 7.19 - 7.02 (m, 3H), 6.90 - 6.83 (m, 2H), 6.72 - 6.62 (m, 2H), 3.91 (d, *J* = 66.8 Hz, 3H), 3.64 (s, 3H), 3.57 - 3.15 (m, 4H), 2.91 (d, *J* = 84.3 Hz, 4H), 2.72 (s, 3H), 2.42 (s, 1H). ¹³C NMR (101 MHz, DMSO) δ 161.1, 157.6, 150.3, 143.1, 139.8, 136.6, 133.2, 131.9, 130.8, 129.9, 129.8, 129.4, 129.2, 128.8, 124.5, 124.4, 124.1, 122.9, 113.6, 108.8, 54.9, 50.5, 32.9, 12.6. HRMS (ESI) [M + H] ⁺, theoretically calculated for C₃₄H₃₁N₃O₆S₃Br: 752.0558, found 752.0567.

### Example 369

### 5-(N-(2-(4-(3-bromothiophene-2-carbonyl)piperazin-1-yl)phenyl)-N-(4-chlorophenylethyl)sulfamoyl)-3-methylbenzothiophene-2-carboxylic (F27-S166)

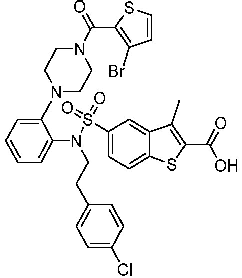

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 21.0 mg of white solid was obtained, with a yield of 65%. ¹H NMR (400 MHz, DMSO-*d*₆) δ 8.28 - 8.12 (m, 2H), 7.87 - 7.73 (m, 2H), 7.36 (t, *J* = 7.4 Hz, 1H), 7.27 (d, *J* = 8.0 Hz, 1H), 7.18 - 7.02 (m, 5H), 6.98 (d, *J* = 8.1 Hz, 2H), 4.15 - 2.90 (m, 9H), 2.85 - 2.68 (m, 5H), 2.65 - 2.55 (m, 1H). ¹³C NMR (101 MHz, DMSO-*d*₆) δ 164.5, 161.6, 150.7, 143.7, 140.3, 137.7, 136.8, 133.6, 132.4, 131.4, 131.3, 130.8, 130.3, 129.7, 129.3, 128.4, 124.9, 124.8, 124.5, 123.3, 118.1, 110.0, 109.2, 50.4, 33.6, 13.1. HRMS (ESI) [M + H] ⁺, theoretically calculated for C₃₃H₂₈BrClN₃O₅S: 756.0068, found 756.0070.

### Example 370

### 5-(N-(2-(4-(3-bromothiophene-2-carbonyl)piperazin-1-yl)phenyl)-N-(4-methylphenylethyl)sulfamoyl)-3-methylbenzothiophene-2-carboxylic acid (F27-S167)

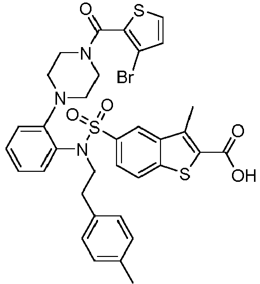

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(4-methylphenylethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 27.1 mg of white solid was obtained, with a yield of 37%. ¹H NMR (400 MHz, DMSO-*d*₆) δ 8.21 (t, *J* = 4.2 Hz, 2H), 7.94 - 7.68 (m, 2H), 7.36 (t, *J* = 7.6 Hz, 1H), 7.27 (d, *J* = 8.0 Hz, 1H), 7.20 - 7.00 (m, 3H), 6.90 - 6.73 (m, 4H), 4.13 - 2.92 (m, 8H), 2.87 - 2.64 (m, 6H), 2.47 - 2.35 (m, 1H), 2.14 (s, 3H). ¹³C NMR (101 MHz, DMSO-*d*₆) δ 164.4, 161.6, 150.8, 143.7, 140.2, 140.0, 137.1, 135.6, 135.4, 133.7, 132.4, 131.3, 130.3, 129.7, 129.3, 129.2, 128.7, 125.1, 124.9, 124.6, 123.5, 123.3, 109.2, 52.3, 50.8, 33.8, 21.0, 13.1. HRMS (ESI) [M + H] ⁺, theoretically calculated for C₃₄H₃₁BrN₃O₅S₃: 736.0615, found 736.0603.

### Example 371

### 5-(N-(2-(4-(3-bromothiophene-2-carbonyl)piperazin-1-yl)phenyl)-N-(4-carboxyphenethyl)sulfamoyl)-3-toluenothiophene-2-carboxylic acid (F27-S168)

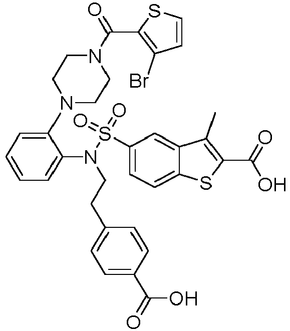

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(4-(methoxycarbonyl)phenethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 58.2 mg of white solid was obtained, with a yield of 76%. ¹H NMR (400 MHz, DMSO-*d*₆) δ 8.30 - 8.15 (m, 2H), 7.86 (dd, *J* = 8.6, 1.9 Hz, 1H), 7.78 (d, *J* = 5.1 Hz, 1H), 7.67 (d, *J* = 8.1 Hz, 2H), 7.41 - 7.32 (m, 1H), 7.30 - 7.22 (m, 1H), 7.17 - 6.98 (m, 5H), 4.23 - 2.90 (m, 8H), 2.86 - 2.65 (m, 6H), 2.61 - 2.54 (m, 1H). ¹³C NMR (101 MHz, DMSO-*d*₆) δ 167.5, 164.2, 161.6, 150.7, 143.9, 143.7, 140.3, 140.1, 137.0, 133.6, 132.4, 131.4, 131.3, 130.3, 129.8, 129.6, 129.3, 129.1, 129.1, 125.2, 125.0, 124.7, 123.6, 123.3, 109.2, 52.4, 50.3, 34.3, 13.2. HRMS (ESI) [M + H] ⁺, theoretically calculated for C₃₅H₃₁BrN₃O₇S₃: 766.0356, found 766.0355.

### Example 372

### 5-(N-(2-(4-(3-bromothiophene-2-carbonyl)piperazin-1-yl)phenyl)-N-(4-cyanophenyl)sulfamoyl)-3-methylbenzothiophene-2-carboxylic acid (F27-S169)

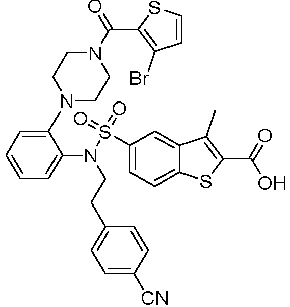

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(4-cyanophenyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 33 mg of white solid was obtained, with a yield of 77%. ¹H NMR (400 MHz, DMSO-*d*₆) δ 13.68 (s, 1H), 8.32 (s, 1H), 8.24 - 8.12 (m, 1H), 7.79 (d, *J* = 9.1 Hz, 2H), 7.68 (d, *J* = 7.8 Hz, 1H), 7.48 (d, *J* = 7.8 Hz, 1H), 7.42 - 7.32 (m, 2H), 7.26 (d, *J* = 7.3 Hz, 1H), 7.17 - 7.06 (m, 4H), 4.21 - 2.89 (m, 6H), 2.80 - 2.71 (m, 9H). ¹³C NMR (101 MHz, DMSO-*d*₆) δ 164.2, 161.4, 150.7, 144.9, 143.5, 140.0, 137.6, 132.4, 132.2, 131.2, 130.3, 130.0, 129.2, 128.8, 127.9, 126.3, 125.7, 124.5, 123.5, 122.9, 122.6, 109.2, 109.1, 52.0, 31.7, 25.8, 13.2 (d, *J* = 6.7 Hz). HRMS (ESI) [M + H] ⁺, theoretically calculated for C₃₄H₂₈BrN₄O₅S₃: 747.0411, found 747.0413.

### Example 373

### 5-(N-(2-(4-(3-bromothiophene-2-carbonyl)piperazin-1-yl)phenyl)-N-(4-(trifluoromethyl)phenethyl)sulfamoyl)-3-methylbenzothiophene-2-carboxylic acid (F27-S170)

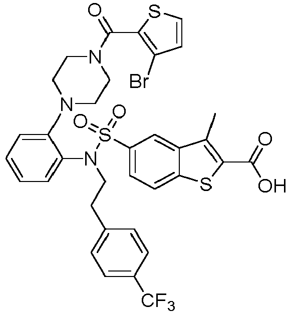

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(4-(trifluoromethyl)phenethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 36 mg of white solid was obtained, with a yield of 72%. ¹H NMR (400 MHz, DMSO-*d*₆) δ 8.17 - 7.95 (m, 2H), 7.72 (dd, *J* = 8.5, 1.8 Hz, 1H), 7.45 (d, *J* = 7.9 Hz, 3H), 7.34 (t, *J* = 7.6 Hz, 1H), 7.25 - 6.84 (m, 6H), 4.02 (d, *J* = 73.5 Hz, 2H), 3.20 - 2.76 (m, 9H), 2.74 - 2.62 (m, 4H). ¹³C NMR (101 MHz, DMSO-*d*₆) δ 165.8, 162.0, 150.9, 144.1, 143.6, 143.3, 141.6, 135.8, 133.6, 131.9, 131.0, 129.7, 129.6, 127.6, 127.2, 126.1, 125.4 (q), 124.5, 123.9, 122.92, 122.7, 122.3, 51.2, 49.9, 44.8, 34.2, 12.5. HRMS (ESI) [M + H] ⁺, theoretically calculated for C₃₄H₂₈BrF₃N₃O₅S₃: 790.0332, found 790.0336.

### Example 374

### 5-(N-(2-(4-(3-bromothiophene-2-carbonyl)piperazin-1-yl)phenyl)-N-(4-(trifluoromethyl)phenethyl)sulfamoyl)-3-methylbenzothiophene-2-carboxylic acid (F27-S171)

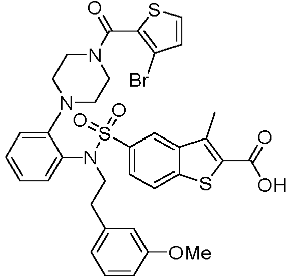

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(3-methoxyphenethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 20.4 mg of white solid was obtained, with a yield of 26%. ¹H NMR (400 MHz, DMSO-*d*₆) δ 8.30 - 8.12 (m, 2H), 7.84 (dd, *J* = 8.6, 1.7 Hz, 1H), 7.79 (d, *J* = 5.2 Hz, 1H), 7.46 - 7.31 (m, 1H), 7.28 - 7.18 (m, 1H), 7.18 - 6.94 (m, 4H), 6.69 - 6.45 (m, 3H), 4.20 - 3.79 (m, 2H), 3.71 - 3.56 (m, 4H), 3.52 - 2.90 (m, 6H), 2.84 - 2.54 (m, 6H). ¹³C NMR (101 MHz, DMSO-*d*₆) δ 164.8, 161.6, 159.6, 150.8, 143.7, 140.4, 140.2, 139.0, 136.9, 133.7, 132.4, 131.2, 130.3, 129.6, 129.3, 124.8, 124.6, 123.4, 123.2, 122.6, 121.1, 114.5, 112.1, 109.2, 55.2, 50.7, 34.7, 13.1. HRMS (ESI) [M + H] ⁺, theoretically calculated for C₃₄H₃₁BrN₃O₆S₃: 752.0564, found 752.0564.

### Example 375

### 5-(N-(2-(4-(3-bromothiophene-2-carbonyl)piperazin-1-yl)phenyl)-N-(3-chlorophenethyl)sulfamoyl)-3-methylbenzothiophene-2-carboxylic acid (F27-S172)

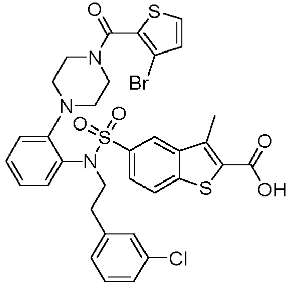

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(3-chlorophenylethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 21.5 mg of white solid was obtained, with a yield of 57%. ¹H NMR (400 MHz, DMSO-*d*₆) δ 8.23 - 8.08 (m, 2H), 7.84 - 7.71 (m, 2H), 7.43 - 7.29 (m, 1H), 7.28 - 7.19 (m, 1H), 7.17 - 7.07 (m, 4H), 7.04 - 6.84 (m, 3H), 4.19 - 2.93 (m, 8H), 2.82 - 2.59 (m, 7H). ¹³C NMR (101 MHz, DMSO-*d*₆) δ 165.8, 161.6, 150.7, 143.5, 141.3, 141.0, 136.2, 133.8, 133.3, 132.4, 131.1, 130.3, 129.6, 129.3, 128.8, 127.6, 126.5, 124.7, 124.3, 123.9, 123.1, 122.8, 118.6, 109.2, 50.3, 33.9, 12.7. HRMS (ESI) [M + H] ⁺, theoretically calculated for C₃₃H₂₈BrClN₃O₅S₃: 756.0068, found 756.0067.

### Example 376

### 5-(N-(2-(4-(3-bromothiophene-2-carbonyl)piperazin-1-yl)phenyl)-N-(2-chlorophenethyl)sulfamoyl)-3-methylbenzothiophene-2-carboxylic acid (F27-S173)

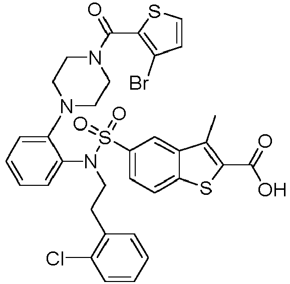

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl-yl)phenyl)-N-(2-chlorophenylethyl) sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 13.5 mg of white solid was obtained, with a yield of 91%. ¹H NMR (400 MHz, DMSO-*d*₆) δ 8.28 - 8.16 (m, 2H), 7.87 (dd, *J* = 8.6, 1.7 Hz, 1H), 7.79 (d, *J* = 5.2 Hz, 1H), 7.41 - 7.34 (m, 1H), 7.26 (d, *J* = 8.3 Hz, 2H), 7.19 - 7.09 (m, 6H), 4.18 - 3.76 (m, 2H), 3.14 - 2.61 (m, 13H). HRMS (ESI) [M + H] ⁺, theoretically calculated for C₃₃H₂₈BrClN₃O₅S₃: 756.0068, found 756.0070.

### Example 377

### 5-(N-(2-(4-(3-bromothiophene-2-carbonyl)piperazin-1-yl)phenyl)-N-(2-methoxyphenethyl)sulfamoyl)-3-methylbenzothiophene-2-carboxylic acid (F27-S174)

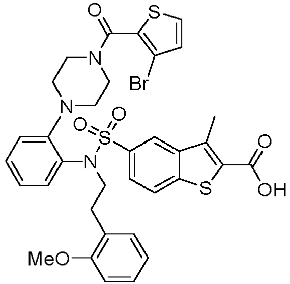

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(2-methoxyphenethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 59.0 mg of white solid was obtained, with a yield of 88%. ¹H NMR (400 MHz, DMSO-*d*₆) δ 8.22 - 8.09 (m, 2H), 7.86 - 7.75 (m, 2H), 7.36 (ddd, *J* = 8.5, 6.8, 2.0 Hz, 1H), 7.26 (dd, *J* = 8.1, 1.5 Hz, 1H), 7.18 - 7.01 (m, 4H), 6.88 (dd, *J* = 7.4, 1.8 Hz, 1H), 6.81 - 6.67 (m, 2H), 4.16 - 3.72 (m, 2H), 3.59 - 3.24 (m, 7H), 3.16 - 2.64 (m, 9H). ¹³C NMR (101 MHz, DMSO-*d*₆) δ 161.6, 159.1, 157.5, 150.7, 143.5, 140.8, 136.7, 133.8, 132.4, 131.1, 130.3, 129.6, 129.3, 128.2, 126.3, 124.7, 124.3, 124.1, 123.0, 122.8, 120.7, 111.0, 109.3, 55.5, 49.0, 29.6, 12.8. HRMS (ESI) [M + H] ⁺, theoretically calculated for C₃₄H₃₁BrN₃O₆S₃: 752.0564, found 752.0566.

### Example 378

### 5-(N-(2-(4-(3-bromothiophene-2-carbonyl)piperazin-1-yl)phenyl)-N-(2-nitrophenylethyl)sulfamoyl)-3-toluothiophene-2-carboxylic acid (F27-S175)

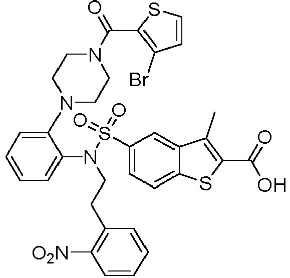

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(2-nitrophenylethyl)sulfamoyl)-3-toluothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 17.0 mg of white solid was obtained, with a yield of 64%. ¹H NMR (400 MHz, DMSO-*d*₆) δ 8.31 (d, *J* = 1.7 Hz, 1H), 8.26 (d, *J=* 8.6 Hz, 1H), 7.91 (dd, *J* = 8.6, 1.8 Hz, 1H), 7.85 - 7.74 (m, 2H), 7.59 - 7.51 (m, 1H), 7.47 - 7.32 (m, 2H), 7.28 (dd, *J* = 12.6, 7.8 Hz, 2H), 7.18 - 7.07 (m, 3H), 4.29 - 3.86 (m, 2H), 3.67 - 2.59 (m, 13H). ¹³C NMR (101 MHz, DMSO-*d*₆) δ 164.3, 161.6, 150.4, 149.4, 143.8, 143.1, 140.2, 137.1, 133.8, 132.9, 132.8, 132.4, 131.2, 130.3, 129.7, 129.3, 128.4, 124.9, 123.5, 123.2, 111.5, 110.0, 109.2, 49.6, 31.9, 13.2. HRMS (ESI) [M + H] ⁺, theoretically calculated for C₃₃H₂₈BrN₄O₇S₃: 767.0309, found 767.0310.

### Example 379

### 5-(N-(2-(4-(3-bromothiophene-2-carbonyl)piperazin-1-yl)phenyl)-N-(3-phenylpropyl)sulfamoyl)-3-methylbenzothiophene-2-carboxylic acid (F27-S176)

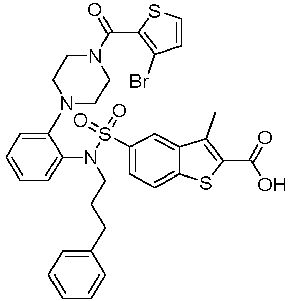

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(3-phenylpropyl) sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 17.0 mg of white solid was obtained, with a yield of 28%. ¹H NMR (400 MHz, DMSO-*d*₆) δ 8.16 - 7.95 (m, 2H), 7.87 - 7.58 (m, 2H), 7.40 - 7.03 (m, 8H), 6.99 - 6.85 (m, 2H), 3.94 - 3.49 (m, 3H), 3.18 - 2.58 (m, 10H), 2.46 - 2.35 (m, 2H), 1.57 - 1.38 (m, 2H). ¹³C NMR (101 MHz, DMSO-*d*₆) δ 165.7, 165.5, 151.0, 149.8, 143.2, 141.7, 141.5, 140.4, 136.8, 136.3, 136.0, 133.9, 131.5, 131.0, 130.1, 129.5, 128.6, 128.5, 126.2, 123.9, 122.9, 122.7, 122.1, 51.5, 45.1, 32.4, 29.7, 12.4. HRMS (ESI) [M + H] ⁺, theoretically calculated for C₃₄H₃₁BrN₃O₅S₃: 736.0615, found 736.0600.

### Example 380

### 5-(N-(2-(4-(3-bromothiophene-2-carbonyl)piperazin-1-yl)phenyl)sulfamoyl)-3-toluothiophene-2-carboxylic acid (F27-S177)

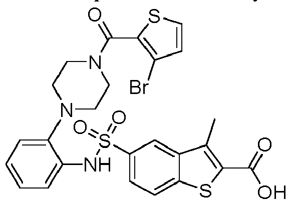

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)sulfamoyl)-3-toluothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 108.0 mg of white solid was obtained, with a yield of 63%. ¹H NMR (400 MHz, DMSO-*d*₆) δ 9.27 (s, 1H), 8.32 (d, *J* = 1.7 Hz, 1H), 8.17 (d, *J* = 8.6 Hz, 1H), 7.84 - 7.65 (m, 2H), 7.50 - 7.32 (m, 1H), 7.22 - 6.98 (m, 4H), 3.89 - 3.00 (m, 4H), 2.69 (s, 3H), 2.49 - 2.26 (m, 4H). ¹³C NMR (101 MHz, DMSO-*d*₆) δ 164.2, 161.4, 144.9, 143.5, 140.0, 137.6, 132.5, 132.4, 131.2, 130.3, 129.2, 126.3, 125.7, 124.5, 123.5, 122.9, 122.6, 109.1, 52.5, 13.1. HRMS (ESI) [M + H] ⁺, theoretically calculated for C₂₅H₂₁BrN₃O₅S₃: 617.9832, found 617.9821.

### Example 381

### 5-(N-(2-(4-(3-bromothiophene-2-formyl)piperazin-1-yl)phenyl)-N-phenethylsulfamoyl)3-methylbenzofuran-2-carboxamide (F27-S178)

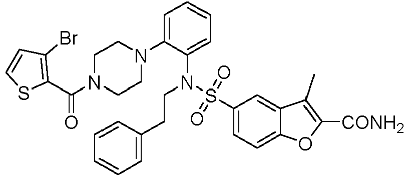

5-(N-(2-(4-(3-bromothiophene-2-carbonyl)piperazin-1-yl)phenyl)-N-phenethylsulfamoyl)-3-methylbenzofuran-2-carboxylic acid (F27-S125) (0.042 mmol, 30.0 mg) and CDI (0.042 mmol, 7.0 mg) were dissolved in 1 mL of DCM and the resulting solution was then stirred at room temperature for 20 min. 0.5 mL of ammonia water was added and then the system was stirred at room temperature for 3 h. After the reaction was found to be completed from the monitoring of the TLC, the reaction was quenched with 0.5 mL of 1M aqueous solution of hydrochloric acid. The product was dissolved in 20 mL of water and then extracted three times with 20 mL of ethyl acetate. The organic phases were combined and then concentrated and then separated by column chromatography (DCM:MeOH = 20:1) to obtain 25.8 mg of white solid, with a yield of 87%. ¹H NMR (400 MHz, Chloroform-*d*) δ 8.11 (s, 1H), 7.88 (d, *J* = 8.8 Hz, 1H), 7.55 (d, *J* = 8.8 Hz, 1H), 7.35 (t, *J* = 5.4 Hz, 2H), 7.21 - 6.87 (m, 7H), 6.57 (s, 1H), 6.11 (s, 1H), 4.30 - 3.75 (m, 3H), 3.44 (d, *J* = 119.4 Hz, 4H), 2.79 (d, *J* = 86.6 Hz, 3H), 2.62 (s, 2H), 1.26 (s, 3H). ¹³C NMR (101 MHz, CDCl₃) δ 162.3, 161.3, 155.0, 151.0, 144.4, 138.2, 136.0, 134.2, 132.1, 130.4, 130.3, 130.1, 129.6, 128.6, 127.4, 126.7, 126.6, 124.8, 123.7, 122.9, 122.0, 112.5, 109.8, 51.6, 34.8, 29.8, 29.4, 9.1. HRMS (ESI) [M + H] ⁺, theoretically calculated for C₃₃H₃₂N₄O₅S₂Br: 707.0997, found 707.1000.

### Example 382

### Ethyl 5-(N-(2-(4-(3-bromothiophene-2-carbonyl)piperazin-1-yl)phenyl)-N-phenethylsulfamoyl)-1,3-dimethyl-1H-indole-2-carboxylate (M10-179)

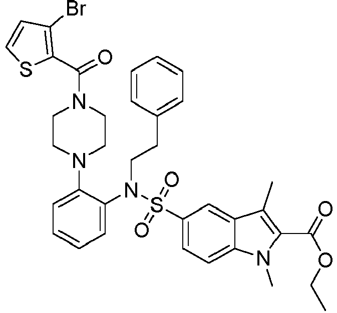

In accordance with the preparation method of ethyl 5-(N-(2-(4-(3-bromothiophene-2-carbonyl)piperazin-1-yl)phenyl)-N-phenethylsulfamoyl)-3-toluothiophene-2-carboxylate (M10-158), this example was implemented under the same condition except that ethyl 5-(chlorosulfonyl)-1,3-dimethyl-1H-indole-2-carboxylate was used instead of ethyl 3-methyl-5-(N-phenethyl-N-(2-(piperazin-1-yl)phenyl)sulfamoyl)benzo[b]thiophene-2-carboxylate. 316.1 mg of white solid was obtained, with a yield of 99%. ¹H NMR (400 MHz, Chloroform-*d*) δ 8.23 (d, *J* = 1.8 Hz, 1H), 7.79 (dd, *J* = 8.9, 1.8 Hz, 1H), 7.44 (d, *J* = 8.8 Hz, 1H), 7.36 (dd, *J* = 12.2, 4.6 Hz, 2H), 7.23 - 7.12 (m, 4H), 7.07 (d, *J* = 4.3 Hz, 2H), 7.01 - 6.95 (m, 3H), 4.47 (q, *J* = 7.1 Hz, 2H), 4.20 - 3.95 (m, 5H), 3.91 - 2.76 (m, 8H), 2.74 - 2.47 (m, 5H). ¹³C NMR (101 MHz, Chloroform-*d*) δ 162.5, 162.2, 150.7, 139.9, 138.3, 134.4, 132.1, 131.5, 130.5, 130.2, 129.2, 128.5, 128.5, 127.2, 126.5, 126.4, 124.4, 123.6, 122.3, 122.3, 121.8, 110.6, 109.6, 60.9, 51.4, 34.7, 32.6, 14.4, 10.9.

### Example 383

### 5-(N-(2-(4-(3-bromothiophene-2-carbonyl)piperazin-1-yl)phenyl)-N-phenethylsulfamoyl)-1,3-dimethyl-1H-indole-2-carboxylic acid (F27-S179)

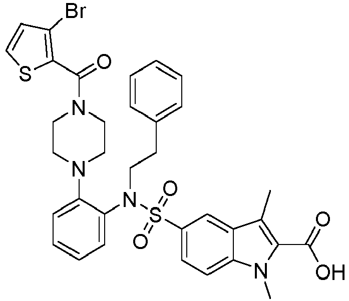

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-phenethylsulfamoyl)-1,3-dimethyl-1H-indole-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 280.5 mg of white solid was obtained, with a yield of 92%. ¹H NMR (400 MHz, DMSO-*d*₆) δ 8.13 (s, 1H), 7.79 - 7.60 (m, 3H), 7.39 - 7.27 (m, 1H), 7.23 - 7.04 (m, 8H), 6.96 (d, *J* = 7.2 Hz, 2H), 4.00 (s, 3H), 3.34 - 2.65 (m, 10H), 2.61 - 2.43 (m, 5H). ¹³C NMR (101 MHz, DMSO-*d*₆) δ 163.9, 161.6, 150.5, 139.8, 138.7, 133.9, 132.3, 1312, 131.1, 130.3, 129.4, 129.3, 128.8, 128.7, 126.6, 126.3, 124.5, 123.3, 122.7, 121.6, 120.2, 112.0, 109.2, 52.1, 50.6, 34.4, 32.8, 10.8. HRMS (ESI) [M - H] -, theoretically calculated for C₃₄H₃₂BrN₄O₅S₂: 719.1003, found 719.0997.

### Example 384

### Ethyl 5-(N-(2-(4-(3-bromothiophene-2-carbonyl)piperazin-1-yl)phenyl)-N-(2-cyclohexylethyl)sulfamoyl)-3-methylbenzothiophene-2-carboxylate (M10-180)

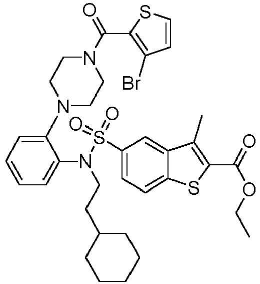

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-((2-cyclohexylethyl)amino)phenyl)piperazin-1-yl)methanone was used instead of (3-bromothien-2-yl)(4-(2-((4-chlorophenylethyl)amino)phenyl)piperazin-1-yl)methanone. 102 mg of white solid was obtained, with a yield of 47%. ¹H NMR (400 MHz, Chloroform-*d*) δ 8.32 (d, *J* = 1.7 Hz, 1H), 8.04 - 7.84 (m, 2H), 7.38 (d, *J* = 5.3 Hz, 1H), 7.34 (ddd, *J* = 8.7, 7.4, 1.6 Hz, 1H), 7.18 (dd, *J* = 8.1, 1.5 Hz, 1H), 7.06 (td, *J* = 7.6, 1.5 Hz, 1H), 7.01 - 6.93 (m, 2H), 4.45 (q, *J* = 7.1 Hz, 2H), 4.26 - 3.39 (m, 6H), 3.01 - 2.71 (m, 6H), 1.64 - 1.53 (m, 4H), 1.50 - 1.40 (m, 4H), 1.16 - 0.99 (m, 5H), 0.75 (q, *J* = 10.6, 9.8 Hz, 2H). ¹³C NMR (101 MHz, Chloroform-*d*) δ 162.8, 162.2, 150.94, 144.0, 140.9, 139.9, 137.3, 134.3, 132.0, 130.2, 130.2, 129.8, 129.3, 127.3, 125.0, 124.6, 123.7, 123.4, 122.7, 109.7, 61.6, 48.2, 35.5, 35.2, 33.1, 26.3, 26.1, 14.4, 13.2.

### Example 385

### 5-(N-(2-(4-(3-bromothiophene-2-carbonyl)piperazin-1-yl)phenyl)-N-(2-cyclohexylethyl)sulfamoyl)-3-methylbenzothiophene-2-carboxylic acid (F27-S180)

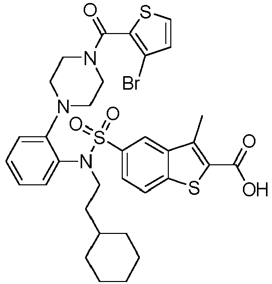

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(2-cyclohexylethyl) sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 73.0 mg of white solid was obtained, with a yield of 72%. ¹H NMR (400 MHz, DMSO-*d*₆) δ 8.27 (t, *J* = 4.2 Hz, 2H), 7.91 (d, *J* = 8.6 Hz, 1H), 7.80 (d, *J* = 5.2 Hz, 1H), 7.29 (dd, *J* = 33.0, 8.3 Hz, 2H), 7.18 - 7.00 (m, 3H), 3.94 - 2.91 (m, 8H), 2.87 - 2.63 (m, 5H), 1.57 - 1.28 (m, 4H), 1.15 - 0.77 (m, 7H), 0.74 - 0.52 (m, 2H). ¹³C NMR (101 MHz, DMSO-*d*₆) δ 164.7, 161.6, 150.8, 143.6, 140.4, 138.9, 137.1, 133.9, 132.4, 131.1, 130.3, 129.6, 129.3, 124.9, 124.6, 123.3, 123.2, 109.2, 52.4, 47.4, 35.4, 34.6, 32.9, 26.4, 26.1, 13.0. HRMS (ESI) [M + H] ⁺, theoretically calculated for C₃₃H₃₅BrN₃O₅S₃: 728.0928, found 728.0918.

### Example 386

### Ethyl 5-(N-benzyl-N-(2-(4-(3-bromothiophene-2-carbonyl)piperazin-1-yl)phenyl)sulfamoyl)-3-methylbenzothiophene-2-carboxylate (M10-181)

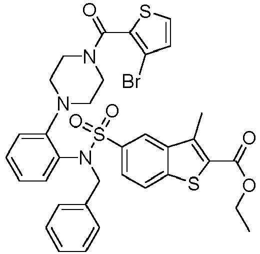

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that 4-(2-(benzylamino)phenyl)piperazin-1-yl)(3-bromothiophen-2-yl)methanone was used instead of (3-bromothien-2-yl)(4-(2-((4-chlorophenylethyl)amino)phenyl)piperazin-1-yl)methanone. 74.0 mg of white solid was obtained, with a yield of 46%. ¹H NMR (400 MHz, Chloroform-*d*) δ 8.24 (d, *J* = 1.6 Hz, 1H), 8.00 - 7.80 (m, 2H), 7.38 (d, *J* = 5.2 Hz, 1H), 7.28 (s, 1H), 7.16 (qd, *J* = 8.0, 7.2, 2.8 Hz, 4H), 7.09 - 6.97 (m, 4H), 6.87 (dd, *J* = 7.9, 1.5 Hz, 1H), 4.81 (d, *J* = 122.3 Hz, 2H), 4.45 (q, *J* = 7.1 Hz, 2H), 4.00 - 3.34 (m, 4H), 3.30 - 2.93 (m, 2H), 2.76 (s, 3H), 2.68 - 2.24 (m, 2H). ¹³C NMR (101 MHz, Chloroform-*d*) δ 162.8, 162.1, 151.4, 144.1, 141.0, 139.9, 137.1, 135.7, 135.1, 132.1, 130.2, 129.8, 129.8, 129.4, 129.4, 128.3, 128.0, 127.2, 125.2, 124.9, 123.9, 123.3, 123.3, 109.7, 61.6, 55.0, 14., 13.2.

### Example 387

### 5-(N-benzyl-N-(2-(4-(3-bromothiophene-2-carbonyl)piperazin-1-yl)phenyl)sulfamoyl)-3-methylbenzothiophene-2-carboxylic acid (F27-S181)

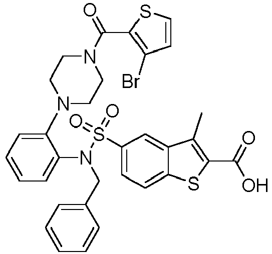

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-benzyl-N-(2-(4-(3-bromothiophene-2-carbonyl)piperazin-1-yl)phenyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 56.0 mg of white solid was obtained, with a yield of 80%. ¹H NMR (400 MHz, DMSO-*d*₆) δ 8.35 - 8.18 (m, 2H), 7.90 (d, *J* = 8.6 Hz, 1H), 7.79 (d, *J* = 5.2 Hz, 1H), 7.40 - 6.83 (m, 10H), 5.18 - 4.68 (m, 2H), 4.14 - 3.07 (m, 6H), 2.95 - 2.58 (m, 7H). ¹³C NMR (101 MHz, DMSO-*d*₆) δ 164.4, 161.6, 150.9, 143.7, 140.2, 137.2, 136.5, 134.3, 132.4, 131.3, 130.3, 129.6, 129.4, 129.3, 128.5, 128.0, 125.3, 125.0, 124.6, 123.7, 123.5, 110.0, 109.2, 53.8, 52.2, 13.2. HRMS (ESI) [M - H] ⁻, theoretically calculated for C₃₂H₂₇BrN₃O₅S₃: 708.0302, found 708.0294.

### Example 388

### Ethyl 5-(N-(2-(4-(3-bromothiophene-2-carbonyl)piperazin-1-yl)phenyl)-N-methylsulfamoyl)-3-methylbenzothiophene-2-carboxylate (M10-182)

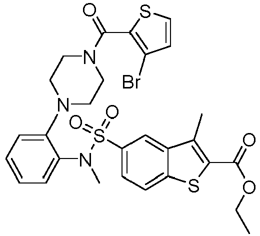

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that (3-bromothien-2-yl)(4-(2-(methylamino)phenyl)piperazin-1-yl)methanone was used instead of (3-bromothien-2-yl)(4-(2-((4-chlorophenylethyl)amino)phenyl)piperazin-1-yl)methanone. 137.7 mg of white solid was obtained, with a yield of 76%. ¹H NMR (400 MHz, Chloroform-*d*) δ 8.24 (s, 1H), 8.07 - 7.69 (m, 2H), 7.50 - 7.17 (m, 2H), 7.14 - 6.66 (m, 4H), 4.43 (q, *J* = 7.2 Hz, 2H), 4.18 - 3.41 (m, 5H), 3.31 - 3.15 (m, 3H), 2.77 (s, 3H), 1.44 (t, *J* = 7.2 Hz, 3H). ¹³C NMR (101 MHz, Chloroform-*d*) δ 162.7, 162.2, 150.3, 144.1, 140.9, 139.9, 135.8, 135.4, 132.1, 130.2, 129.8, 129.3, 128.1, 127.3, 125.1, 123.9, 123.7, 123.4, 121.6, 109.7, 61.6, 51.7, 38.5, 14.4, 13.2.

### Example 389

### 5-(N-(2-(4-(3-bromothiophene-2-carbonyl)piperazin-1-yl)phenyl)-N-methylsulfamoyl)-3-methylbenzothiophene-2-carboxylic acid (F27-S182)

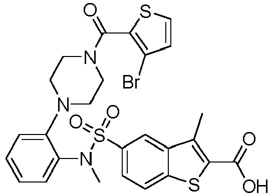

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-methylsulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 78.0 mg of white solid was obtained, with a yield of 58%. ¹H NMR (400 MHz, DMSO-*d*₆) δ 8.26 (d, *J* = 8.5 Hz, 1H), 8.19 (s, 1H), 7.82 (dd, *J* = 15.7, 7.0 Hz, 2H), 7.29 (t, *J* = 7.9 Hz, 1H), 7.15 (q, *J* = 8.7, 5.5 Hz, 2H), 6.97 (dd, *J* = 26.4, 7.9 Hz, 2H), 3.98 - 3.10 (m, 8H), 2.98 (s, 3H), 2.74 (s, 3H). ¹³C NMR (101 MHz, DMSO-*d*₆) δ 164.7, 161.6, 150.3, 143.7, 140.3, 139.1, 135.8, 132.4, 130.3, 129.5, 129.3, 129.0, 125.1, 124.5, 124.0, 123.5, 122.2, 109.2, 51.7, 38.6, 13.1. HRMS (ESI) [M - H] ⁻, theoretically calculated for C₂₆H₂₃BrN₃O₅S₃: 631.9989, found 631.9986.

### Example 390

### Ethyl 5-(N-(2-(4-(3-bromothiophene-2-carbonyl)piperazin-1-yl)phenyl)-N-(2-(thiophen-2-yl)ethyl)sulfamoyl)-3-methylbenzothiophene-2-carboxylate (M10-183)

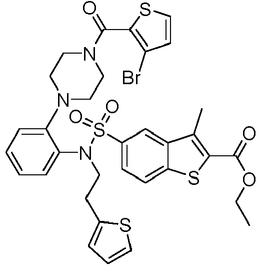

In accordance with the preparation method of ethyl 5-(N-(2-(4-(3-bromothiophene-2-carbonyl)piperazin-1-yl)phenyl)-N-(4-chlorophenylethyl)sulfamoyl)-3-methylbenzothiophene-2-carboxylate (M10-165), this example was implemented under the same condition except that 3-bromothien-2-yl)(4-(2-(thien-2-yl)ethyl)amino)phenyl)piperazin-1-yl)methanone was used instead of (3-bromothien-2-yl)(4-(2-((4-chlorophenylethyl)amino)phenyl)piperazin-1-yl)methanone. 10.0 mg of white solid was obtained, with a yield of 7%. ¹H NMR (400 MHz, Chloroform-*d*) δ 8.31 (s, 1H), 8.04 - 7.82 (m, 2H), 7.45 - 7.33 (m, 2H), 7.26 - 7.15 (m, 2H), 7.13 - 6.95 (m, 3H), 6.87 - 6.78 (m, 1H), 6.65 (s, 1H), 4.46 (q, *J* = 6.6 Hz, 2H), 4.22 - 3.12 (m, 9H), 3.02 - 2.69 (m, 6H), 1.45 (t, 3H). ¹³C NMR (101 MHz, Chloroform-*d*) δ 162.8, 162.2, 150.8, 140.3, 139.9, 137.1, 134.0, 132.0, 130.5, 130.2, 129.9, 129.6, 127.2, 126.9, 125.2, 124.8, 123.8, 123.7, 123.5, 123.2, 122.9, 109.7, 61.6, 51.6, 29.7, 22.7, 14.3.

### Example 391

### 5-(N-(2-(4-(3-bromothiophene-2-carbonyl)piperazin-1-yl)phenyl)-N-(2-(thiophen-2-yl)ethyl)sulfamoyl)-3-methylbenzothiophene-2-carboxylic acid (F27-S183)

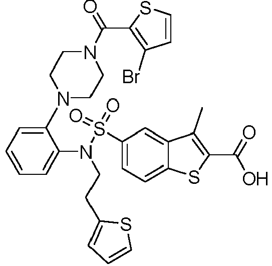

3-methyl-5-(N-(2-(4-(4-methylbenzoyl)piperazin-1-yl)phenyl)-N-phenethylsulfamoyl)benzofuran-2-carboxylic acid (F27-S106), this example was implemented under the same condition except that ethyl 5-(N-(2-(4-(3-bromothiophene-2-carbonyl)piperazin-1-yl)phenyl)-N-(2-(thiophen-2-yl)ethyl)sulfamoyl)-3-methylbenzothiophene-2-carboxylate was used instead of ethyl 3-methyl-5-(N-(2-(4-(4-methylbenzoyl)piperazin-1-yl)phenyl)-N-phenethylsulfamoyl)benzofuran-2-carboxylate. 14.0 mg of white solid was obtained, with a yield of 100%. ¹H NMR (400 MHz, DMSO-*d*₆) δ 8.26 (s, 2H), 7.92 - 7.73 (m, 2H), 7.36 (s, 1H), 7.30 - 7.07 (m, 4H), 6.97 (s, 1H), 6.82 (s, 1H), 6.74 (s, 1H), 4.22 - 3.37 (m, 5H), 3.09 - 2.67 (m, 10H). HRMS (ESI) [M - H] -, theoretically calculated for C₃₁H₂₇BrN₃O₅S₄: 728.0022, found 728.0023.

### Example 392

### N-(2-(4-(3-bromothiophene-2-formyl)piperazin-1-yl)phenyl)-2-hydroxymethyl-3-methyl-N-phenethylbenzofuran-5-sulfonamide (F27-S184)

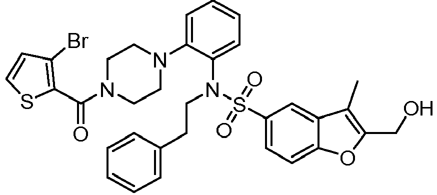

Ethyl 3-methyl-5-(N-(2-(4-(benzoylpiperazin-1-yl)phenyl)-N-phenethylsulfamoyl)benzofuran-2-carboxylate (M10-96) (248 mg, 0.34 mmol) was dissolved in anhydrous tetrahydrofuran (0.5 mL), and the resulting solution was then slowly added dropwise to a stirred lithium aluminum hydride-tetrahydrofuran solution (14 mg of lithium aluminum hydride dissolved in 1 mL of anhydrous tetrahydrofuran) at 0 °C to react at room temperature for 2 h, until it was found from the monitoring of the TLC that there was no raw material left. 1.5 mL of saturated potassium carbonate solution was added dropwise to the reaction system and the system was then stirred for 15 min. The reaction system was then poured into 5 mL of ethyl acetate and stirred for 15 min. The product was dissolved in 20 mL of water and then extracted three times, and the organic layer was then dried with anhydrous sodium sulfate. Column chromatography (PE:EA=3:1~1:1) was carried out to obtain 105 mg of the target product, with a yield of 45%. ¹H NMR (400 MHz, CDCl₃) δ 8.05 (d, *J* = 2.0 Hz, 1H), 7.81 (dd, *J* = 8.7, 2.0 Hz, 1H), 7.61 - 7.53 (m, 2H), 7.37 (dd, *J* = 9.7, 6.2 Hz, 2H), 7.24 - 7.14 (m, 5H), 7.03 - 6.93 (m, 3H), 5.49 (s, 2H), 4.32 - 2.48 (m, 14H), 2.39 (s, 3H). ¹³C NMR (101 MHz, CDCl₃) δ 162.2, 151.0, 149.4, 138.2, 135.0, 134.3, 133.6, 133.2, 132.1, 131.9, 130.2, 129.8, 129.4, 128.5, 127.2, 126.5, 124.8, 124.6, 122.7, 120.8, 116.8, 112.0, 111.9, 109.7, 63.5, 56.9, 51.5, 34.6, 8.0. HRMS (ESI) [M - H] -, theoretically calculated for C₃₃H₃₁BrN₃O₅S₂: 692.0894, found 692.0898.

### Preparation Example 1

### 5-(2,5-dimethylphenoxy)-N-(2-(4-hydroxyphenyl)-2-oxoethyl)-2,2-dimethylpentanamide (SIPI-7623) (SIPI-7623)

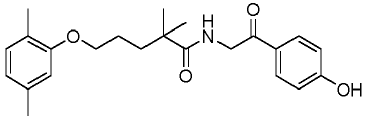

2-amino-4'-hydroxyacetophenone hydrochloride (0.38 g, 2 mmol), 2,2-dimethyl-5-(2,5-dimethylphenoxy)pentanoic acid (0.5 g, 2 mmol), triethylamine (0.6 g, 4 mmol), 1-(3-dimethylaminopropyl)-3-ethylcarbodiimide hydrochloride (0.77g, 4 mmol), and 1-hydroxybenzotriazole (0.54 g, 4 mmol) were added to anhydrous dichloromethane (10 mL) and reacted at room temperature for 13 h. After the reaction was found to be completed from the monitoring of the TLC, the system was washed with saturated sodium carbonate solution (10 mL), and extraction with ethyl acetate (30 mL) was carried out three times. The organic phase was washed with saturated aqueous NaCl solution and then dried with anhydrous sodium sulfate. The crude product was separated by column chromatography (PE/EA=1/1) to obtain 0.39 g of white solid, with a yield of 51%. After verification according to NMR and mass spectrometry data, the compound obtained in this preparation example was consistent with the compound reported!

### Preparation Example 2

### 1-((3S, 8R, 9S, 10R, 13S, 14S)-3-hydroxy-10,13-dimethyl-2,3,4,7,8,9,10,11,12,13,14,15-dodecahydro-1H-cyclopenta[a]phenanthren-17-yl)ethan-1-one (Centatin)

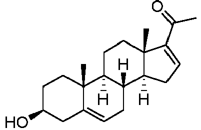

(3S, 8R, 9S, 10R, 13S, 14S)-17-acetyl-10,13-dimethyl-2,3,4,7,8,9,10,11,12,13,14,15-dodecahydro-1*H*-cyclopenta[a]phenanthrene-3-acetate (5.34 g, 15 mmol) was dissolved in tertbutanol (125 mL), and the aqueous solution (5 mL) of potassium hydroxide (4.2 g, 75 mmol) was then added, and the resulting solution was stirred overnight at 30 °C. After the reaction was found to be completed from the monitoring of the TLC, the solvent was distilled off under reduced pressure, and the product was washed five times with ice water (25 mL), and then recrystallized with DCM/CH₃OH (9:1) to obtain 4.58 g of white solid, with a yield of 97%. After verification according to NMR and mass spectrometry data, the compound obtained in this preparation example was consistent with the compound reported!

### Bioactivity

### 1. Experimental methods for bioactivity evaluation

### 1.1 Evaluation on FXR activity

FXR activity was evaluated by time-resolved fluorescence resonance energy transfer (TR-FRET) assay, using LanthaScreen^{™} TR-FRET FXR co-activation assay kit (ThermoFisher, Cat. PV4833). For agonist screening, 10 µL of Complete Coregulator buffer G containing the corresponding concentration of the compound to be tested, 5 µL of 20 nM FXR-LBD Complete Coregulator buffer G, and 5 µL of 2.0 µM Fluorescein-SRC2-2/20 nM Tb-anti-GST antibody Complete Coregulator buffer G were added to each well of a 384-well black plate (Coring) in sequence. After incubation at room temperature for 1 h, the fluorescence values at 520 nm and 495 nm were detected by a microplate reader, and the obtained values were expressed in 520 nm/495 nm. Three duplicate wells were set up for each compound, with CDCA as the positive compound and DMSO as the solvent control. For antagonist screening, 5 µL of Complete Coregulator buffer G containing the corresponding concentration of the compound to be tested, 5 µL of 20 nM FXR-LBD Complete Coregulator buffer G, 5 µL of 200 µM CDCA FXR-LBD Complete Coregulator buffer G, and 5 µL of 2.0 µM Fluorescein-SRC2-2/20 nM Tb-anti-GST antibody Complete Coregulator buffer G were added to each well of a 384-well black plate (Coring) in sequence and incubated at room temperature for 10 h. Following operations were the same as those for agonist screening

### 1.2. Cell culture and passaging

The human embryonic kidney cell line HEK293Awas subcultured in a medium containing penicillin (final concentration: 100 U/mL), streptomycin (final concentration: 100 µg/mL) and 10% FBS, in a humidified atmosphere of 95% air and 5% carbon dioxide at a constant temperature of 37°C. When the cells were 90% confluent, the old culture medium was discarded and the cells were washed twice with 2 mL of PBS. After the PBS was discarded, 2 mL of 0.25% trypsin-0.25% EDTA mixed digestion solution was added. When it was observed under a microscope that the cells became round, 2 mL of complete culture medium was added quickly to stop the digestion. The cell solution was pipetted gently to collect cells. The cells were centrifuged at 800 rpm, 4 °C for 5 min, the supernatant was discarded, the cells were resuspended in complete culture medium and cultured in bottles, and the medium was changed every other day.

The human liver cancer cell line HepG2 was subcultured in a DMEM medium containing penicillin (final concentration: 100 U/mL), streptomycin (final concentration: 100 µg/mL) and 10% FBS under the same conditions as those in the subculture of HEK293A.

### 1.3 Cell proliferation inhibition assay

The cell proliferation inhibition ability of compounds was tested by MTT assay. HepG2 and HEK293 cells were inoculated in a 96-well plate at a density of 30%. After incubation for 18 h to allow the cells to adhere, the original culture medium was removed, a given concentration of the compound to be tested was added to each well, and an equal amount of DMSO was added to the control group. Three replicate wells were set up for each compound. 48 hours after administration, cell viability was tested by MTT assay. The blank group was the wells where no cells were added, and then its OD value was read at 490 nm with a microplate reader. Calculation formula for cell viability: cell viability (%) = (OD_{to be tested} - OD_{blank}) / (OD_{control} - OD_{blank}) × 100.

### 1.4 Testing of TC/TG in cells

Cell TC/TG was tested using Nanjing Jiancheng TG test kit and TC test kit. HepG2 cells were incubated with 0.5 mmol/L FFA (oleic acid and palmitate mixed at a ratio of 2:1) and a solvent or different concentrations (20 µM, 40 µM, 80 µM) of compounds for 24 h. The culture medium was discarded and the cells were washed once with 0.01 M PBS (pH 7.4). The cells were scraped off with a cell scraper. 2-5 mL of 0.01 M PBS (pH 7.4) was then added. The cell suspension was collected and then centrifuged at 1000 × g, 4 °C for 10 min. According to the ratio of 10⁶ cells/ 300-500 µL of homogenization medium, isopropyl alcohol was added for mechanical homogenization, such that the cells were thoroughly disrupted until there was no obvious cell precipitation. The cell solution was centrifuged at 10000 × g, 4 °C for 10 min. The supernatant was taken and placed on ice for testing. 2.5 µL of distilled water, standard products or samples were well mixed with 250 µL of distilled water respectively. After incubation at 37 °C for 10 min, the absorbance value of each well was tested with a microplate reader at 510 nm. Calculation formula for TC/TG concentration: sample concentration (mmol/g protein) = (ODₛₐₘₚₗₑ - OD_{blank}) / (OD_{standard} - OD_{blank}) × standard product concentration (mmol/L) /protein concentration (g protein /L).

### BCA protein quantification

Preparation of protein standard: a. 1.2 mL of standard protein solution was added to a tube of protein standard (30 mg BSA), and a 25 mg/mL standard protein solution was obtained after the protein standard was thoroughly dissolved. **b.** An appropriate amount of 25 mg/mL protein standard was diluted to the final concentration of 0.5 mg/mL. Preparation of BCA working solution: according to the number of samples, reagent A for the BCA working solution and reagent B for BCA working solution (v/v: 50:1) were well mixed to prepare an appropriate amount of BCA working solution. Determination of protein concentration: **a.** 0, 1, 2, 4, 8, 12, 16, and 20 µL of the protein standard were respectively added into the standard wells of the 96-well plate, and the diluted standard solution was then added to make up to 20 µL, which is equivalent to obtaining standard concentrations of 0, 0.025, 0.05, 0.1, 0.2, 0.3, 0.4, and 0.5 mg/mL, respectively. **b.** An appropriate volume of sample was added to the sample wells of the 96-well plate. If the sample is less than 20 µL, the diluted standard solution should be added to make up to 20 µL. **c.** 20 µL of BCA working solution was added to each well and the well plate was set still at 37 °C for 20-30 min. **d.** The absorbance at A562 nm was determined with a microplate reader. **e.** The protein concentrations of the samples were calculated according to the standard curves and the sample volumes used.

### 2 Conclusion and discussion

Conclusion and discussion on FXR antagonistic activity
A: 0.1 µM < IC₅₀ < 10 µM
B: 10 µM < IC₅₀ < 50 µM
C: 50 µM < IC₅₀ < 100 µM

**Table 1.1 FXR antagonistic activity of compounds**

| Compound number | FXR IC₅₀ (µM)*^{a}* |
|---|---|
| Z-GS*^{a}* | C |
| SIPI-7623*^{a}* | B |
| Centatin*^{a}* | B |
| F27 | B |
| F27-S8 | C |
| M8-10 | C |
| F27-S10 | C |
| M8-11 | C |
| F27-S11 | A |
| M8-12 | C |
| F27-S12 | A |
| M8-13 | C |
| F27-S13 | B |
| M4-14 | C |
| F27-S14 | A |
| M4-15 | C |
| F27-S15 | B |
| M4-16 | C |
| F27-S16 | B |
| M4-17 | C |
| F27-S17 | B |
| M4-18 | C |
| F27-S18 | B |
| M4-19 | C |
| F27-S19 | B |
| M4-20 | C |
| F27-S20 | B |
| M4-21 | C |
| F27-S21 | B |
| M4-22 | C |
| F27-S22 | B |
| M4-23 | C |
| F27-S23 | B |
| M4-24 | C |
| F27-S24 | C |
| M4-25 | C |
| F27-S25 | B |
| M4-26 | C |
| F27-S26 | B |
| M4-27 | C |
| F27-S27 | B |
| M4-28 | C |
| F27-S28 | B |
| M4-29 | C |
| F27-S29 | B |
| M4-30 | C |
| F27-S30 | B |
| M4-31 | C |
| F27-S31 | B |
| M4-32 | C |
| F27-S32 | B |
| M4-33 | C |
| F27-S33 | C |
| M4-34 | C |
| F27-S34 | B |
| M4-35 | C |
| F27-S35 | B |
| M4-36 | C |
| F27-S36 | B |
| M4-37 | C |
| F27-S37 | B |
| M4-38 | C |
| F27-S38 | C |
| M4-39 | C |
| F27-S39 | C |
| M4-40 | C |
| F27-S40 | B |
| M4-41 | C |
| F27-S41 | C |
| M4-42 | C |
| F27-S42 | C |
| M4-43 | C |
| F27-S43 | B |
| M4-44 | C |
| F27-S44 | B |
| M4-45 | C |
| F27-S45 | B |
| M4-46 | C |
| F27-S46 | B |
| M4-47 | C |
| F27-S47 | A |
| M4-48 | C |
| F27-S48 | B |
| M4-49 | C |
| F27-S49 | A |
| M4-50 | C |
| F27-S50 | A |
| M4-51 | C |
| F27-S51 | B |
| M4-52 | C |
| F27-S52 | B |
| M4-53 | C |
| F27-S53 | B |
| M4-54 | C |
| M4-55 | C |
| F27-S55 | C |
| M4-56 | C |
| F27-S56 | B |
| M4-57 | C |
| F27-S57 | B |
| M4-58 | C |
| F27-S58 | C |
| M4-59 | C |
| F27-S59 | C |
| M4-60 | C |
| F27-S60 | B |
| M4-61 | C |
| F27-S61 | B |
| M4-62 | C |
| F27-S62 | B |
| M8-63 | C |
| F27-S63 | C |
| M8-64 | C |
| F27-S64 | C |
| M8-65 | C |
| F27-S65 | B |
| M8-66 | C |
| F27-S66 | B |
| M8-67 | C |
| F27-S67 | B |
| M8-68 | C |
| F27-S68 | C |
| M8-69 | C |
| F27-S69 | B |
| M8-70 | C |
| F27-S70 | B |
| M8-71 | C |
| F27-S71 | C |
| M8-72 | C |
| F27-S72 | C |
| M8-73 | C |
| F27-S73 | A |
| M8-74 | C |
| F27-S74 | A |
| M8-75 | C |
| F27-S75 | B |
| M8-76 | C |
| F27-S76 | B |
| M8-77 | C |
| F27-S77 | A |
| M8-78 | C |
| F27-S78 | C |
| M8-79 | C |
| F27-S79 | C |
| M8-80 | C |
| F27-S80 | C |
| M8-81 | C |
| F27-S81 | B |
| M4-82 | C |
| F27-S82 | C |
| M4-83 | C |
| F27-S83 | B |
| M4-84 | C |
| F27-S84 | B |
| M4-85 | C |
| F27-S85 | B |
| M8-86 | C |
| F27-S86 | B |
| M8-87 | C |
| F27-S87 | A |
| M8-88 | C |
| F27-S88 | A |
| M9-1 | C |
| F27-S89 | C |
| M10-90 | C |
| F27-S90 | C |
| M10-91 | C |
| F27-S91 | C |
| M10-92 | C |
| F27-S92 | B |
| M10-93 | C |
| F27-S93 | B |
| M10-94 | C |
| F27-S94 | A |
| M10-95 | C |
| F27-S95 | B |
| M10-96 | C |
| F27-S96 | A |
| M9-2 | C |
| F27-S97 | C |
| M10-98 | C |
| F27-S98 | A |
| M10-99 | C |
| F27-S99 | C |
| M10-100 | C |
| F27-S100 | B |
| M10-101 | C |
| F27-S101 | A |
| M10-102 | C |
| F27-S102 | C |
| M10-103 | C |
| F27-S103 | B |
| M10-104 | C |
| F27-S104 | C |
| M10-105 | C |
| F27-S105 | A |

**Table 1.2 FXR antagonistic activity of compounds**

| Compound number | FXR IC₅₀ (µM)^{a} |
|---|---|
| M10-106 | C |
| M10-107 | C |
| M10-108 | C |
| M10-109 | C |
| M10-110 | C |
| M10-111 | C |
| M10-112 | C |
| M10-113 | C |
| M10-114 | C |
| M10-115 | C |
| M10-116 | C |
| M10-117 | C |
| M10-118 | C |
| M10-119 | C |
| M10-120 | C |
| M10-121 | C |
| M10-122 | C |
| M10-123 | C |
| M10-124 | C |
| M10-125 | C |
| M10-126 | C |
| M10-127 | C |
| M10-128 | C |
| M10-129 | C |
| M10-130 | C |
| M10-131 | C |
| M10-132 | C |
| M10-133 | C |
| M10-134 | C |
| M10-135 | C |
| M10-136 | C |
| M10-137 | C |
| M10-138 | C |
| M10-139 | C |
| M10-140 | C |
| M10-141 | C |
| M10-142 | C |
| M10-143 | C |
| M10-144 | C |
| M10-145 | C |
| M10-146 | C |
| M10-147 | C |
| M10-148 | C |
| M10-149 | C |
| M10-150 | C |
| M10-151 | C |
| M10-152 | C |
| M10-153 | C |
| M10-154 | C |
| M10-155 | C |
| M10-156 | C |
| M10-157 | C |
| M10-158 | C |
| M10-159 | C |
| M10-160 | C |
| M10-161 | C |
| M10-162 | C |
| M10-163 | C |
| M10-164 | C |
| M10-165 | C |
| M10-166 | C |
| M10-167 | C |
| M10-168 | C |
| M10-169 | C |
| M10-170 | C |
| M10-171 | C |
| M10-172 | C |
| M10-173 | C |
| M10-174 | C |
| M10-175 | C |
| M10-176 | C |
| M10-177 | C |
| M10-178 | C |
| F27-S106 | A |
| F27-S107 | A |
| F27-S108 | A |
| F27-S109 | A |
| F27-S110 | A |
| F27-S111 | A |
| F27-S112 | A |
| F27-S113 | A |
| F27-S114 | A |
| F27-S115 | A |
| F27-S116 | A |
| F27-S117 | A |
| F27-S118 | A |
| F27-S119 | A |
| F27-S120 | A |
| F27-S121 | A |
| F27-S122 | A |
| F27-S123 | A |
| F27-S124 | A |
| F27-S125 | A |
| F27-S126 | A |
| F27-S127 | A |
| F27-S128 | A |
| F27-S129 | A |
| F27-S130 | A |
| F27-S131 | A |
| F27-S132 | A |
| F27-S133 | A |
| F27-S134 | A |
| F27-S135 | A |
| F27-S136 | A |
| F27-S137 | A |
| F27-S138 | A |
| F27-S139 | A |
| F27-S140 | A |
| F27-S141 | A |
| F27-S142 | A |
| F27-S143 | A |
| F27-S144 | A |
| F27-S145 | A |
| F27-S146 | A |
| F27-S147 | A |
| F27-S148 | A |
| F27-S149 | A |
| F27-S150 | A |
| F27-S151 | A |
| F27-S152 | A |
| F27-S153 | A |
| F27-S 154 | A |
| F27-S155 | A |
| F27-S156 | A |
| F27-S157 | A |
| F27-S158 | A |
| F27-S159 | A |
| F27-S160 | A |
| F27-S161 | A |
| F27-S162 | A |
| F27-S163 | A |
| F27-S164 | A |
| F27-S165 | A |
| F27-S166 | A |
| F27-S167 | A |
| F27-S168 | A |
| F27-S169 | A |
| F27-S170 | A |
| F27-S171 | A |
| F27-S172 | A |
| F27-S173 | A |
| F27-S174 | A |
| F27-S175 | A |
| F27-S176 | A |
| F27-S177 | A |
| F27-S178 | A |
| M10-179 | C |
| F27-S179 | A |
| M10-180 | C |
| F27-S180 | A |
| M10-181 | C |
| F27-S181 | A |
| M10-182 | C |
| F27-S182 | A |
| M10-183 | C |
| F27-S183 | A |
| F27-S184 | A |

| | |
|---|---|
| *^{a}* data are presented as geometric means of at least two independent runs. *^{b}*Z-GS, SIPI-7623 and Centatin are positive control. SIPI-7623 was in Phase I clinical research, and Centatin was in Phase III clinical research. | |

### Conclusion and discussion on lipid-lowering activity

Evaluation on cytotoxicity of compounds in human embryonic kidney cell line HEK293A and human liver cancer cell line HepG2. As shown in Table 1.3, all benzofuran FXR antagonists tested had no significant cytotoxicity (CC₅₀ > 160 µM).

**Table 1.3 Cytotoxicity results of benzofuran FXR antagonists**

| Compound | HEK293A | HepG2 |
|---|---|---|
| | CC₅₀ ( µM) | CC₅₀ ( µM) |
| F27-S11 | > 160 | > 160 |
| F27-S12 | > 160 | > 160 |
| F27-S96 | > 160 | > 160 |
| F27-S101 | > 160 | > 160 |
| F27-S105 | > 160 | > 160 |

The data in the table are the average of three independent repeated experiments.

The inventors selected Centatin in Phase III clinical research and SIPI-7623 and Z-GS in Phase I clinical research as positive control. Analysis of TG and TC results shows (Figure 1) that 20 µM Z-GS, SIPI-7623, Centatin, and F27-S96 can significantly reduce the increase in TG and TC levels of HepG2 cell induced by oleic acid and palmitate. Among the positive drugs, Centatin has the greatest effect, followed by SIPI-7623, and Z-GS worst. The compound F27-S96 achieves the similar effect in reducing TG and TC to SIPI-7623, and its inhibitory effect on TG and TC is dose-dependent to some extent.

## Claims

1. A benzofuran derivative of formula (I): 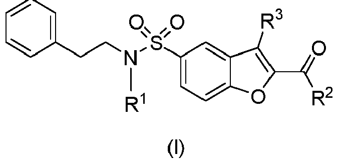
wherein, R¹ is -(CH₂)ₙ-R⁴, wherein n is an integer from 0 to 6, R⁴ is an unsubstituted aromatic hydrocarbyl, a substituted aromatic hydrocarbyl, an unsubstituted heteroaryl, or a substituted heteroaryl; the substituted aromatic hydrocarbyl or the substituted heteroaryl means that the aromatic hydrocarbyl or heteroaryl is substituted with one or more groups selected from Group A;
in formula (I), R² is a hydroxyl, an unsubstituted C1-C6 alkoxy, a substituted C1-C6 alkoxy, or -NR⁵R⁶, wherein R⁵ and R⁶ here are each independently selected from a group consisting of hydrogen, unsubstituted C1-C6 alkyl, and substituted C1-C6 alkyl; the substituted C1-C6 alkoxy means that one or more hydrogens on the C1-C6 alkoxy are substituted by groups selected from a group consisting of phenyl, halogen, nitro, hydroxyl and C1-C6 alkyl; the substituted C1-C6 alkyl means that one or more hydrogens on the C1-C6 alkyl are substituted by groups selected from a group consisting of phenyl, halogen, hydroxyl and C1-C6 alkyl;
R³ is an unsubstituted C1-C6 alkyl or a substituted C1-C6 alkyl;
the group in Group A refers to one or more of the following groups: halogen, nitro, cyano, substituted or unsubstituted aromatic hydrocarbyl, substituted or unsubstituted aryloxy, unsubstituted C1-C6 alkyl, unsubstituted C2-C6 alkenyl, carboxyl-substituted C2-C6 alkenyl, unsubstituted C2-C6 alkynyl, carboxyl-substituted C2-C6 alkynyl, carbamoyl, halogenated C1-C6 alkyl, aromatic hydrocarbyl-substituted C1-C6 alkyl, carboxyl-substituted C1-C6 alkyl, C1-C6 alkoxycarbonyl, unsubstituted C1-C6 alkoxy, halogenated C1-C6 alkoxy, unsubstituted C1-C6 alkylthio group, halogenated C1-C6 alkylthio group, C1-C6 alkylamide group, and substituted or unsubstituted heterocycloalkyl, wherein the substituted aromatic hydrocarbyl, substituted aryloxy or substituted heterocycloalkyl means that an aromatic hydrocarbyl, aryloxy or heterocycloalkyl is substituted with one or more groups selected from Group B, or that a C1-C6 alkyl and N in a heterocycloalkyl form a quaternary ammonium;
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C6 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, and C1-C6 alkoxycarbonyl;
it is also required that,
when R¹ is -(CH₂)ₙ-R⁴, n is 1, R⁴ is phenyl and R³ is methyl, R² is not hydroxyl;
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

2. The benzofuran derivative according to claim 1, wherein, in formula (I), R³ is methyl, and/or R² is hydroxyl.

3. The benzofuran derivative according to claim 1, wherein, in formula (I), R² is methoxy, ethoxy, n-propoxy, isopropoxy, n-butoxy, isobutoxy, sec-butoxy, tert-butoxy or benzyl; preferably, R² is ethoxy.

4. The benzofuran derivative according to claim 1, wherein, in formula (I), R² is -NR⁵R⁶, wherein R⁵ and R⁶ are each independently selected from a group consisting of hydrogen, methyl, ethyl, n-propyl, isopropyl, n-butyl, isobutyl, sec-butyl, tert-butyl, n-pentyl, neopentyl, benzyl, and phenethyl; preferably, R² is phenylethylamino.

5. The benzofuran derivative according to any one of claims 1 to 4, wherein, in formula (I), n is 0; R⁴ is phenyl, naphthyl, pyridyl, or quinoline, or, phenyl, naphthyl, pyridyl, or quinoline substituted with one or more of the following groups: benzyl, phenoxy, halogen, nitro, cyano, carboxyl, unsubstituted C1-C6 alkyl, halogenated C1-C6 alkyl, halogenated C1-C6 alkoxy, carbamoyl, halogenated C1-C6 alkylthio, C1-C6 alkanoyl, morpholinyl, piperazinyl, piperazinmethyl, and piperidinyl, wherein the piperazinyl, piperazinylmethyl and piperidinyl are optionally substituted with one or more of the following groups: unsubstituted C1-C6 alkyl, phenyl-substituted C1-C6 alkyl, unsubstituted C1-C6 alkoxycarbonyl, unsubstituted C1-C6 alkanoyl, phenyl-substituted C1-C6 alkanoyl, C1-C6 alkane sulfonyl, single- or double- C1-C6 substituted carbamoyl; or C1-C6 alkyl and N in morpholinyl, piperazinyl, piperazinmethyl, and piperidinyl form a quaternary ammonium.

6. The benzofuran derivative according to any one of claims 1 to 4, wherein, in formula (I), n is 1 or 2; R⁴ is phenyl, naphthyl, pyridyl, or quinoline, or, phenyl, naphthyl, pyridyl or quinoline substituted with one or more of the following groups: benzyl, phenoxy, halogen, nitro, cyano, carboxyl, unsubstituted C1-C6, halogenated C1-C4 alkyl, halogenated C1-C4 alkoxy, C1-C6 alkanoyl, carboxyl-substituted C1-C6 alkyl, C1-C6 alkoxycarbonyl, carbamoyl, halogenated C1-C4 alkylthio, unsubstituted C2-C6 alkenyl, carboxyl-substituted C2-C6 alkenyl, carbamoyl, morpholinyl, piperazinyl, piperazinylmethyl and piperidinyl, wherein the piperazinyl, piperazinylmethyl and piperidinyl are optionally substituted with one or more of the following groups: C1-C6 alkyl, phenyl-substituted C1-C6 alkyl, C1-C6 alkoxycarbonyl, C1-C6 alkanoyl, phenyl-substituted C1-C6 alkanoyl, C1-C4 alkane sulfonyl, single- or double-C1-C4 substituted carbamoyl.

7. The benzofuran derivative according to claim 1, is selected from the following compounds:
ethyl 3-methyl-5-(N-benzyl-N-phenethylsulfamoyl)benzofuran-2-carboxylate (F27-S8);
ethyl 3-methyl-5-(N-(2-fluorobenzyl)-N-phenethylsulfamoyl)benzofuran-2-carboxylate (M4-14);
ethyl 3-methyl-5-(N-(2-chlorobenzyl)-N-phenethylsulfamoyl)benzofuran-2-carboxylate (M4-15);
ethyl 3-methyl-5-(N-(2-bromobenzyl)-N-phenethylsulfamoyl)benzofuran-2-carboxylate (M4-16);
ethyl 3-methyl-5-(N-(2-methylbenzyl)-N-phenethylsulfamoyl)benzofuran-2-carboxylate (M4-17);
ethyl 3-methyl-5-(N-(2-trifluoromethylbenzyl)-N-phenethylsulfamoyl)benzofuran-2-carboxylate (M4-18);
ethyl 3-methyl-5-(N-(2-nitrobenzyl)-N-phenethylsulfamoyl)benzofuran-2-carboxylate (M4-19);
ethyl 3-methyl-5-(N-(3-fluorobenzyl)-N-phenethylsulfamoyl)benzofuran-2-carboxylate (M4-20);
ethyl 3-methyl-5-(N-(3-chlorobenzyl)-N-phenethylsulfamoyl)benzofuran-2-carboxylate (M4-21);
ethyl 3-methyl-5-(N-(3-bromobenzyl)-N-phenethylsulfamoyl)benzofuran-2-carboxylate (M4-22);
ethyl 3-methyl-5-(N-(3-methylbenzyl)-N-phenethylsulfamoyl)benzofuran-2-carboxylate (M4-23);
ethyl 3-methyl-5-(N-(3-trifluoromethylbenzyl)-N-phenethylsulfamoyl)benzofuran-2-carboxylate (M4-24);
ethyl 3-methyl-5-(N-(3-nitrobenzyl)-N-phenethylsulfamoyl)benzofuran-2-carboxylate (M4-25);
ethyl 3-methyl-5-(N-([1,1'-biphenyl]-3-ylmethyl)-N-phenethylsulfamoyl)benzofuran-2-carboxylate (M4-26);
ethyl 3-methyl-5-(N-3-phenoxybenzyl)-N-phenethylsulfamoyl)benzofuran-2-carboxylate (M4-27);
ethyl 3-methyl-5-(N-(4-fluorobenzyl)-N-phenethylsulfamoyl)benzofuran-2-carboxylate (M4-28);
ethyl 3-methyl-5-(N-(4-chlorobenzyl)-N-phenethylsulfamoyl)benzofuran-2-carboxylate (M4-29);
ethyl 3-methyl-5-(N-(4-bromobenzyl)-N-phenethylsulfamoyl)benzofuran-2-carboxylate (M4-30);
ethyl 3-methyl-5-(N-(4-methylbenzyl)-N-phenethylsulfamoyl)benzofuran-2-carboxylate (M4-31);
ethyl 3-methyl-5-(N-(4-trifluoromethylbenzyl)-N-phenethylsulfamoyl)benzofuran-2-carboxylate (M4-32);
ethyl 3-methyl-5-(N-(4-cyanobenzyl)-N-phenethylsulfamoyl)benzofuran-2-carboxylate (M4-33);
ethyl 3-methyl-5-(N-(4-(trifluoromethoxy)benzyl)-N-phenethylsulfamoyl)benzofuran-2-carboxylate (M4-34);
ethyl 3-methyl-5-(N-(4-(trifluoromethylthio)benzyl)-N-phenethylsulfamoyl)benzofuran-2-carboxylate (M4-35);
ethyl 3-methyl-5-(N-(4-isopropylbenzyl)-N-phenethylsulfamoyl)benzofuran-2-carboxylate (M4-36);
ethyl 3-methyl-5-(N-(4-(tert-butyl)benzyl)-N-phenethylsulfamoyl)benzofuran-2-carboxylate (M4-37);
ethyl 3-methyl-5-(N-(4-acetylbenzyl)-N-phenethylsulfamoyl)benzofuran-2-carboxylate (M4-38);
ethyl 3-methyl-5-(N-(4-(methoxycarbonyl)benzyl)-N-phenethylsulfamoyl)benzofuran-2-carboxylate (M4-39);
ethyl 3-methyl-5-(N-(4-(tert-butoxycarbonyl)benzyl)-N-phenethylsulfamoyl)benzofuran-2-carboxylate (M4-40);
ethyl 3-methyl-5-(N-(4-(2-methoxy-2-oxoethyl)benzyl)-N-phenethylsulfamoyl)benzofuran-2-carboxylate (M4-41);
(E)-ethyl 3-methyl-5-(N-(4-(3-methoxy-3-oxoprop-1-en-1-yl)benzyl)-N-phenethylsulfamoyl)benzofuran-2-carboxylate (M4-42);
ethyl 3-methyl-5-(N-(naphthalen-1-ylmethyl)-N-phenethylsulfamoyl)benzofuran-2-carboxylate (M4-43);
ethyl 3-methyl-5-(N-(quinolin-8-ylmethyl)-N-phenethylsulfamoyl)benzofuran-2-carboxylate (M4-44);
ethyl 3-methyl-5-(N-(naphthalen-2-ylmethyl)-N-phenethylsulfamoyl)benzofuran-2-carboxylate (M4-45);
ethyl 3-methyl-5-(N-(2,6-difluorobenzyl)-N-phenethylsulfamoyl)benzofuran-2-carboxylate (M4-46);
ethyl 3-methyl-5-(N-(2-fluoro-6-chlorobenzyl)-N-phenethylsulfamoyl)benzofuran-2-carboxylate (M4-47);
ethyl 3-methyl-5-(N-(2-fluoro-6-(trifluoromethyl)benzyl)-N-phenethylsulfamoyl)benzofuran-2-carboxylate (M4-48);
ethyl 3-methyl-5-(N-(2,6-dichlorobenzyl)-N-phenethylsulfamoyl)benzofuran-2-carboxylate (M4-49);
ethyl 3-methyl-5-(N-(2,6-dimethylbenzyl)-N-phenethylsulfamoyl)benzofuran-2-carboxylate (M4-50);
ethyl 3-methyl-5-(N-(2,4-difluorobenzyl)-N-phenethylsulfamoyl)benzofuran-2-carboxylate (M4-51);
ethyl 3-methyl-5-(N-(2-fluoro-4-chlorobenzyl)-N-phenethylsulfamoyl)benzofuran-2-carboxylate (M4-52);
ethyl 3-methyl-5-(N-(2-fluoro-4-bromobenzyl)-N-phenethylsulfamoyl)benzofuran-2-carboxylate (M4-53);
ethyl 3-methyl-5-(N-(2-fluoro-4-cyanobenzyl)-N-phenethylsulfamoyl)benzofuran-2-carboxylate (M4-54);
ethyl 3-methyl-5-(N-(2-fluoro-4-(trifluoromethyl)benzyl)-N-phenethylsulfamoyl)benzofuran-2-carboxylate (M4-55);
ethyl 3-methyl-5-(N-(2-chloro-4-fluorobenzyl)-N-phenethylsulfamoyl)benzofuran-2-carboxylate (M4-56);
ethyl 3-methyl-5-(N-(2-(trifluoromethyl)-4-fluorobenzyl)-N-phenethylsulfamoyl)benzofuran-2-carboxylate (M4-57);
ethyl 3-methyl-5-(N-(2,4-bis(trifluoromethyl)benzyl)-N-phenethylsulfamoyl)benzofuran-2-carboxylate (M4-58);
ethyl 3-methyl-5-(N-(2-trifluoromethyl-4-bromobenzyl)-N-phenethylsulfamoyl)benzofuran-2-carboxylate (M4-59);
ethyl 3-methyl-5-(N-(2,4-dichlorobenzyl)-N-phenethylsulfamoyl)benzofuran-2-carboxylate (M4-60);
ethyl 3-methyl-5-(N-(2-methyl-5-fluorobenzyl)-N-phenethylsulfamoyl)benzofuran-2-carboxylate (M4-61);
ethyl 3-methyl-5-(N-(2,6-difluoro-4-chlorobenzyl)-N-phenethylsulfamoyl)benzofuran-2-carboxylate (M4-62);
ethyl 3-methyl-5-(N-([1,1'-biphenyl]-4-ylmethyl-N-phenethylsulfamoyl)benzofuran-2-carboxylate (M4-82);
ethyl 3-methyl-5-(N-((2'-cyano-[1,1'-biphenyl]-4-yl)methyl)-N-phenethylsulfamoyl)benzofuran-2-carboxylate (M4-83);
ethyl 3-methyl-5-(N-((2'-(tert-butoxycarbonyl)-[1,1'-biphenyl]-4-yl)methyl)-N-phenethylsulfamoyl)benzofuran-2-carboxylate (M4-84);
ethyl 3-methyl-5-(N-(4-benzoylbenzyl)-N-phenethylsulfamoyl)benzofuran-2-carboxylate (M4-85);
3-methyl-5-(N-(2-fluorobenzyl)-N-phenethylsulfamoyl)benzofuran-2-carboxylic acid (F27-S14);
3-methyl-5-(N-(2-chlorobenzyl)-N-phenethylsulfamoyl)benzofuran-2-carboxylic acid (F27-S15);
3-methyl-5-(N-(2-bromobenzyl)-N-phenethylsulfamoyl)benzofuran-2-carboxylic acid (F27-S16);
3-methyl-5-(N-(2-methylbenzyl)-N-phenethylsulfamoyl)benzofuran-2-carboxylic acid (F27-S17);
3-methyl-5-(N-(2-trifluoromethylbenzyl)-N-phenethylsulfamoyl)benzofuran-2-carboxylic acid (F27-S18);
3-methyl-5-(N-(2-nitrobenzyl)-N-phenethylsulfamoyl)benzofuran-2-carboxylic acid (F27-S19);
3-methyl-5-(N-(3-fluorobenzyl)-N-phenethylsulfamoyl)benzofuran-2-carboxylic acid (F27-S20);
3-methyl-5-(N-(3-chlorobenzyl)-N-phenethylsulfamoyl)benzofuran-2-carboxylic acid (F27-S21);
3-methyl-5-(N-(3-bromobenzyl)-N-phenethylsulfamoyl)benzofuran-2-carboxylic acid (F27-S22);
3-methyl-5-(N-(3-methylbenzyl)-N-phenethylsulfamoyl)benzofuran-2-carboxylic acid (F27-S23);
3-methyl-5-(N-(3-trifluoromethylbenzyl)-N-phenethylsulfamoyl)benzofuran-2-carboxylic acid (F27-S24);
3-methyl-5-(N-(3-nitrobenzyl)-N-phenethylsulfamoyl)benzofuran-2-carboxylic acid (F27-S25);
3-methyl-5-(N-([1,1'-biphenyl]-3-ylmethyl)-N-phenethylsulfamoyl)benzofuran-2-carboxylic acid (F27-S26);
3-methyl-5-(N-(3-phenoxybenzyl)-N-phenethylsulfamoyl)benzofuran-2-carboxylic acid (F27-S27);
3-methyl-5-(N-(4-fluorobenzyl)-N-phenethylsulfamoyl)benzofuran-2-carboxylic acid (F27-S28);
3-methyl-5-(N-(4-chlorobenzyl)-N-phenethylsulfamoyl)benzofuran-2-carboxylic acid (F27-S29);
3-methyl-5-(N-(4-bromobenzyl)-N-phenethylsulfamoyl)benzofuran-2-carboxylic acid (F27-S30);
3-methyl-5-(N-(4-methylbenzyl)-N-phenethylsulfamoyl)benzofuran-2-carboxylic acid (F27-S31);
3-methyl-5-(N-(4-(trifluoromethyl)benzyl)-N-phenethylsulfamoyl)benzofuran-2-carboxylic acid (F27-S32);
3-methyl-5-(N-(4-carbamoylbenzyl)-N-phenethylsulfamoyl)benzofuran-2-carboxylic acid (F27-S33);
3-methyl-5-(N-(4-(trifluoromethoxy)benzyl)-N-phenethylsulfamoyl)benzofuran-2-carboxylic acid (F27-S34);
3-methyl-5-(N-(4-(trifluoromethylthio)benzyl)-N-phenethylsulfamoyl)benzofuran-2-carboxylic acid (F27-S35);
3-methyl-5-(N-(4-isopropylbenzyl)-N-phenethylsulfamoyl)benzofuran-2-carboxylic acid (F27-S36);
3-methyl-5-(N-(4-tert-butylbenzyl)-N-phenethylsulfamoyl)benzofuran-2-carboxylic acid (F27-S37);
3-methyl-5-(N-(4-acetylbenzyl)-N-phenethylsulfamoyl)benzofuran-2-carboxylic acid (F27-S38);
3-methyl-5-(N-(4-carboxybenzyl)-N-phenethylsulfamoyl)benzofuran-2-carboxylic acid (F27-S39);
3-methyl-5-(N-(4-(tert-butoxycarbonyl)benzyl)-N-phenethylsulfamoyl)benzofuran-2-carboxylic acid (F27-S40);
3-methyl-5-(N-(4-(carboxymethyl)benzyl)-N-phenethylsulfamoyl)benzofuran-2-carboxylic acid (F27-S41);
(E)-3-methyl-5-(N-(4-(2-carboxyvinyl)benzyl)-N-phenethylsulfamoyl)benzofuran-2-carboxylic acid (F27-S42);
3-methyl-5-(N-(naphthalen-2-ylmethyl)-N-phenethylsulfamoyl)benzofuran-2-carboxylic acid (F27-S43);
3-methyl-5-(N-(quinolin-8-ylmethyl)-N-phenethylsulfamoyl)benzofuran-2-carboxylic acid (F27-S44);
3-methyl-5-(N-(naphthalen-2-ylmethyl)-N-phenethylsulfamoyl)benzofuran-2-carboxylic acid (F27-S45);
3-methyl-5-(N-(2,6-difluorobenzyl)-N-phenethylsulfamoyl)benzofuran-2-carboxylic acid (F27-S46);
3-methyl-5-(N-(2-fluoro-6-chlorobenzyl)-N-phenethylsulfamoyl)benzofuran-2-carboxylic acid (F27-S47);
3-methyl-5-(N-(2-fluoro-6-(trifluoromethyl)benzyl)-N-phenethylsulfamoyl)benzofuran-2-carboxylic acid (F27-S48);
3-methyl-5-(N-(2,6-dichlorobenzyl)-N-phenethylsulfamoyl)benzofuran-2-carboxylic acid (F27-S49);
3-methyl-5-(N-(2,6-dimethylbenzyl)-N-phenethylsulfamoyl)benzofuran-2-carboxylic acid (F27-S50);
3-methyl-5-(N-(2,4-difluorobenzyl)-N-phenethylsulfamoyl)benzofuran-2-carboxylic acid (F27-S51);
3-methyl-5-(N-(2-fluoro-4-chlorobenzyl)-N-phenethylsulfamoyl)benzofuran-2-carboxylic acid (F27-S52);
3-methyl-5-(N-(2-fluoro-4-bromobenzyl)-N-phenethylsulfamoyl)benzofuran-2-carboxylic acid (F27-S53);
3-methyl-5-(N-(2-fluoro-4-(trifluoromethyl)benzyl)-N-phenethylsulfamoyl)benzofuran-2-carboxylic acid (F27-S55);
3-methyl-5-(N-(2-chloro-4-fluorobenzyl)-N-phenethylsulfamoyl)benzofuran-2-carboxylic acid (F27-S56);
3-methyl-5-(N-(2-(trifluoromethyl)-4-fluorobenzyl)-N-phenethylsulfamoyl)benzofuran-2-carboxylic acid (F27-S57);
3-methyl-5-(N-(2,4-bis(trifluoromethyl)benzyl)-N-phenethylsulfamoyl)benzofuran-2-carboxylic acid (F27-S58);
3-methyl-5-(N-(2-(trifluoromethyl)-4-bromobenzyl)-N-phenethylsulfamoyl)benzofuran-2-carboxylic acid (F27-S59);
3-methyl-5-(N-(2,4-dichlorobenzyl)-N-phenethylsulfamoyl)benzofuran-2-carboxylic acid (F27-S60);
3-methyl-5-(N-(2-methyl-5-fluorobenzyl)-N-phenethylsulfamoyl)benzofuran-2-carboxylic acid (F27-S61);
3-methyl-5-(N-(2,6-difluoro-4-chlorobenzyl)-N-phenethylsulfamoyl)benzofuran-2-carboxylic acid (F27-S62);
3-methyl-5-(N-([1,1'-biphenyl]-4-ylmethyl)-N-phenethylsulfamoyl)benzofuran-2-carboxylic acid (F27-S82);
3-methyl-5-(N-(2'-cyano-[1,1'-biphenyl]-4-yl)methyl)-N-phenethylsulfamoyl)benzofuran-2-carboxylic acid (F27-S83);
3-methyl-5-(N-((2'-(tert-butoxycarbonyl)-[1,1'-biphenyl]-4-yl)methyl)-N-phenethylsulfamoyl)benzofuran-2-carboxylic acid (F27-S84);
3-methyl-5-(N-(4-benzoylbenzyl)-N-phenethylsulfamoyl)benzofuran-2-carboxylic acid (F27-S85);
ethyl 3-methyl-5-(N-phenyl-N-phenethylsulfamoyl)benzofuran-2-carboxylate (M8-10);
ethyl 3-methyl-5-(N-(4-benzylphenyl)-N-phenethylsulfamoyl)benzofuran-2-carboxylate (M8-11);
ethyl 3-methyl-5-(N-(4-phenoxyphenyl)-N-phenethylsulfamoyl)benzofuran-2-carboxylate (M8-12);
ethyl 3-methyl-5-(N,N-diphenylethylsulfamoyl)benzofuran-2-carboxylate (M8-13);
ethyl 3-methyl-5-(N-(4-morpholinophenyl)-N-phenethylsulfamoyl)benzofuran-2-carboxylate (M8-63);
ethyl 3-methyl-5-(N-(4-(4-methylpiperazin-1-yl)phenyl)-N-phenethylsulfamoyl)benzofuran-2-carboxylate (M8-64);
ethyl 3-methyl-5-(N-(4-(4-(tert-butoxycarbonyl)piperazin-1-yl)phenyl)-N-phenethylsulfamoyl)benzofuran-2-carboxylate (M8-65);
ethyl 3-methyl-5-(N-(4-(4-(3,3-dimethylbutyryl)piperazin-1-yl)phenyl)-N-phenethylsulfamoyl)benzofuran-2-carboxylate (M8-66);
ethyl 3-methyl-5-(N-(4-(4-(propoxycarbonyl)piperazin-1-yl)phenyl)-N-phenethylsulfamoyl)benzofuran-2-carboxylate (M8-67);
ethyl 3-methyl-5-(N-(4-(4-acetylpiperazin-1-yl)phenyl)-N-phenethylsulfamoyl)benzofuran-2-carboxylate (M8-68);
ethyl 3-methyl-5-(N-(4-(4-benzoylpiperazin-1-yl)phenyl)-N-phenethylsulfamoyl)-3-methylbenzofuran-2-carboxylate (M8-69);
ethyl 3-methyl-5-(N-(4-(4-benzylpiperazin-1-yl)phenyl)-N-phenethylsulfamoyl)benzofuran-2-carboxylate (M8-70);
ethyl 3-methyl-5-(N-(4-(4-(dimethylcarbamoyl)piperazin-1-yl)phenyl)-N-phenethylsulfamoyl)benzofuran-2-carboxylate (M8-71);
ethyl 3-methyl-5-(N-(4-(4-(methylsulfonyl)piperazin-1-yl)phenyl)-N-phenethylsulfamoyl)benzofuran-2-carboxylate (M8-72);
ethyl 3-methyl-5-(N-(4-(1-(tert-butoxycarbonyl)piperidin-4-yl)phenyl)-N-phenethylsulfamoyl)benzofuran-2-carboxylate (M8-73);
ethyl 3-methyl-5-(N-(4-(4-(tert-butoxycarbonyl)piperazin-1-yl)-3-(trifluoromethyl)phenyl)-N-phenethylsulfamoyl)benzofuran-2-carboxylate (M8-74);
ethyl 3-methyl-5-(N-(6-(4-(tert-butoxycarbonyl)piperazin-1-yl) pyridin-3-yl)-N-phenethylsulfamoyl)benzofuran-2-carboxylate (M8-75);
ethyl 3-methyl-5-(N-(4-((4-(tert-butoxycarbonyl)piperazin-1-yl)methyl)phenyl)-N-phenethylsulfamoyl) benzofuran-2-carboxylate (M8-76);
ethyl 3-methyl-5-(N-(2-(4-(tert-butoxycarbonyl)piperazin-1-yl)phenyl)-N-phenethylsulfamoyl)benzofuran-2-carboxylate (M8-77);
ethyl 3-methyl-5-(N-(3-(4-methylpiperazin-1-yl)phenyl)-N-phenethylsulfamoyl)benzofuran-2-carboxylate (M8-78);
ethyl 3-methyl-5-(N-(4-morpholinobenzyl)-N-phenethylsulfamoyl)benzofuran-2-carboxylate (M8-79);
ethyl 3-methyl-5-(N-(4-(4-methylpiperazin-1-yl)benzyl)-N-phenethylsulfamoyl)benzofuran-2-carboxylate (M8-80);
ethyl 3-methyl-5-(N-(4-(4-(tert-butoxycarbonyl)piperazin-1-yl)benzyl)-N-phenethylsulfamoyl)benzofuran-2-carboxylate (M8-81);
ethyl 3-methyl-5-(N-phenethyl-N-(4-phenoxybenzyl)sulfamoyl)benzofuran-2-carboxylate (M8-86);
ethyl 3-methyl-5-(N-(2-(4-(tert-butoxycarbonyl)piperazin-1-yl)benzyl)-N-phenethylsulfamoyl)benzofuran-2-carboxylate (M8-87);
ethyl 3-methyl-5-(N-(3-(4-(tert-butoxycarbonyl)piperazin-1-yl)benzyl)-N-phenethylsulfamoyl)benzofuran-2-carboxylate (M8-88);
3-methyl-5-(N-phenethyl-N-phenylsulfamoyl)benzofuran-2-carboxylic acid (F27-S10);
3-methyl-5-(N-(4-benzylphenyl)-N-phenethylsulfamoyl)benzofuran-2-carboxylic acid (F27-S 11);
3-methyl-5-(N-(4-phenoxyphenyl)-N-phenethylsulfamoyl)benzofuran-2-carboxylic acid (F27-S12);
3-methyl-5-(N,N-diphenylethylsulfamoyl)benzofuran-2-carboxylic acid (F27-S13);
3-methyl-5-(N-(4-morpholinophenyl)-N-phenethylsulfamoyl)benzofuran-2-carboxylic acid (F27-S63);
3-methyl-5-(N-(4-(4-methylpiperazin-1-yl)phenyl)-N-phenethylsulfamoyl)benzofuran-2-carboxylic acid (F27-S64);
3-methyl-5-(N-(4-(4-(tert-butoxycarbonyl)piperazin-1-yl)phenyl)-N-phenethylsulfamoyl)benzofuran-2-carboxylic acid (F27-S65);
3-methyl-5-(N-(4-(4-(3,3-dimethylbutyryl)piperazin-1-yl)phenyl)-N-phenethylsulfamoyl)benzofuran-2-carboxylic acid (F27-S66);
3-methyl-5-(N-(4-(4-(propoxycarbonyl)piperazin-1-yl)phenyl)-N-phenethylsulfamoyl)benzofuran-2-carboxylic acid (F27-S67);
3-methyl-5-(N-(4-(4-acetylpiperazin-1-yl)phenyl)-N-phenethylsulfamoyl)benzofuran-2-carboxylic acid (F27-S68);
3-methyl-5-(N-(4-(4-benzoylpiperazin-1-yl)phenyl)-N-phenethylsulfamoyl)benzofuran-2-carboxylic acid (F27-S69);
3-methyl-5-(N-(4-(4-benzylpiperazin-1-yl)phenyl)-N-phenethylsulfamoyl)benzofuran-2-carboxylic acid (F27-S70);
3-methyl-5-(N-(4-(4-(dimethylcarbamoyl)piperazin-1-yl)phenyl)-N-phenethylsulfamoyl)benzofuran-2-carboxylic acid (F27-S71);
3-methyl-5-(N-(4-(4-(methylsulfonyl)piperazin-1-yl)phenyl)-N-phenethylsulfamoyl)benzofuran-2-carboxylic acid (F27-S72);
3-methyl-5-(N-(4-(1-(tert-butoxycarbonyl)piperidin-4-yl)phenyl)-N-phenethylsulfamoyl)benzofuran-2-carboxylic acid (F27-S73);
3-methyl-5-(N-(4-(4-(tert-butoxycarbonyl)piperazin-1-yl)-3-(trifluoromethyl)phenyl)-N-phenethylsulfamoyl)benzofuran-2-carboxylic acid (F27-S74);
3-methyl-5-(N-(6-(4-(tert-butoxycarbonyl)piperazin-1-yl)pyridin-3-yl)-N-phenethylsulfamoyl)benzofuran-2-carboxylic acid (F27-S75);
3-methyl-5-(N-(4-((4-(tert-butoxycarbonyl)piperazin-1-yl)methyl)phenyl)-N-phenethylsulfonamide)benzofuran-2-carboxylic acid (F27-S76);
3-methyl-5-(N-(2-(4-(tert-butoxycarbonyl)piperazin-1-yl)phenyl)-N-phenethylsulfamoyl)benzofuran-2-carboxylic acid (F27-S77);
3-methyl-5-(N-(3-(4-methylpiperazin-1-yl)phenyl)-N-phenethylsulfamoyl)benzofuran-2-carboxylic acid (F27-S78);
3-methyl-5-(N-(4-(4-morpholinobenzyl)-N-phenethylsulfamoyl)benzofuran-2-carboxylic acid (F27-S79);
3-methyl-5-(N-(4-(4-methylpiperazin-1-yl)benzyl)-N-phenethylsulfamoyl)benzofuran-2-carboxylic acid (F27-S80);
3-methyl-5-(N-(4-(4-(tert-butoxycarbonyl)piperazin-1-yl)benzyl)-N-phenethylsulfamoyl)benzofuran-2-carboxylic acid (F27-S81);
3-methyl-5-(N-(4-(4-phenoxybenzyl)-N-phenethylsulfamoyl)benzofuran-2-carboxylic acid (F27-S86);
3-methyl-5-(N-(2-(4-(tert-butoxycarbonyl)piperazin-1-yl)benzyl)-N-phenethylsulfamoyl)benzofuran-2-carboxylic acid (F27-S87);
3-methyl-5-(N-(3-(4-(tert-butoxycarbonyl)piperazin-1-yl)benzyl)-N-phenethylsulfamoyl)benzofuran-2-carboxylic acid (F27-S88);
ethyl 3-methyl-5-(N-phenethyl-N-(2-(piperazin-1-yl)phenyl)sulfamoyl)benzofuran-2-carboxylate (M9-1);
ethyl 3-methyl-5-(N-phenethyl-N-(2-(piperazin-1-yl)benzyl)sulfamoyl)benzofuran-2-carboxylate (M9-2);
4-(2-((2-(ethoxycarbonyl)-3-methyl-N-phenethylbenzofuran)-5-sulfonylamino)phenyl)-1,1-dimethylpiperazin-1-ium(M10-90);
ethyl 3-methyl-5-(N-(2-(4-acetylpiperazin-1-yl)phenyl)-N-phenethylsulfamoyl)benzofuran-2-carboxylate (M10-91);
ethyl 3-methyl-5-(N-(2-(4-acetylpiperazin-1-yl)phenyl)-N-phenethylsulfamoyl)benzofuran-2-carboxylate (M10-92);
ethyl 3-methyl-5-(N-(2-(4-(methylsulfonyl)piperazin-1-yl)phenyl)-N-phenethylsulfamoyl)benzofuran-2-carboxylate (M10-93);
ethyl 3-methyl-5-(N-(2-(4-(4-(3,3-dimethylbutyryl)piperazin-1-yl)phenyl)-N-phenethylsulfamoyl)benzofuran-2-carboxylate (M10-94);
ethyl 3-methyl-5-(N-(2-(4-(4-(benzylpiperazin-1-yl)phenyl)-N-phenethylsulfamoyl)benzofuran-2-carboxylate (M10-95);
ethyl 3-methyl-5-(N-(2-(4-(benzoylpiperazin-1-yl)phenyl)-N-phenethylsulfamoyl)benzofuran-2-carboxylate (M10-96);
ethyl 3-methyl-5-(N-(2-(4-methylpiperazin-1-yl)benzyl)-N-phenethylsulfamoyl)benzofuran-2-carboxylate (M10-98);
4-(2-((2-(ethoxycarbonyl)-3-methyl-N-benzylethylbenzofuran)-5-sulfonylamino)phenyl)-1,1-dimethylpiperazin-1-ium(M10-99);
ethyl 3-methyl-5-(N-(2-(4-acetylpiperazin-1-yl)benzyl)-N-phenethylsulfamoyl)benzofuran-2-carboxylate (M10-100);
ethyl 3-methyl-5-(N-(2-(4-acetylpiperazin-1-yl)benzyl)-N-phenethylsulfamoyl)benzofuran-2-carboxylate (M10-101);
ethyl 3-methyl-5-(N-(2-(4-methanesulfonylpiperazin-1-yl)benzyl)-N-phenethylsulfamoyl)benzofuran-2-carboxylate (M10-102);
ethyl 3-methyl-5-(N-(2-(4-(3,3-dimethylbutyryl)piperazin-1-yl)benzyl)-N-phenethylsulfamoyl)benzofuran-2-carboxylate (M10-103);
ethyl 3-methyl-5-(N-(2-(4-benzylpiperazin-1-yl)benzyl)-N-phenethylsulfamoyl)benzofuran-2-carboxylate (M10-104);
ethyl 3-methyl-5-(N-(2-(4-benzoylpiperazin-1-yl)benzyl)-N-phenethylsulfamoyl)benzofuran-2-carboxylate (M10-105);
3-methyl-5-(N-phenethyl-N-(2-(piperazin-1-yl)phenyl)sulfamoyl)benzofuran-2-carboxylic acid (F27-S89);
4-(2-((2-carboxy-3-methyl-N-phenethylbenzofuran)-5-sulfonylamino)phenyl)-1,1-dimethylpiperazin-1-ium(F27-S90);
3-methyl-5-(N-(2-(4-acetylpiperazin-1-yl)phenyl)-N-phenethylsulfamoyl)benzofuran-2-carboxylic acid (F27-S91);
3-methyl-5-(N-(2-(4-(dimethylcarbamoyl)piperazin-1-yl)phenyl)-N-phenethylsulfamoyl)benzofuran-2-carboxylic acid (F27-S92);
3-methyl-5-(N-(2-(4-(methylsulfonyl)piperazin-1-yl)phenyl)-N-phenethylsulfamoyl)benzofuran-2-carboxylic acid (F27-S93);
3-methyl-5-(N-(2-(4-(4-(3,3-dimethylbutyryl)piperazin-1-yl)phenyl)-N-phenethylsulfamoyl)benzofuran-2-carboxylic acid (F27-S94);
3-methyl-5-(N-(2-(4-(4-(benzylpiperazin-1-yl)phenyl)-N-phenethylsulfamoyl)benzofuran-2-carboxylic acid (F27-S95);
3-methyl-5-(N-(2-(4-(benzoylpiperazin-1-yl)phenyl)-N-phenethylsulfamoyl)benzofuran-2-carboxylic acid (F27-S96);
3-methyl-5-(N-(2-(piperazin-1-yl)benzyl)-N-phenethylsulfamoyl)benzofuran-2-carboxylic acid (F27-S97);
3-methyl-5-(N-(2-(4-acetylpiperazin-1-yl)benzyl)-N-phenethylsulfamoyl)benzofuran-2-carboxylic acid (F27-S98);
4-(2-((2-carboxy-3-methyl-N-phenethylbenzofuran)-5-sulfonamido)methyl)phenyl)-1,1-dimethylpiperazin-1-ium(F27-S99);
3-methyl-5-(N-(2-(4-acetylpiperazin-1-yl)benzyl)-N-phenethylsulfamoyl)benzofuran-2-carboxylicacid (F27-S100);
3-methyl-5-(N-(2-(4-(dimethylcarbamoyl)piperazin-1-yl)benzyl)-N-phenethylsulfamoyl)benzofuran-2-carboxylic acid (F27-S101);
3-methyl-5-(N-(2-(4-(methylsulfonyl)piperazin-1-yl)benzyl)-N-phenethylsulfamoyl)benzofuran-2-carboxylic acid (F27-S102);
3-methyl-5-(N-(2-(4-(4-(3,3-dimethylbutyryl)piperazin-1-yl)benzyl)-N-phenethylsulfamoyl)benzofuran-2-carboxylic acid (F27-S103);
3-methyl-5-(N-(2-(4-benzylpiperazin-1-yl)benzyl)-N-phenethylsulfamoyl)benzofuran-2-carboxylic acid (F27-S104) and
3-methyl-5-(N-(2-(4-benzoylpiperazin-1-yl)benzyl)-N-phenethylsulfamoyl)benzofuran-2-carboxylic acid (F27-S105);
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

8. A pharmaceutical composition, comprising the benzofuran derivative according to any one of claims 1 to 7.

9. Use of a benzofuran derivative of formula (II) as an FXR modulator: 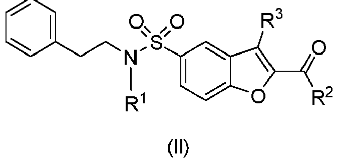
wherein, R¹ is -(CH₂)ₙ-R⁴, wherein n is an integer from 0 to 6, R⁴ is an unsubstituted aromatic hydrocarbyl, a substituted aromatic hydrocarbyl, an unsubstituted heteroaryl, or a substituted heteroaryl; the substituted aromatic hydrocarbyl or the substituted heteroaryl means that the aromatic hydrocarbyl or heteroaryl is substituted with one or more groups selected from Group A;
in formula (II), R² is a hydroxyl, an unsubstituted C1-C6 alkoxy, a substituted C1-C6 alkoxy, or -NR⁵R⁶, wherein R⁵ and R⁶ here are each independently selected from a group consisting of hydrogen, unsubstituted C1-C6 alkyl, and substituted C1-C6 alkyl; the substituted C1-C6 alkoxy means that one or more hydrogens on the C1-C6 alkoxy are substituted by groups selected from a group consisting of phenyl, halogen, nitro, hydroxyl and C1-C6 alkyl; the substituted C1-C6 alkyl means that one or more hydrogens on the C1-C6 alkyl are substituted by groups selected from a group consisting of phenyl, halogen, hydroxyl and C1-C6 alkyl;
R³ is an unsubstituted C1-C6 alkyl or a substituted C1-C6 alkyl;
the group in Group A refers to one or more of the following groups: halogen, nitro, cyano, substituted or unsubstituted aromatic hydrocarbyl, substituted or unsubstituted aryloxy, unsubstituted C1-C6 alkyl, unsubstituted C2-C6 alkenyl, carboxyl-substituted C2-C6 alkenyl, unsubstituted C2-C6 alkynyl, carboxyl-substituted C2-C6 alkynyl, carbamoyl, halogenated C1-C6 alkyl, aromatic hydrocarbyl-substituted C1-C6 alkyl, carboxyl-substituted C1-C6 alkyl, C1-C6 alkoxycarbonyl, unsubstituted C1-C6 alkoxy, halogenated C1-C6 alkoxy, unsubstituted C1-C6 alkylthio group, halogenated C1-C6 alkylthio group, C1-C6 alkylamide group, and substituted or unsubstituted heterocycloalkyl, wherein the substituted aromatic hydrocarbyl, substituted aryloxy or substituted heterocycloalkyl means that an aromatic hydrocarbyl, aryloxy or heterocycloalkyl is substituted with one or more groups selected from Group B, or that a C1-C6 alkyl and N in a heterocycloalkyl form a quaternary ammonium;
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C6 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, and C1-C6 alkoxycarbonyl;
or a pharmaceutically acceptable salt or ester, stereoisomer, tautomer, geometric isomer, prodrug, hydrate, solvate and crystal form of the benzofuran derivative, metabolite forms thereof, or any combination or mixture thereof.

10. The use according to claim 9, wherein, the use of the benzofuran derivative as an FXR modulator, preferably, as an FXR antagonist or inhibitor, includes but is not limited to lipid-lowering activity, anti-hyperlipidemic activity, anti-dyslipidemic activity, anti-atherosclerotic activity, anti-obesity activity, and/or anti-diabetic activity.

11. A compound of formula (I), or a pharmaceutically acceptable salt or ester, stereoisomer, tautomer, geometric isomer, prodrug, hydrate, solvate and crystal form of the compound, metabolite forms thereof, or any combination or mixture thereof; 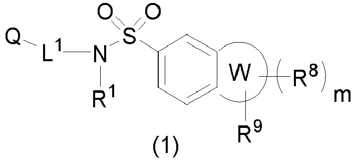
wherein, Q is selected from a group consisting of hydrogen, 6- to 10-membered aryl, 5- to 14-membered heteroaryl, 3- to 6-membered cycloalkyl, and 3- to 6-membered heterocyclyl; optionally, the 6- to 10-membered aryl, the 5- to 14-membered heteroaryl, the 3- to 6-membered cycloalkyl, and the 3- to 6-membered heterocyclyl are substituted with one or more M, M is selected from the following groups: halogen, nitro, hydroxyl, cyano, C₁-C₆ alkyl, C₂-C₆ alkenyl, C₂-C₆ alkynyl, C₁-C₆ haloalkyl, C₁-C₆ alkoxy, C₁-C₆ alkanoyl, and C₁-C₆ amido;
L¹ is absent or selected from -(CH₂)ₙ-, n is an integer selected from 1 to 6; optionally, - (CH₂)ₙ- is substituted with one or more groups selected from the following: phenyl, halogen , nitro, cyano, hydroxyl, C₁-C₆ alkyl, deuterium, and tritium;
R¹ is -(CH₂)ₙ-R⁴, wherein n is an integer from 0 to 6, R⁴ and the group in Group A are as defined in any one of claims 1-6 or 9.
the group in Group B is selected from: cyano, aromatic hydrocarbyl-substituted C1-C6 alkanoyl, aromatic hydrocarbyl-substituted C1-C6 alkyl, unsubstituted C1-C6 alkyl, unsubstituted C1-C7 alkanoyl, unsubstituted C1-C6 alkane sulfonyl, aromatic hydrocarbyl-substituted C1-C6 alkane sulfonyl, single- or double- C1-C6 substituted carbamoyl, carbamoyl, C1-C6 alkoxycarbonyl, unsubstituted 3- to 6-membered cycloalkylcarbonyl, substituted 3- to 6-membered cycloalkylcarbonyl, substituted 5- to 14-membered heteroarylcarbonyl, unsubstituted 5- to 14-membered heteroarylcarbonyl, unsubstituted C2-C6 alkenylcarbonyl, unsubstituted 5- to 10-membered heterocyclylcarbonyl, and substituted 5- to 10-membered heterocyclylcarbonyl;
W is 5- to 6-membered heteroaryl;
R⁸ is selected from hydrogen, halogen, unsubstituted C₁-C₆ alkyl or substituted C₁-C₆ alkyl; m is selected from 1, 2, 3, and 4; in a case of multiple R⁸, all the R⁸ can be the same or different;
R⁹ is selected from -CH₂OH or -(C=O)R², and R² is as defined in any one of claims 1-6.

12. The compound, or a pharmaceutically acceptable salt or ester, stereoisomer, tautomer, geometric isomer, prodrug, hydrate, solvate and crystal form of the compound, metabolite forms thereof, or any combination or mixture thereof according to claim 11;
wherein, Q is selected from 6- to 10-membered aryl, 5- to 8-membered monocyclic heteroaryl, 8- to 14-membered fused heteroaryl, 5- to 6-membered cycloalkyl, and 5- to 6-membered heterocyclyl; optionally, the 6- to 10-membered aryl, the 5- to 8-membered monocyclic heteroaryl, the 8- to 14-membered fused heteroaryl, the 5- to 6-membered cycloalkyl, and the 5- to 6-membered heterocyclyl are substituted with one or more M.
preferably, Q is selected from 6- to 10-membered aryl, 5- to 8-membered monocyclic heteroaryl, and 5- to 6-membered cycloalkyl;
preferably, Q is selected from phenyl, thienyl, and cyclohexyl;
preferably, Q is phenyl.

13. The compound, or a pharmaceutically acceptable salt or ester, stereoisomer, tautomer, geometric isomer, prodrug, hydrate, solvate and crystal form of the compound, metabolite forms thereof, or any combination or mixture thereof according to claim 11 or 12;
wherein, L¹ is absent or selected from -(CH₂)ₙ-, wherein n is 1, 2 or 3;
preferably, L¹ is -(CH₂)₂-.

14. The compound, or a pharmaceutically acceptable salt or ester, stereoisomer, tautomer, geometric isomer, prodrug, hydrate, solvate and crystal form of the compound, metabolite forms thereof, or any combination or mixture thereof according to any one of claims 11-13;
wherein, R¹ is phenyl substituted with one or more groups selected from Group A;
preferably, A is heterocycloalkyl that is unsubstituted or substituted with one or more groups selected from Group B;
preferably, the heterocyclyl is 5- to 7-membered monoheterocyclyl, 9- to 10-membered spiroheterocyclyl, or 6- to 8-membered bridged heterocyclyl;
preferably, the heterocyclyl is 5- to 7-membered nitrogen-containing monoheterocyclyl, 9- to 10-membered nitrogen-containing spiroheterocyclyl, or 6- to 8-membered nitrogen-containing bridged heterocyclyl;
preferably, the heterocyclyl is piperazinyl;
preferably, B is selected from: -(C=O)-phenyl, -(C=O)-5- to 6-membered heteroaryl, - (C=O)-propenyl, -(C=O)-5- to 6-membered cycloalkyl, -(C=O)-5- to 6-membered heterocyclyl, and -(C=O)-C₁-C₆ alkyl;
optionally, the phenyl, the 5- to 6-membered heteroaryl, the 5- to 6-membered cycloalkyl, 5- to 6-membered heterocyclyl are substituted with 1, 2 or 3 substituents selected from the following: C₁-C₆ alkyl (e.g., methyl), formamido, acetamido, C₁-C₆ haloalkyl (e.g., trifluoromethyl), cyano, halogen (e.g., fluorine, chlorine, bromine), amino, nitro, C₁-C₆ alkanoyl (e.g., acetyl), C₁-C₆ alkoxy (e.g., methoxy), benzyl, and ethinyl phenyl;
preferably, the 5- to 6-membered heteroaryl is selected from pyridyl, thienyl, furyl, pyrrolyl, imidazolyl, oxazolyl, thiazolyl, and pyrazolyl; optionally, the 5- to 6-membered heteroaryl is substituted with one or more C₁-C₆ alkyls (e.g., methyl) or halogen (e.g., bromine);
preferably, the 5- to 6-membered heterocyclyl is tetrahydrothiophenyl;
preferably, B is selected from -(C=O)-phenyl and -(C=O)-thienyl.

15. The compound, or a pharmaceutically acceptable salt or ester, stereoisomer, tautomer, geometric isomer, prodrug, hydrate, solvate and crystal form of the compound, metabolite forms thereof, or any combination or mixture thereof according to any one of claims 11-14, the compound is as represented by formula (2), (3), (4), or (5), 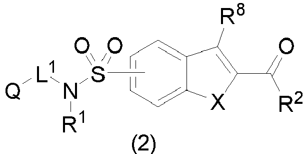 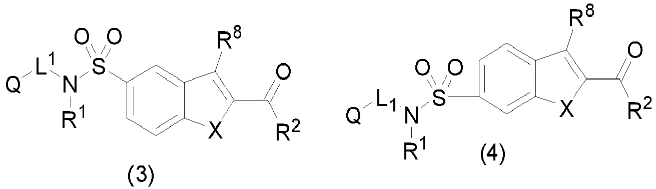 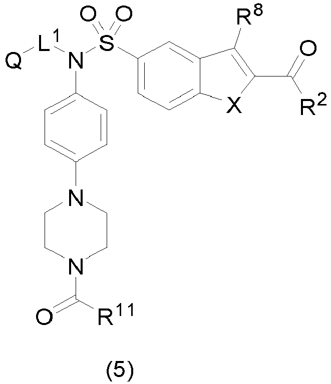
wherein, X is O, S, or N-R⁸, and R⁸ is hydrogen or unsubstituted C1-C6 alkyl;
preferably, in formula (2), (3), (4), or (5), R⁸ is methyl;
preferably, in formula (2), (3), (4), or (5), R² is hydroxyl or unsubstituted C1-C6 alkoxy.

16. The compound, or a pharmaceutically acceptable salt or ester, stereoisomer, tautomer, geometric isomer, prodrug, hydrate, solvate and crystal form of the compound, metabolite forms thereof, or any combination or mixture thereof according to any one of claims 11-15, the compound is selected from:

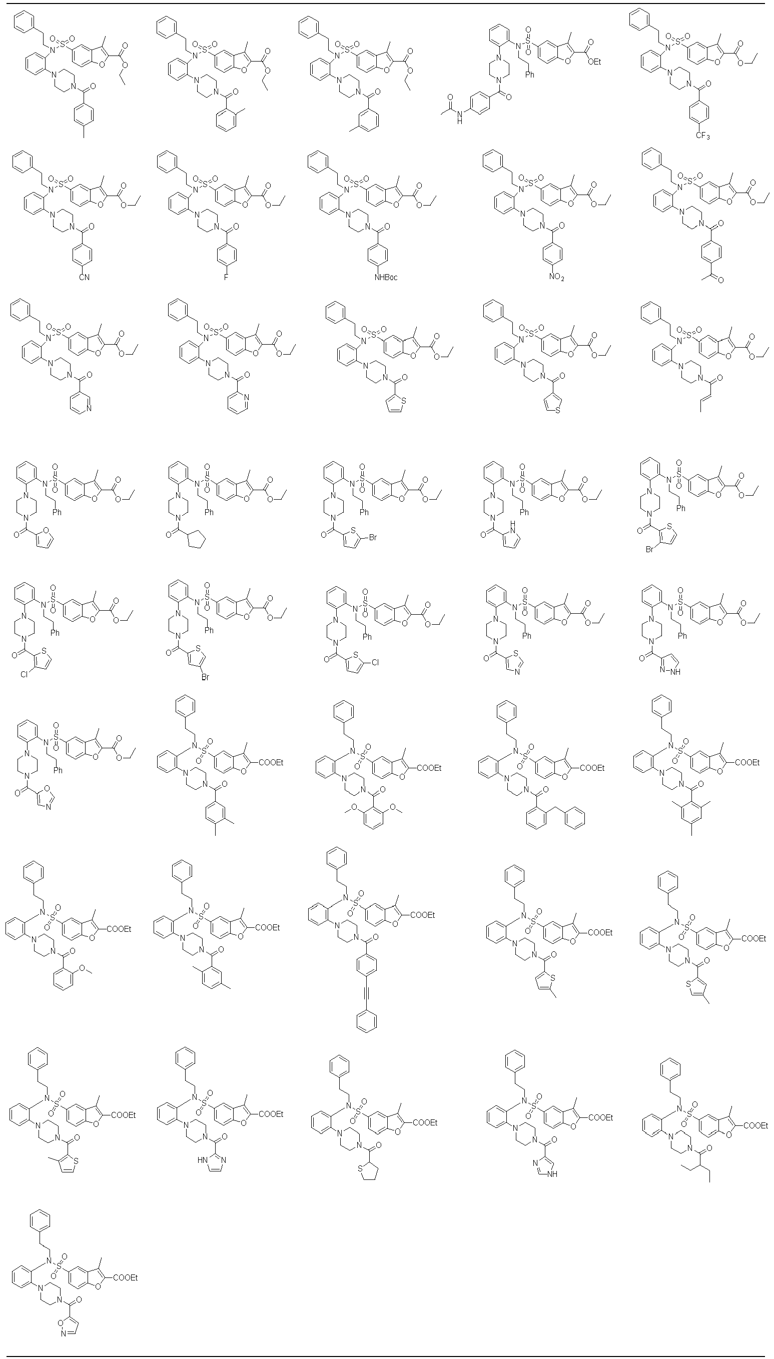

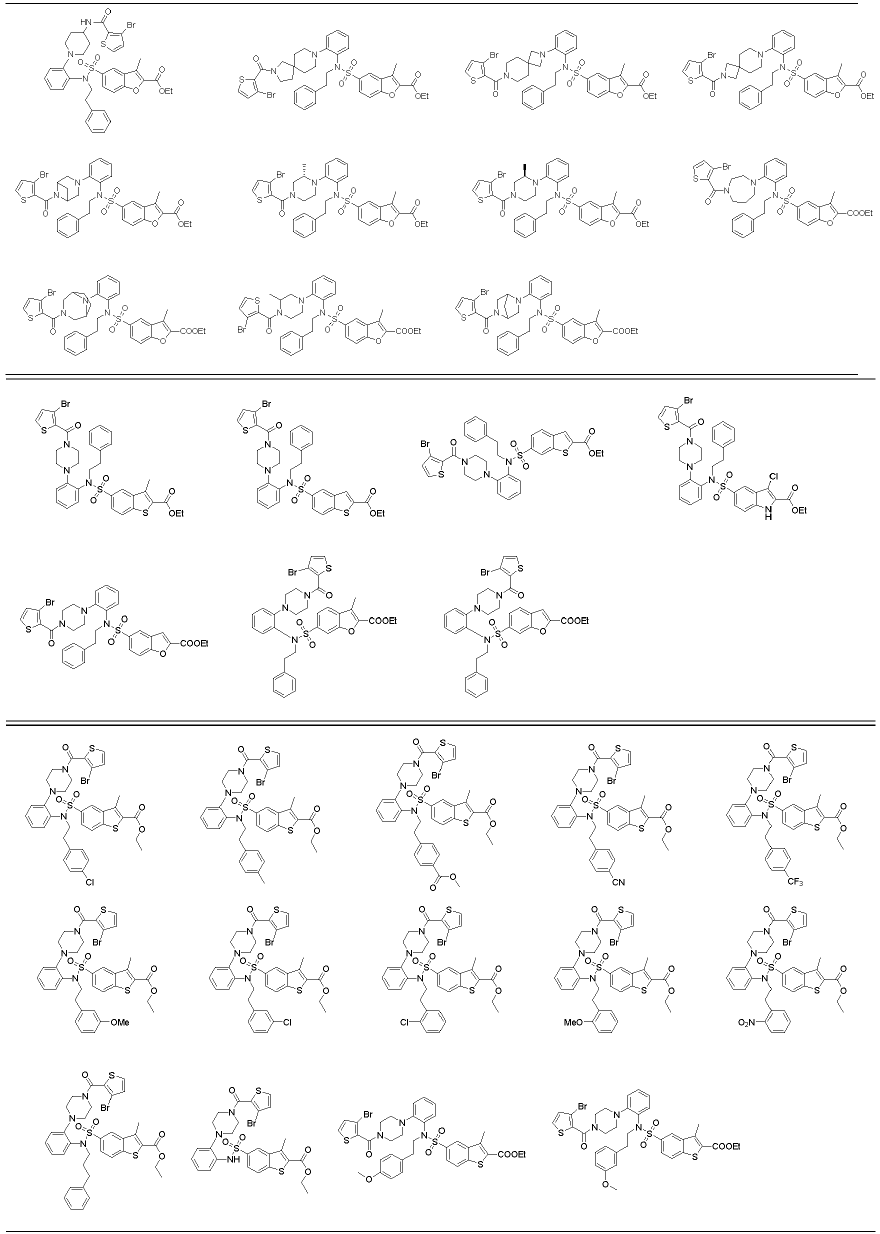

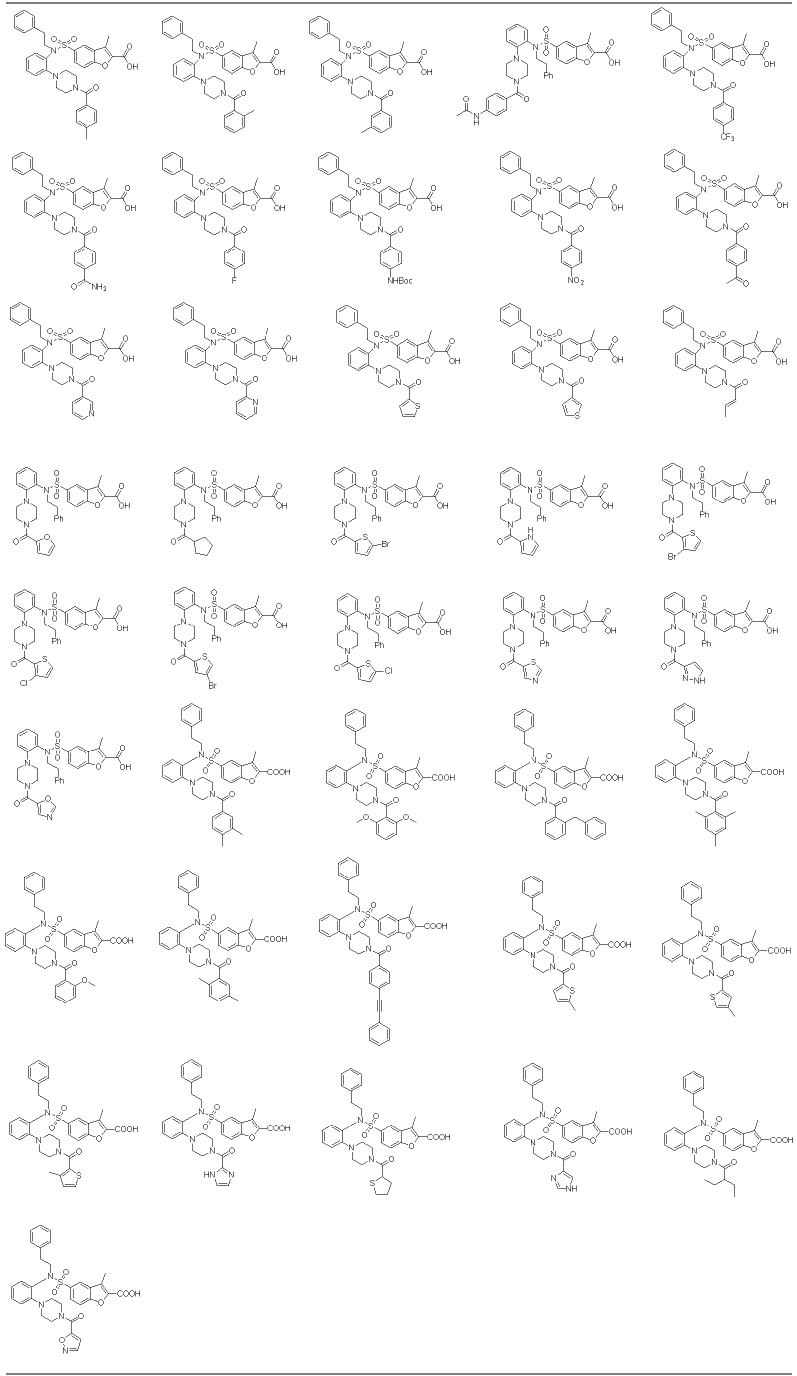

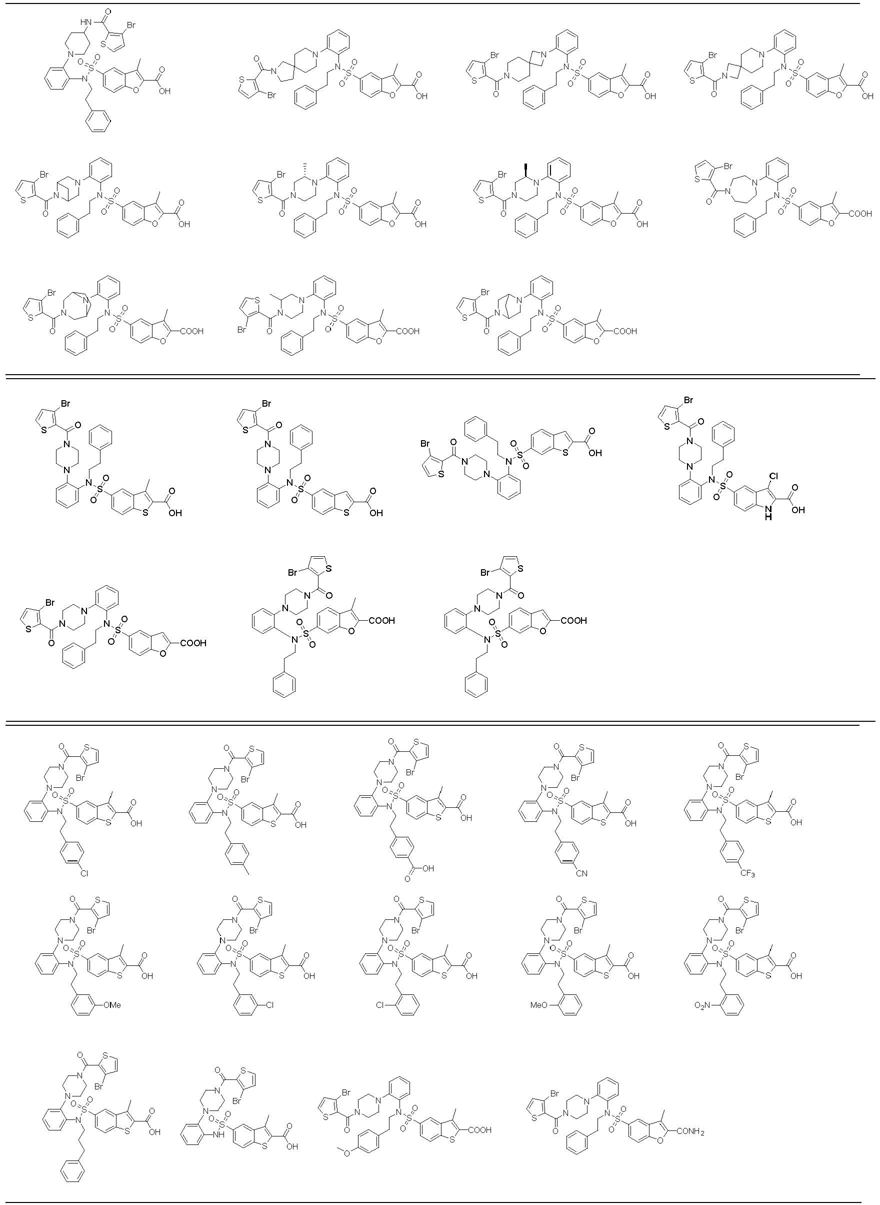

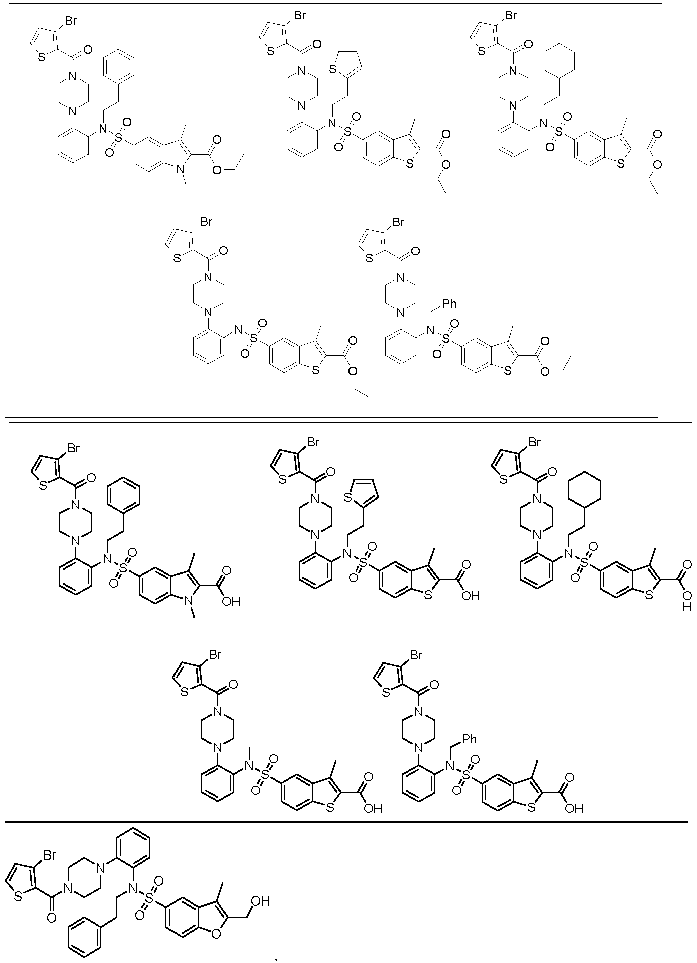

17. A pharmaceutical composition, comprising the compound, or a pharmaceutically acceptable salt or ester, stereoisomer, tautomer, geometric isomer, prodrug, hydrate, solvate and crystal form of the compound, metabolite forms thereof according to any one of claims 11-16, or any combination or mixture thereof;

18. Use of the compound, or a pharmaceutically acceptable salt or ester, stereoisomer, tautomer, geometric isomer, prodrug, hydrate, solvate and crystal form of the compound, metabolite forms thereof, or any combination or mixture thereof according to any one of claims 1-7 or 11-16 in preparation of a medicament, wherein the medicament is used for treating an FXR-related disease;
preferably, the compound, or a pharmaceutically acceptable salt or ester, stereoisomer, tautomer, geometric isomer, prodrug, hydrate, solvate and crystal form of the compound, metabolite forms thereof, or any combination or mixture thereof is used as an FXR agonist or FXR antagonist;
preferably, the FXR-related disease is selected from metabolism-related diseases, cardiovascular diseases, liver diseases, cancers, kidney diseases, enteropathies, inflammation or any combination thereof;
preferably, the metabolism-related diseases are selected from glycolipid metabolism diseases, cholesterol metabolism diseases, lipid metabolism diseases, bile acid metabolism diseases or any combination thereof;
preferably, the glycolipid metabolism diseases are selected from obesity, diabetes (such as type 2 diabetes), dyslipidemia, hyperlipidemia, hypercholesterolemia, cholestasis, non-alcoholic fatty liver disease, non-alcoholic steatohepatitis or any combination thereof;
preferably, the cardiovascular diseases are selected from atherosclerosis, myocardial ischemia-reperfusion injury, or combinations thereof;
preferably, the liver diseases are selected from liver resection, liver injury, or combinations thereof;
preferably, the cancer is selected from breast cancer, melanoma, meningioma, soft tissue sarcoma, salivary gland tumor, primary liver cancer, intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia, gastric cancer, nasopharyngeal cancer, laryngeal cancer, oral cancer, esophageal cancer, lung cancer, kidney cancer, cervical cancer, choriocarcinoma, vulvar cancer, skin cancer, endometrial cancer, ovarian cancer, prostate cancer, pancreatic cancer, colon cancer, rectum cancer, colorectal cancer, Kaposi's sarcoma, non-melanoma skin cancer (including squamous cell carcinoma and basal cell carcinoma), hemangioma, glioma, and secondary complications of these diseases;
preferably, the kidney diseases are selected from nephritis, nephrotic syndrome, atheroembolic renal disease, or any combination thereof.
